(19) Europäisches Patentamt
European Patent Office
Office européen des brevets

(11) EP 4 487 874 A1

(12) EUROPEAN PATENT APPLICATION
published in accordance with Art. 153(4) EPC

(43) Date of publication:
**08.01.2025 Bulletin 2025/02**

(21) Application number: **23760232.1**

(22) Date of filing: **28.02.2023**

(51) International Patent Classification (IPC):
***A61K 47/68*** *(2017.01)*

(52) Cooperative Patent Classification (CPC):
**A61K 31/337; A61K 31/357; A61K 31/407; A61K 31/4375; A61K 31/4745; A61K 31/475; A61K 31/513; A61K 31/537; A61K 31/5513; A61K 31/5517; A61K 31/675; A61K 31/7034; A61K 31/7036; A61K 31/706; A61K 38/07;** (Cont.)

(86) International application number:
**PCT/JP2023/007434**

(87) International publication number:
**WO 2023/163234 (31.08.2023 Gazette 2023/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **28.02.2022 JP 2022030070**
**15.04.2022 JP 2022067814**
**30.06.2022 JP 2022106430**
**06.01.2023 JP 2023001221**

(71) Applicant: **UBE Corporation**
**Ube-shi, Yamaguchi 755-8633 (JP)**

(72) Inventors:
- **TSUZAKI, Yasunori**
**Ube-shi, Yamaguchi 755-8633 (JP)**
- **MIZUNO, Gen**
**Ube-shi, Yamaguchi 755-8633 (JP)**
- **KASHIWAGI, Takamasa**
**Ube-shi, Yamaguchi 755-8633 (JP)**
- **TANAKA, Masayuki**
**Ube-shi, Yamaguchi 755-8633 (JP)**
- **SHIMIZU, Hayato**
**Ube-shi, Yamaguchi 755-8633 (JP)**
- **NONOUCHI, Shimpei**
**Ube-shi, Yamaguchi 755-8633 (JP)**
- **MATSUSHITA, Takashi**
**Ube-shi, Yamaguchi 755-8633 (JP)**
- **KIMURA, Tomio**
**Tokyo 108-0075 (JP)**

(74) Representative: **Hoffmann Eitle**
**Patent- und Rechtsanwälte PartmbB**
**Arabellastraße 30**
**81925 München (DE)**

(54) **ANTIBODY-DRUG CONJUGATE PRECURSOR AND INTERMEDIATE FOR SYNTHESIS THEREOF**

(57) The present invention provides a method for improving the stability of an antibody-drug conjugate in a living body, and a conjugate precursor for producing a stabilized antibody-drug conjugate. Specifically, the present invention provides an antibody-drug conjugate precursor represented by the following general formula (I):

Z-L-D (I)

[wherein:
Z is a reactive group which can react with a functional group present in an antibody or a modified antibody;
D is an antitumor drug residue in which one hydrogen atom or one hydroxy group is removed from any position of an antitumor drug molecule or an analog thereof or a derivative thereof;
L is a linker which links Z to D; and
at least any one of D and L has one or more lactonyl group(s) at any position(s) as substituent(s) or protecting group(s)] or a salt thereof, a synthetic intermediate thereof or a salt thereof, and the like.

EP 4 487 874 A1

Figure 1

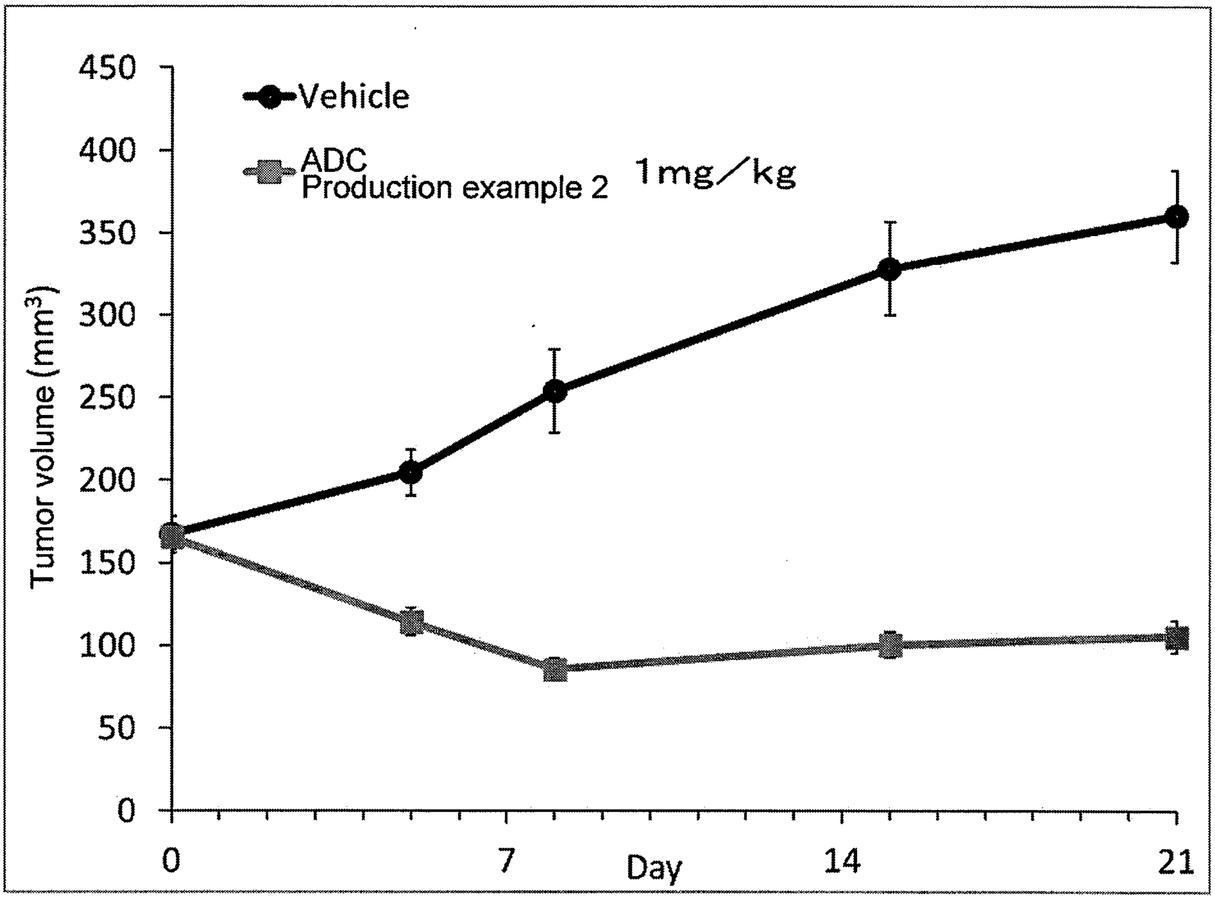


(52) Cooperative Patent Classification (CPC): (Cont.)
**A61K 38/14; A61K 39/395; A61K 45/00;**
**A61K 47/68; A61P 35/00; C07K 2/00;**
**C07K 14/435; C07K 16/30; C07K 19/00;**
Y02P 20/55

## Description

### TECHNICAL FIELD

The present invention relates to an antibody-drug conjugate precursor (hereinafter also referred to as "conjugate precursor") useful for synthesizing an "antibody-drug conjugate" (Antibody Drug Conjugate; ADC) used as an antitumor drug, and a synthetic intermediate thereof (hereinafter also referred to as "synthetic intermediate of conjugate precursor").

### BACKGROUND ART

An antibody-drug conjugate (Antibody Drug Conjugate; ADC) in which a cytotoxic drug (payload) is conjugated to an antibody that binds to an antigen expressed on the surface of a cancer cell and can be internalized in the cell, can be expected to "selectively deliver the drug to the cancer cell, release and accumulate the drug in the cancer cell, and kill the cancer cell" (Nonpatent Documents 1 to 2). As examples of ADC, Adcetris (brentuximab vedotin) (Patent Document 1) in which monomethyl auristatin E is conjugated to an anti-CD30 antibody is approved as a therapeutic drug for Hodgkin's lymphoma and anaplastic large cell lymphoma, Kadcyla (trastuzumab emtansine) in which emtansine is conjugated to an anti-HER2 antibody is approved as a therapeutic drug for HER2-positive metastatic breast cancer, Enhertu (trastuzumab deruxtecan) in which deruxtecan is conjugated to an anti-HER2 antibody is approved as a therapeutic drug for HER2-positive metastatic breast cancer (Patent Document 2), Trodelvy (sacituzumab govitecan) in which SN-38 is conjugated to an anti-TROP-2 antibody is approved as a therapeutic drug for TROP-2-positive metastatic breast cancer, Blenrep (belantamab mafodotin) in which MMAF is conjugated to an anti-CD269 (BCMA) antibody is approved as a therapeutic drug for multiple myeloma, Tivdak (tisotumab vedotin) in which monomethyl auristatin E is conjugated to an anti-CD142 antibody is approved as a therapeutic drug for metastatic cervical cancer, and Zynlonta (loncastuximab tesirine) in which pyrrolobenzodiazepine dimer is conjugated to an anti-CD19 antibody is approved as a therapeutic drug for B cell lymphoma (Nonpatent Documents 3 to 10). In addition, many novel ADCs are under clinical development as antitumor drugs (Nonpatent Documents 11 to 14).

In order for an ADC to exert an excellent antitumor effect, it is necessary to be stable in the body (blood) after administration. However, in general, when DAR (the average number of bound drug per antibody, or the drug/antibody ratio) increases, due to the reasons such as the increase in hydrophobicity of the ADC resulting in the tendency for aggregation to occur, the pharmacokinetics deteriorates and the drug efficacy is weakened. For example, it is reported that an ADC with MMAE (monomethyl auristatin E) as a payload is the most stable and the in vivo drug efficacy is potent when the DAR is around 3 to 4, and when the DAR is higher than that, it is destabilized and the drug efficacy is weakened (Nonpatent Document 15). In order to solve this problem, research to increase the hydrophilicity of the ADC has been actively carried out (Patent Document 3 and Nonpatent Documents 16 to 19).

In order to obtain such a "hydrophilic ADC stable in the blood", a "conjugate precursor consisting of linker(s) and antitumor drug(s)" to be reacted with a desired antibody for conjugating is required, and said conjugate precursor must have adequate hydrophilicity.

### CITATION LIST

#### PATENT DOCUMENT

Patent Document 1: WO 2003/043583 pamphlet
Patent Document 2: JP 2016-196484 A
Patent Document 3: WO 2016/001485 pamphlet


#### NONPATENT DOCUMENT

Nonpatent Document 1: Current Opin. Chem. Biol., 2010, 14, 529-537
Nonpatent Document 2: Expert Opin. Biol. Ther., 2004, 4, 1445-1452
Nonpatent Document 3: Biotechnol Lett., 2016, 38, 1655-1664.
Nonpatent Document 4: Chem Pharm Bull., 2019, 67, 173-185.
Nonpatent Document 5: N Engl J Med., 2012, 367, 1783-1791
Nonpatent Document 6: N Engl J Med., 2018, 378, 331-344.

Nonpatent Document 7: Expert Opin. Biol. Ther., 2020, 8, 871-875
Nonpatent Document 8: Drugs Today, 2021, 57, 653-663
Nonpatent Document 9: Drugs, 2021, 81, 2141-2147
Nonpatent Document 10: Drugs, 2021, 81, 1229-1233
Nonpatent Document 11: Clin Cancer Res., 2019, 25, 5441-5448.
Nonpatent Document 12: Molecules. 2020, 25, 4764
Nonpatent Document 13: Methods Mol Biol., 2020, 2078, 1-22
Nonpatent Document 14: Nat. Rev. Drug Discov., 2017, 16, 315
Nonpatent Document 15: Clin Cancer Res., 2004, 10, 7063-7070
Nonpatent Document 16: Mol Cancer Ther., 2017, 16, 116-123
Nonpatent Document 17: Antibodies (Basel), 2018, 7, 15
Nonpatent Document 18: Nat. Biotech., 2015, 33, 733-735
Nonpatent Document 19: Separations 2019, 6(1), 1

SUMMARY OF THE INVENTION

PROBLEMS TO BE SOLVED BY INVENTION

An object of the present invention is to provide a method for improving the stability of an ADC in a living body, and a conjugate precursor for producing a stabilized ADC.

MEANS TO SOLVE PROBLEMS

The present inventors have earnestly studied a conjugate precursor in order to improve the stability of a conventional ADC in a living body, and develop a more useful antitumor drug. As a result, they have created a conjugate precursor in which antitumor drug(s) or linker(s) constituting said conjugate precursor has/have one or more lactonyl group(s) as substituent(s) or protecting group(s). An ADC synthesized by using a conjugate precursor of the present invention has higher hydrophilicity as compared to an ADC having no lactonyl group, is stable in the blood after administered, and showed potent antitumor actions as an ADC in in vitro and in vivo tests.

As stated above, many trials to introduce a hydrophilicity group into a conjugate precursor in order to produce an ADC stable in a living body have been made, but a "conjugate precursor having lactonyl group(s)" of the present invention has not been reported so far.

The present invention provides the following [1] to [26] .

[1] An antibody-drug conjugate precursor represented by the following general formula (I):

Z-L-D (I)

[wherein:

Z is a reactive group (hereinafter also simply referred to as "reactive group") which can react with a functional group (hereinafter also referred to as "functional group in antibody") present in an antibody or a modified antibody (hereinafter also collectively referred to as "antibody");
D is an antitumor drug residue in which one hydrogen atom or one hydroxy group is removed from any position of an antitumor drug molecule or an analog thereof or a derivative thereof (hereinafter also referred to as "antitumor drug residue");
L is a linker which links Z to D; and
at least any one of D and L has one or more lactonyl group(s) at any position(s) as substituent(s) or protecting group(s)]

(hereinafter also referred to as conjugate precursor (I)) or a salt thereof.

[2] The conjugate precursor (I) according to [1] or a salt thereof, wherein Z is a maleimidyl group (the following formula (i)), an $\alpha$-halogenomethylcarbonyl group (the following formula (ii)), an ethynylphosphonamidate group (the following formula (iii)), a carboxy group, an active ester of carboxy group, a sulfhydryl group, a hydroxy group, an amino group, an alkynyl group, a cycloalkynyl group, an azacycloalkynyl group, or an azide group ($-N_3$ group).

formula (i)

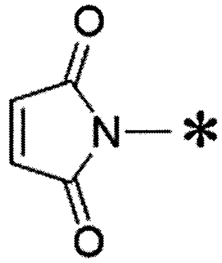

formula (ii)

$$Hal\text{-}CH_2\text{-}C(=O)\text{-}*$$

formula (iii)

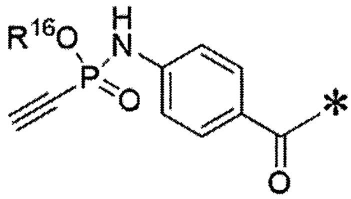

[wherein:

* is a point of attachment to L;
Hal is a fluorine atom, a chlorine atom, a bromine atom, or an iodine atom; and
$R^{16}$ is a methyl group, an ethyl group, or a - $CH_2CH_2OCH_2CH_2OH$ group]

[3] The conjugate precursor (I) according to [1] or [2] or a salt thereof, wherein Z is a maleimidyl group (the above formula (i)), an α-halogenomethylcarbonyl group (the above formula (ii)), an ethynylphosphonamidate group (the above formula (iii)), a carboxy group, an active ester of carboxy group, an alkynyl group, a cycloalkynyl group, an azacycloalkynyl group, or an azide group (-$N_3$ group).
[4] The conjugate precursor (I) according to any one of [1] to [3] or a salt thereof, wherein Z is a maleimidyl group (the above formula (i)), an α-halogenomethylcarbonyl group (the above formula (ii)), a carboxy group, or an active ester of carboxy group.
[5] The conjugate precursor (I) according to any one of [1] to [4] or a salt thereof, wherein D is an antitumor drug residue in which one hydrogen atom or one hydroxy group is removed from any position of camptothecin; auristatin E (AE) or monomethyl auristatin E (MMAE); maytansine; PBD (parabenzodiazepine) dimer; eribulin; 5-FU; PD-318088; AS-703026; TAK-733; LY-3023414; calicheamicin; paclitaxel; docetaxel; mitomycin C; bleomycin; cyclocytidine; vincristine; vinblastine; daunomycin; doxorubicin; dolastatin 10; superdox; ciprofloxacin; cadrofloxacin (CS-940); or an analog of said antitumor drug; or a derivative of said antitumor drug molecule or an analog thereof.
[6] The conjugate precursor (I) according to any one of [1] to [5] or a salt thereof, wherein D is an antitumor drug residue in which one hydrogen atom or one hydroxy group is removed from any position of camptothecin; auristatin E (AE) or monomethyl auristatin E (MMAE); maytansine; PBD (parabenzodiazepine) dimer; eribulin; 5-FU; PD-318088; AS-703026; TAK-733; LY-3023414; calicheamicin; paclitaxel; docetaxel; mitomycin C; bleomycin; cyclocytidine; vincristine; vinblastine; daunomycin; doxorubicin; dolastatin 10; superdox; or an analog of said antitumor drug; or a derivative of said antitumor drug molecule or an analog thereof.
[7] The conjugate precursor (I) according to any one of [1] to [6] or a salt thereof, wherein D is an antitumor drug residue in which one hydrogen atom or one hydroxy group is removed from any position of camptothecin; auristatin E (AE) or monomethyl auristatin E (MMAE); maytansine; PBD (parabenzodiazepine) dimer; eribulin; or an analog of said antitumor drug; or a derivative of said antitumor drug molecule or an analog thereof.
[8] The conjugate precursor (I) according to any one of [1] to [7] or a salt thereof, wherein D is an antitumor drug residue in which one hydrogen atom or one hydroxy group is removed from any position of an antitumor drug or an analog of

said antitumor drug in which at least one hydroxy group present in the molecule is phosphorylated; or a derivative of said antitumor drug molecule or an analog thereof.

[9] The conjugate precursor (I) according to any one of [1] to [8] or a salt thereof, wherein

L is an optionally substituted alkylene group;

one or more methylene group(s) in the chain of said alkylene group is/are optionally replaced with one or more divalent group(s) independently selected from the group consisting of -C($R^1$)($R^2$)-; -O-; -N($R^3$)-; -N($R^3$)-N($R^3$)-; -S-; -Se-; -Si($R^4$)($R^5$) -S-S-; -Se-Se-; -SOm-; -SeOn-; - C(=C($R^6$)($R^7$))-; -C(=O)-; -C(=S)-; -C(=N($R^8$))-; -C(=N-O$R^9$)-; -C(=N-N($R^{10}$)($R^{11}$))-; -P(=O)($R^{12}$)-; -P(=O)(O$R^{13}$)-; -OP(=O)($R^{12}$)-O-; -O-P(=O)(O$R^{13}$)-O-; -C($R^{14}$)=; =C($R^{14}$)-; - C($R^{14}$)=C($R^{14}$)-; -N=; =N-; -C≡C-; -(O-C($R^1$)($R^2$)-C($R^1$)($R^2$))$_{1-30}$-; -(C($R^1$)($R^2$)-C($R^1$)($R^2$)-0)$_{1-30}$-; an optionally substituted alkenylene group; an optionally substituted alkynylene group; an optionally substituted cycloalkylene group; an optionally substituted cycloalkenylene group; an optionally substituted cycloalkynylene group; an optionally substituted azacycloalkynylene group; an optionally substituted arylene group; an optionally substituted heteroarylene group; and an optionally substituted heterocyclylene group;

$R^1$ , $R^2$, $R^3$, $R^4$, $R^5$, $R^6$, $R^7$, $R^8$, $R^9$, $R^{10}$, $R^{11}$, $R^{12}$, $R^{13}$, and $R^{14}$ are each independently a group selected from the group consisting of a hydrogen atom, an optionally substituted alkyl group, an optionally substituted alkenyl group, an optionally substituted alkynyl group, an optionally substituted cycloalkyl group, an optionally substituted cycloalkenyl group, an optionally substituted cycloalkynyl group, an optionally substituted azacycloalkynyl group, an optionally substituted aryl group, an optionally substituted heteroaryl group, and an optionally substituted heterocyclyl group,

wherein when $R^3$ is an alkyl group, said alkyl group is optionally combined with an alkyl group in an adjacent methylene group to form a cyclic structure; and

m and n are each independently an integer of 0 to 2.

[10] The conjugate precursor (I) according to [9] or a salt thereof, wherein

L is an optionally substituted alkylene group; and

one or more methylene group(s) in the chain of said alkylene group is/are replaced with one or more divalent group(s) independently selected from the following formula group:

formula group

-O-C(H or $C_1$-$C_4$ alkyl) (H or $C_1$-$C_4$ alkyl)-;
-S-C(H or $C_1$-$C_4$ alkyl) (H or $C_1$-$C_4$ alkyl)-;
-N(H or $C_1$-$C_4$ alkyl)-;
-N(H or $C_1$-$C_4$ alkyl)-O-C(H or $C_1$-$C_4$ alkyl) (C (H or $C_1$-$C_4$ alkyl)-;
-C(H or $C_1$-$C_4$ alkyl)-$SO_{0-2}$-C(H or $C_1$-$C_4$ alkyl)-;
-C(H or $C_1$-$C_4$ alkyl)-S-S-C(H or $C_1$-$C_4$ alkyl)-;
-C(H or $C_1$-$C_4$ alkyl)=C(H or $C_1$-$C_4$ alkyl) -;
-C(H or $C_1$-$C_4$ alkyl)=N-;
-C(H or $C_1$-$C_4$ alkyl)=N-O-;
-C(H or $C_1$-$C_4$ alkyl) =N-N(H or $C_1$-$C_4$ alkyl)-;
-C(=C(H or $C_1$-$C_4$ alkyl) (H or $C_1$-$C_4$ alkyl))-;
-C(=N-O(H or $C_1$-$C_4$ alkyl))-;
-C(=N-N(H or $C_1$-$C_4$ alkyl) (H or $C_1$-$C_4$ alkyl))-;
-O-Si($C_1$-$C_4$ alkyl $CH_3$)($C_1$-$C_4$ alkyl) -O-;
-C(H or $C_1$-$C_4$ alkyl) -N ($C_1$-$C_4$ alkyl) -$CH_2$-;
-C(H or $C_1$-$C_4$ alkyl) (H or $C_1$-$C_4$ alkyl)-C(=O)-C(H or $C_1$-$C_4$ alkyl) (H or $C_1$-$C_4$ alkyl)-;
-C(=O)-O-;
-C(=O)-S-;
-C(=O)-N(H or $C_1$-$C_4$ alkyl)-;
-O-C(=O)-O-;
-N(H or $C_1$-$C_4$ alkyl)-C(=O)-N(H or $C_1$-$C_4$ alkyl)-;
-C(=O)-N(H or $C_1$-$C_4$ alkyl)-C(H or $C_1$-$C_4$ alkyl) (H or $C_1$-$C_4$ alkyl)-;
-C(=O)-$(CH_2)_{1-10}$-$(O-CH_2CH_2)_{1-20}$-O-$(CH_2)_{1-10}$-C(=O)-;
-C(=O)-$(CH_2)_{1-10}$-$(O-CH_2CH_2)_{1-20}$-O-$(CH_2)_{1-10}$-$CH_2$-NH-;
-NH-$CH_2$-$(CH_2)_{1-10}$$(O-CH_2CH_2)_{1-20}$-O-$(CH_2)_{1-10}$-$CH_2$-NH-;
-C(=O)-N($C_1$-$C_4$ alkyl)-$CH_2CH_2$-N($C_1$-$C_4$ alkyl)-C(=O)-;

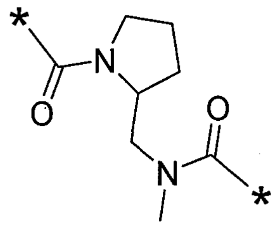

[wherein * is a point of attachment to an adjacent group];
-P(=O)(OH)-O-;
-O-P(=O)(OH)-O-;
-O-P(=S)(OH)-O-;
-O-P(=O)(OH)-S-;
-O-P(=O)(OH)-O-P(=O)(OH)-O-;
-O-P(=O) (OH)-O-P(=O) (OH)-O-P(=O) (OH)-O-;
-CH($CH_2$-$NH_2$)-;
-CH($CH_2$-NH($C_1$-$C_4$ alkyl))-;
-CH($CH_2$-N($C_1$-$C_4$ alkyl$)_2$)-;
-CH($CH_2$-NH-C(=O)-($CH_2CH_2$-O-$)_{0-20}$-$C_1$-$C_4$ alkyl)-;
-CH($CH_2$-NH-C(=O)-($CH_2$$)_{0-20}$-lactonyl)-;
-CH($CH_2$-NH-C(=O)-($CH_2CH_2$-O-$)_{1-20}$-($CH_2$$)_{1-10}$-$CH_2$-lactonyl)-;
-CH($CH_2$-NH-C(=O)-($CH_2CH_2$-O-$)_{1-20}$-($CH_2$$)_{1-10}$-$CH_2$-NH-lactonyl)-;
-CH($CH_2$-NH-C(=O)-($CH_2CH_2$-O-$)_{1-20}$-($CH_2$$)_{1-10}$-$CH_2$-NH-C(=O)-lactonyl)-;
-CH($CH_2$-NH-C(=O)-($CH_2CH_2$-O-$)_{1-20}$-($CH_2$$)_{1-10}$-C(=O)-NH-lactonyl)-;
-CH($CH_2$-NH-($CH_2$$)_{1-20}$-(O$)_{0-1}$-(P(=O)(OH$)_2$)-;
-CH($CH_2$-NH-C(=O)-($CH_2$$)_{1-20}$-(P(=O)(OH$)_2$)-;
-CH($CH_2$-NH-C(=O)-($CH_2$$)_{1-20}$-C(=O)-NH-($CH_2$$)_{1-10}$ (P(=O)(OH$)_2$)-;
-CH($CH_2$-NH-C(=O)- ($CH_2CH_2$-O-$)_{1-20}$- ($CH_2$) $_{1-10}$- $CH_2$-(P(=O)(OH$)_2$)-;
-CH($CH_2$-NH-C(=O)-($CH_2CH_2$-O-$)_{1-20}$-($CH_2$$)_{1-10}$-C(=O)-NH-($CH_2$$)_{1-10}$-(P(=O)(OH$)_2$)-;
-CH($CH_2$-NH-($CH_2$$)_{1-10}$-C(H, OH, Cl, $NH_2$, or $C_1$-$C_4$ alkyl) (P(=O)(OH$)_2$$)_2$)-;
-CH($CH_2$-NH-C(=O)-($CH_2$$)_{1-10}$-C(=O)-NH-($CH_2$$)_{1-10}$-C(H, OH, Cl, $NH_2$, or $C_1$-$C_4$ alkyl) (P(=O)(OH$)_2$$)_2$)-;
-CH($CH_2$-NH-C(=O)-($CH_2CH_2$-O-$)_{1-20}$-($CH_2$$)_{1-10}$-C(=O)-NH-($CH_2$$)_{1-10}$-C(H, OH, Cl, $NH_2$, or $C_1$-$C_4$ alkyl) (P(=O)(OH$)_2$$)_2$)-;
-(cis)-CH=CH-P(=O)(O-$CH_2CH_3$)-NH-(phenylene)-C(=O)-;
-C(=O)-(cyclohexylene)-;
-(succinimidylene)-;
-Ser-;
-Cys-;
-Ama-:

-Asp-;
-Glu-;
-Orn-;
-Lys-;
-ValLys-;
-ValCit-;
-ValAla-;
-GlyGly-C(=O)-NH-(optionally substituted phenylene)-$CH_2$-;
-PheLys-C(=O)-NH-(optionally substituted phenylene)-$CH_2$-;
-ValLys-C(=O)-NH-(optionally substituted phenylene)-$CH_2$-;
-ValCit-C(=O)-NH-(optionally substituted phenylene)-$CH_2$-;
-ValAla-C(=O)-NH-(optionally substituted phenylene)-$CH_2$-;
-AspValCit-C(=O)-NH-(optionally substituted phenylene)-$CH_2$-;
-LysAspValCit-C(=O)-NH-(optionally substituted phenylene)-$CH_2$-;
GluValCit-C(=O)-NH-(optionally substituted phenylene)-$CH_2$-;
-LysValCit-C(=O)-NH-(optionally substituted phenylene)-$CH_2$-;
-SerValCit-C(=O)-NH-(optionally substituted phenylene)-$CH_2$-;
-AspValAla-C(=O)-NH-(optionally substituted phenylene)-$CH_2$-;

-GluValAla-C(=O)-NH-(optionally substituted phenylene)-$CH_2$-;
-LysValAla-C(=O)-NH-(optionally substituted phenylene)-$CH_2$-;
-SerValAla-C(=O)-NH-(optionally substituted phenylene)-$CH_2$-;
-GlyGlyPheGly-C(=O)-NH-(optionally substituted phenylene)-$CH_2$-;
-AspGlyGlyPheGly-C(=O)-NH-(optionally substituted phenylene)-$CH_2$-;
-GluGlyGlyPheGly-C(=O)-NH-(optionally substituted phenylene)-$CH_2$-;
-LysGlyGlyPheGly-C(=O)-NH-(optionally substituted phenylene)-$CH_2$-;
-SerGlyGlyPheGly-C(=O)-NH-(optionally substituted phenylene)-$CH_2$-;
-AspAspAspAspAsp-C(=O)-NH-(optionally substituted phenylene)-$CH_2$-;
-GlyGly-C(=O)-NH-(optionally substituted phenylene)-$CH_2$-O-C(=O)-;
-PheLys-C(=O)-NH-(optionally substituted phenylene)-$CH_2$-O-C(=O)-;
-ValLys-C(=O)-NH-(optionally substituted phenylene)-$CH_2$-O-C(=O)-;
-ValCit-C(=O)-NH-(optionally substituted phenylene)-$CH_2$-O-C(=O)-;
-ValAla-C(=O)-NH-(optionally substituted phenylene)-$CH_2$-O-C(=O)-;
-AspValCit-C(=O)-NH-(optionally substituted phenylene)-$CH_2$-O-C(=O)-;
-GluValCit-C(=O)-NH-(optionally substituted phenylene)-$CH_2$-O-C(=O)-;
-LysValCit-C(=O)-NH-(optionally substituted phenylene)-$CH_2$-O-C(=O)-;
-SerValCit-C(=O)-NH-(optionally substituted phenylene)-$CH_2$-O-C(=O)-;
-AspValAla-C(=O)-NH-(optionally substituted phenylene)-$CH_2$-O-C(=O)-;
-GluValAla-C(=O)-NH-(optionally substituted phenylene)-$CH_2$-O-C(=O)-;
-LysValAla-C(=O)-NH-(optionally substituted phenylene)-$CH_2$-O-C(=O)-;
-SerValAla-C(=O)-NH-(optionally substituted phenylene)-$CH_2$-O-C(=O)-;
-GlyGlyPheGly-C(=O)-NH-(optionally substituted phenylene)-$CH_2$-O-C(=O)-;
-AspGlyGlyPheGly-C(=O)-NH-(optionally substituted phenylene)-$CH_2$-O-C(=O)-;
-GluGlyGlyPheGly-C(=O)-NH-(optionally substituted phenylene)-$CH_2$-O-C(=O)-;
-LysGlyGlyPheGly-C(=O)-NH-(optionally substituted phenylene)-$CH_2$-O-C(=O)-;
-SerGlyGlyPheGly-C(=O)-NH-(optionally substituted phenylene)-$CH_2$-O-C(=O)-;
-AspAspAspAspAsp-C(=O)-NH-(optionally substituted phenylene)-$CH_2$-O-C(=O)-;
-GlyGlyPheGly-C(=O)-NH-$CH_2$-;
-AspGlyGlyPheGly-C(=O)-NH-$CH_2$-;
(in said amino acid residue, and an amino acid residue in said peptide,

the carboxy group(s) in the side chain(s) of Ama, Asp, and Glu is/are optionally converted into (alkyl) ester(s), (aminoalkyl) ester(s), ((N-alkylamino)alkyl) ester(s), ((N,N-dialkylamino)alkyl) ester(s), ((trialkylammonium)alkyl) ester(s), (lactonyl) ester(s), ((lactonyl)alkyl) ester(s), ((phosphoryl)alkyl) ester(s), or ((bisphosphoryl)alkyl) ester(s), or unsubstituted amide(s), alkylamide(s), dialkylamide(s), ((N-alkylamino)alkyl)amide(s), ((N,N-dialkylamino)alkyl)amide(s), ((trialkylammonium)alkyl)amide(s), (lactonyl)amide(s), ((lactonyl)alkyl)amide(s), (phosphoryl)amide(s), ((phosphoryl)alkyl)amide(s), or ((bisphosphoryl)alkyl)amide(s);
the amino group(s) in the side chain(s) of Lys and Orn optionally has/have one or two alkyl group(s), alkylcarbonyl group(s), (aminoalkyl)carbonyl group(s), ((N-alkylamino)alkyl)carbonyl group(s), ((N,N-dialkylamino)alkyl)carbonyl group(s), ((trialkylammonium)alkyl)carbonyl group(s), lactonyl group(s), (lactonyl)alkyl group(s), (lactonyl) carbonyl group(s), ((lactonyl)alkyl)carbonyl group(s), phosphoryl group(s), (phosphoryl)alkyl group(s), ((phosphoryl)alkyl)carbonyl group(s), (bisphosphoryl)alkyl group(s), or ((bisphosphoryl)alkyl)carbonyl group(s) as substituent(s); and
the hydroxy group in the side chain of Ser and the sulfhydryl group in the side chain of Cys optionally has/have alkyl group(s), alkylcarbonyl group(s), aminoalkyl group(s), (aminoalkyl)carbonyl group(s), (N-alkylamino)alkyl group(s), ((N-alkylamino)alkyl)carbonyl group(s), (N,N-dialkylamino)alkyl group(s), ((N,N-dialkylamino)alkyl) carbonyl group(s), (trialkylammonium)alkyl group(s), ((trialkylammonium)alkyl)carbonyl group(s), lactonyl group(s), (lactonyl)alkyl group(s), (lactonyl)carbonyl group(s), ((lactonyl)alkyl)carbonyl group(s), phosphoryl group(s), (phosphoryl)alkyl group(s), ((phosphoryl)alkyl)carbonyl group(s), (bisphosphoryl)alkyl group(s), or ((bisphosphoryl)alkyl)carbonyl group(s) as substituent(s)); and
1,2,3-triazolylene groups represented by the following formulae:

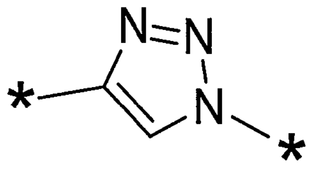

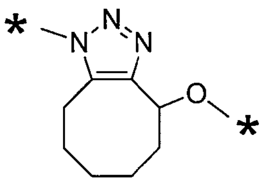

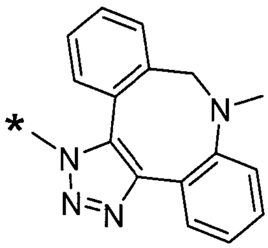

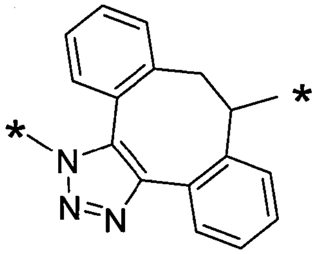

and

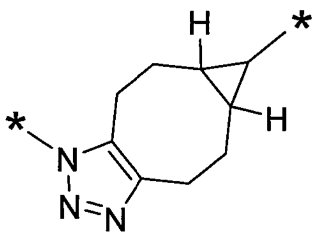

[wherein * is a point of attachment to an adjacent group].

[11] The conjugate precursor (I) according to [9] or a salt thereof, wherein L is any one divalent group selected from the following formula group:

formula group

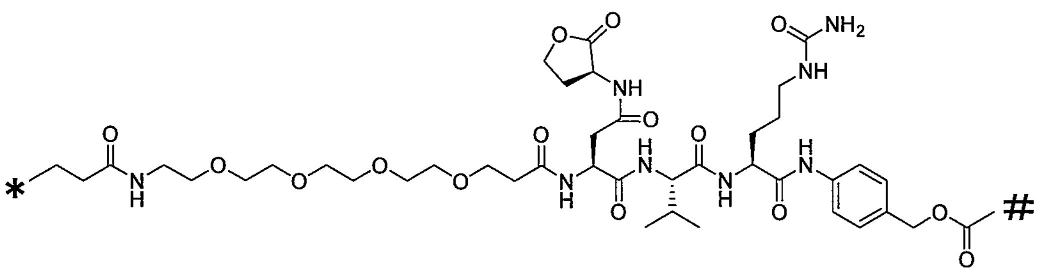

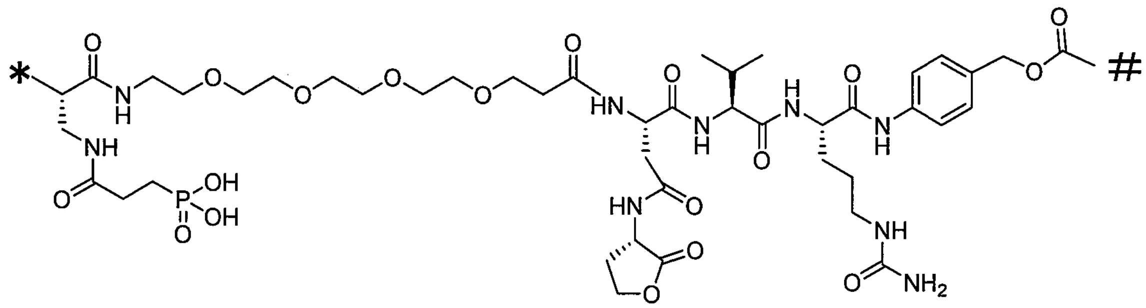

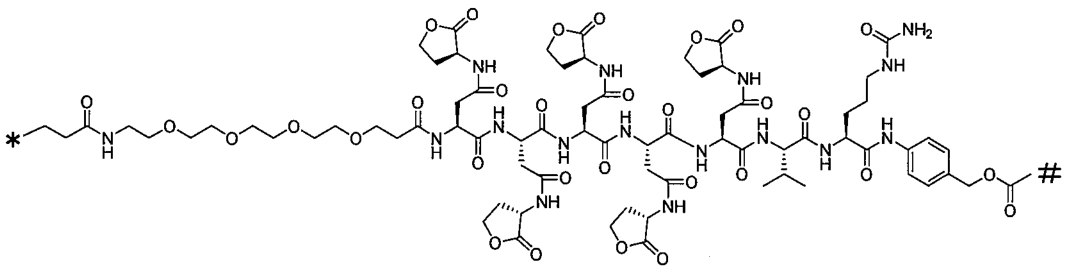

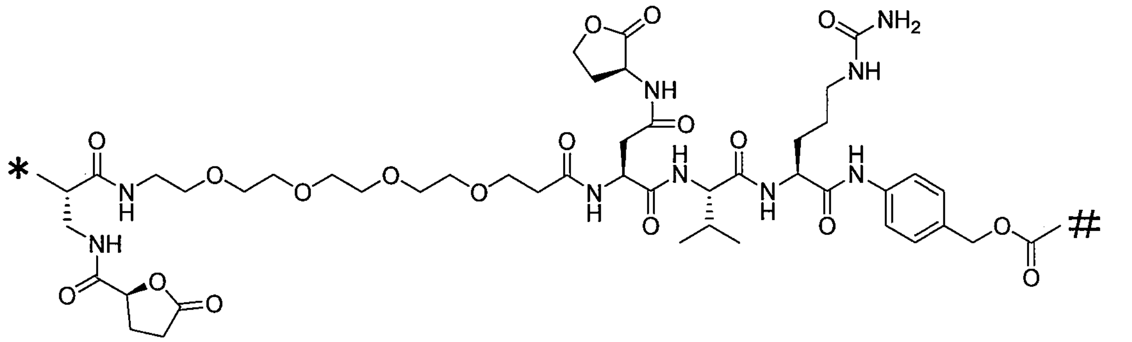

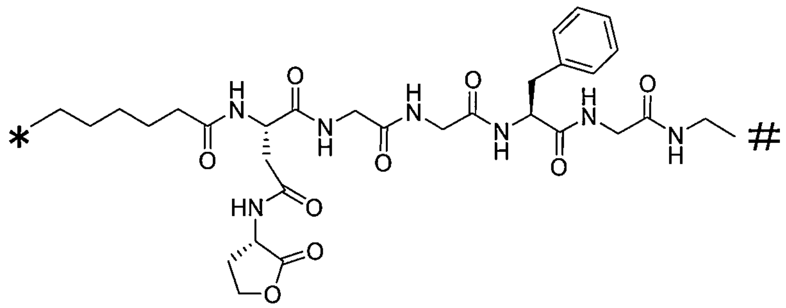

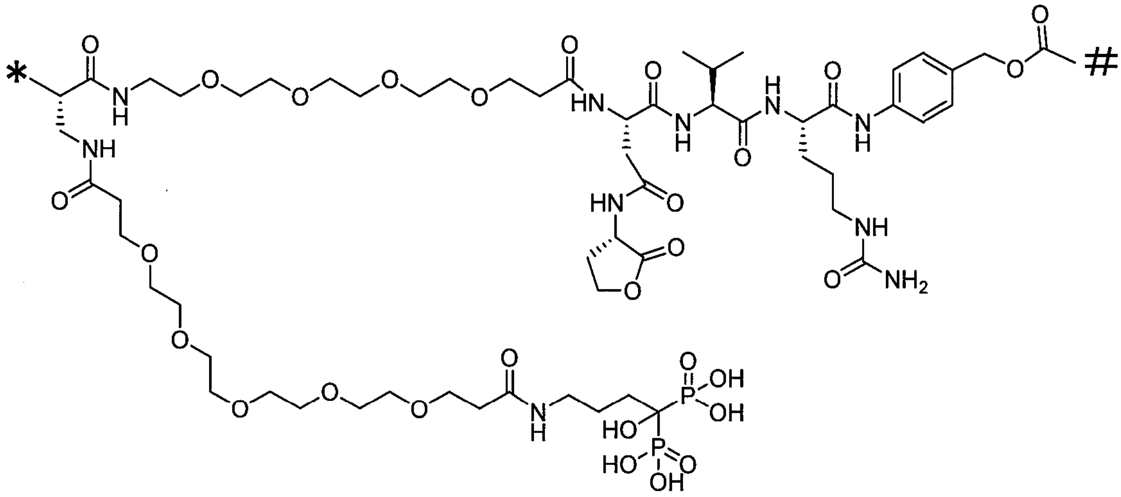

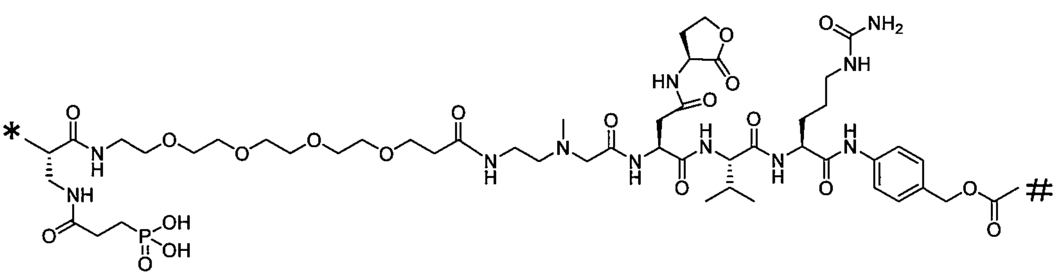

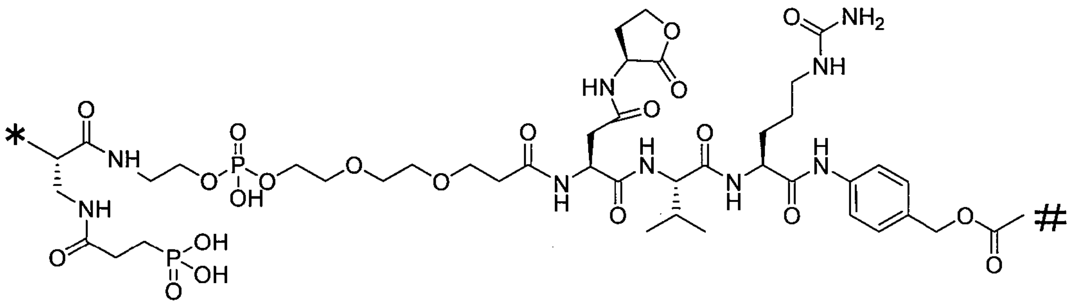

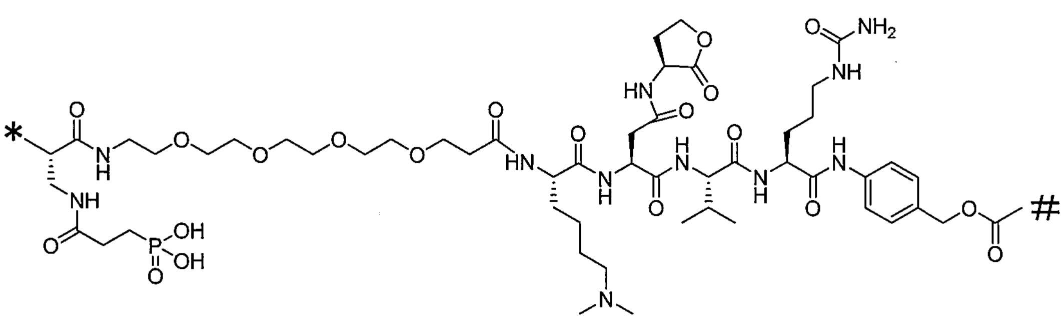

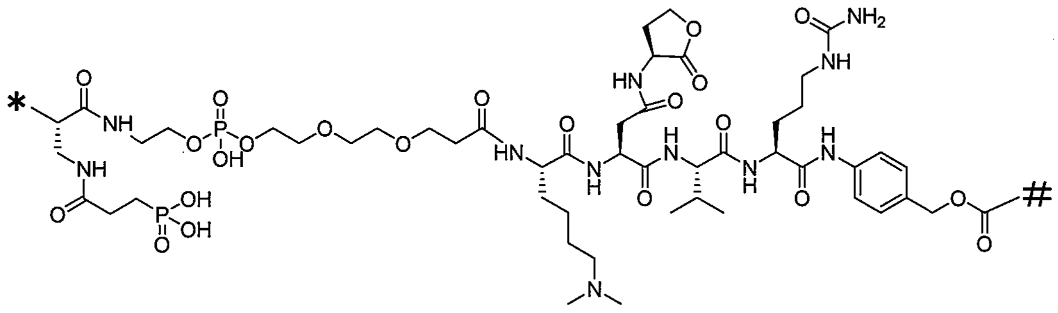

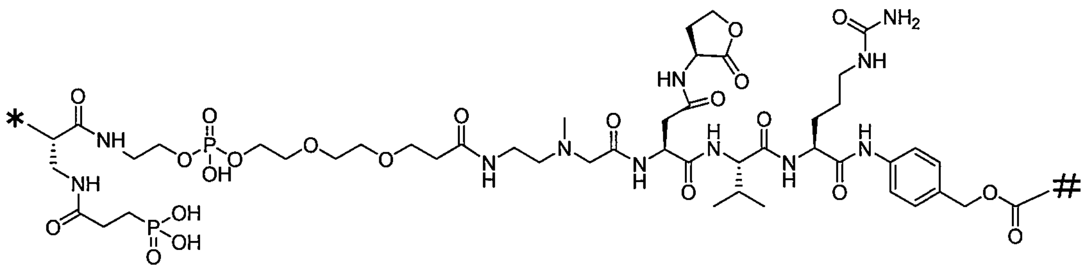

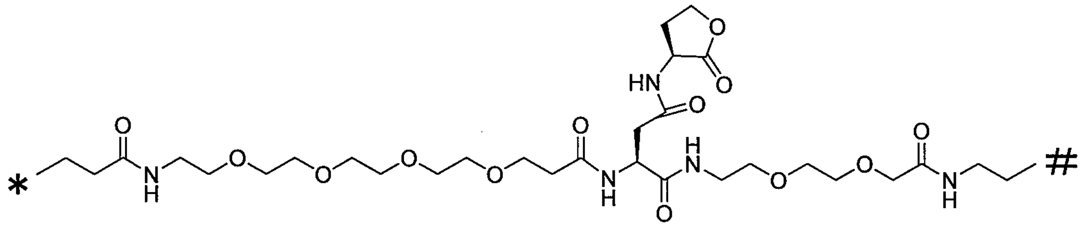

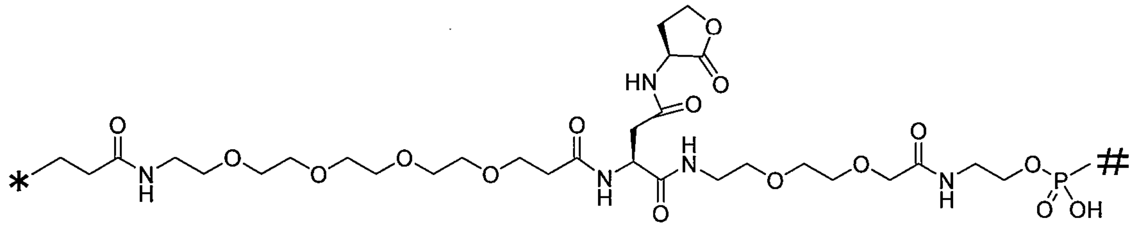

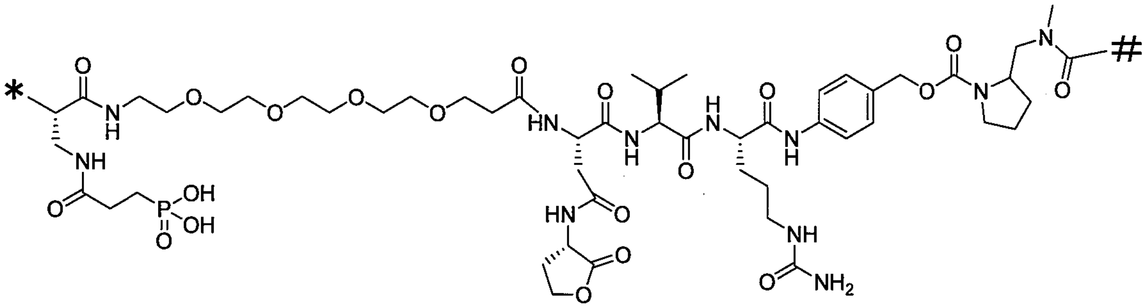

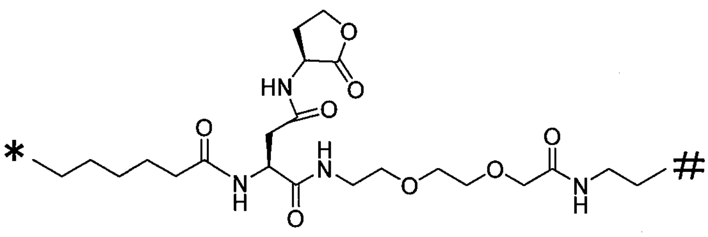

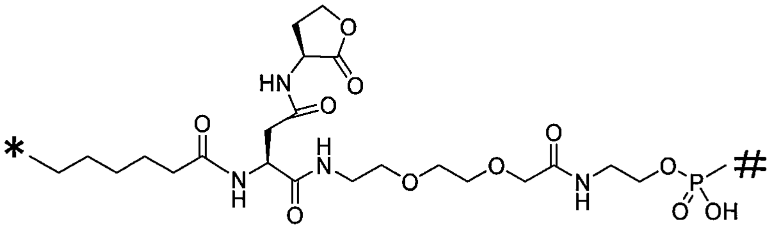

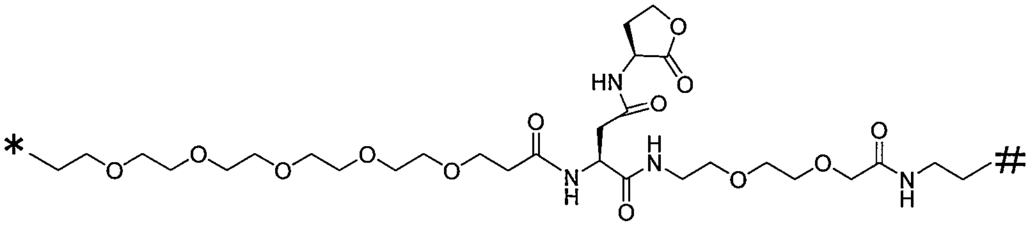

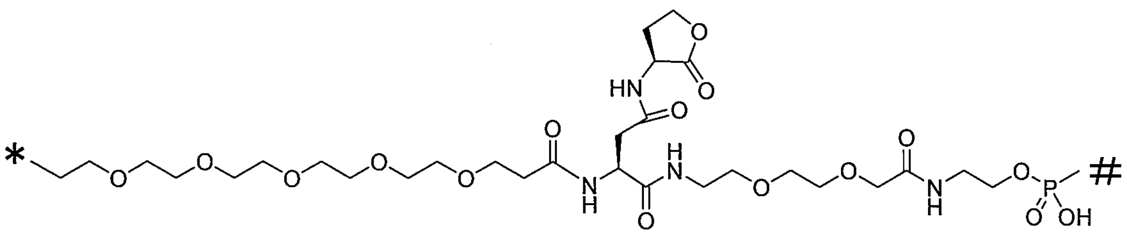

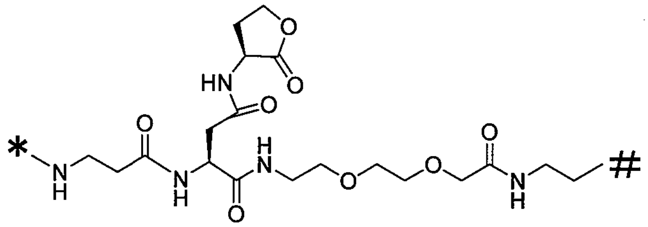

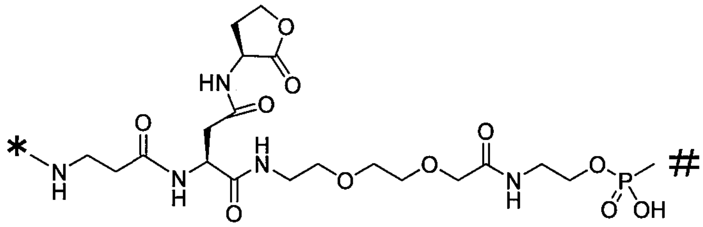

[wherein:

* is a point of attachment to the reactive group Z; and
# is a point of attachment to the antitumor drug residue D].

[12] The conjugate precursor (I) according to any one of [1] to [11] or a salt thereof, wherein L has one or more group(s) selected from a lactonyl group, a phosphoryl group ($-P(=O)(OH)_2$); a phosphorylalkylcarbonyl group ($-C(=O)$-alkylene-$P(=O)(OH)_2$); a bisphosphorylmethyl group ($-C(H, OH, NH_2$, Cl, or alkyl) $(P(=O)(OH)_2)_2$); an amino group; a monoalkylamino group; a dialkylamino group; a cyclic amino group formed by combining two alkyl groups of said dialkylamino group with the nitrogen atom to which they are attached; and a trialkylammonium group, at any position(s) as substituent(s) or protecting group(s),
wherein when the total number of said group(s) is two or more, they are identical to or different from each other.
[13] A conjugate precursor (I) selected from the group consisting of:

Example 1

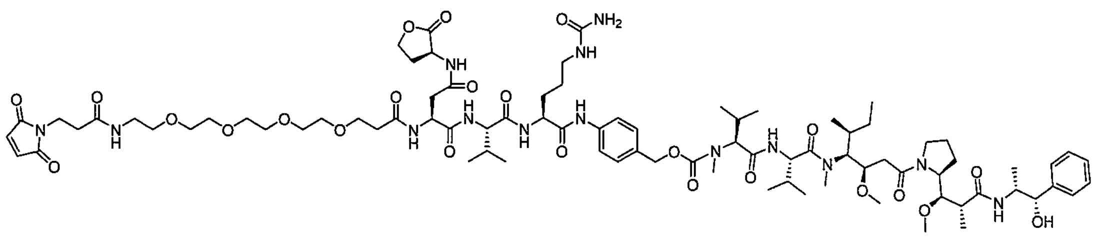

Example 2

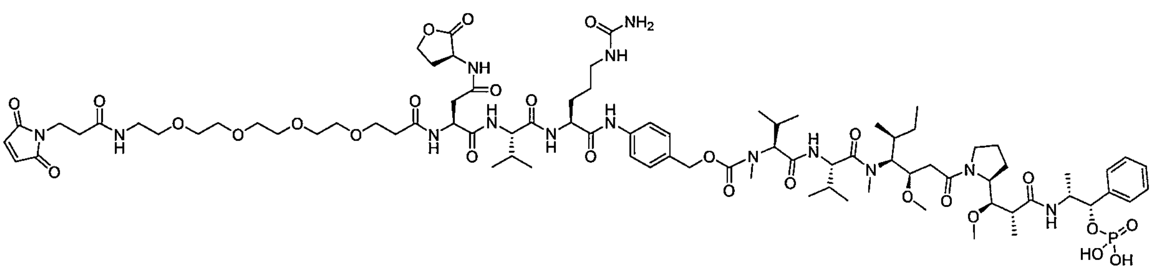

Example 3

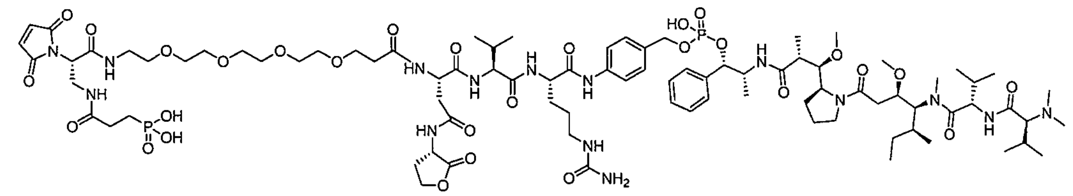

Example 4

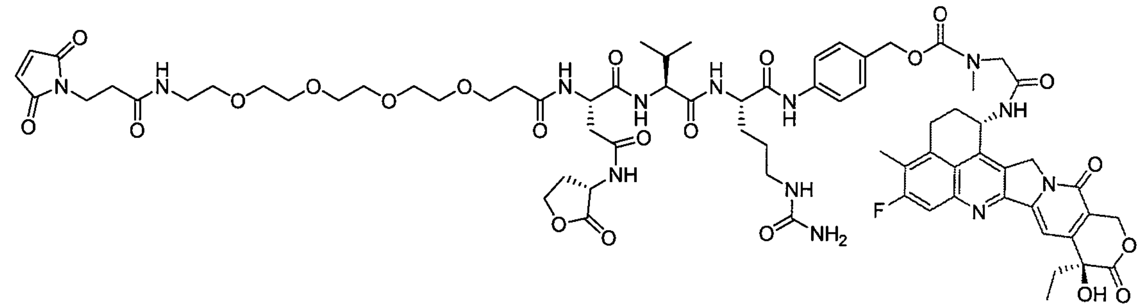

Example 5

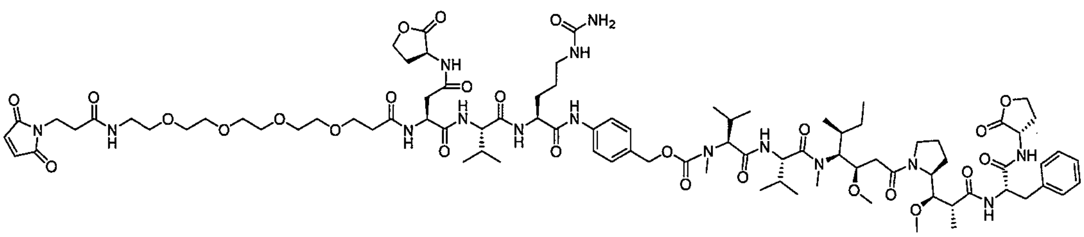

Example 6

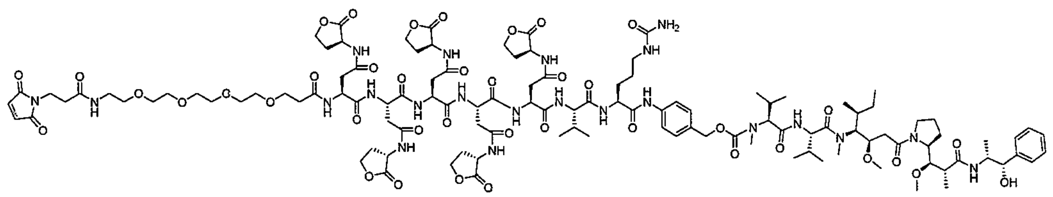

Example 7

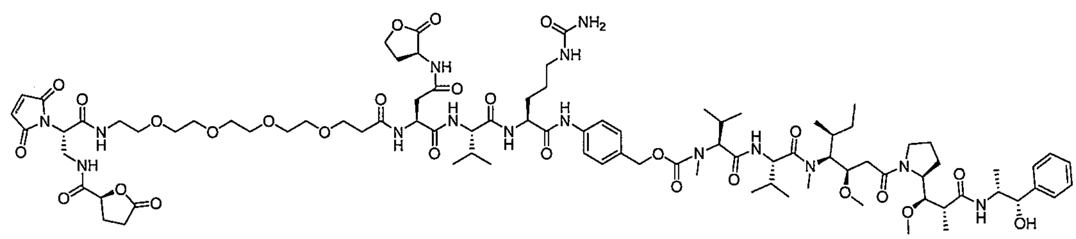

Example 8

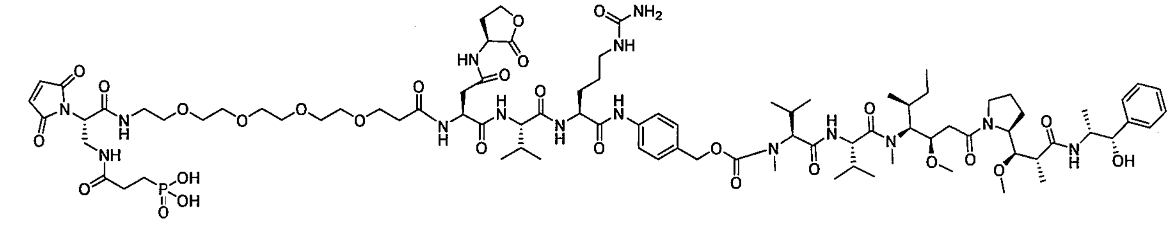

Example 9

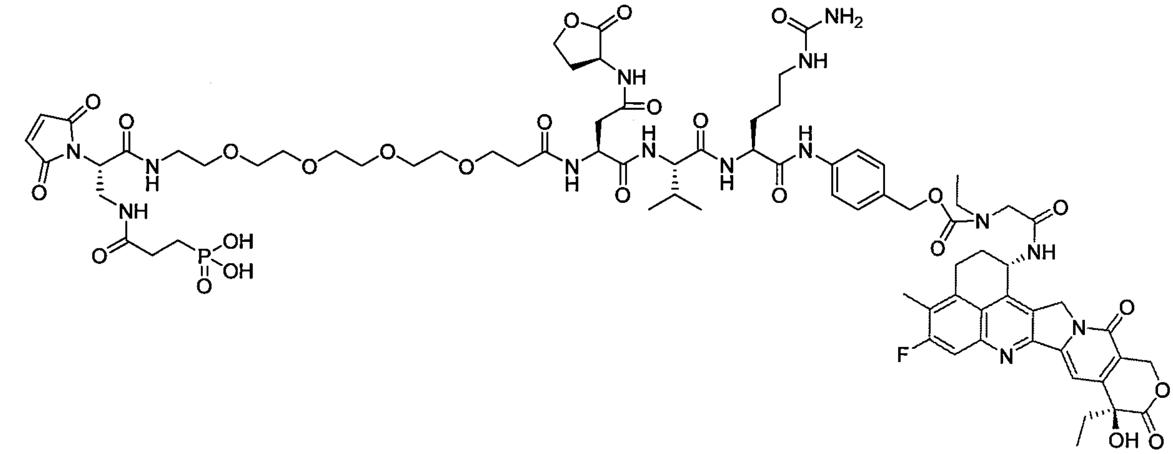

Example 10

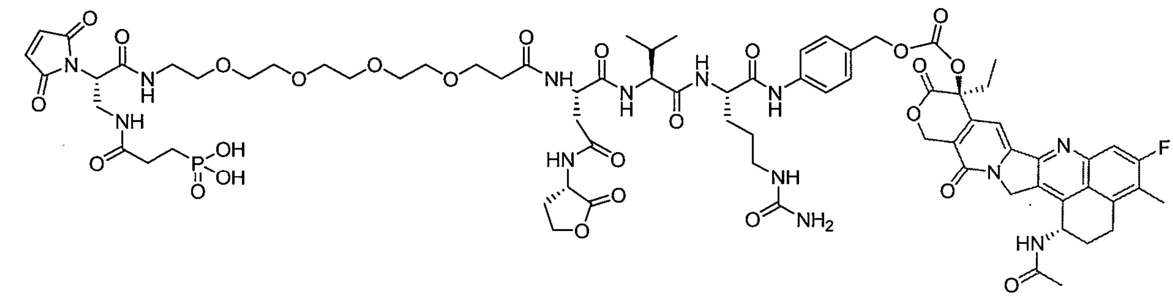

Example 11

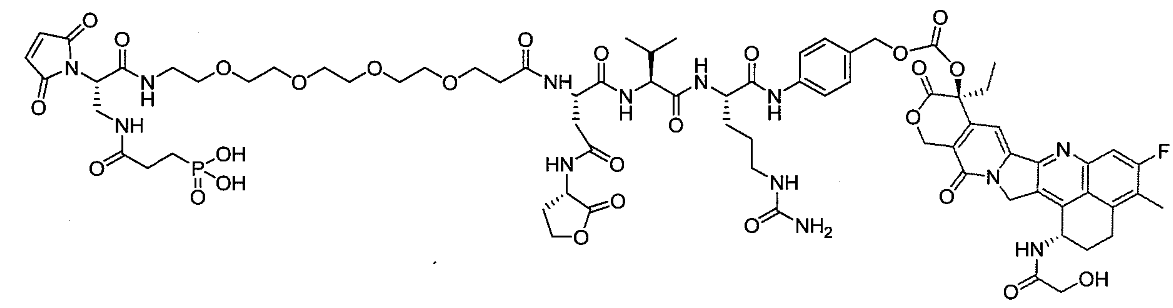

Example 12

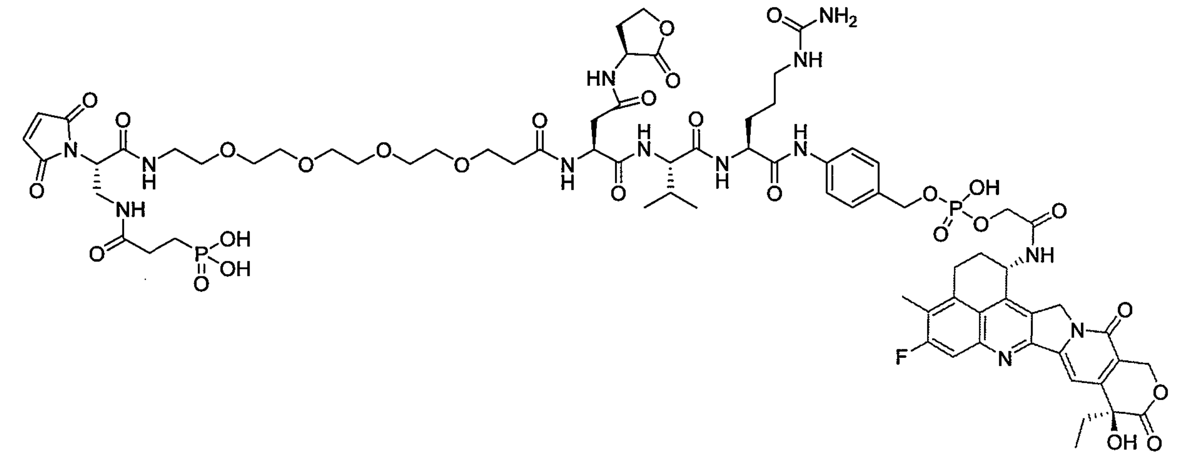

Example 13

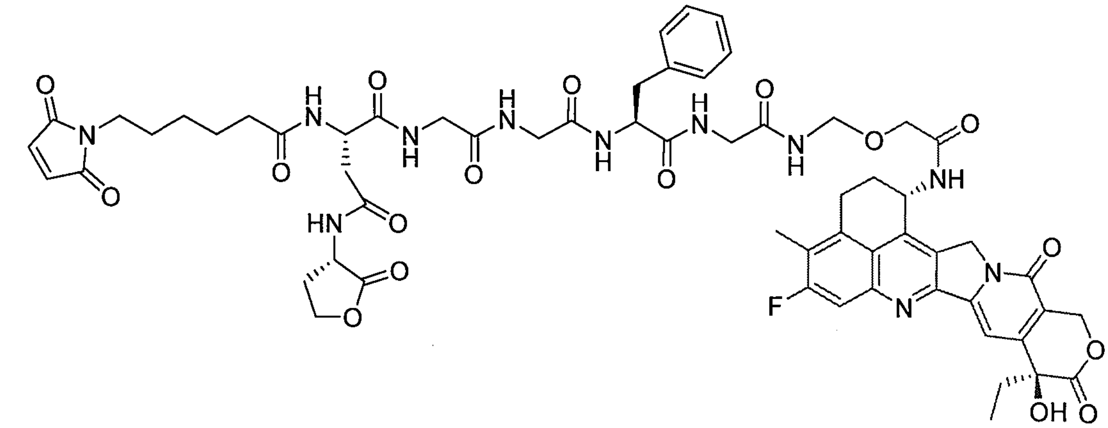

Example 14

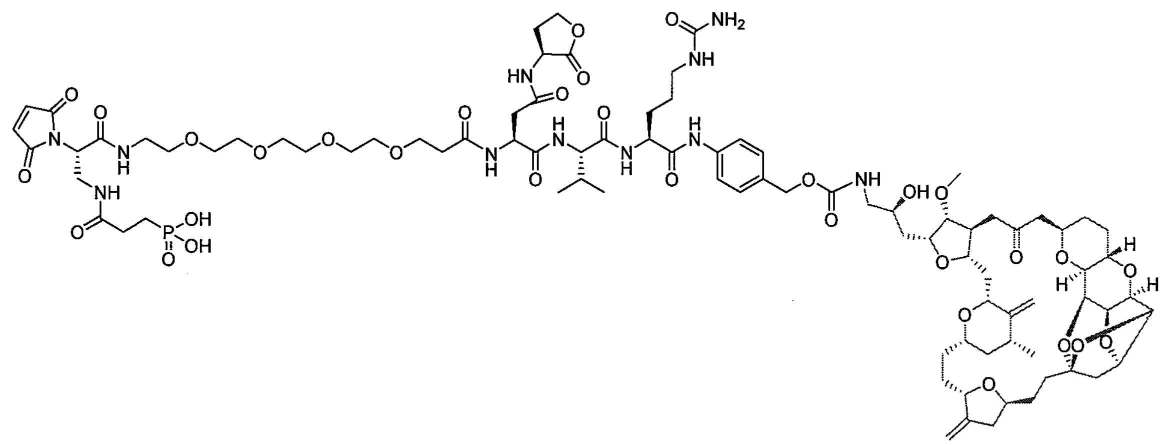

Example 15

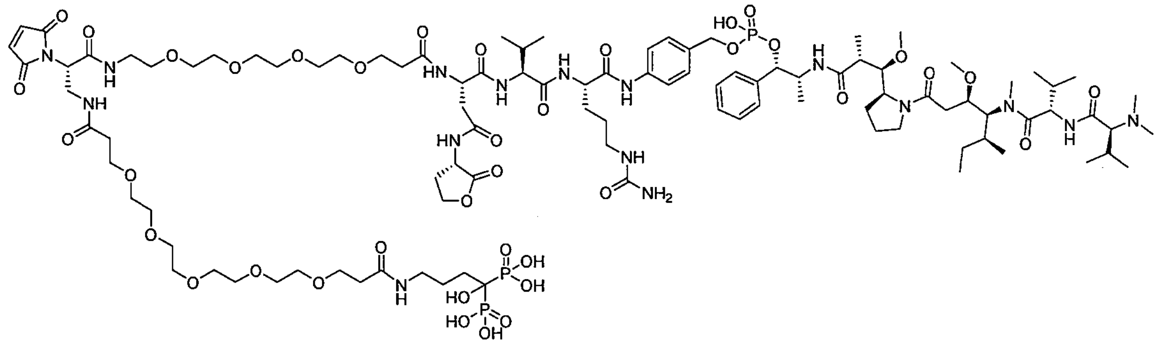

Example 16

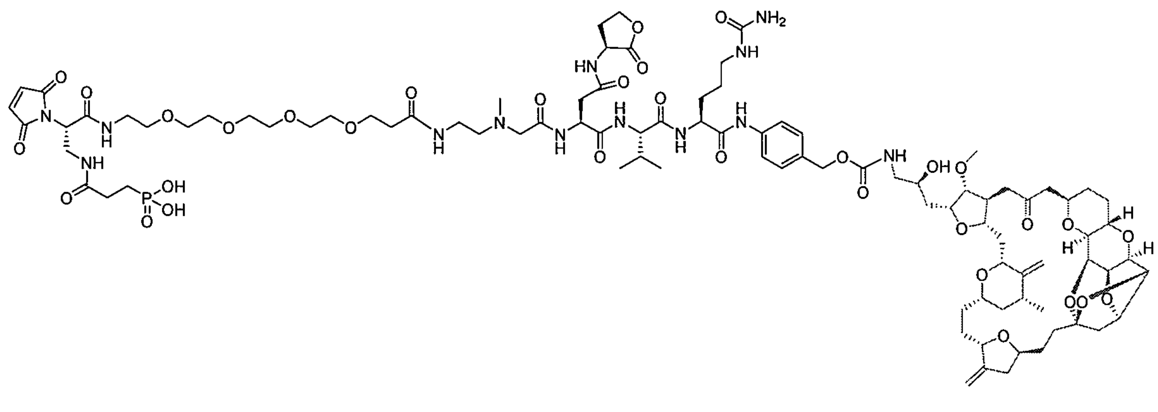

Example 17

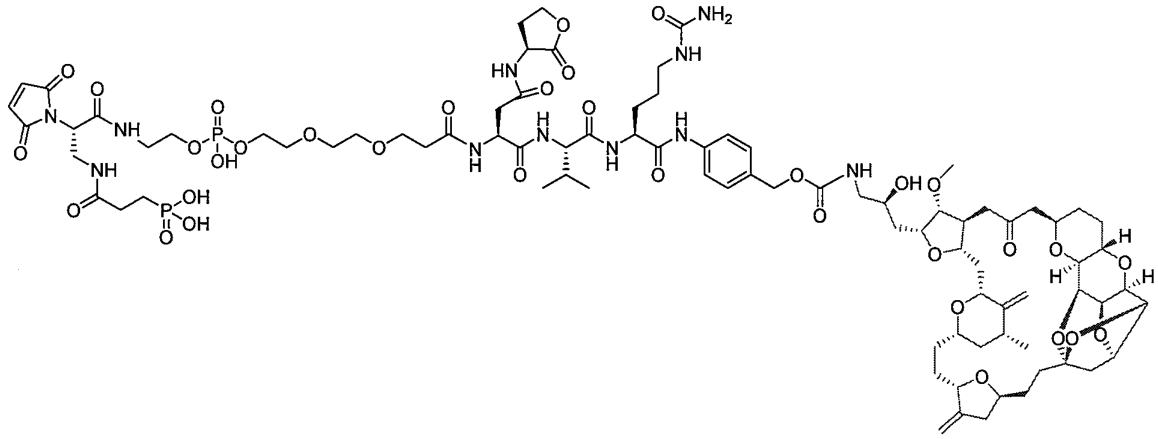

Example 18

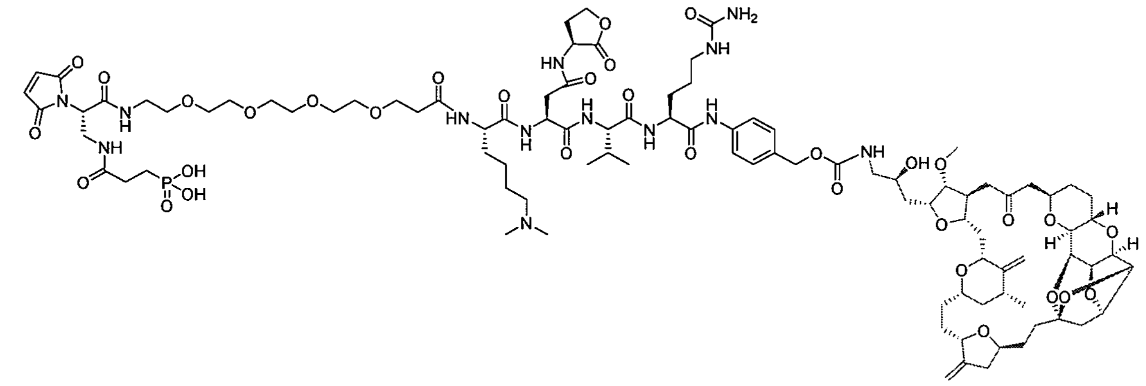

Example 19

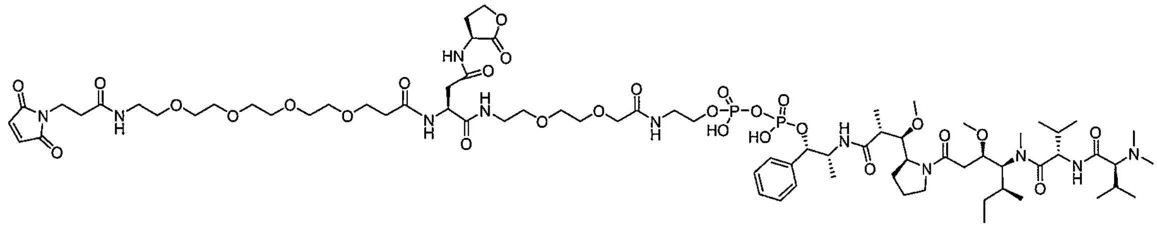

Example 20

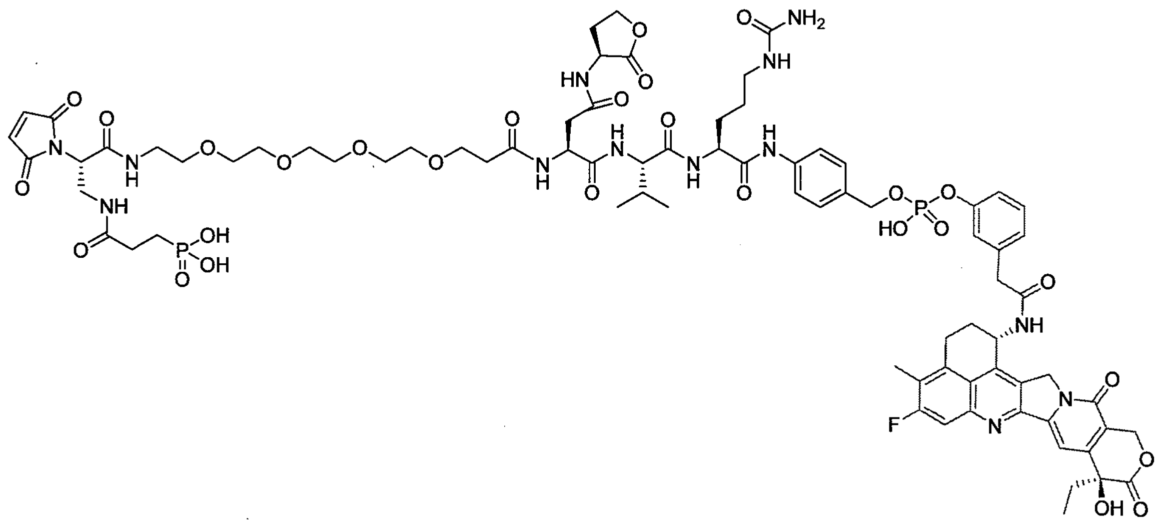

Example 21

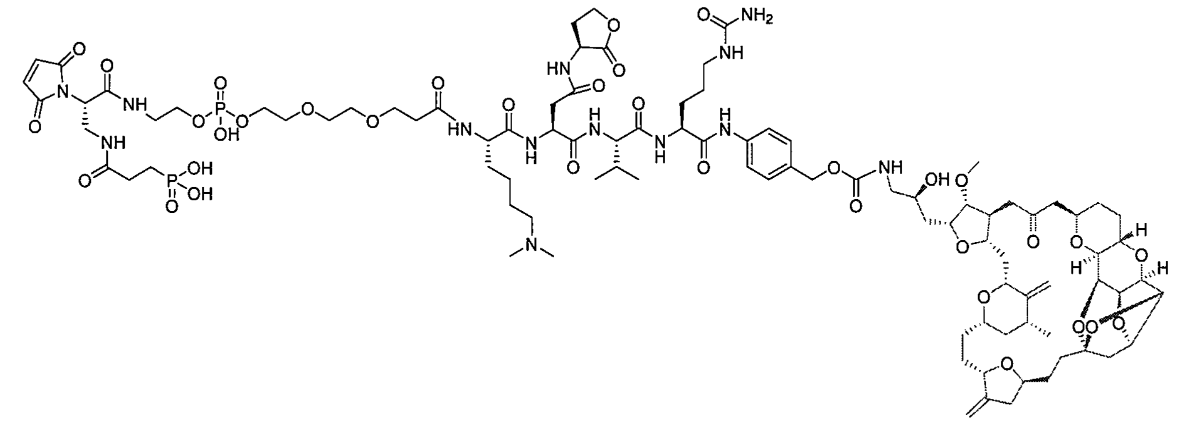

Example 22

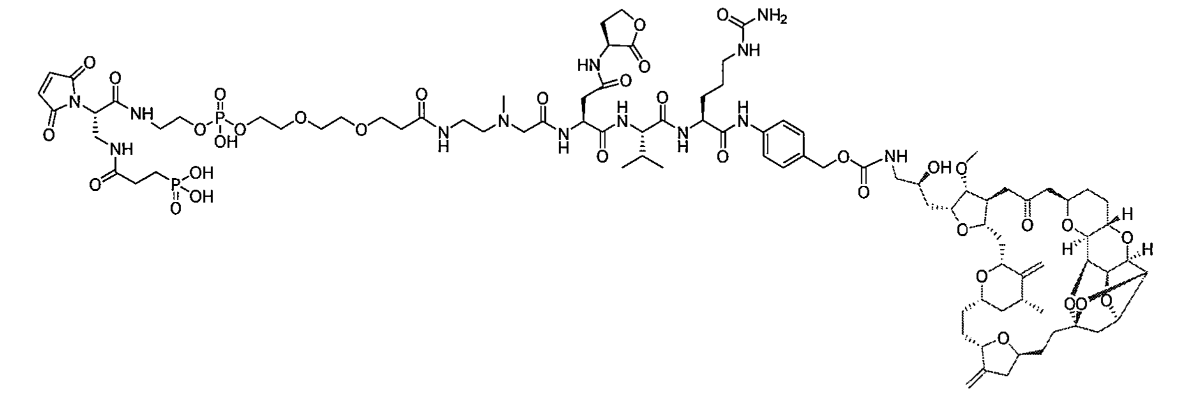

Example 23

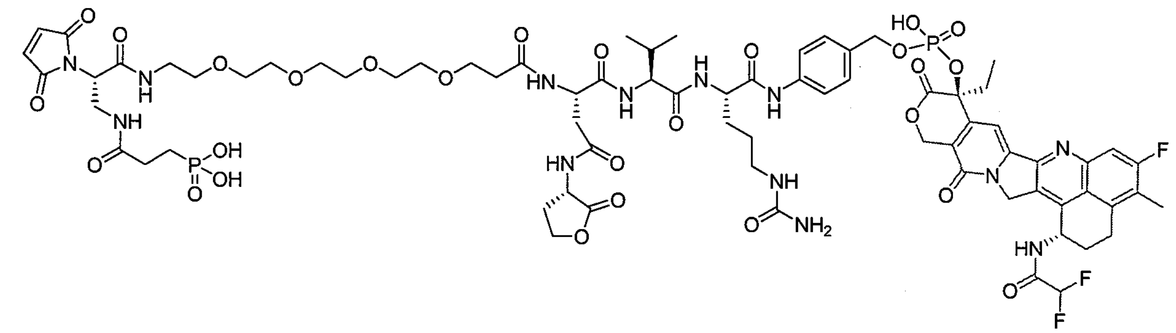

Example 24

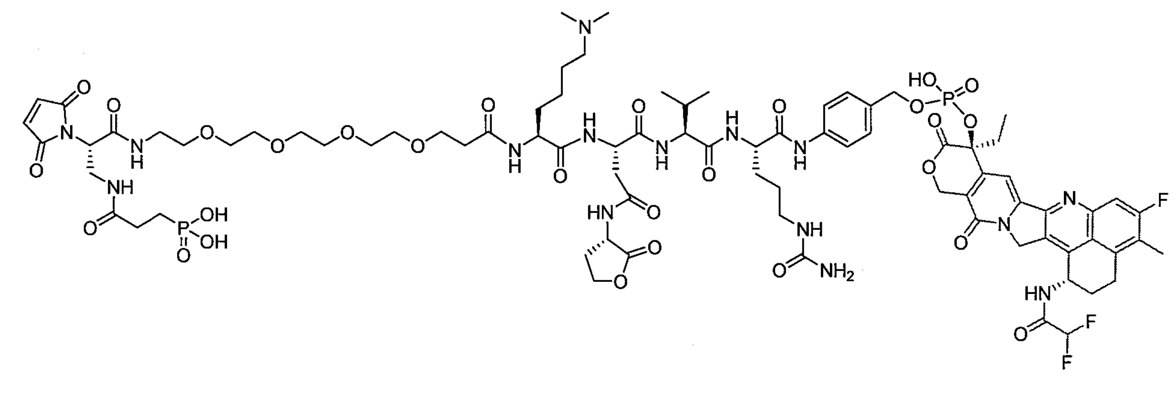

Example 25

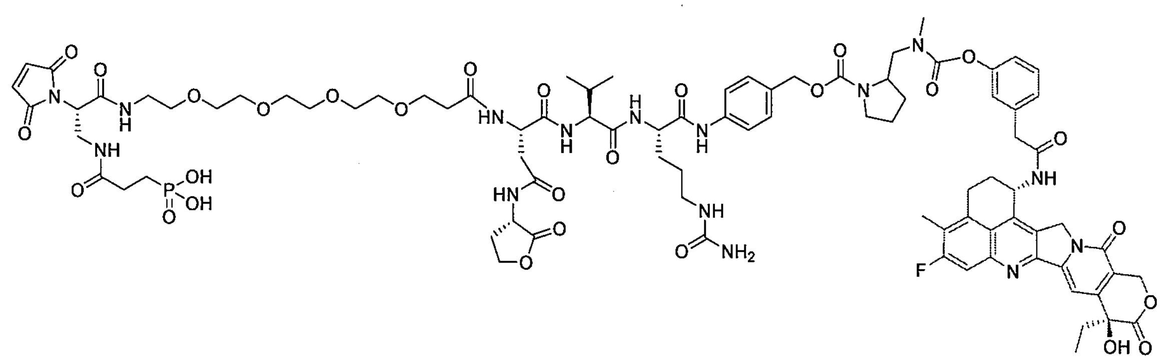

Example 26

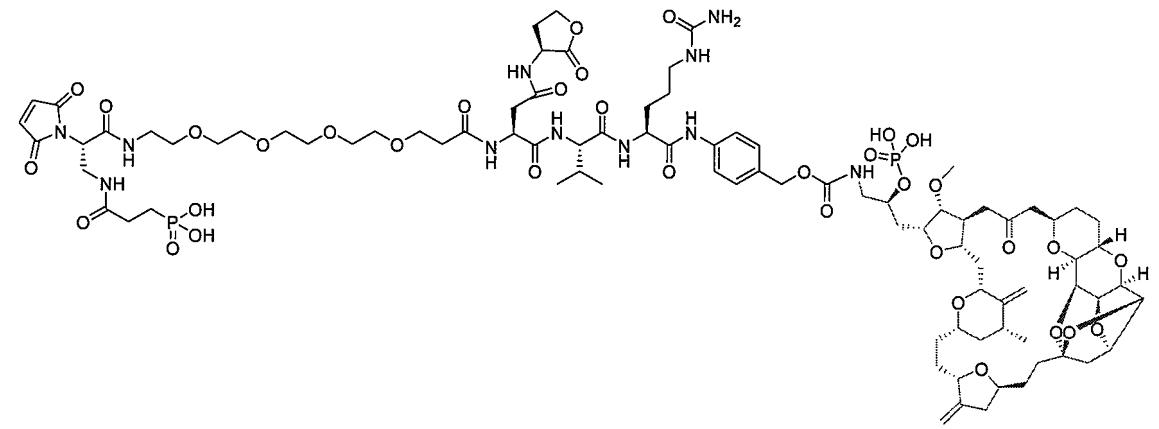

Example 27

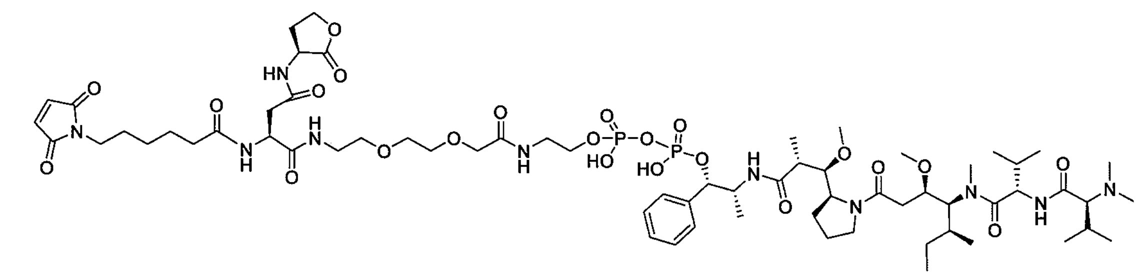

Example 28

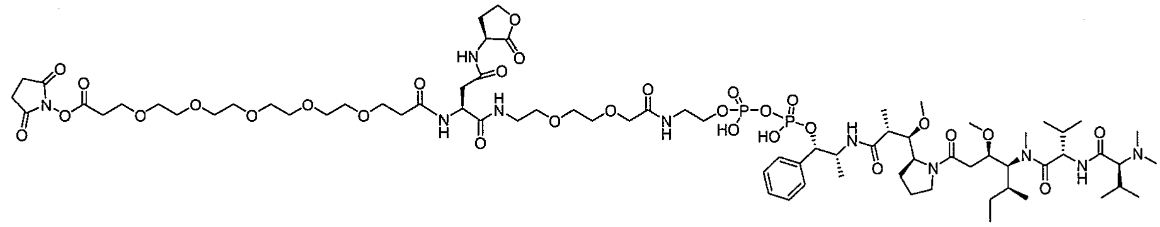

and
Example 29

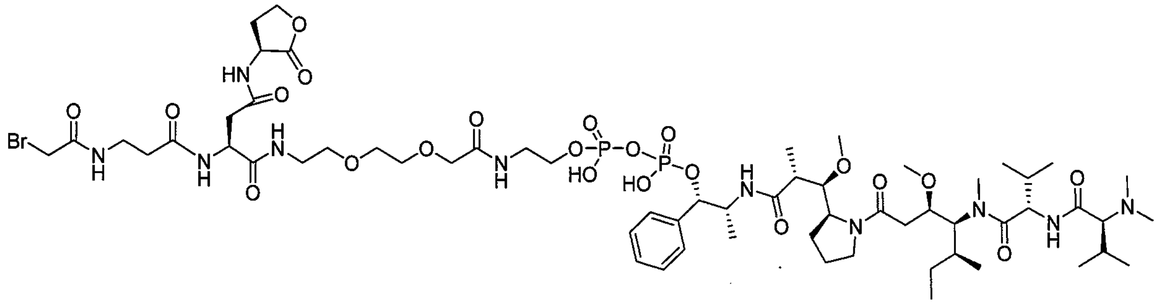

or a salt thereof.

[14] A synthetic intermediate (II) of the conjugate precursor (I) represented by the following general formula (II):

$$Z^1 \text{-} L^1 \text{-} D^1 \qquad (II)$$

[wherein:

$Z^1$ is the reactive group defined in the above [1], or
a jointing group for jointing said reactive group or the antitumor drug residue defined in the above [1] to linker (hereinafter also simply referred to as "jointing group"), wherein said jointing group is optionally in a protected form protected by a protecting group (hereinafter also simply referred to as "protected form");
$D^1$ is an antitumor drug residue, or a jointing group or a protected form thereof;
when $Z^1$ is the reactive group, then $D^1$ is the jointing group;
$L^1$ is a linker which links $Z^1$ to $D^1$;
when $D^1$ is the antitumor drug residue, then at least any one of $D^1$ and $L^1$ has one or more lactonyl group(s) at any position(s) as substituent(s) or protecting group(s); and
when $D^1$ is the jointing group, then
$L^1$ has one or more group(s) selected from a lactonyl group; a phosphoryl group (-P(=O)$(OH)_2$); a phosphorylalkylcarbonyl group (-C(=O)-alkylene-P(=O)$(OH)_2$); a bisphosphorylmethyl group (-C(H, OH, $NH_2$, Cl, or alkyl) $(P(=O)(OH)_2)_2$); an amino group; a monoalkylamino group; a dialkylamino group; a cyclic amino group formed by combining two alkyl groups of said dialkylamino group with the nitrogen atom to which they are attached; and a trialkylammonium group, at any position(s) as substituent(s) or protecting group(s),
wherein when the total number of said group(s) is two or more, they are identical to or different from each other]

(hereinafter also referred to as "synthetic intermediate (II) of conjugate precursor) or a salt thereof.

[15] The synthetic intermediate (II) of conjugate precursor according to [14] or a salt thereof, wherein the jointing group is a hydroxy group, a nitro group, a cyano group, an amide group, an oxo group, an azide group, an amino group, a monoalkylamino group optionally having substituent(s), an imino group (-N=, also including an imino group in a cyclic amine), a carboxy group, a phosphoryl group, an alkynyl group, a cycloalkynyl group, an azacycloalkynyl group, or a sulfhydryl group, or a protected form thereof.

[16] The synthetic intermediate (II) of conjugate precursor according to [14] or [15] or a salt thereof, wherein the jointing group is a hydroxy group, a nitro group, a cyano group, an oxo group, an azide group, an amino group, a monoalkylamino group optionally having substituent(s), an imino group (-N=, also including an imino group in a cyclic amine), a carboxy group, a phosphoryl group, an alkynyl group, a cycloalkynyl group, an azacycloalkynyl group, or a sulfhydryl group, or a protected form thereof.

[17] The synthetic intermediate (II) of conjugate precursor according to any one of [14] to [16] or a salt thereof, wherein the jointing group is a hydroxy group, a nitro group, an oxo group, an azide group, an amino group, a monoalkylamino group optionally having substituent(s), an imino group (-N=, also including an imino group in a cyclic amine), a carboxy group, a phosphoryl group, an alkynyl group, a cycloalkynyl group, an azacycloalkynyl group, or a sulfhydryl group, or a protected form thereof.

[18] The synthetic intermediate (II) of conjugate precursor according to any one of [14] to [17] or a salt thereof, wherein the jointing group is a hydroxy group, a nitro group, an oxo group, an azide group, an amino group, a monoalkylamino group optionally having substituent(s), an imino group (-N=, also including an imino group in a cyclic amine), a carboxy group, a phosphoryl group, an alkynyl group, a cycloalkynyl group, or an azacycloalkynyl group, or a protected form thereof.

[19] The synthetic intermediate (II) of conjugate precursor according to any one of [14] to [18] or a salt thereof, wherein

$L^1$ is an optionally substituted alkylene group; and
one or more methylene group(s) in the chain of said alkylene group is/are replaced with one or more group(s) selected from the divalent groups according to any one of the above [9] to [11].

[20] The synthetic intermediate (II) of conjugate precursor according to any one of [14] to [19] or a salt thereof, wherein

$Z^1$ is a group selected from the jointing groups according to any one of the above [15] to [18]; and
$D^1$ is a group selected from the antitumor drug residues according to any one of the above [5] to [8].

[21] The synthetic intermediate (II) of conjugate precursor according to any one of [14] to [19] or a salt thereof, wherein $Z^1$ and $D^1$ are each independently a group selected from the jointing groups according to any one of the above [15] to [18].
[22] The synthetic intermediate (II) of conjugate precursor according to any one of [14] to [19] or a salt thereof, wherein

$Z^1$ is a group selected from the reactive groups according to any one of the above [2] to [4]; and
$D^1$ is a group selected from the jointing groups according to any one of the above [15] to [18].

[23] The intermediate (II) of conjugate precursor according to any one of [14] to [20] or a salt thereof, wherein

$Z^1$ is a group selected from the jointing groups according to any one of the above [15] to [18];
$D^1$ is a group selected from the antitumor drug residues according to any one of the above [5] to [8]; and
at least any one of $D^1$ and $L^1$ has one or more lactonyl group(s) at any position(s) as substituent(s) or protecting group(s), and independently thereof,
at least any one of $D^1$ and $L^1$ has one or more group(s) selected from a phosphoryl group (-P(=O)(OH)$_2$); a phosphorylalkylcarbonyl group (-C(=O)-alkylene-P(=O) (OH)$_2$); a bisphosphorylmethyl group (-C(H, OH, NH$_2$, Cl, or alkyl)(P(=O)(OH)$_2$)$_2$); an amino group; a monoalkylamino group; a dialkylamino group; a cyclic amino group formed by combining two alkyl groups of said dialkylamino group with the nitrogen atom to which they are attached; and a trialkylammonium group, at any position(s) as substituent(s) or protecting group(s),
wherein when the total number of said group(s) is two or more, they are identical to or different from each other.

[24] The synthetic intermediate (II) of conjugate precursor according to any one of [14] to [19], [21], and [22] or a salt thereof, wherein

$Z^1$ is a group selected from the reactive groups according to any one of the above [2] to [4], or a group selected from the jointing groups according to any one of the above [15] to [18];
$D^1$ is a group selected from the jointing groups according to any one of the above [15] to [18]; and
$L^1$ has one or more group(s) selected from a lactonyl group; a phosphoryl group (-P(=O)(OH)$_2$); a phosphorylalkylcarbonyl group (-C(=O)-alkylene-P(=O)(OH)$_2$); a bisphosphorylmethyl group (-C(H, OH, NH$_2$, Cl, or alkyl)(P(=O)(OH)$_2$)$_2$); an amino group; a monoalkylamino group; a dialkylamino group; a cyclic amino group formed by combining said dialkyl group; and a trialkylammonium group, at any position(s) as substituent(s) or protecting group(s),
wherein when the total number of said group(s) is two or more, they are identical to or different from each other.

[25] A synthetic intermediate (II) of conjugate precursor selected from the group consisting of:

Example 1-1

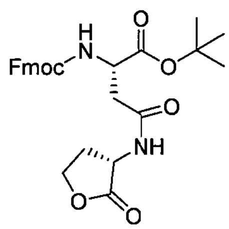

Example 1-2

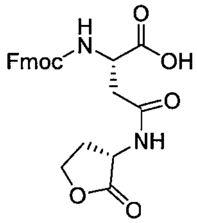

Example 1-3

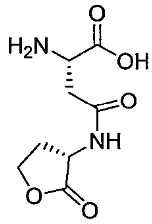

Example 1-6

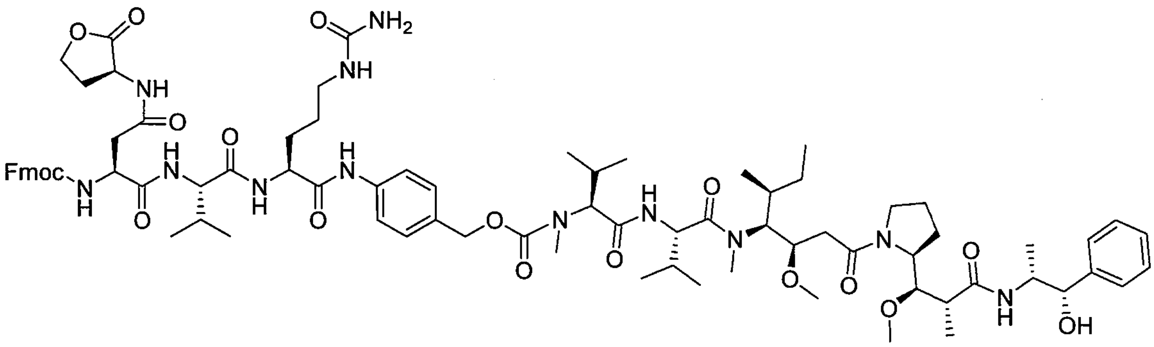

Example 1-7

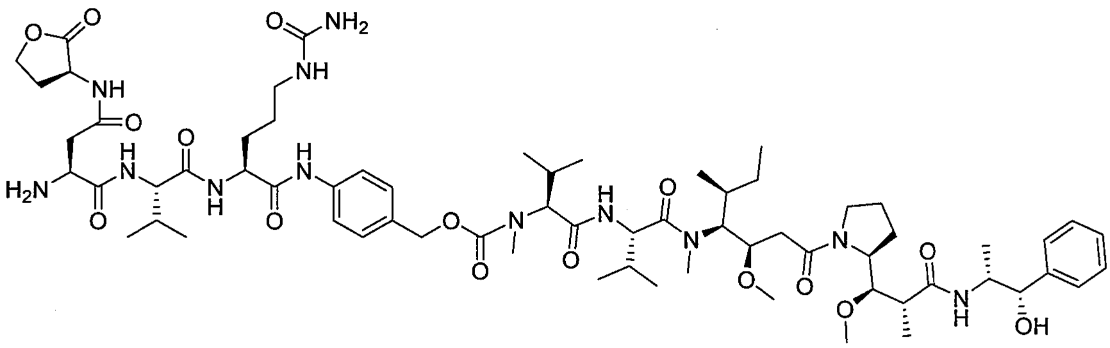

Example 2-1

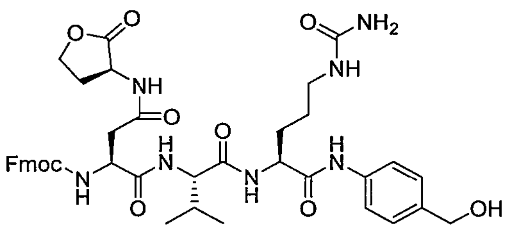

Example 2-2

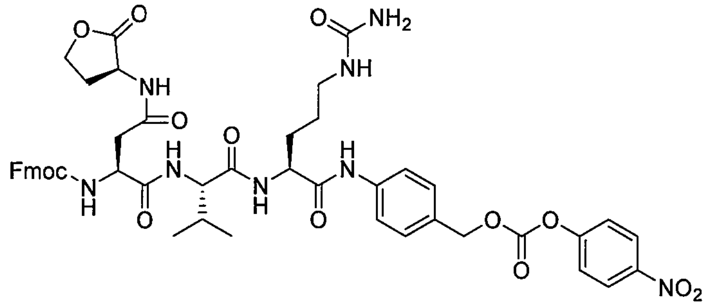

Example 2-3

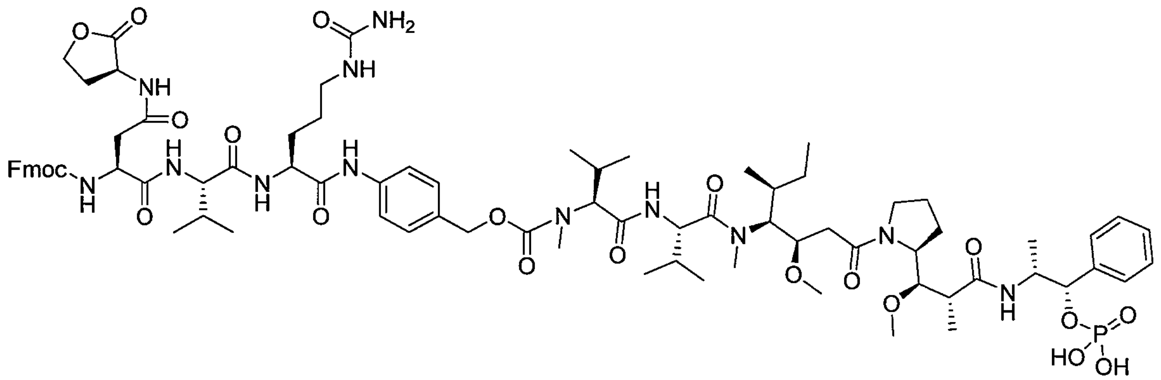

Example 2-4

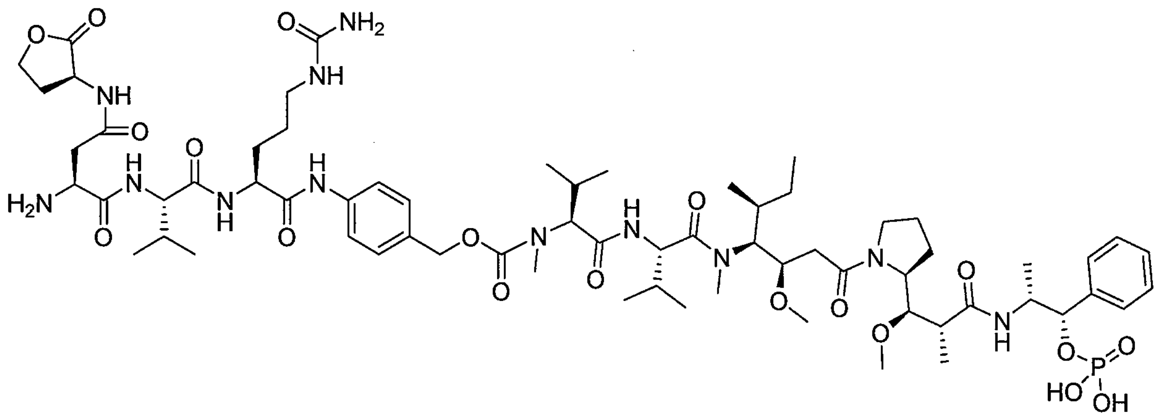

Example 3-4

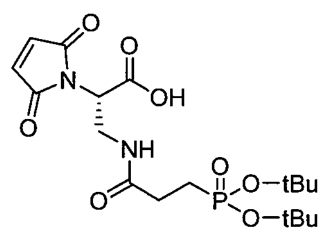

Example 3-5

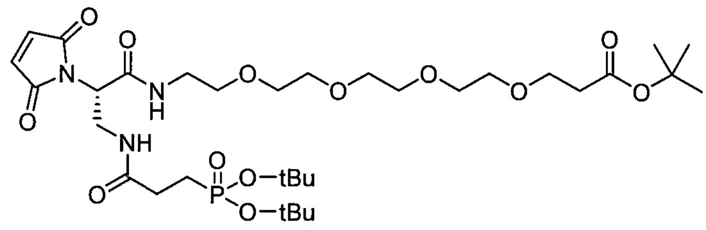

Example 3-6

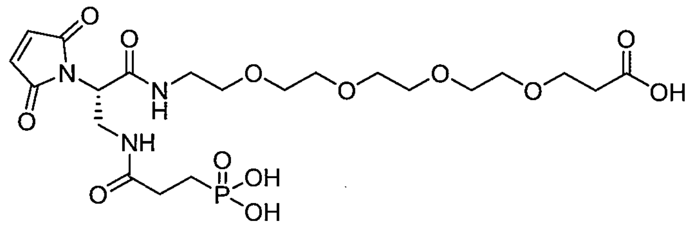

Example 3-7

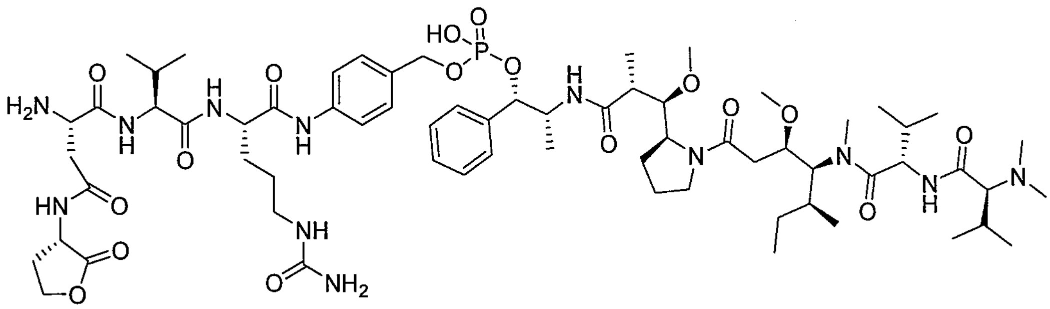

Example 4-2

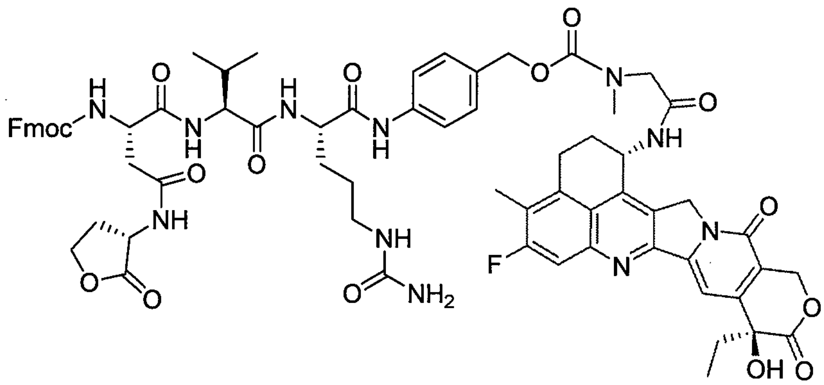

Example 5-1

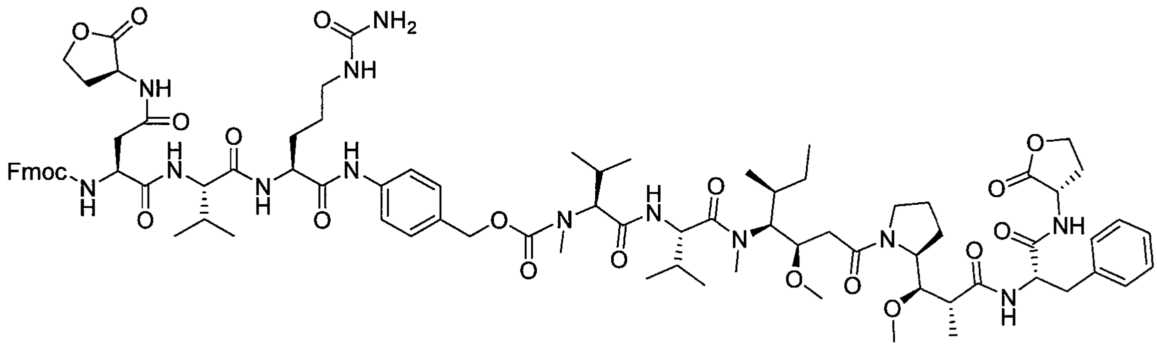

Example 6-1

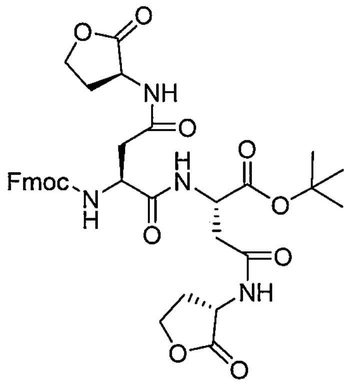

Example 6-2

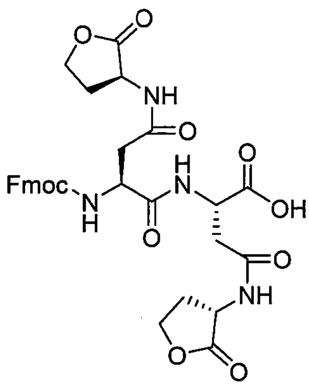

Example 6-3

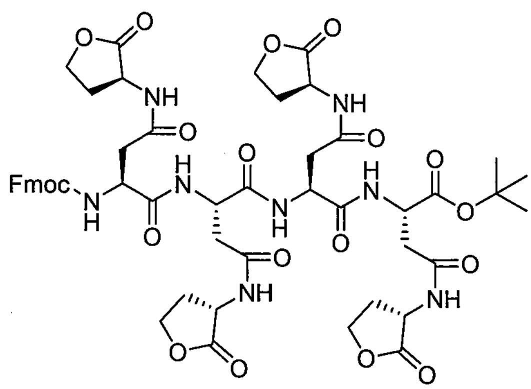

Example 6-4

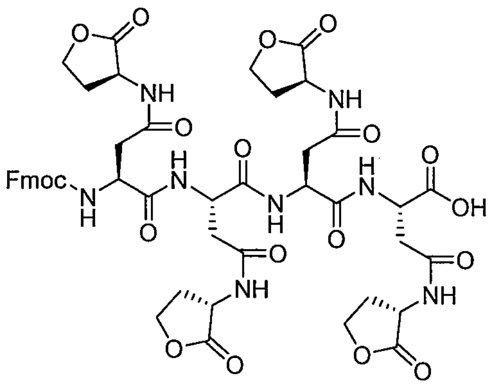

Example 6-5

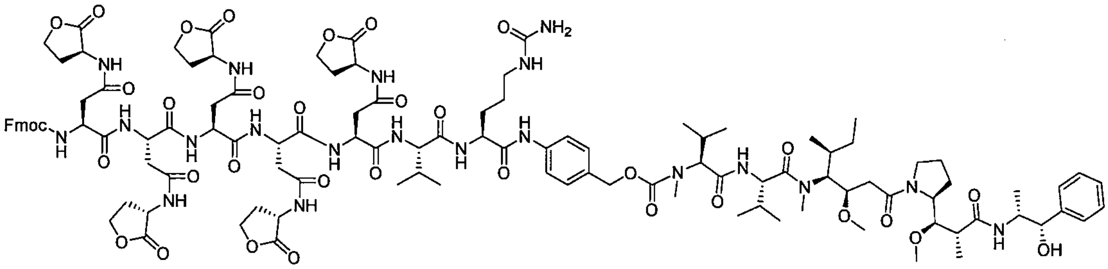

Example 7-4

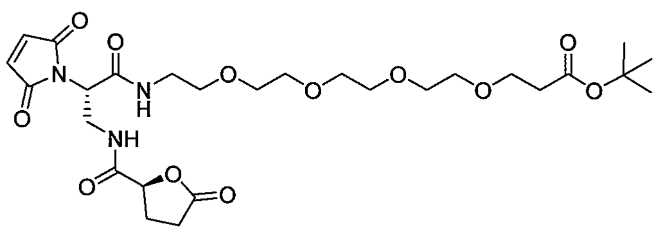

Example 7-5

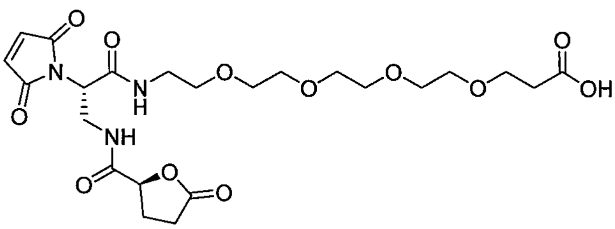

Example 9-1

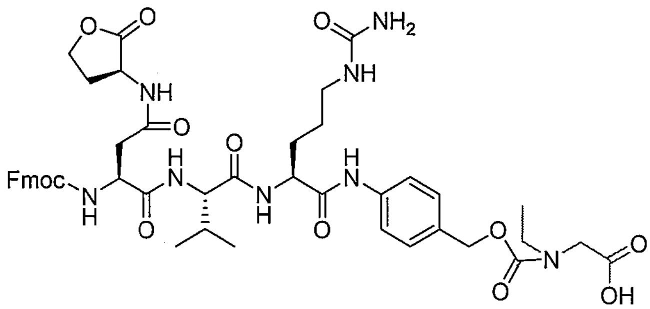

Example 9-2

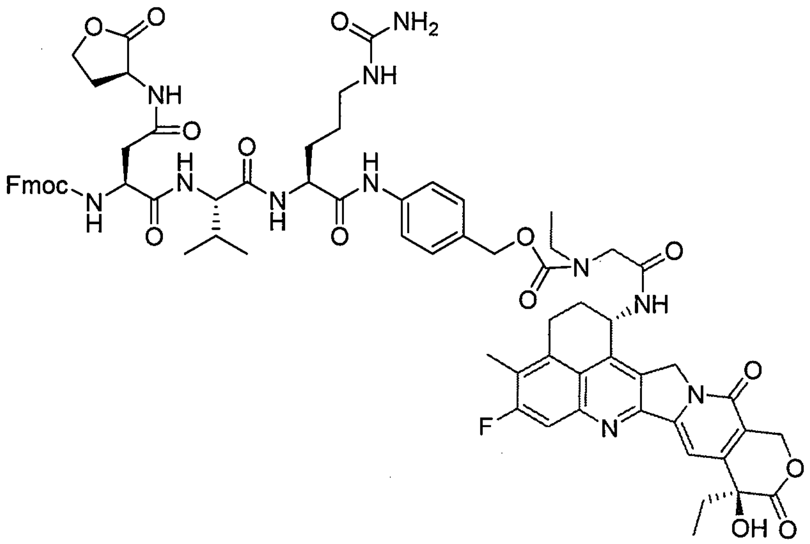

Example 10-1

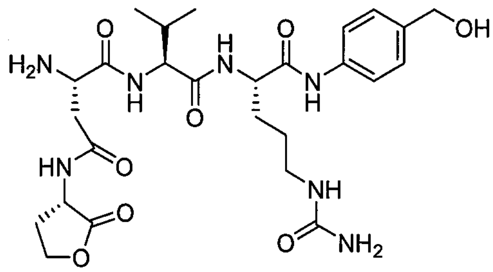

Example 10-2

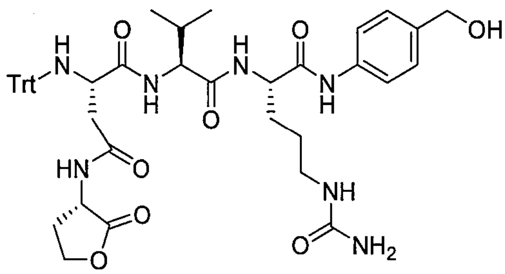

Example 10-5

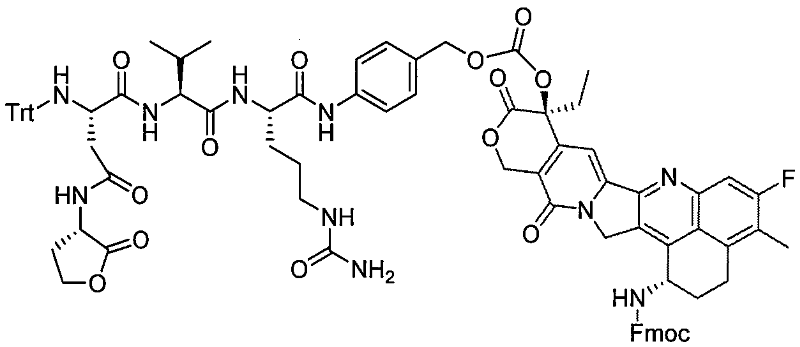

Example 10-6

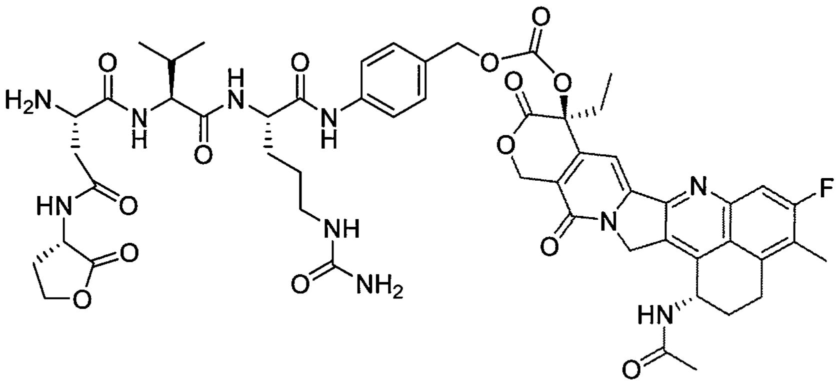

Example 11-1

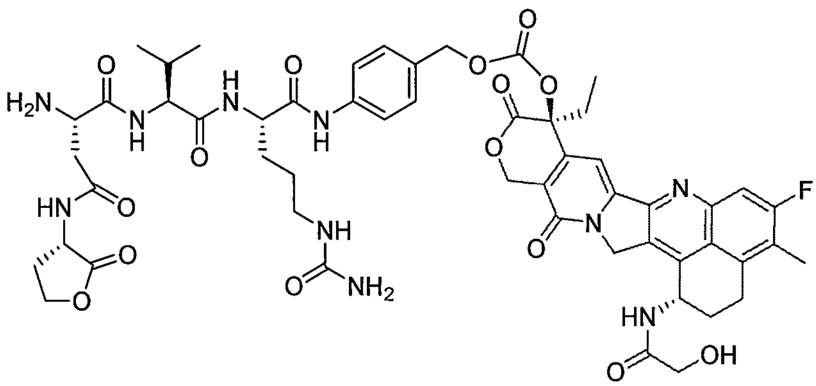

Example 12-1

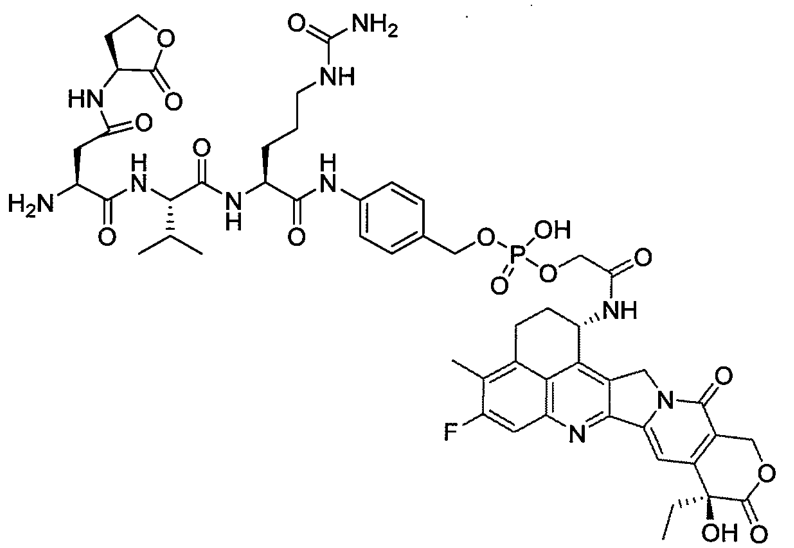

Example 13-1

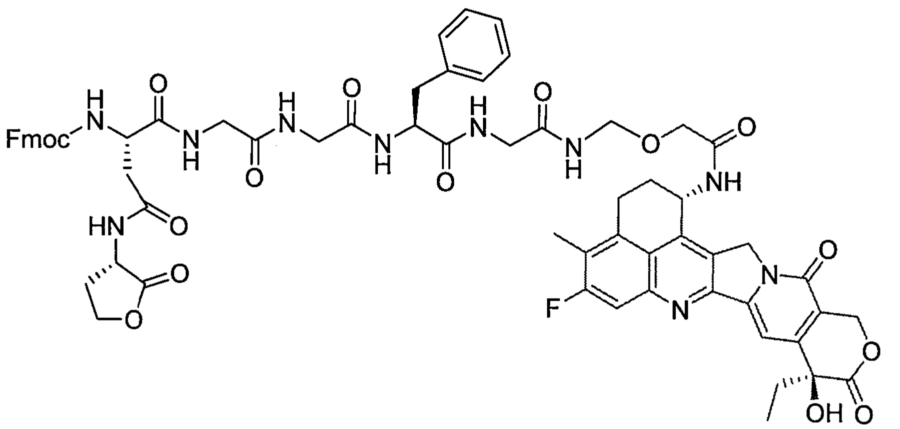

Example 14-1

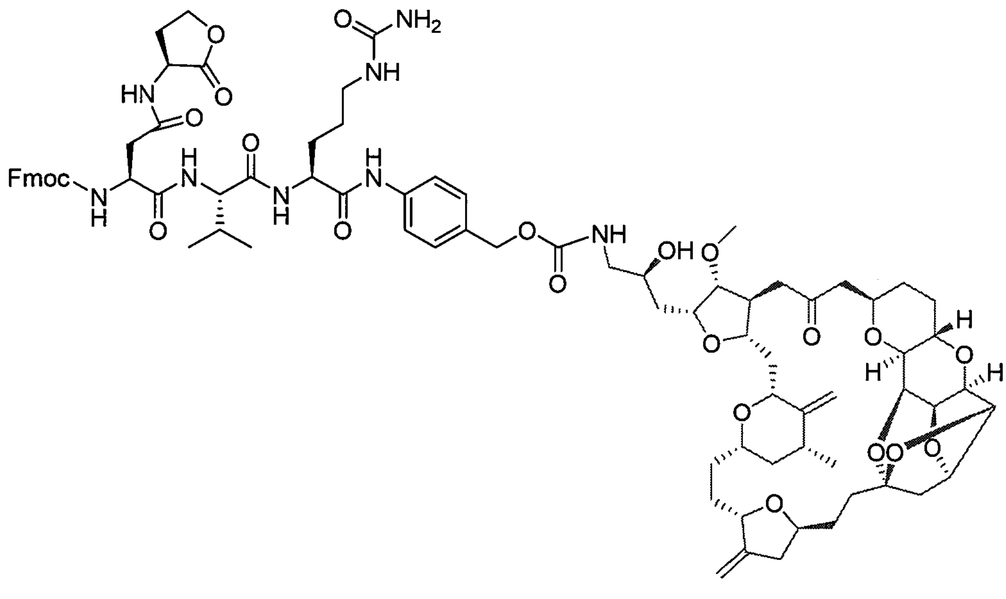

Example 15-1

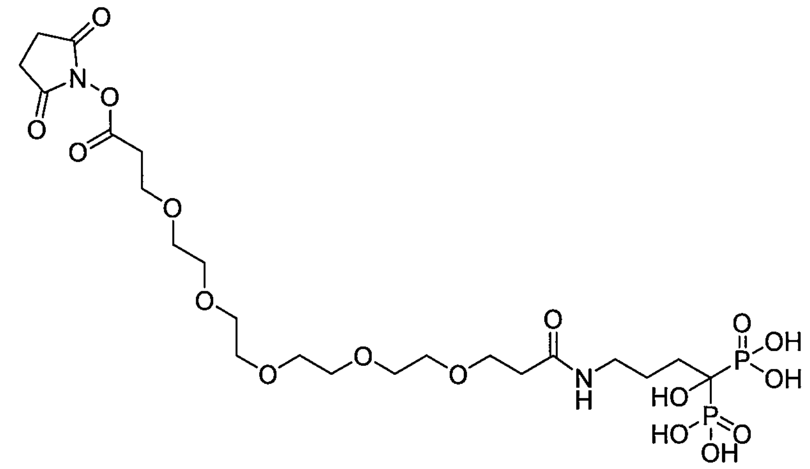

Example 15-2

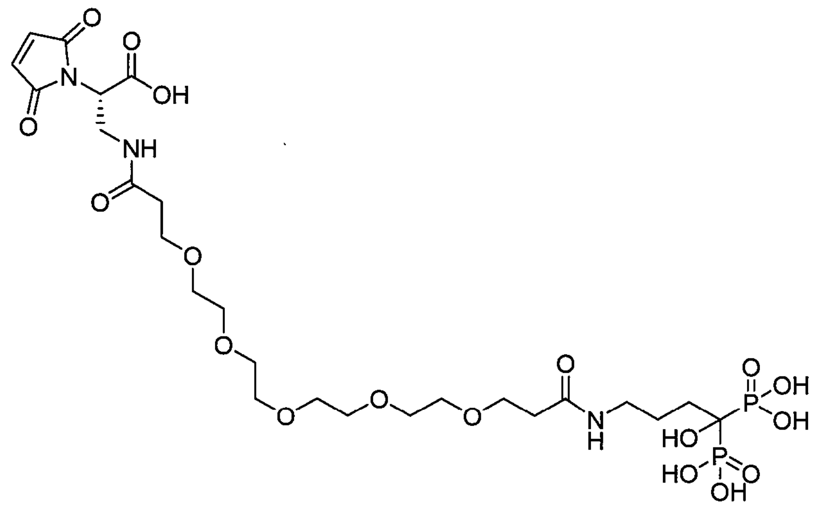

Example 15-3

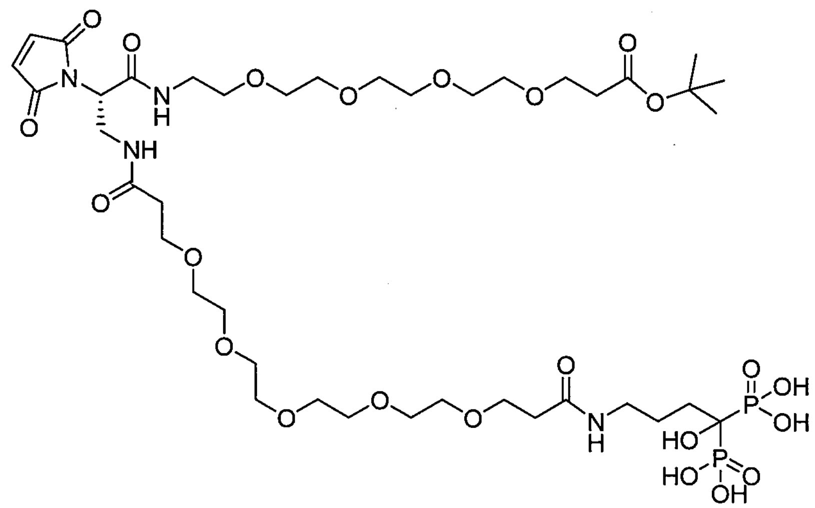

Example 15-4

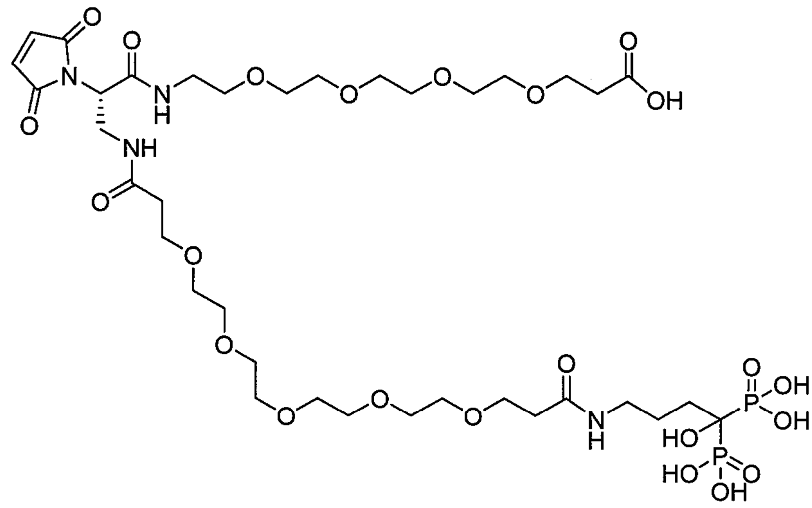

Example 16-2

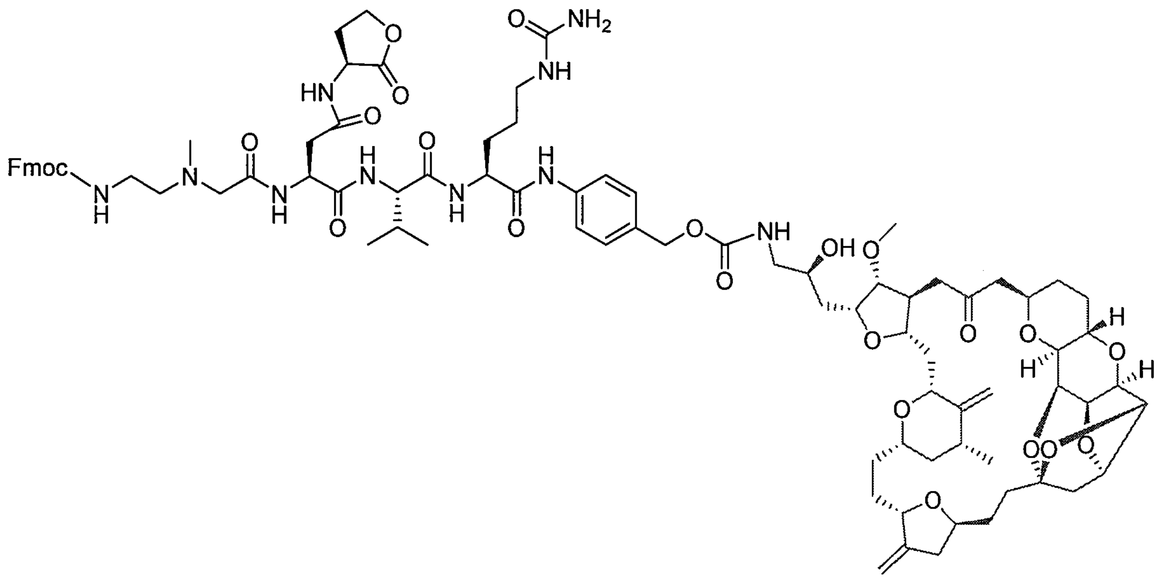

Example 17-1

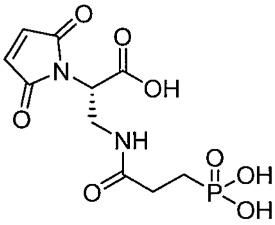

Example 17-2

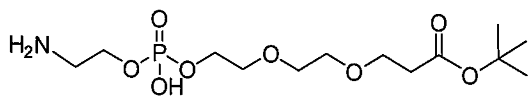

Example 17-3

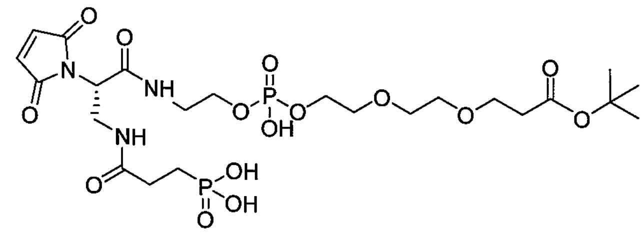

Example 17-4

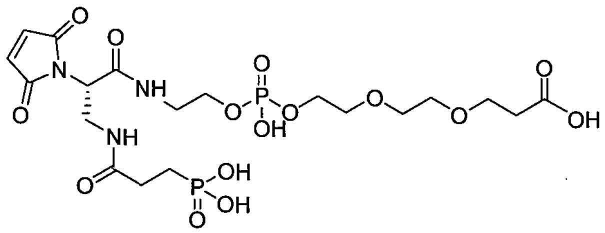

Example 18-1

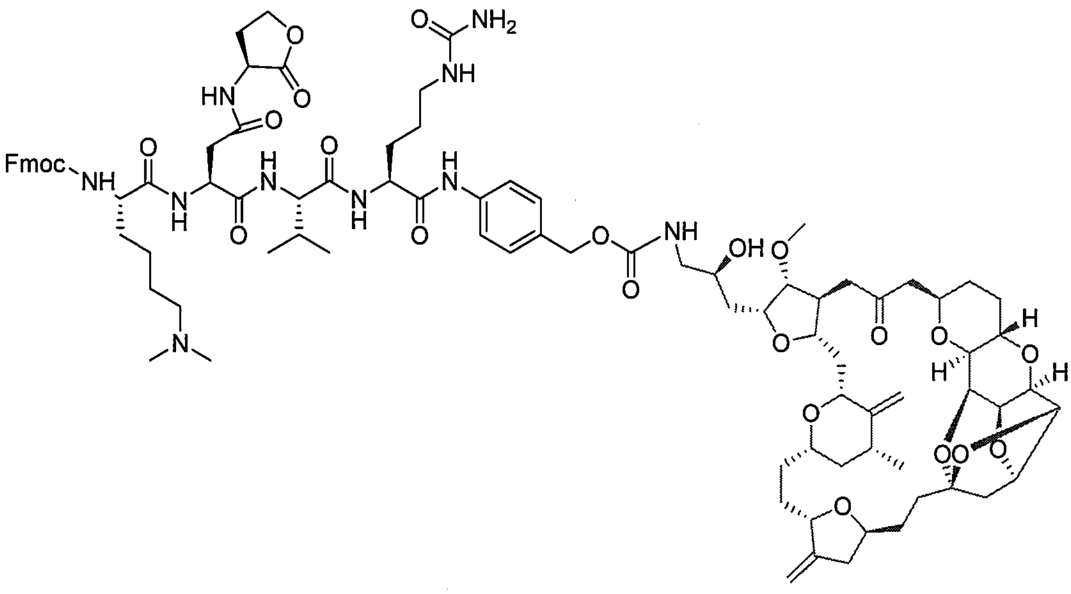

Example 19-3

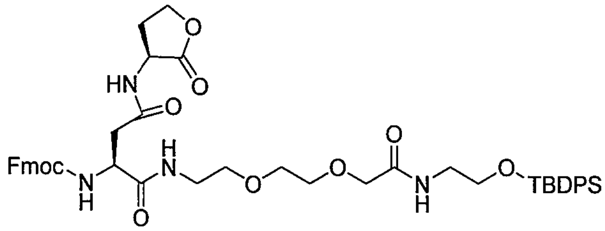

Example 19-4

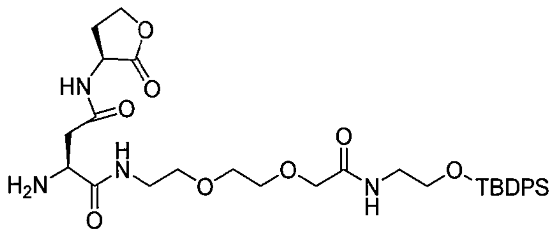

Example 19-5

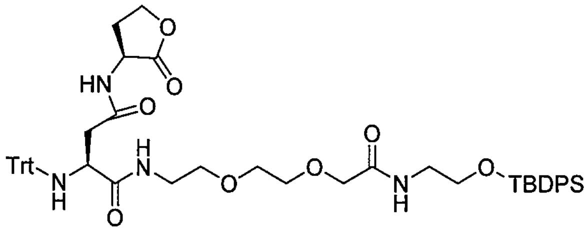

Example 19-6

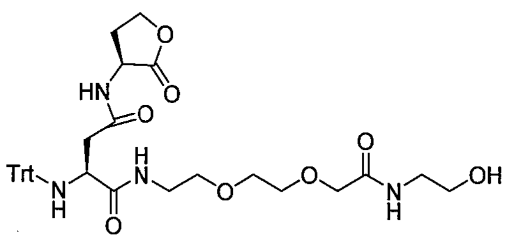

Example 19-7

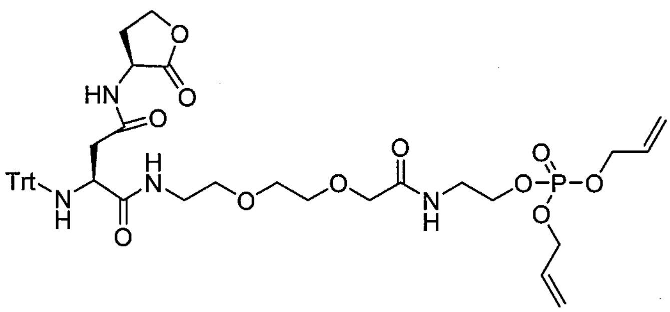

Example 19-8

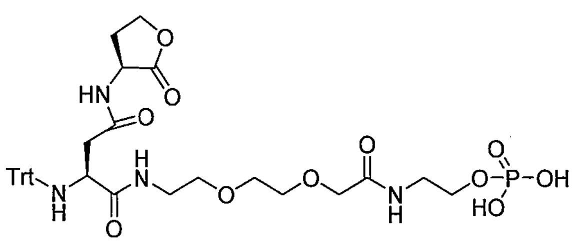

Example 19-9

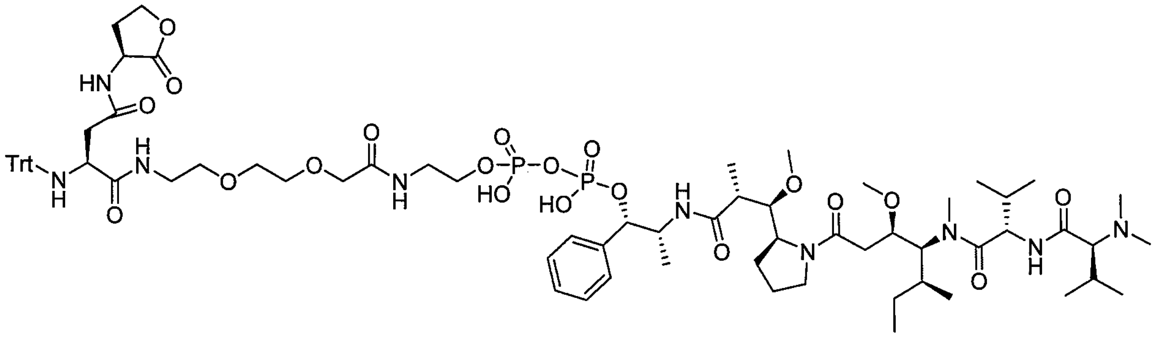

Example 19-10

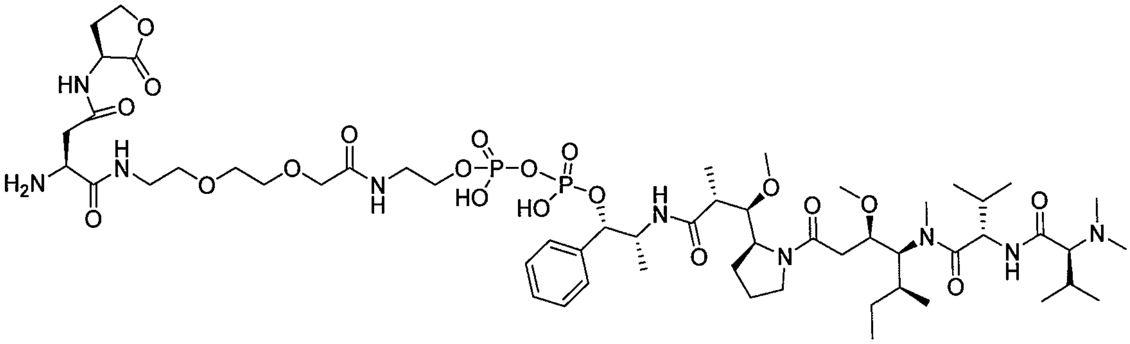

Example 20-1

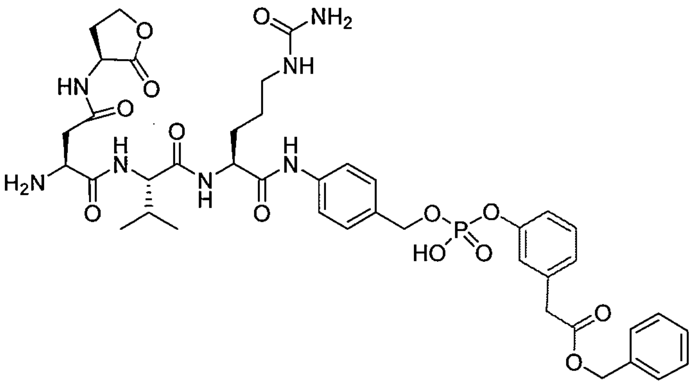

Example 20-2

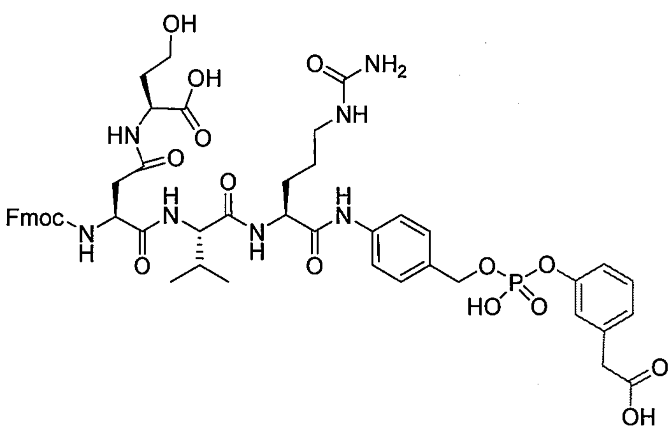

Example 20-3

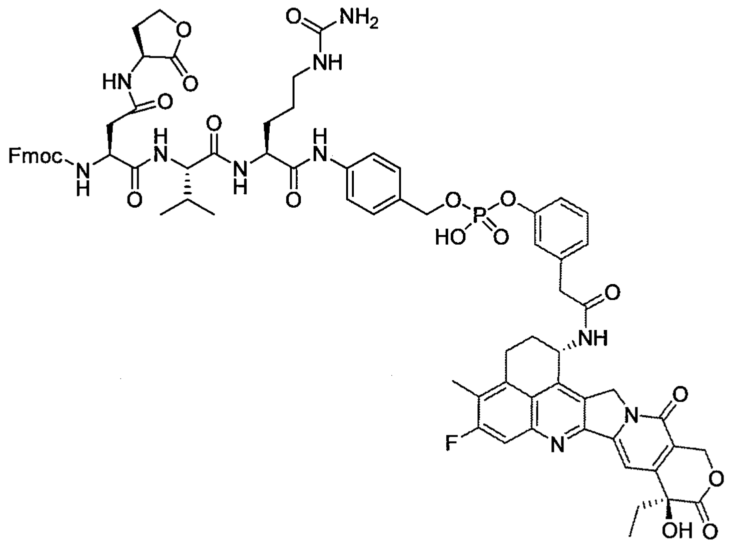

Example 23-1

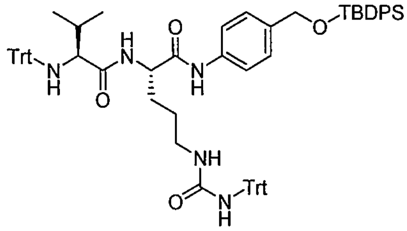

Example 23-2

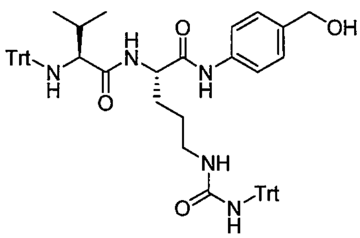

Example 23-3

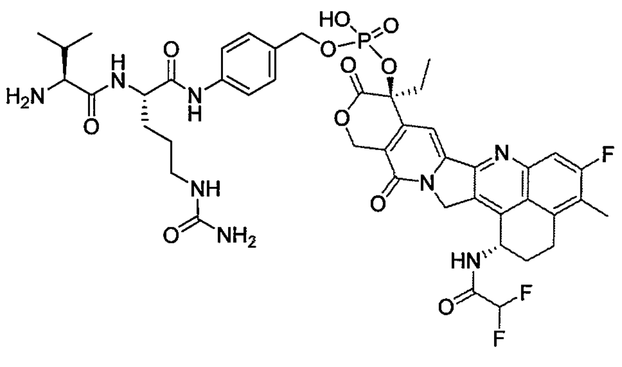

Example 23-4

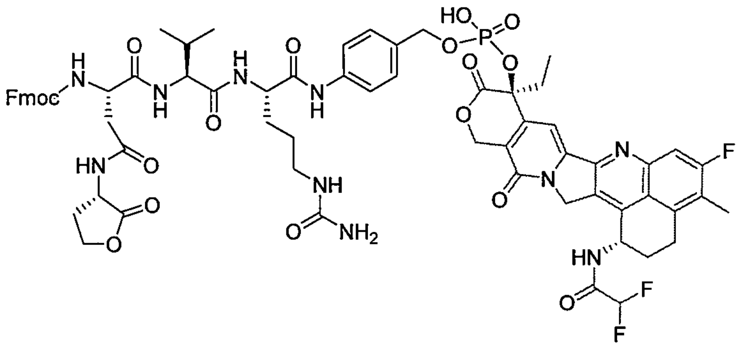

Example 23-5

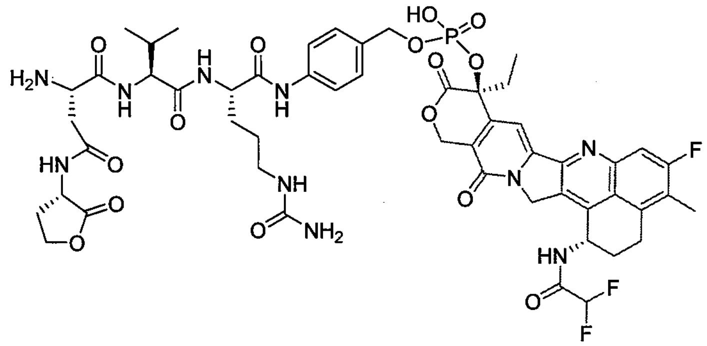

Example 24-1

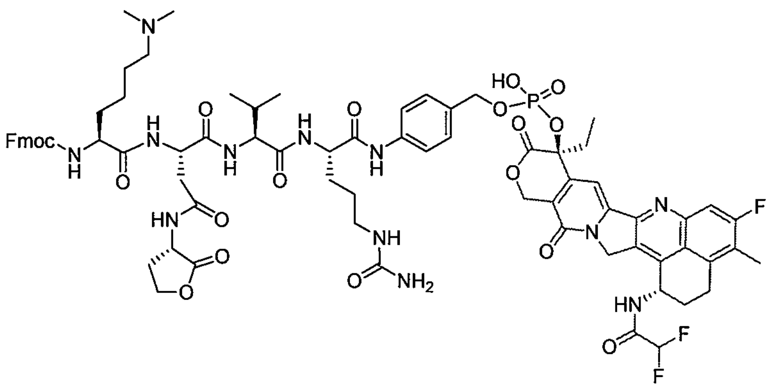

Example 24-2

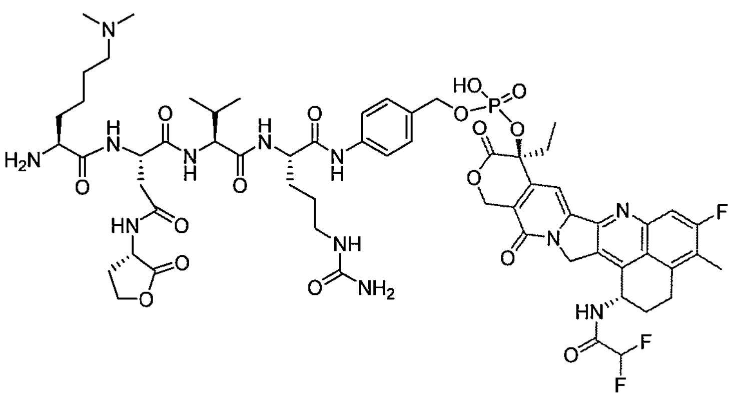

Example 25-3

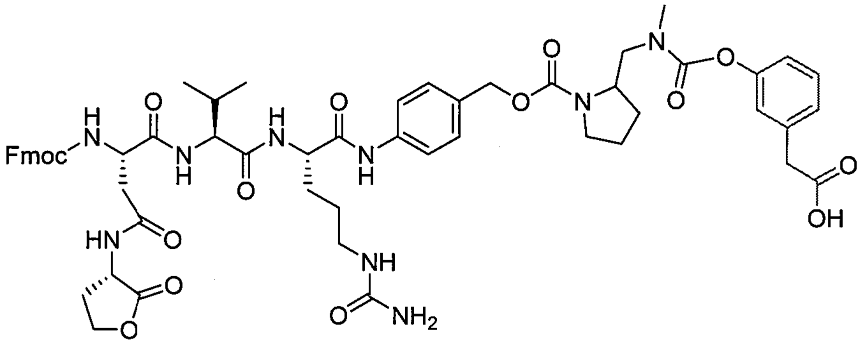

Example 25-4

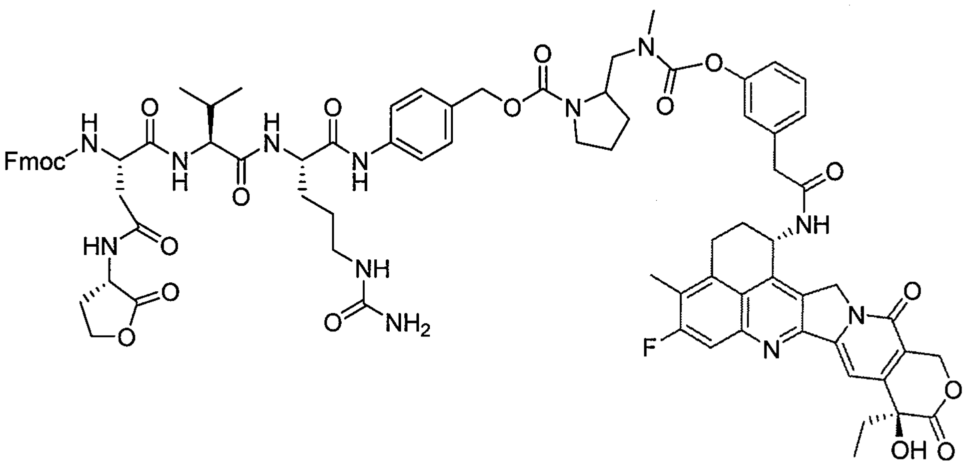

Example 26-1

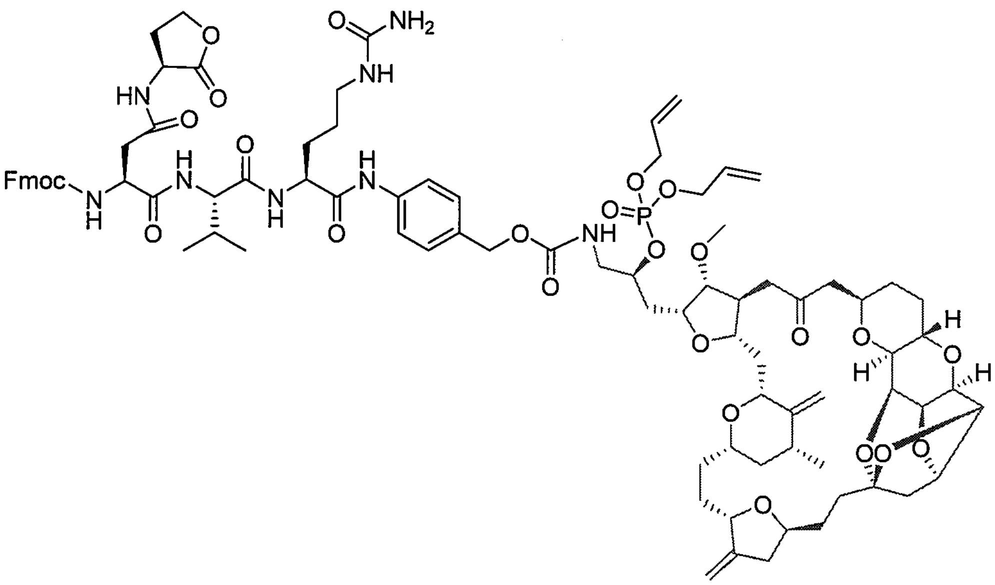

and
Example 26-2

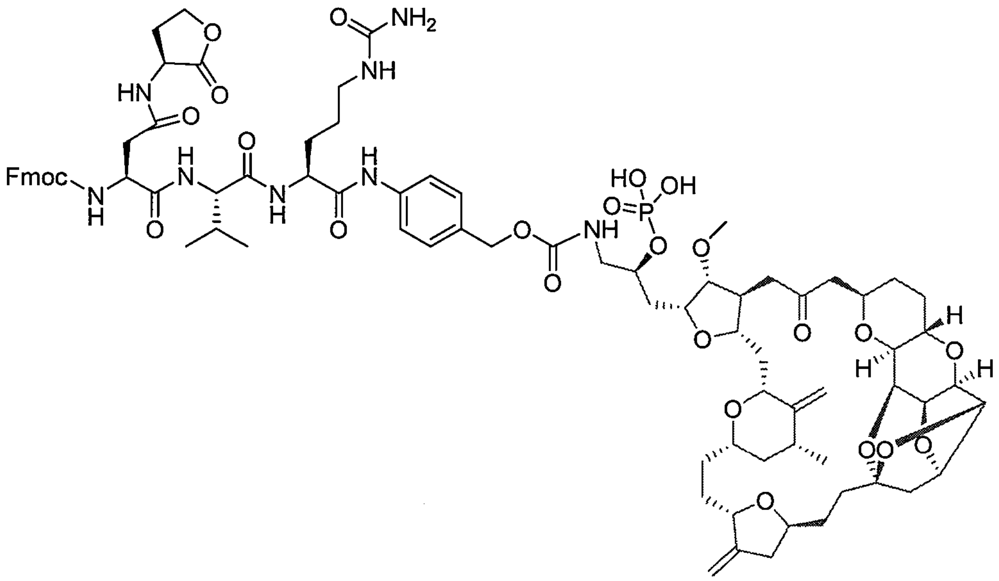

or a salt thereof.

Hereinafter, aspects of the present invention are described. Further, aspects created by optionally selecting and combining the following each aspect and aspects created by optionally combining the following each aspect with any aspect(s) or embodiment(s) etc. described herein are also encompassed by the present invention.

[Aspect A1]

The conjugate precursor (I), wherein in the above general formula (I):

Z-L-D (I)

Z is a reactive group which can react with a functional group present in an antibody or a modified antibody,
or a salt thereof.

[Aspect A2]

The conjugate precursor (I) according to [Aspect A1] or a salt thereof, wherein Z is a maleimidyl group (the above formula (i)), an α-halogenomethylcarbonyl group (the above formula (ii)), an ethynylphosphonamidate group (the above formula (iii)), a carboxy group, an active ester of carboxy group, a sulfhydryl group, a hydroxy group, an amino group, an alkynyl group, a cycloalkynyl group, an azacycloalkynyl group, or an azide group ($-N_3$ group).

[Aspect A3]

The conjugate precursor (I) according to [Aspect A1] or [Aspect A2] or a salt thereof, wherein Z is a maleimidyl group (the above formula (i)), an α-halogenomethylcarbonyl group (the above formula (ii)), an ethynylphosphonamidate group (the above formula (iii)), a carboxy group, an active ester of carboxy group, an alkynyl group, a cycloalkynyl group, an azacycloalkynyl group, or an azide group ($-N_3$ group).

[Aspect A4]

The conjugate precursor (I) according to any one of [Aspect A1] to [Aspect A3] or a salt thereof, wherein Z is a maleimidyl group (the above formula (i)), an α-halogenomethylcarbonyl group (the above formula (ii)), a carboxy group, or an active ester of carboxy group.

[Aspect B1]

The conjugate precursor (I) according to any one of [Aspect A1] to [Aspect A4] or a salt thereof, wherein D is an

antitumor drug residue in which one hydrogen atom or one hydroxy group is removed from any position of an antitumor drug molecule or an analog thereof or a derivative thereof (hereinafter also referred to as "antitumor drug residue").

[Aspect B2]

The conjugate precursor (I) according to any one of [Aspect A1] to [Aspect A4] and [Aspect B1] or a salt thereof, wherein D is an antitumor drug residue of camptothecin; auristatin E (AE) or monomethyl auristatin E (MMAE); maytansine; PBD (parabenzodiazepine) dimer; eribulin; 5-FU; PD-318088; AS-703026; TAK-733; LY-3023414; calicheamicin; paclitaxel; docetaxel; mitomycin C; bleomycin; cyclocytidine; vincristine; vinblastine; daunomycin; doxorubicin; dolastatin 10; superdox; ciprofloxacin; cadrofloxacin (CS-940); or an analog of said antitumor drug; or a derivative of said antitumor drug molecule or an analog thereof.

[Aspect B3]

The conjugate precursor (I) according to any one of [Aspect A1] to [Aspect A4], [Aspect B1], and [Aspect B2] or a salt thereof, wherein D is an antitumor drug residue of camptothecin; auristatin E (AE) or monomethyl auristatin E (MMAE); maytansine; PBD (parabenzodiazepine) dimer; eribulin; 5-FU; PD-318088; AS-703026; TAK-733; LY-3023414; calicheamicin; paclitaxel; docetaxel; mitomycin C; bleomycin; cyclocytidine; vincristine; vinblastine; daunomycin; doxorubicin; dolastatin 10; superdox; or an analog of said antitumor drug; or a derivative of said antitumor drug molecule or an analog thereof.

[Aspect B4]

The conjugate precursor (I) according to any one of [Aspect A1] to [Aspect A4] and [Aspect B1] to [Aspect B3] or a salt thereof, wherein D is an antitumor drug residue of camptothecin; auristatin E (AE) or monomethyl auristatin E (MMAE); maytansine; PBD (parabenzodiazepine) dimer; eribulin; or an analog of said antitumor drug; or a derivative of said antitumor drug molecule or an analog thereof.

[Aspect B5]

The conjugate precursor (I) according to any one of [Aspect A1] to [Aspect A4] and [Aspect B1] to [Aspect B4] or a salt thereof, wherein D is an antitumor drug residue in which one hydrogen atom or one hydroxy group is removed from any position of an antitumor drug or an analog of said antitumor drug in which at least one hydroxy group present in the molecule is phosphorylated; or a derivative of said antitumor drug molecule or an analog thereof.

[Aspect C1]

The conjugate precursor (I) according to any one of [Aspect A1] to [Aspect A4] and [Aspect B1] to [Aspect B5] or a salt thereof, wherein

L is an optionally substituted alkylene group;
one or more methylene group(s) in the chain of said alkylene group is/are optionally replaced with one or more divalent group(s) independently selected from the group consisting of -C($R^1$)($R^2$) -; -O-; -N($R^3$)-; -N($R^3$)-N($R^3$)-; -S-; -Se-; -Si($R^4$)($R^5$)-; -S-S-; -Se-Se-; -SOm-; -SeOn-; - C(=C($R^6$)($R^7$)) -; -C(=O)-; -C(=S)-; -C(=N($R^8$))-; -C(=N-O$R^9$)-; -C(=N-N($R^{10}$)($R^{11}$))-; -P(=O)($R^{12}$)-; -P(=O)(O$R^{13}$)-; -O-P(=O)($R^{12}$)-O-; -O-P(=O)(O$R^{13}$)-O-; -C($R^{14}$)=; =C($R^{14}$)-; - C($R^{14}$) =C($R^{14}$)-; -N=; =N-; -C≡C-; -(O-C($R^1$)($R^2$)-C($R^1$)($R^2$))$_{1-30}$-; - (C($R^1$)($R^2$)-C($R^1$)($R^2$)-0)$_{1-30}$-; an optionally substituted alkenylene group, an optionally substituted alkynylene group, an optionally substituted cycloalkylene group; an optionally substituted cycloalkenylene group; an optionally substituted cycloalkynylene group; an optionally substituted azacycloalkynylene group; an optionally substituted arylene group; an optionally substituted heteroarylene group; and an optionally substituted heterocyclylene group;
$R^1$, $R^2$, $R^3$, $R^4$, $R^5$, $R^6$, $R^7$, $R^8$, $R^9$, $R^{10}$, $R^{11}$, $R^{12}$, $R^{13}$, and $R^{14}$ are each independently a group selected from the group consisting of a hydrogen atom, an optionally substituted alkyl group, an optionally substituted alkenyl group, an optionally substituted alkynyl group, an optionally substituted cycloalkyl group, an optionally substituted cycloalkenyl group, an optionally substituted cycloalkynyl group, an optionally substituted azacycloalkynyl group, an optionally substituted aryl group, an optionally substituted heteroaryl group, and an optionally substituted heterocyclyl group, wherein when $R^3$ is an alkyl group, said alkyl group is optionally combined with an alkyl group on an adjacent methylene group to form a cyclic structure; and
m and n are each independently an integer of 0 to 2.

[Aspect C2]

The conjugate precursor (I) according to [Aspect C1] or a salt thereof, wherein

L is an optionally substituted alkylene group;
one or more methylene group(s) in the chain of said alkylene group is/are replaced with one or more divalent group(s) independently selected from the following formula group:
formula group

-O-C(H or $C_1$-$C_4$ alkyl) (H or $C_1$-$C_4$ alkyl)-;
-S-C(H or $C_1$-$C_4$ alkyl) (H or $C_1$-$C_4$ alkyl)-;
-N(H or $C_1$-$C_4$ alkyl) -;
-N(H or $C_1$-$C_4$ alkyl)-O-C(H or $C_1$-$C_4$ alkyl) (C(H or $C_1$-$C_4$ alkyl)-;
-C(H or $C_1$-$C_4$ alkyl)-$SO_{0\text{-}2}$-C(H or $C_1$-$C_4$ alkyl)-;
-C(H or $C_1$-$C_4$ alkyl)-S-S-C(H or $C_1$-$C_4$ alkyl)-;
-C(H or $C_1$-$C_4$ alkyl)=C(H or $C_1$-$C_4$ alkyl)-;
-C(H or $C_1$-$C_4$ alkyl)=N-;
-C(H or $C_1$-$C_4$ alkyl)=N-O-;
-C(H or $C_1$-$C_4$ alkyl)=N-N(H or $C_1$-$C_4$ alkyl)-;
-C(=C(H or $C_1$-$C_4$ alkyl) (H or $C_1$-$C_4$ alkyl)
-C(=N-O(H or $C_1$-$C_4$ alkyl))-;
-C(=N-N(H or $C_1$-$C_4$ alkyl) (H or $C_1$-$C_4$ alkyl)
-O-Si ($C_1$-$C_4$ alkyl $CH_3$) ($C_1$-$C_4$ alkyl) -O-;
-C (H or $C_1$-$C_4$ alkyl) -N ($C_1$-$C_4$ alkyl) -$CH_2$-;
-C(H or $C_1$-$C_4$ alkyl) (H or $C_1$-$C_4$ alkyl)-C(=O)-C(H or $C_1$-$C_4$ alkyl) (H or $C_1$-$C_4$ alkyl)-;
-C(=O)-O-;
-C(=O)-S-;
-C(=O)-N(H or $C_1$-$C_4$ alkyl)-;
-O-C(=O)-O-;
-N(H or $C_1$-$C_4$ alkyl)-C(=O)-N(H or $C_1$-$C_4$ alkyl)-;
-C(=O)-N(H or $C_1$-$C_4$ alkyl)-C(H or $C_1$-$C_4$ alkyl) (H or $C_1$-$C_4$ alkyl)-;
-C(=O)-$(CH_2)_{1\text{-}10}$-$(O\text{-}CH_2CH_2)_{1\text{-}20}$-O-$(CH_2)_{1\text{-}10}$-C(=O)-;
-C(=O)-$(CH_2)_{1\text{-}10}$-$(O\text{-}CH_2CH_2)_{1\text{-}20}$-O-$(CH_2)_{1\text{-}10}$-$CH_2$-NH-;
-NH-$CH_2$-$(CH_2)_{1\text{-}10}$$(O\text{-}CH_2CH_2)_{1\text{-}20}$-O-$(CH_2)_{1\text{-}10}$-$CH_2$-NH-;
-C(=O)-N($C_1$-$C_4$ alkyl)-$CH_2CH_2$-N($C_1$-$C_4$ alkyl)-C(=O)-;

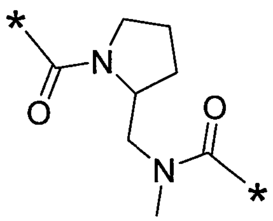

[wherein * is a point of attachment to an adjacent group];
-P(=O)(OH)-O-;
-O-P(=O)(OH)-O-;
-O-P(=S)(OH)-O-;
-O-P(=O)(OH)-S-;
-O-P(=O)(OH)-O-P(=O)(OH)-O-;
-O-P(=O) (OH)-O-P(=O) (OH)-O-P(=O) (OH)-O-;
-CH($CH_2$-$NH_2$)-;
-CH($CH_2$-NH($C_1$-$C_4$ alkyl))-;
-CH($CH_2$-N($C_1$-$C_4$ alkyl)$_2$)-;
-CH($CH_2$-NH-C(=O)-$(CH_2CH_2\text{-}O\text{-})_{0\text{-}20}$-$C_1$-$C_4$ alkyl)-;
-CH($CH_2$-NH-C(=O)-$(CH_2)_{0\text{-}20}$-lactonyl)-;
-CH($CH_2$-NH-C(=O)-$(CH_2CH_2\text{-}O\text{-})_{1\text{-}20}$-$(CH_2)_{1\text{-}10}$-$CH_2$-lactonyl)-;
-CH ($CH_2$-NH-C(=O)-$(CH_2CH_2\text{-}O\text{-})_{1\text{-}20}$- ($CH_2P_{1\text{-}10}$-$CH_2$-NH-lactonyl)-;

$-CH(CH_2-NH-C(=O)-(CH_2CH_2-O-_{1-20}-(CH_2)_{1-10}-CH_2-NH-C(=O)-lactonyl)-;$
$-CH(CH_2-NH-C(=O)-(CH_2CH_2-O-)_{1-20}-(CH_2)_{1-10}-C(=O)-NH-lactonyl)-;$
$-CH(CH_2-NH-(CH_2)_{1-20}-(O)_{0-1}-(P(=O) (OH)_2) -;$
$-CH(CH_2-NH-C(=O)-(CH_2)_{1-20}-(P(=O)(OH)_2) -;$
$-CH(CH_2-NH-C(=O)-(CH_2)_{1-20}-C(=O)-NH-(CH_2)_{1-10}-(P(=O)(OH)_2)-;$
$-CH(CH_2-NH-C(=O)-(CH_2CH_2-O-)_{1-20}-(CH_2)_{1-10}-CH_2-(P(=O)(OH)_2)-;$
$-CH(CH_2-NH-C(=O)-(CH_2CH_2-O-)_{1-20}-(CH_2)_{1-10}-C(=O)-NH-(CH_2)_{1-10}-(P(=O)(OH)_2)-;$
$-CH(CH_2-NH-(CH_2)_{1-10}-C(H, OH, Cl, NH_2, or C_1-C_4 alkyl)(P(=O)(OH)_2)_2)-;$
$-CH(CH_2-NH-C(=O)-(CH_2)_{1-10}-C(=O)-NH-(CH_2)_{1-10}-C(H, OH, Cl, NH_2, or C_1-C_4 alkyl) (P(=O)(OH)_2)_2)-;$
$-CH(CH_2-NH-C(=O)-(CH_2CH_2-O-)_{1-20}-(CH_2)_{1-10}-C(=O)-NH-(CH_2)_{1-10}-C(H, OH, Cl, NH_2, or C_1-C_4 alkyl) (P(=O)(OH)_2)_2)-;$
$-N((CH_2)_{1-10}-(O-CH_2CH_2)_{1-20}-NH-C(=O)-(CH_2)_{1-10}-(D-glucose-6-O-yl))-;$
$-N((CH_2)_{1-10}-(O-CH_2CH_2)_{1-10}-C(=O)-NH-(CH_2)_{1-10}-(O-CH_2CH_2)_{1-10}-NH-C(=O)-(CH_2)_{1-10}-(D-glucose-6-O-yl))-;$
$-N((CH_2)_{1-10}-(O-CH_2CH_2)_{1-10}-C(=O)-NH-(CH_2)_{1-10}-(O-CH_2CH_2)_{1-10}-C(=O)-NH-(CH_2)_{1-10}-(O-CH_2CH_2)_{1-10}-NH-C(=O)-(CH_2)_{1-10}-(D-glucose-6-O-yl))-;$
$-(cis)-CH=CH-P(=O)(O-CH_2CH_3)-NH-(phenylene)-C(=O)-;$
-C(=O)-(cyclohexylene)-;
-(succinimidylene)-;
-Ser-;
-Cys-;
-Ama-:

-Asp-;
-Glu-;
-Orn-;
-Lys-;
-ValLys-;
-ValCit-;
-ValAla-;
$-GlyGly-C(=O)-NH-(optionally substituted phenylene)-CH_2-;$
$-PheLys-C(=O)-NH-(optionally substituted phenylene)-CH_2-;$
$-ValLys-C(=O)-NH-(optionally substituted phenylene)-CH_2-;$
$-ValCit-C(=O)-NH-(optionally substituted phenylene)-CH_2-;$
$-ValAla-C(=O)-NH-(optionally substituted phenylene)-CH_2-;$
$-AspValCit-C(=O)-NH-(optionally substituted phenylene)-CH_2-;$
$-LysAspValCit-C(=O)-NH-(optionally substituted phenylene)-CH_2-;$
$-GluValCit-C(=O)-NH-(optionally substituted phenylene)-CH_2-;$
$-LysValCit-C(=O)-NH-(optionally substituted phenylene)-CH_2-;$
$-SerValCit-C(=O)-NH-(optionally substituted phenylene)-CH_2-;$
$-AspValAla-C(=O)-NH-(optionally substituted phenylene)-CH_2-;$
$-GluValAla-C(=O)-NH-(optionally substituted phenylene)-CH_2-;$
$-LysValAla-C(=O)-NH-(optionally substituted phenylene)-CH_2-;$
$-SerValAla-C(=O)-NH-(optionally substituted phenylene)-CH_2-;$
$-GlyGlyPheGly-C(=O)-NH-(optionally substituted phenylene)-CH_2-;$
$-AspGlyGlyPheGly-C(=O)-NH-(optionally substituted phenylene)-CH_2-;$
$-GluGlyGlyPheGly-C(=O)-NH-(optionally substituted phenylene)-CH_2-;$
$-LysGlyGlyPheGly-C(=O)-NH-(optionally substituted phenylene)-CH_2-;$
$-SerGlyGlyPheGly-C(=O)-NH-(optionally substituted phenylene)-CH_2-;$
$-AspAspAspAspAsp-C(=O)-NH-(optionally substituted phenylene)-CH_2-;$
$-GlyGly-C(=O)-NH-(optionally substituted phenylene)-CH_2-O-C (=O) -;$
$-PheLys-C(=O)-NH-(optionally substituted phenylene)-CH_2-O-C(=O)-;$
$-ValLys-C(=O)-NH-(optionally substituted phenylene)-CH_2-O-C(=O)-;$
$-ValCit-C(=O)-NH-(optionally substituted phenylene)-CH_2-O-C(=O)-;$
$-ValAla-C(=O)-NH-(optionally substituted phenylene)-CH_2-O-C(=O)-;$
$-AspValCit-C(=O)-NH-(optionally substituted phenylene)-CH_2-O-C(=O)-;$
$-GluValCit-C(=O)-NH-(optionally substituted phenylene)-CH_2-O-C(=O)-;$
$-LysValCit-C(=O)-NH-(optionally substituted phenylene)-CH_2-O-C(=O)-;$

-SerValCit-C(=O)-NH-(optionally substituted phenylene)-$CH_2$-O-C(=O)-;
-AspValAla-C(=O)-NH-(optionally substituted phenylene)-$CH_2$-O-C(=O)-;
-GluValAla-C(=O)-NH-(optionally substituted phenylene)-$CH_2$-O-C(=O)-;
-LysValAla-C(=O)-NH-(optionally substituted phenylene)-$CH_2$-O-C(=O)-;
-SerValAla-C(=O)-NH-(optionally substituted phenylene)-$CH_2$-O-C(=O)-;
-GlyGlyPheGly-C(=O)-NH-(optionally substituted phenylene)-$CH_2$-O-C(=O)-;
-AspGlyGlyPheGly-C(=O)-NH-(optionally substituted phenylene)-$CH_2$-O-C(=O)-;
-GluGlyGlyPheGly-C(=O)-NH-(optionally substituted phenylene)-$CH_2$-O-C(=O)-;
-LysGlyGlyPheGly-C(=O)-NH-(optionally substituted phenylene)-$CH_2$-O-C(=O)-;
-SerGlyGlyPheGly-C(=O)-NH-(optionally substituted phenylene)-$CH_2$-O-C(=O)-;
-AspAspAspAspAsp-C(=O)-NH-(optionally substituted phenylene)-$CH_2$-O-C(=O)-;
-GlyGlyPheGly-C(=O)-NH-$CH_2$-;
-AspGlyGlyPheGly-C(=O)-NH-$CH_2$-;
(in said amino acid residue, and an amino acid residue in said peptide,

the carboxy group(s) in the side chain(s) of Ama, Asp, and Glu is/are optionally converted into (alkyl) ester(s), (aminoalkyl) ester(s), ((N-alkylamino)alkyl) ester(s), ((N,N-dialkylamino)alkyl) ester(s), ((trialkylammonium)alkyl) ester(s), (lactonyl) ester(s), ((lactonyl)alkyl) ester(s), ((phosphoryl)alkyl) ester(s), or ((bisphosphoryl)alkyl) ester(s), or unsubstituted amide(s), alkylamide(s), dialkylamide(s), ((N-alkylamino)alkyl)amide(s), ((N,N-dialkylamino)alkyl)amide(s), ((trialkylammonium)alkyl)amide(s), (lactonyl)amide(s), ((lactonyl)alkyl)amide(s), (phosphoryl)amide(s), ((phosphoryl)alkyl)amide(s), or ((bisphosphoryl)alkyl)amide(s);
the amino group(s) in the side chain(s) of Lys and Orn optionally has/have one or two alkyl group(s), alkylcarbonyl group(s), (aminoalkyl)carbonyl group(s), ((N-alkylamino)alkyl)carbonyl group(s), ((N,N-dialkylamino)alkyl)carbonyl group(s), ((trialkylammonium)alkyl)carbonyl group(s), lactonyl group(s), (lactonyl)alkyl group(s), (lactonyl)carbonyl group(s), ((lactonyl)alkyl)carbonyl group(s), phosphoryl group(s), (phosphoryl)alkyl group(s), ((phosphoryl)alkyl)carbonyl group(s), (bisphosphoryl)alkyl group(s), or ((bisphosphoryl)alkyl)carbonyl group(s), as substituent(s); and
the hydroxy group in the side chain of Ser and the sulfhydryl group in the side chain of Cys optionally has/have alkyl group(s), alkylcarbonyl group(s), aminoalkyl group(s), (aminoalkyl)carbonyl group(s), (N-alkylamino)alkyl group(s), ((N-alkylamino)alkyl)carbonyl group(s), (N,N-dialkylamino)alkyl group(s), ((N,N-dialkylamino)alkyl)carbonyl group(s), (trialkylammonium)alkyl group(s), ((trialkylammonium)alkyl)carbonyl group(s), lactonyl group(s), (lactonyl)alkyl group(s), (lactonyl)carbonyl group(s), ((lactonyl)alkyl)carbonyl group(s), phosphoryl group(s), (phosphoryl)alkyl group(s), ((phosphoryl)alkyl)carbonyl group(s), (bisphosphoryl)alkyl group(s), or ((bisphosphoryl)alkyl)carbonyl group(s), as substituent(s)); and
1,2,3-triazolylene groups represented by the following formulae:

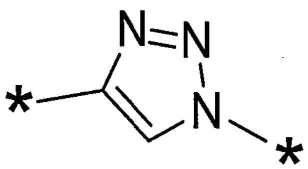

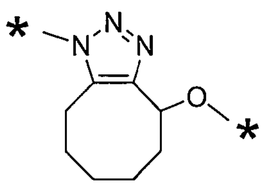

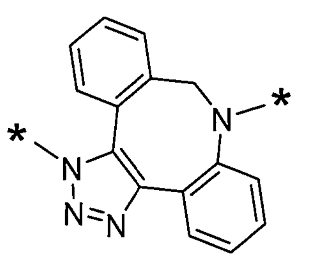

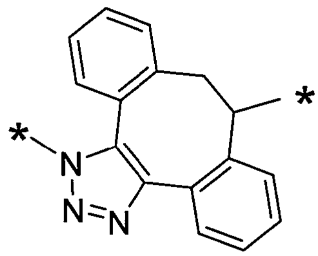

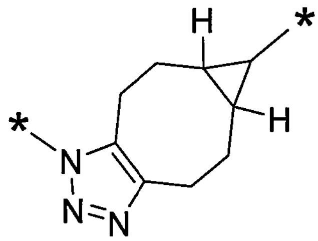

[wherein * is a point of attachment to an adjacent group].

[Aspect C3]

The conjugate precursor (I) according to [Aspect C1] or a salt thereof, wherein one or more structure(s) selected from the followings is/are contained in L:

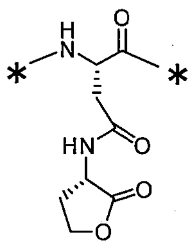

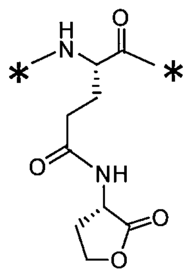

[wherein * is a point of attachment to an adjacent group].

[Aspect C4]

The conjugate precursor (I) according to [Aspect C1] or a salt thereof, wherein one or more structure(s) selected from the followings is/are contained in L:

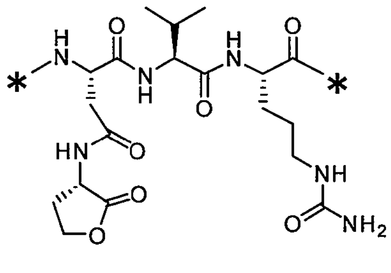

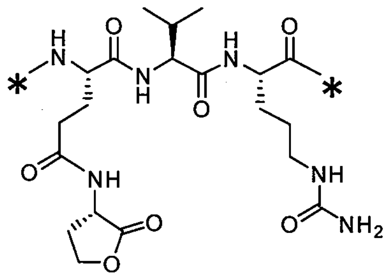

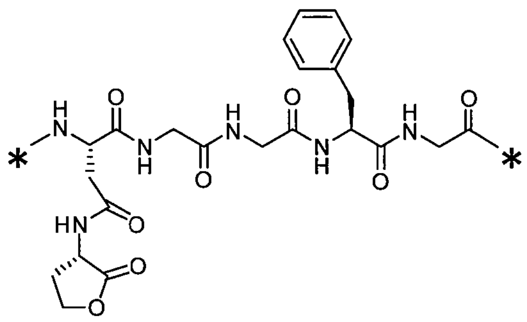

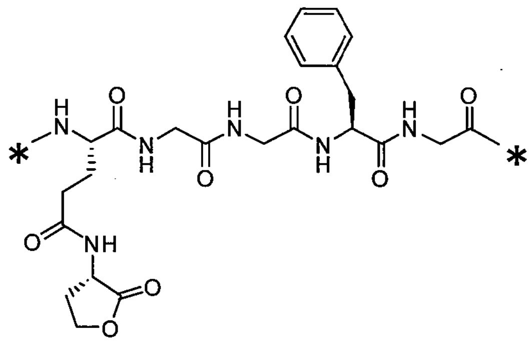

[wherein * is a point of attachment to an adjacent group].

[Aspect C5]

The conjugate precursor (I) according to [Aspect C3] or [Aspect C4] or a salt thereof, wherein one or more structure(s) selected from the followings is/are further contained in L:

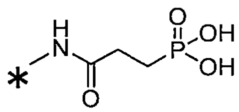

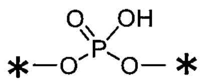

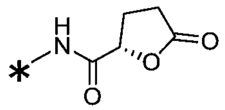

[wherein * is a point of attachment to an adjacent group].

[Aspect C6]

The conjugate precursor (I) according to [Aspect C1] or a salt thereof, wherein L is any one divalent group selected from the following formula group:

formula group

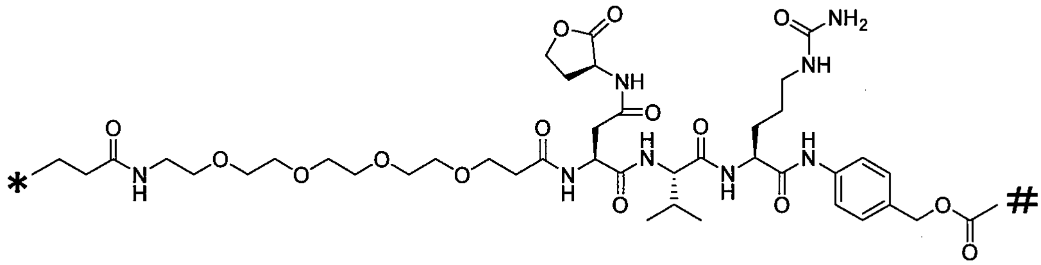

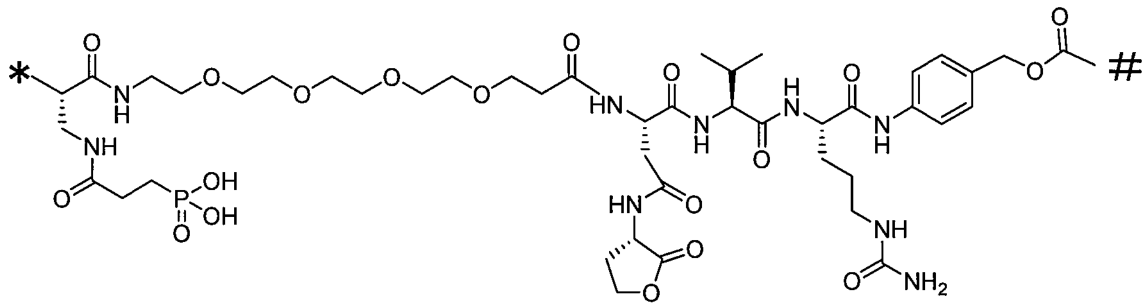

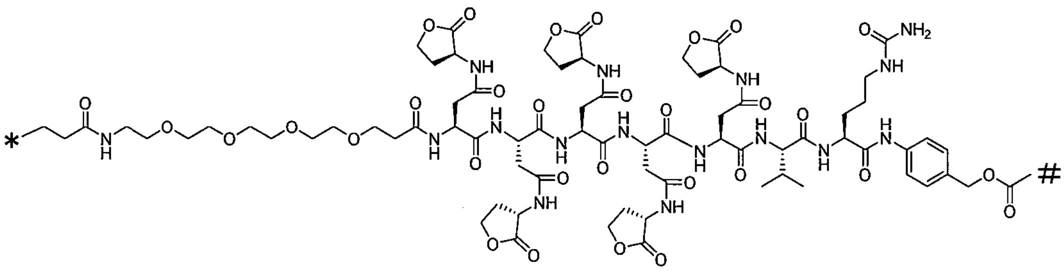

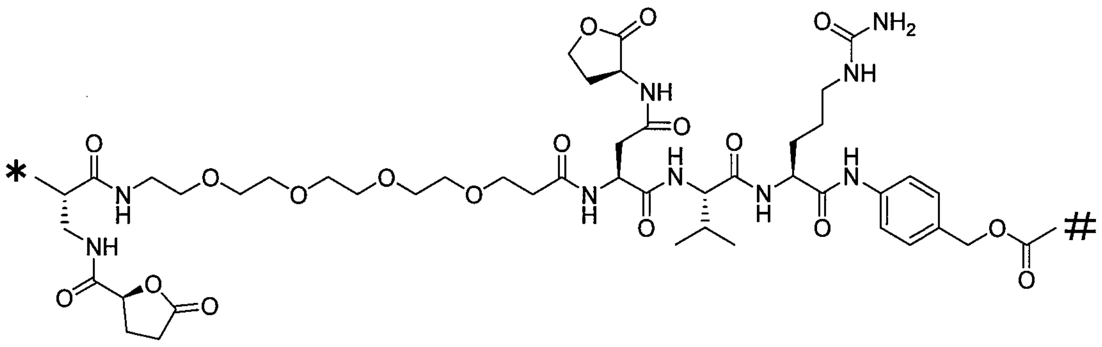

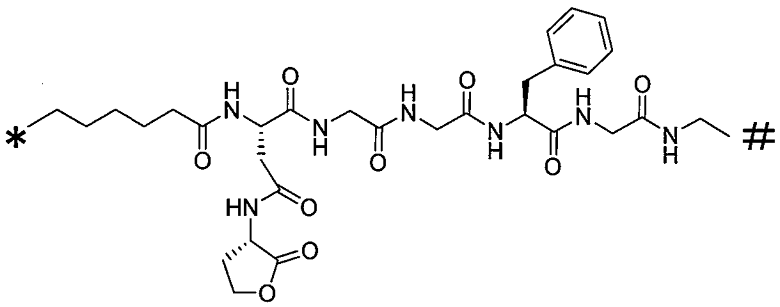

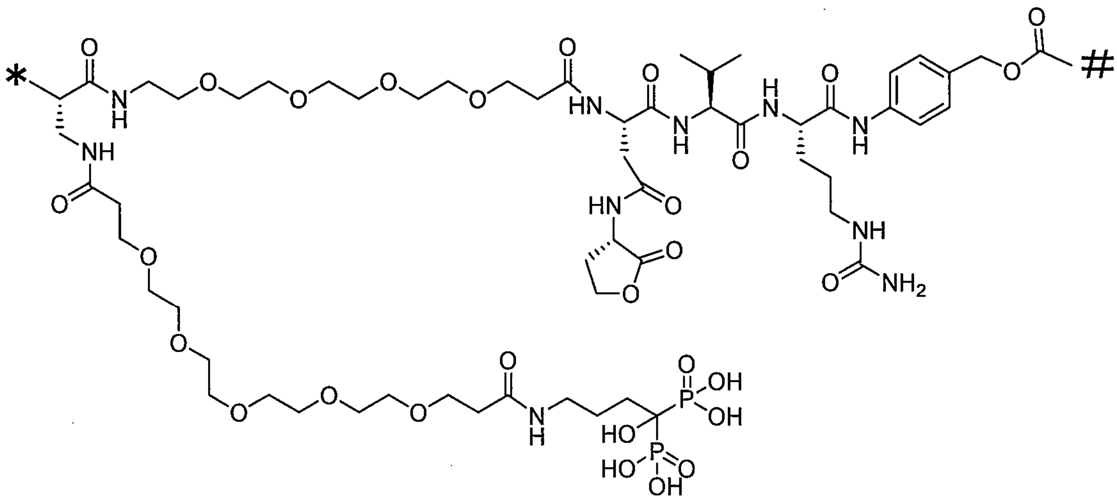

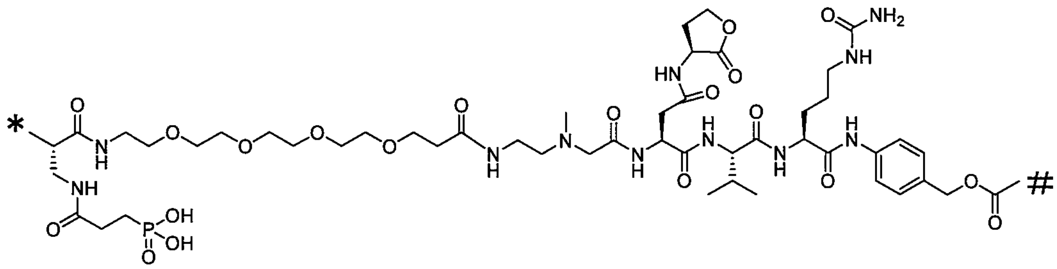

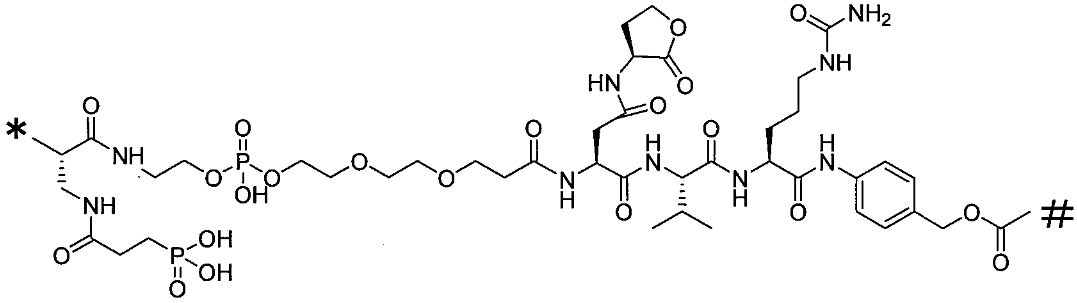

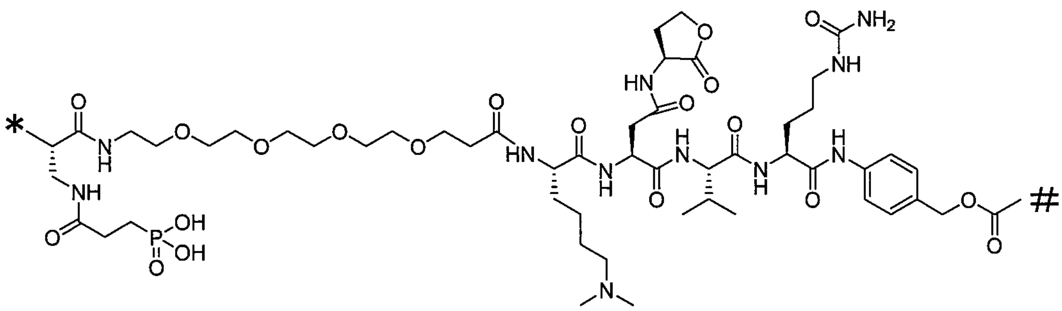

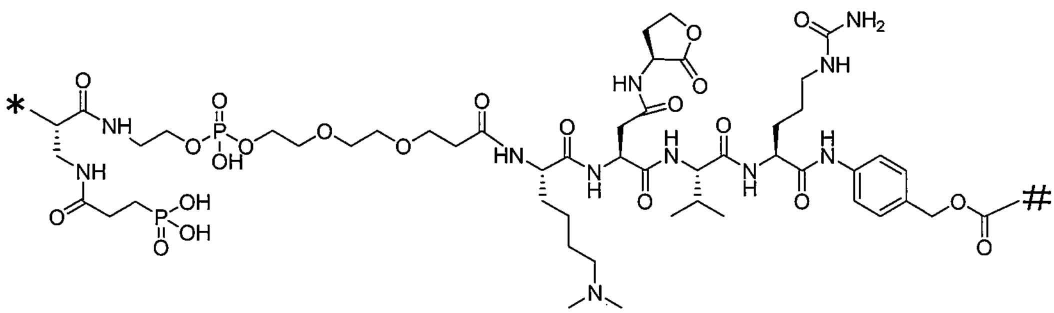

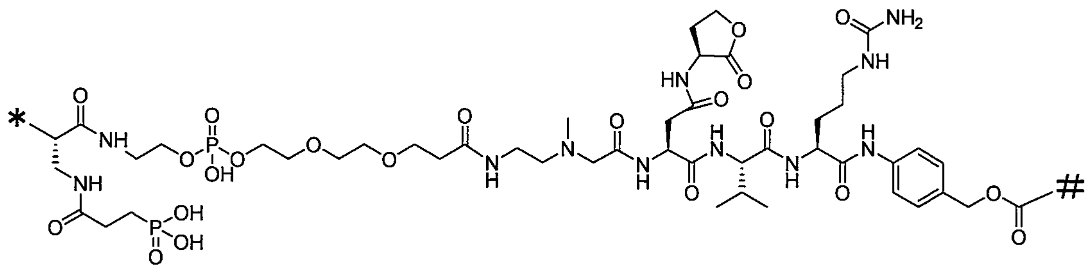

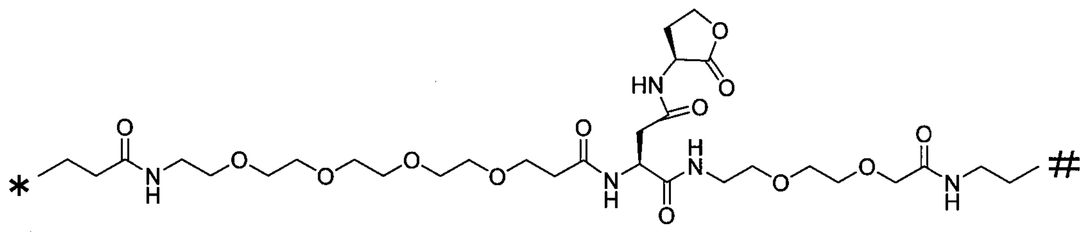

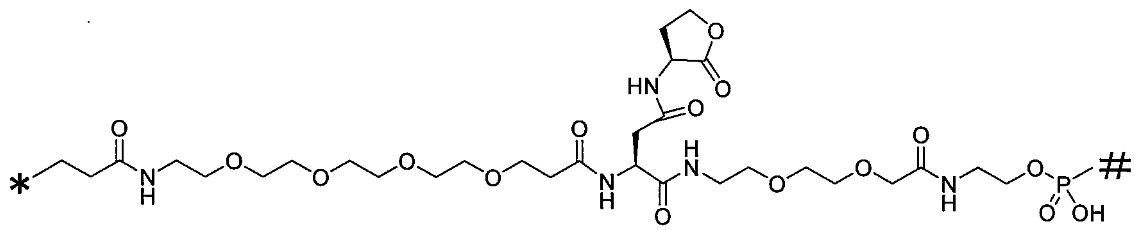

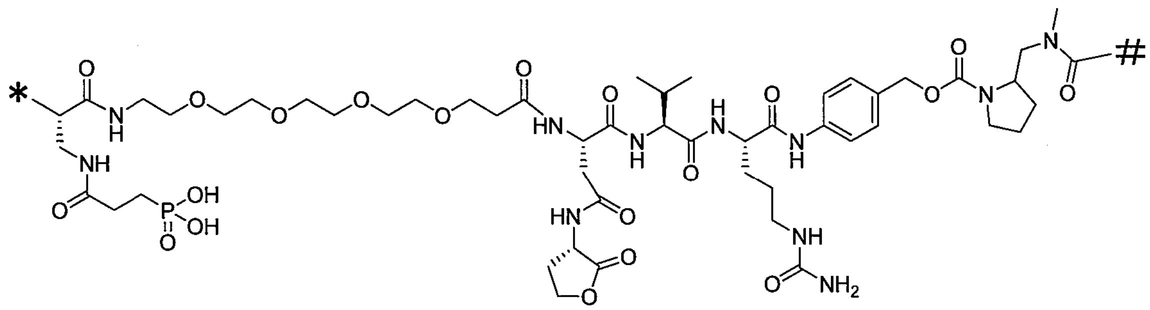

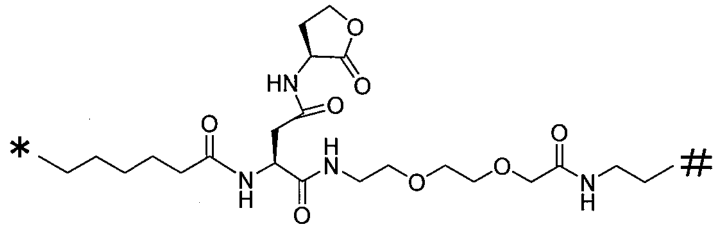

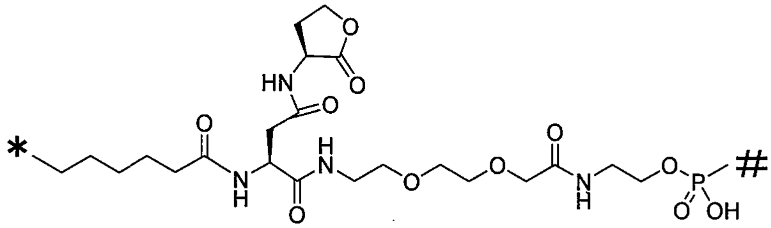

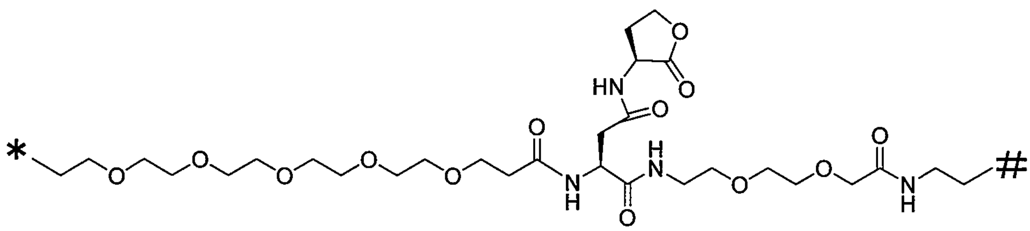

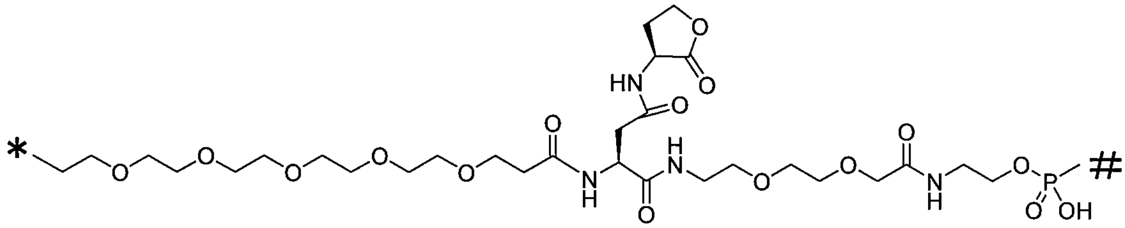

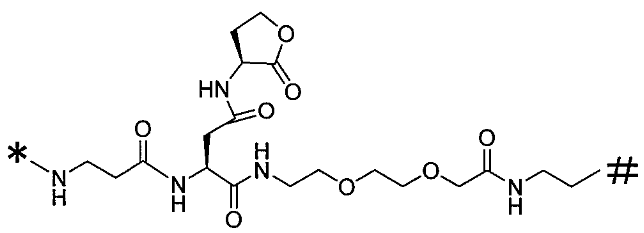

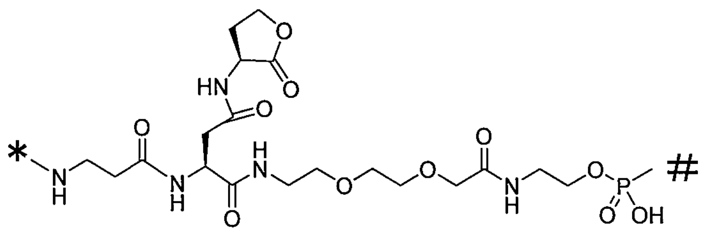

[wherein:

* is a point of attachment to the reactive group Z; and
# is a point of attachment to the antitumor drug residue D].

[Aspect C7]

The conjugate precursor (I) according to any one of [Aspect A1] to [Aspect A4], [Aspect B1] to [Aspect B5], and [Aspect C1] to [Aspect C6] or a salt thereof, wherein

L has one or more group(s) selected from a lactonyl group, a phosphoryl group (-P(=O)$(OH)_2$); a phosphorylalkylcarbonyl group (-C(=O)-alkylene-P(=O)$(OH)_2$); a bisphosphorylmethyl group (-C(H, OH, $NH_2$, Cl, or alkyl)(P(=O)$(OH)_2)_2$); an amino group; a monoalkylamino group; a dialkylamino group; a cyclic amino group formed by combining two alkyl groups of said dialkylamino group with the nitrogen atom to which they are attached; and a trialkylammonium group, at any position(s) as substituent(s) or protecting group(s),
wherein when the total number of said group(s) is two or more, they are identical to or different from each other.

[Aspect D1]

The synthetic intermediate (II) of conjugate precursor or a salt thereof, wherein in the above general formula (II) :

$Z^1$-$L^1$-$D^1$ (II)

$Z^1$ is the jointing group, wherein said jointing group is a hydroxy group, a nitro group, a cyano group, an amide group, an oxo group, an azide group, an amino group, a monoalkylamino group optionally having substituent(s), an imino group (-N=, also including an imino group in a cyclic amine), a carboxy group, a phosphoryl group, an alkynyl group, a cycloalkynyl group, an azacycloalkynyl group, or a sulfhydryl group; or a protected form thereof.

[Aspect D2]

The synthetic intermediate (II) of conjugate precursor according to [Aspect D1] or a salt thereof, wherein
$Z^1$ is the jointing group, wherein said jointing group is a hydroxy group, a nitro group, a cyano group, an oxo group, an azide group, an amino group, a monoalkylamino group optionally having substituent(s), an imino group (-N=, also including an imino group in a cyclic amine), a carboxy group, a phosphoryl group, an alkynyl group, a cycloalkynyl group, an azacycloalkynyl group, or a sulfhydryl group; or a protected form thereof.

[Aspect D3]

The synthetic intermediate (II) of conjugate precursor according to [Aspect D1] or [Aspect D2] or a salt thereof, wherein
$Z^1$ is the jointing group, wherein said jointing group is a hydroxy group, a nitro group, an oxo group, an azide group, an amino group, a monoalkylamino group optionally having substituent(s), an imino group (-N=, also including an imino group in a cyclic amine), a carboxy group, a phosphoryl group, an alkynyl group, a cycloalkynyl group, an azacycloalkynyl group, or a sulfhydryl group; or a protected form thereof.

[Aspect D4]

The synthetic intermediate (II) of conjugate precursor according to any one of [Aspect D1] to [Aspect D3] or a salt thereof, wherein
$Z^1$ is the jointing group, wherein said jointing group is a hydroxy group, a nitro group, an oxo group, an azide group, an amino group, a monoalkylamino group optionally having substituent(s), an imino group (-N=, also including an imino group in a cyclic amine), a carboxy group, a phosphoryl group, an alkynyl group, a cycloalkynyl group, or an azacycloalkynyl group; or a protected form thereof.

[Aspect E1]

The synthetic intermediate (II) of conjugate precursor according to any one of [Aspect D1] to [Aspect D4] or a salt thereof, wherein

$L^1$ is an optionally substituted alkylene group; and
one or more methylene group(s) in the chain of said alkylene group is/are replaced with one or more group(s) selected from the divalent groups according to the above [Aspect C1] to [Aspect C4].

[Aspect F1]

The synthetic intermediate (II) of conjugate precursor according to any one of [Aspect D1] to [Aspect D4] and [Aspect E1] or a salt thereof, wherein

$Z^1$ is a group selected from the jointing groups according to the above [Aspect D1] to [Aspect D4]; and
$D^1$ is a group selected from the antitumor drug residues according to the above [Aspect B1] to [Aspect B5].

[Aspect F2]

The synthetic intermediate (II) of conjugate precursor according to any one of [Aspect D1] to [Aspect D4] and [Aspect E1] or a salt thereof, wherein
$Z^1$ and $D^1$ are each independently a group selected from the jointing groups according to the above [Aspect D1] to [Aspect D4].

[Aspect F3]

The synthetic intermediate (II) of conjugate precursor according to any one of [Aspect D1] to [Aspect D4] and [Aspect E1] or a salt thereof, wherein

$Z^1$ is a group selected from the reactive groups according to the above [Aspect A1] to [Aspect A4]; and
$D^1$ is a group selected from the jointing groups according to the above [Aspect D1] to [Aspect D4].

[Aspect G1]

The intermediate (II) of conjugate precursor according to any one of [Aspect D1] to [Aspect D4], [Aspect E1], and [Aspect F1] or a salt thereof, wherein

$Z^1$ is a group selected from the jointing groups according to the above [Aspect D1] to [Aspect D4];
$D^1$ is a group selected from the antitumor drug residues according to the above [Aspect B1] to [Aspect B5]; and
at least any one of $D^1$ and $L^1$ has one or more lactonyl group(s) at any position(s) as substituent(s) or protecting group(s), and independently thereof,
at least any one of $D^1$ and $L^1$ has one or more group(s) selected from a phosphoryl group ($-P(=O)(OH)_2$); a phosphorylalkylcarbonyl group ($-C(=O)$-alkylene-$P(=O)(OH)_2$) ; a bisphosphorylmethyl group (-C(H, OH, $NH_2$, Cl, or alkyl)($P(=O)(OH)_2)_2$); an amino group; a monoalkylamino group; a dialkylamino group; a cyclic amino group formed by combining two alkyl groups of said dialkylamino group with the nitrogen atom to which they are attached; and a trialkylammonium group, at any position(s) as substituent(s) or protecting group(s),
wherein when the total number of said group(s) is two or more, they are identical to or different from each other.

[Aspect G2]

The synthetic intermediate (II) of conjugate precursor according to any one of [Aspect D1] to [Aspect D4], [Aspect E1], [Aspect F2], and [Aspect F3] or a salt thereof, wherein

$Z^1$ is a group selected from the reactive groups according to the above [Aspect A1] to [Aspect A4], or a group selected from the jointing groups according to the above [Aspect D1] to [Aspect D4];
$D^1$ is a group selected from the jointing groups according to the above [Aspect D1] to [Aspect D4]; and
$L^1$ has one or more group(s) selected from a lactonyl group; a phosphoryl group ($-P(=O)(OH)_2$); a phosphorylalkylcarbonyl group ($-C(=O)$-alkylene-$P(=O)(OH)_2$) ; a bisphosphorylmethyl group (-C(H, OH, $NH_2$, Cl, or alkyl)($P(=O)(OH)_2)_2$); an amino group; a monoalkylamino group; a dialkylamino group; a cyclic amino group formed by combining two alkyl groups of said dialkylamino group with the nitrogen atom to which they are attached; and a trialkylammonium group, at any position(s) as substituent(s) or protecting group(s),
wherein when the total number of said group(s) is two or more, they are identical to or different from each other.

In the divalent group described above, if a part of L and $L^1$ of the general formula (I) and (II) respectively is left-right asymmetric or can be left-right asymmetric, said divalent group may face either the left, right, top, or bottom direction on the paper surface. For example, when - $N(R^3)$-$C(R^1)(R^2)$- exists as a part of L, -$N(R^3)$- may be in the B side and -$C(R^1)(R^2)$- may be in the D side, or - $C(R^1)(R^2)$- may be in the B side and -$N(R^3)$- may be in the D side.

Further, "three-letter alphabet" is a symbol for indicating the following amino acid, and especially in the present description, it indicates the divalent residue of each amino acid (-NH-CH ($R^{15}$)-C(=O)-) . In said formula, $R^{15}$ is a hydrogen atom (Gly), a methyl group (Ala), an isopropyl group (Val), an isobutyl group (Leu), a (butan-2-yl) group (Ile), a phenylmethyl group (Phe), a hydroxymethyl group (Ser), a sulfhydrylmethyl group (Cys), a carboxymethyl group (Asp), a 2-carboxyethyl group (Glu), a 3-aminopropyl group (Orn), a 4-aminobutyl group (Lys), a 3-(ureido)propyl group (Cit), a 3-guanidinopropyl group (Arg), or a (1H-imidazol-4-yl)methyl group (His) (wherein each amino acid in parentheses indicates the corresponding amino acid). Also, in the above formula (-NH-CH($R^{15}$)-C(=O)-), when $R^{15}$ is a propyl group and said propyl group is combined with the nitrogen atom of "-NH-" in the formula to form a 5-membered ring, then a cyclic amino acid (Pro; proline) is formed.

Gly; glycine
Ala; alanine
Val; valine
Leu; leucin
Ile; isoleucine
Phe; phenylalanine
Ser; serine
Cys; cysteine
Asp; aspartic acid
Glu; glutamic acid
Orn; ornithine
Lys; lysine
Cit; citrulline
Arg; arginine
His; histidine
Pro; proline

Further, when the "-L-D" moiety and the "-$L^1$-$D^1$" moiety of the conjugate precursor represented by the general formula (I) and the synthetic intermediate of conjugate precursor represented by the general formula (II) respectively of the present invention comprise amino acid residue(s) having asymmetric center(s) and optical isomer(s) may exist, both D-type and L-type optical isomers are encompassed by the present invention.

## EFFECT OF INVENTION

The antibody-drug conjugate produced by reacting the conjugate precursor (I) or a salt thereof of the present invention with an antibody has been confirmed that it is selectively delivered to a target tumor cell after administered to a living body and releases an antitumor drug in said cell, and thus can become a cancer therapeutic drug having excellent antitumor effects and safety. Also, it has been confirmed that the synthetic intermediate (II) of conjugate precursor or a salt thereof of the present invention is useful for synthesizing said conjugate precursor (I).

## BRIEF DESCRIPTION OF DRAWINGS

[Figure 1] Figure 1 is a graph showing the tumor volume inhibition rate of a compound of ADC Production example 2 (1 mg/kg) in the Test Example 4.
[Figure 2] Figure 2 is a graph showing the tumor volume inhibition rate of a compound of ADC Production example 3 (1 mg/kg) in the Test Example 4.
[Figure 3] Figure 3 is a graph showing the tumor volume inhibition rate of a compound of ADC Production example 15 (1 mg/kg) in the Test Example 4.
[Figure 4] Figure 4 is a graph showing the tumor volume inhibition rate of a compound of ADC Production example 19 (1 mg/kg) in the Test Example 4.
[Figure 5] Figure 5 is a chart showing the SEC analysis HPLC of ADC Production example 2 in the Test Example 8.

## MODE FOR CARRYING OUT THE INVENTION

Hereinafter, the definitions of terms used herein are described and each of them is specifically illustrated, but the present invention is not limited to them. Also, various compounds (starting materials, intermediates, and the like), drug

molecules, substituents, protecting groups, and the like defined or illustrated below are optionally selected and combined to prepare the conjugate precursors (I) or salts thereof and the synthetic intermediates (II) of conjugate precursor or salts thereof of the present invention.

Unless otherwise specified, the following terms and phrases as described herein have the following meanings.

The term of "tumor" as described herein means "malignant tumor", and includes "carcinoma", "blood cancer", "cancer" "sarcoma" "brain tumor", and the like.

The term of "antibody" as described herein refers to intact monoclonal antibody, polyclonal antibody, monospecific antibody, multispecific antibody (for example, bispecific antibody), heavy chain antibody, and antibody fragment. The "monoclonal antibody" described above is an antibody obtained from a substantially homogeneous group of antibodies, and the individual antibodies included in the group are identical except for the possibility of naturally occurring mutation(s) that may be present slightly. The monoclonal antibodies are highly specific and target a single antigenic site. The "polyclonal antibody" described above is a heterogeneous group of antibody molecules derived from the serum of an immunized animal. The "monospecific antibody" described above is an antibody that exhibits binding specificity to a single antigen. The "multispecific antibody" described above is an antibody that exhibits binding specificity to two or more different antigens (two in the case of bispecific antibodies). The bispecific antibody may be prepared by the method disclosed in Journal of Hematology & Oncology 8, 130 (2015), Frontiers in Immunology 8, 38 (2017), BioDrugs 24 (2), 89-98 (2010), MAbs 1 (6), 539-547 (2009), Journal of Immunology 155 (1), 219-25 (1995), and the like. The heavy chain antibody described above is an antibody composed only of two heavy chains having no light chain.

The "antibody fragment" described above includes an antigen-binding region or a variable region and includes a part of an intact antibody. In order to be used in the present invention, it is necessary for the antibody fragment to have a functional group (for example, a sulfhydryl group, an amino group, etc.) or a disulfide bond that can generate a sulfhydryl group by reduction, so that it can be bound to the drug moiety via a linker. The "antigen" described above is a substance to which the antibody specifically binds.

The "antibody" used in the present invention can be obtained by known means, for example, animals such as rats, mice and rabbits are immunized with various antigens, and antibodies produced in vivo can be prepared by selecting and purifying them according to various methods. Human monoclonal antibodies can be prepared according to known methods (for example, Immunology Today, Vol. 4 72-79 (1983), etc.). When the antibodies are derived from a species other than human, it is preferable to chimerize or humanize them using well-known techniques.

The "antibody" used in the present invention can also be obtained by means such as purchasing it as a commercially available reagent, extracting and purifying it from an antibody pharmaceutical formulation, or producing it according to a known method. The nucleotide sequence of the antibody required for the production of the antibody is obtained, for example, from databases such as the GenBank database or literatures. It can also be obtained by cloning and sequencing by known methods.

The "antibody" used in the present invention is an immunoglobulin, which is a molecule containing an antigen binding site that immunospecifically binds to an antigen. Said antibody may be any class of IgG, IgE, IgM, IgD, IgA, and IgY, and IgG is preferable. The subclass may be any of IgG1, IgG2, IgG3, IgG4, IgA1, and IgA2, and IgG1, IgG2, and IgG4 are preferable. These antibodies may be polyclonal antibodies or monoclonal antibodies, and monoclonal antibodies are preferable.

The antibody used in the present invention may be an antibody which can target tumor cells. In the antibody-drug conjugate of the present invention, since the antibody binds to the antitumor active drug(s) via linker(s), it is preferable that the antibody has any one or more property(ies) of a property which can recognize tumor cells, a property which can specifically bind to tumor cells, a property which are incorporated into tumor cells and internalized therein, and a property which injures tumor cells. The phrase of "specifically bind" means that an antibody or antibody derivative binds to the corresponding target antigen in a very selective manner and does not bind to other antigens present in large numbers, and the binding can be confirmed using flow cytometry. The phrase of "antibody is internalized" means that the antibody binds to an antigen on the cell membrane surface and is incorporated into the cell by endocytosis, and the incorporation can be confirmed by using a secondary antibody (fluorescent label) that binds to said antibody, and then visualizing the antibody incorporated into the cell with fluorescence microscopy (Cell Death and Differentiation 15, 751-761 (2008)) or measuring the fluorescence amount (Molecular Biology of the Cell 15, 5268-5282 (2004)), or the Mab-ZAP method (BioTechniques 28, 162-165 (2000)) in which an immunotoxin that binds to said antibody is used and suppresses cell growth by the release of a toxin when incorporated into the cell, and the like.

The phrase of "injure(s) tumor cell(s)" means that the antibody kills the cells, and its cytotoxic activity can be confirmed by measuring the cell growth inhibitory activity in vitro.

The "antibody" as described herein also includes a modified antibody. A modified antibody means one in which the antibody has been chemically or biologically modified. Examples of the chemical modifications include chemical modifications of the amino acid backbone moiety of the antibody and chemical modifications to N-linked or O-linked sugar chain. Examples of the biological modifications include post-translational modifications (for example, glycosylation to an oxygen atom or a nitrogen atom, N-terminal or C-terminal processing, deamidation, aspartic acid isomerization, and

methionine oxidation), and the addition of a methionine residue to the N-terminus by the expression using prokaryotic host cells.

Said chemically or biologically modified antibody also includes those in which an auxiliary linker is extended from a functional group (for example, a hydroxy group, a sulfhydryl group, an amino group, or a carboxy group) present in the original antibody, and "a sulfhydryl group, a hydroxy group, an amino group, a maleimidyl group (the above formula (i)), an α-halogenomethylcarbonyl group, an ethynylphosphonamidate group (the above formula (ii)), a carboxy group, an active ester of carboxy group (for example, the above formula (iii)), an azide group ($-N_3$ group), an alkynyl group, a cycloalkynyl group, an azacycloalkynyl group, or the like" is added to the terminal of said linker. In the present description, the above defined "antibody" and the above defined "modified antibody" are optionally collectively referred to as "antibody". Said "auxiliary linker" is a part of the linkers "-L-" and "$-L^1-$" in the above general formulae (I) and (II).

Specific examples of the antibody used in the reaction with the conjugate precursor (I) of the present invention include the followings, but the present invention is not limited to them.

an anti-HER2 antibody, an anti-EGFR antibody, an antiLymphocyte Antigen 6E (Ly-6E) antibody, an anti-Hepatocyte Growth Factor Receptor (HGFR) antibody, an anti-CD22 antibody, an anti-Folate Receptor alpha (FRα) antibody, an anti-PSMA (FOLH1) antibody, an anti-CD30 (TNFRSF8) antibody, an anti-TROP-2 antibody, an anti-CD19 antibody, an anti-5T4 antibody, an anti-Mesothelin antibody, an anti-CD20 antibody, an anti-CD33 antibody, an anti-CDB7-H3 antibody, an anti-CD269 (BCMA) antibody, an anti-CD142 antibody, an anti-CD70 antibody, an anti-CD123 antibody, an anti-TAG-72 antibody, an anti-TFRC antibody, an anti-EPHA2 antibody, an anti-EPHA3 antibody, an anti-c-KIT antibody, an anti-PD-L1 antibody, an anti-Epithelial Cell Adhesion Molecule (EPCAM) antibody, an anti-AXL antibody, an anti-Interleukin-1 Receptor Accessory Protein antibody, an anti-Mucin-16 (CA125) antibody, an anti-MUC1 antibody, an anti-GPC3 antibody, an anti-CLEC12A (CLL1) antibody, an anti-LRRC15 antibody, an anti-ADAM9 antibody, an anti-Sodium-Dependent Phosphate Transport Protein 2B (SLC34A2) antibody, an anti-CD25 antibody, an anti-ROR1 antibody, an anti-TweakR antibody, an anti-CD74 antibody, an anti-CEACAM5 antibody, an anti-CD105 antibody, an anti-CD79b antibody, an anti-CD147 antibody, an anti-Sialyl-Tn antibody, an anti-GCC antibody, an anti-HER3 antibody, an anti-Integrin antibody, an anti-EGFR variant III antibody, an anti-Claudin 6 antibody, an anti-Leucine-Rich Repeat-Containing G-Protein Coupled Receptor 5 antibody, an anti-PTK7 antibody, an anti-LYPD3 antibody, an anti-ASCT2 antibody, an anti-ASPH antibody, an anti-GPC1 antibody, an anti-CD174 antibody, an anti-CD38 antibody, an anti-ANGPT2 antibody, an anti-CD44 antibody, an anti-DDL3 antibody, an anti-FGFR2 antibody, an anti-KAAG1 antibody, an anti-CD73 antibody, an anti-CLDN18.2 antibody, an anti-PRLR antibody, an anti-SEZ6 antibody, an anti-Neprilysin antibody, an anti-Neural Cell Adhesion Molecule 1 antibody, an anti-CD166 (ALCAM) antibody, an anti-CD24 antibody, an anti-CD27 antibody, an anti-CDH3 antibody, an anti-EPHA4 antibody, an anti-FGFR3 antibody, an anti-GFRAL antibody, an anti-Globo H antibody, an anti-IL-13Ra2 antibody, an anti-STEAP-1 antibody, an anti-TMCC3 antibody, an anti-CD209 antibody, an anti-CD37, an anti-CD48 antibody, an anti-CDCP1 antibody, an anti-CD46 antibody, an anti-CD200 antibody, an anti-CTLA4 antibody, an anti-CXCR5 antibody, an anti-FLT3 antibody, an anti-SDC1 (CD138) antibody, an anti-CLDN6/9 antibody, an anti-CEACAM6 antibody, an anti-CA9 antibody, an anti-EDNRB antibody, an anti-GD3 Ganglioside antibody, an anti-MICA/B antibody, an anti-JAG1/2 antibody, an anti-TM4SF1 antibody, an anti-uPAR antibody, an anti-Carbonic anhydrase IX antibody, an anti-ALK1 antibody, an anti-IGF-1R antibody, an anti-KDR antibody, an anti-CEACAM8 antibody, an anti-IL5R antibody, an anti-CD205 (Ly75) antibody, an anti-MSR1 antibody, an anti-KREMEN2 antibody, an anti-SSEA-4 antibody, an anti-CD228 antibody, an anti-CD5 antibody, an anti-DLL4 antibody, an anti-FAP antibody, an anti-Notch3 antibody, an anti-AG7 antibody, an anti-Guanylate Cyclases antibody, an anti-Nectin-4 antibody, an anti-CD226 (DNAM-1) antibody, an anti-FcRL5 antibody, an anti-HLA-DR antibody, an anti-ITGB3 antibody, an anti-DLK1 antibody, an anti-CD157 (BST1) antibody, an anti-CD56 (NCAM1) antibody, an anti-MICA (MHC class I Polypeptide-Related Sequence A) antibody, an anti-SSEA-1 antibody, an anti-TRAIL-R2 (DR5) antibody, an anti-GPNMB antibody, an anti-CCR5 antibody, an anti-LAMP1 antibody, an anti-LGALS3BP antibody, an anti-ROR2 antibody, an anti-DLL3 antibody, an anti-ETBR antibody, an anti-LIV-1 antibody, an anti-Integrin αvβ6 antibody, an anti-TIM-1 antibody, an anti-AGS-16 (ENPP3) antibody, an anti-SLITRK6 antibody, an anti-GD2 antibody, an anti-CD52 antibody, an anti-CCR4 antibody, an anti-VEGFR2 antibody, an anti-PDGFR antibody, an anti-FGFR antibody, an anti-SLAMF7 antibody, an anti-GD2 Ganglioside antibody, an anti-EPHA3 antibody, an anti-Integrin αvβ3 antibody, an anti-AGS-5 antibody, an anti-CA19-9 antibody, an anti-PSA antibody, an anti-MAGE3 antibody, an anti-Transferrin receptor 1 (CD71) antibody, an anti-CEACAM1 antibody, an anti-SLC3A2 (CD98) antibody, an anti-ACVR1 antibody, an anti-AG-7 antibody, an anti-AMHR2 antibody, an anti-ABCB1 antibody, an anti-C16orf54 antibody, an anti-CathepsinD antibody, an anti-CCR7 antibody, an anti-SLC44A4 antibody, an anti-CD300LF antibody, an anti-DPEP3 antibody, an anti-FucGM1 antibody, an anti-GPR20 antibody, an anti-ITGB6 antibody, an anti-Lewis-A-like carbohydrate antibody, an anti-prolactin receptor antibody, an anti-Sialyl Tn antibody, an anti-SLAMF6 antibody, an anti-SLAMF7 antibody, an anti-TRA-1-60 antibody, an anti-matriptase antibody, an anti-B7-H4 antibody, an anti-Cripto antibody, an anti-CD99 antibody, an anti-CanAg antibody, an anti-A33 antibody, an anti-α10β1 integrin antibody, an anti-ALPP antibody, an anti-CD248 antibody, an anti-GPRC5D antibody.

The term of "functional group in antibody" as described herein refers to a group present in the above defined

"antibody" and "modified antibody", and said group reacts with a reactive group present in the conjugate precursor (I) of the present invention to produce an antibody-drug conjugate. Specific examples thereof include a sulfhydryl group, a hydroxy group, an amino group, a maleimidyl group (the above formula (i)), an α-halogenomethylcarbonyl group (the above formula (ii)), an ethynylphosphonamidate group (the above formula (iii)), a carboxy group, an active ester of carboxy group, an azide group (-$N_3$ group), an alkynyl group, and a cycloalkynyl group.

The term of "reactive group" as described herein refers to a group present in the conjugate precursor (I) or the intermediate (II) of conjugate precursor of the present invention, and said group reacts with the above defined "functional group in antibody" to produce an antibody-drug conjugate. Specific examples thereof include a maleimidyl group (the above formula (i)), an α-halogenomethylcarbonyl group (the above formula (ii)), an ethynylphosphonamidate group (the above formula (iii)), a carboxy group, an active ester of carboxy group, a sulfhydryl group, a hydroxy group, an amino group, an alkynyl group, a cycloalkynyl group, or an azide group (-$N_3$ group).

The term of "jointing group" as described herein refers to a group which is present in the terminal of the linker component of the synthetic intermediate (II) of conjugate precursor of the present invention, and joints the above "reactive group" or the below-described "antitumor drug residue" to the linker component by etherification reaction, thioetherification reaction, carboxy esterification reaction, (mono- or di-) phosphorylation reaction, amidation reaction, reductive amination reaction, disulfidation reaction, Click reaction, or the like. Specific examples thereof include a hydroxy group, a nitro group, a cyano group, an amide group, an oxo group, an azide group, an amino group, a monoalkylamino group optionally having substituent(s), an imino group (-N=, also including an imino group in a cyclic amine), a carboxy group, a phosphoryl group, an alkynyl group, a cycloalkynyl group, or a sulfhydryl group. In said examples of group, the nitro group can be a precursor of an amino group, the cyano group can be a precursor of an amino group and an amide group, and the amide group can be a precursor of an amino group and a carboxy group, and thus these groups are also encompassed by the "jointing group" in the present description.

The term of "antitumor drug molecule" as described herein refers to a drug molecule having an antitumor effect regardless of the action mechanism, and is not specifically limited as long as it has a substituent or a partial structure which can bind to the linker (L in the conjugate precursor (I) and $L^1$ in the synthetic intermediate (II) of conjugate precursor). The antitumor drug molecule is released in a tumor cell by the cleavage of a part or the whole of the linker to exert the antitumor effect.

Further, the term of "antitumor drug residue" as described herein refers to a residue in which one hydrogen atom or one hydroxy group is removed from any position of said "an antitumor drug molecule or an analog thereof or a derivative thereof".

Examples of said antitumor drug molecule include camptothecin; MMAE; maytansine; PBD (parabenzodiazepine) dimer; eribulin; 5-FU; PD-318088; AS-703026; TAK-733; LY-3023414; calicheamicin; paclitaxel; docetaxel; mitomycin C; bleomycin; cyclocytidine; vincristine; vinblastine; daunomycin; doxorubicin; dolastatin 10; superdox; ciprofloxacin; cadrofloxacin (CS-940); or an analog of said antitumor drug; or a derivative of said antitumor drug molecule or an analog thereof.

The term of "analog" in "an antitumor drug molecule or an analog thereof, or a derivative thereof" as described herein means a compound having a similar chemical structure and activity to each antitumor drug molecule. For example, the term of "analog of camptothecin" means a compound having a chemical structure similar to camptothecin, and having a type I topoisomerase inhibitory action like camptothecin.

The term of "derivative" in "an antitumor drug molecule or an analog thereof, or a derivative thereof" as described herein refers to a derivative in which a functional group present in said antitumor drug molecule such as a hydroxy group, a sulfhydryl group (-SH), an amino group (-$NH_2$ and -NH- (also including -NH- in an amide group and -NH- in the ring of a heteroaryl group or a heterocyclyl group)), and a carboxy group is protected by a "protecting group", or a glycoside derivative produced by reacting said functional group with a saccharide. The protecting group of said derivative may be deprotected in vivo by various chemical reactions, biochemical reactions, or metabolic reactions (for example, pH fluctuations, hydrolysis reactions, and redox reactions), converted to a mother compound (original antitumor drug molecule), and exhibit antitumor activity in some cases, or may not be deprotected and exhibit antitumor activity in its intact form in other cases.

Said "protecting group" is optionally selected from the protecting groups disclosed in, for example, Greene's Protective Groups in Organic Synthesis 5th Edition, P. G. M. Wuts, JohnWiley & Sons Inc. (2014) and the like.

Examples of the protecting group of hydroxy group, which is a functional group, include alkyloxyalkyl groups such as a methyloxymethyl group, a methoxyethyloxymethyl group, and an ethoxy-2-ethyl group; arylalkyloxymethyl groups such as a benzyloxymethyl group; aryloxymethyl groups such as a phenyloxymethyl group; alkylthiomethyl groups such as a methylthiomethyl group; arylalkylthiomethyl groups such as a benzylthiomethyl group; arylthiomethyl groups such as a phenylthiomethyl group; aminomethyl groups in which the N atom is optionally protected by an alkyl group or a below-described "protecting group of amino group"; arylmethyl groups such as a benzyl group, a 4-methoxybenzyl group, and a triphenylmethyl group; alkylcarbonyl groups such as a methylcarbonyl group and an ethylcarbonyl group; alkenylcarbonyl groups such as an allylcarbonyl group; alkynylcarbonyl groups such as a propargylcarbonyl group: arylcarbonyl groups

such as a phenylcarbonyl group; heteroarylcarbonyl groups such as a pyridine-2, 3, or 4-ylcarbonyl group; heterocyclylcarbonyl groups such as a tetrahydrofuran-2 or 3-ylcarbonyl group and a 5-oxotetrahydrofuran-2-ylcarbonyl group; alkyloxycarbonyl groups such as a tert-butyloxycarbonyl group, a methyloxycarbonyl group, and an ethyloxycarbonyl group; an allyloxycarbonyl group; aryloxycarbonyl groups such as a phenyloxycarbonyl group, a 4-nitrophenyloxycarbonyl group, and a 4-methoxyphenyloxycarbonyl group; arylmethyloxycarbonyl groups such as a 9-fluorenylmethyloxycarbonyl group, a benzyloxycarbonyl group, a 4-methoxybenzyloxycarbonyl group, a 4 (or 2)-nitrobenzyloxycarbonyl group, a 4 (or 2)-aminobenzyloxycarbonyl group or an N-alkylcarbonylated or N-arylcarbonylated derivative thereof, a 4 (or 2)-hydroxybenzyloxycarbonyl group or an O-alkylcarbonylated or O-arylcarbonylated derivative thereof; alkylaminocarbonyl groups such as a tert-butylaminocarbonyl group, a methylaminocarbonyl group, and an ethylaminocarbonyl group; silyl groups such as a trimethylsilyl group, a trimethylsilylethoxymethyl group, a triisopropylsilyl group, a tert-butyldimethylsilyl group, and a tert-butyldiphenylsilyl group; tetrahydropyranyl groups such as a tetrahydropyranyl group and a 2 (or 4)-methoxytetrahydropyranyl group. Also, said hydroxy group is optionally protected as an ester with formic acid, boric acid, phosphoric acid, phosphonic acid, phosphinic acid, sulfuric acid, sulfonic acid, sulfinic acid, sulfenic acid, an amino acid, or a peptide.

In one embodiment, the protecting group of hydroxy group is an alkyloxyalkyl group; an arylalkyloxymethyl group; aminomethyl groups in which the N atom is optionally protected by an alkyl group or a below-described "protecting group of amino group"; an alkylcarbonyl group; an alkenylcarbonyl group; an alkynylcarbonyl group: an arylcarbonyl group; an alkyloxycarbonyl group; an allyloxycarbonyl group; an aryloxycarbonyl group; an arylmethyloxycarbonyl group; an alkylaminocarbonyl group; a tetrahydropyranyl group; a tetrahydrofuranyl group; a silyl group, a lactonyl group; a lactonylalkyl group; a lactonylcarbonyl group; a lactonylalkylcarbonyl group; a phosphoryl group; a phosphorylalkyl group; or a phosphorylalkylcarbonyl group.

Examples of the protecting group of sulfhydryl group, which is a functional group, include the above protecting groups listed as protecting groups of hydroxy group, and in addition to them, a disulfide bond (-S-S-) may be a protecting group.

Examples of the protecting group of carboxy group, which is a functional group, include alkyl groups such as a methyl group, an ethyl group, and a tert-butyl group; an allyl group; arylmethyl groups such as a benzyl group; a lactonyl group; silyl groups such as a trimethylsilyl group, a trimethylsilylethoxymethyl group, a tert-butyldimethylsilyl group, and a tert-butyldiphenylsilyl group. Also, a carboxy group is also optionally protected as an amide (for example, an amide with ammonia, alkylamine, dialkylamine, lactonylamine, an amino acid ester, an amino acid amide, or the like), wherein the amino group of said amide moiety optionally has lactonyl group(s), lactonylalkyl group(s), or phosphorylalkyl group(s) as substituent(s).

In one embodiment, the protecting group of carboxy group is an alkyl group; an allyl group; an arylmethyl group; a lactonyl group; a lactonylalkyl group; or a phosphorylalkyl group.

The functional groups, a sulfenic acid group, a sulfinic acid group, a sulfonic acid group, a phosphinic acid group, a phosphonic acid group, and a boric acid group can also use the same protecting groups as those of the above carboxy group.

Examples of the protecting group of amino group, which is a functional group, include alkyloxycarbonyl groups such as a tert-butyloxycarbonyl group, a methyloxycarbonyl group, and an ethyloxycarbonyl group; an allyloxycarbonyl group; aryloxycarbonyl groups such as a phenyloxycarbonyl group, a 4-nitrophenyloxycarbonyl group, and a 4-methoxyphenyloxycarbonyl group; arylmethyloxycarbonyl groups such as a 9-fluorenylmethyloxycarbonyl group, a benzyloxycarbonyl group, a 4-methoxybenzyloxycarbonyl group, a 4 (or 2)-nitrobenzyloxycarbonyl group, a 4 (or 2)-aminobenzyloxycarbonyl group or an N-alkylcarbonylated or N-arylcarbonylated derivative thereof, a 4 (or 2)-hydroxybenzyloxycarbonyl group or an O-alkylcarbonylated or O-arylcarbonylated derivative thereof; arylmethyl groups such as a benzyl group and a triphenylmethyl group; alkyloxyalkyl groups such as a methyloxymethyl group, a methoxyethyloxymethyl group, and an ethoxy-2-ethyl group; arylalkyloxymethyl groups such as a benzyloxymethyl group; aryloxymethyl groups such as a phenyloxymethyl group; alkylthiomethyl groups such as a methylthiomethyl group; arylalkylthiomethyl groups such as a benzylthiomethyl group; arylthiomethyl groups such as a phenyloxymethyl group; an aminomethyl group in which the N atom is optionally protected by an alkyl group or a protecting group of amino group; alkylcarbonyl groups such as a methylcarbonyl group and an ethylcarbonyl group; alkenylcarbonyl groups such as an allylcarbonyl group; alkynylcarbonyl groups such as a propargylcarbonyl group; arylcarbonyl groups such as a phenylcarbonyl group; heteroarylcarbonyl groups such as a pyridine-2, 3, or 4-ylcarbonyl group; heterocyclylcarbonyl groups such as a tetrahydrofuran-2 or 3-ylcarbonyl group and a 5-oxotetrahydrofuran-2-ylcarbonyl group; silyl groups such as a trimethylsilyl group, a trimethylsilylethoxymethyl group, a triisopropylsilyl group, a tert-butyldimethylsilyl group, and a tert-butyldiphenylsilyl group; and arylsulfonyl groups such as a 2,4-dinitrobenzenesulfonyl group and a 4-nitrobenzenesulfonyl group; and protecting groups of amino group usually used in the peptide synthesis. Also, said amino group is optionally protected as an amide with formic acid, phosphoric acid, phosphonic acid, phosphinic acid, sulfuric acid, sulfonic acid, sulfinic acid, sulfenic acid, an amino acid, or a peptide.

In one embodiment, the protecting group of amino group is an alkyloxycarbonyl group; an allyloxycarbonyl group;

an arylmethyloxycarbonyl group; an alkylcarbonyl group; an alkenylcarbonyl group; an arylcarbonyl group; a silyl group, a lactonyl group; a lactonylalkyl group; a lactonylcarbonyl group; a lactonylalkylcarbonyl group; a phosphoryl group; a phosphorylalkyl group; or a phosphorylalkylcarbonyl group.

The protection by or removal of these protecting groups is carried out according to a method usually used (for example, see Greene's Protective Groups in Organic Synthesis 5th Edition, P. G. M. Wuts, JohnWiley & Sons Inc. (2014)).

Said functional groups are optionally reacted with various saccharides as described above to be protected as glycosides, namely, optionally protected by glycosyl groups. Specific examples of said glycosyl group include a D-glucosyl group, a D-galactosyl group, a D-mannosyl group, and a D-glucuronosyl group, and the functional groups (-OH, -SH, $-NH_2$, -NH-, -COOH groups, and the like) of their saccharide moieties are also optionally protected as above. These glycosides and protected forms thereof may be produced according to usual methods widely used in the field of sugar chemical (for example, Comprehensive Glycoscience From Chemistry to Systems Biology, Hans Kamerling, (2007)).

Among said functional groups, a hydroxy group, a sulfhydryl group (-SH), and an amino group ($-NH_2$ and -NH-(also including -NH- in an amide group and -NH- in the ring of a heteroaryl group or a heterocyclyl group)) are also optionally protected as an ester, a thio ester, an amide, or an imide, respectively, with various sugar acids (for example, gluconic acid, glucuronic acid, and galacturonic acid).

The "protecting groups" of various functional groups in the "derivative" in "an antitumor drug molecule or an analog thereof, or a derivative thereof" as described herein are described above, and they can also be applied to said functional groups in "linkers (L, $L^1$)", "jointing groups", or various protecting groups or substituents as described herein.

Further, the selection of and methods for protecting or deprotecting said protecting groups are also applied to the production of the following compounds of general formula (I) and (II) (conjugate precursor and synthetic intermediate of conjugate precursor).

Specific examples of "an antitumor drug molecule or an analog thereof, or a derivative thereof" as described herein are shown in the following Exemplification 1, but the present invention is not limited to them.

Exemplification 1. Exemplification of "an antitumor drug molecule or an analog thereof, or a derivative thereof"

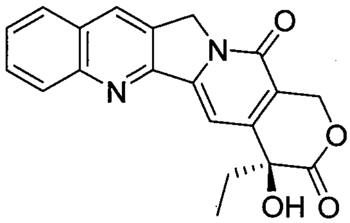

camptothecin

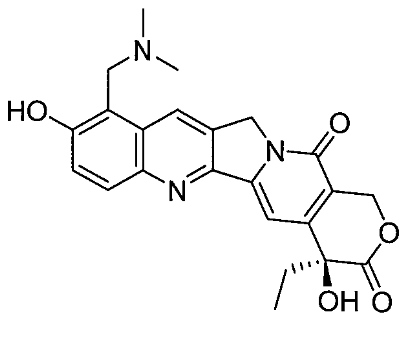

topotecan

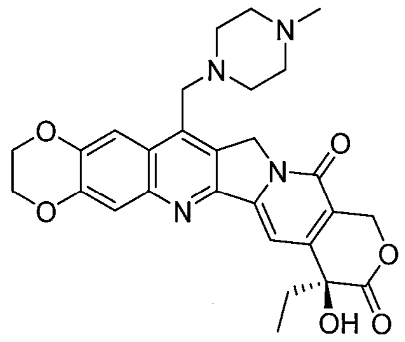

lurtotecan

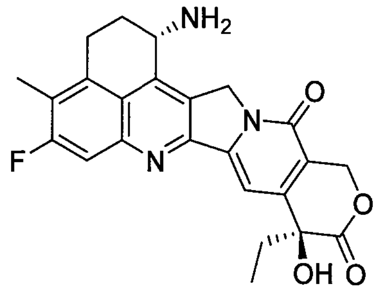

exatecan

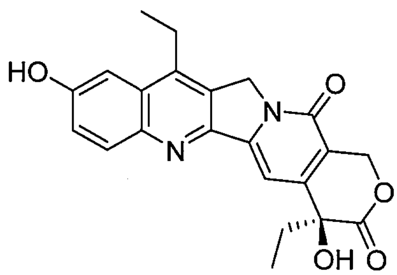

SN-38

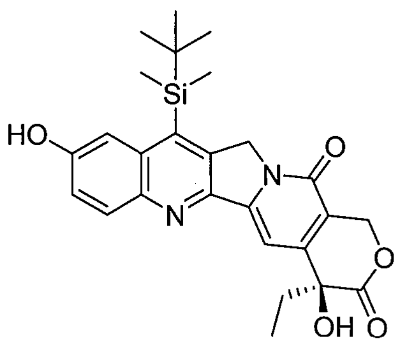

DB-67

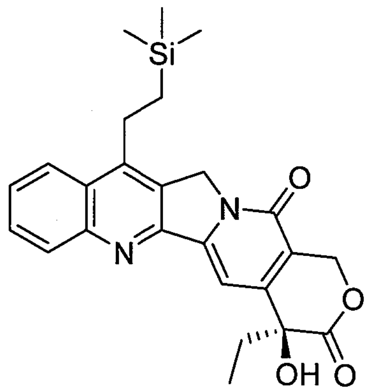

BNP-1350

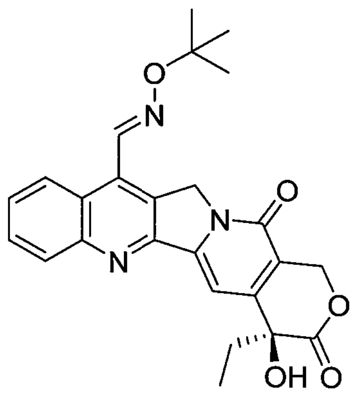

ST-1481

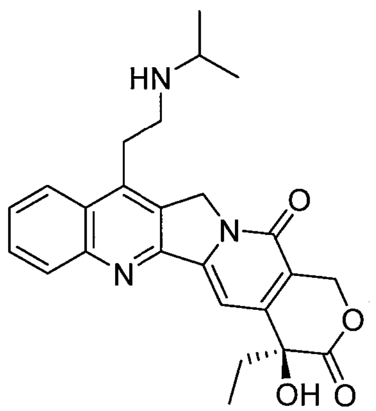

CKD-602

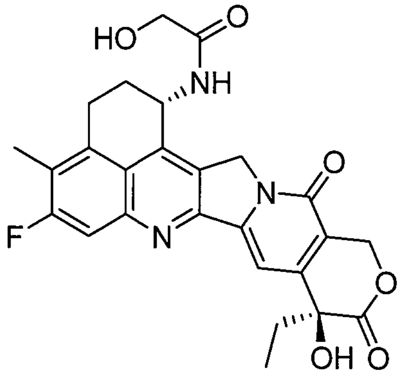

Dxd (N-(2-hydroxyacetyl)exatecan)

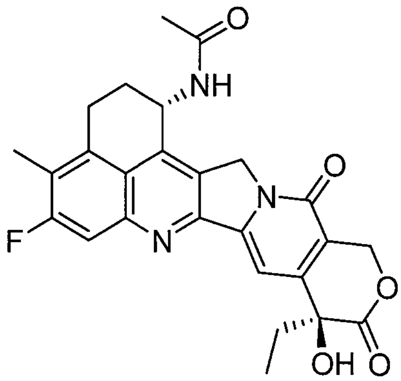

U-001

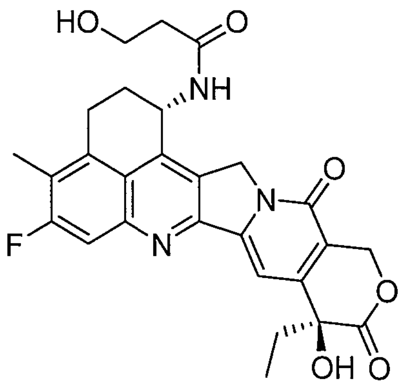

U-002

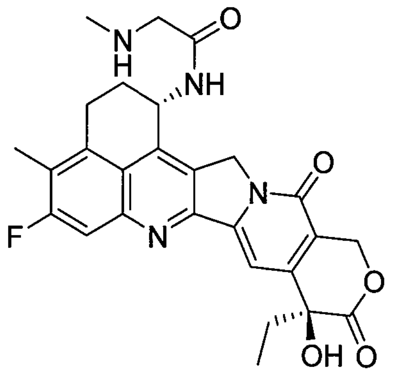

U-003

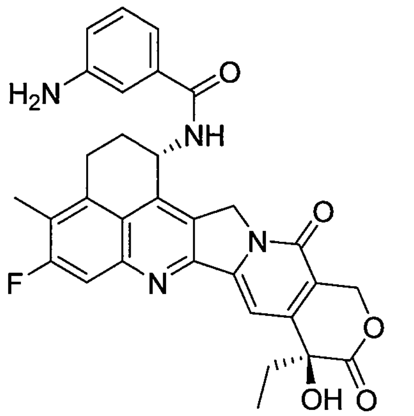

U-004

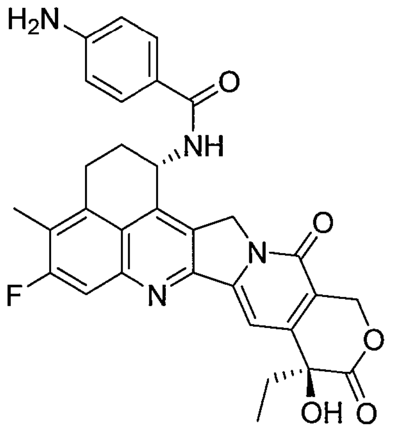

U-005

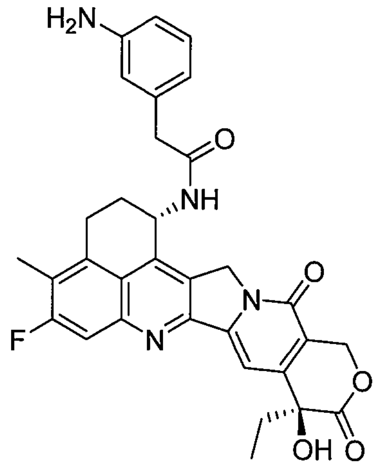

U-006

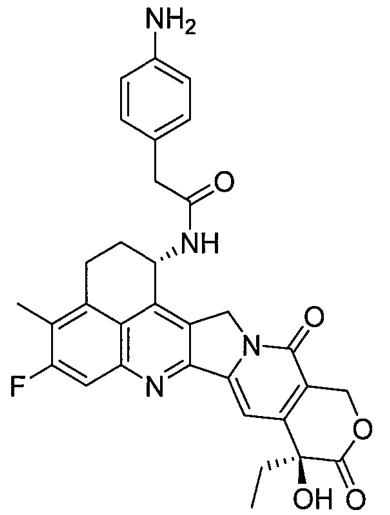

U-007

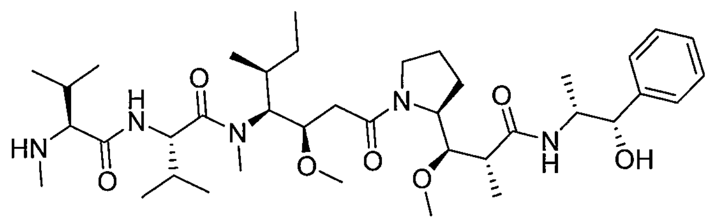

MMAE (monomethyl auristatin E)

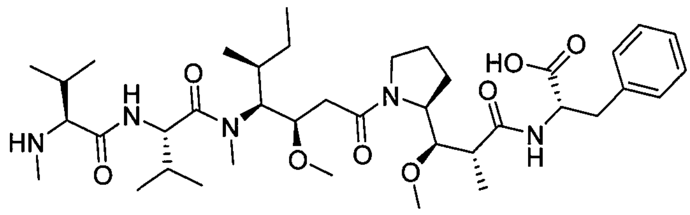

MMAF (monomethyl auristatin F)

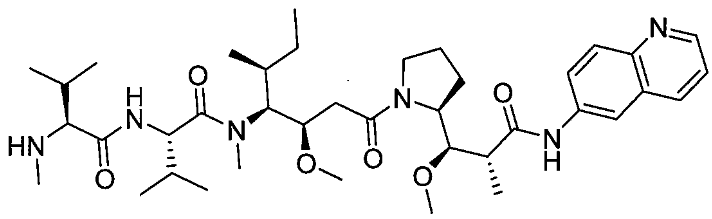

MMAQ (monomethyl auristatin Q)

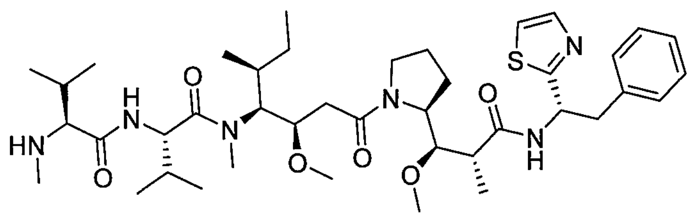

MMAD (monomethyldolastatin 10)

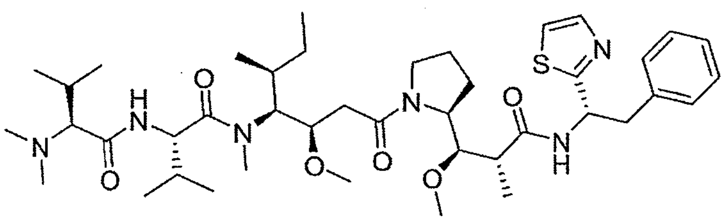

dolastatin 10

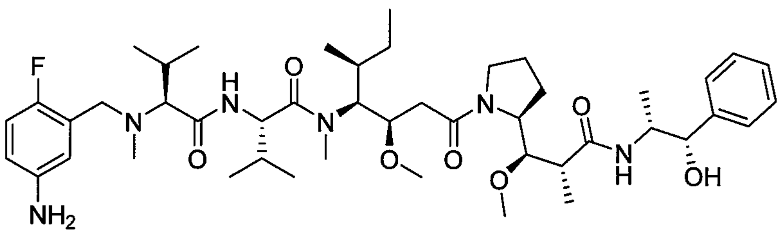

U-008

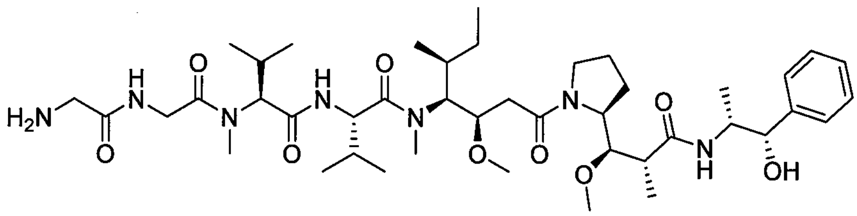

U-009

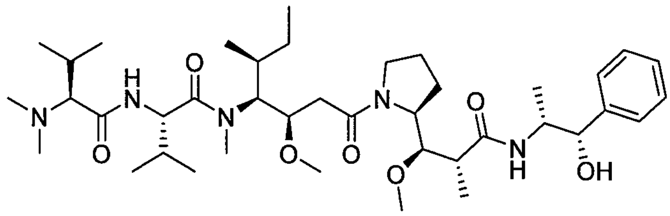

U-010

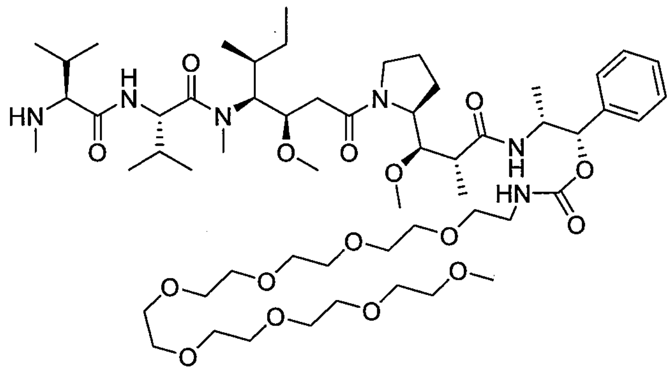

U-011

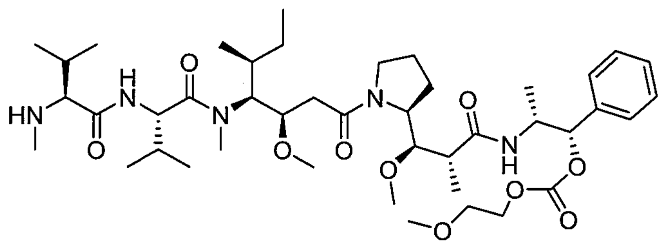

U-012

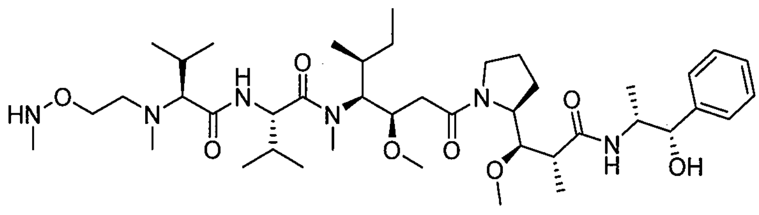

U-013

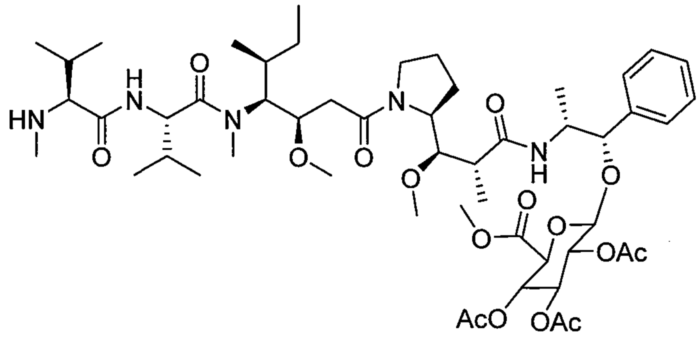

U-014

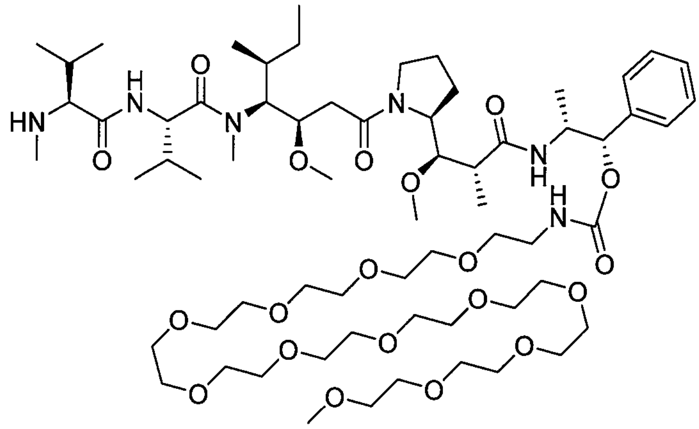

U-015

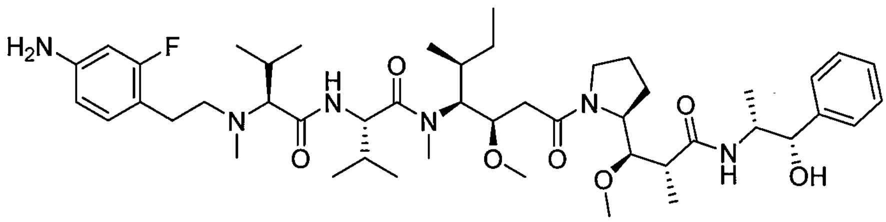

U-016

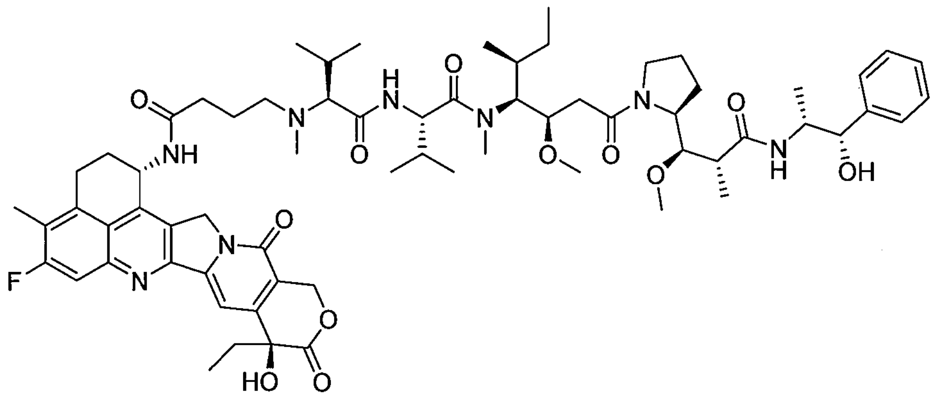

U-017

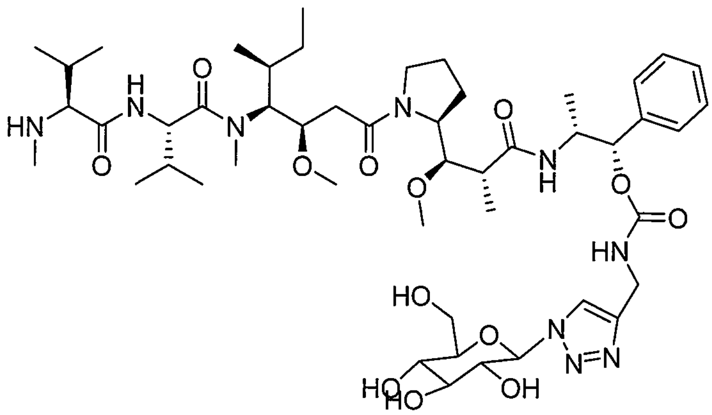

U-018

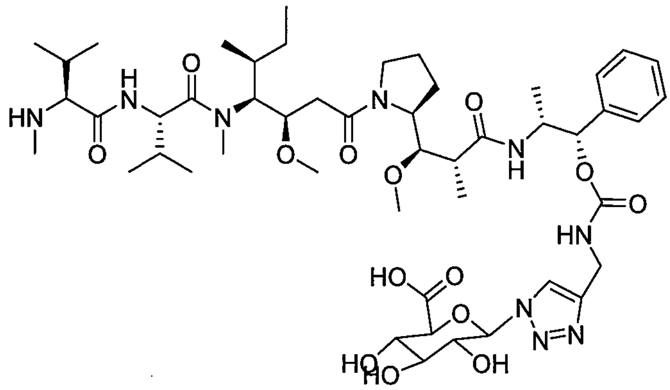

U-019

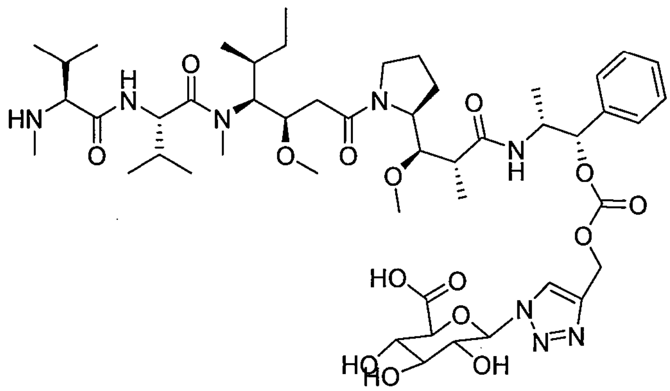

U-020

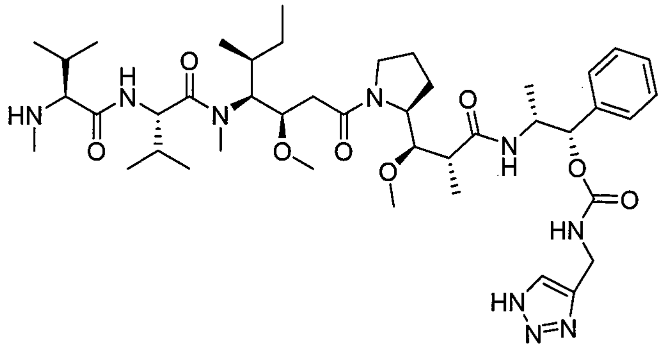

U-021

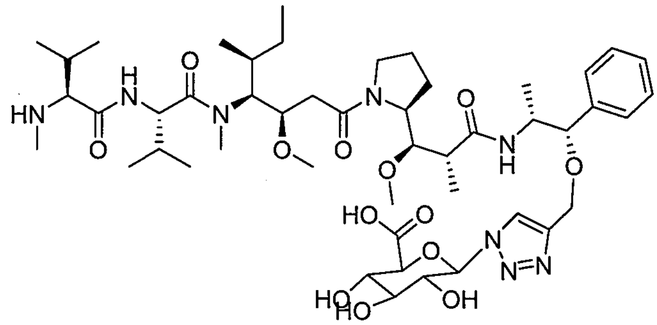

U-022

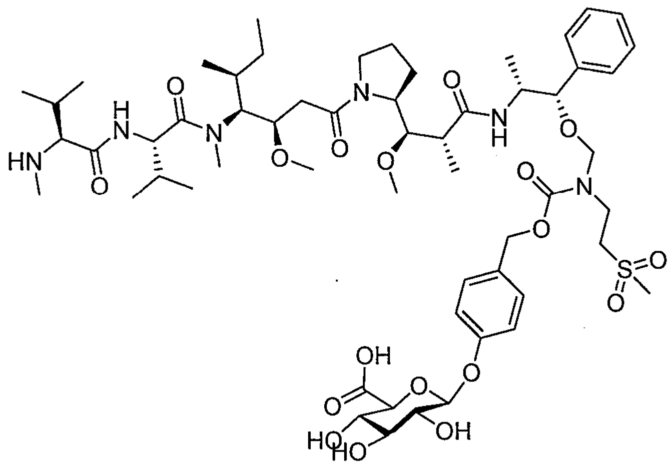

U-023

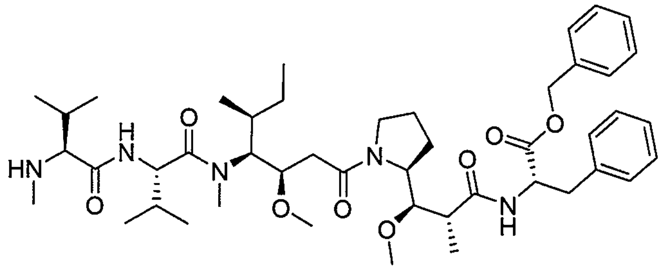

U-024

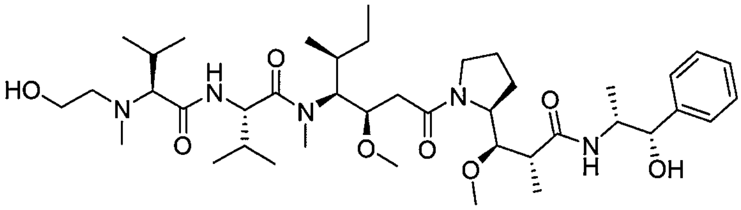

U-025

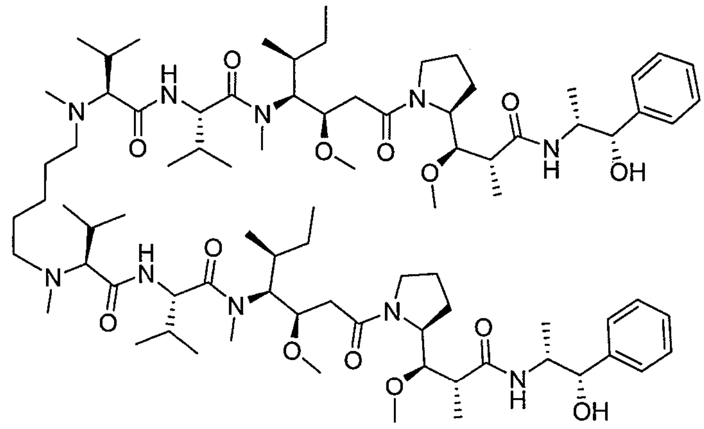

U-026

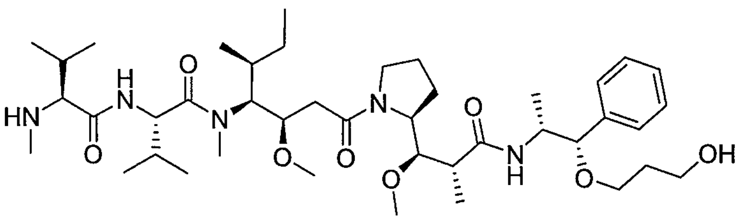

U-027

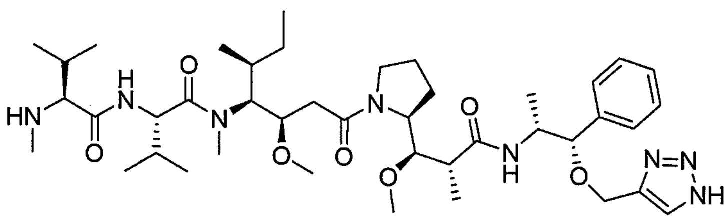

U-028

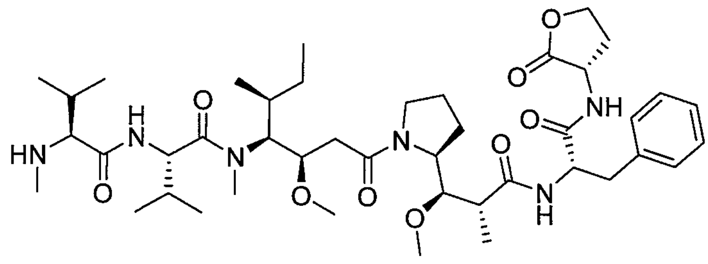

U-029

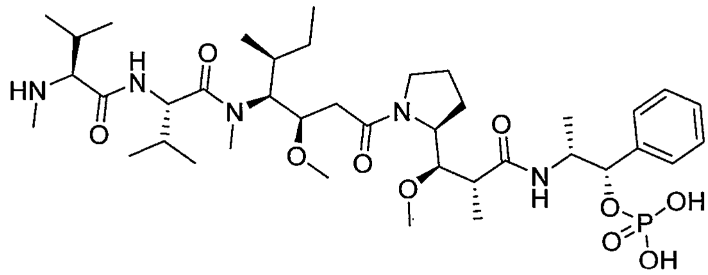

U-030

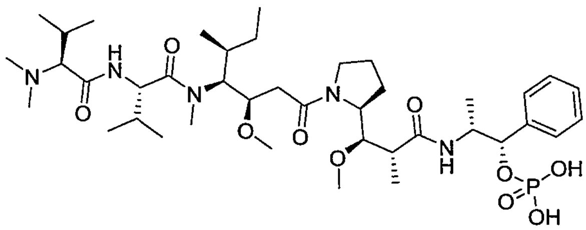

U-031

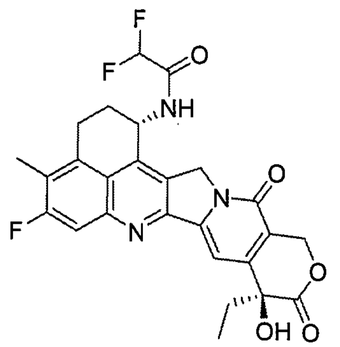

U-032

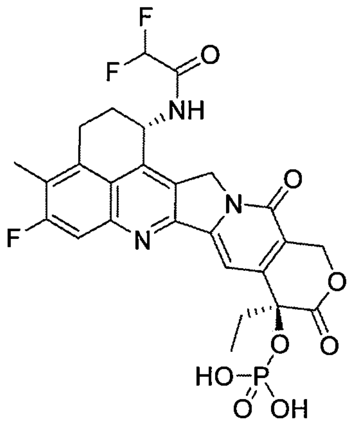

U-033

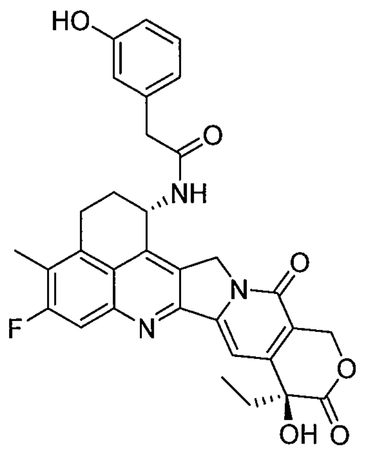

U-034

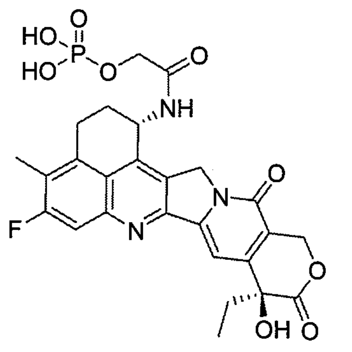

U-035

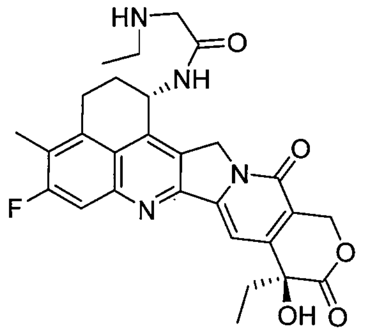

U-036

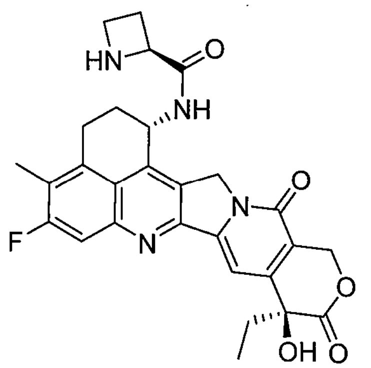

U-037

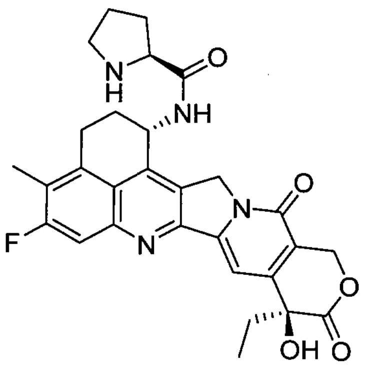

U-038

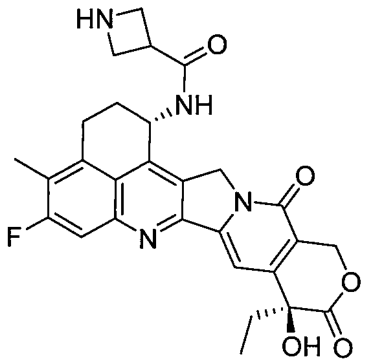

U-039

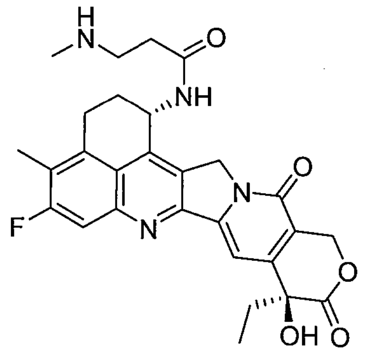

U-040

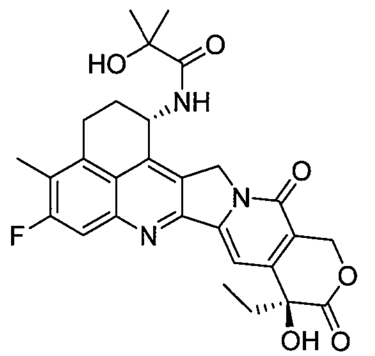

U-041

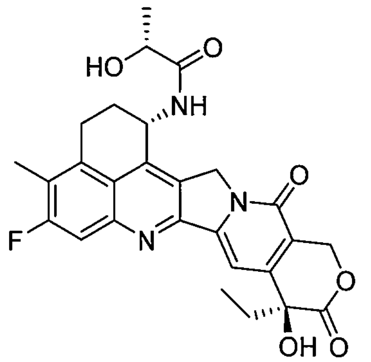

U-042

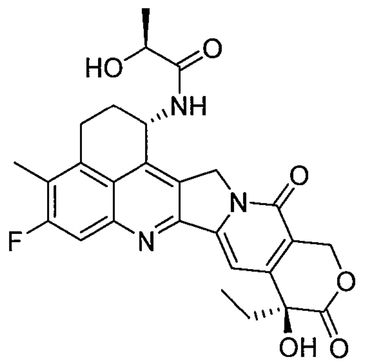

U-043

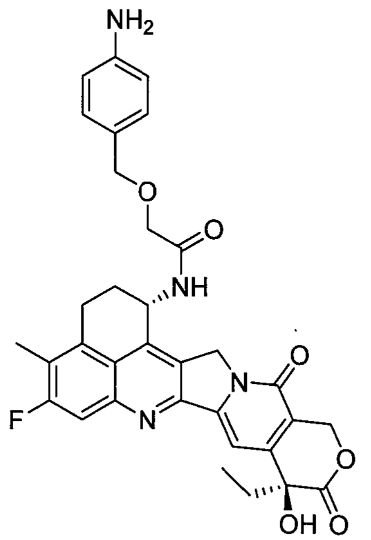

U-044

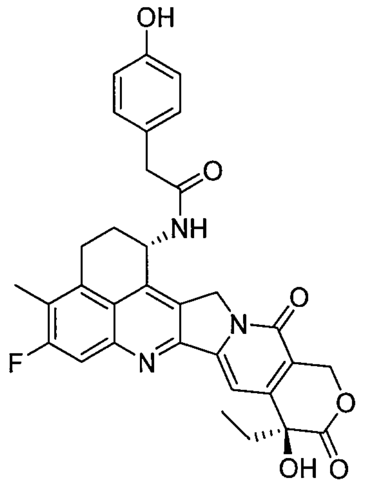

U-045

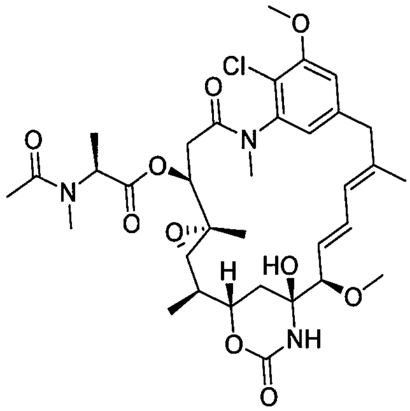

maytansine

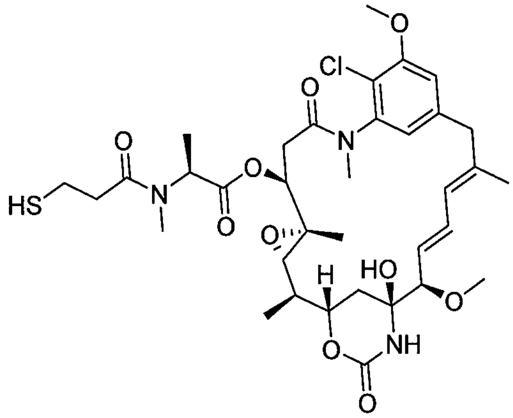

DM-1 (mertansine)

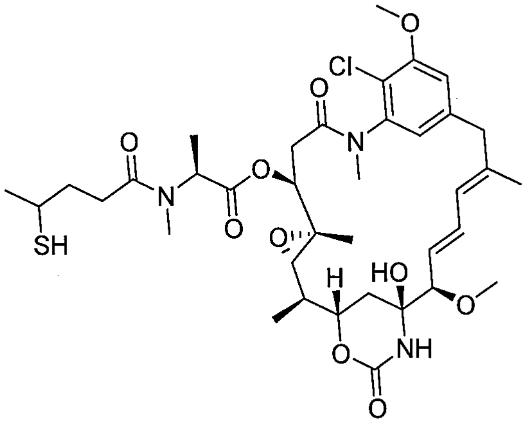

DM-3

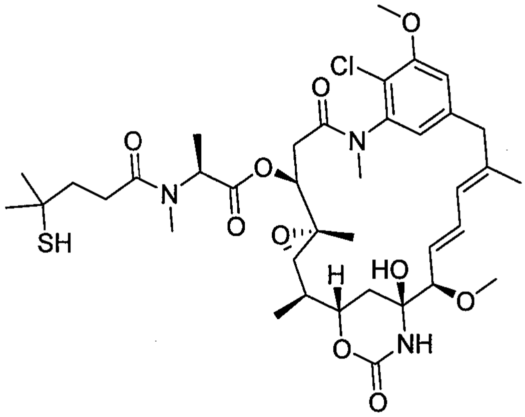

DM-4

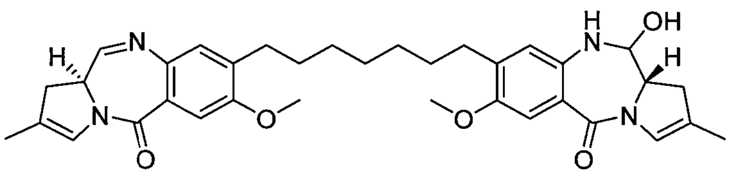

PBD dimer

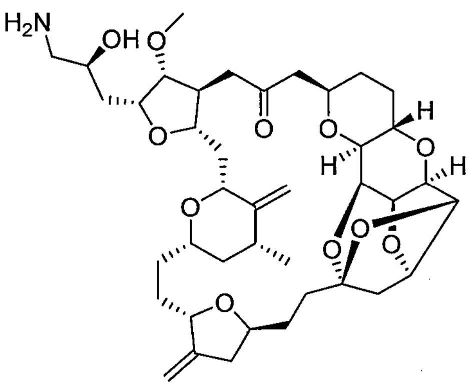

eribulin

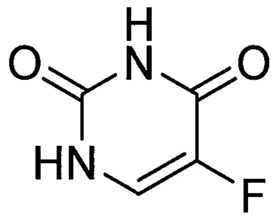

5-FU

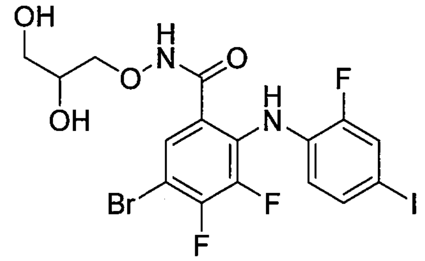

PD-318088

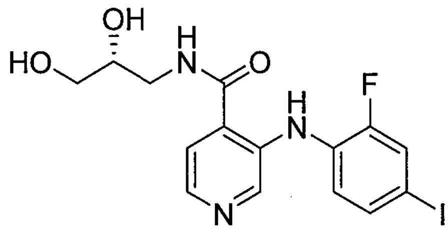

AS-703026

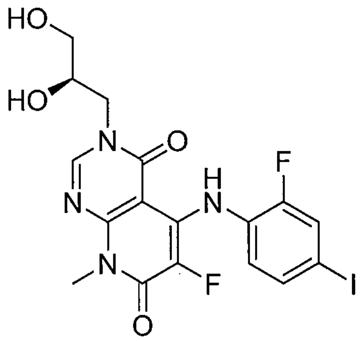

TAK-733

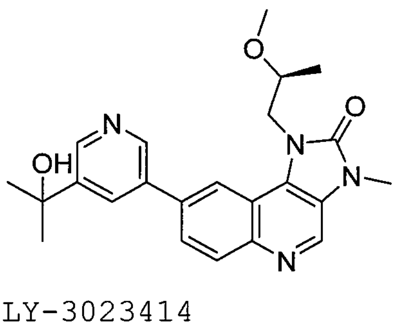

LY-3023414

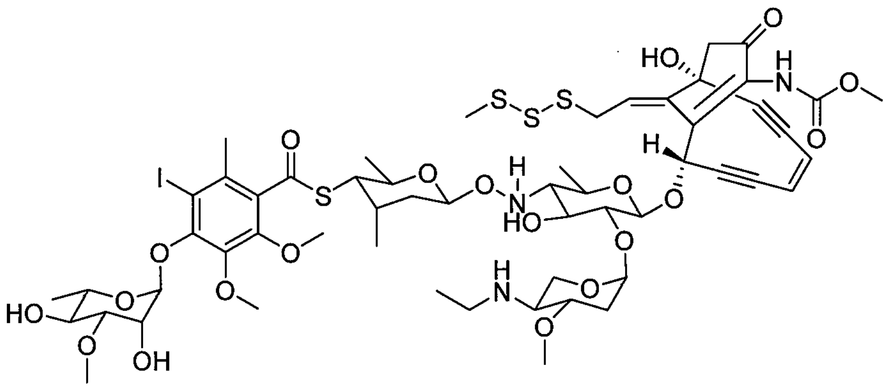

calicheamicin γ1

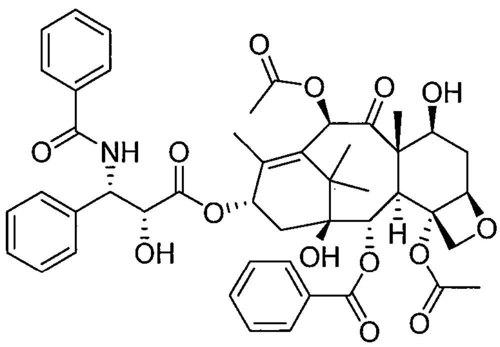

paclitaxel

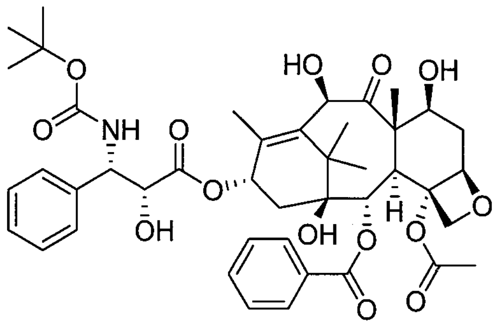

docetaxel

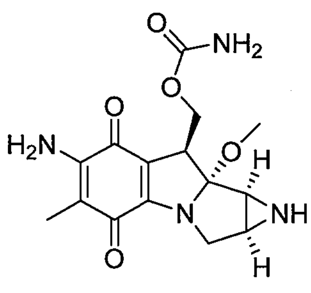

mitomycin C

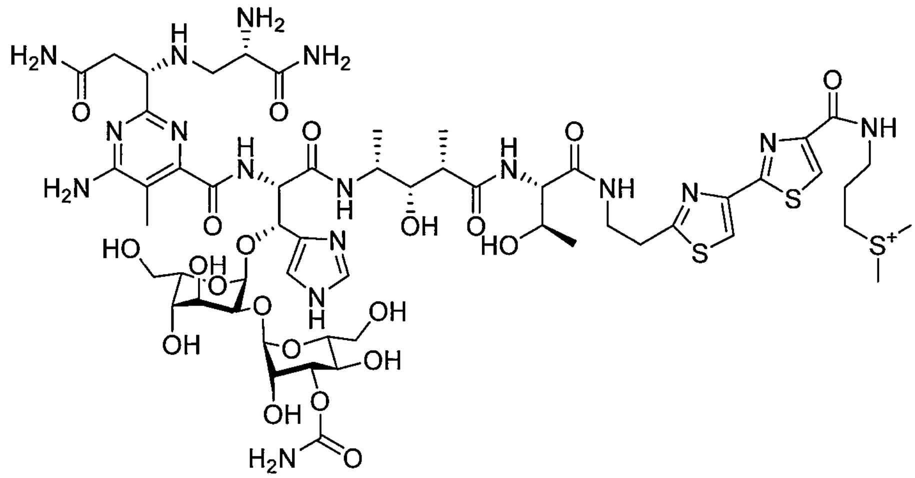

bleomycin A2

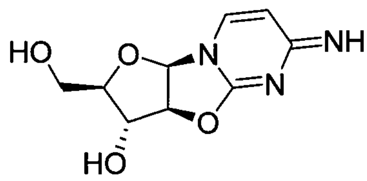

cyclocytidine

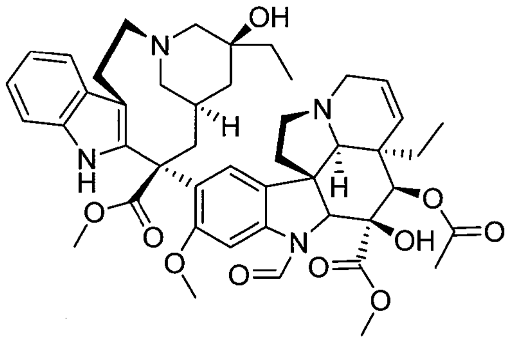

vincristine

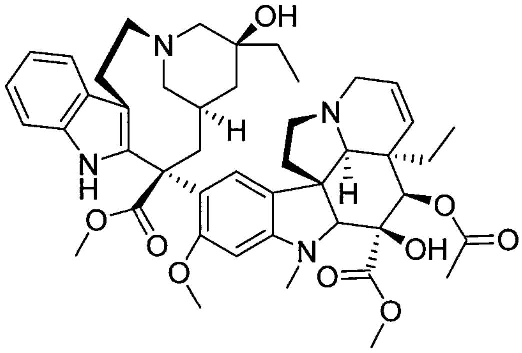

vinblastine

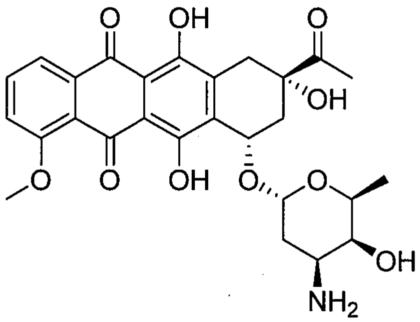

daunomycin

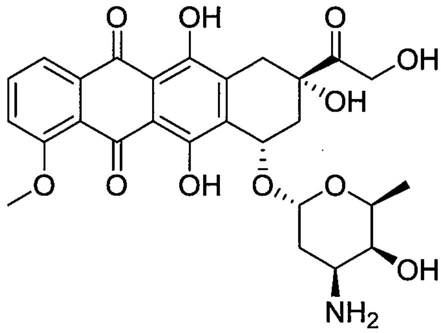

doxorubicin

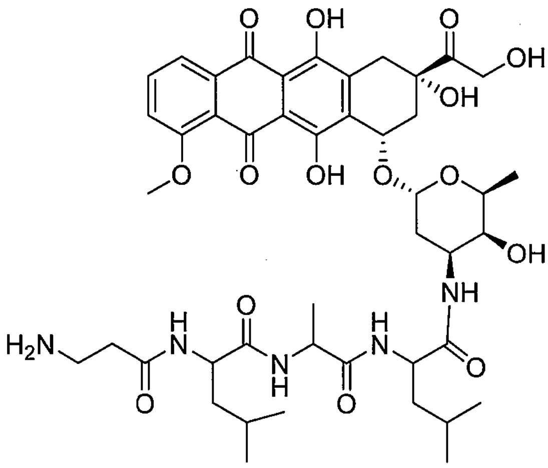

superdox

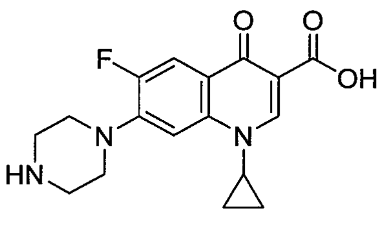

ciprofloxacin

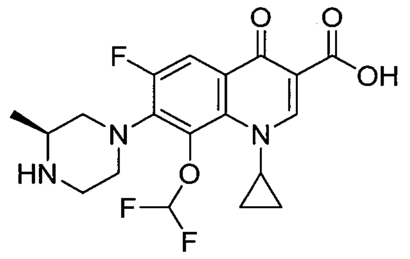

cadrofloxacin (CS-940)

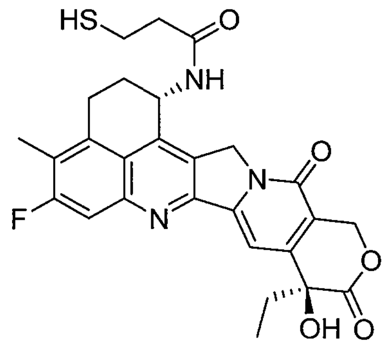

U-046

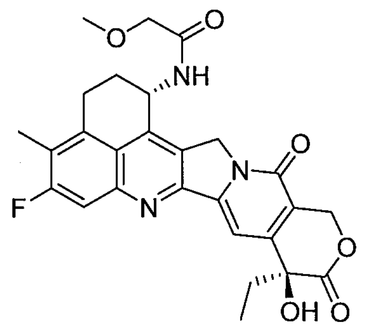

U-047

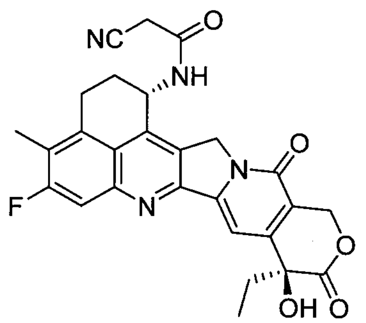

U-048

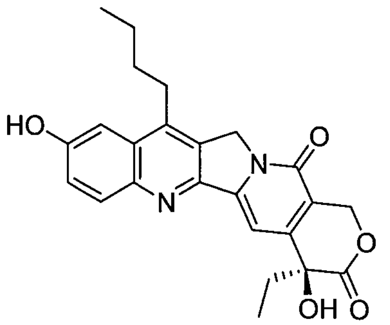

U-049

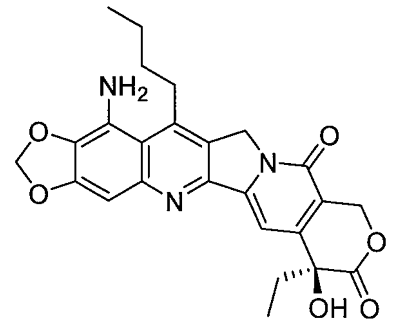

U-050

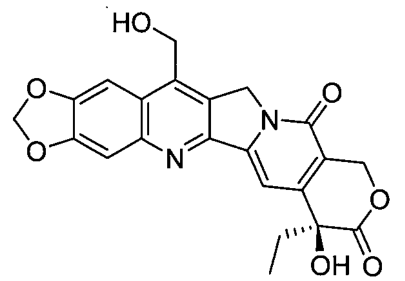

U-051

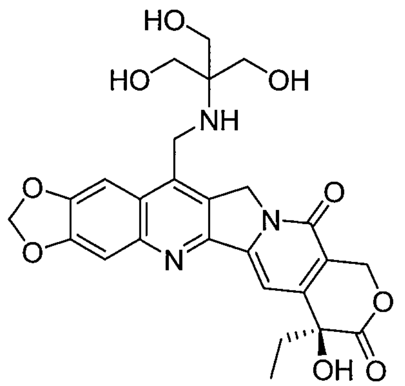

U-052

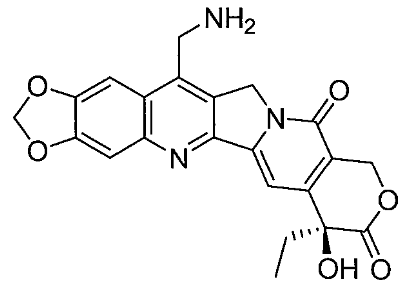

U-053

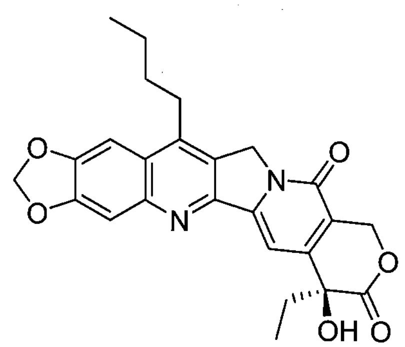

U-054

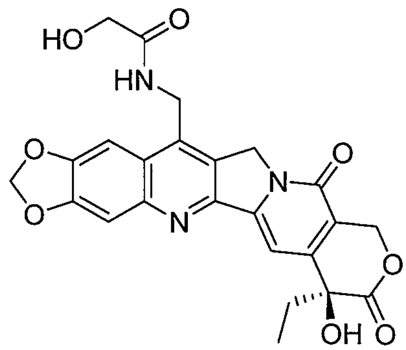

U-055

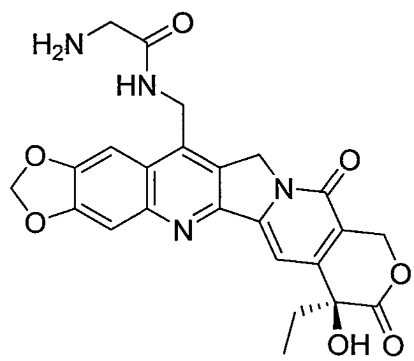

U-056

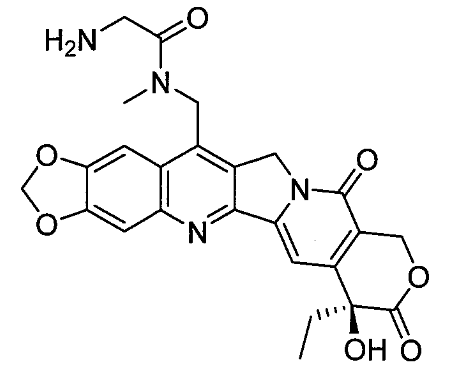

U-057

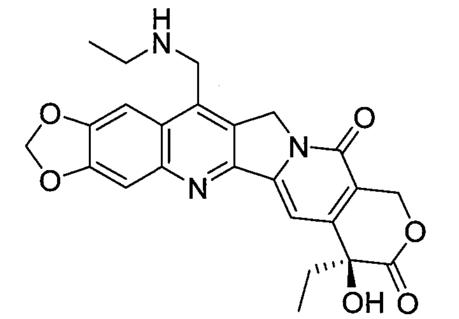

U-058

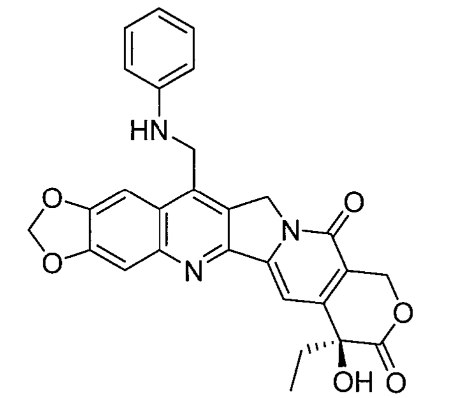

U-059

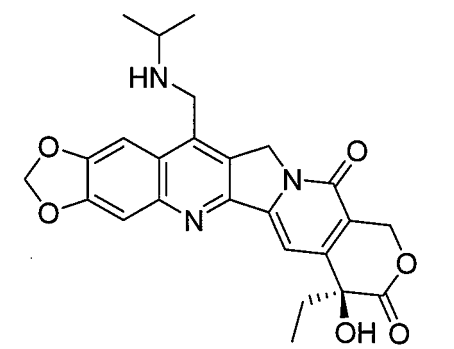

U-060

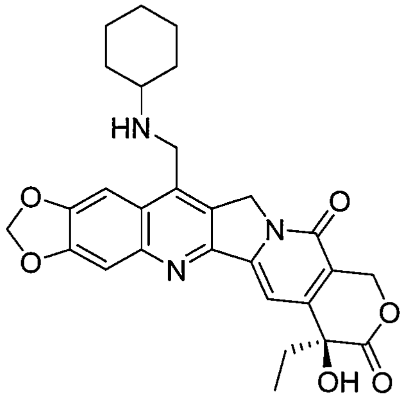

U-061

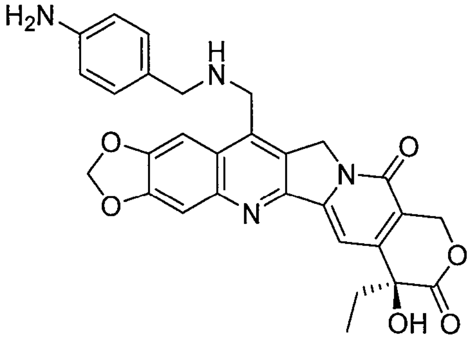

U-062

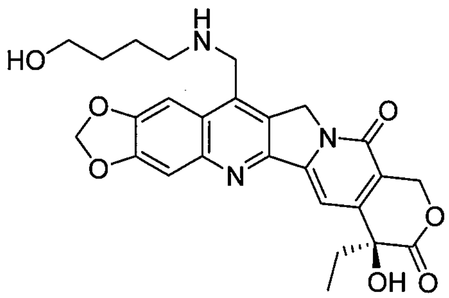

U-063

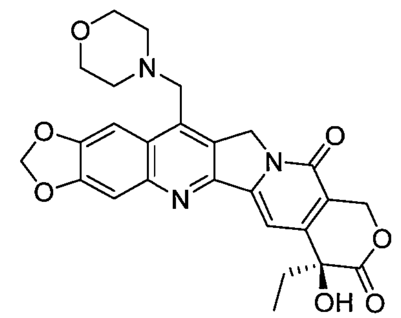

U-064

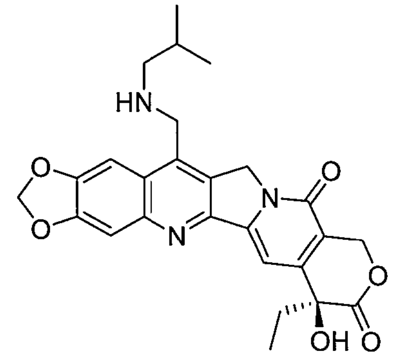

U-065

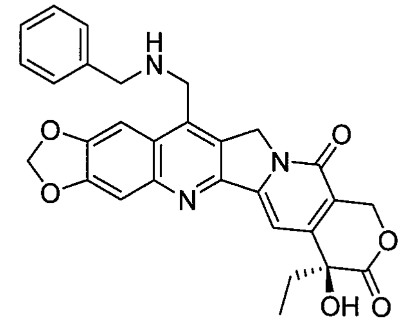

U-066

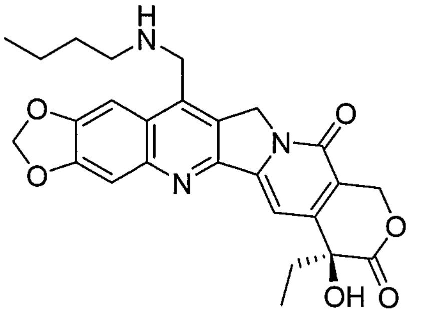

U-067

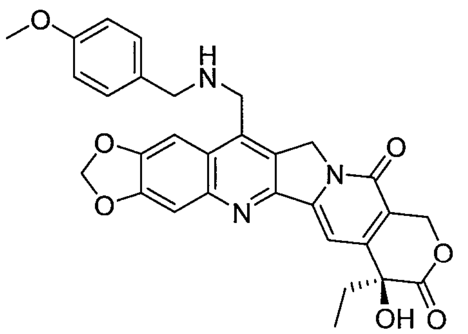

U-068

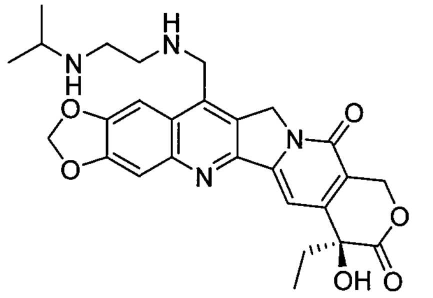

U-069

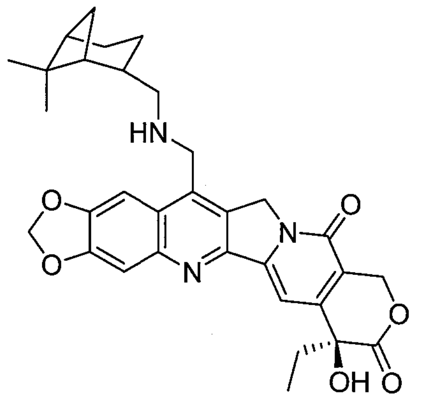

U-070

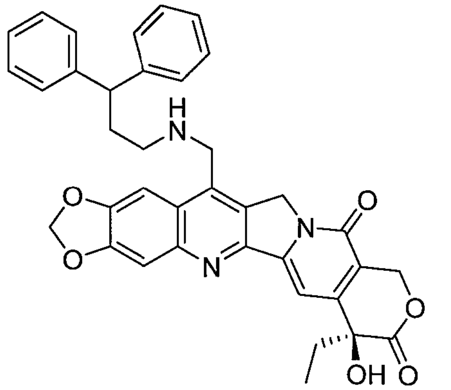

U-071

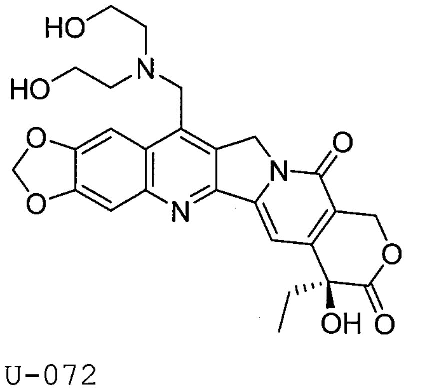

U-072

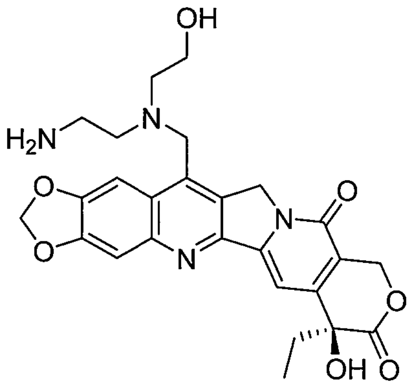

U-073

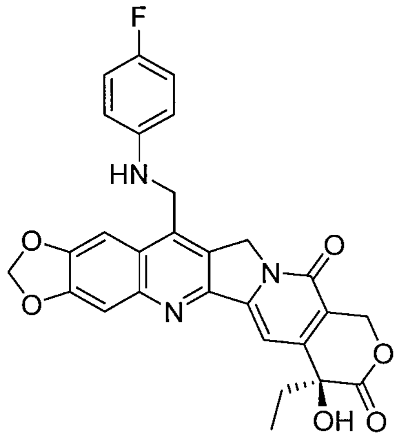

U-074

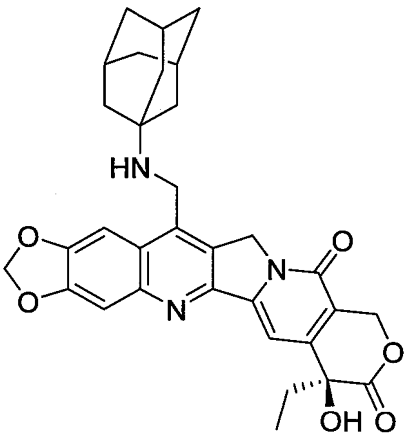

U-075

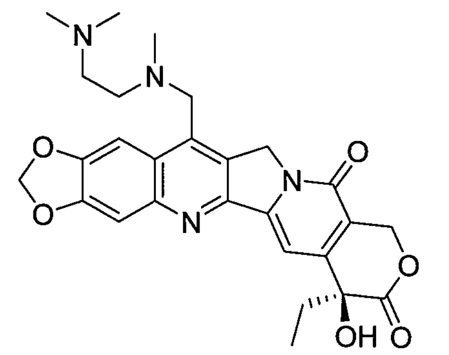

U-076

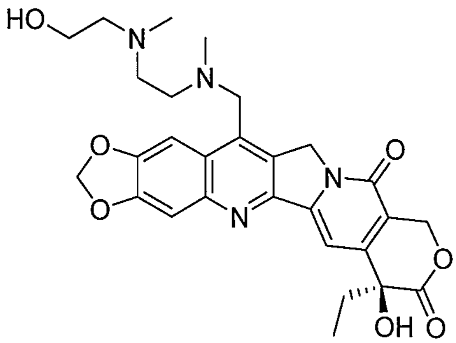

U-077

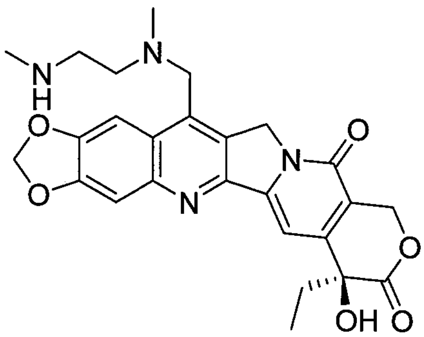

U-078

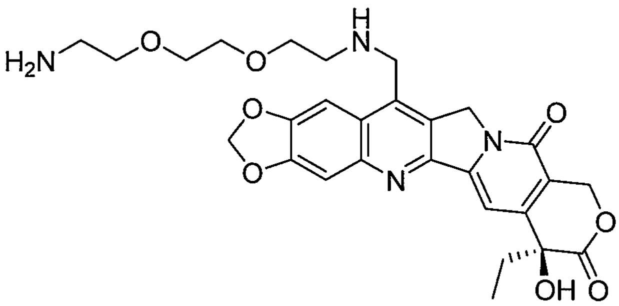

U-079

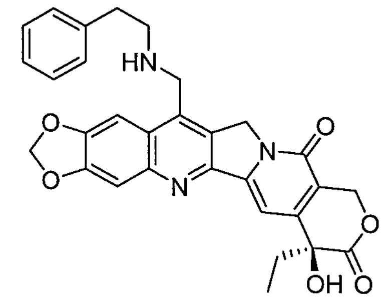

U-080

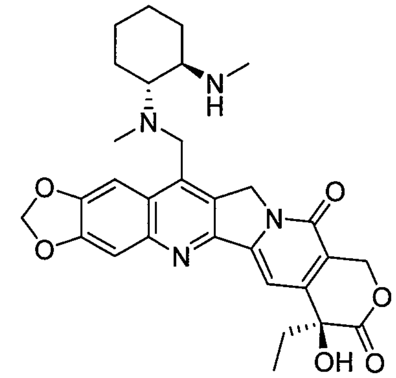

U-081

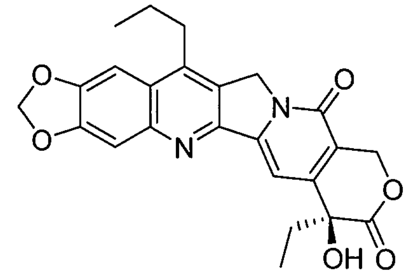

U-082

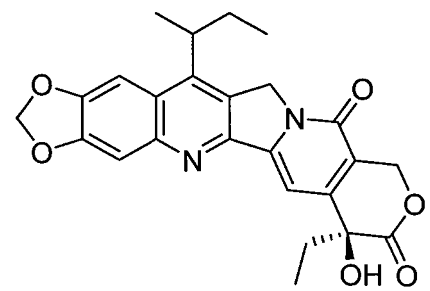

U-083

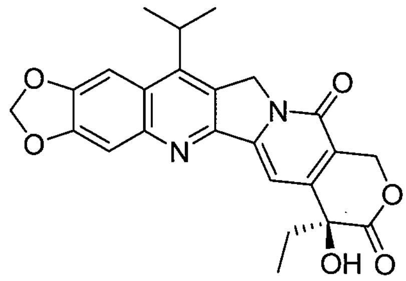

U-084

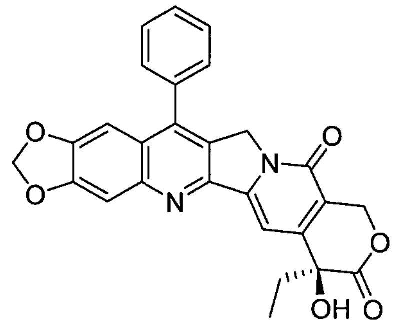

U-085

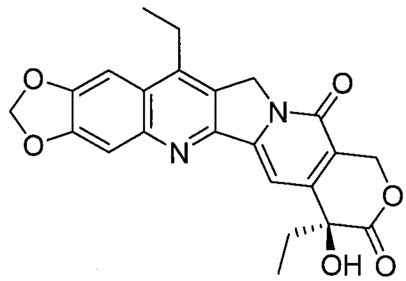

U-086

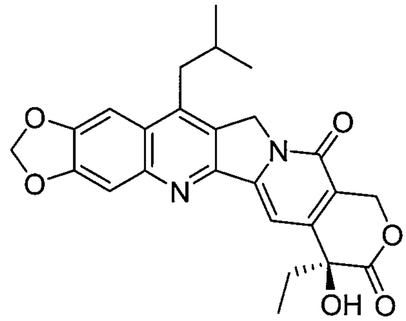

U-087

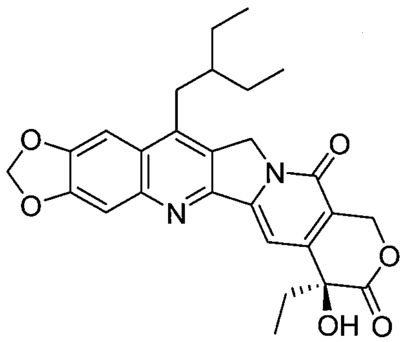

U-088

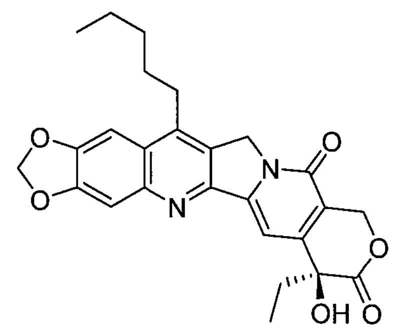

U-089

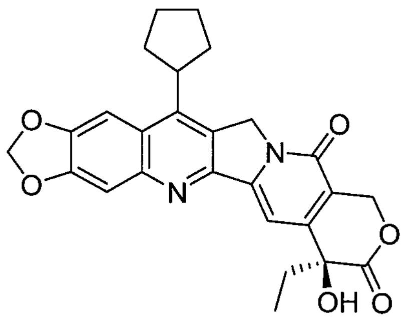

U-090

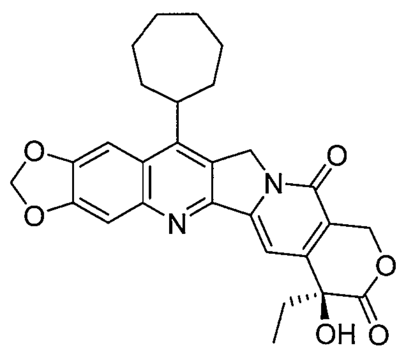

U-091

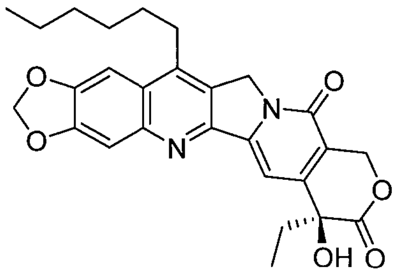

U-092

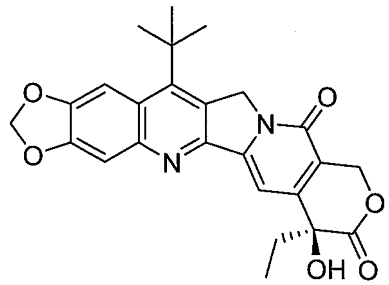

U-093

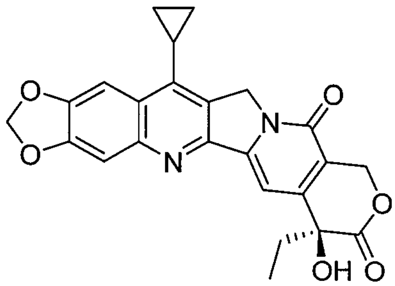

U-094

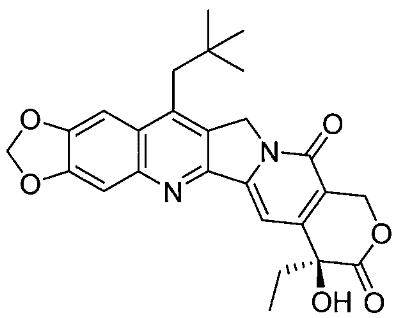

U-095

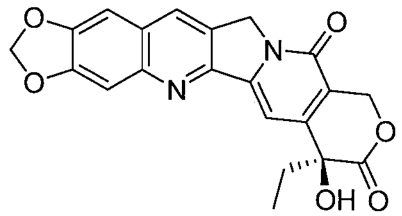

U-096

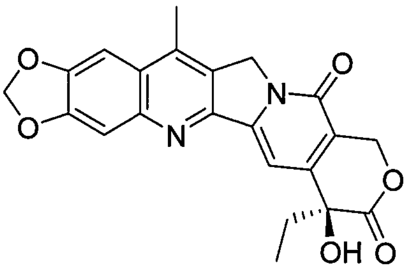

U-097

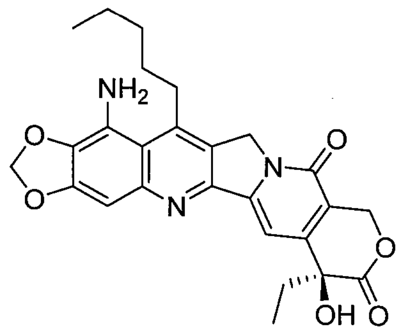

U-098

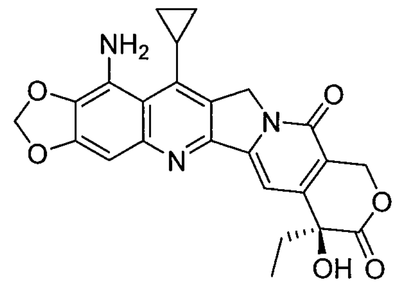

U-099

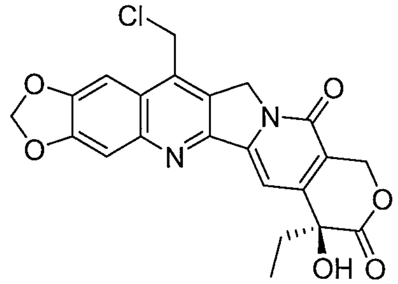

U-100

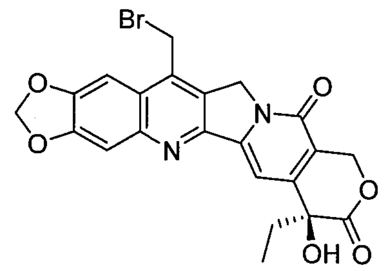

U-101

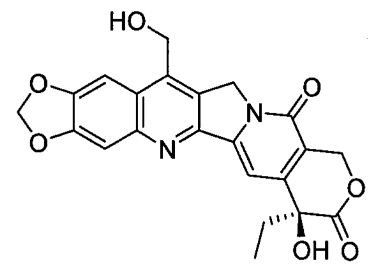

U-102

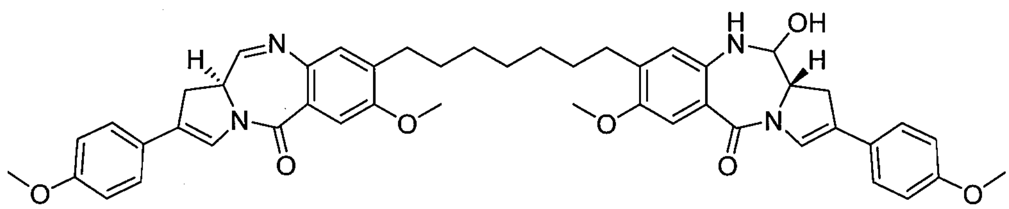

U-103

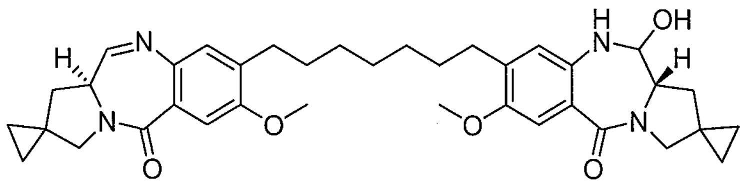

U-104

The term of "active ester of carboxy group" as described herein refers to a group in which a carboxy group is converted into an ester with an alcohol having high acidity to enhance the reactivity, and examples thereof include N-hydroxysuccinimidyl esters, sulfosuccinimidyl esters, N-hydroxyphthalimidyl esters, N-hydroxysulfophthalimidyl esters, ortho-nitrophenyl esters, para-nitrophenyl esters, 2,4-dinitrophenyl esters, 3-sulfonyl-4-nitrophenyl esters, 3-carboxy-4-nitrophenyl esters, and pentafluorophenyl esters, but the present invention is not limited to them.

The term of "alkyl group" as described herein refers to a group in which one hydrogen atom is removed from a straight or branched saturated hydrocarbon (i.e., alkane) having 1 to 10 carbon atom(s). Further, even if it is not specified, said alkyl group also encompasses "an optionally substituted alkyl group".

Examples of the substituent of an optionally substituted alkyl group include one or more substituent(s) independently selected from the following Group A.

Group A
a group consisting of:

halogen atom (fluorine atom, chlorine atom, bromine atom, or iodine atom);

alkyloxy group;
hydroxy group;
sulfhydryl group;
amino group;
alkylamino group;
dialkylamino group (wherein two alkyl groups are optionally combined to form a heterocyclyl group containing an N atom);
trialkylammonium group;
carboxy group;
cyano group;
nitro group;
alkylcarbonyl group;
alkenylcarbonyl group;
alkynylcarbonyl group;
arylcarbonyl group;
heteroarylcarbonyl group;
heterocyclylcarbonyl group;
lactonylcarbonyl group;
phosphorylalkylcarbonyl group;
alkyloxycarbonyl group;
aryloxycarbonyl group;
heteroaryloxycarbonyl group;
aminocarbonyl group;
aminocarbonyl group wherein the amino moiety is an amino group of any amino acid or peptide;
N-alkylaminocarbonyl group;
N,N-dialkylaminocarbonyl group (wherein two alkyl groups are optionally combined to form a heterocyclyl group containing an N atom);
aminocarbonyloxy group;
N-alkylaminocarbonyloxy group;
N,N-dialkylaminocarbonyloxy group (wherein two alkyl groups are optionally combined to form a heterocyclyl group containing an N atom);
alkyloxycarbonyloxy group;
aryloxycarbonyloxy group;
heteroaryloxycarbonyloxy group;
ureido group;
N-alkylureido group;
N,N-dialkylureido group (wherein two alkyl groups are optionally combined to form a heterocyclyl group containing an N atom);
N,N,N'-trialkylureido group (wherein two alkyl groups on the same N atom are optionally combined to form a heterocyclyl group containing an N atom);
guanidino group;
amidino group;
hydrazino group;
alkylhydrazino group;
N,N-dialkylhydrazino group;
N,N'-dialkylhydrazino group;
alkylthio group;
alkylsulfinyl group;
alkylsulfonyl group;
arylthio group;
arylsulfinyl group;
arylsulfonyl group;
heteroarylthio group;
heteroarylsulfinyl group;
heteroarylsulfonyl group;
sulfenic acid group;
sulfinic acid group;
sulfonic acid group;

sulfuric acid group;
sulfinamide group;
sulfonamide group;
phosphine oxide group;
phosphinic acid group;
phosphoryl group (phosphonic acid group $-P(=O)(OH)_2$);
bisphosphorylmethyl group ($-C(H, OH, NH_2$, Cl, or an optionally substituted alkyl)$(P(=O)(OH)_2)_2$);
phosphoric acid group (phosphate group $-O-P(=O)(OH)_2$); boric acid group;
alkenyl group;
alkynyl group;
cycloalkyl group;
cycloalkenyl group;
aryl group;
heteroaryl group;
heterocyclyl group;
lactonyl group;

D-glucose-(1, 2, 3, 4, or 6)-O-yl group, and
$-(OCH_2CH_2)_x-Y$ group
(wherein x is an integer of 1 to 30; and
Y is
hydrogen atom;
alkyl group;
halogen atom (fluorine atom, chlorine atom, bromine atom, or iodine atom);
alkyloxy group;
alkylthio group;
hydroxy group;
sulfhydryl group;
amino group;
alkylamino group;
dialkylamino group (wherein two alkyl groups are optionally combined to form a heterocyclyl group containing an N atom);
trialkylammonium group;
carboxy group;
cyano group;
nitro group;
alkylcarbonyl group;
alkenylcarbonyl group;
alkynylcarbonyl group;
arylcarbonyl group;
heteroarylcarbonyl group;
heterocyclylcarbonyl group;
lactonylcarbonyl group;
phosphorylalkylcarbonyl group;
alkyloxycarbonyl group;
aryloxycarbonyl group;
heteroaryloxycarbonyl group;
aminocarbonyl group;
aminocarbonyl group wherein the amino group moiety is an amino group of any amino acid or peptide;
N-alkylaminocarbonyl group;
N,N-dialkylaminocarbonyl group (wherein two alkyl groups are optionally combined to form a heterocyclyl group containing an N atom);
aminocarbonyloxy group;
N-alkylaminocarbonyloxy group;
N,N-dialkylaminocarbonyloxy group (wherein two alkyl groups are optionally combined to form a heterocyclyl group containing an N atom);
alkyloxycarbonyloxy group;
aryloxycarbonyloxy group;

heteroaryloxycarbonyloxy group;
ureido group;
N-alkylureido group;
N,N-dialkylureido group (wherein two alkyl groups are optionally combined to form a heterocyclyl group containing an N atom);
N,N,N'-trialkylureido group (wherein two alkyl groups on the same N atom are optionally combined to form a heterocyclyl group containing an N atom);
guanidino group;
amidino group;
hydrazino group;
alkylhydrazino group;
N,N-dialkylhydrazino group;
N,N'-dialkylhydrazino group;
alkylthio group;
alkylsulfinyl group;
alkylsulfonyl group;
arylthio group;
arylsulfinyl group;
arylsulfonyl group;
heteroarylthio group;
heteroarylsulfinyl group;
heteroarylsulfonyl group;
sulfenic acid group;
sulfinic acid group;
sulfonic acid group;
sulfuric acid group;
sulfinamide group;
sulfonamide group;
phosphine oxide group;
phosphinic acid group;
phosphoryl group (phosphonic acid group $-P(=O)(OH)_2$);
bisphosphorylmethyl group ($-C(H, OH, NH_2, Cl$, or an optionally substituted alkyl)$(P(=O)(OH)_2)_2$);
phosphoric acid group (phosphate group $-O-P(=O)(OH)_2$); boric acid group;
alkyl group;
alkenyl group;
alkynyl group;
cycloalkyl group;
cycloalkenyl group;
aryl group;
heteroaryl group;
heterocyclyl group;
lactonyl group); or
D-glucose-(1, 2, 3, 4, or 6)-O-yl group.

Specific examples of said alkyl group include a methyl group, an ethyl group, a n-propyl group, an isopropyl group, a n-butyl group, an isobutyl group, a sec-butyl group, a tert-butyl group, a n-pentyl group, a n-hexyl group, a n-heptyl group, a n-octyl group, a n-nonyl group, and a n-decyl group.

When the term of "alkane" is used in the present description, said alkane means "a straight or branched saturated hydrocarbon having 1 to 10 carbon atom(s)" formed by adding one hydrogen atom to the above-defined "alkyl group".

The term of "alkenyl group" as described herein refers to a straight or branched unsaturated hydrocarbon group comprising one or more double bond(s) (-C=C-) in the chain of the above-defined alkyl group.

Examples of the substituent of an optionally substituted alkenyl group include one or more substituent(s) independently selected from the Group A.

Specific examples of said alkenyl group include a methylidene group ($=CH_2$ group), an ethenyl group, a 1-propenyl group, a 2-propenyl group, a 1-butenyl group, a 2-butenyl group, a 3-butenyl group, a 1,3-butadienyl group, a 1-methyl-2-propenyl group, a 1,1-dimethyl-2-propenyl group, a 1-pentenyl group, a 2-pentenyl group, a 3-pentenyl group, a 4-pentenyl group, a 1-methyl-2-butenyl group, a 3-methyl-1-butenyl group, a 1-hexenyl group, a 2-hexenyl group, a 3-hexenyl group, a 4-hexenyl group, a 5-hexenyl group, a 1-methyl-2-pentenyl group, a 3-methyl-1-pentenyl group, a 2-heptenyl group, a 4-octenyl group, a 1-nonenyl group, and a 2-decenyl group.

The term of "alkynyl group" as described herein refers to a straight or branched unsaturated hydrocarbon group comprising one or more triple bond(s) (-C≡C-) in the chain of the above-defined alkyl group (provided that the number of carbon atom is 2 to 10).

Examples of the substituent of an optionally substituted alkynyl group include one or more substituent(s) independently selected from the Group A.

Specific examples of said alkynyl group include an ethynyl group, a 1-propynyl group, a 2-propynyl group, a 1-butynyl group, a 2-butynyl group, a 3-butynyl group, a 1-methyl-2-propynyl group, a 1,1-dimethyl-2-propynyl group, a 1-pentynyl group, a 2-pentynyl group, a 3-pentynyl group, a 4-pentynyl group, a 1-methyl-2-butynyl group, a 3-methyl-1-butynyl group, a 1-hexynyl group, a 2-hexynyl group, a 3-hexynyl group, a 4-hexynyl group, a 5-hexynyl group, a 1-methyl-2-pentynyl group, a 3-methyl-1-pentynyl group, a 2-heptynyl group, a 4-octynyl group, a 1-nonynyl group, and a 2-decynyl group.

The term of "alkyloxy group" as described herein refers to a monovalent group in which the above-defined alkyl group having 1 to 10 carbon atom(s) is bound to an oxy group (alkyl-O- group). Specific examples of said alkyloxy group include a methoxy group, an ethoxy group, a n-propoxy group, an isopropoxy group, a n-butyloxy group, an isobutyloxy group, a sec-butyloxy group, a tert-butyloxy group, a n-pentyloxy group, a n-hexyloxy group, a n-heptyloxy group, a n-octyloxy group, a n-nonyloxy group, and a n-decyloxy group.

The term of "alkylthio group" as described herein refers to a monovalent group in which the above-defined alkyl group having 1 to 10 carbon atom(s) is bound to a thio group (alkyl-S- group). Specific examples of said alkylthio group include a methylthio group, an ethylthio group, a n-propylthio group, an isopropylthio group, a n-butylthio group, an isobutylthio group, a sec-butylthio group, a tert-butylthio group, a n-pentylthio group, a n-hexylthio group, a n-heptylthio group, a n-octylthio group, a n-nonylthio group, and a n-decylthio group.

Examples of the substituent of an optionally substituted alkyloxy group and an alkylthio group include one or more substituent(s) independently selected from the Group A.

The term of "cycloalkyl group" as described herein refers to a group in which one hydrogen atom is removed from a monocyclic hydrocarbon (i.e., cycloalkane) having 3 to 10 carbon atoms and a group in which one hydrogen atom is removed from a bicyclic hydrocarbon (i.e., bicyclic cycloalkane) having 4 to 10 carbon atoms.

Examples of the substituent of an optionally substituted cycloalkyl group include one or more substituent(s) independently selected from an oxo group (=O); a thioxo group (=S); an imino group (=N($R^8$)); an oxime group (=N-O$R^9$); a hydrazono group (=N-N($R^{10}$)($R^{11}$)); an alkyl group; and the Group A (hereinafter the group consisting of an oxo group; a thioxo group; an imino group; an oxime group; a hydrazono group; an alkyl group; and the Group A is referred to as "Group B") (wherein $R^8$, $R^9$, $R^{10}$, and $R^{11}$ are each independently a hydrogen atom, an alkyl group, an alkenyl group, a cycloalkyl group, a cycloalkenyl group, an aryl group, a heteroaryl group, or a heterocyclyl group).

Specific examples of said cycloalkyl group include monocyclic cycloalkyl groups having 3 to 10 carbon atoms such as a cyclopropyl group, a cyclobutyl group, a cyclopentyl group, a cyclohexyl group, a cycloheptyl group, a cyclooctyl group, a cyclononyl group, and a cyclodecyl group; and bicyclic cycloalkyl groups having 4 to 10 carbon atoms such as a bicyclo[1.1.0]butyl group, a bicyclo[2.1.0]pentyl group, a bicyclo[3.1.0]hexyl group, a bicyclo[3.2.0]heptyl group, a bicyclo [2.2.1]heptyl group, a bicyclo[3.3.0]octyl group, a bicyclo[3.2.1]octyl group, a bicyclo[2.2.2]octyl group, a bicyclo[6.1.0] nonyl group, and a bicyclo[4.4.0]decyl group.

When the term of "cycloalkane" is used in the present description, said cycloalkane means "a monocyclic hydrocarbon having 3 to 10 carbon atoms and a bicyclic hydrocarbon having 4 to 10 carbon atoms" in which one hydrogen atom is added to the above-defined "cycloalkyl group".

The term of "cycloalkenyl group" as described herein refers to a monocyclic or bicyclic unsaturated hydrocarbon group comprising one or more double bond(s) (-C=C-) in the ring of the above-defined cycloalkyl group.

Examples of the substituent of an optionally substituted cycloalkenyl group include one or more substituent(s) independently selected from the Group B.

Specific examples of said cycloalkenyl group include monocyclic cycloalkenyl groups having 3 to 10 carbon atoms such as a 1-cyclopropenyl group, a 1-cyclobutenyl group, a 1-cyclopentenyl group, a 2-cyclopentenyl group, a 1-cyclohexenyl group, a 2-cyclohexenyl group, a 3-cyclohexenyl group, a 1,3-cyclohexadienyl group, a 1,4-cyclohexadienyl group, a 1-cycloheptenyl group, a 2-cyclooctenyl group, a 3-cyclooctenyl group, a 4-cyclooctenyl group, a 1-cyclononenyl group, and a 1-cyclodecenyl group; and bicyclic cycloalkenyl groups having 4 to 10 carbon atoms such as a bicyclo[1.1.0] but-1-enyl group, a bicyclo[2.1.0]pent-2-enyl group, a bicyclo[3.1.0]hex-2-enyl group, a bicyclo[3.2.0]hept-3-enyl group, a bicyclo[2.2.1]hept-2-enyl group, a bicyclo[3.3.0]oct-2-enyl group, a bicyclo[3.2.1]oct-2-enyl group, a bicyclo[2.2.2]oct-2-enyl group, a bicyclo[6.1.0]non-4-enyl group, and a bicyclo[4.4.0]dec-3-enyl group.

The term of "aryl group" as described herein refers to a group in which one hydrogen atom is removed from a monocyclic or bicyclic aromatic hydrocarbon (i.e., arene) having 6 to 11 ring carbon atoms.

Examples of the substituent of an optionally substituted aryl group include one or more substituent(s) independently selected from an alkyl group and the Group A (hereinafter the group consisting of an alkyl group and the Group A is

referred to as "Group C").

Specific examples of said aryl group include monocyclic aryl groups such as a phenyl group; and optionally partially saturated bicyclic aryl groups having 9 to 11 ring carbon atoms ($C_9$ to $C_{11}$) such as a naphthyl group, a tetrahydronaphthyl group, an indenyl group, and an indanyl group.

When the term of "arene" is used in the present description, said arene means "a monocyclic aromatic hydrocarbon having 6 to 11 ring carbon atoms or a bicyclic aromatic hydrocarbon having 9 to 11 ring carbon atoms" formed by adding one hydrogen atom to the above-defined "aryl group".

The term of "heteroaryl group" as described herein refers to a group in which one hydrogen atom is removed from a 5 to 14 membered monocyclic, bicyclic, or tricyclic aromatic heterocyclic ring comprising 1 to 4 heteroatom(s) selected from an oxygen atom, a sulfur atom, and a nitrogen atom other than carbon atom(s) (i.e., heteroarene).

Examples of the substituent of an optionally substituted heteroaryl group include one or more substituent(s) independently selected from the Group C.

Specific examples of said heteroaryl group include 5 to 6 membered monocyclic heteroaryl groups comprising 1 to 4 heteroatom(s) selected from an oxygen atom, a sulfur atom, and a nitrogen atom other than carbon atom(s) such as a pyrrolyl group, a furyl group, a thienyl group, a pyrazolyl group, an imidazolyl group, an oxazolyl group, an isoxazolyl group, a thiazolyl group, an isothiazolyl group, a thiadiazolyl group, a triazolyl group, a tetrazolyl group, a pyridyl group, a pyrazinyl group, a pyrimidinyl group, a pyridazinyl group, and a triazinyl group; 8 to 11 membered bicyclic heteroaryl groups comprising 1 to 4 heteroatom(s) selected from an oxygen atom, a sulfur atom, and a nitrogen atom other than carbon atom(s) such as an indolyl group, an isoindolyl group, an indazolyl group, a tetrahydroindazolyl group, a benzofuranyl group, a dihydrobenzofuranyl group, a dihydroisobenzofuranyl group, a benzothiophenyl group, a dihydrobenzothiophenyl group, a dihydroisobenzothiophenyl group, a benzooxazolyl group, a dihydrobenzooxazolyl group, a benzothiazolyl group, a dihydrobenzothiazolyl group, a quinolyl group, a tetrahydroquinolyl group, an isoquinolyl group, a tetrahydroisoquinolyl group, a naphthyridinyl group, a tetrahydronaphthyridinyl group, a quinoxalinyl group, a tetrahydroquinoxalinyl group, and a quinazolinyl group; and 11 to 14 membered tricyclic heteroaryl groups comprising 1 to 4 heteroatom(s) selected from an oxygen atom, a sulfur atom, and a nitrogen atom other than carbon atom(s) such as a dibenzofuran group, a dibenzopyrrole group, a dibenzothiophene group, and a dibenzopyridine group.

When the term of "heteroarene" is used in the present description, said heteroarene means "a 5 to 14 membered monocyclic, bicyclic, or tricyclic aromatic heterocyclic ring comprising 1 to 4 heteroatom(s) selected from an oxygen atom, a sulfur atom, and a nitrogen atom other than carbon atom(s)" formed by adding one hydrogen atom to the above-defined "heteroaryl group".

The term of "heterocyclyl group" as described herein refers to a monovalent group formed by removing one hydrogen atom from a 3 to 12 membered monocyclic nonaromatic heterocyclic ring comprising 1 to 4 heteroatom(s) selected from an oxygen atom, a sulfur atom, and a nitrogen atom other than carbon atom(s) (i.e., heterocyclene).

Examples of the substituent of an optionally substituted heterocyclyl group include one or more substituent(s) independently selected from an oxo group (=O); a thioxo group (=S); an imino group (=$NR^9$; wherein $R^9$ is the same as defined above); a hydrazono group (=N-N($R^{10}$)($R^{11}$); wherein $R^{10}$ and $R^{11}$ are the same as defined above); an alkyl group; and the Group A (hereinafter the group consisting of an oxo group; a thioxo group; an imino group; a hydrazono group; an alkyl group; and the Group A is referred to as "Group D").

Specific examples of said heterocyclyl group include an azetidinyl group, an oxetanyl group, a thietanyl group, a pyrrolidinyl group, a piperidinyl group, a piperidinyl group, a tetrahydrofuranyl group, a tetrahydropyranyl group, a tetrahydrothienyl group, a piperazinyl group, a morpholinyl group, a perhydroazepinyl group, an azacyclooctyl group, an azacyclooct-3-enyl group, an azacyclooct-4-enyl group, an azacyclononyl group, an azacyclodecyl group, 6 to 12 membered azabicycloalkyl groups (for example, an azabicyclohexyl group, an azabicycloheptyl group, an azabicyclooctyl group, an azabicyclononyl group, an azabicyclodecyl group, an azabicycloundecyl group, or an azabicyclododecyl group), 6 to 12 membered azabicycloalkenyl groups (for example, an azabicyclohexenyl group, an azabicycloheptenyl group, an azabicyclooctenyl group, an azabicyclononenyl group, an azabicyclodecenyl group, an azabicycloundecenyl group, or an azabicyclododecenyl group), 6 to 12 membered azaspiroalkyl groups (for example, an azaspirohexyl group, an azaspiroheptyl group, an azaspirooctyl group, an azaspirononyl group, an azaspirodecyl group, an azaspiroundecyl group, or an azaspirododecyl group).

When the term of "heterocyclene" is used in the present description, said heterocyclene means "a 4 to 12 membered monocyclic nonaromatic heterocyclic ring" comprising 1 to 4 heteroatom(s) selected from an oxygen atom, a sulfur atom, and a nitrogen atom other than carbon atom(s) formed by adding one hydrogen atom to the above-defined "heterocyclyl group".

The above cyclic compounds, i.e., cycloalkane, arene, heteroarene, and heterocyclene are each optionally fused to any other cyclic compound(s) to form bi- to tetracyclic compounds, and monovalent groups or divalent groups may be produced by removing one or two hydrogen atom(s) from these bi- to tetracyclic compounds.

The term of "lactonyl group" as described herein refers to a kind of said heterocyclyl group in which a heterocyclyl group has an oxygen atom in the ring as a heteroatom and the carbon atom adjacent to said oxygen atom is substituted with

an oxo group. Examples of said lactonyl group include an α-acetolactonyl group, a β-propiolactonyl group, a γ-butyrolactonyl group, a δ-valerolactonyl group, an ε-caprolactonyl group, a γ-nonalactonyl group, a γ-decalactonyl group, a γ-undecalactonyl group, a glucono-5-lactonyl group, and a pantolactonyl group (monovalent residue of pantolactone), and each of which is optionally substituted like said heterocyclyl group, and optionally fused to any other cyclic compound(s) to form a bi- to tetracyclic compound.

Said lactonyl group can be a substituent bound to a carbon atom or a heteroatom (for example, N, O, S, Ser, or P) present in L, $L^1$, D, or $D^1$ in the conjugate precursor or the synthetic intermediate of conjugate precursor of the present invention represented by general formula (I) or (II) .

Examples of the substitution of a lactonyl group to a heteroatom present in L, $L^1$, D, or $D^1$ include, but are not limited to, an α-acetolactonyl-3-yl group, a β-propiolactonyl-3-yl group, a β-propiolactonyl-4-yl group, a γ-butyrolactonyl-3-yl group, a γ-butyrolactonyl-4-yl group, a γ-butyrolactonyl-5-yl group, a 5-valerolactonyl-3-yl group, a 5-valerolactonyl-4-yl group, a δ-valerolactonyl-5-yl group, a 5-valerolactonyl-6-yl group, an ε-caprolactonyl-3-yl group, an ε-caprolactonyl-4-yl group, an ε-caprolactonyl-5-yl group, an ε-caprolactonyl-6-yl group, an ε-caprolactonyl-7-yl group, a (3-hydroxy-γ-butyrolactonyl-5-yl)methyl group, a 4-hydroxy-γ-butyrolactonyl group, a (3,4-dihydroxy-γ-butyrolactonyl-5-yl)methyl group, a glucurono-γ-lactonyl-3-yl group, a 2-(3,4-dihydroxy-γ-butyrolactonyl-5-yl)-1-hydroxyethyl group, a 4,4-dimethyl-β-propiolactonyl-3-yl group, a 4,5-dihydroxy-6-hydroxymethyl-5-valerolactonyl-3-yl group, a 4,5-dihydroxy-δ-valerolactonyl-3-yl group, and a (3,4,5-trihydroxy-5-valerolactonyl-3-yl)methyl group.

As said lactonyl group, both (S) configuration and (R) configuration thereof are encompassed by the present invention.

The term of "alkylene group" as described herein means a divalent group formed by removing any one hydrogen atom from the above-defined "alkyl group". Also, when said alkyl group has substituent(s), the term of "alkylene group" means a divalent group formed by removing any one additional hydrogen atom from an alkyl chain carbon atom other than said substituent(s).

In the general formula (I) and (II) of the present invention, L and $L^1$ are each independently an optionally substituted alkylene group, and one or more methylene group(s) in the chain of said alkylene group is/are optionally replaced with one or more divalent group(s) independently selected from the group consisting of - $C(R^1)(R^2)$-; -O-; $-N(R^3)-$; $-N(R^3)-N(R^3)-$; -S-; -Se-; - $Si(R^4)(R^5)$-; -S-S-; -Se-Se-; -SOm-; -SeOn-; $-C(=C(R^6)(R^7))-$; -C(=O)-; -C(=S)-; $-C(=N(R^8))-$; $-C(=N-OR^9)-$; $-C(=N-N(R^{10})(R^{11}))-$; $-P(=O)(R^{12})-$; $-P(=O)(OR^{13})-$; $-O-P(=O)(R^{12})-O-$; $-O-P(=O)(OR^{13})-O-$; $-C(R^{14})=$; $=C(R^{14})-$; $-C(R^{14})=C(R^{14})-$; -N=; =N-; -C=C-; $-(O-C(R^1)(R^2)-C(R^1)(R^2))_{1-30}-$; $-(C(R^1)(R^2)-C(R^1)(R^2)-0)_{1-30}-$ (wherein $R^1$, $R^2$, $R^3$, $R^4$, $R^5$, $R^6$, $R^7$, $R^8$, $R^9$, $R^{10}$, $R^{11}$, $R^{12}$, $R^{13}$, and $R^{14}$ are independently a group selected from a hydrogen atom, an alkyl group, an alkenyl group, an alkynyl group, a cycloalkyl group, a cycloalkenyl group, an aryl group, a heteroaryl group, a heterocyclyl group, wherein when $R^3$ is an alkyl group, said alkyl group is optionally combined with an alkyl group in an adjacent methylene group to form a cyclic structure, and m and n are each independently an integer of 0 to 2); an optionally substituted alkenylene group, an optionally substituted alkynylene group, an optionally substituted cycloalkylene group; an optionally substituted cycloalkenylene group; an optionally substituted arylene group; an optionally substituted heteroarylene group; and an optionally substituted heterocyclylene group.

Examples of the substituent of an optionally substituted alkylene group include one or more substituent(s) independently selected from the Group A.

Specific examples of said alkylene group include a methylene group, an ethylene group, a methylmethylene group, a trimethylene group, an ethylmethylene group, a dimethylmethylene group, a n-propylene group, a n-butylene group, a n-pentylene group, a n-hexylene group, a n-heptylene group, a n-octylene group, a n-nonylene group, a n-decylene group, a n-undecylene group, a n-dodecylene group, a n-tridecylene group, a n-tetradecylene group, a n-pentadecylene group, a n-hexadecylene group, a n-heptadecynylene group, a n-octadecylene group, a n-nonadecylene group, a n-icosenylene group, a n-triacontylene group, a n-tetracontylene group, and a n-pentacontylene group.

The term of "alkenylene group" as described herein refers to a group constituting the linker chain represented by L and $L^1$ in the above general formula (I) and (II) respectively, and means a divalent group formed by removing any one hydrogen atom from the above-defined "alkenyl group". Also, when said alkenyl group has substituent(s), the term of "alkenylene group" means a divalent group formed by removing any one additional hydrogen atom from an alkenyl chain carbon atom other than said substituent(s).

Examples of the substituent of an optionally substituted alkenylene group include one or more substituent(s) independently selected from the Group A.

Specific examples of said alkenylene group include an ethenylene group, a 1-propenylene group, a 2-propenylene group, a 1-butenylene group, a 2-butenylene group, a 3-butenylene group, a 1,3-butadienylene group, a 1-methyl-2-propenylene group, a 1,1-dimethyl-2-propenylene group, a 1-pentenylene group, a 2-pentenylene group, a 3-pentenylene group, a 4-pentenylene group, a 1-methyl-2-butenylene group, a 3-methyl-1-butenylene group, a 1-hexenylene group, a 2-hexenylene group, a 3-hexenylene group, a 4-hexenylene group, a 5-hexenylene group, a 1-methyl-2-pentenylene group, a 3-methyl-1-pentenylene group, a 2-heptenylene group, a 4-octenylene group, a 1-nonenylene group, a 2-decenylene group, a 1-undecenylene group, a 3-dodecenylene group, a 2-tridecenylene group,

a 4-tetradecenylene group, a 1-pentadecenylene group, a 2-hexadecenylene group, a 3-heptadecenylene group, a 2-octadecenylene group, a 1-nonadecenylene group, and a 1-icosenylene group.

The term of "alkynylene group" as described.herein refers to a group constituting the linker chain represented by L and $L^1$ in the above general formula (I) and (II) respectively, and means a divalent group formed by removing any one hydrogen atom from the above-defined "alkynyl group". Also, when said alkynyl group has substituent(s), the term of "alkynylene group" means a divalent group formed by removing any one additional hydrogen atom from the alkynyl chain carbon atom(s) other than said substituent(s).

Examples of the substituent of an optionally substituted alkynylene group include one or more substituent(s) independently selected from the Group A.

Specific examples of said alkynylene group include an ethynylene group, a 1-propynylene group, a 2-propynylene group, a 1-butynylene group, a 2-butynylene group, a 3-butynylene group, a 1-methyl-2-propynylene group, a 1,1-dimethyl-2-propynylene group, a 1-pentynylene group, a 2-pentynylene group, a 3-pentynylene group, a 4-pentynylene group, a 1-methyl-2-butynylene group, a 3-methyl-1-butynylene group, a 1-hexynylene group, a 2-hexynylene group, a 3-hexynylene group, a 4-hexynylene group, a 5-hexynylene group, a 1-methyl-2-pentynylene group, a 3-methyl-1-pentynylene group, a 2-heptynylene group, a 4-octynylene group, a 1-nonynylene group, a 2-decynylene group, a 1-undecynylene group, a 3-dodecynylene group, a 2-tridecynylene group, a 4-tetradecynylene group, a 1-pentadecynylene group, a 2-hexadecynylene group, a 3-heptadecynylene group, a 2-octadecynylene group, a 1-nonadecynylene group, and a 1-icosinylene group.

The term of "cycloalkylene group" as described herein refers to a group constituting the linker chain represented by L and $L^1$ in the above general formula (I) and (II) respectively, and means a divalent group formed by removing any one hydrogen atom from the above-defined "cycloalkyl group". Also, when said cycloalkyl group has substituent(s), the term of "cycloalkylene group" means a divalent group formed by removing any one additional hydrogen atom from a cycloalkyl ring carbon atom other than said substituent(s).

Examples of the substituent of an optionally substituted cycloalkylene group include one or more substituent(s) independently selected from the Group B.

Specific examples of said cycloalkylene group include monocyclic cycloalkylene groups having 3 to 10 carbon atoms such as a cyclopropylene group, a cyclobutylene group, a cyclopentylene group, a cyclohexylene group, a cycloheptylene group, a cyclooctylene group, a cyclononylene group, and a cyclodecylene group; and bicyclic cycloalkylene groups having 4 to 10 carbon atoms such as a bicyclo[1.1.0]butylene group, a bicyclo[2.1.0]pentylene group, a bicyclo[3.1.0]hexylene group, a bicyclo[3.2.0]heptylene group, a bicyclo[2.2.1]heptylene group, a bicyclo[3.3.0]octylene group, a bicyclo[3.2.1]octylene group, a bicyclo[2.2.2]octylene group, a bicyclo[6.1.0]nonylene group, and a bicyclo[4.4.0]decylene group.

The term of "cycloalkenylene group" as described herein refers to a group constituting the linker chain represented by L and $L^1$ in the above general formula (I) and (II) respectively, and means a divalent group formed by removing any one hydrogen atom from the above-defined "cycloalkenyl group". Also, when said cycloalkenyl group has substituent(s), the term of "cycloalkenylene group" means a divalent group formed by removing any one additional hydrogen atom from a cycloalkenyl ring carbon atom other than said substituent(s).

Examples of the substituent of an optionally substituted cycloalkenylene group include one or more substituent(s) independently selected from the Group B.

Specific examples of said cycloalkenylene group include monocyclic cycloalkenylene groups having 3 to 10 carbon atoms such as a 1-cyclopropenylene group, a 1-cyclobutenylene group, a 1-cyclopentenylene group, a 2-cyclopentenylene group, a 1-cyclohexenylene group, a 2-cyclohexenylene group, a 3-cyclohexenylene group, a 1, 3-cyclohexadienylene group, a 1,4-cyclohexadienylene group, a 1-cycloheptenylene group, a 2-cyclooctenylene group, a 3-cyclooctenylene group, a 4-cyclooctenylene group, a 1-cyclononenylene group, and a 1-cyclodecenylene group; and bicyclic cycloalkenyl groups having 4 to 10 carbon atoms such as a bicyclo[1.1.0]but-1-enylene group, a bicyclo[2.1.0]pent-2-enylene group, a bicyclo[3.1.0]hex-2-enylene group, a bicyclo[3.2.0]hept-3-enylene group, a bicyclo[2.2.1]hept-2-enylene group, a bicyclo[3.3.0]oct-2-enylene group, a bicyclo[3.2.1]oct-2-enylene group, a bicyclo[2.2.2]oct-2-enylene group, a bicyclo[6.1.0]non-4-enylene group, and a bicyclo[4.4.0]dec-3-enylene group.

The term of "arylene group" as described herein refers to a group constituting the linker chain represented by L and $L^1$ in the above general formula (I) and (II) respectively, and means a divalent group formed by removing any one hydrogen atom from the above-defined "aryl group". Also, when said aryl group has substituent(s), the term of "arylene group" means a divalent group formed by removing any one additional hydrogen atom from an aryl ring carbon atom other than said substituent(s).

Examples of the substituent of an optionally substituted arylene group include one or more substituent(s) independently selected from the Group C.

Specific examples of said arylene group include monocyclic arylene groups having 6 to 11 ring carbon atoms such as a phenylene group; and optionally partially saturated bicyclic arylene groups having 9 to 11 ring carbon atoms such as a naphthylene group, a tetrahydronaphthylene group, an indenylene group, and an indanylene group.

The term of "heteroarylene group" as described herein refers to a group constituting the linker chain represented by L and $L^1$ in the above general formula (I) and (II) respectively, and means a divalent group formed by removing any one hydrogen atom from the above defined "heteroaryl group". Also, when said heteroarylene group has substituent(s), the term of "heteroarylene group" means a divalent group formed by removing any one additional hydrogen atom from a heteroaryl ring atom other than said substituent(s).

Examples of the substituent of an optionally substituted heteroarylene group include one or more substituent(s) independently selected from the Group C.

Specific examples of said heteroarylene group include 5 to 6 membered monocyclic heteroarylene groups comprising 1 to 4 heteroatom(s) selected from an oxygen atom, a sulfur atom, and a nitrogen atom other than carbon atom(s) such as a pyrrolylene group, a furylene group, a thienylene group, a pyrazolylene group, an imidazolylene group, an oxazolylene group, an isoxazolylene group, a thiazolylene group, an isothiazolylene group, a thiadiazolylene group, a triazolylene group, a tetrazolylene group, a pyridylene group, a pyrazinylene group, a pyrimidinylene group, a pyridazinylene group, and a triazinylene group; and 8 to 11 membered bicyclic heteroarylene groups comprising 1 to 4 heteroatom(s) selected from an oxygen atom, a sulfur atom, and a nitrogen atom other than carbon atom(s) such as an indolylene group, an isoindolylene group, an indazolylene group, a tetrahydroindazolylene group, a benzofuranylene group, a dihydrobenzofuranylene group, a dihydroisobenzofuranylene group, a benzothiophenylene group, a dihydrobenzothiophenylene group, a dihydroisobenzothiophenylene group, a benzoxazolylene group, a dihydrobenzoxazolylene group, a benzothiazolylene group, a dihydrobenzothiazolylene group, a quinolylene group, a tetrahydroquinolylene group, an isoquinolylene group, a tetrahydroisoquinolylene group, a naphthyridinylene group, a tetrahydronaphthyridinylene group, a quinoxalinylene group, a tetrahydroquinoxalinylene group, and a quinazolinylene group.

The term of "heterocyclylene group" as described herein refers to a group constituting the linker chain represented by L and $L^1$ in the above general formula (I) and (II) respectively, and means a divalent group formed by removing any one hydrogen atom from the above defined "heterocyclyl group".

Examples of the substituent of an optionally substituted heterocyclylene group include one or more substituent(s) independently selected from the Group D.

Specific examples of said heterocyclylene group include an azetidinylene group, an oxetanylene group, a thietanylene group, a pyrrolidinylene group, a piperidinylene group, a piperidinylene group, a tetrahydrofurylene group, a tetrahydropyranylene group, a tetrahydrothienylene group, a piperazinylene group, a morpholinylene group, a perhydroazepinylene group, an azacyclooctylene group, an azacyclooct-3-enylene group, an azacyclooct-4-enylene group, an azacyclononylene group, an azacyclodecylene group, 6 to 12 membered azabicycloalkylene groups (for example, an azabicyclohexylene group, an azabicycloheptylene group, an azabicyclooctylene group, an azabicyclononylene group, an azabicyclodecylene group, an azabicycloundecylene group, or an azabicyclododecylene group), 6 to 12 membered azabicycloalkenylene groups (for example, an azabicyclohexenylene group, an azabicycloheptenylene group, an azabicyclooctenylene group, an azabicyclononenylene group, an azabicyclodecenylene group, an azabicycloundecenylene group, or an azabicyclododecenylene group), 6 to 12 membered azaspiroalkylene groups (for example, an azaspirohexylene group, an azaspiroheptylene group, an azaspirooctylene group, an azaspirononylene group, an azaspirodecylene group, an azaspiroundecylene group, or an azaspirododecylene group).

Said heterocyclylene group is optionally fused to one or more above aryl ring(s) and/or above heteroaryl ring(s) to form a bicyclic to tetracyclic heterocyclylene group.

Also, when said alkyl group, said alkenyl group, said alkynyl group, said cycloalkyl group, said cycloalkenyl group, said aryl group, said heteroaryl group, said heterocyclyl group, said alkylene group, said alkenylene group, said alkynylene group, said cycloalkylene group, said cycloalkenylene group, said arylene group, said heteroarylene group, and said heterocyclylene group have functional group(s) such as a hydroxy group, a sulfhydryl group, an amino group ($-NH_2$ and -NH- (also including -NH-in the ring of an heteroaryl group and a heterocyclyl group)), a carboxy group, a sulfenic acid group, a sulfinic acid group, a sulfonic acid group, a phosphinic acid group, a phosphonic acid group, or a boric acid group as substituent(s), said group(s) is/are optionally protected by the above protecting group(s) defined in relation to "an antitumor drug molecule or an analog thereof, or a derivative thereof".

Examples of the "salt" as described herein include salts with alkali metals such as lithium, sodium, and potassium; salts with alkaline earth metals such as magnesium and calcium; a salt with aluminum or zinc; salts with amines such as ammonia, choline, diethanolamine, lysine, ethylenediamine, tert-butylamine, tert-octylamine, tris(hydroxymethyl)aminomethane, N-methyl-glucosamine, triethanolamine, and dehydroabietylamine; salts with inorganic acids such as hydrogen chloride, hydrogen bromide, hydrogen iodide, sulfuric acid, nitric acid, and phosphoric acid; salts with organic acids such as formic acid, acetic acid, trifluoroacetic acid, propionic acid, oxalic acid, malonic acid, succinic acid, fumaric acid, maleic acid, lactic acid, malic acid, tartaric acid, citric acid, methanesulfonic acid, ethanesulfonic acid, and benzenesulfonic acid; and salts with acidic amino acids such as aspartic acid and glutamic acid. Further, the term of "salt" also includes intramolecular salts.

Further, all of the conjugate precursor (I) and the synthetic intermediate (II) of conjugate precursor, and a salt thereof of the present invention may be obtained as a hydrate or solvate, and the present invention encompasses all of

them.

Specific examples of the conjugate precursor (I) of the present invention are shown in the following Exemplification 2, and specific examples of the synthetic intermediate (II) of conjugate precursor of the present invention are shown in the following Exemplification 3, but the present invention is not limited to them.

Exemplification 2. Exemplification of "conjugate precursor (I)"

Exemplification 2-1

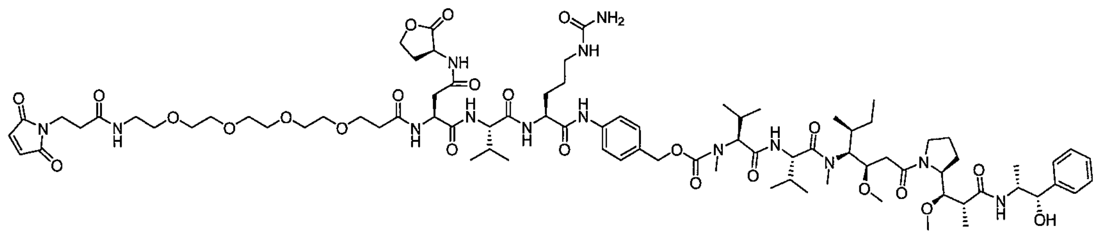

Exemplification 2-2

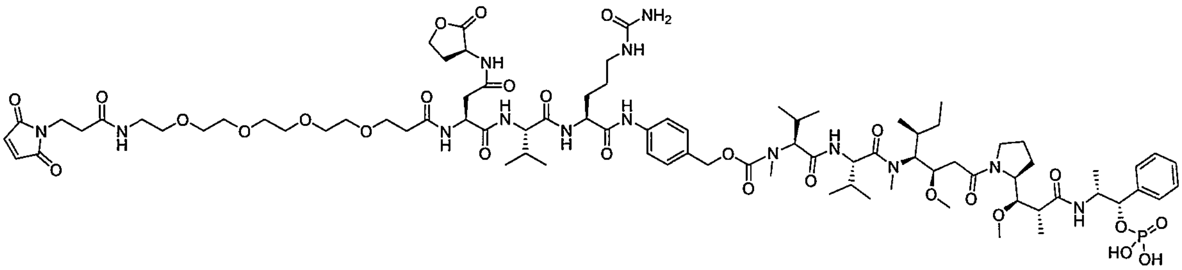

Exemplification 2-3

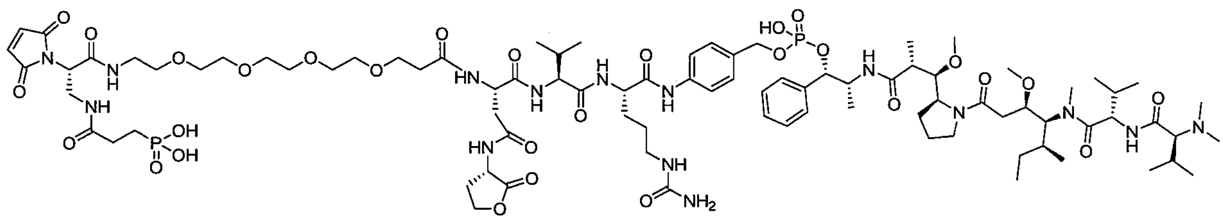

Exemplification 2-4

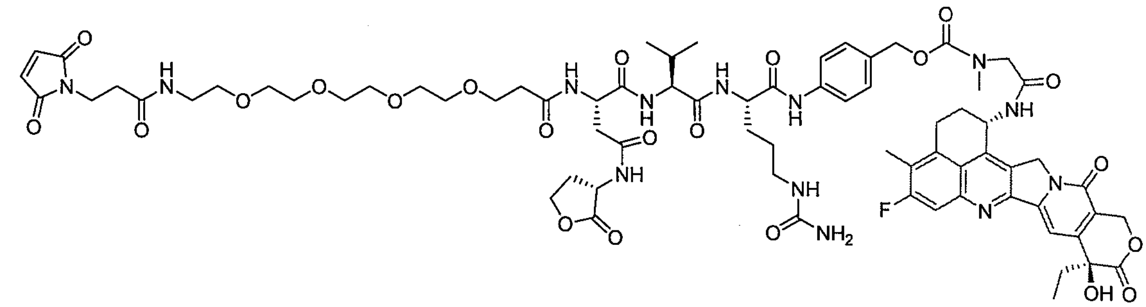

Exemplification 2-5

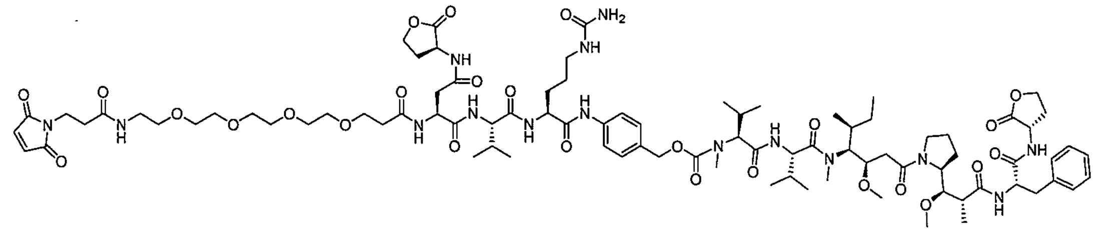

Exemplification 2-6

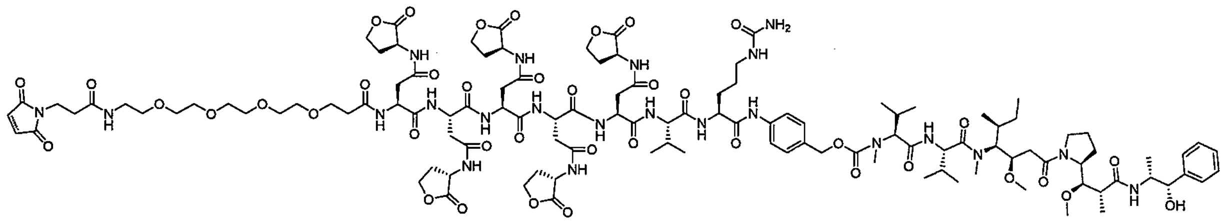

Exemplification 2-7

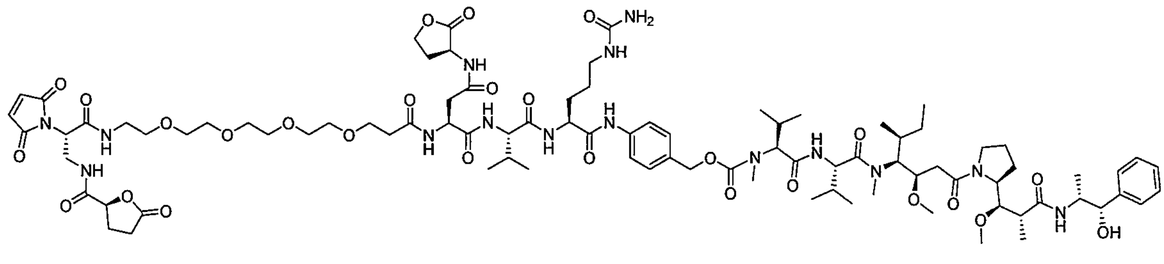

Exemplification 2-8

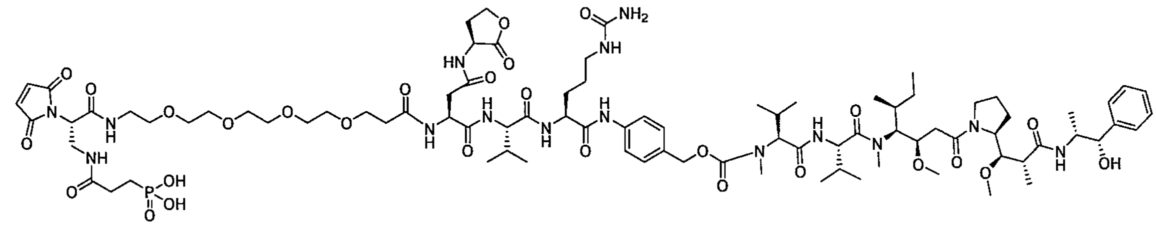

Exemplification 2-9

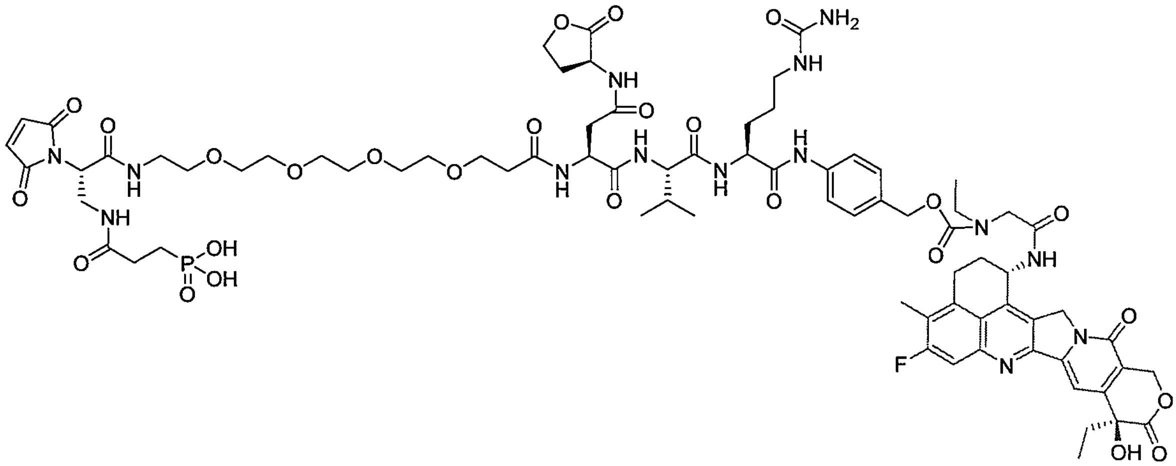

Exemplification 2-10

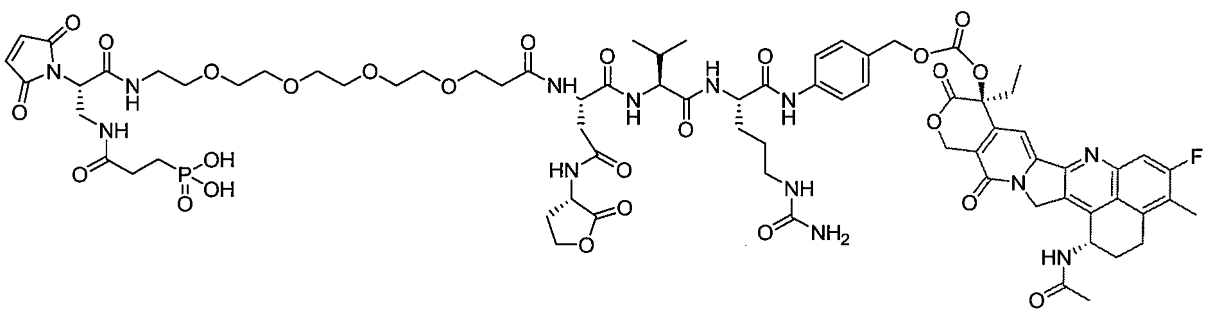

Exemplification 2-11

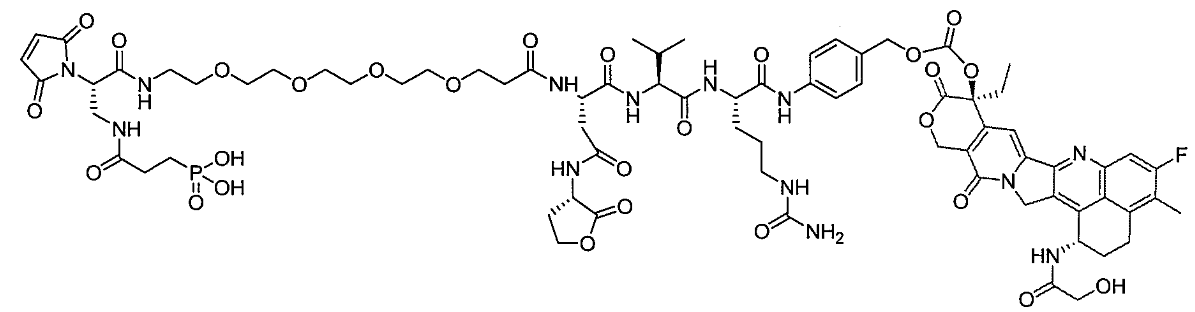

Exemplification 2-12

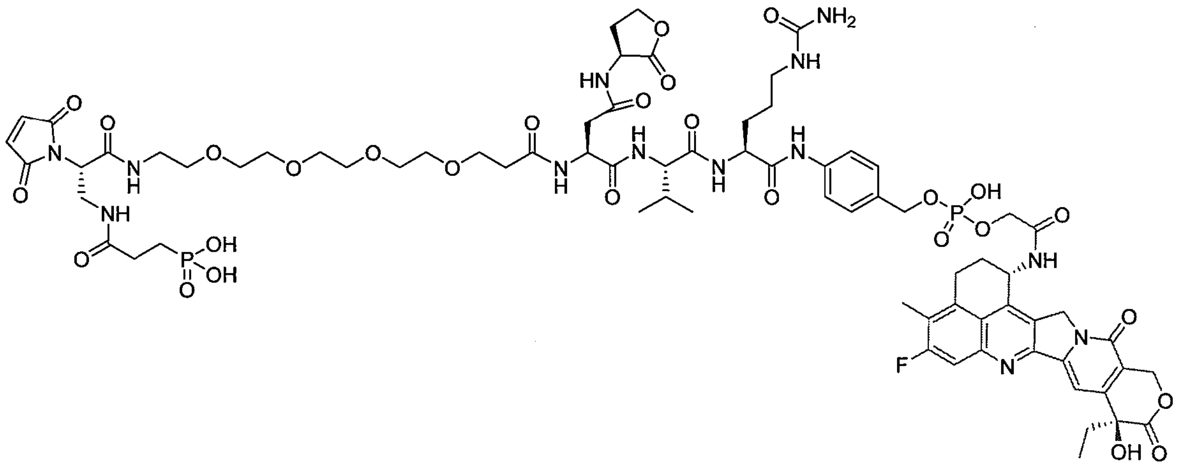

Exemplification 2-13

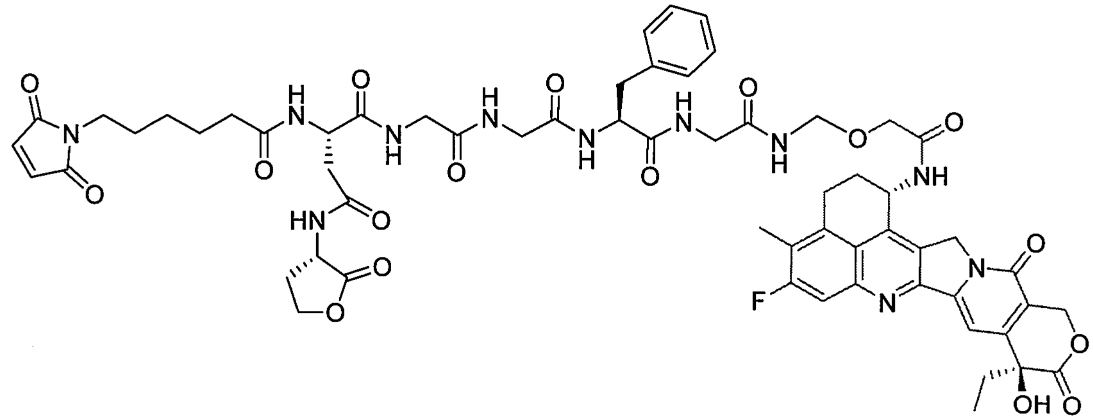

Exemplification 2-14

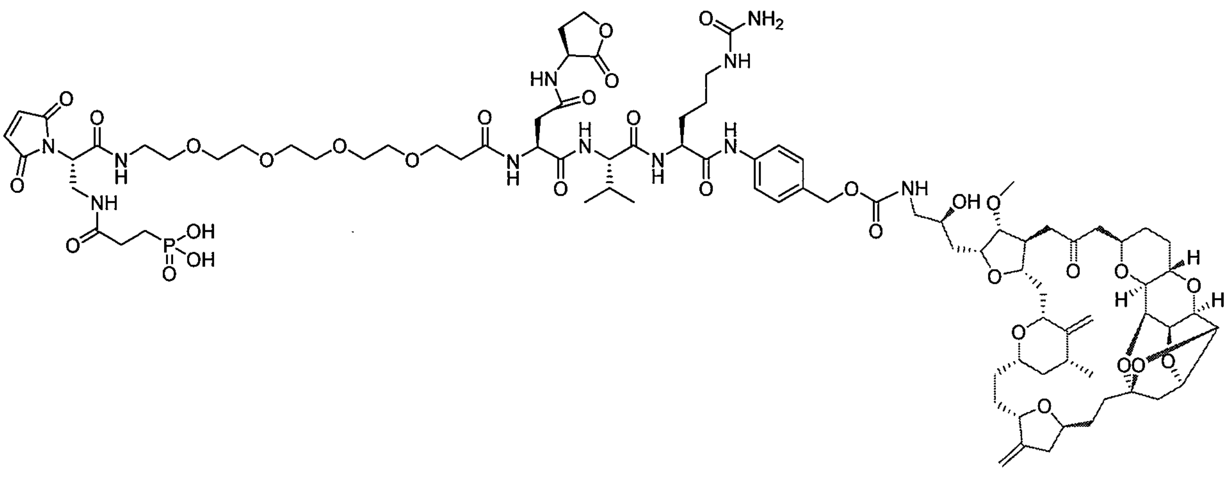

Exemplification 2-15

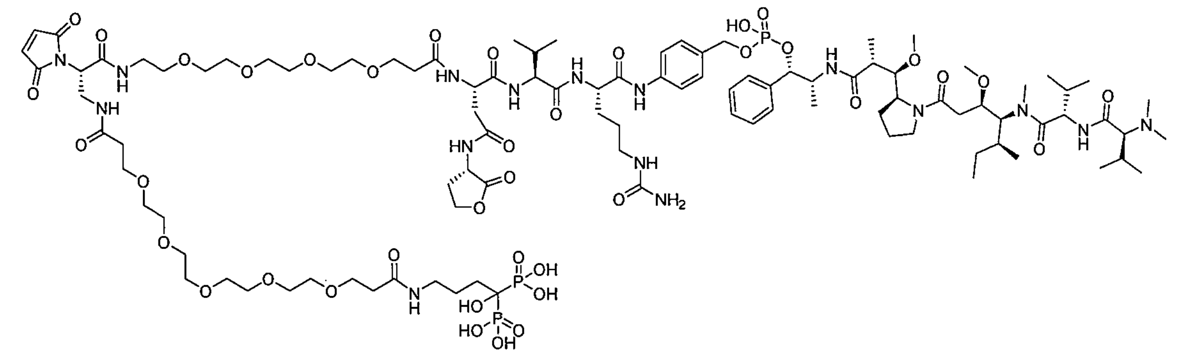

Exemplification 2-16

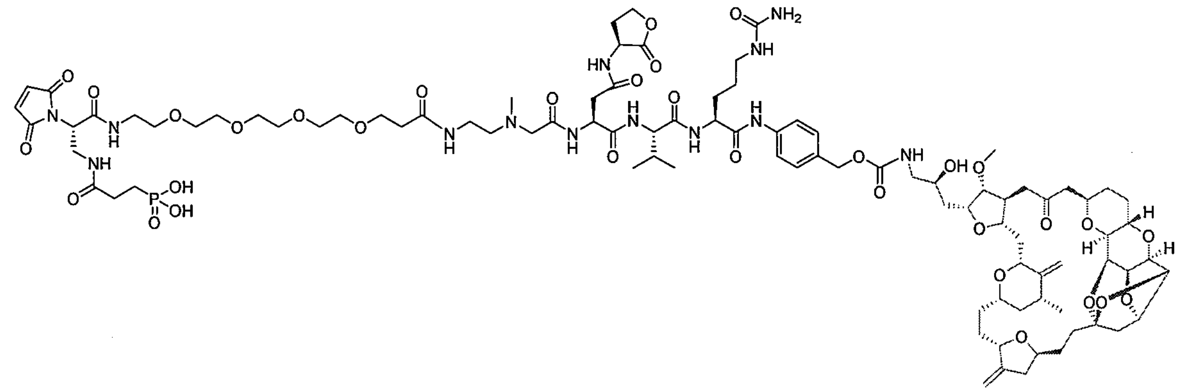

Exemplification 2-17

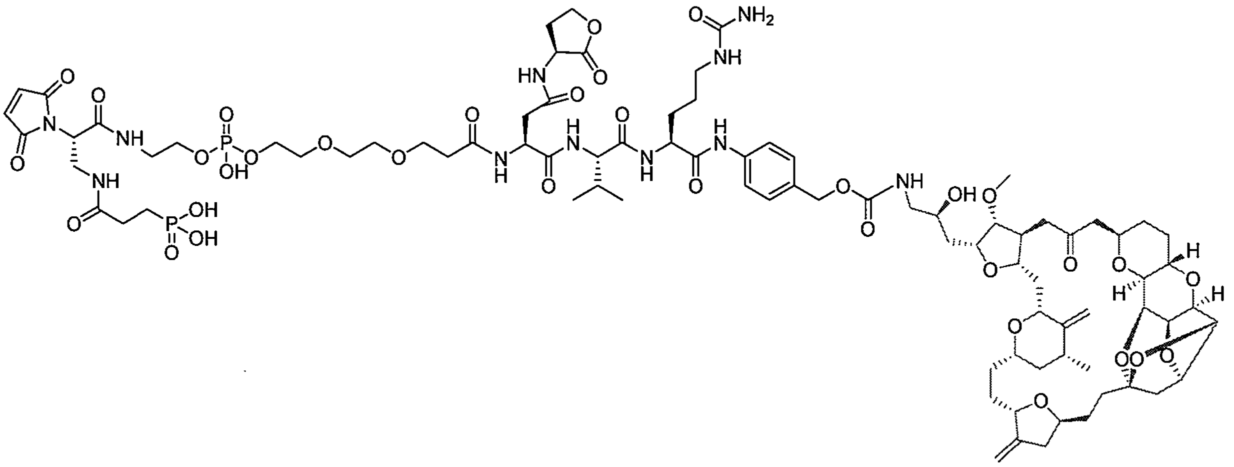

Exemplification 2-18

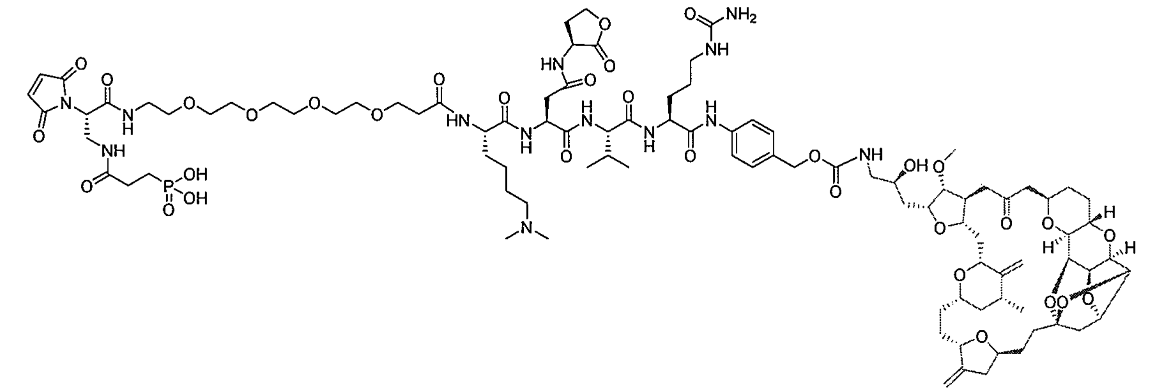

Exemplification 2-19

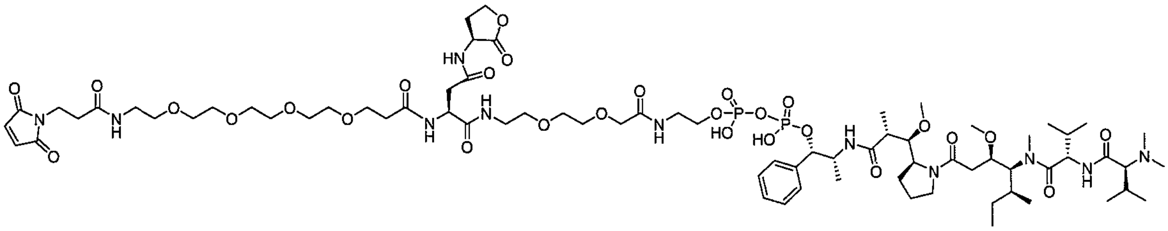

Exemplification 2-20

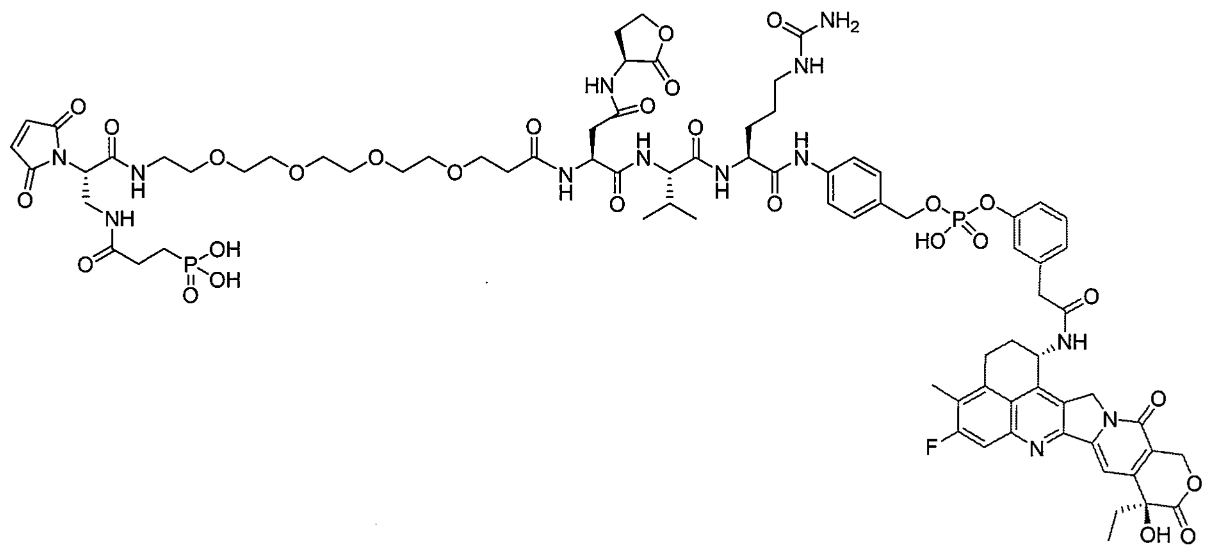

Exemplification 2-21

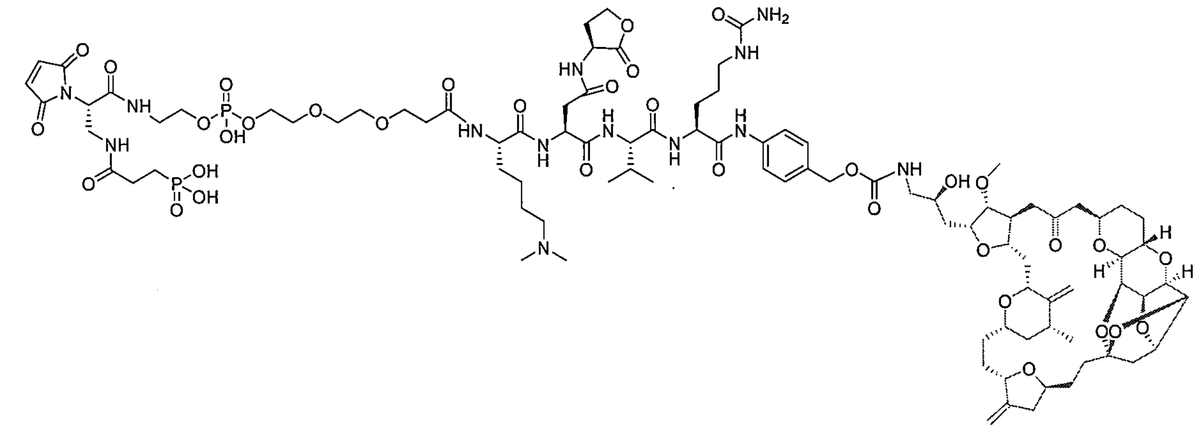

Exemplification 2-22

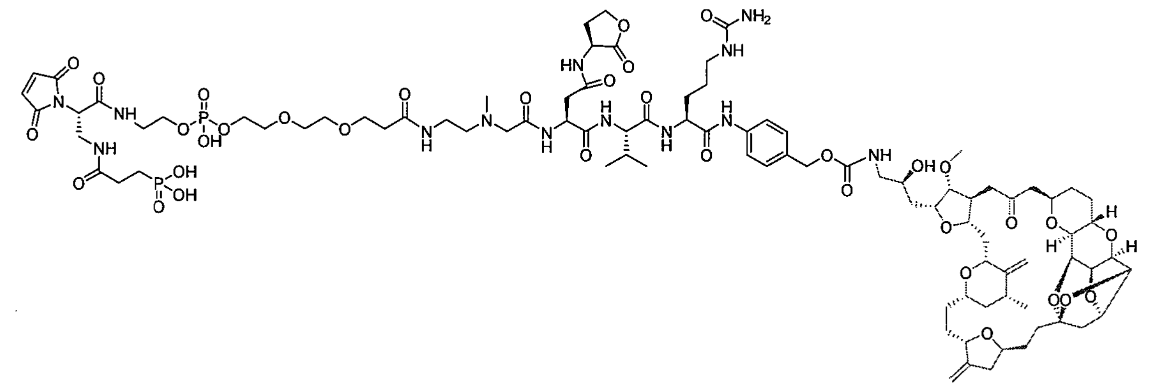

Exemplification 2-23

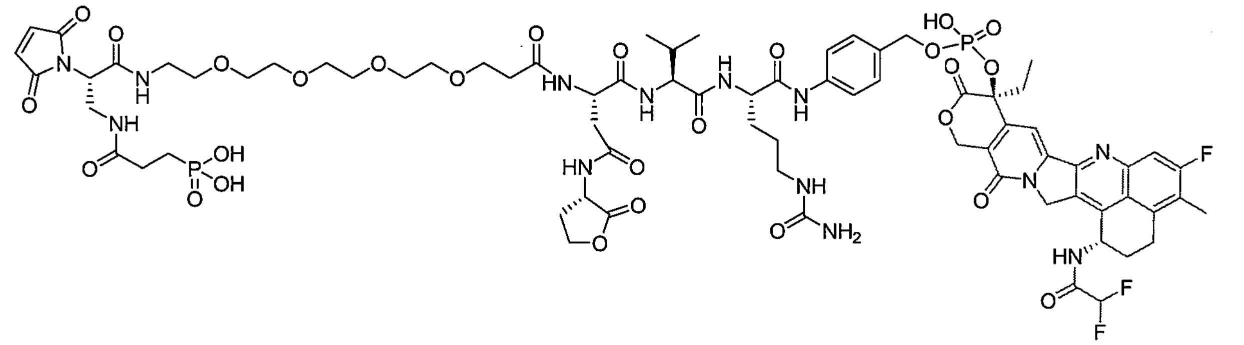

Exemplification 2-24

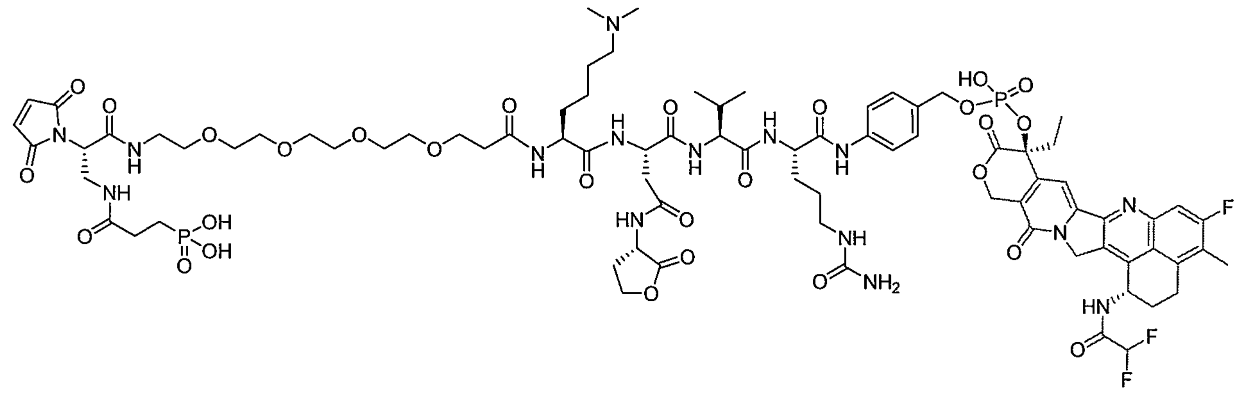

Exemplification 2-25

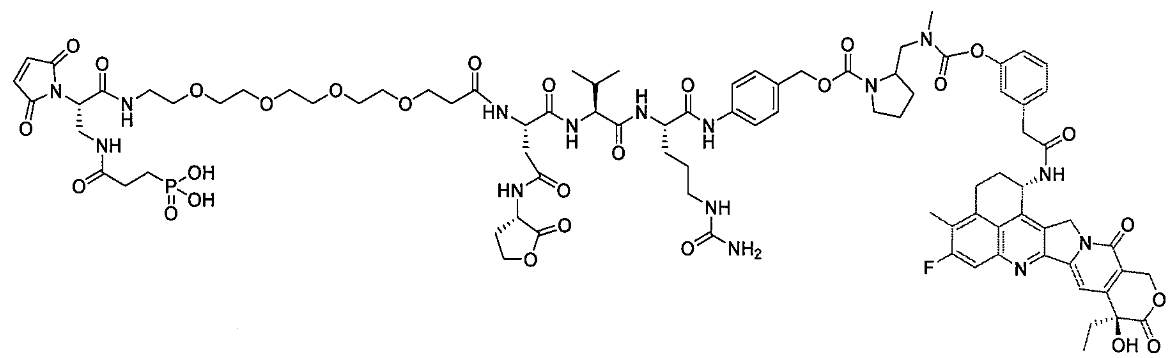

Exemplification 2-26

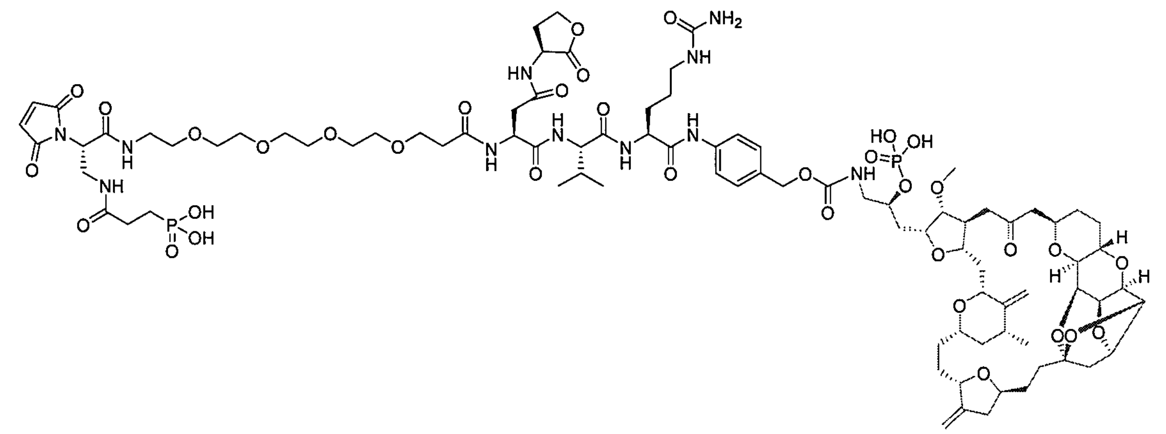

Exemplification 2-27

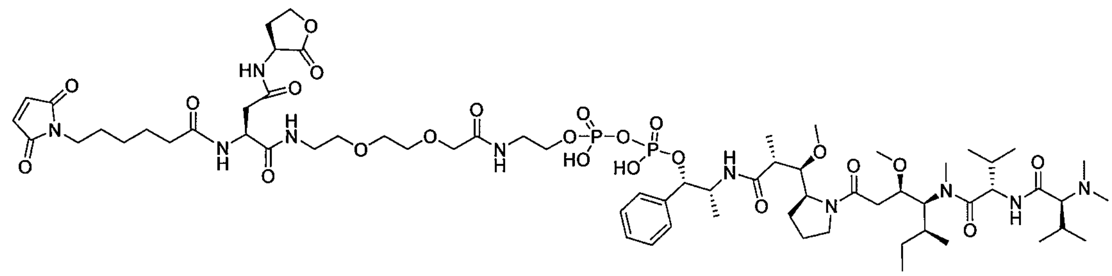

Exemplification 2-28

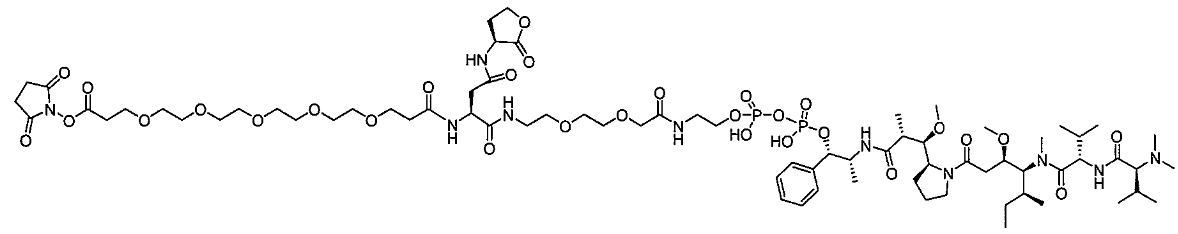

Exemplification 2-29

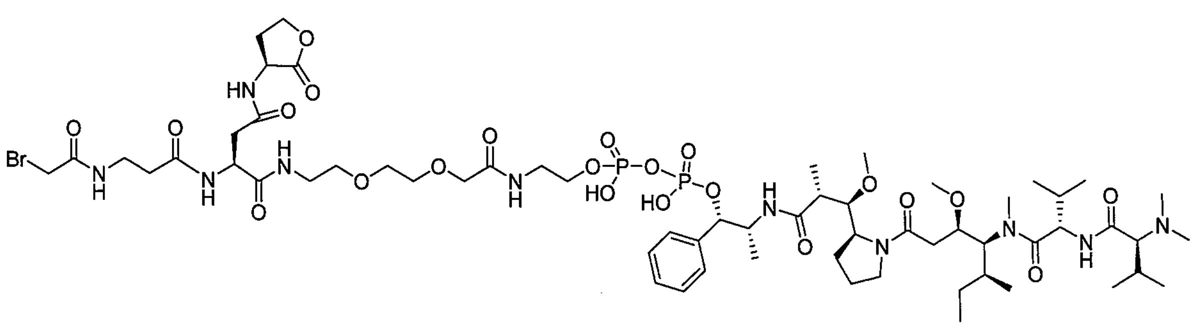

Exemplification 2-30

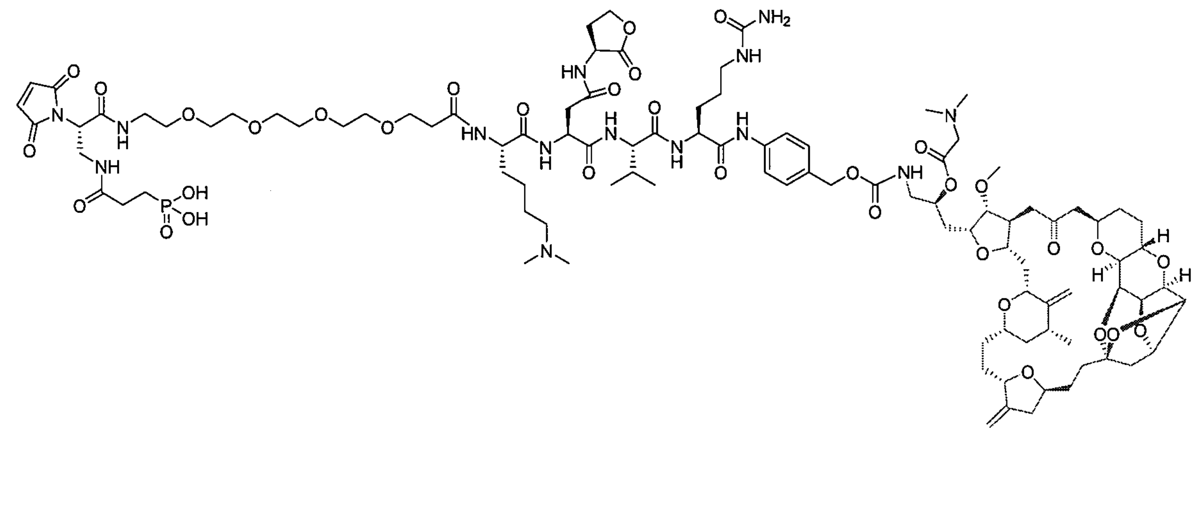

Exemplification 2-31

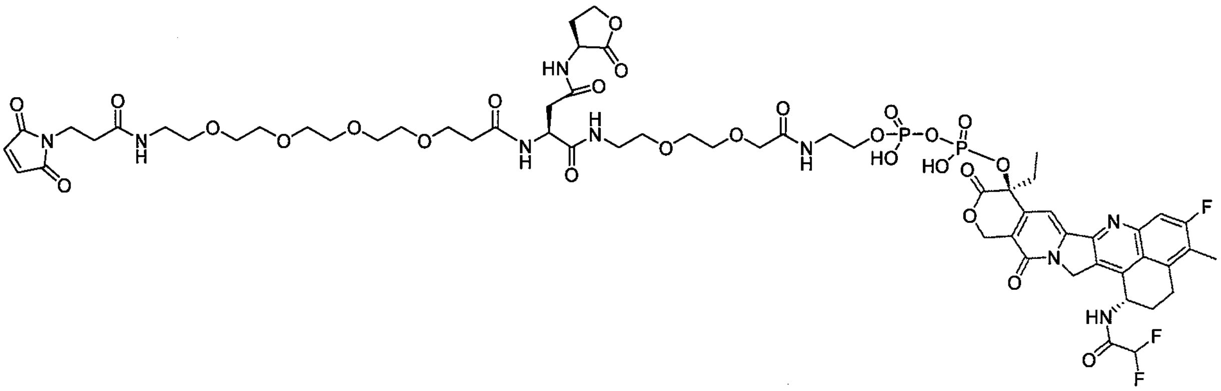

Exemplification 2-32

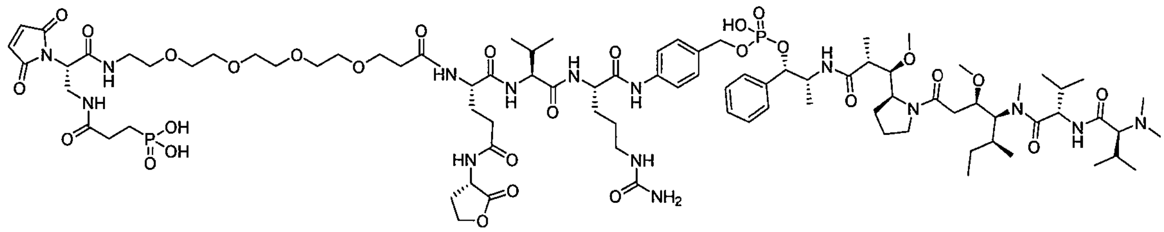

Exemplification 2-33

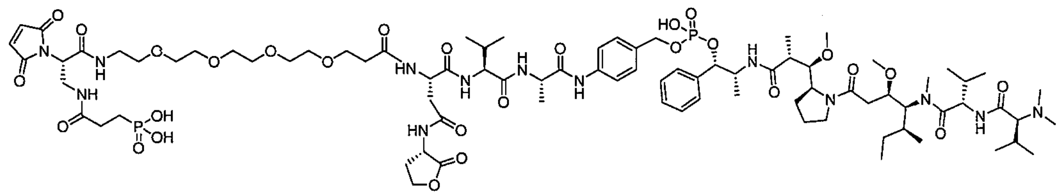

Exemplification 2-34

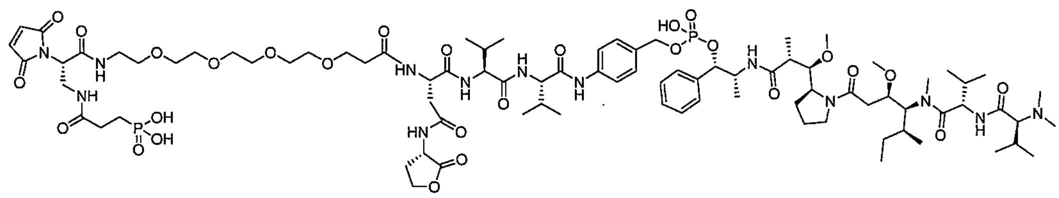

Exemplification 2-35

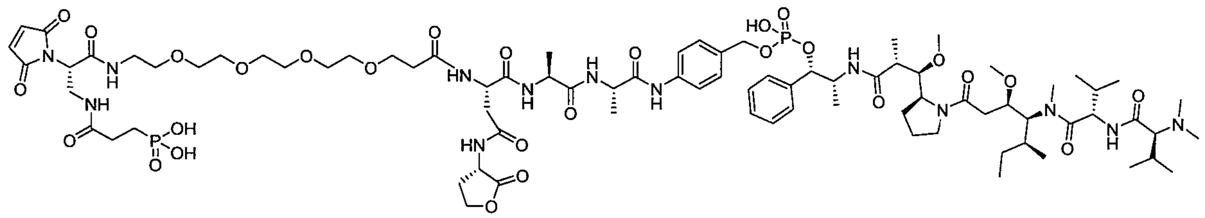

Exemplification 2-36

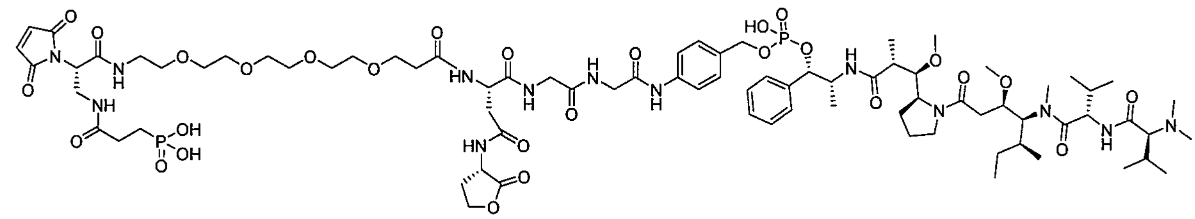

Exemplification 2-37

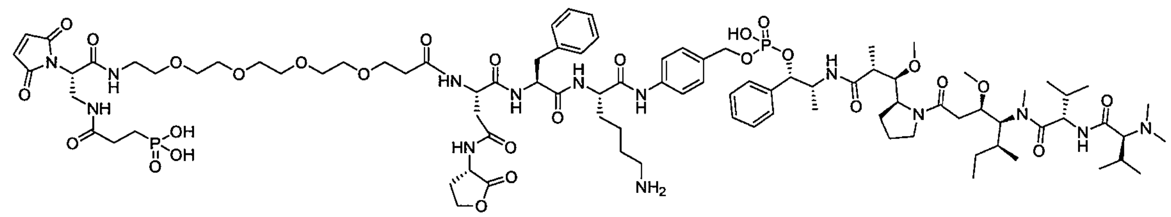

Exemplification 2-38

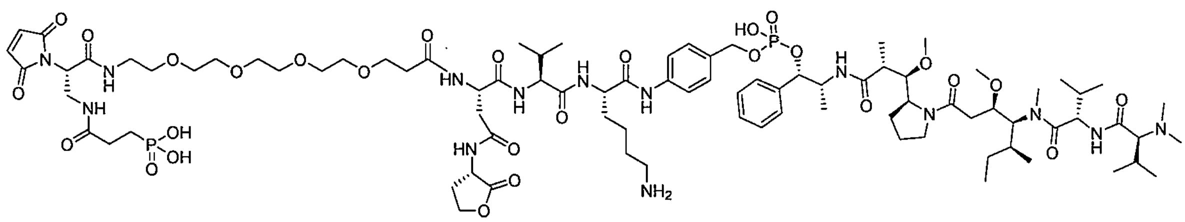

Exemplification 2-39

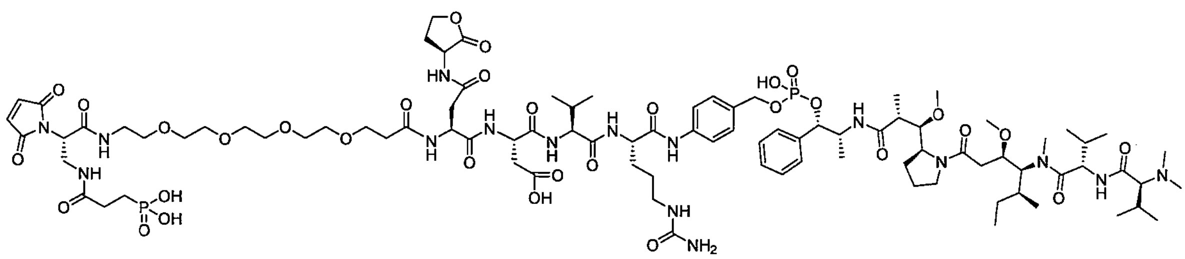

Exemplification 2-40

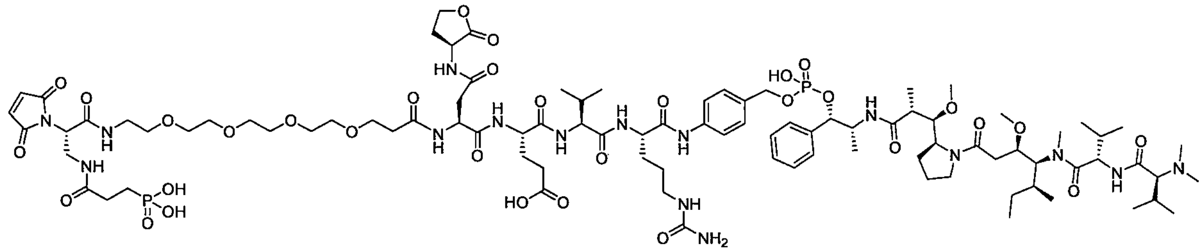

Exemplification 2-41

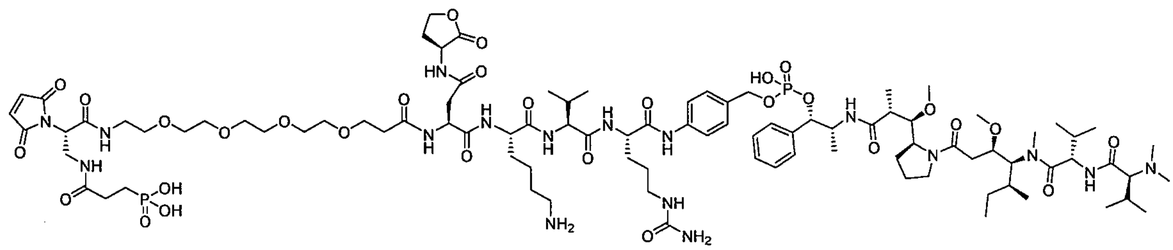

Exemplification 2-42

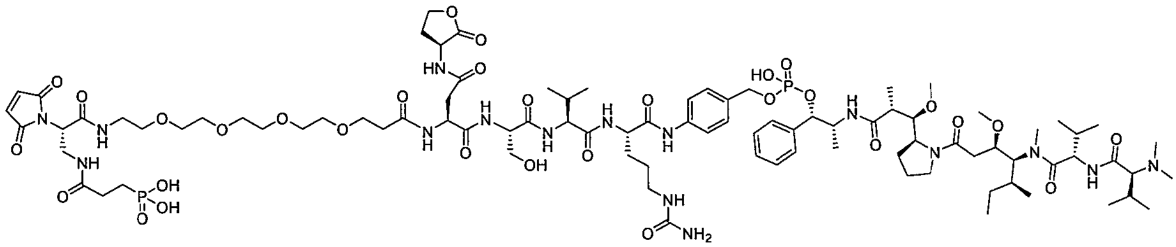

Exemplification 2-43

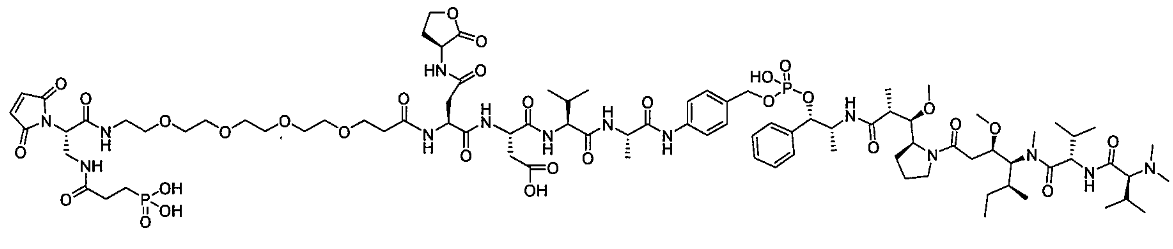

Exemplification 2-44

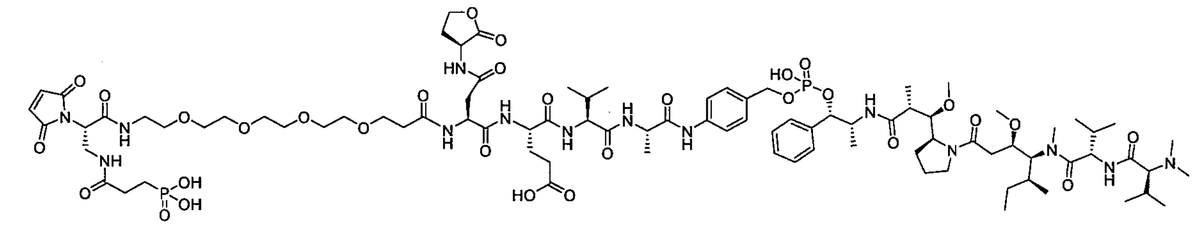

Exemplification 2-45

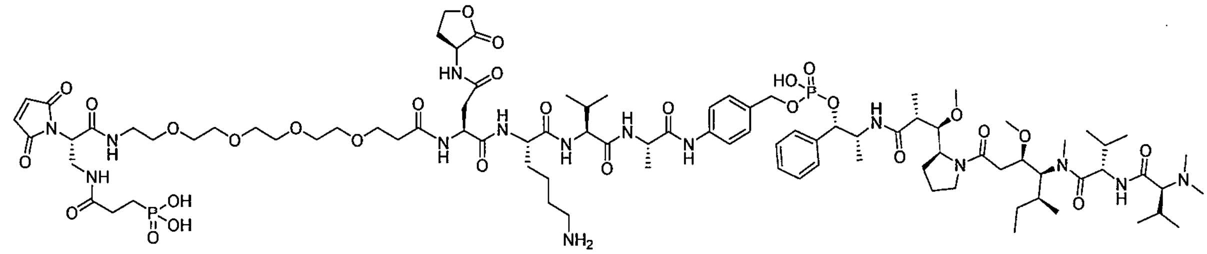

Exemplification 2-46

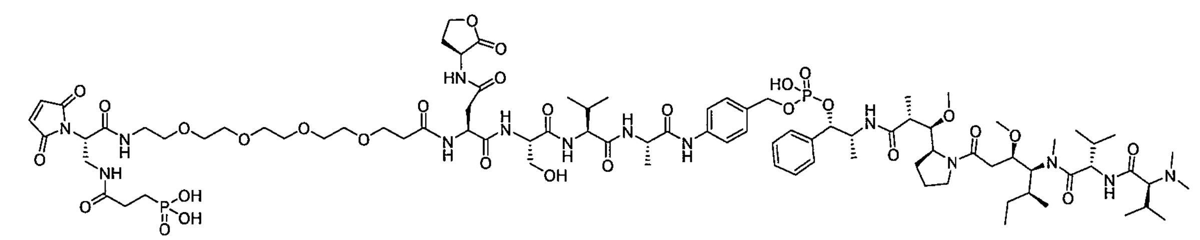

Exemplification 2-47

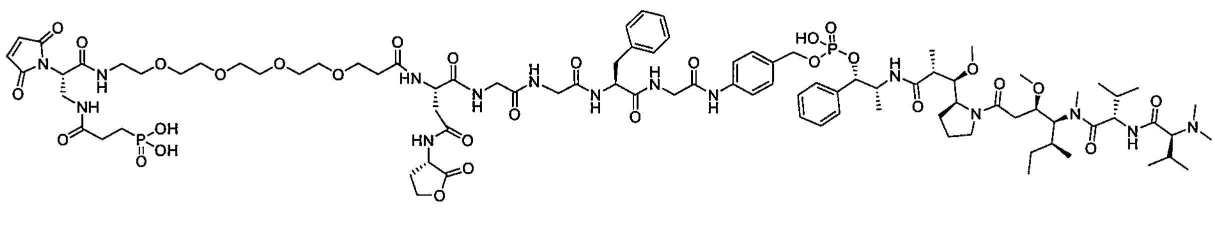

Exemplification 2-48

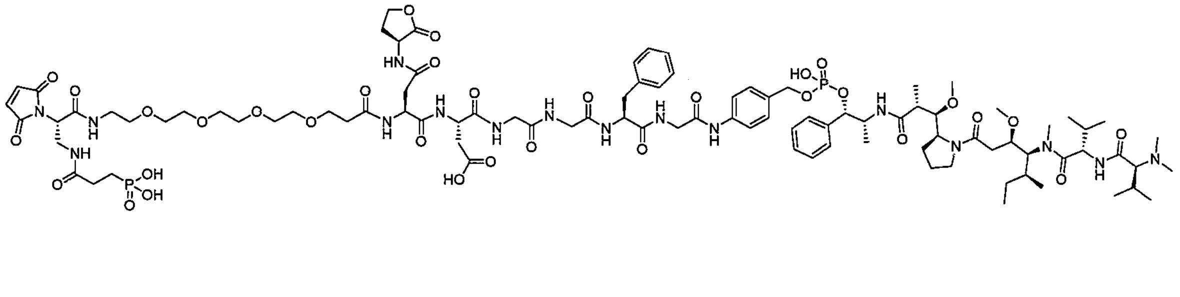

Exemplification 2-49

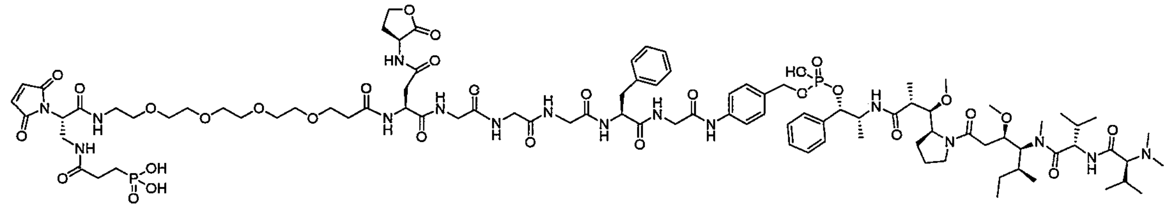

Exemplification 2-50

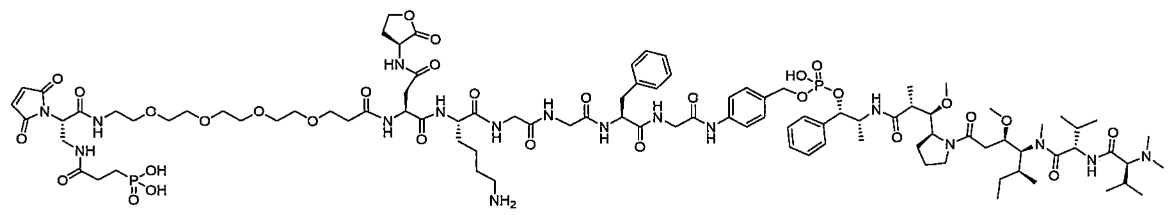

Exemplification 2-51

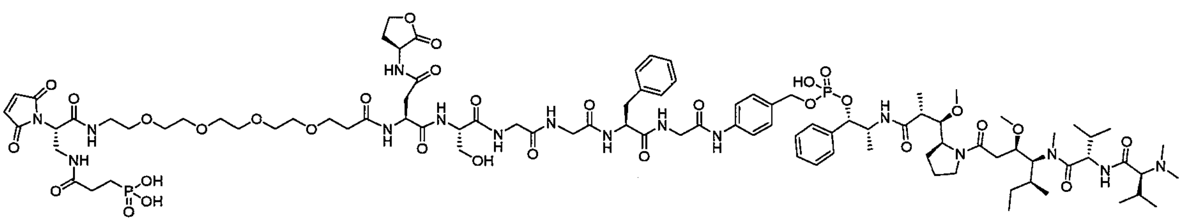

Exemplification 2-52

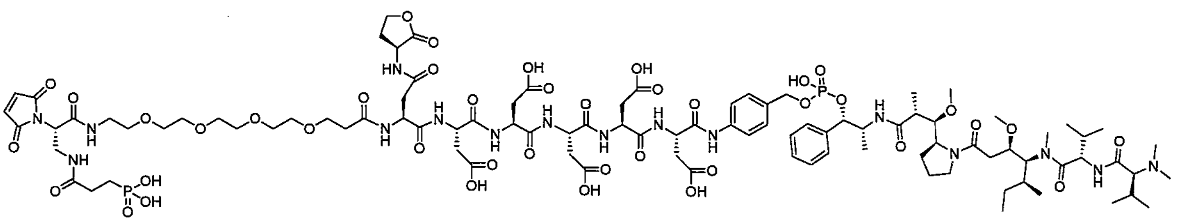

Exemplification 2-53

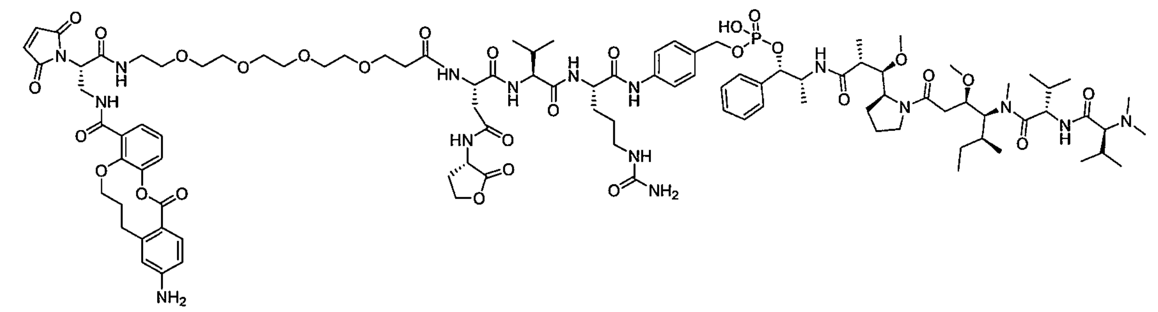

Exemplification 2-54

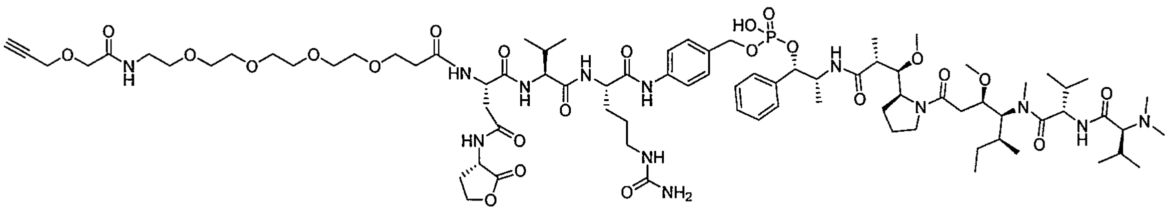

Exemplification 2-55

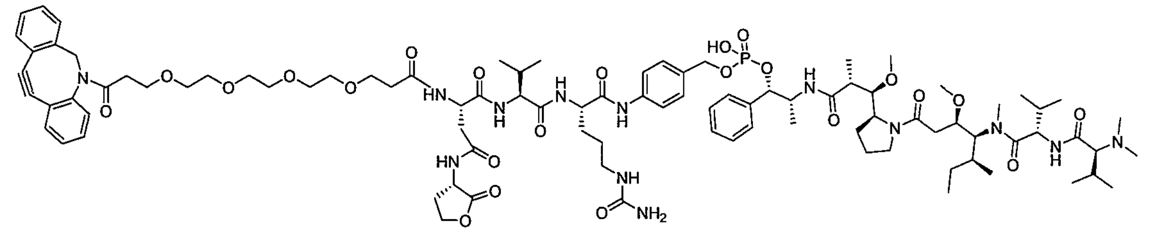

Exemplification 2-56

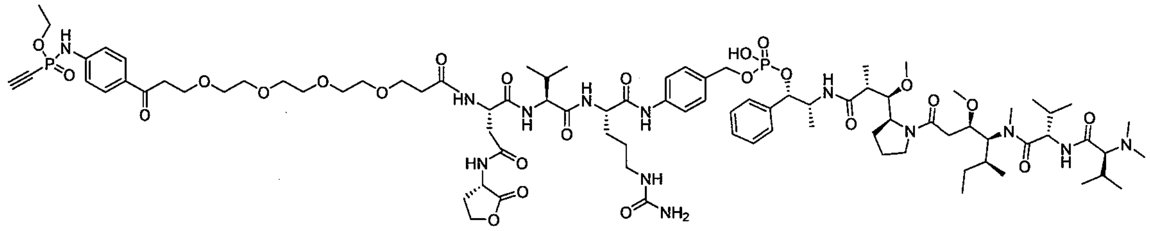

Exemplification 2-57

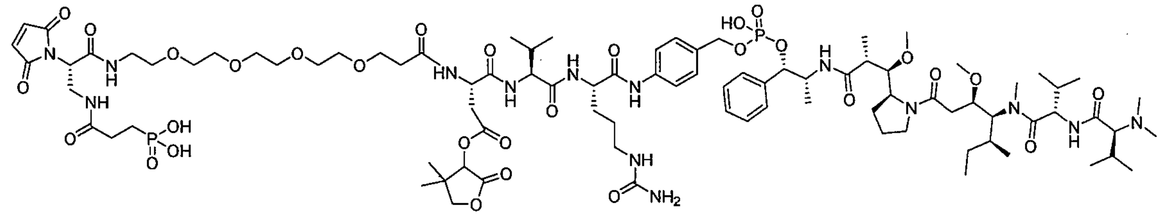

Exemplification 2-58

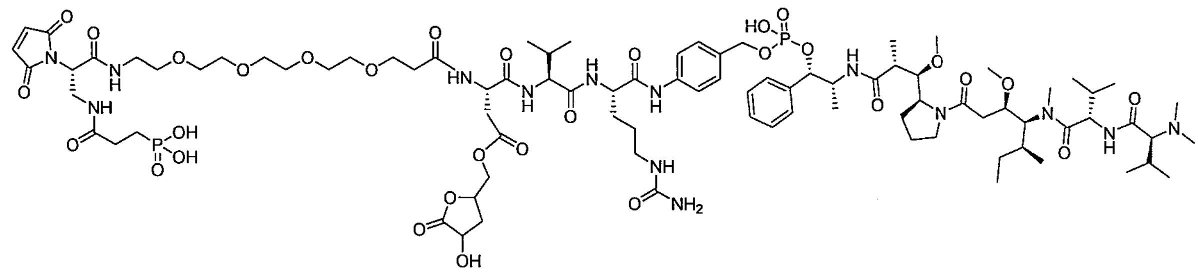

Exemplification 2-59

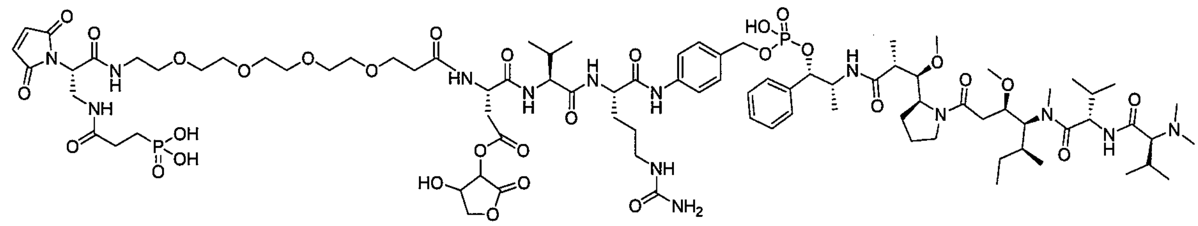

Exemplification 2-60

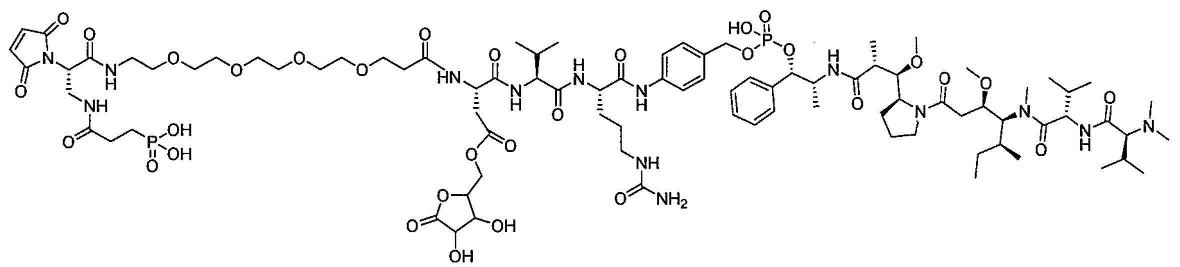

Exemplification 2-61

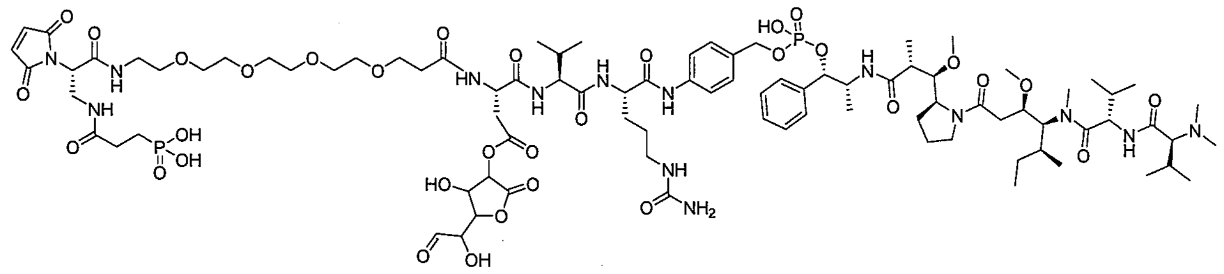

Exemplification 2-62

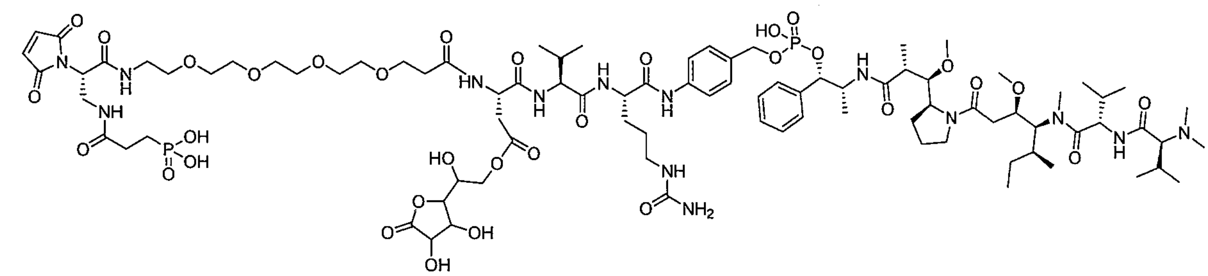

Exemplification 2-63

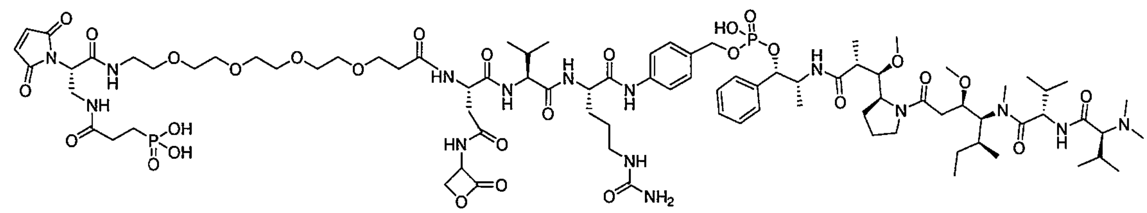

Exemplification 2-64

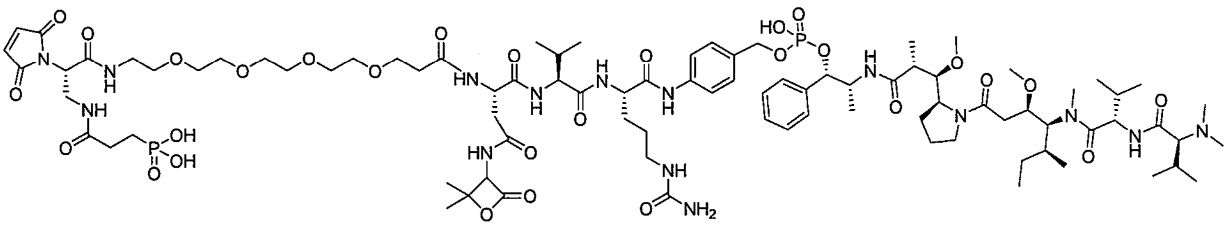

Exemplification 2-65

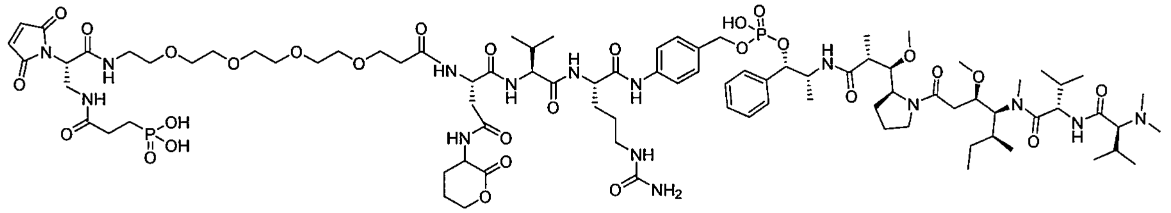

Exemplification 2-66

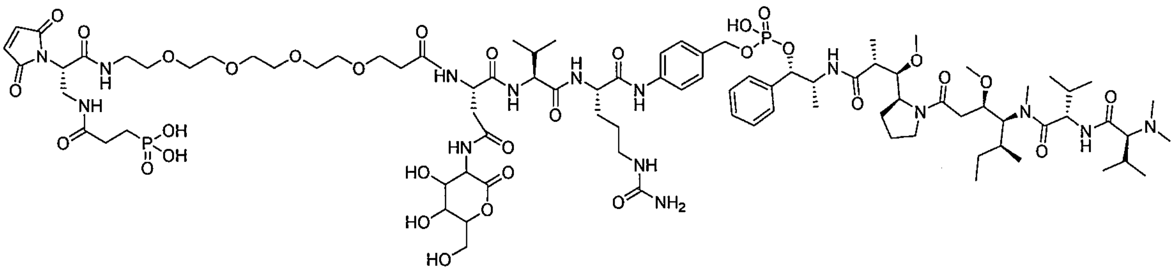

Exemplification 2-67

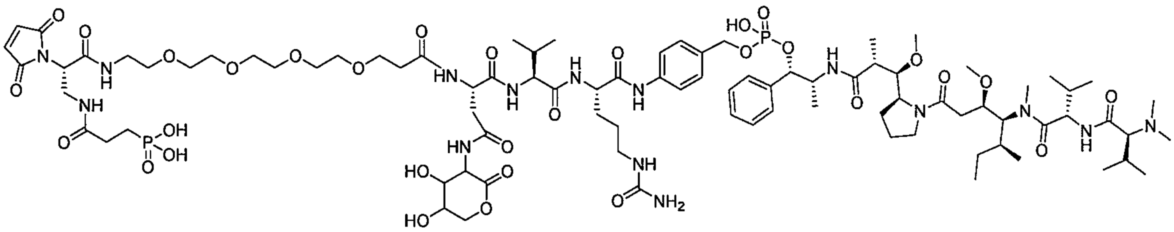

Exemplification 2-68

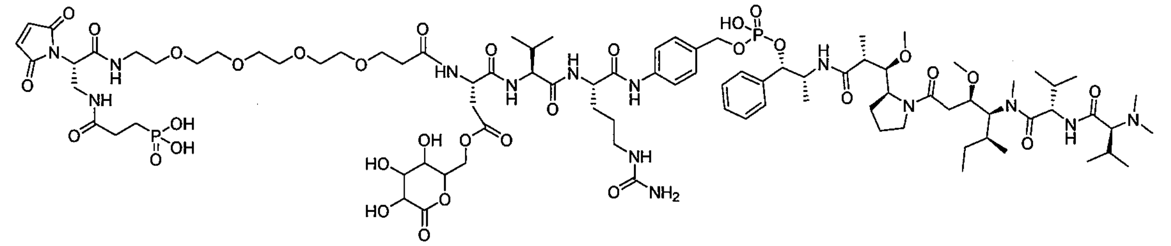

Exemplification 2-69

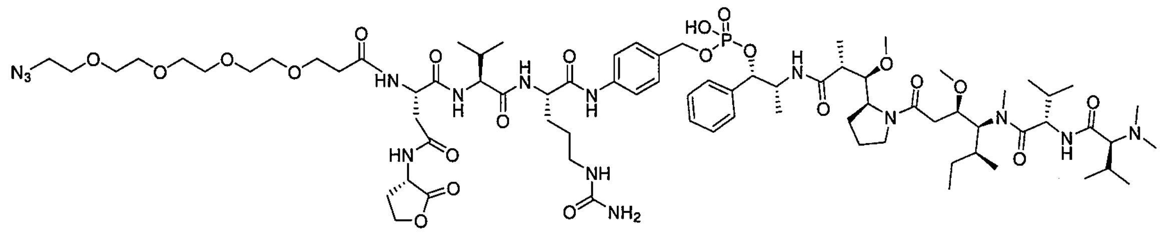

Exemplification 3. Exemplification of "synthetic intermediate (II) of conjugate precursor"

Exemplification 3-1-1

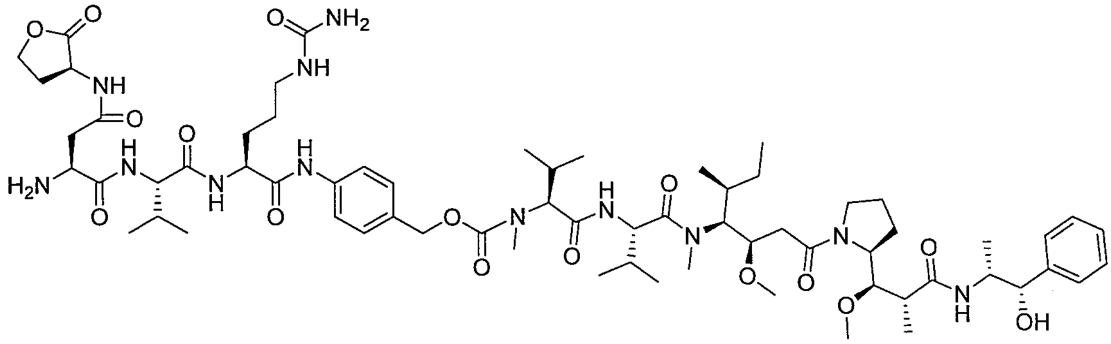

Exemplification 3-1-2

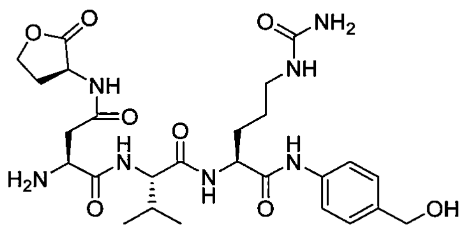

Exemplification 3-1-3

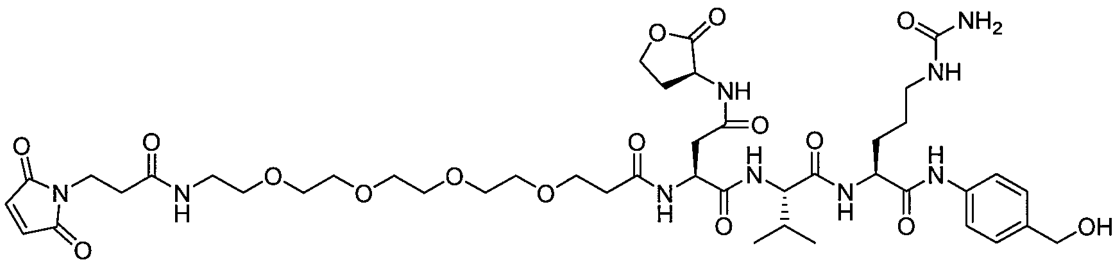

Exemplification 3-2-1

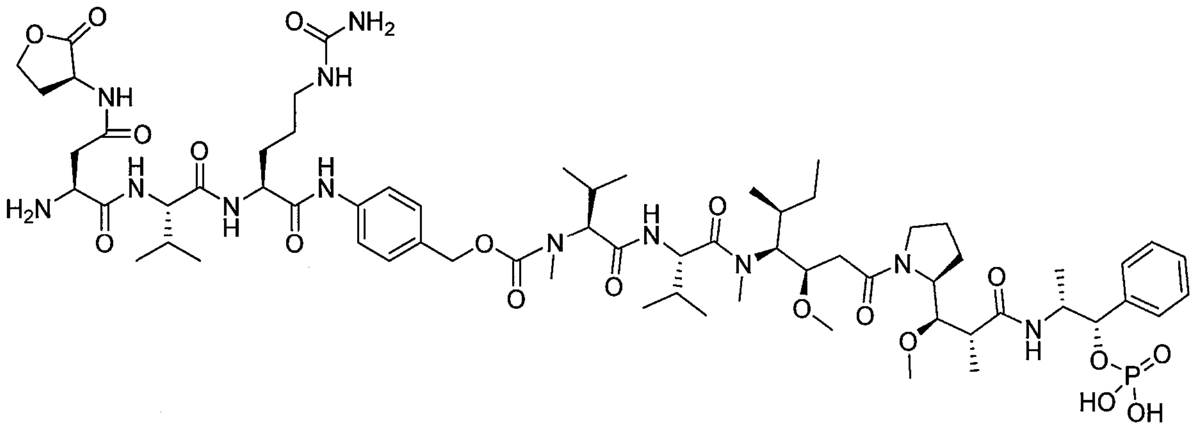

Exemplification 3-3-1

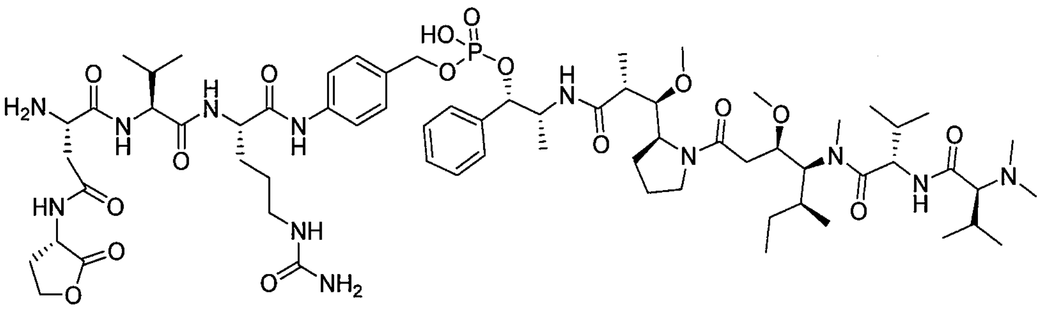

Exemplification 3-3-3

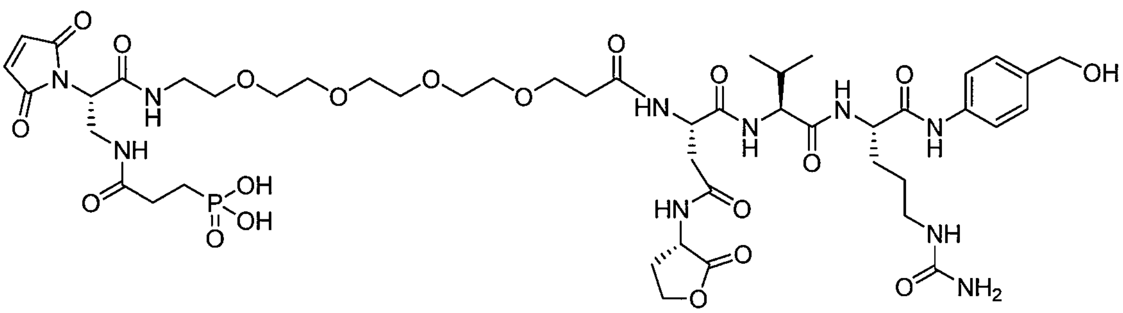

Exemplification 3-4-1

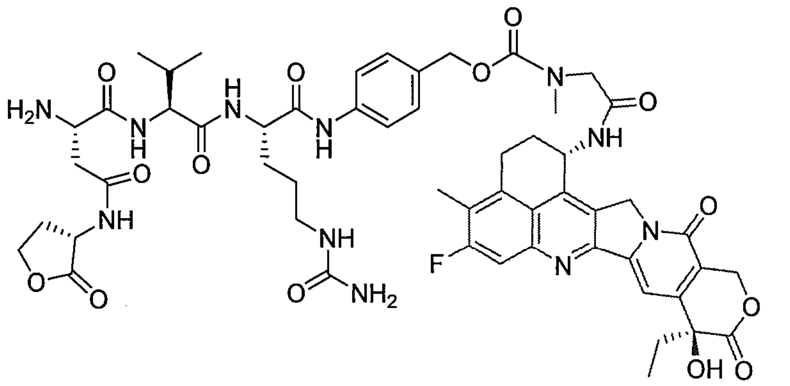

Exemplification 3-5-1

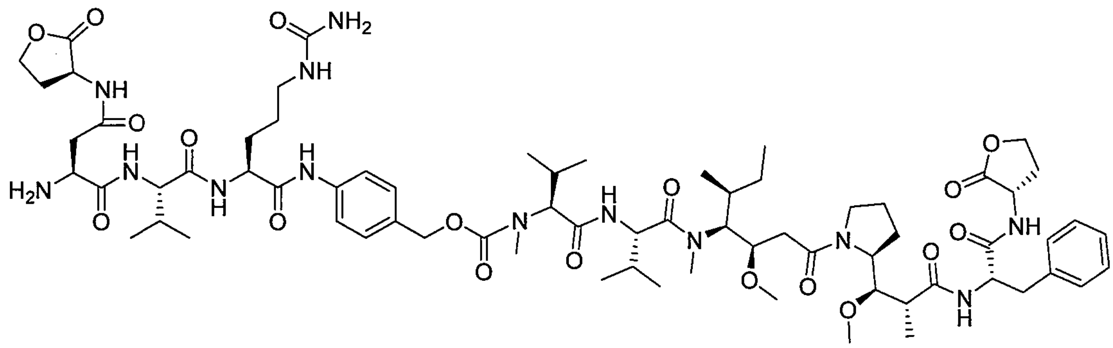

Exemplification 3-6-1

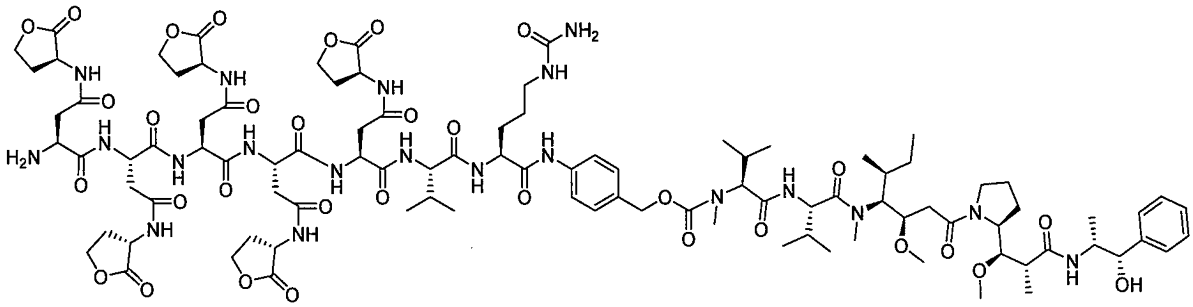

Exemplification 3-6-2

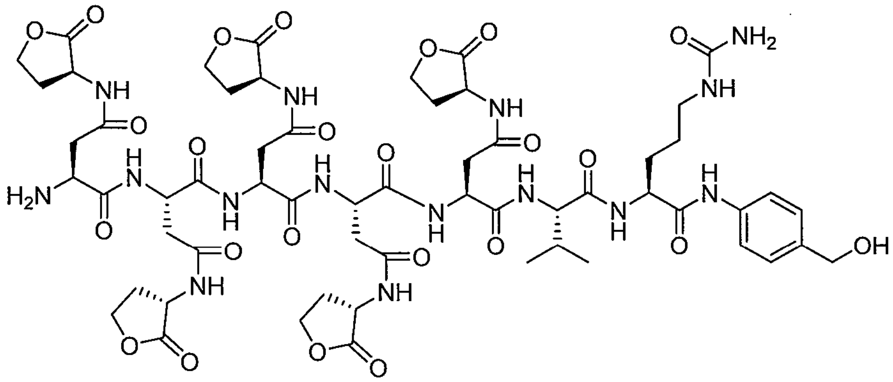

Exemplification 3-6-3

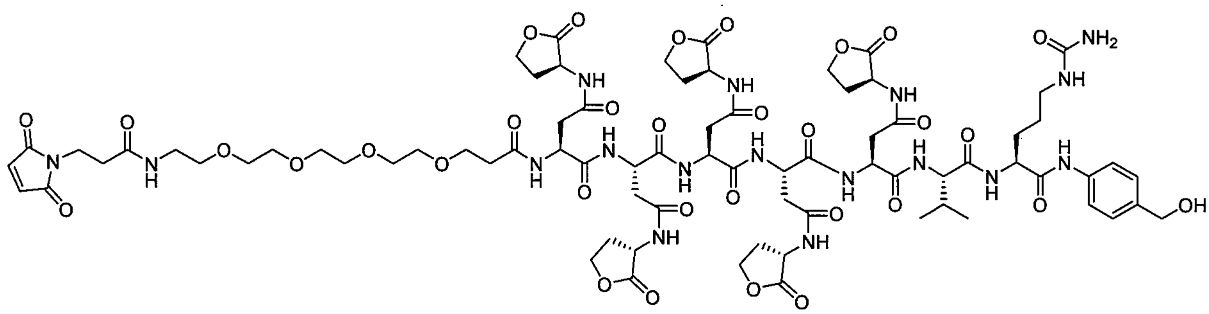

Exemplification 3-7-3

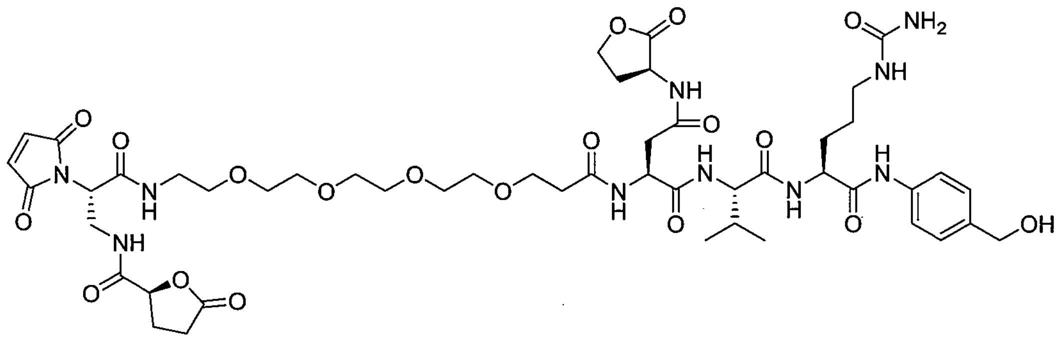

Exemplification 3-9-1

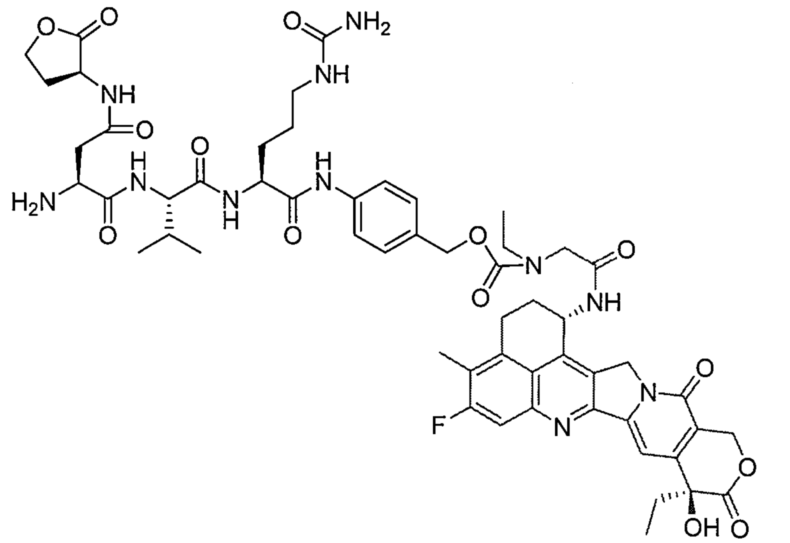

Exemplification 3-10-1

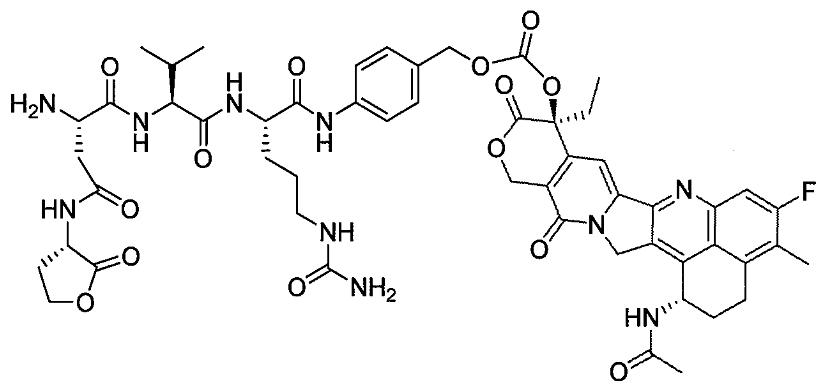

Exemplification 3-11-1

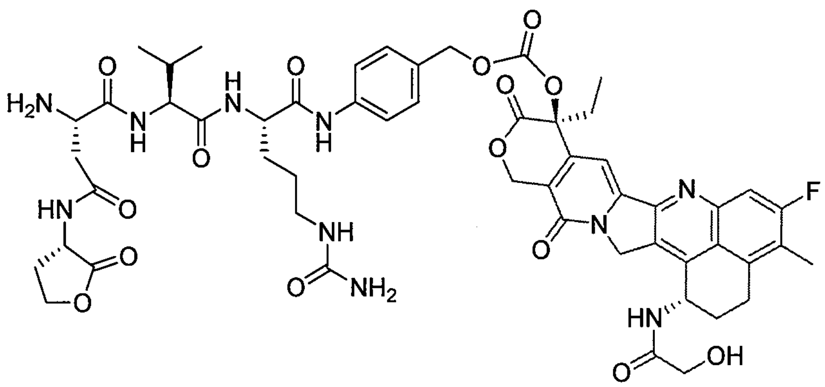

Exemplification 3-12-1

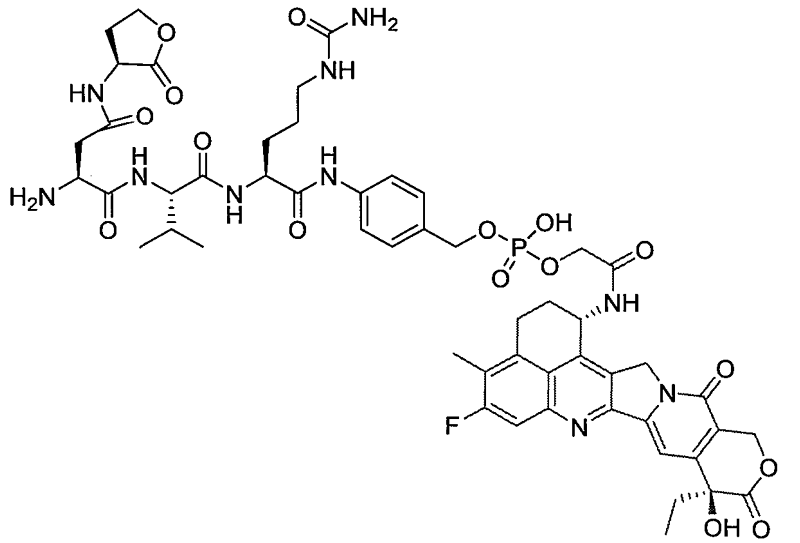

Exemplification 3-13-1

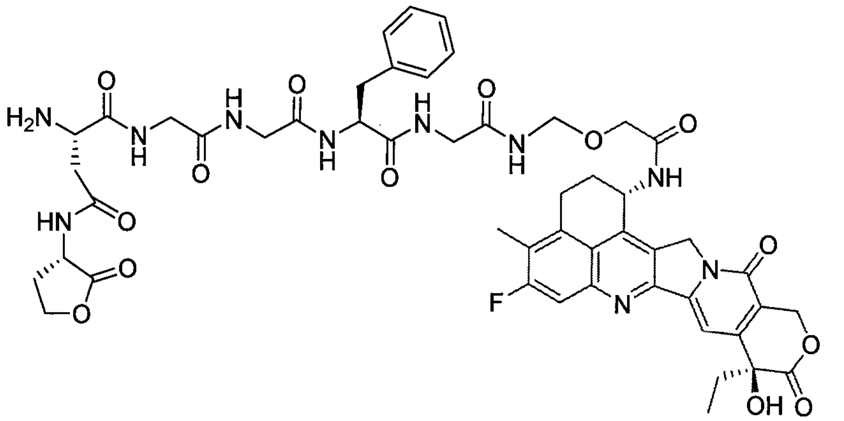

Exemplification 3-13-2

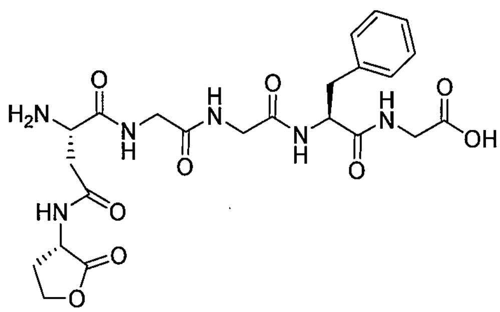

Exemplification 3-13-3

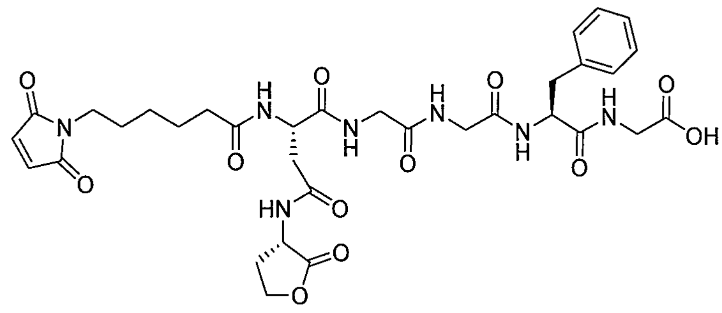

Exemplification 3-14-1

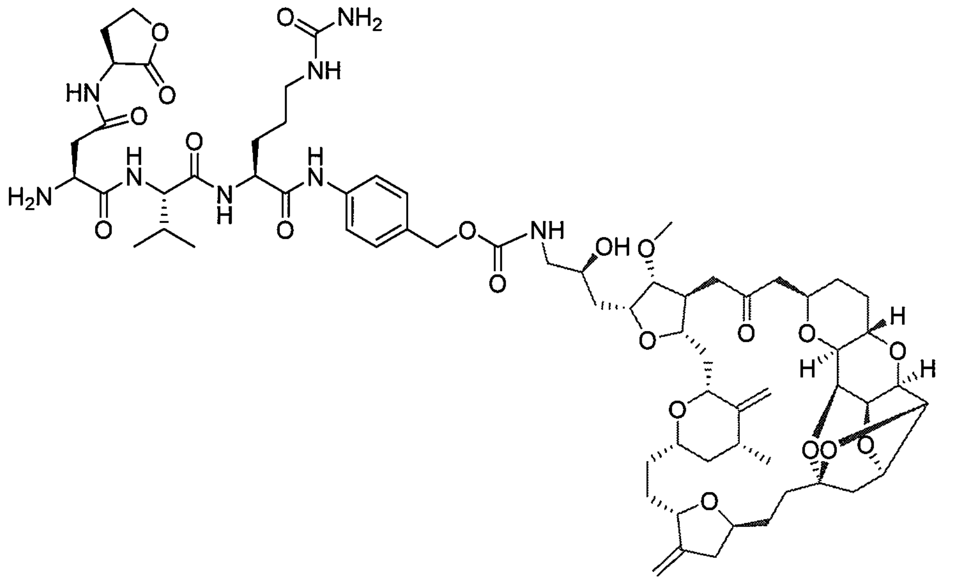

Exemplification 3-15-3

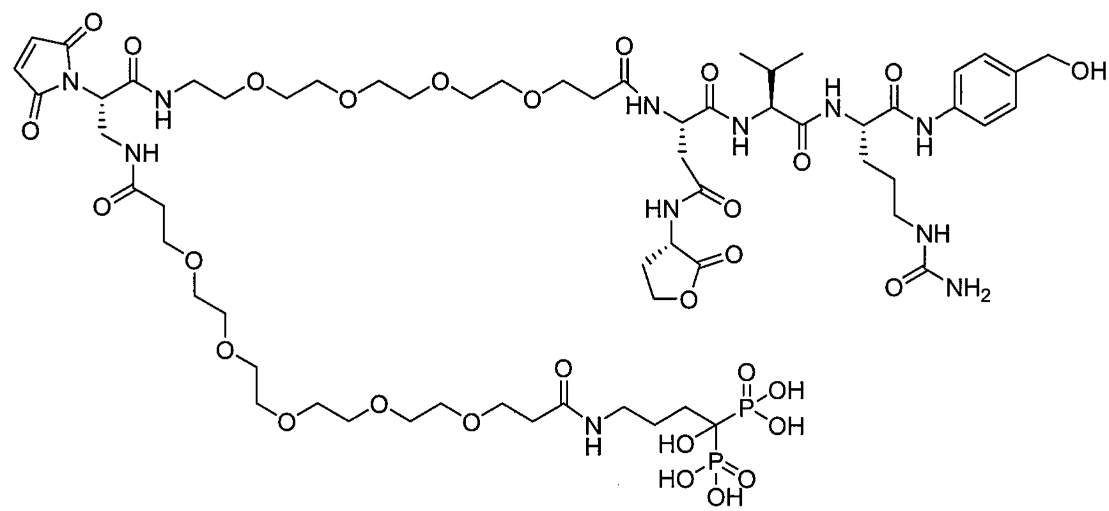

Exemplification 3-16-1

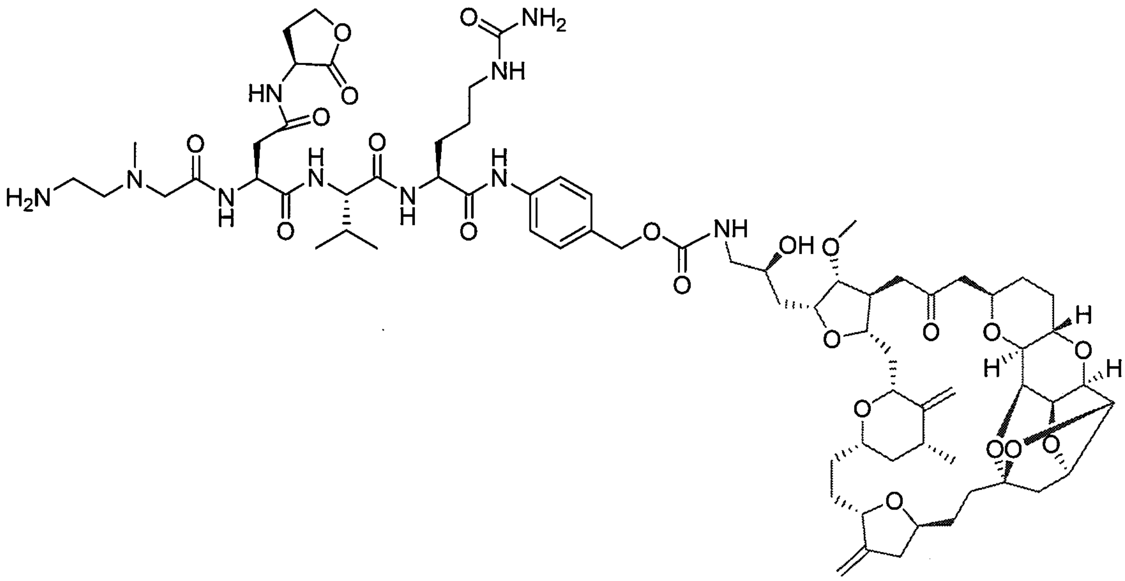

Exemplification 3-16-2

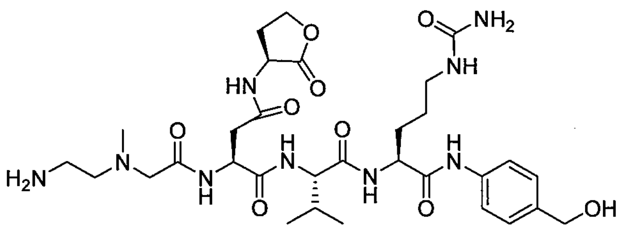

Exemplification 3-16-3

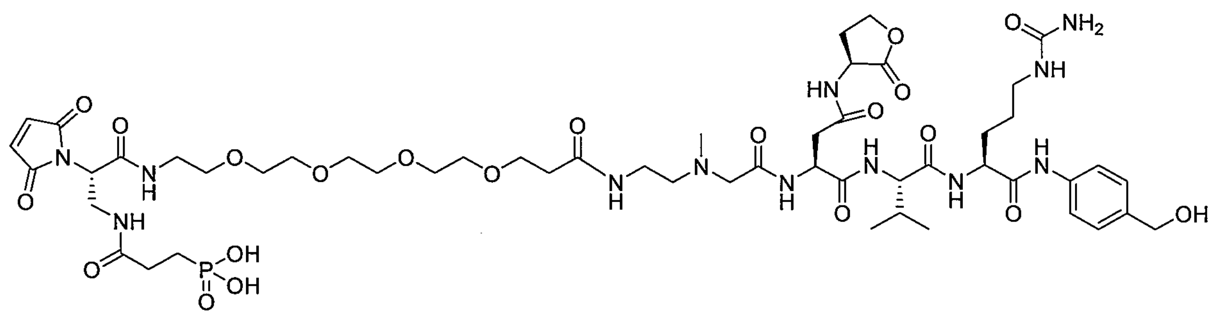

Exemplification 3-17-3

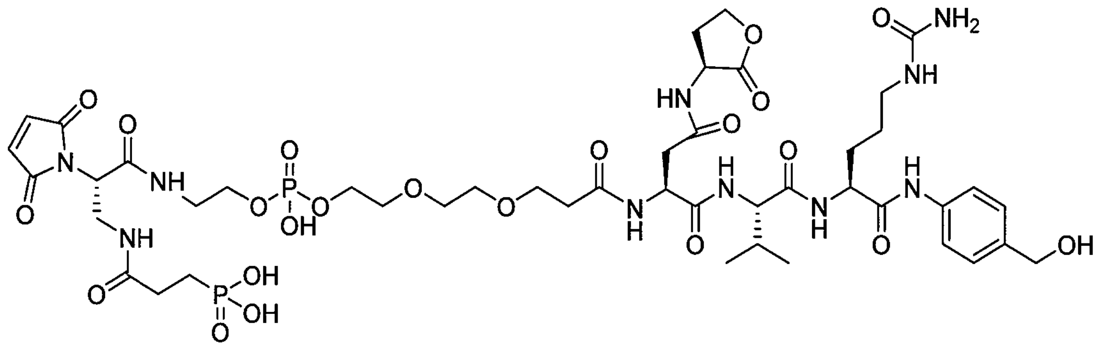

Exemplification 3-18-1

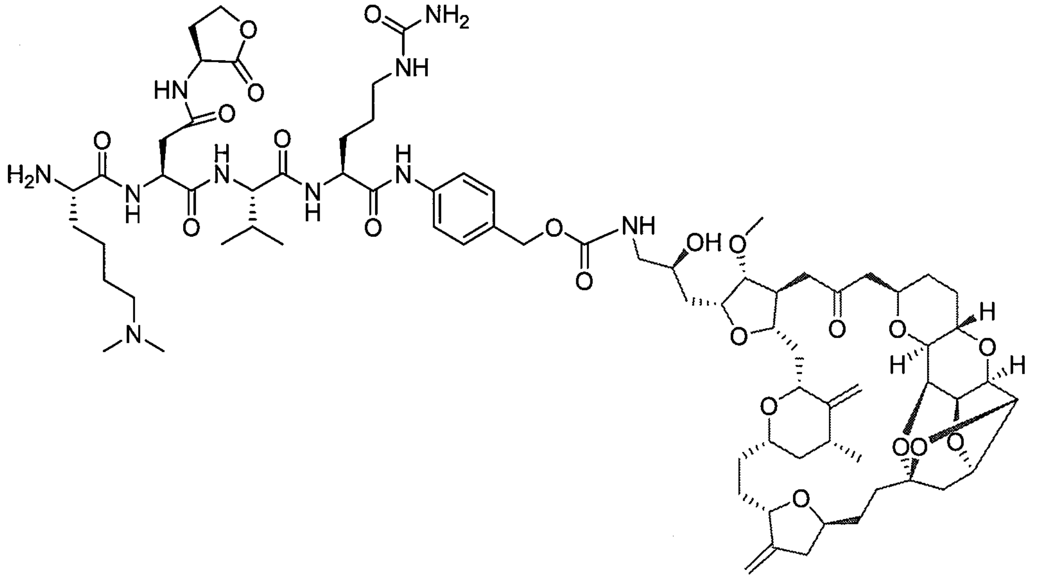

Exemplification 3-18-2

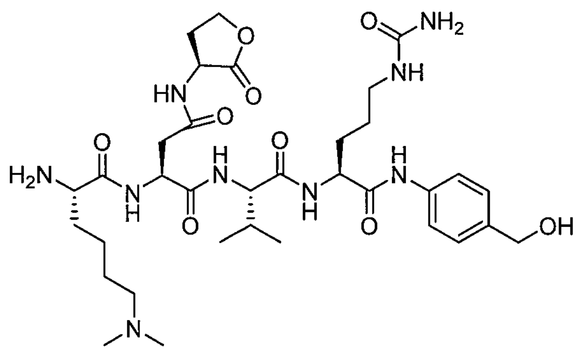

Exemplification 3-18-3

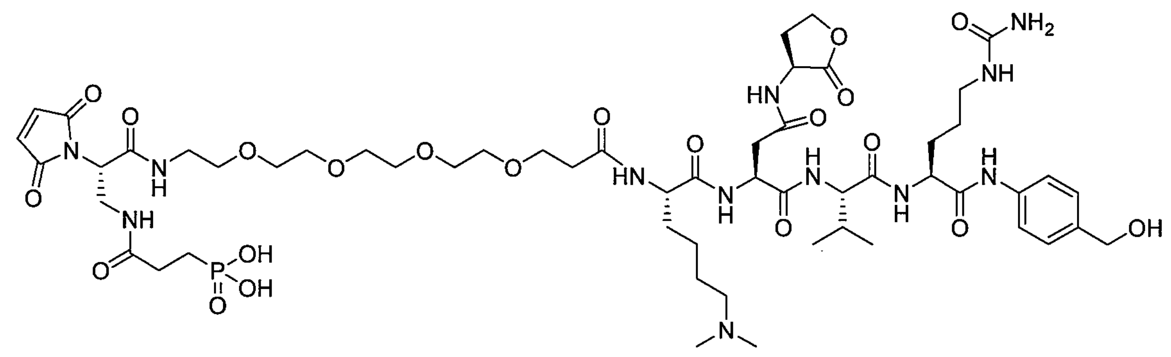

Exemplification 3-19-1

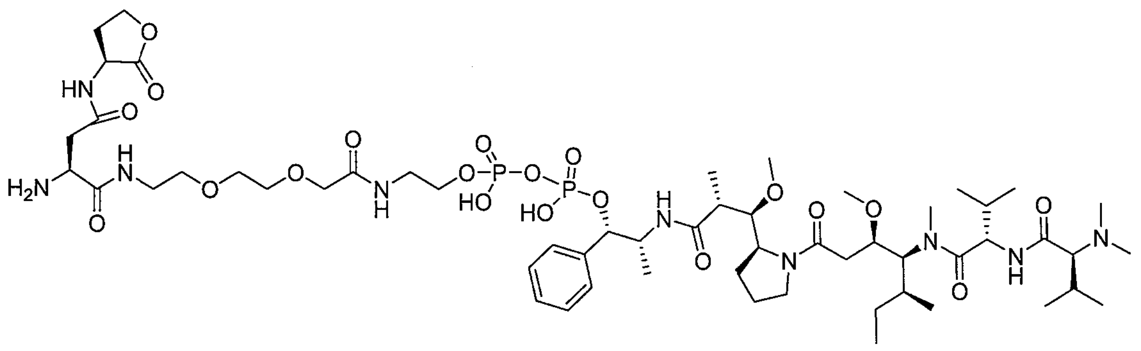

Exemplification 3-19-2

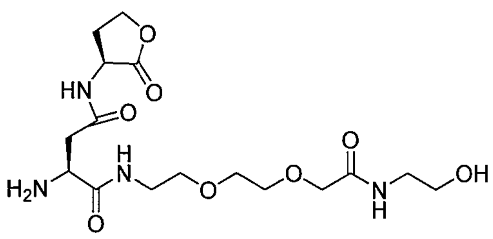

Exemplification 3-19-3

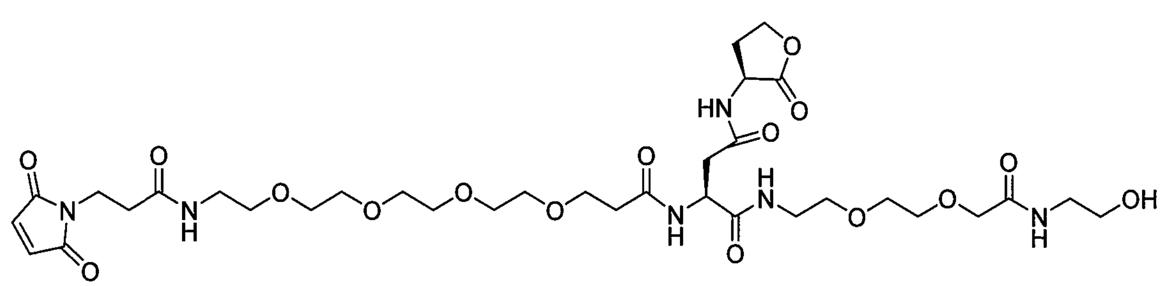

Exemplification 3-20-1

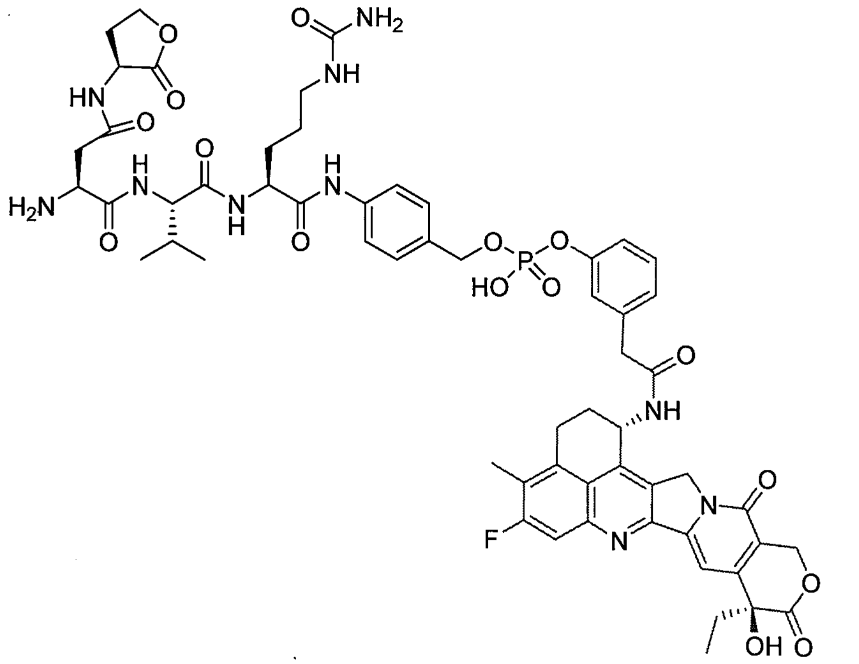

Exemplification 3-21-3

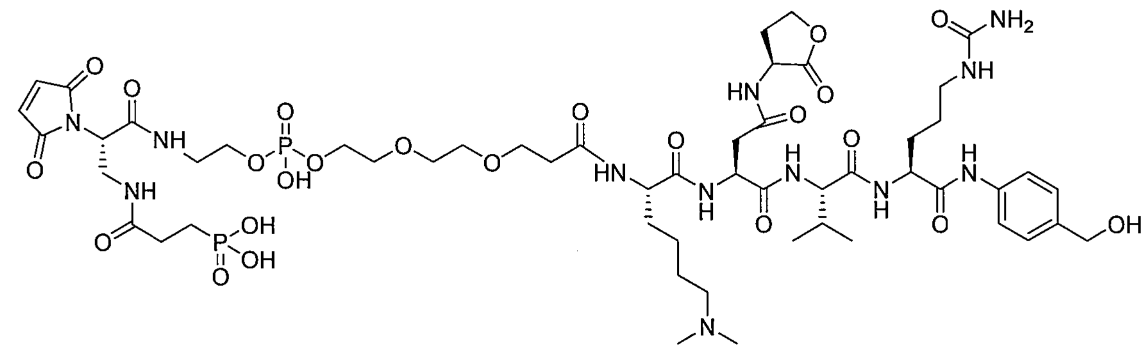

Exemplification 3-22-3

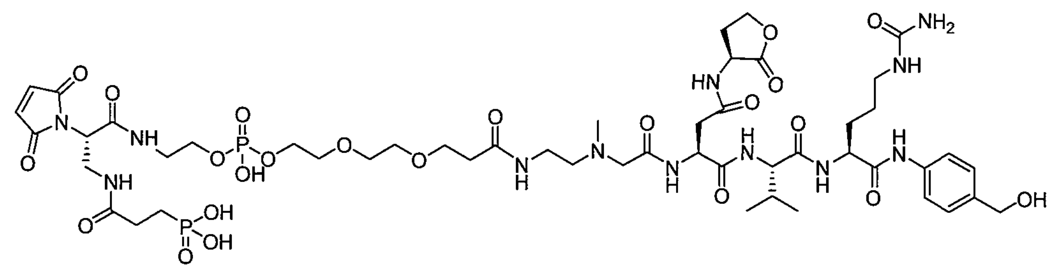

Exemplification 3-23-1

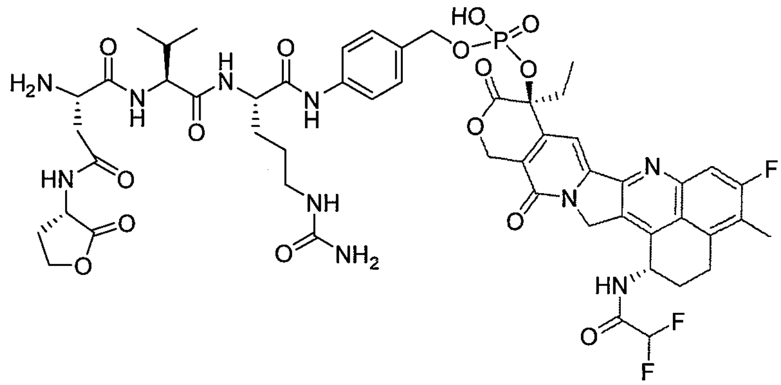

Exemplification 3-24-1

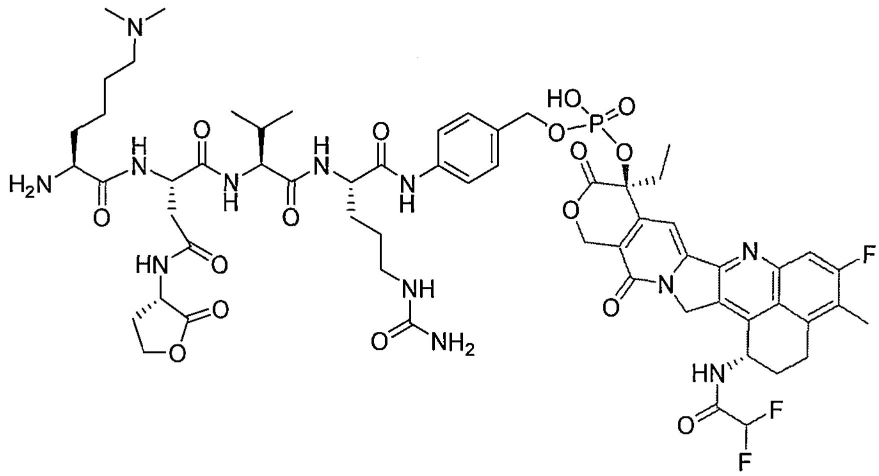

Exemplification 3-25-1

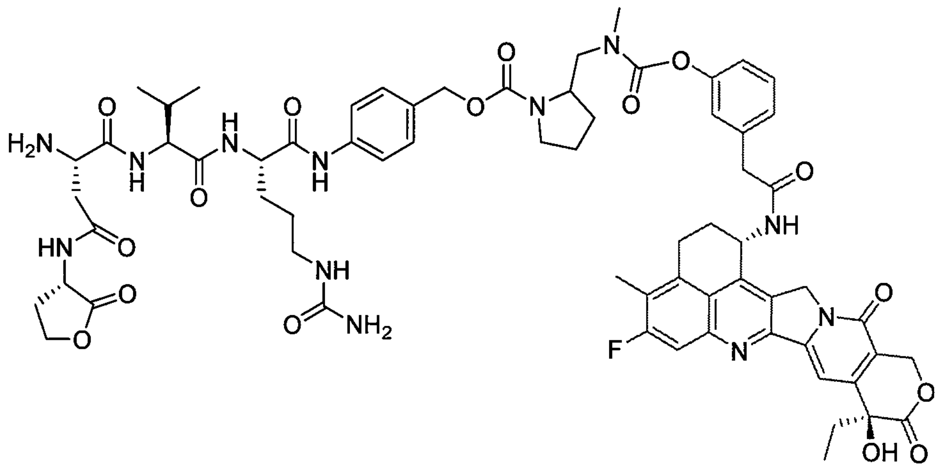

Exemplification 3-26-1

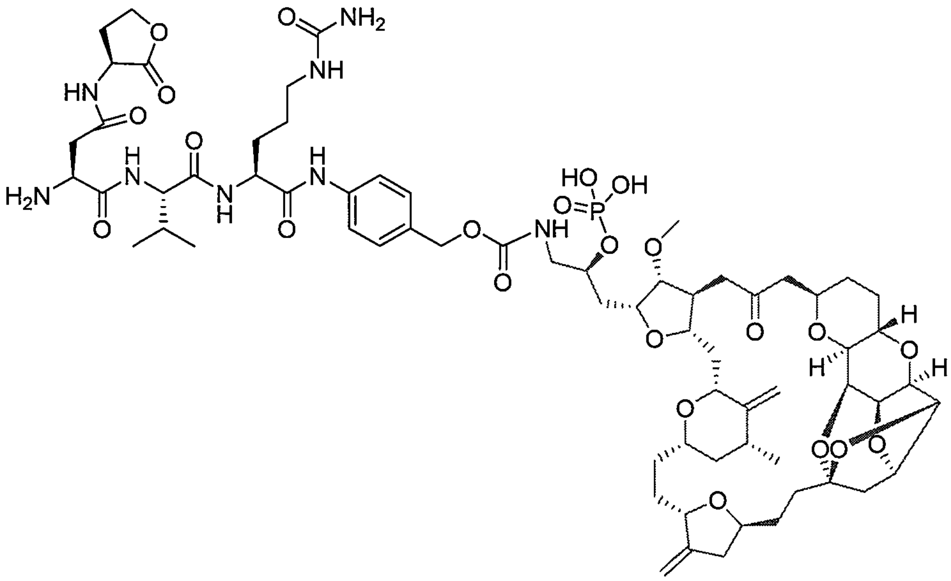

Exemplification 3-27-3

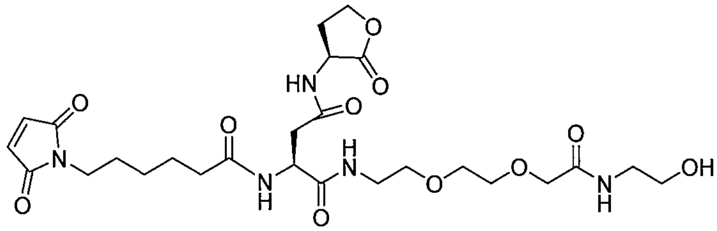

Exemplification 3-28-3

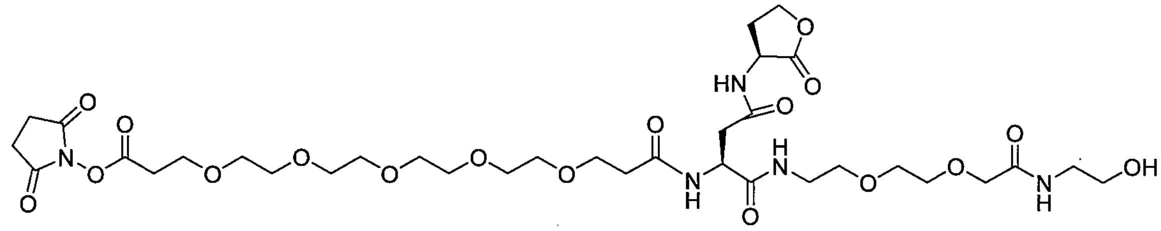

Exemplification 3-29-3

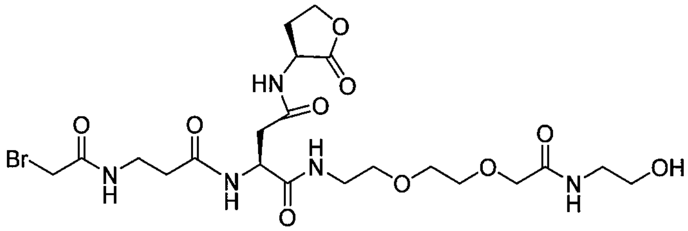

Exemplification 3-30-1

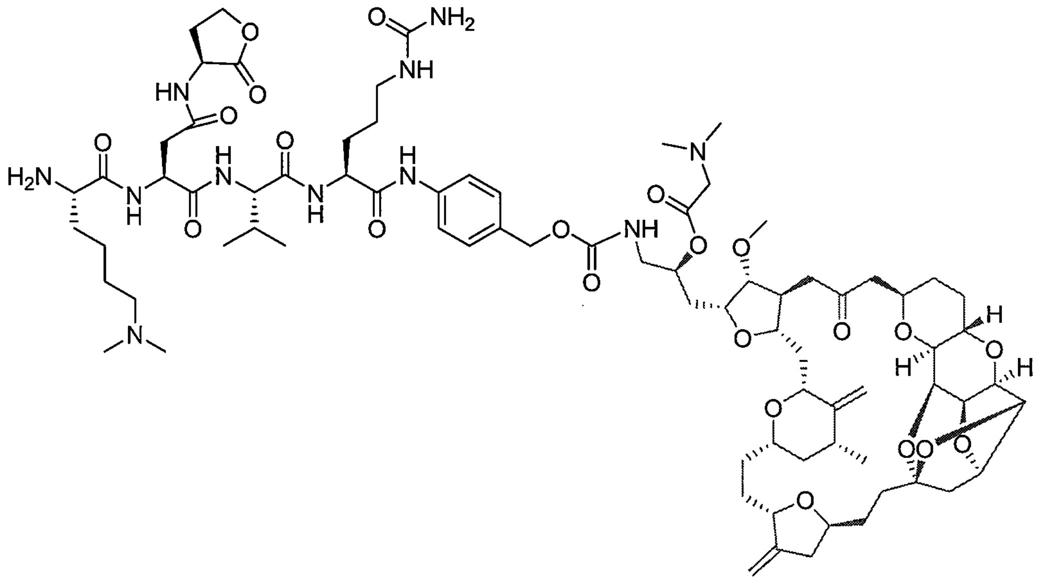

Exemplification 3-31-1

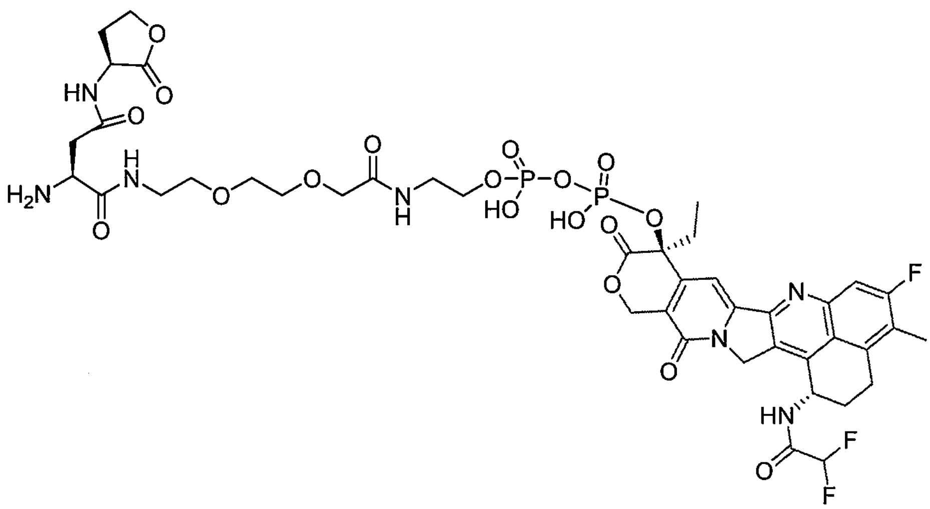

Exemplification 3-32-1

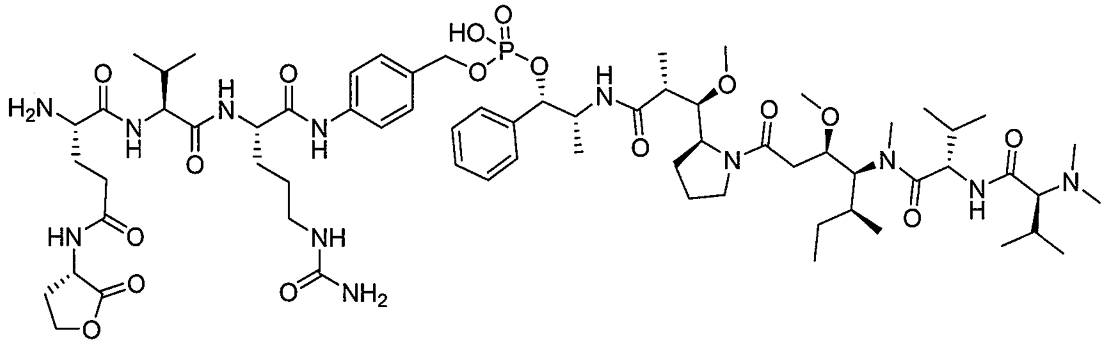

Exemplification 3-32-2

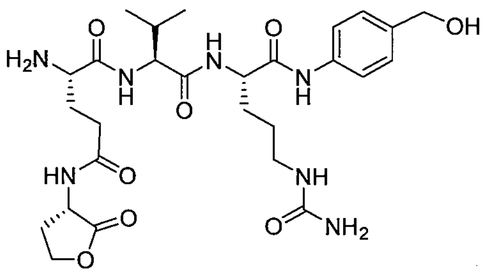

Exemplification 3-32-3

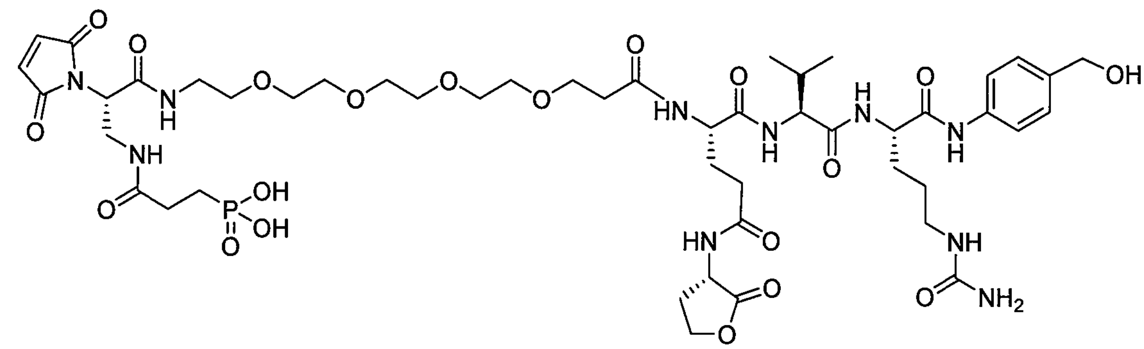

Exemplification 3-33-1

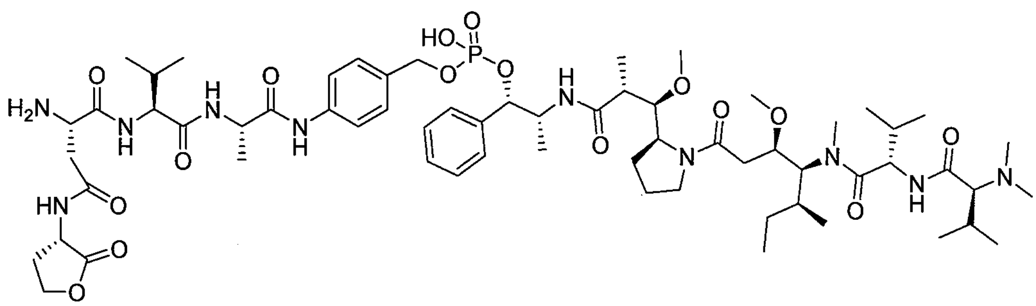

Exemplification 3-33-2

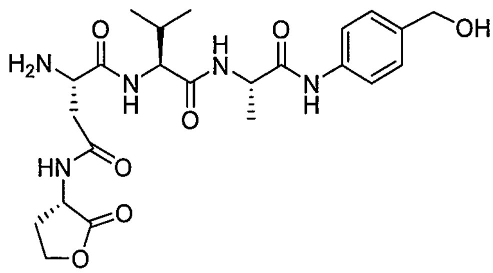

Exemplification 3-33-3

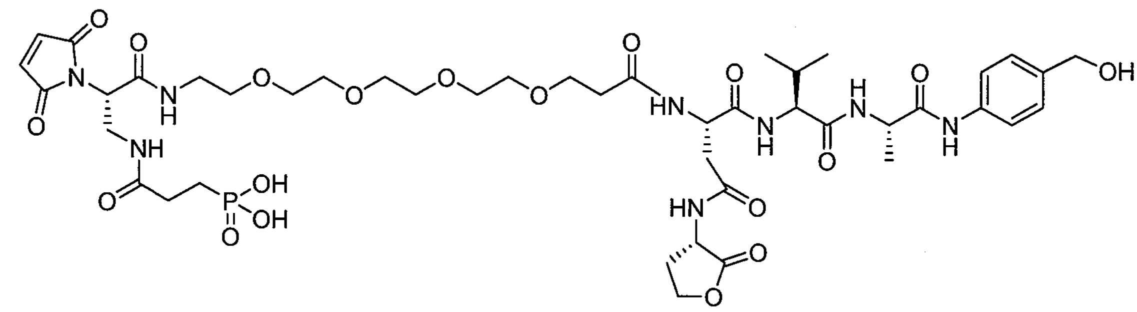

Exemplification 3-34-1

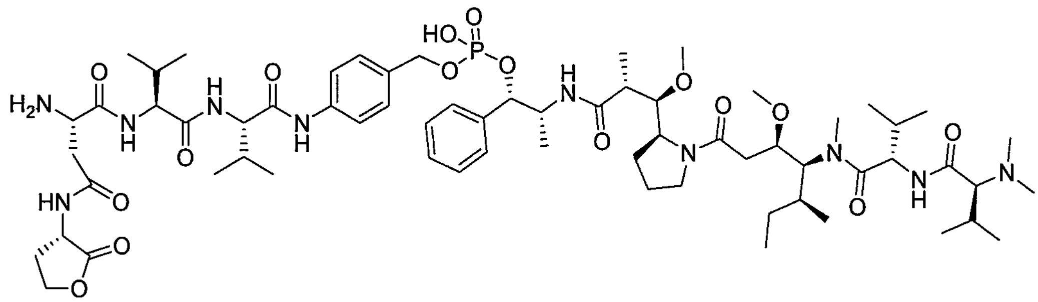

Exemplification 3-34-2

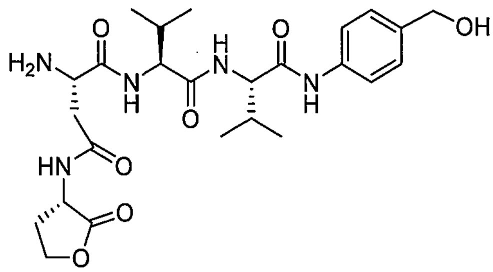

Exemplification 3-34-3

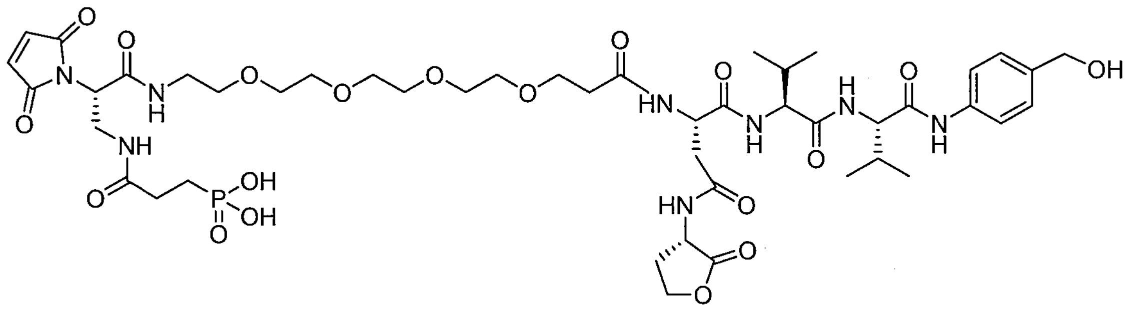

Exemplification 3-35-1

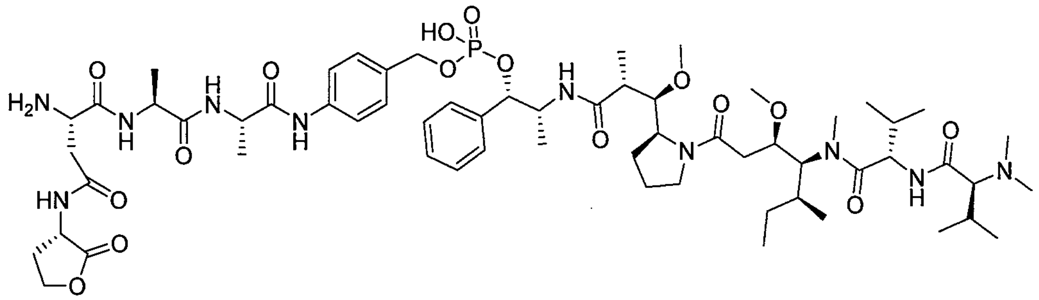

Exemplification 3-35-2

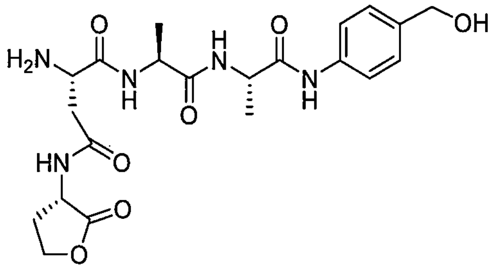

Exemplification 3-35-3

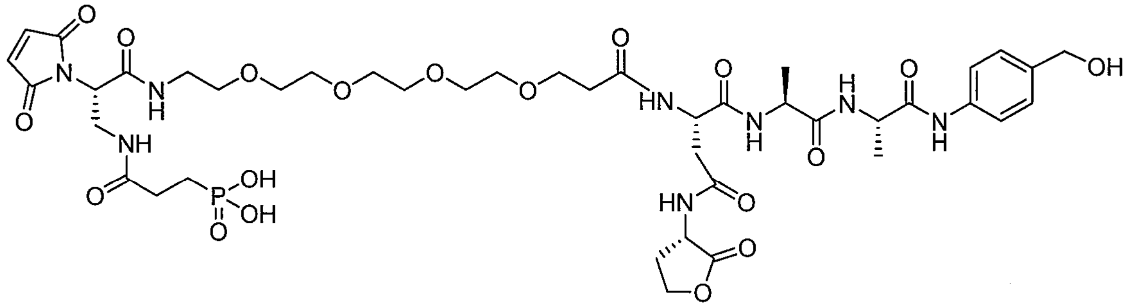

Exemplification 3-36-1

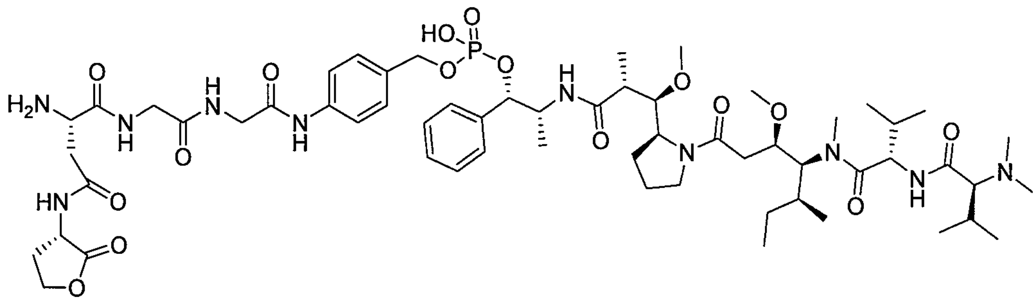

Exemplification 3-36-2

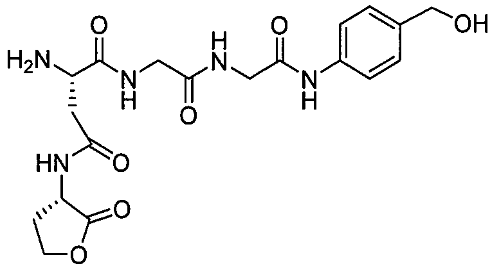

Exemplification 3-36-3

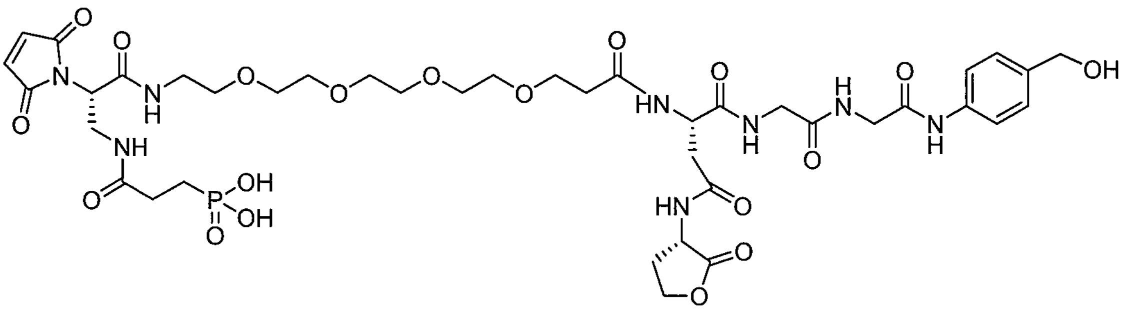

Exemplification 3-37-1

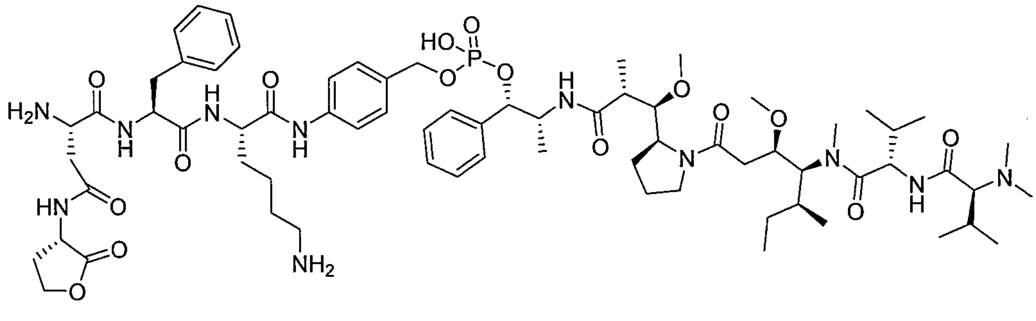

Exemplification 3-37-2

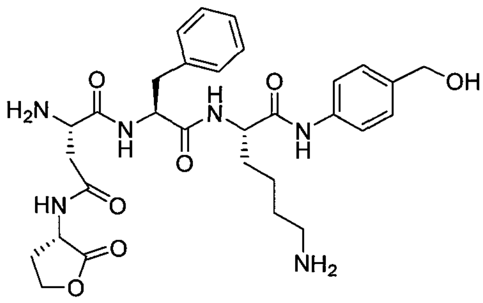

Exemplification 3-37-3

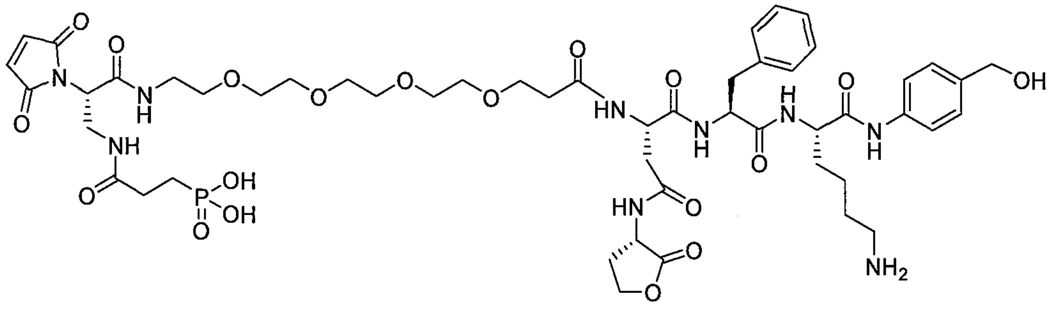

Exemplification 3-38-1

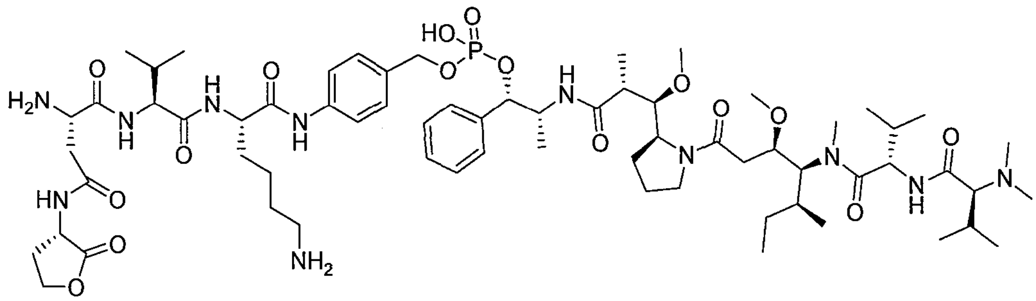

Exemplification 3-38-2

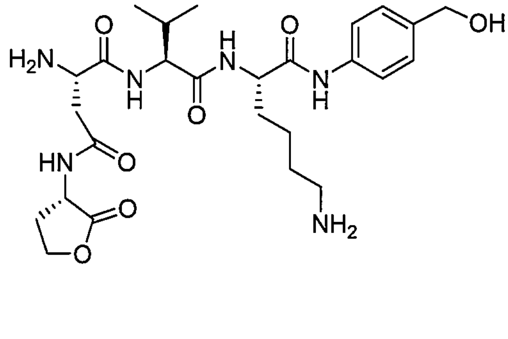

Exemplification 3-38-3

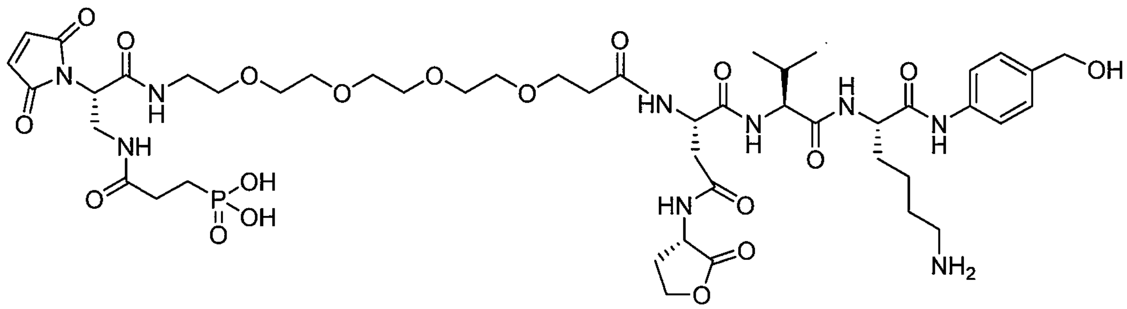

Exemplification 3-39-1

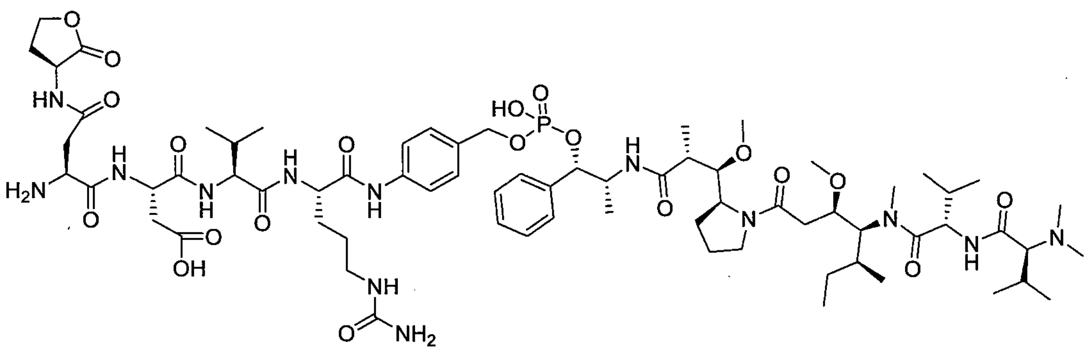

Exemplification 3-39-2

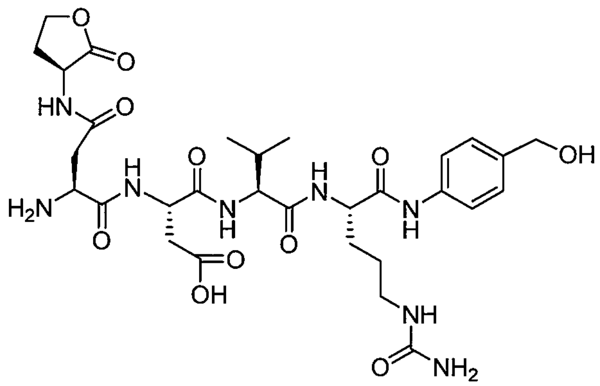

Exemplification 3-39-3

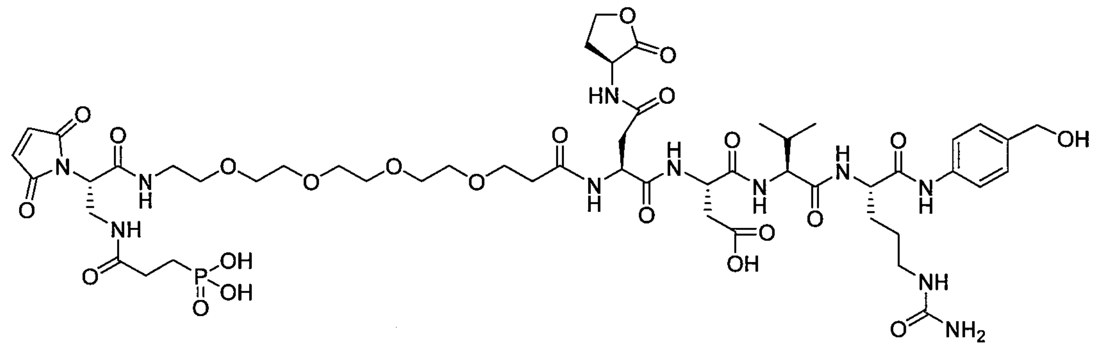

Exemplification 3-40-1

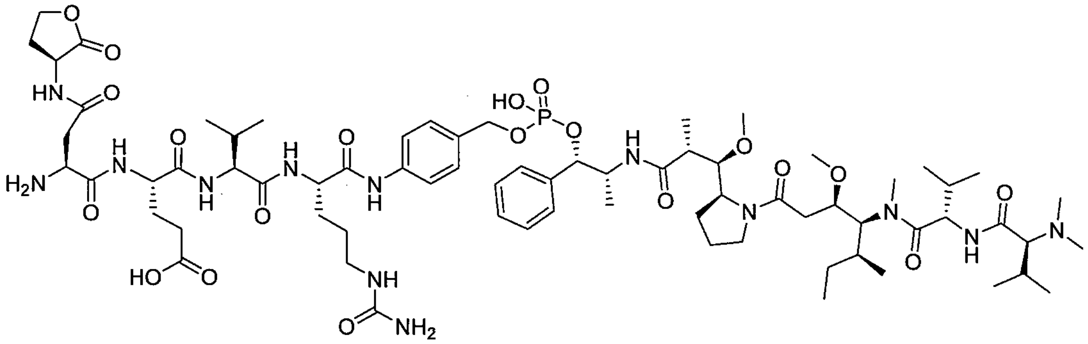

Exemplification 3-40-2

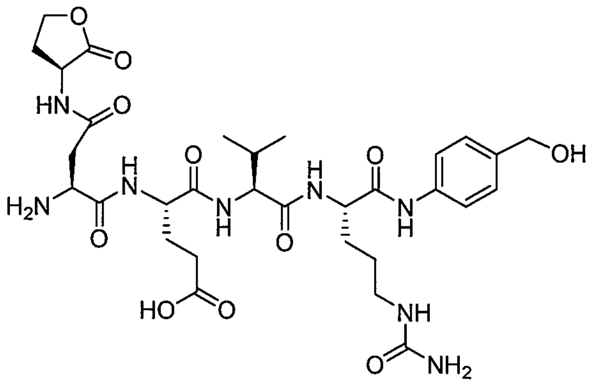

Exemplification 3-40-3

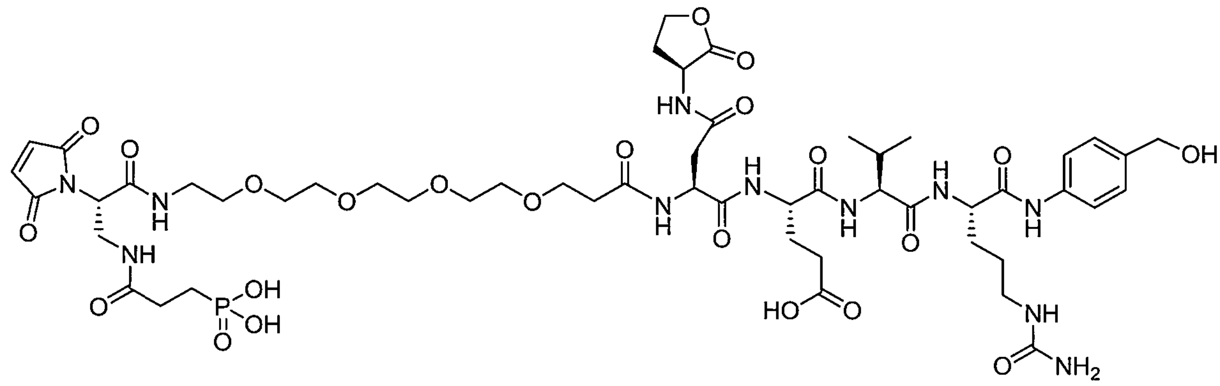

Exemplification 3-41-1

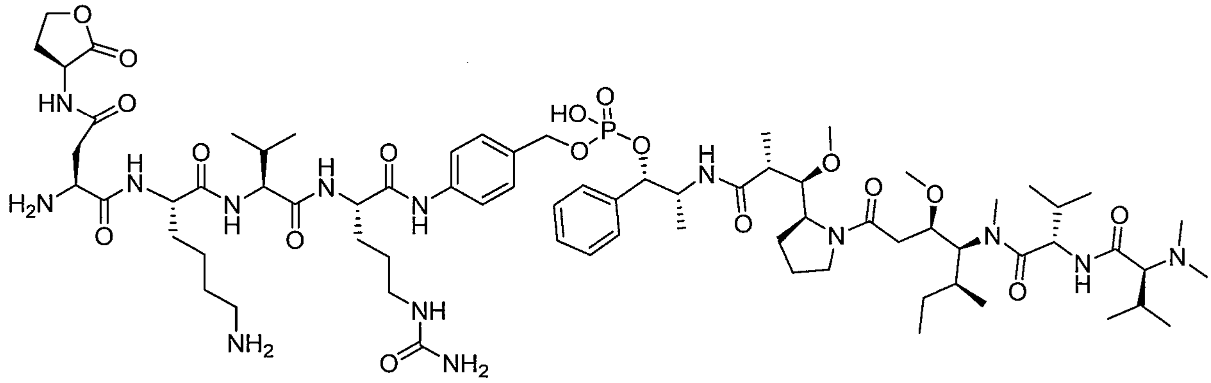

Exemplification 3-41-2

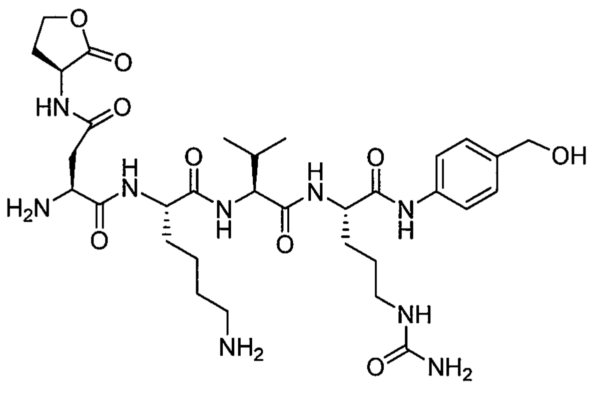

Exemplification 3-41-3

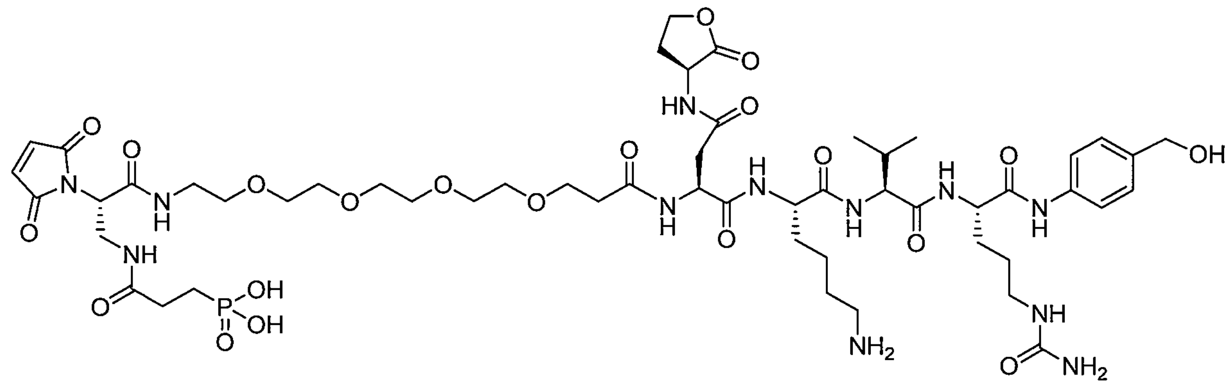

Exemplification 3-42-1

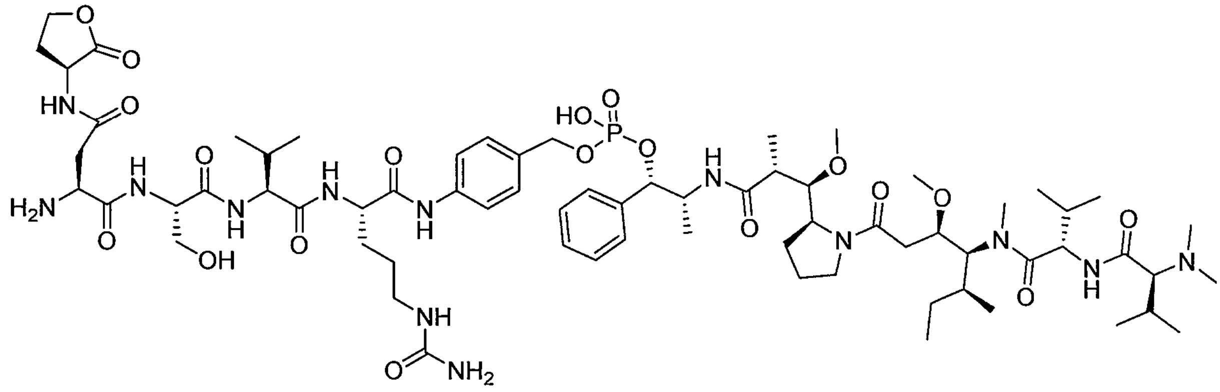

Exemplification 3-42-2

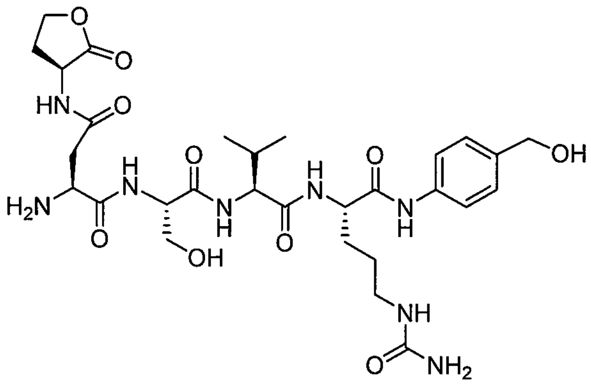

Exemplification 3-42-3

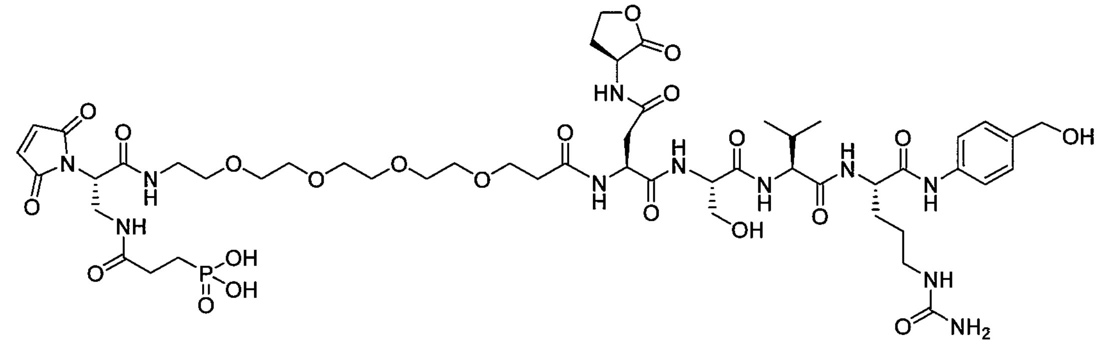

Exemplification 3-43-1

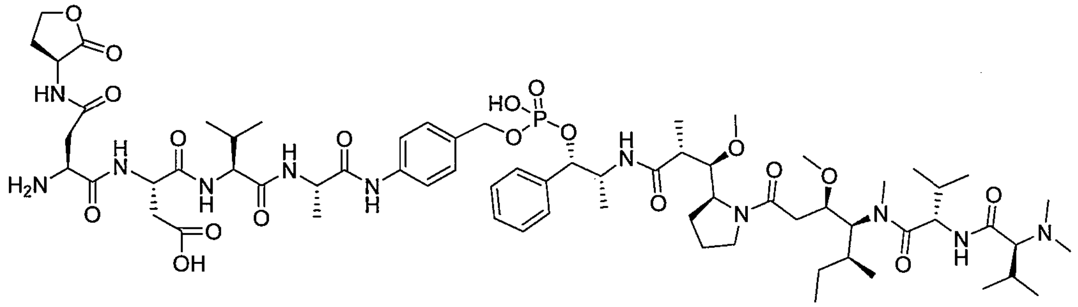

Exemplification 3-43-2

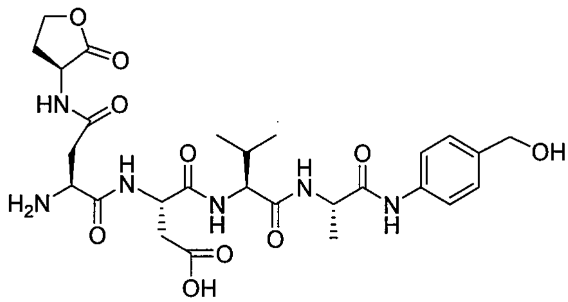

Exemplification 3-43-3

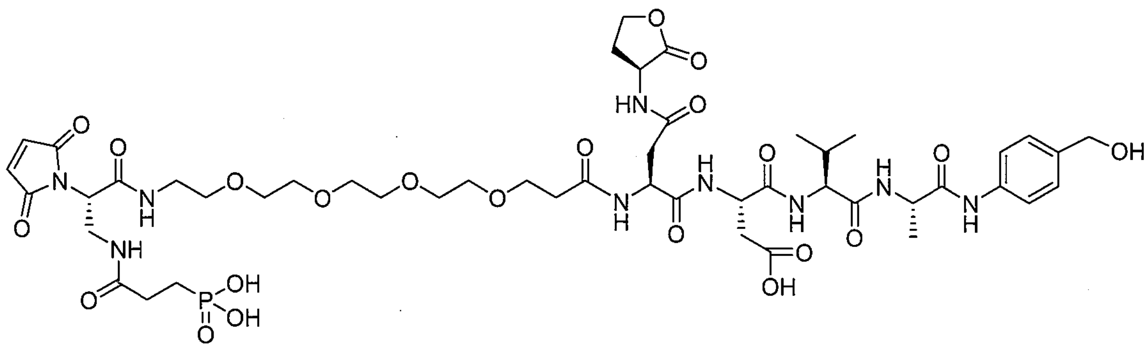

Exemplification 3-44-1

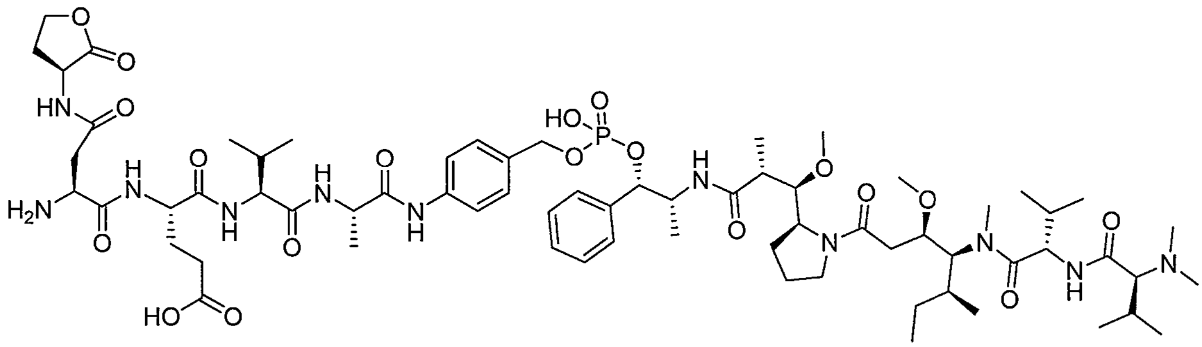

Exemplification 3-44-2

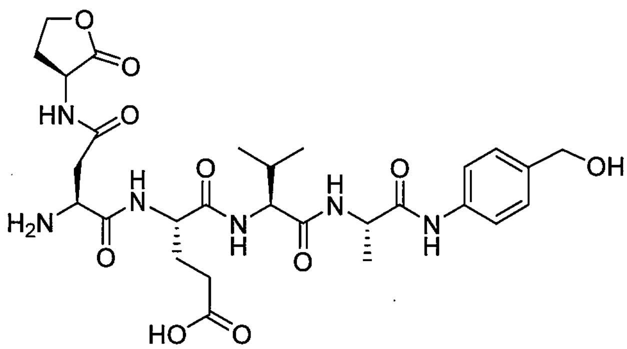

Exemplification 3-44-3

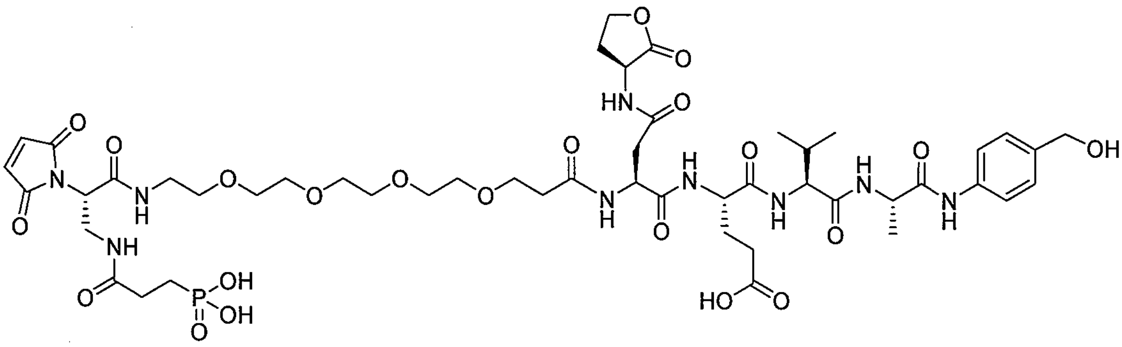

Exemplification 3-45-1

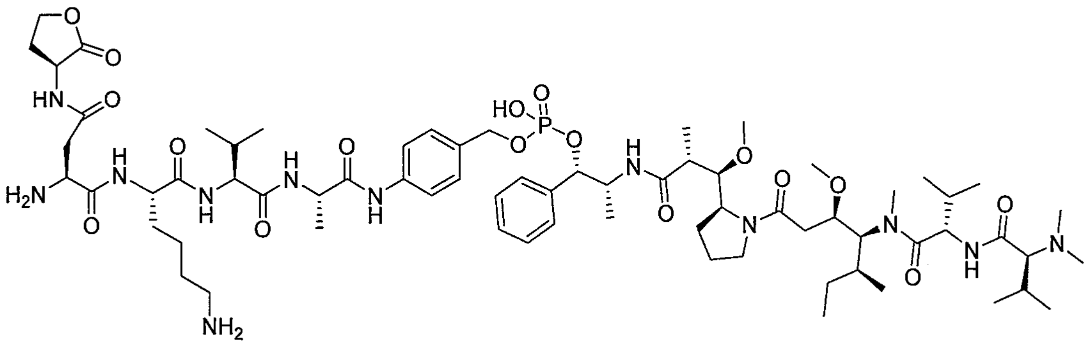

Exemplification 3-45-2

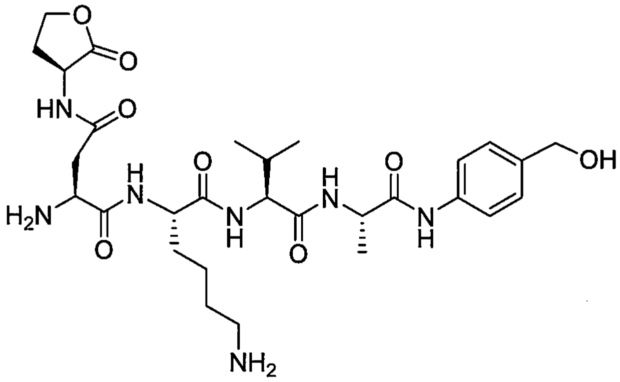

Exemplification 3-45-3

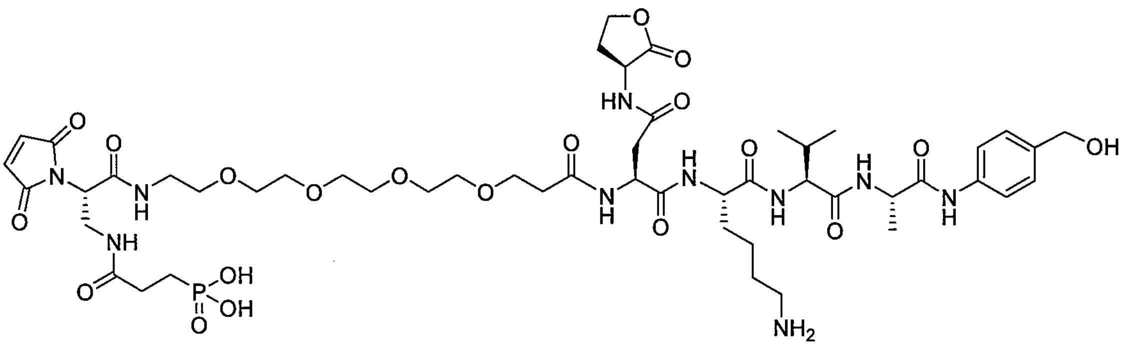

Exemplification 3-46-1

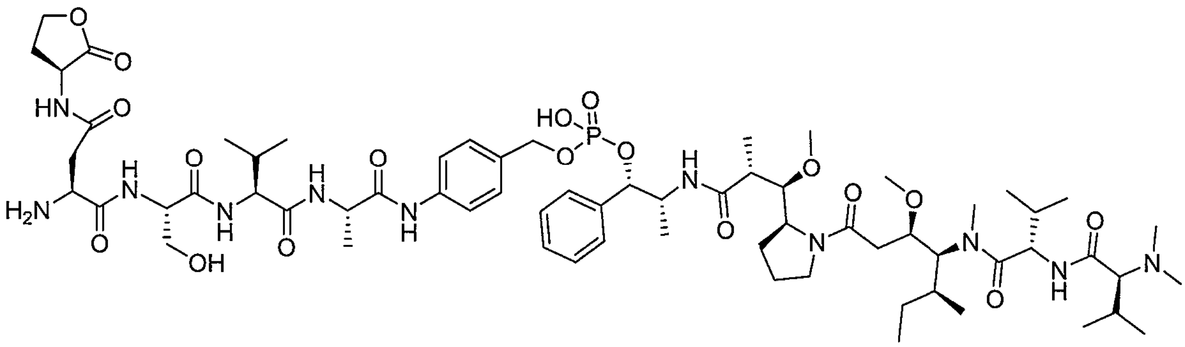

Exemplification 3-46-2

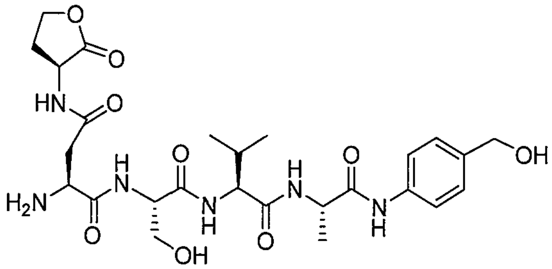

Exemplification 3-46-3

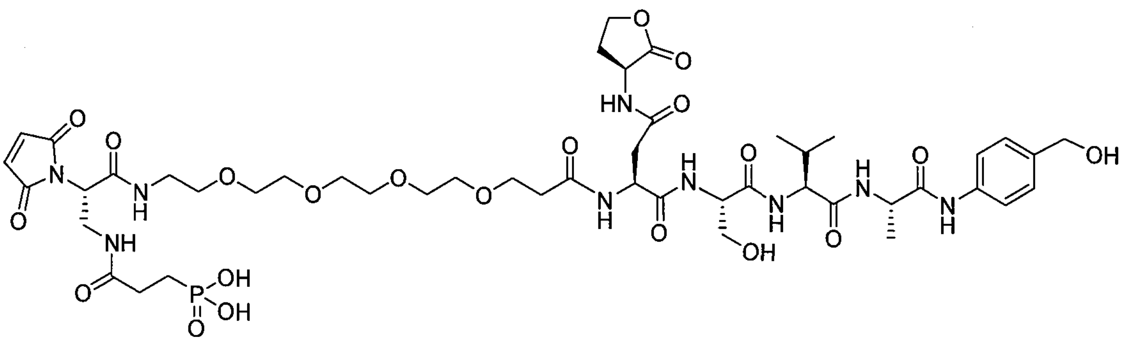

Exemplification 3-47-1

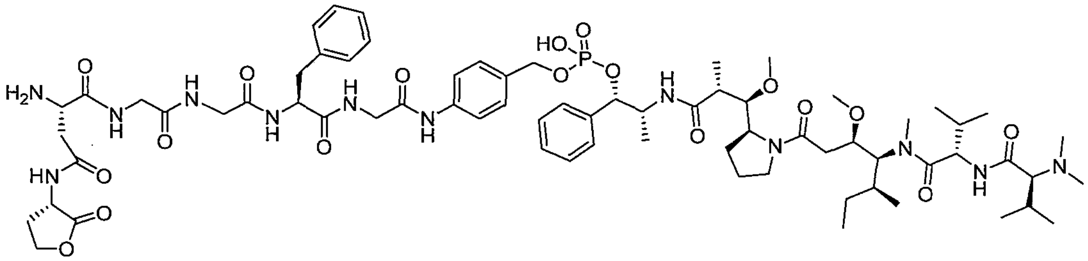

Exemplification 3-47-2

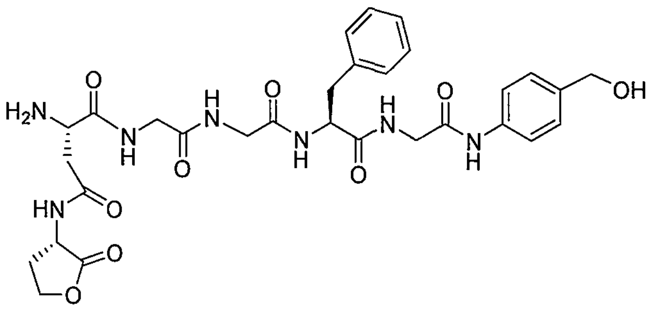

Exemplification 3-47-3

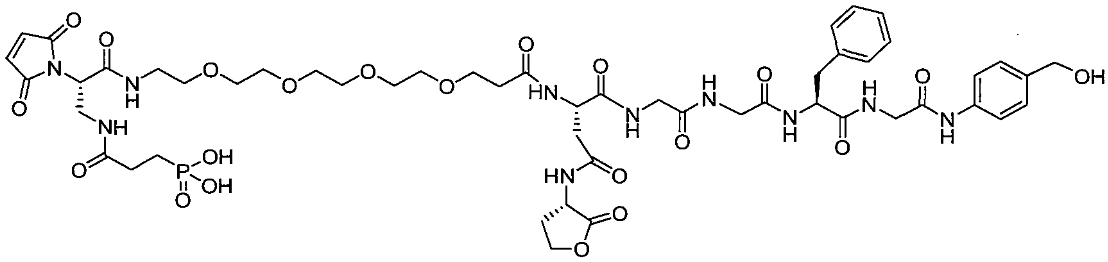

Exemplification 3-48-1

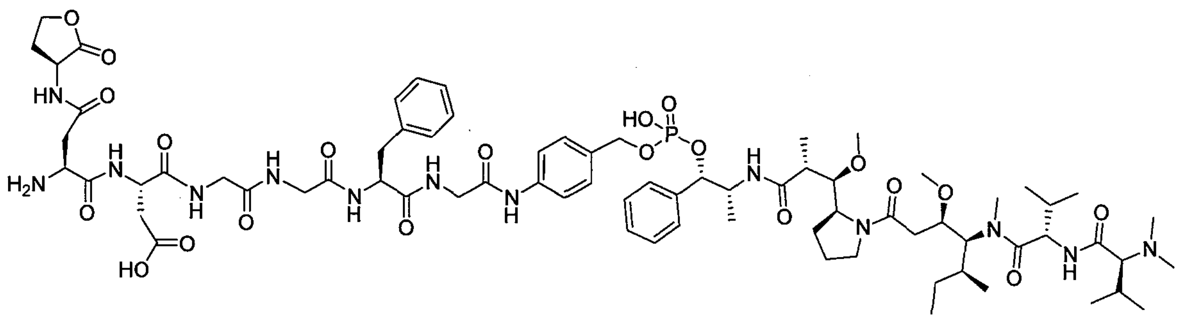

Exemplification 3-48-2

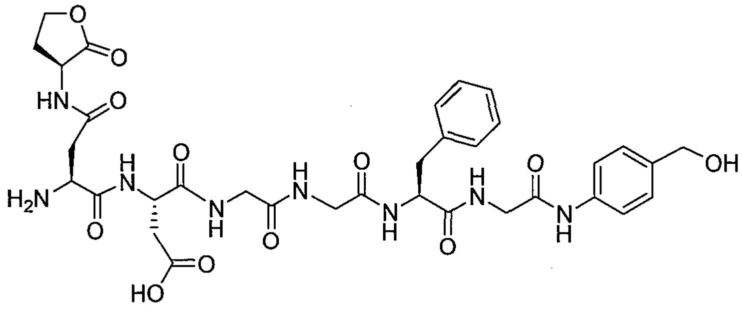

Exemplification 3-48-3

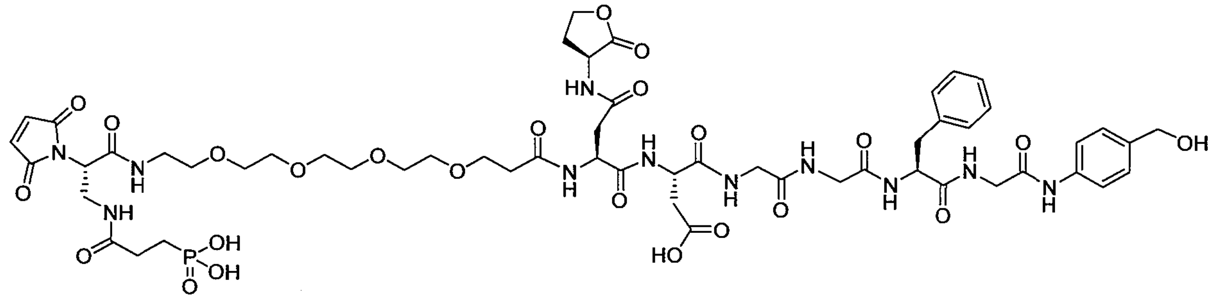

Exemplification 3-49-1

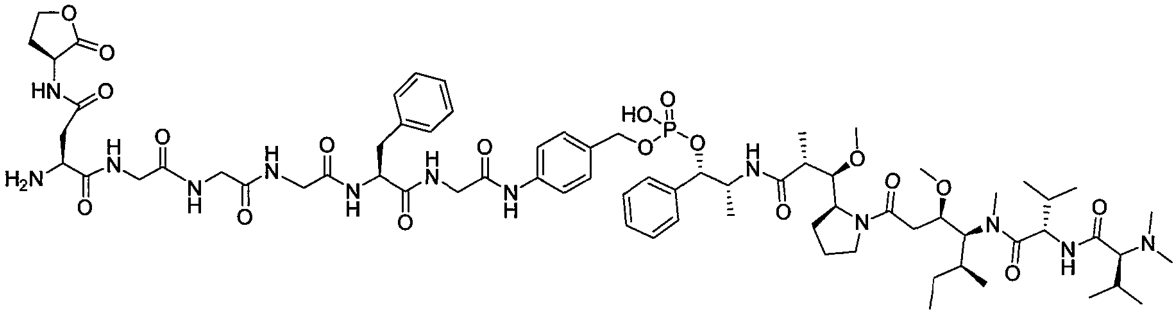

Exemplification 3-49-2

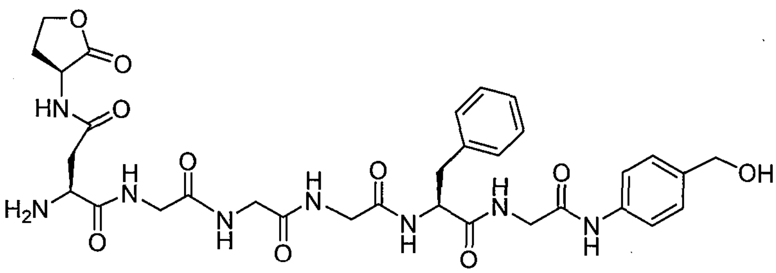

Exemplification 3-49-3

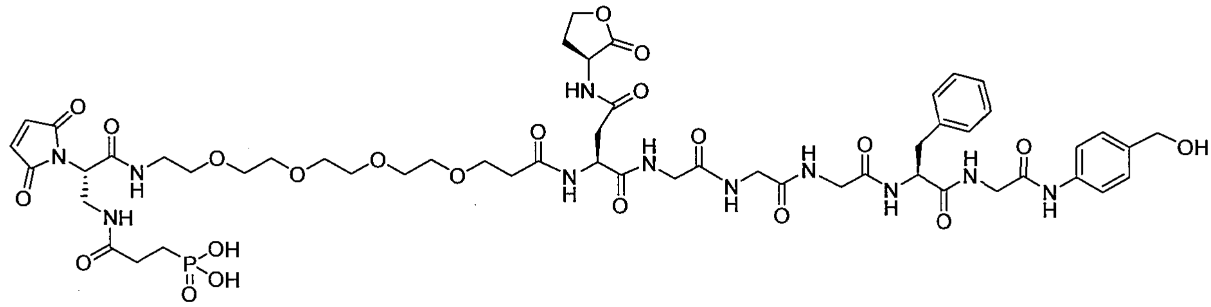

Exemplification 3-50-1

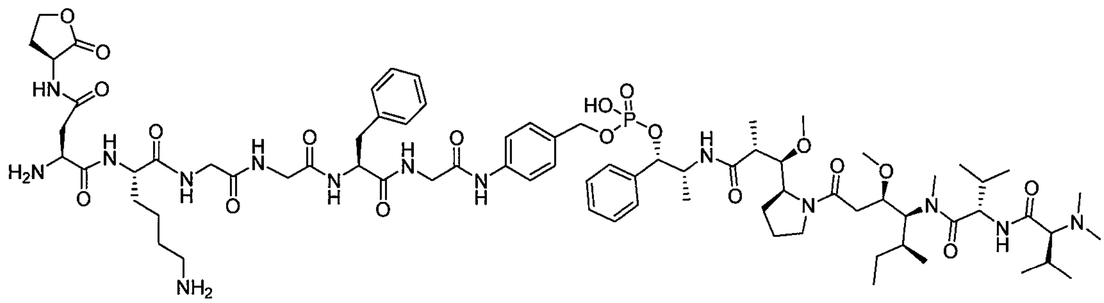

Exemplification 3-50-2

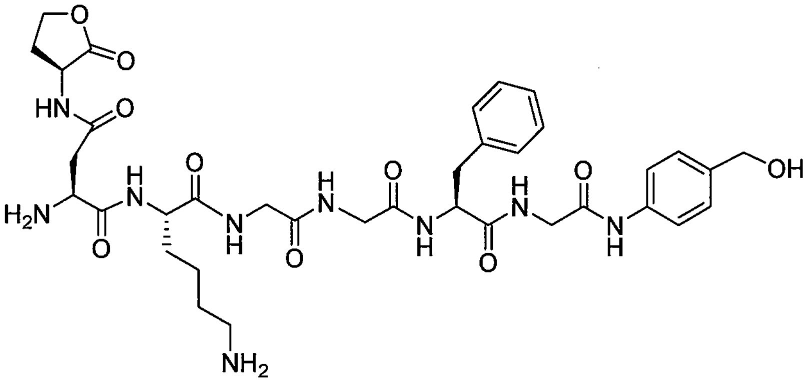

Exemplification 3-50-3

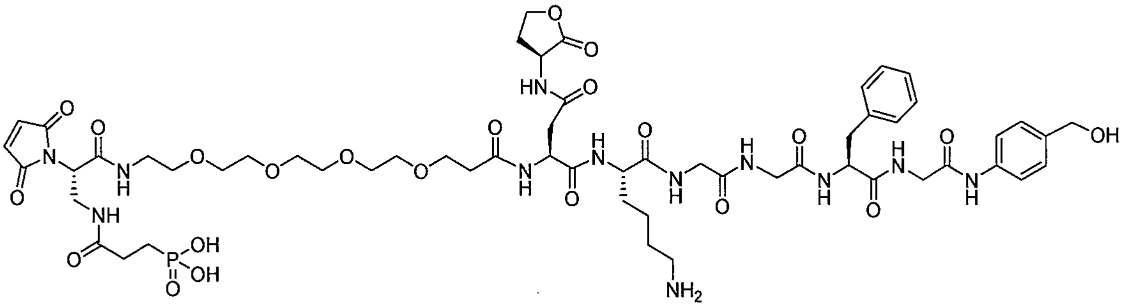

Exemplification 3-51-1

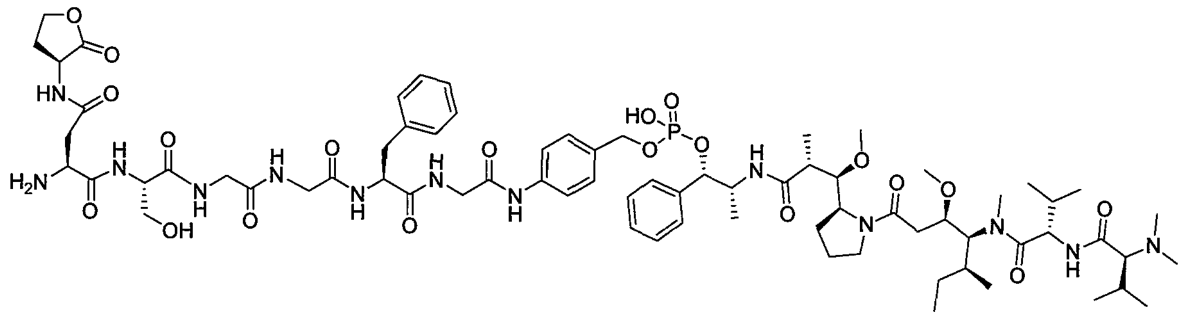

Exemplification 3-51-2

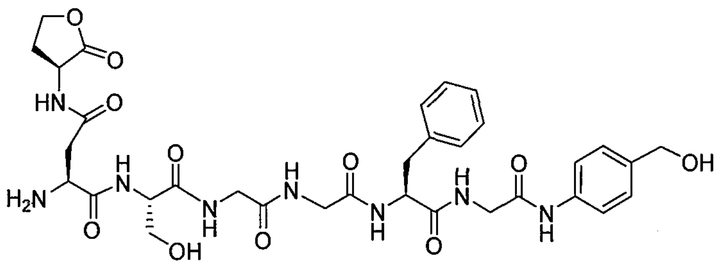

Exemplification 3-51-3

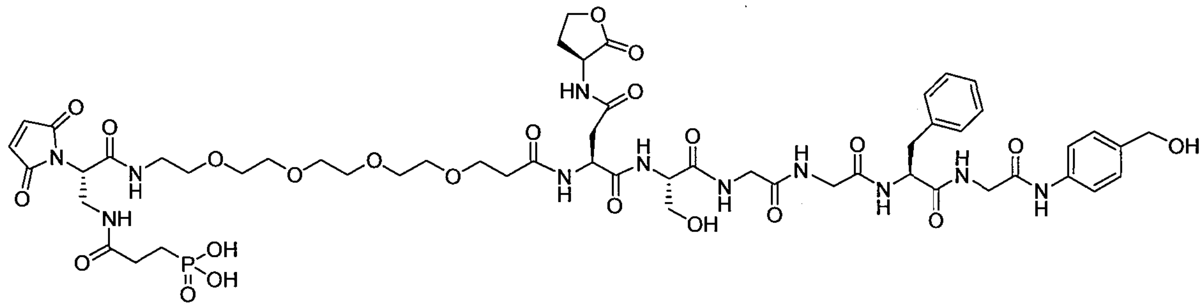

Exemplification 3-52-1

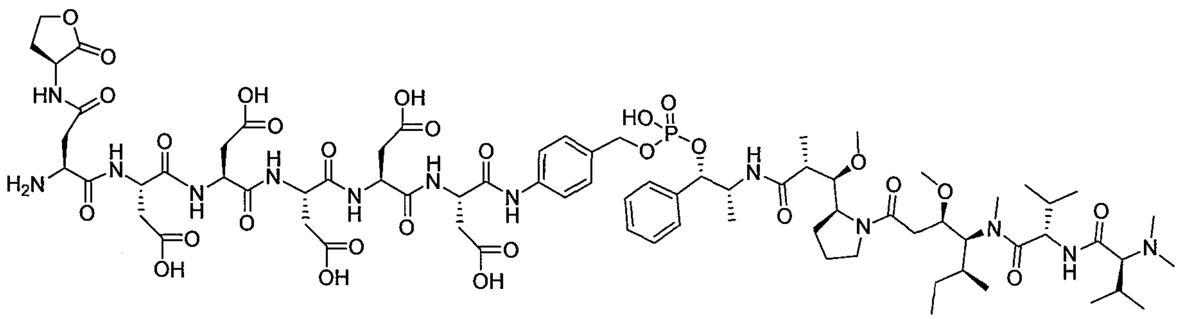

Exemplification 3-52-2

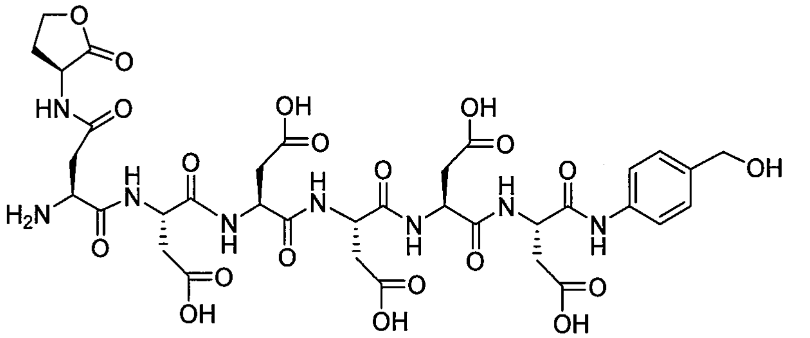

Exemplification 3-52-3

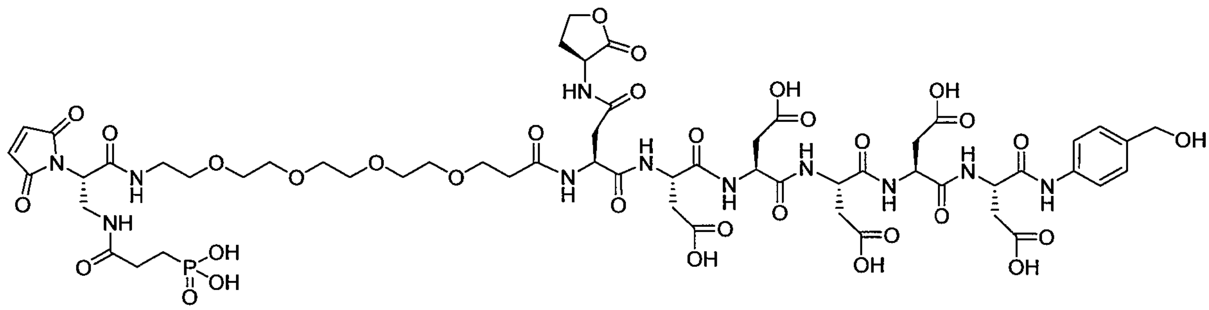

Exemplification 3-53-3

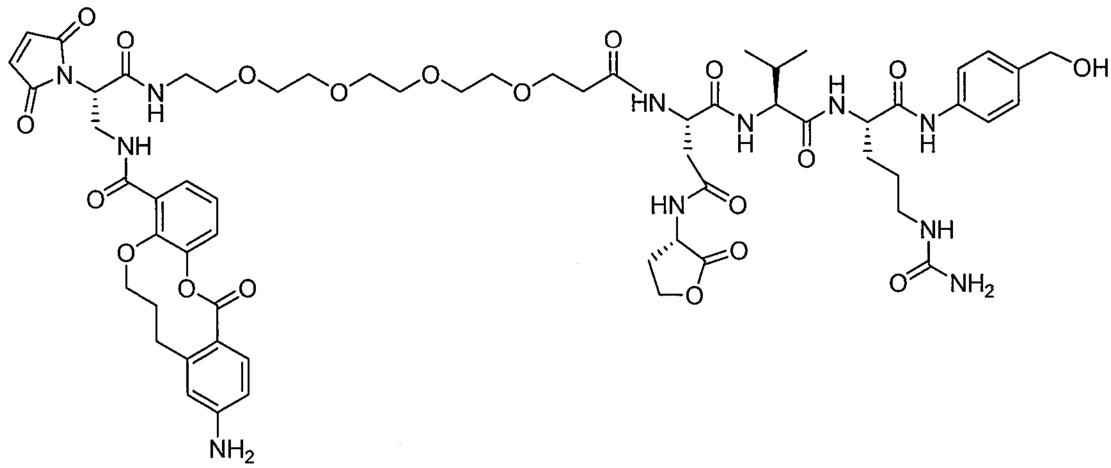

Exemplification 3-54-3

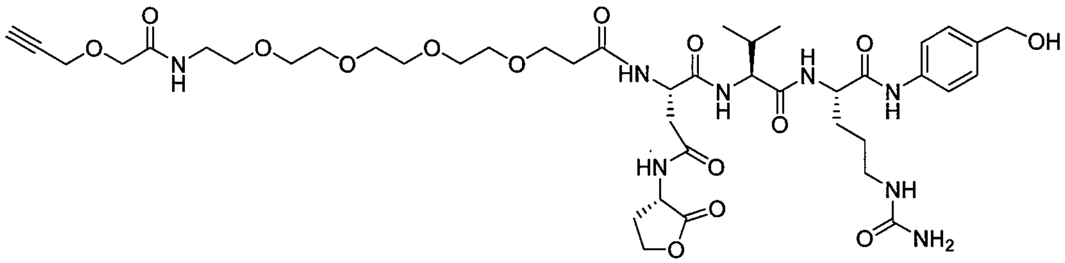

Exemplification 3-55-3

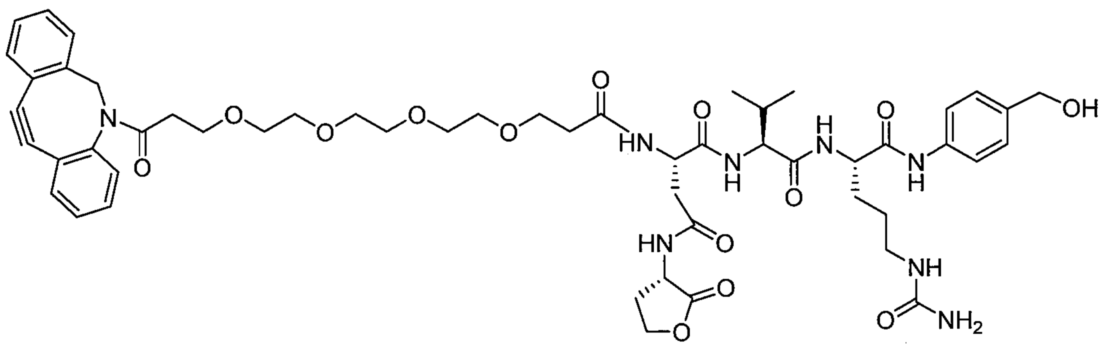

Exemplification 3-56-3

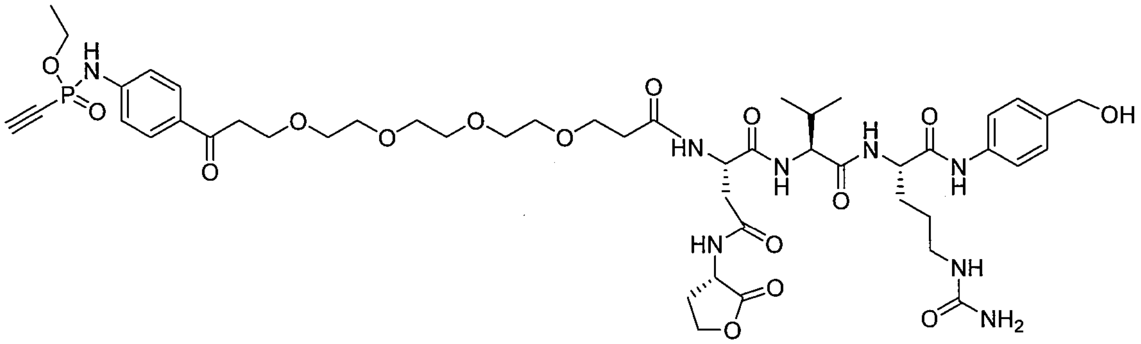

Exemplification 3-57-1

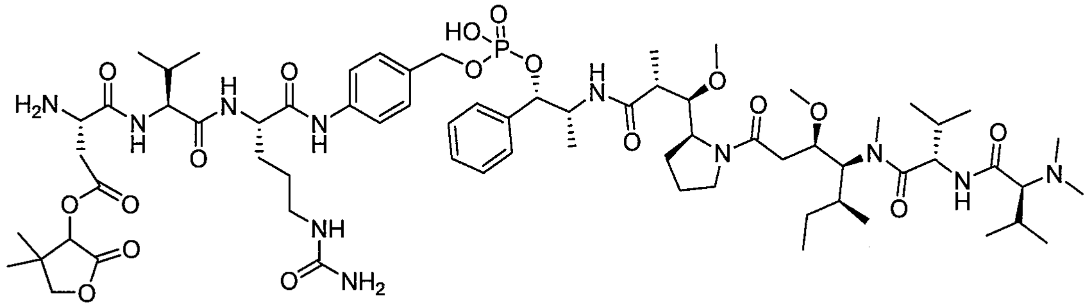

Exemplification 3-57-2

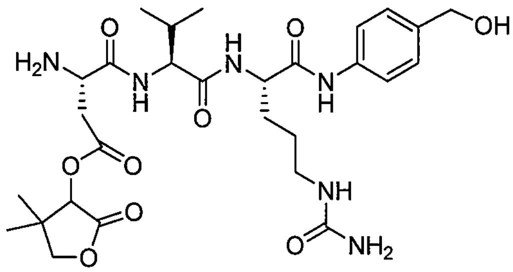

Exemplification 3-57-3

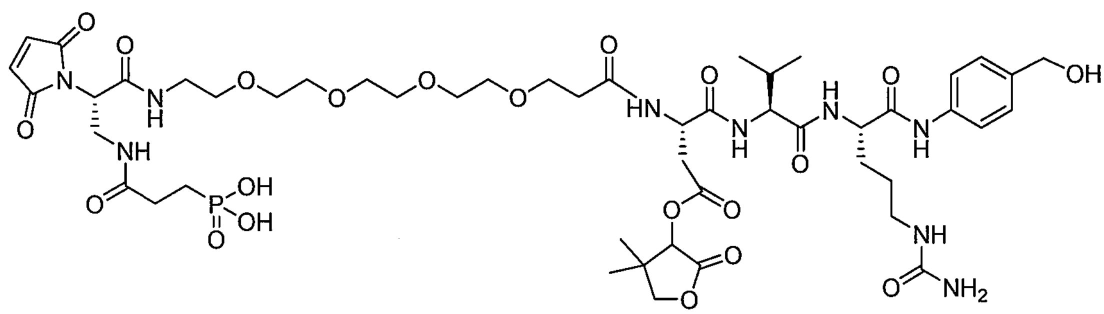

Exemplification 3-58-1

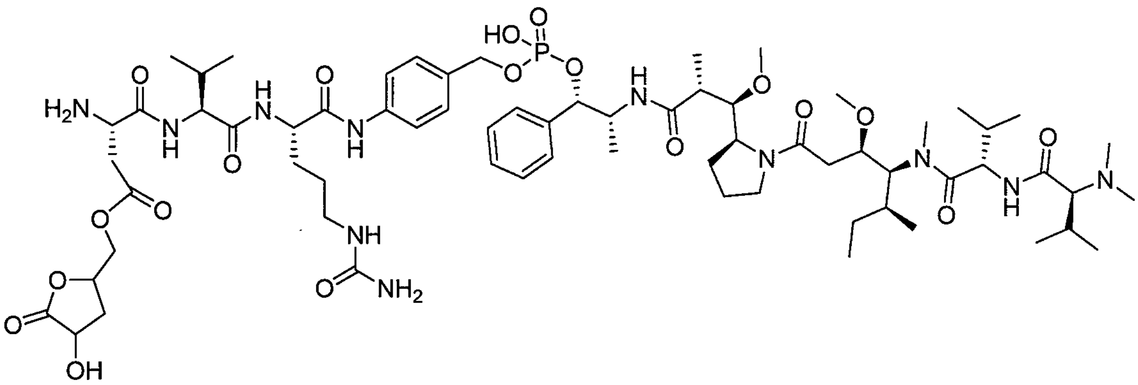

Exemplification 3-58-2

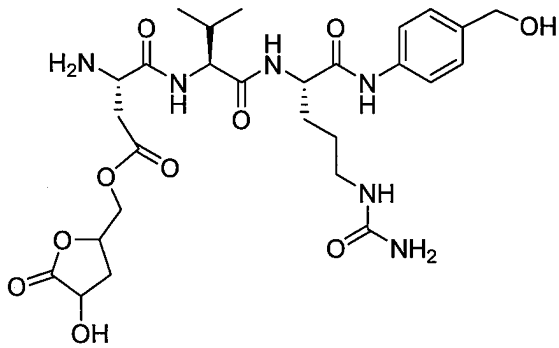

Exemplification 3-58-3

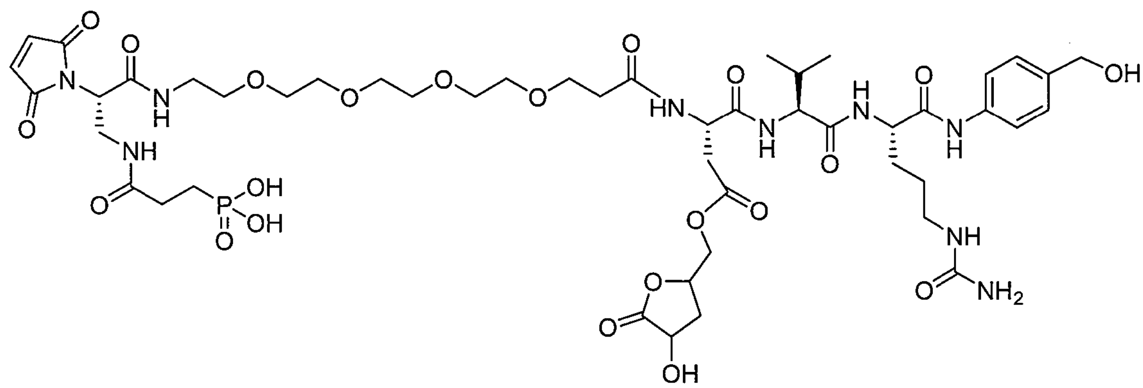

Exemplification 3-59-1

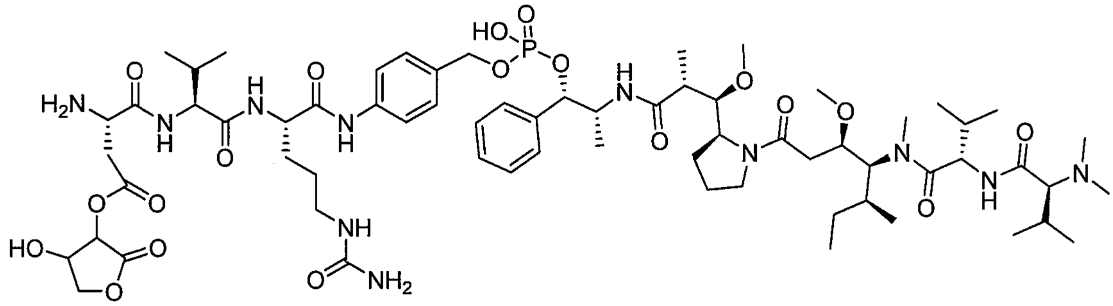

Exemplification 3-59-2

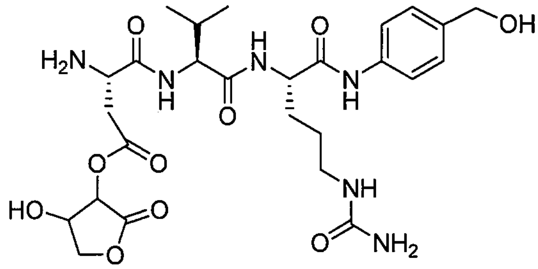

Exemplification 3-59-3

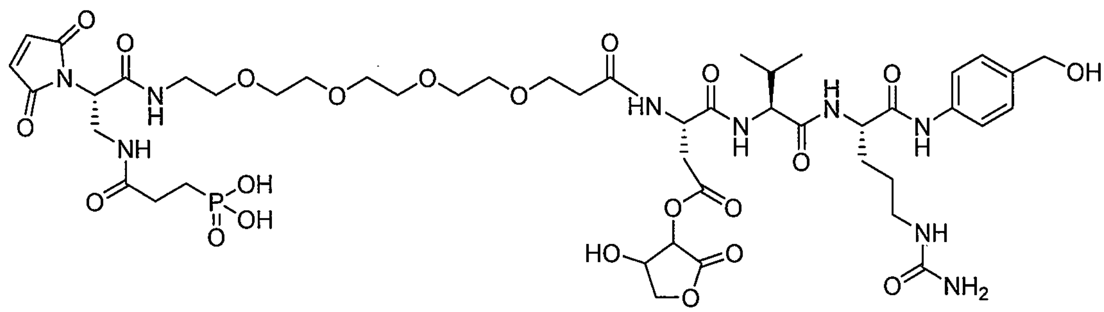

Exemplification 3-60-1

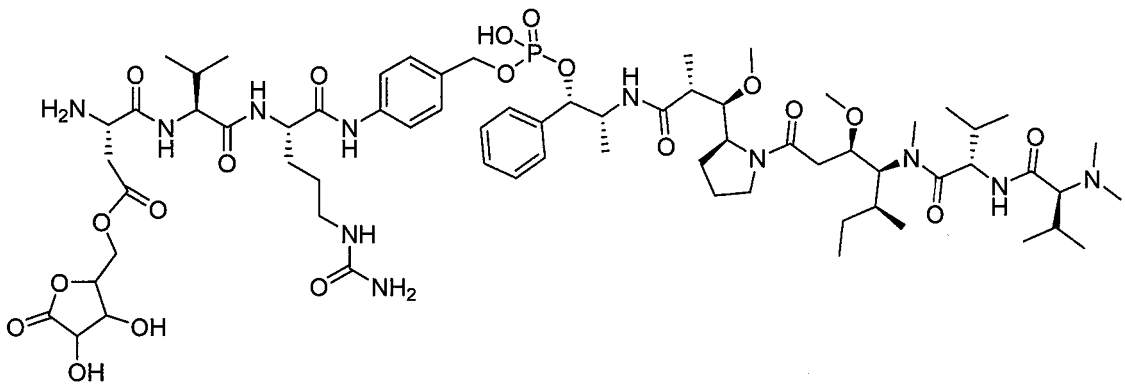

Exemplification 3-60-2

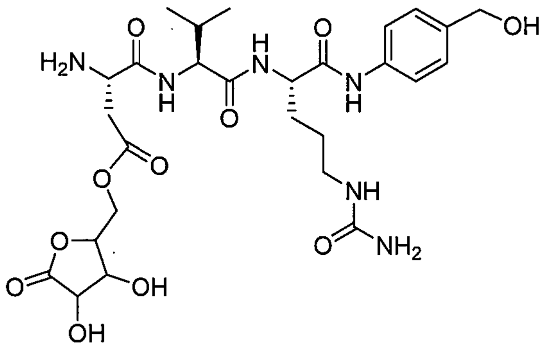

Exemplification 3-60-3

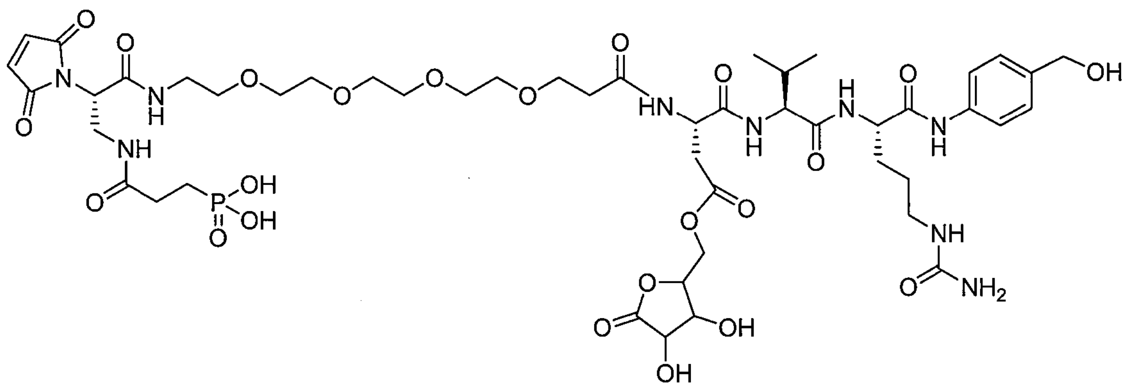

Exemplification 3-61-1

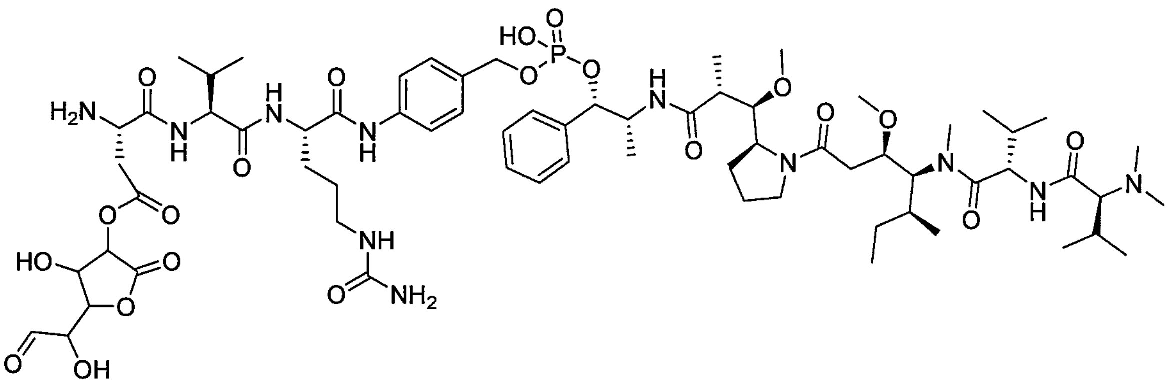

Exemplification 3-61-2

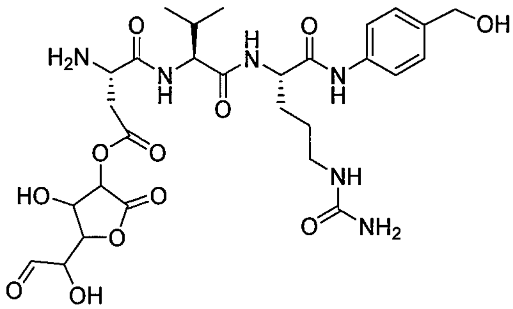

Exemplification 3-61-3

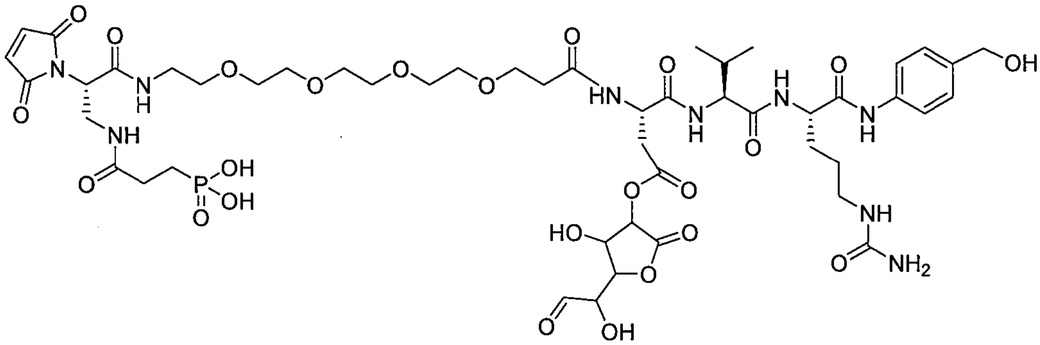

Exemplification 3-62-1

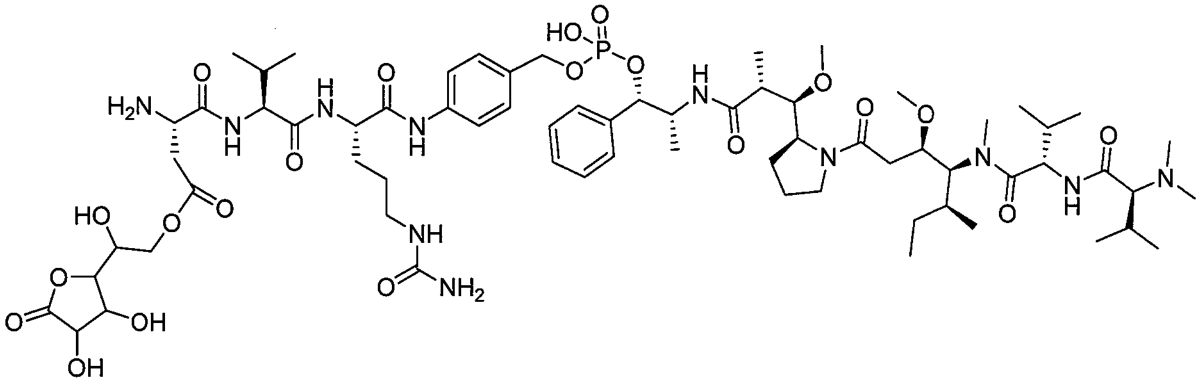

Exemplification 3-62-2

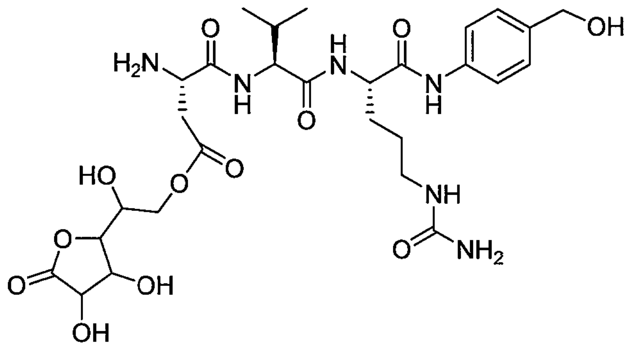

Exemplification 3-62-3

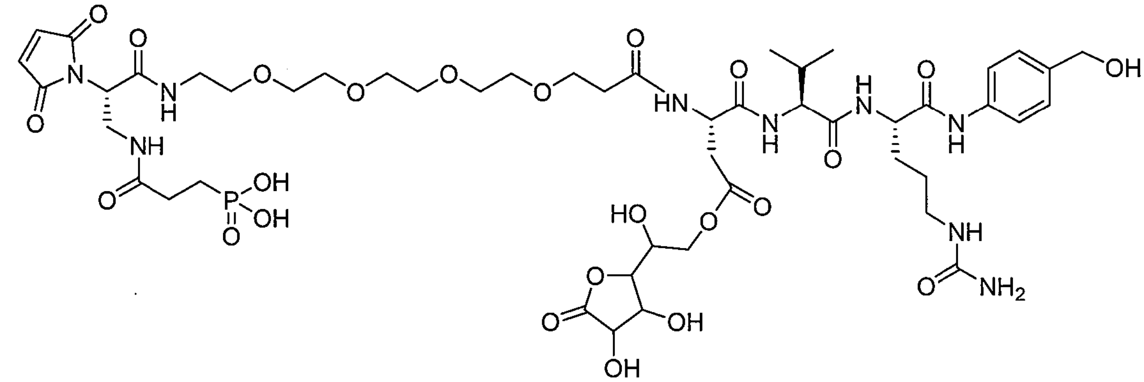

Exemplification 3-63-1

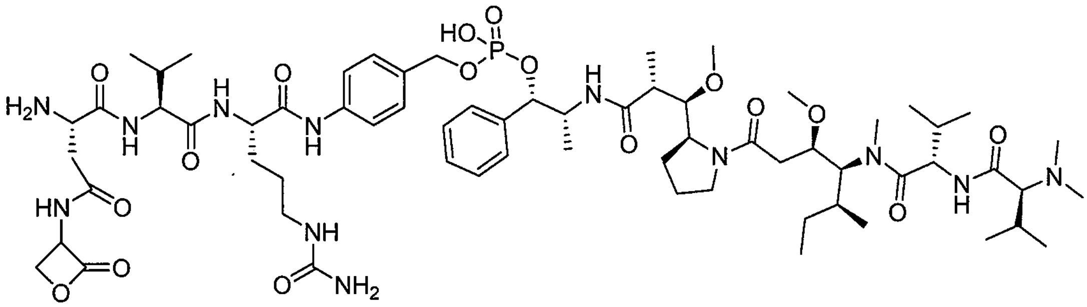

Exemplification 3-63-2

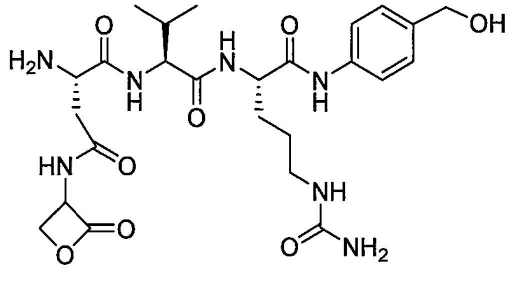

Exemplification 3-63-3

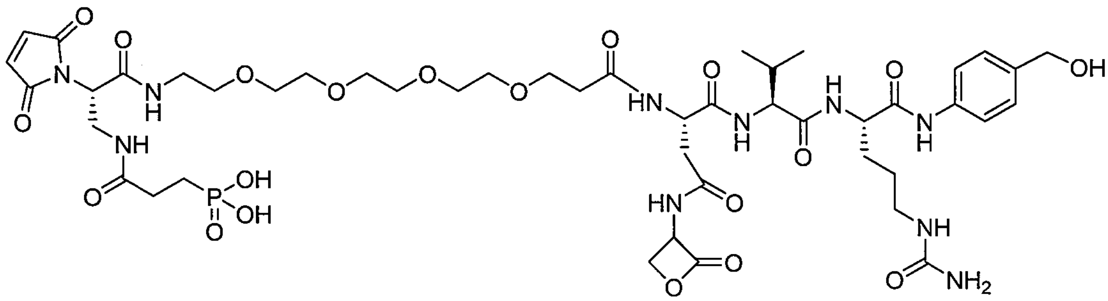

Exemplification 3-64-1

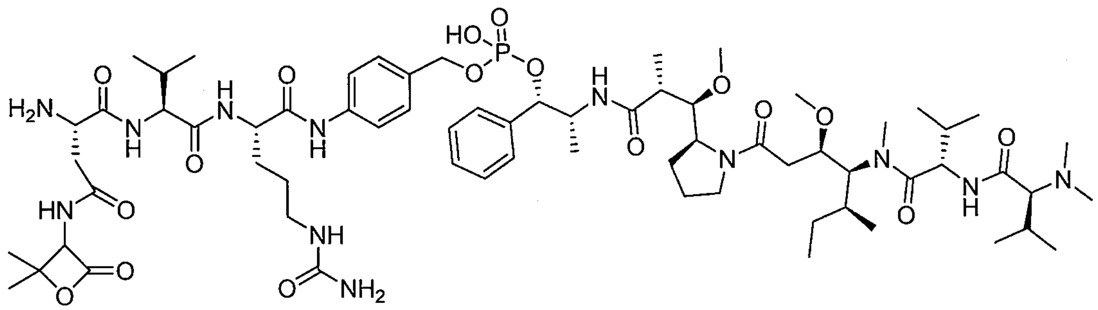

Exemplification 3-64-2

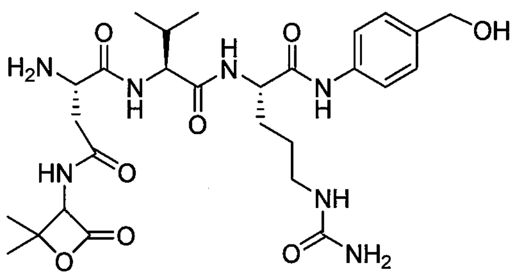

Exemplification 3-64-3

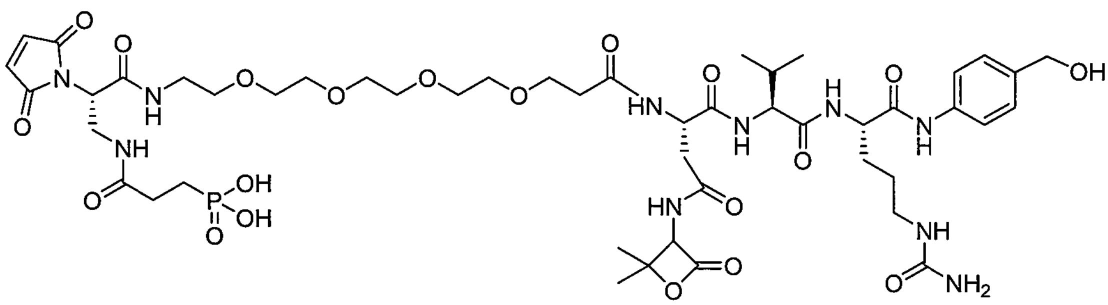

Exemplification 3-65-1

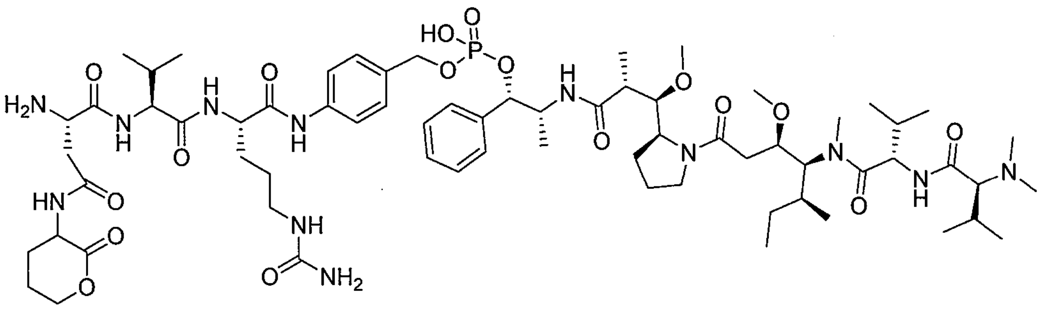

Exemplification 3-65-2

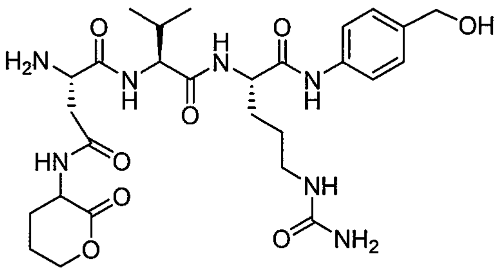

Exemplification 3-65-3

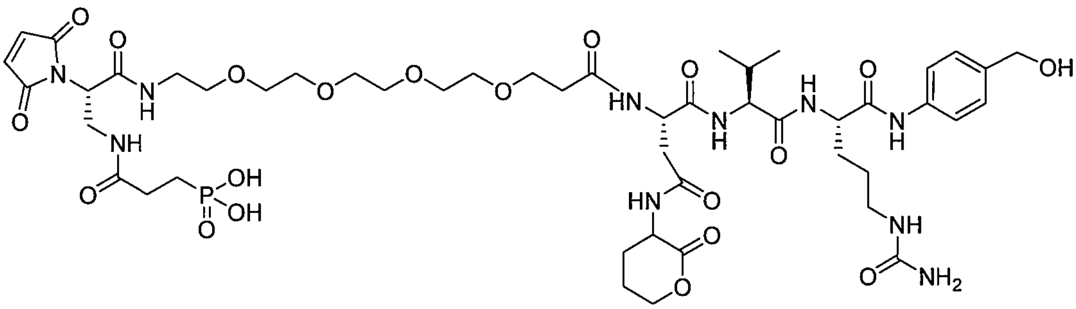

Exemplification 3-66-1

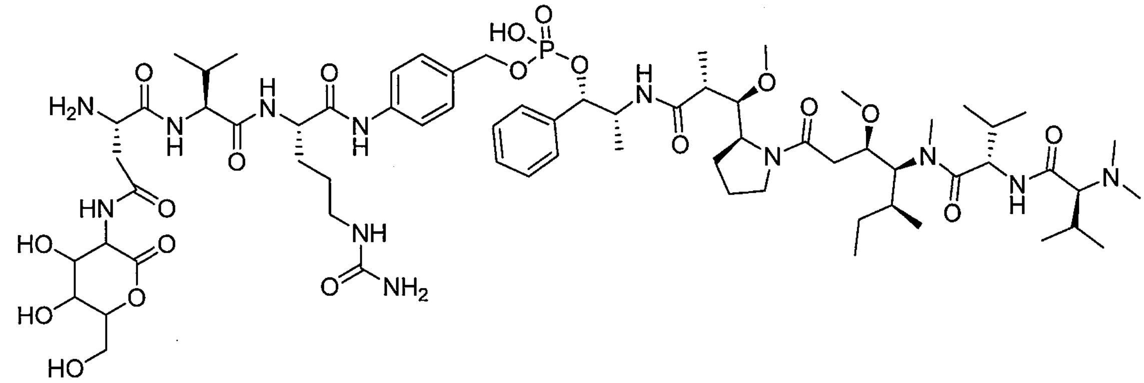

Exemplification 3-66-2

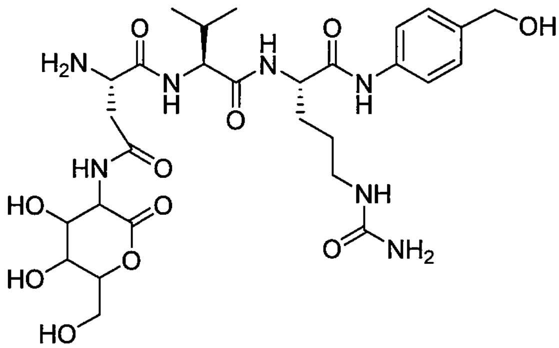

Exemplification 3-66-3

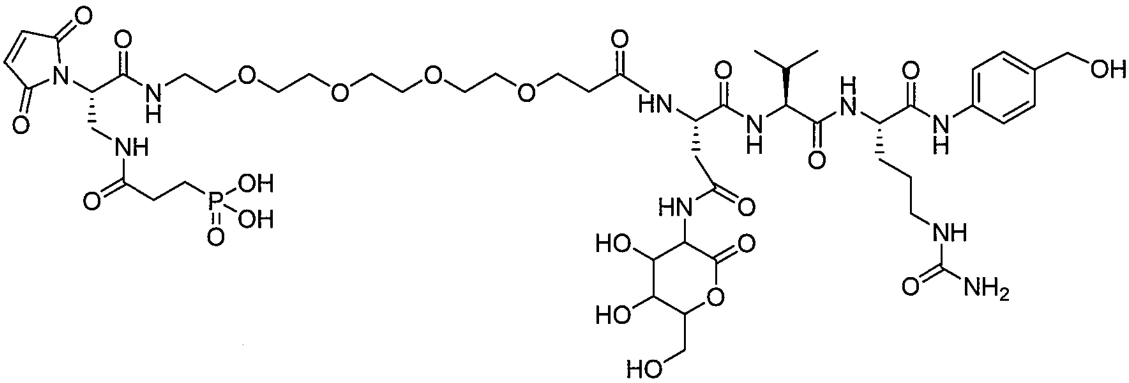

Exemplification 3-67-1

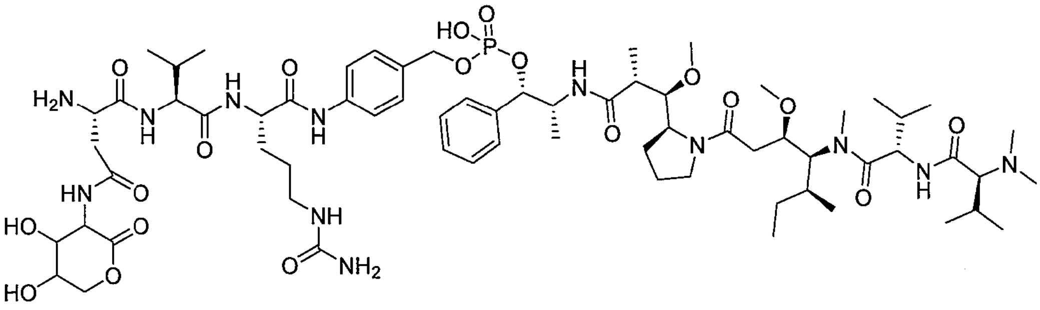

Exemplification 3-67-2

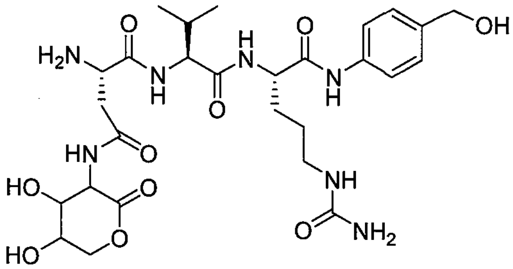

Exemplification 3-67-3

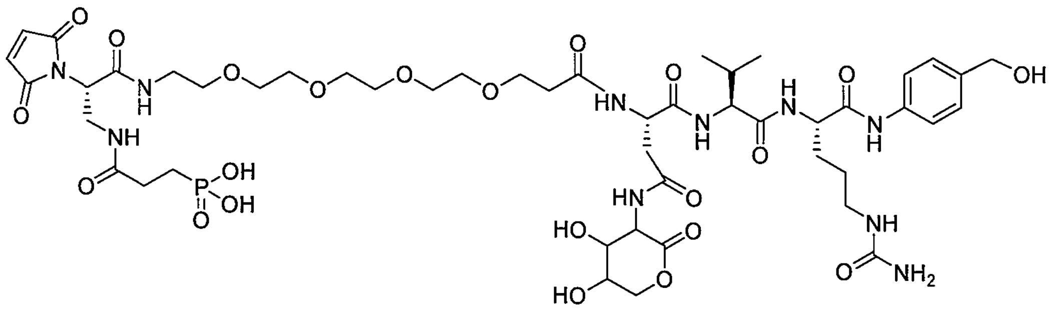

Exemplification 3-68-1

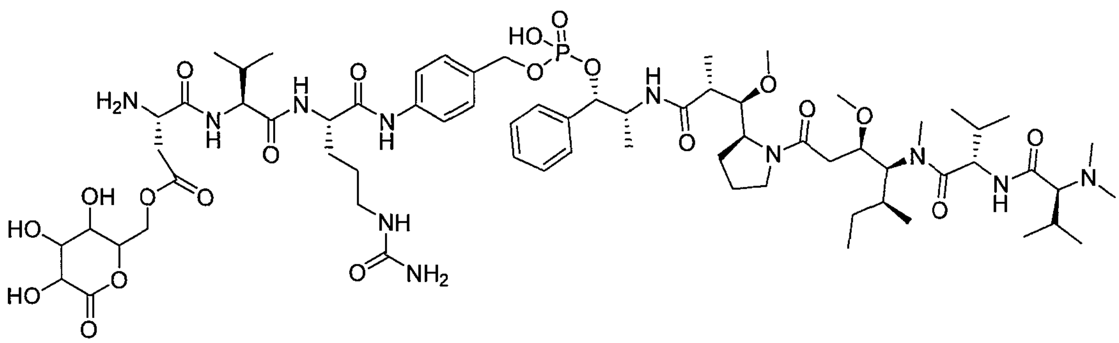

Exemplification 3-68-2

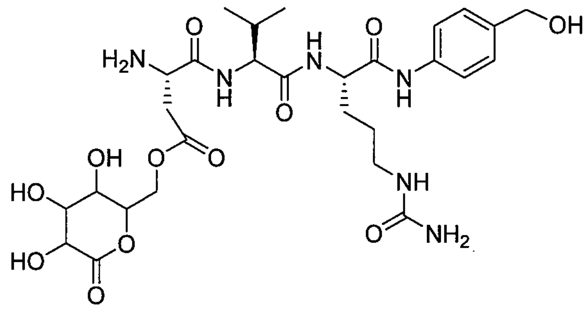

Exemplification 3-68-3

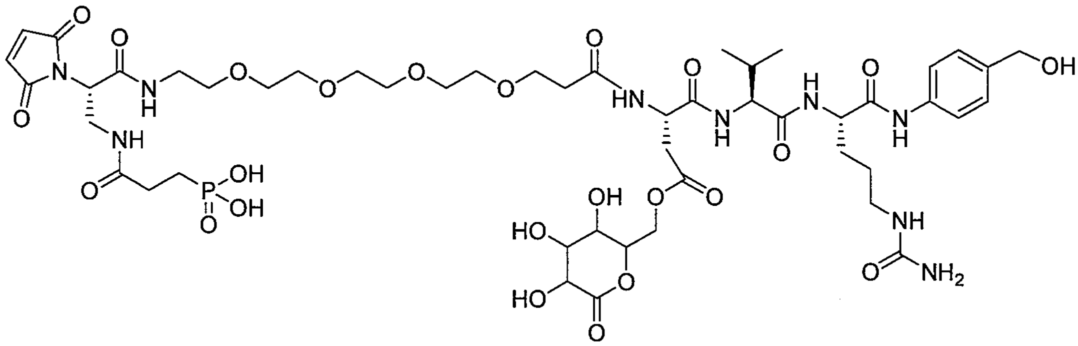

Exemplification 3-69-3

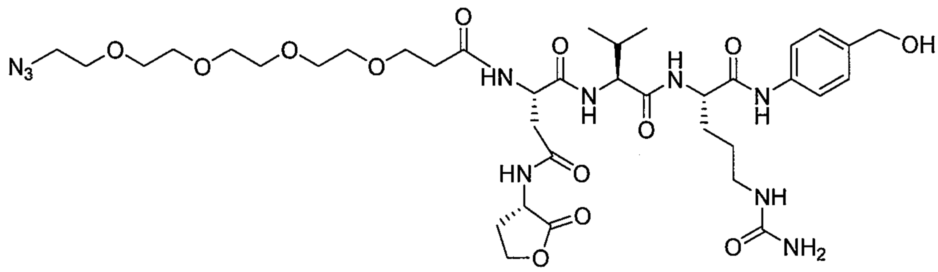

EXAMPLES

Hereinafter, synthesis examples of "an antitumor drug molecule or an analog thereof, or a derivative thereof" used in the present invention are described as "Production examples", synthesis examples of the conjugate precursor (I) and the synthetic intermediate (II) of conjugate precursor of the present invention are described as "Examples", and further production examples of antibody-drug conjugate (ADC) produced by the reaction of the conjugate precursor with an antibody and the evaluation test examples of said ADC are described as "Reference Example 1" and "Reference Example 2" respectively, but the present invention is not limited to them.

In the synthesis examples of the conjugate precursor (I) and the synthetic intermediate (II) of conjugate precursor (i.e. Examples), the stereoisomerism of amino acid residues of linker moieties is shown as L-type, but they may be each independently in D-type in the present invention, and the reaction of D-type is carried out in the same manner.

Also, in the Production examples, Examples, and Reference Examples, reagents, solvents, and starting

materials are easily available from commercially available supply sources, unless otherwise specified.

Abbreviations used below correspond to structures as follows.

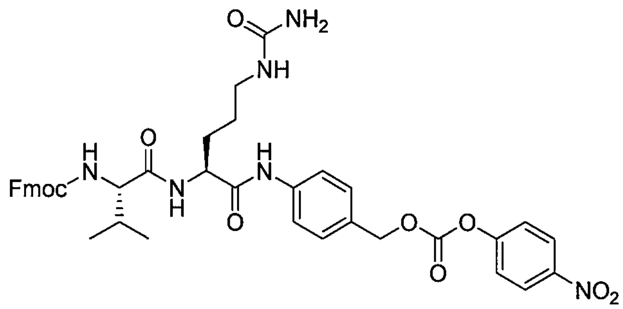

Fmoc-(L)-Val-(L)-Cit-PAB-PNP

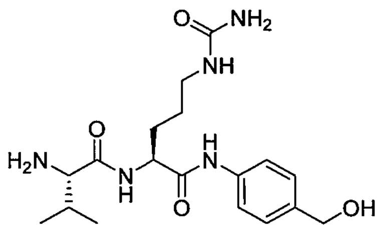

(L)-Val-(L)-Cit-PAB

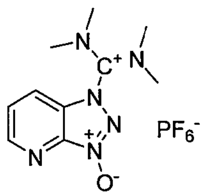

1-[bis(dimethylamino)methylene]-1H-1,2,3-triazolo[4,5-b]pyridinium 3-oxide hexafluorophosphate (hereinafter referred to as "HATU")

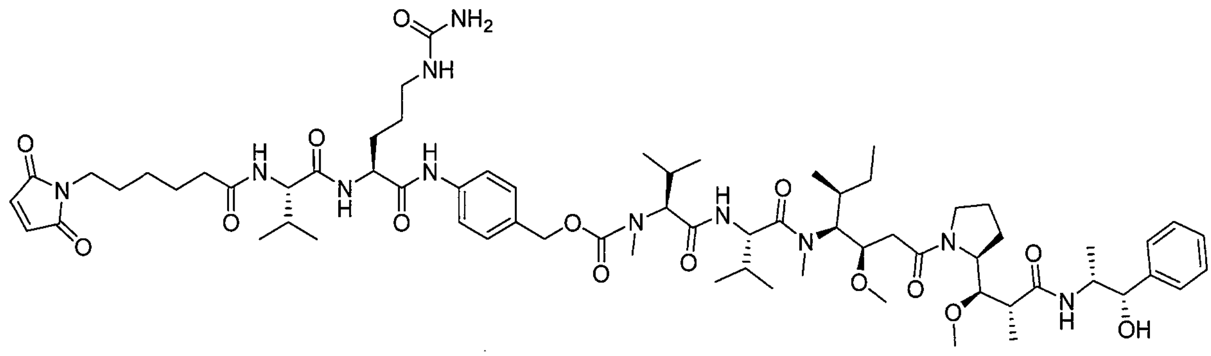

MC-Val-Cit-PAB-MMAE

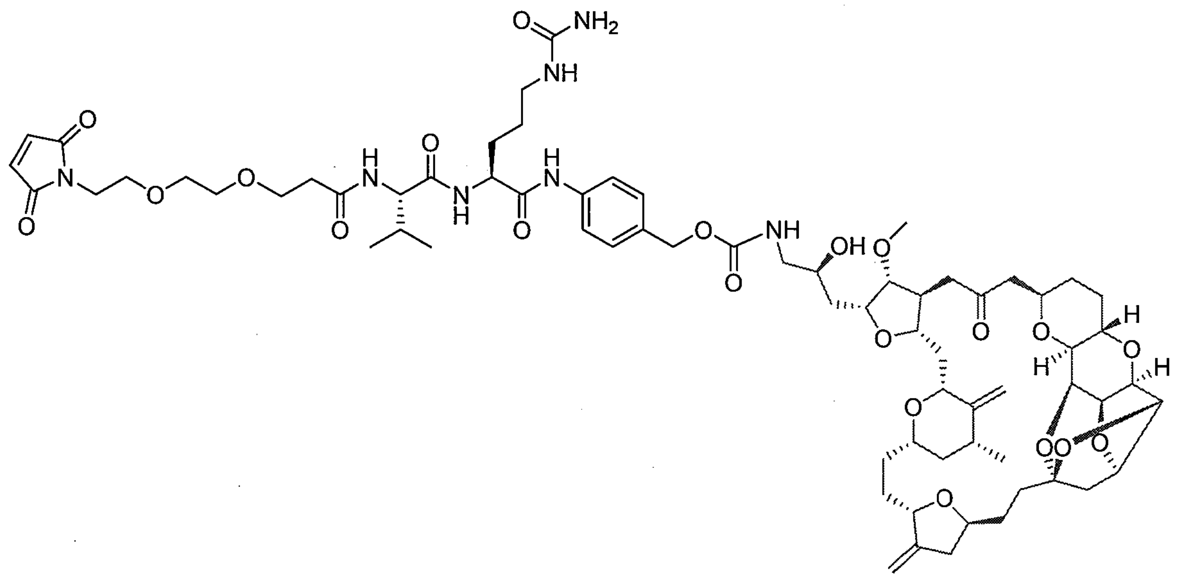

Mal-PEG2-Val-Cit-PAB-Eribulin

Production examples: Synthesis of antitumor drug molecules or analogs thereof, or derivatives thereof Production example 1. U-001

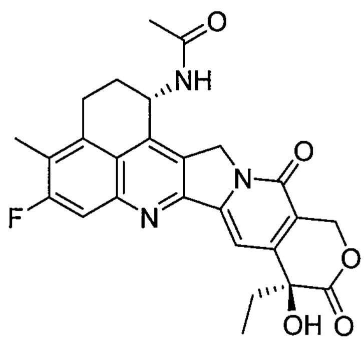

(U-001)

To a solution of exatecan mesylate (284.1 mg, 0.534 mmoL) and triethylamine (0.70 mL, 508.2 mg, 5.02 mmoL) in dichloromethane (5 mL) in a 30 mL cylindrical flask was added acetic anhydride (0.14 mL, 151.2 mg, 1.481 mmoL) under argon atmosphere with stirring at room temperature, and the resulting mixture was stirred at room temperature for 2 hours. After the reaction was completed, the resulting precipitates were filtered, and washed with dichloromethane to give U-001 (239.9 mg, yield: 94.0%) as light brown solids.
MS (ESI) m/z 478 $(M+H)^+$

Production example 2. U-002

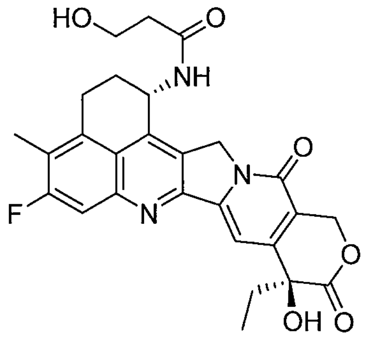

(U-002)

To a solution of exatecan mesylate (20.4 mg, 0.038 mmoL) in N,N-dimethylformamide (0.6 mL) in a 10 mL cylindrical flask were added 3-hydroxypropanoic acid (0.015 mL, 4.86 mg, 0.054 mmoL), 1-hydroxybenzotriazole (15.6 mg, 0.115 mmoL), 1-ethyl-3-(3-dimethylaminopropyl)carbodiimide hydrochloride (21.6 mg, 0.113 mmoL), and triethylamine (0.040 mL, 29.04 mg, 0.287 mmoL) under argon gas flow with stirring at room temperature, and the resulting mixture was stirred at room temperature for 3 hours. After the reaction was completed, to the reaction solution was added a saturated aqueous solution of sodium hydrogen carbonate, and the resulting mixed solution was subjected to extraction with ethyl acetate. The resulting organic layer was washed with saturated brine, dried over anhydrous magnesium sulfate, filtered, and concentrated under reduced pressure. The resulting residues were subjected to YAMAZEN medium pressure preparative (Silica, M (16 g), ethyl acetate / methanol = 97/3 (V/V) → 60/40 (V/V)), and the fraction comprising the target compound (Rf value = 0.44 (ethyl acetate / methanol = 85/15 (V/V))) was concentrated under reduced pressure to give U-002 (9.8 mg, yield: 50.31%) as pale yellow solids.
MS (ESI) m/z 508 $(M+H)^+$

Production example 3. U-003 3-1. U-003-1

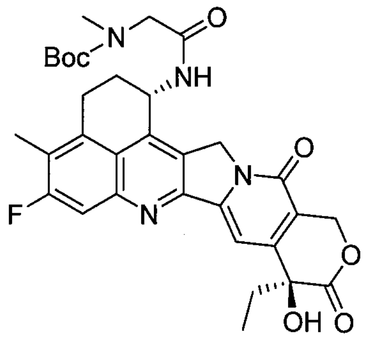

(U-003-1)

To a solution of exatecan mesylate (22 mg, 0.041 mmoL) in N,N-dimethylformamide (0.8 mL) in a 10 mL cylindrical flask were added N-(tert-butoxycarbonyl)-N-methylglycine (11 mg, 0.058 mmoL), 1-hydroxybenzotriazole (13 mg, 0.096 mmoL), 1-ethyl-3-(3-dimethylaminopropyl)carbodiimide hydrochloride (13 mg, 0.068 mmoL), and triethylamine (0.032 mL, 23.23 mg, 0.230 mmoL) under argon gas flow with stirring at room temperature, the resulting mixture was stirred at room temperature for 2 hours, and then left to stand overnight. After the reaction was completed, to the reaction solution was added a saturated aqueous solution of sodium hydrogen carbonate (5 mL × 2), and the resulting mixed solution was subjected to extraction with dichloromethane (15 mL). The resulting organic layer was washed with saturated brine (5 mL), dried over anhydrous magnesium sulfate, filtered, and concentrated under reduced pressure to give U-003-1 (29 mg, yield: quantitative).
MS (ESI) m/z 607 $(M+H)^+$

3-2. U-003

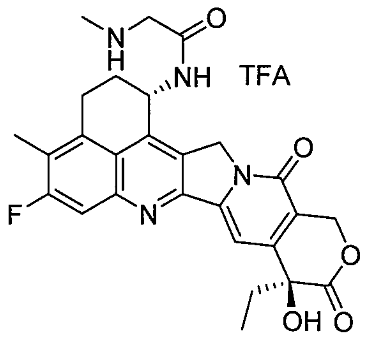

(U-003)

To a solution of U-003-1 (25 mg, 0.041 mmoL) in dichloromethane (0.6 mL) in a 20 mL cylindrical flask was added trifluoroacetic acid (0.050 mL, 74 mg, 0.649 mmoL) under argon gas flow with stirring at room temperature, the resulting mixture was stirred at room temperature for 1.5 hours, and then concentrated under reduced pressure. To the resulting residues was added methanol, the resulting mixture was concentrated under reduced pressure, then ethyl acetate was added thereto, and the resulting solids were separated by filtration. The resulting solids were dried under reduced pressure to give trifluoroacetate of U-003 (13 mg, yield: 50.83%) as brown solids.
MS (ESI) m/z 507 $(M+H)^+$

Production example 4. U-004

4-1. U-004-1

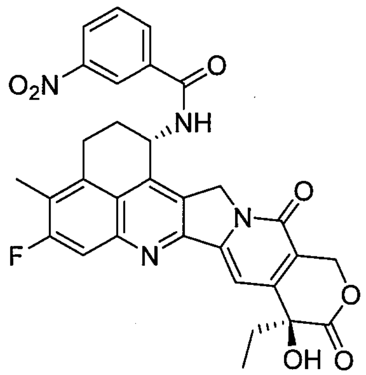

(U-004-1)

To a solution of exatecan mesylate (20.6 mg, 0.039 mmoL) and N,N-diisopropylethylamine (0.020 mL, 14.8 mg, 0.115 mmoL) in dichloromethane (1 mL) in a 10 mL cylindrical flask was added 3-nitrobenzoyl chloride (9.6 mg, 0.052 mmoL) with stirring at room temperature, and the resulting mixture was stirred at room temperature for 1 hour. After the reaction was completed, to the reaction solution was added water, and the resulting mixed solution was subjected to extraction with ethyl acetate. The resulting organic layer was concentrated under reduced pressure to give U-004-1 (22.6 mg, yield: 99.76%).

4-2. U-004

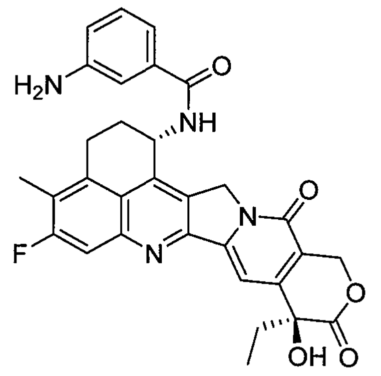

(U-004)

A solution of U-004-1 (22.6 mg, 0.039 mmoL) and 10% palladium carbon NX-Type (20.6 mg, 0.0968 mmoL) in a mixture of ethanol (1 mL) / dichloromethane (1 mL) in a 30 mL round-bottom flask was stirred under hydrogen atmosphere at room temperature for 2 hours. After the reaction was completed, the catalyst was separated by filtration, and the solvent was concentrated under reduced pressure. The resulting residues were subjected to YAMAZEN medium pressure preparative (Silica, M (16 g), dichloromethane/methanol = 100/0 (V/V) -> 94/6 (V/V)), and the fraction comprising the target compound was concentrated under reduced pressure to give U-004 (9.4 mg, yield: 43.84%) as slightly yellow solids.
MS (DUIS) m/z 555 $(M+H)^+$

Production example 5. U-005

5-1. U-005-1

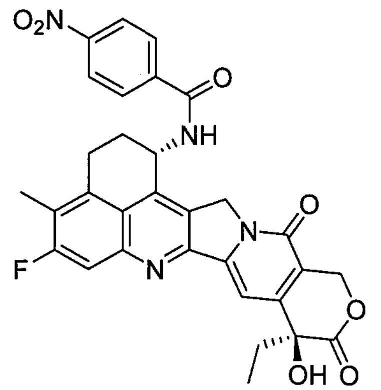

(U-005-1)

To a solution of exatecan mesylate (21.2 mg, 0.040 mmoL) and N,N-diisopropylethylamine (0.020 mL, 14.8 mg, 0.115 mmoL) in dichloromethane (1 mL) in a 10 mL cylindrical flask was added 4-nitrobenzoyl chloride (10.6 mg, 0.057 mmoL) with stirring at room temperature, and the resulting mixture was stirred at room temperature for 1 hour. Subsequently, 4-nitrobenzoyl chloride (10.6 mg, 0.057 mmoL) was added thereto under stirring at room temperature, and the resulting mixture was stirred at room temperature for 1 hour. After the reaction was completed, to the reaction solution were added water and tert-butyl methyl ether, the resulting mixture was stirred, the resulting solids were filtered, and sequentially washed with water and tert-butyl methyl ether to give U-005-1 (22.1 mg, yield: 94.79%).

5-2. U-005

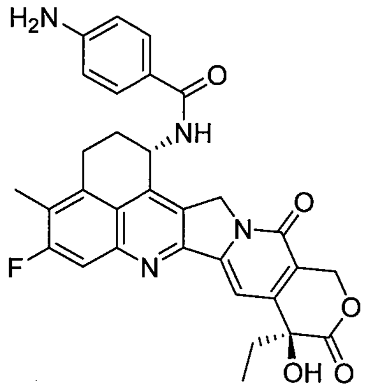

(U-005)

A solution of U-005-1 (22.6 mg, 0.039 mmoL) and 10% palladium carbon NX-Type (15.4 mg, 0.0724 mmoL) in a mixture of ethanol (1 mL) / dichloromethane (1 mL) in a 30 mL round-bottom flask was stirred under hydrogen atmosphere at room temperature for 2 hours. After the reaction was completed, the catalyst was separated by filtration, and the solvent was concentrated under reduced pressure. The resulting residues were subjected to YAMAZEN medium pressure preparative (Silica, M (16 g), dichloroethane/methanol = 100/0 (V/V) -> 94/6 (V/V)), and the fraction comprising the target compound was concentrated under reduced pressure to give crude products of U-005. The resulting crude products were subjected to preparative HPLC chromatography under the following conditions, the fraction comprising the target compound was neutralized by a saturated aqueous solution of sodium hydrogen carbonate, the resulting mixture was concentrated under reduced pressure, then to the precipitated solids was added water, the resulting insoluble matters were collected by filtration, and washed with water to give U-005 (7.1 mg, yield: 33.11%) as slightly yellow solids.
MS (ESI) m/z 555 $(M+H)^+$

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) → 95% (10.00 min.)

Production example 6. U-006

6-1. U-006-1

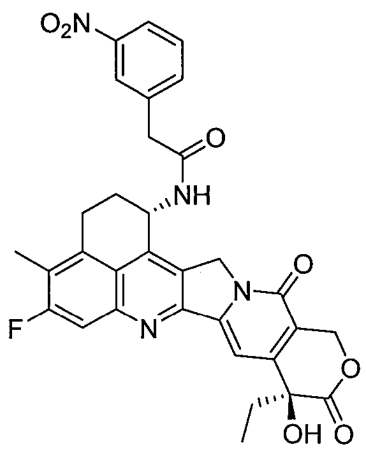

(U-006-1)

To a solution of exatecan mesylate (23.1 mg, 0.043 mmoL), 2-(3-nitrophenyl)acetic acid (15.7 mg, 0.087 mmoL), 1-ethyl-3-(3-dimethylaminopropyl)carbodiimide hydrochloride (16.7 mg, 0.087 mmoL), and 1-hydroxybenzotriazole (13.3

mg, 0.087 mmoL) in N,N-dimethylformamide in a 10 mL cylindrical flask was added triethylamine (0.012 mL, 8.71 mg, 0.086 mmoL) under argon atmosphere with stirring at room temperature, and the resulting mixture was stirred at room temperature for 1 hour. After the reaction was completed, to the reaction solution was added a saturated aqueous solution of sodium hydrogen carbonate, and the resulting mixed solution was subjected to extraction with ethyl acetate. The resulting organic layer was concentrated under reduced pressure to give crude products of U-006-1 (38.4 mg).
MS (ESI) m/z 599 $(M+H)^+$

6-2. U-006

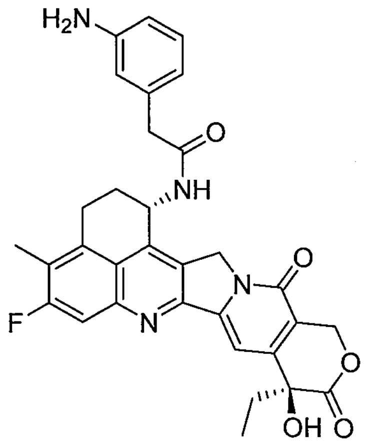

(U-006)

A solution of U-006-1 (38.4 mg, 0.044 mmoL) and 10% palladium carbon NX-Type (21.3 mg, 0.200 mmoL) in a mixture of ethanol (1 mL) / dichloromethane (1 mL) in a 30 mL round-bottom flask was stirred under hydrogen atmosphere at room temperature for 2 hours. After the reaction was completed, the catalyst was separated by filtration, and the solvent was concentrated under reduced pressure. The resulting residues were subjected to YAMAZEN medium pressure preparative (Silica, M (16 g), dichloroethane/methanol = 100/0 (V/V) → 94/6 (V/V)), and the fraction comprising the target compound was concentrated under reduced pressure. To the resulting residues was added ethanol, the precipitated solids were collected by filtration, and washed with ethanol to give U-006 (16.6 mg, total yield of 6-1 and 6-2: 66.92%) as colorless solids.
MS (DUIS) m/z 569 $(M+H)^+$

Production example 7. U-007

7-1. U-007-1

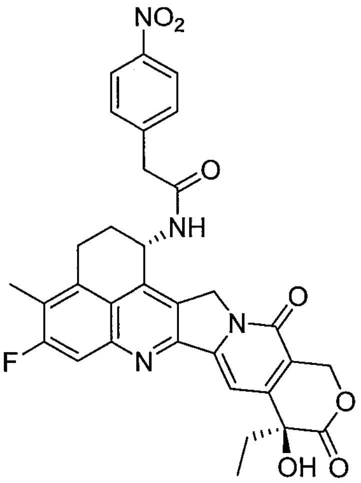

(U-007-1)

To a solution of exatecan mesylate (32.5 mg, 0.061 mmoL), 2-(4-nitrophenyl)acetic acid (22.1 mg, 0.122 mmoL), 1-ethyl-3-(3-dimethylaminopropyl)carbodiimide hydrochloride (23.4 mg, 0.122 mmoL), and 1-hydroxybenzotriazole (16.7 mg, 0.109 mmoL) in N,N-dimethylformamide (0.61 mL) in a 10 mL cylindrical flask was added triethylamine (0.017 mL, 12.34 mg, 0.122 mmoL) with stirring at room temperature, and the resulting mixture was stirred at room temperature for 1 hour. After the reaction was completed, to the reaction solution was added a saturated aqueous solution of sodium hydrogen carbonate, and the resulting mixed solution was subjected to extraction with ethyl acetate. The resulting organic layer was concentrated under reduced pressure to give U-007-1 (26.8 mg, yield: 73.23%).
MS (ESI) m/z 598 $(M+H)^+$

7-2. U-007

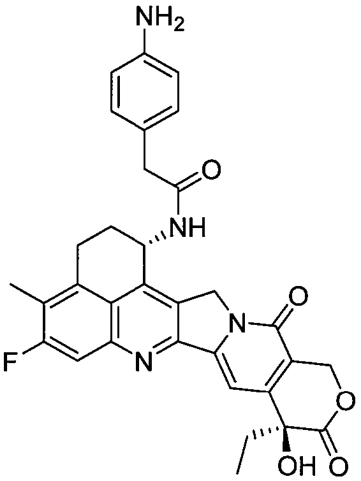

(U-007)

A solution of U-007-1 (26.8 mg, 0.045 mmoL) and 10% palladium carbon NX-Type (20.3 mg, 0.0954 mmoL) in a mixture of ethanol (1 mL) / dichloromethane (1 mL) in a 30 mL round-bottom flask was stirred under hydrogen atmosphere at room temperature for 2 hours. After the reaction was completed, the catalyst was separated by filtration, and the solvent was concentrated under reduced pressure. To the resulting residues was added ethanol, the precipitated solids were collected by filtration, and washed with ethanol to give U-007 (24.2 mg, yield: 95.06%) as light brown solids.
MS (DUIS) m/z 569 $(M+H)^+$

Production example 8. U-008

8-1. U-008-1

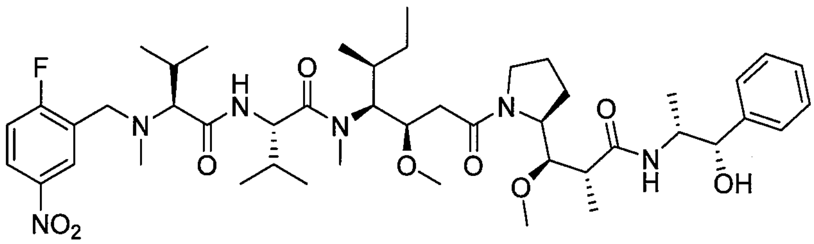

(U-008-1)

To a solution of MMAE (0.36 g, 0.501 mmoL) and 2-fluoro-5-nitrobenzaldehyde (0.25 g, 1.478 mmoL) in dichloromethane (5 mL) in a 30 mL cylindrical flask was added sodium triacetoxyborohydride (0.32 g, 1.510 mmoL) under argon atmosphere with stirring at room temperature, and the resulting mixture was stirred at room temperature for 1 hour. 2-fluoro-5-nitrobenzaldehyde (0.25 g, 1.478 mmoL) and sodium triacetoxyborohydride (0.32 g, 1.510 mmol) were additionally added thereto, and the resulting mixture was stirred at room temperature for 1 hour. After the reaction was completed, to the reaction solution was added a saturated aqueous solution of sodium hydrogen carbonate, and the resulting mixed solution was subjected to extraction with dichloromethane. The resulting organic layer was dried over anhydrous magnesium sulfate, filtered, and concentrated under reduced pressure. The resulting residues were subjected to YAMAZEN medium pressure preparative (Silica, M (16 g), ethyl acetate : methanol = 100/0 (V/V) → 90/10 (V/V)), and the fraction comprising the target compound was concentrated under reduced pressure to give U-008-1 (406.6 mg, yield: 93.09%) as pale yellow foam. MS (DUIS) m/z 873 $(M+H)^+$

8-2. U-008

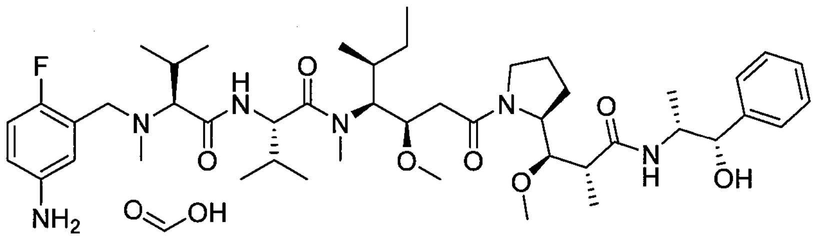

(U-008)

A solution of U-008-1 (45.1 mg, 0.052 mmoL) and zinc (89.3 mg, 1.366 mmoL) in acetic acid (0.2 mL) in a 30 mL cylindrical flask was stirred under argon atmosphere at room temperature for 2 hours. After the reaction was completed, to the reaction solution was added a saturated aqueous solution of sodium hydrogen carbonate, and the resulting mixed solution was subjected to extraction with ethyl acetate. The resulting organic layer was dried over anhydrous magnesium sulfate, filtered, and concentrated under reduced pressure. The resulting residues were subjected to YAMAZEN medium pressure preparative (Silica, M (16 g), ethyl acetate / methanol = 100/0 (V/V) → 90/10 (V/V)), and the fraction comprising the target compound was concentrated under reduced pressure. The resulting residues were subjected to preparative HPLC chromatography under the following conditions, the fraction comprising the target compound was concentrated under reduced pressure to distill away acetonitrile, and then the resulting residues were freeze-dried to give formate of U-008 (13.3 mg, yield: 28.96%) as colorless foam.
MS (DUIS) m/z 841 $(M+H)^+$

Column: Waters SunFire Prep C18 5 μm ODB 19*150 mm
Flow rate: 17 mL/min.

Elution solvent: 0.1% aqueous solution of formic acid (Solution A) - acetonitrile (Solution B)
Gradient (Solution B): 30% (0.00 min.) → 95% (10.00 min.)

Production example 9. U-009

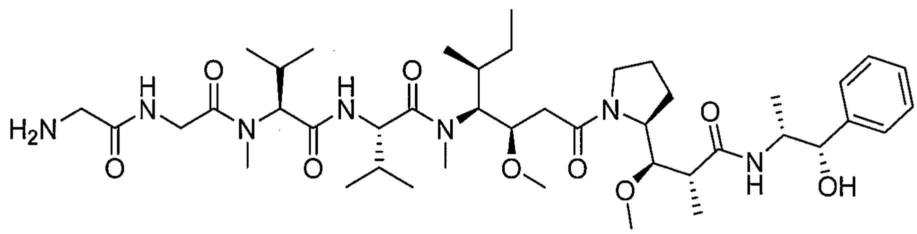

(U-009)

To a solution of (tert-butoxycarbonyl)glycylglycine (8.4 mg, 0.036 mmoL), 1-hydroxybenzotriazole (6.2 mg, 0.046 mmoL), and MMAE (22.3 mg, 0.031 mmoL) in N,N-dimethylformamide (0.1 mL) in a 10 mL cylindrical flask was added 1-ethyl-3-(3-dimethylaminopropyl)carbodiimide hydrochloride (8.8 mg, 0.046 mmoL) under argon gas flow with stirring at room temperature, and the resulting mixture was stirred at 60°C for 1 hour. The resulting reaction solution was subjected to preparative HPLC chromatography under the following conditions, and the fraction comprising the target compound was concentrated under reduced pressure to give a colorless oil.

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) → 95% (10.00 min.)

The resulting compound was dissolved in dichloromethane (1 mL) and trifluoroacetic acid (1 mL), the resulting mixture was stirred at room temperature for 1 hour, and then concentrated under reduced pressure. The resulting reaction solution was subjected to preparative HPLC chromatography under the following conditions, and the fraction comprising the target compound was concentrated under reduced pressure. Subsequently, the resulting residues were subjected to DNH silica gel column chromatography, eluted with dichloromethane/methanol (9/1 (V/V)), and the fraction comprising the target compound was concentrated under reduced pressure. Subsequently, the resulting residues were dissolved in a mixed solvent of water/acetonitrile, and freeze-dried to give the Production example 9 (U-009) (2.2 mg, yield: 8.51%) as colorless solids.
MS (ESI) m/z 833 $(M+H)^+$

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) → 95% (10.00 min.)

Production example 10. U-010

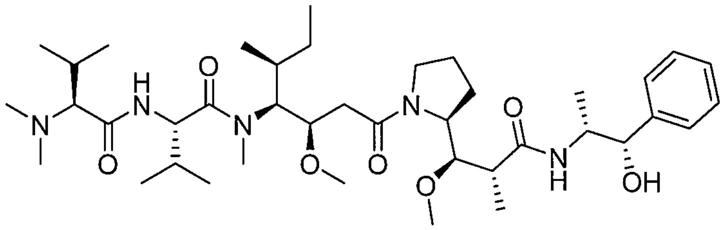

(U-010)

To a solution of MMAE (0.37 g, 0.515 mmoL) and a 37% aqueous solution of formaldehyde (0.37 mL, 0.4 g, 4.97 mmoL) in methanol (5 mL) in a 30 mL cylindrical flask was added sodium triacetoxyborohydride (0.32 g, 1.510 mmoL) under argon atmosphere with stirring at room temperature, and the resulting mixture was stirred at room temperature for 1 hour. After the reaction was completed, to the reaction solution was added a saturated aqueous solution of sodium hydrogen carbonate, and the resulting mixed solution was subjected to extraction with ethyl acetate. The resulting organic layer was dried over anhydrous magnesium sulfate, filtered, and concentrated under reduced pressure. The resulting residues were subjected to YAMAZEN medium pressure preparative (Silica, M (16 g), dichloromethane/methanol = 100/0 (V/V) → 90/10 (V/V)), and the fraction comprising the target compound was concentrated under reduced pressure to give U-010 (0.18 g, yield: 47.72%) as colorless foam.
MS (DUIS) m/z 732 $(M+H)^+$

Production example 11. U-011

11-1. U-011-1

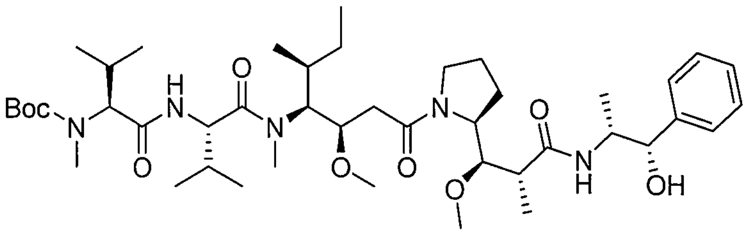

(U-011-1)

To a solution of MMAE (0.4996 g, 0.696 mmoL) and sodium carbonate (0.1503 g, 1.418 mmoL) in tetrahydrofuran (5 mL) in a 30 mL cylindrical flask was added water (5 mL), added di-tert-butyl dicarbonate (0.185 mL, 0.19 g, 0.848 mmoL) with stirring at room temperature, and the resulting mixture was stirred at room temperature for 18 hours and then stirred at 50°C for 1 hour. Di-tert-butyl dicarbonate (0.015 mL, 0.02 g, 0.069 mmoL) was added thereto, and the resulting mixture was stirred at 50°C for 1 hour. Di-tert-butyl dicarbonate (0.015 mL, 0.02 g, 0.069 mmoL) was additionally added thereto, and the resulting mixture was stirred at 50°C for 2 hours. After the reaction was completed, to the reaction solution were added ethyl acetate and saturated brine, and the resulting mixture was stirred at room temperature for a while. The resulting organic layer was dried over anhydrous magnesium sulfate, filtered, and concentrated under reduced pressure to give U-011-1 (570.5 mg, yield: quantitative) as white foam.
MS (ESI) m/z 818 $(M+H)^+$

11-2. U-011-2

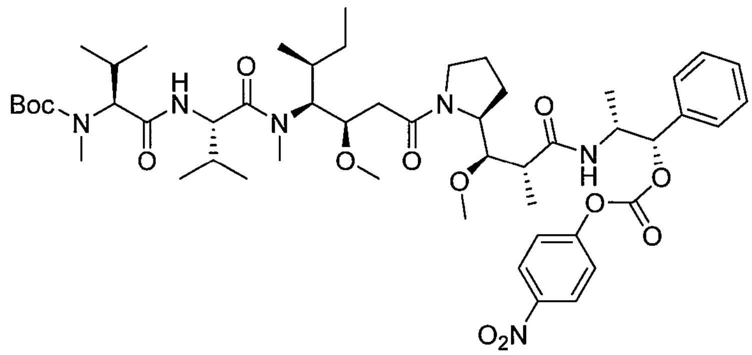

(U-011-2)

To a solution of U-011-1 (51.0 mg, 0.062 mmoL) and bis(4-nitrophenyl)carbonate (38.8 mg, 0.128 mmoL) in dichloromethane (1 mL) in a 0.2-0.5 mL microwave reaction container was added N,N-diisopropylethylamine (0.035 mL, 25.97 mg, 0.201 mmoL), the container was nitrogen-sealed, and the resulting mixture was stirred at room temperature for 3 hours. Subsequently, the mixture was subjected to a microwave reactor (Biotage), and reacted at 80°C for 1 hour. Subsequently, bis(4-nitrophenyl)carbonate (100.0 mg, 0.329 mmoL) and dichloromethane (0.5 mL) were additionally added thereto, and the resulting mixture was stirred at room temperature for 66 hours. The reaction solution was subjected to YAMAZEN medium pressure preparative (Universal Premium silica gel, S (7 g) (biconnected), eluted with hexane / ethyl acetate (= 33/67 (V/V) → 12/88 (V/V))), and the fraction comprising the target compound was concentrated under reduced pressure to give U-011-2 (46.7 mg, yield: 76.19%) as slightly yellow foam.
MS (ESI) m/z 983 $(M+H)^+$

11-3. U-011-3

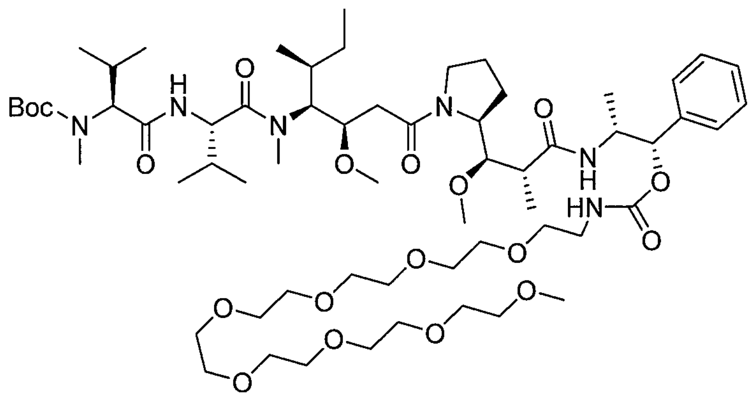

(U-011-3)

To a solution of U-011-2 (46.7 mg, 0.047 mmoL) and 3,6,9,12,15,18,21,24-octaoxapentacosan-1-amine (34.3 mg, 0.089 mmoL) in dichloromethane (2 mL) in a 20 mL pear-shaped flask was added N,N-diisopropylethylamine (0.030 mL, 22.26 mg, 0.172 mmoL), the resulting mixture was stirred under argon atmosphere at room temperature for 4 hours, and then left to stand at room temperature for 16.75 hours. The reaction solution was diluted with dichloromethane, packaged in Fuji Silysia Chromatorex Q-Pack DIOL-60 Size 20, eluted with hexane / ethyl acetate (= 0,100), and the fraction comprising the target compound was concentrated under reduced pressure to give U-011-3 (49.2 mg, yield: 84.38%) as a colorless oil.
MS (ESI) m/z 1227 $(M+H)^+$

11-4. U-011

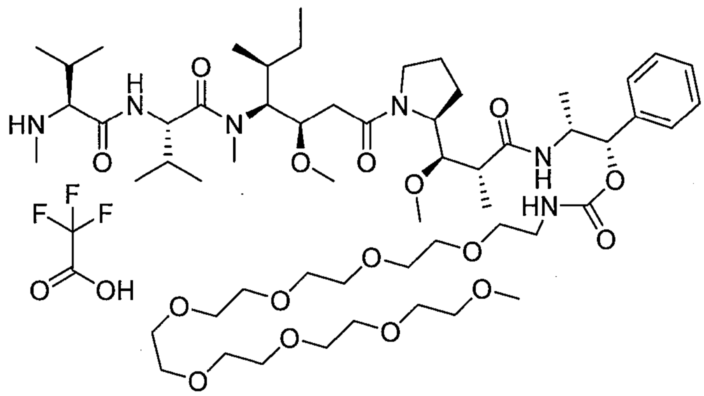

(U-011)

To a solution of U-011-3 (47 mg, 0.038 mmoL) in dichloromethane (2 mL) in a 30 mL pear-shaped flask was added trifluoroacetic acid (0.50 mL, 744.5 mg, 6.53 mmoL) at room temperature, and the resulting mixture was stirred at room temperature for 0.67 hour. Subsequently, the reaction solution was concentrated under reduced pressure. To the concentrated residues was added 0.1% formic acid solution in acetonitrile / water (= 1/9 (V/V)), and the resulting mixture was freeze-dried to give trifluoroacetate of U-011 (47.3 mg, yield: 99.51%) as white solids.
MS (ESI) m/z 1127 $(M+H)^+$

Production example 12. U-012

12-1. U-012-1

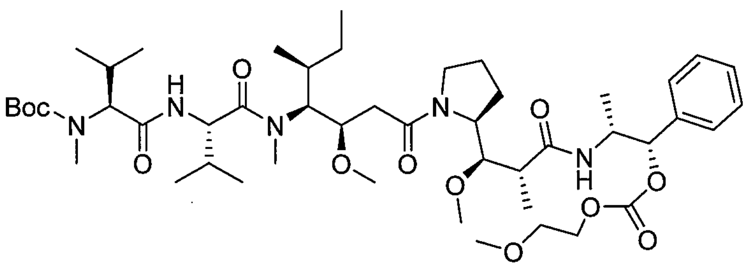

(U-012-1)

To a solution of U-011-1 (12.95 mg, 0.016 mmoL) and 4-dimethylaminopyridine (1.46 mg, 0.012 mmoL) in pyridine (0.3 mL) in a 0.2 mL - 0.5 mL microwave reaction container were added 2,4,6-trimethylpyridine (0.010 mL, 9.17 mg, 0.076 mmoL) and 2-methoxyethyl 2-chloroformate (0.050 mL, 59.6 mg, 0.43 mmoL), the container was nitrogen-sealed, the mixture was subjected to a microwave reactor (Biotage), and reacted at 100°C for 3 hours. The reaction solution was diluted with ethyl acetate, sequentially washed with water once, with a 5% aqueous solution of potassium hydrogen sulfate three times, with a saturated aqueous solution of sodium hydrogen carbonate once, and with saturated brine once, dried over anhydrous magnesium sulfate, filtered, and the resulting filtrate was concentrated under reduced pressure. The concentrated residues were dissolved in hexane / ethyl acetate (= 4/6 (V/V)), packaged in Fuji Silysia Chromatorex Q-Pack DIOL-60 Size 10, eluted with hexane / ethyl acetate (= 39/61 (V/V) → 18/82 (V/V)), and the fraction comprising the resulting target compound was concentrated under reduced pressure to give U-012-1 (9.4 mg, yield: 64.53%) as a colorless oil.
MS (ESI) m/z 920 $(M+H)^+$

12-2. U-012

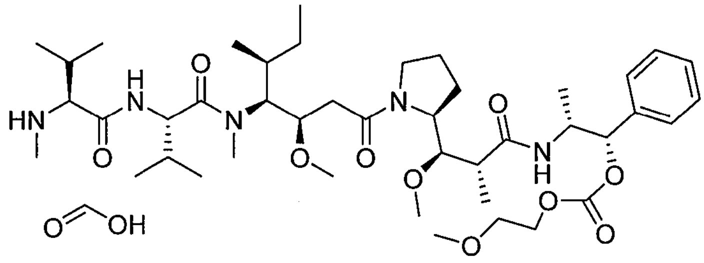

(U-012)

To a solution of U-012-1 (9.4 mg, 0.01022 mmoL) in dichloromethane (1 mL) in a 20 mL pear-shaped flask was added trifluoroacetic acid (0.050 mL, 74.45 mg, 0.653 mmoL) at room temperature, and the resulting mixture was stirred at room temperature for 1 hour. Subsequently, trifluoroacetic acid (0.450 mL, 670.05 mg, 5.88 mmoL) was additionally added thereto at room temperature, and the resulting mixture was stirred at room temperature for 0.5 hour. Subsequently, the reaction solution was concentrated under reduced pressure. The concentrated residues were fractionated by HPLC (Column: Waters SunFire Prep C18 5 μm ODB 19*150 mm, 0.1% formic acid solution in acetonitrile / water (V/V) = 30/70 → 95/5), and the fraction comprising the target compound was concentrated under reduced pressure. To the concentrated residues was added a 0.1% aqueous solution of formic acid, and the resulting mixture was freeze-dried to give formate of U-012 (3.8 mg, yield: 42.95%) as white solids.
MS (ESI) m/z 820 $(M+H)^+$

Production example 13. U-013

13-1. U-013-1

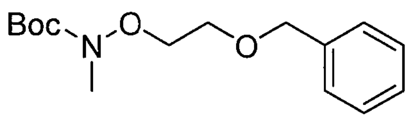

(U-013-1)

To a solution of tert-butyl hydroxy(methyl)carbamate (7.40 g, 50.3 mmoL) and potassium carbonate (13.84 g, 100 mmol) in acetonitrile (50 mL) in a 200 mL round-bottom flask was added ((2-bromoethoxy)methyl)benzene (11.36 g, 52.8 mmoL) under argon atmosphere with stirring at room temperature, and the resulting mixture was stirred at 80°C for 5 hours. After the reaction was completed, the reaction mixture was allowed to cool to room temperature, and concentrated under reduced pressure. To the resulting residues was added a saturated aqueous solution of sodium hydrogen carbonate, and the resulting mixture was subjected to extraction with diisopropyl ether twice. The resulting organic layers were combined, dried over anhydrous magnesium sulfate, filtered, and concentrated under reduced pressure. The resulting residues were subjected to YAMAZEN medium pressure preparative (Silica, L (40 g), hexane / ethyl acetate = 95/5 (V/V) --+ 80/20 (V/V)), and the fraction comprising the target compound was concentrated under reduced pressure to give U-013-1 (15.11 g, yield: quantitative) as a colorless oil.

13-2. U-013-2

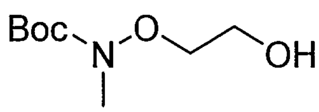

(U-013-2)

A solution of U-013-1 (2.8 g, 9.95 mmoL) and 10% palladium carbon (1.4 g, 0.658 mmoL) in ethanol (50 mL) in a

200 mL round-bottom flask was stirred under hydrogen atmosphere at room temperature for 4 hours. After the reaction was completed, the catalyst was separated by filtration, and the reaction solution was concentrated under reduced pressure to give U-013-2 (1.90 g, yield: 99.84%) as a pale yellow oil.

13-3. U-013-3

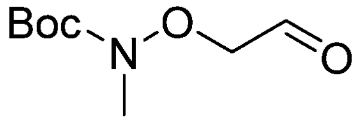

(U-013-3)

To a solution of U-013-2 (0.19 g, 0.994 mmoL) in dichloromethane (5 mL) in a 30 mL cylindrical flask was added Dess-Martin periodinane (0.51 g, 1.202 mmoL) with stirring at room temperature, and the resulting mixture was stirred at room temperature for 1 hour. After the reaction was completed, to the reaction solution was added a saturated aqueous solution of sodium hydrogen carbonate, further added an aqueous solution of sodium thiosulfate, the resulting mixture was stirred at room temperature for 20 minutes, and then the resulting mixed solution was subjected to extraction with dichloromethane. The resulting organic layer was dried over anhydrous magnesium sulfate, filtered, and concentrated under reduced pressure to give U-013-3 (162.7 mg, yield: 86.54%) as a slightly yellow oil.

13-4. U-013-4

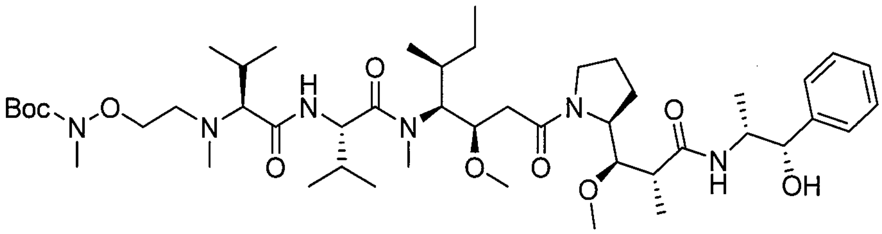

(U-013-4)

To a solution of MMAE (0.30 g, 0.418 mmoL) and U-013-3 (0.16 g, 0.846 mmoL) in dichloromethane (5 mL) in a 30 mL cylindrical flask was added sodium triacetoxyborohydride (0.18 g, 0.849 mmoL) under argon atmosphere with stirring at room temperature, and the resulting mixture was stirred at room temperature for 2 hours. After the reaction was completed, to the reaction solution was added a saturated aqueous solution of sodium hydrogen carbonate, and the resulting mixed solution was subjected to extraction with dichloromethane. The resulting organic layer was dried over anhydrous magnesium sulfate, filtered, and concentrated under reduced pressure to give crude products of U-013-4 as yellow solids. The resulting crude products were subjected to YAMAZEN medium pressure preparative (Silica, M (16 g), ethyl acetate / methanol = 100/0 (V/V) → 95/5 (V/V)), and the fraction comprising the target compound was concentrated under reduced pressure to give U-013-4 (206.5 mg, yield: 55.46%) as colorless foam.
MS (DUIS) m/z 893 $(M+H)^+$

13-5. U-013

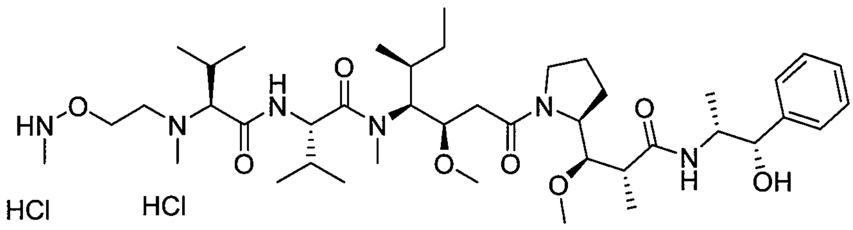

(hydrochloride of U-013)

To a solution of U-013-4 (45.3 mg, 0.051 mmoL) in dichloromethane (0.2 mL) in a 30 mL cylindrical flask was added a 4 M solution of hydrogen chloride in 1,4-dioxane (1 mL, 4 mmoL) under argon atmosphere with stirring at room temperature, and the resulting mixture was stirred at room temperature for 2 hours. After the reaction was completed, the reaction solution was concentrated under reduced pressure, then dissolved in a mixed solvent of water and acetonitrile, and freeze-dried to give hydrochloride of U-013 (44.3 mg, yield: quantitative) as slightly yellow foam. MS (DUIS) m/z 791 $(M+H)^+$

Production example 14. U-014

14-1. U-014-1

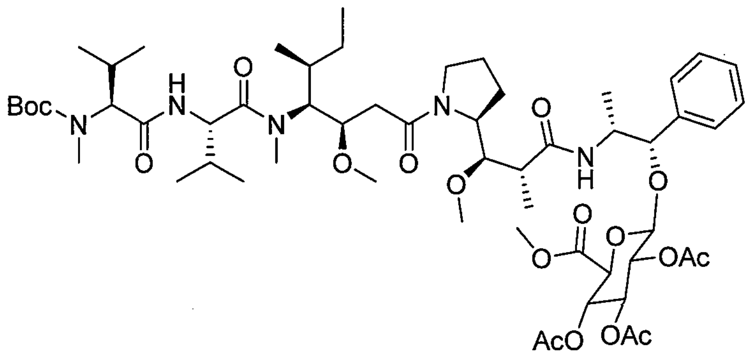

(U-014-1)

To a solution of U-011-1 (102.1 mg, 0.125 mmol) and (2R,3R,4S,5R,6S)-2-bromo-6-(methoxycarbonyl)tetrahydro-2H-pyran-3,4,5-triyl triacetate (463.2 mg, 1.17 mmoL) in dichloromethane (1.8 mL) in a 10 mL cylindrical flask was added 2,4,6-trimethylpyridine (0.255 mL, 233.8 mg, 1.93 mmoL) at room temperature. Under argon atmosphere, silver trifluoromethanesulfonate (387.2 mg, 1.93 mmoL) was added thereto, and then the resulting mixture was stirred at room temperature for 1 hour. After the reaction was completed, the reaction solution was diluted with ethyl acetate, filtered through Celite 545, and washed with ethyl acetate. The resulting filtrate and wash liquid were sequentially washed with a 5% aqueous solution of potassium hydrogen sulfate, a saturated aqueous solution of sodium hydrogen carbonate, and saturated brine, dried over anhydrous magnesium sulfate, filtered, and the resulting filtrate was concentrated under reduced pressure to give concentrated residues. The concentrated residues were purified under the following conditions to give U-014-1 (128 mg, yield: 90.4%) as white foam.
MS (DUIS) m/z 1134 $(M+H)^+$

Recycle preparative device (YMC Co., Ltd.): LC Forte/R
Column (YMC Co., Ltd.): triconnection of T-30000 (21.2 mmφ × 600 mm, 50 nm), T-4000 (21.2 mmφ × 600 mm, 10 nm), and T-2000 (21.2 mmφ × 600 mm, 5 nm).
Eluent: ethyl acetate
Temperature: room temperature

14-2. U-014

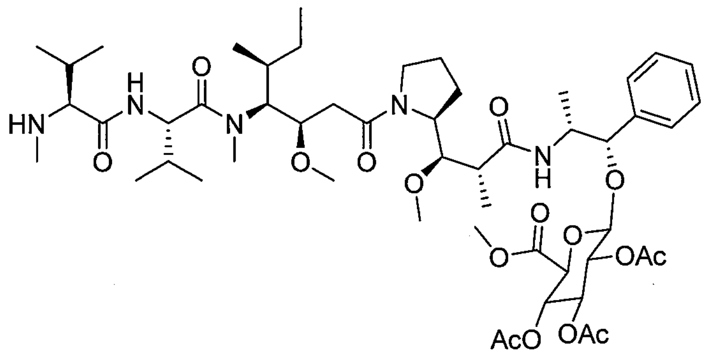

(U-014)

To a solution of U-014-1 (166 mg, 0.146 mmoL) and 2,6-dimethylpyridine (0.090 mL, 83.27 mg, 0.777 mmoL) in dichloromethane (5 mL) in a 100 mL round-bottom flask was added trimethylsilyl trifluoromethanesulfonate (0.135 mL, 166.05 mg, 0.747 mmoL) under argon atmosphere with stirring under ice-cooling, and the resulting mixture was stirred under ice-cooling for 1 hour. After the reaction was completed, to the reaction solution was added a saturated aqueous solution of sodium hydrogen carbonate under ice-cooling, and the resulting solution was separated. The resulting aqueous layer was subjected to extraction with dichloromethane, the resulting organic layers were combined, and dried over anhydrous sodium sulfate to give concentrated residues. The concentrated residues were purified under the following conditions, and the fraction comprising the target compound was concentrated under reduced pressure to give a colorless oil. Acetonitrile (2 mL) and distilled water (5 mL) were added thereto, and then the resulting mixture was freeze-dried to give U-014 (43.3 mg, yield: 28.61%) as a white powder.
MS (DUIS) m/z 1034 $(M+H)^+$

Recycle preparative device (YMC Co., Ltd.): LC Forte/R
Column (YMC Co., Ltd.): triconnection of T-30000 (21.2 mmφ × 600 mm, 50 nm), T-4000 (21.2 mmφ × 600 mm, 10 nm), and T-2000 (21.2 mmφ × 600 mm, 5 nm).
Eluent: ethyl acetate
Temperature: room temperature

Production example 15. U-015

15-1. U-015-1

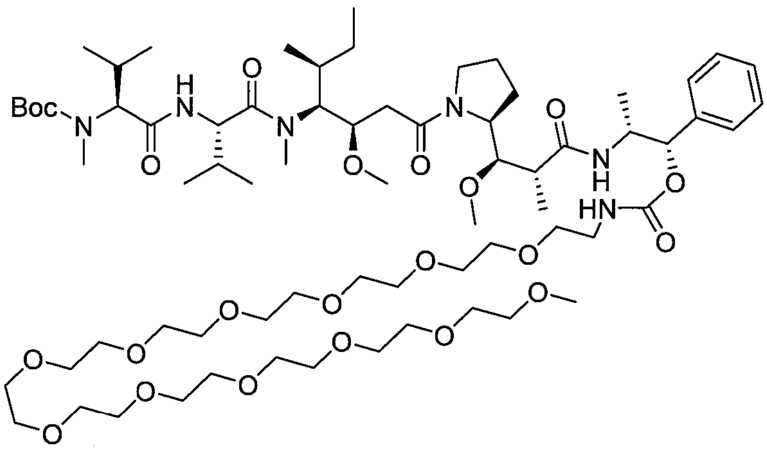

(U-015-1)

To a solution of U-011-2 (101.3 mg, 0.103 mmoL) and 2,5,8,11,14,17,20,23,26,29,32,35-dodecaoxaheptatriacontane-37-amine (98.1 mg, 0.175 mmoL) in dichloromethane (3 mL) in a 30 mL cylindrical flask was added N,N-diisopropylethylamine (0.060 mL, 44.52 mg, 0.344 mmoL), and the resulting mixture was stirred under argon atmosphere at room temperature for 7 hours. After the reaction was completed, the reaction mixture was concentrated under reduced pressure. The resulting residues were separated and purified under the following conditions, the resulting ethyl acetate solution (150 mL) was sequentially washed with water (50 mL) twice and with saturated brine (50 mL) once, dried over anhydrous magnesium sulfate, filtered, and the resulting filtrate was concentrated under reduced pressure to give U-015-1 (91.8 mg, yield: 63.47%) as a colorless oil.
MS (DUIS) m/z 702 $(M+2H)^{2+}$

Recycle preparative device (YMC Co., Ltd.): LC Forte/R
Column (YMC Co., Ltd.): triconnection of T-30000 (21.2 mmφ × 600 mm, 50 nm), T-4000 (21.2 mmφ × 600 mm, 10 nm), and T-2000 (21.2 mmφ × 600 mm, 5 nm) .
Eluent: ethyl acetate
Temperature: room temperature

15-2. U-015

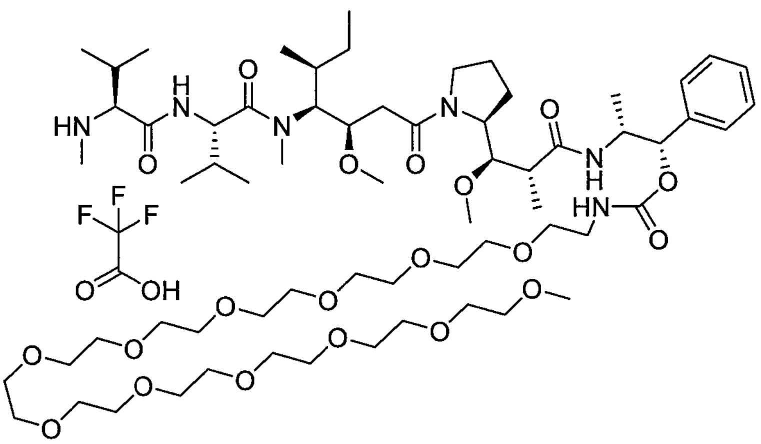

(U-015)

To a solution of U-015-1 (88.2 mg, 0.063 mmoL) in dichloromethane (5 mL) in a 50 mL round-bottom flask was added trifluoroacetic acid (0.48 mL, 714.72 mg, 6.27 mmoL) under ice-cooling, and the resulting mixture was stirred under ice-cooling for 1.25 hours and then stirred at room temperature for 1.5 hours. After the reaction was completed, the reaction

solution was concentrated under reduced pressure. To the concentrated residues were added a 0.1% solution of formic acid in acetonitrile / water (= 1/9 (V/V) (5 mL)) solution and a 0.1% solution of formic acid in acetonitrile / water (= 5/5 (V/V) (5 mL)) solution, and the resulting mixture was freeze-dried to give trifluoroacetate of U-015 (96.1 mg, yield: quantitative) as a colorless oil.
MS (ESI) m/z 1304 $(M+H)^+$

Production example 16. U-016

16-1. U-016-1

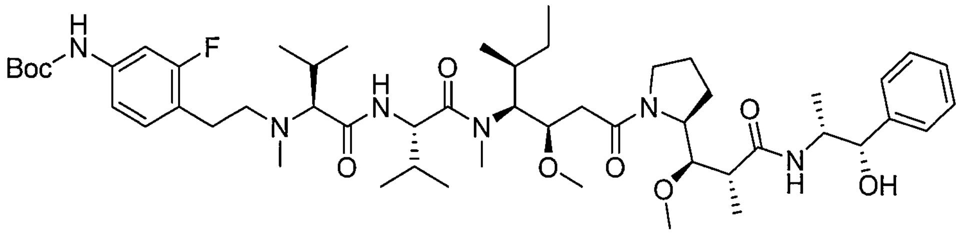

(U-016-1)

To a solution of tert-butyl (3-fluoro-4-(2-oxoethyl)phenyl)carbamate (57.0 mg, 0.225 mmoL) and MMAE (106.3 mg, 0.148 mmoL) in dichloromethane (2.5 mL) in a 50 mL round-bottom flask was added acetic acid (0.015 mL, 15.83 mg, 0.264 mmoL), then sodium triacetoxyborohydride (65.0 mg, 0.307 mmoL) was added thereto under ice-cooling under argon atmosphere with stirring, and the resulting mixture was stirred at room temperature for 1.5 hours. After the reaction was completed, the reaction solution was diluted with ethyl acetate (5 mL), a saturated aqueous solution of sodium hydrogen carbonate (3 mL) was added thereto, the resulting mixture was stirred at room temperature for a while, and then the resulting solution was separated. The resulting organic layer was washed with saturated brine, dried over anhydrous magnesium sulfate, filtered, and the resulting filtrate was concentrated under reduced pressure. The concentrated residues were dissolved in hexane / ethyl acetate (= 50/50 (V/V)), subjected to Fuji Silysia Chromatorex Q-Pack DIOL-60 Size 10, eluted with hexane / ethyl acetate (= 50/50 (V/V) → 19/81 (V/V)), and the fraction comprising the target compound was concentrated under reduced pressure to give U-016-1 (140.2 mg, yield: 99.13%) as white foam.
MS (ESI) m/z 955 $(M+H)^+$

16-2. U-016

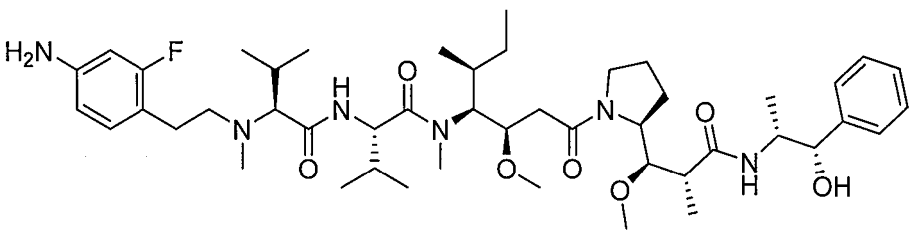

(U-016)

To a solution of U-016-1 (136 mg, 0.142 mmoL) in dichloromethane (3.6 mL) in a 50 mL round-bottom flask was added trifluoroacetic acid (0.545 mL, 811.51 mg, 7.12 mmoL) under argon atmosphere with stirring under ice-cooling, and the resulting mixture was stirred under ice-cooling for 0.5 hour and then stirred at room temperature for 1 hour. After the reaction was completed, the reaction mixture was concentrated under reduced pressure, dichloromethane (5 mL) and a saturated aqueous solution of sodium hydrogen carbonate (5 mL) were added thereto, and the resulting mixture was separated. The resulting organic layer was dried over anhydrous sodium sulfate, filtered, and the resulting filtrate was concentrated under reduced pressure to give concentrated residues. The concentrated residues were separated and

purified under the following conditions, and the resulting fraction was concentrated under reduced pressure to give U-016 (93.9 mg, yield: 77.13%) as white foam.
MS (ESI) m/z 855 $(M+H)^+$

Recycle preparative device (YMC Co., Ltd.): LC Forte/R
Column (YMC Co., Ltd.): triconnection of T-30000 (21.2 mmφ × 600 mm, 50 nm), T-4000 (21.2 mmφ × 600 mm, 10 nm), and T-2000 (21.2 mmφ × 600 mm, 5 nm) .
Eluent: acetonitrile
Temperature: room temperature

Production example 17. U-017

17-1. U-017-1

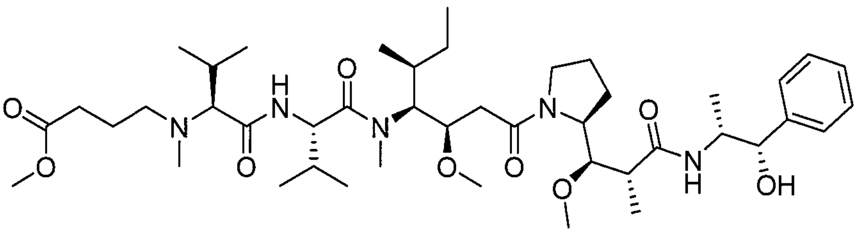

(U-017-1)

To a solution of MMAE (71.8 mg, 0.100 mmoL) and methyl 4-oxobutanoate (34.8 mg, 0.300 mmoL) in dichloromethane (1 mL) in a 30 mL cylindrical flask was added sodium triacetoxyborohydride (63.6 mg, 0.300 mmoL) under argon atmosphere with stirring at room temperature, and the resulting mixture was stirred at room temperature for 1 hour. After the reaction was completed, to the reaction solution was added a saturated aqueous solution of sodium hydrogen carbonate, and the resulting mixed solution was subjected to extraction with ethyl acetate. The resulting organic layer was dried over anhydrous magnesium sulfate, filtered, and concentrated under reduced pressure to give crude products of U-017-1 as yellow solids. The resulting solids were subjected to YAMAZEN medium pressure preparative (Silica, M (16 g), dichloroethane/methanol = 100/0 (V/V) -> 90/10 (V/V)), and the fraction comprising the target compound was concentrated under reduced pressure to give U-017-1 (20.2 mg, yield: 24.69%) as colorless foam.

17-2. U-017

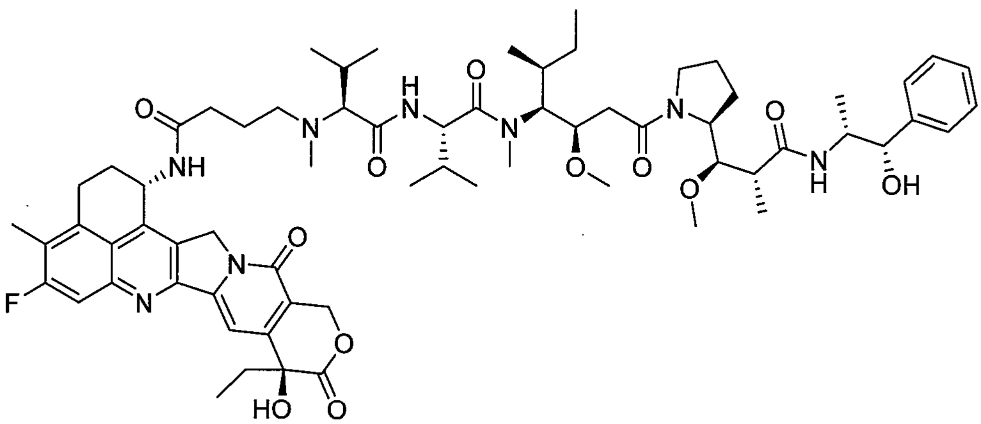

(U-017)

To a solution of U-017-1 (20.2 mg, 0.025 mmoL) in ethanol (0.4 mL) in a 10 mL cylindrical flask was added a 2N aqueous solution of sodium hydroxide (0.050 mL) under argon atmosphere with stirring at room temperature, and the resulting mixture was stirred at room temperature for 2 hours. 2N hydrochloric acid (0.2 mL) was added thereto to neutralize the mixture, and then the solvent was distilled away under reduced pressure. To the resulting residues was added N,N-dimethylformamide (0.4 mL), further added 1-ethyl-3-(3-dimethylaminopropyl)carbodiimide hydrochloride (7.5 mg, 0.039 mmoL), 1-hydroxybenzotriazole (6.5 mg, 0.042 mmoL), exatecan mesylate (13.1 mg, 0.025 mmoL), and triethylamine (0.01 mL, 7.26 mg, 0.072 mmoL), and the resulting mixture was stirred at room temperature for 14 hours. After the reaction was completed, to the reaction solution was added water, ethyl acetate was added thereto to separate the rection solution, and then the resulting insoluble matters were separated by filtration. The resulting organic layer was subjected to YAMAZEN medium pressure preparative (Silica, S (7 g), dichloroethane/methanol = 90/10 (V/V) → 80/20 (V/V)), and the fraction comprising the target compound (Rf value = 0.4 (dichloroethane/methanol = 90/10 (V/V))) was concentrated under reduced pressure. The resulting residues were subjected to preparative HPLC chromatography under the following conditions, the fraction comprising the target compound was neutralized by a saturated aqueous solution of sodium hydrogen carbonate, and then concentrated under reduced pressure. To the resulting residues was added water, and then the resulting mixture was subjected to extraction with ethyl acetate twice. The resulting organic layer was dried over anhydrous magnesium sulfate, the solvent was distilled away under reduced pressure, then the resulting residues were dissolved in water/acetonitrile, and freeze-dried to give U-017 (3.8 mg, yield: 12.6%) as colorless solids.
MS (DUIS) m/z 1222 $(M+H)^+$

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) → 50% (10.00 min.)

Production example 18. U-018

18-1. U-018-1

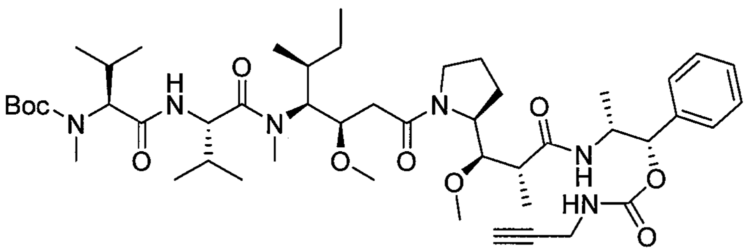

(U-018-1)

To a solution of U-011-2 (61.5 mg, 0.063 mmoL) in dichloromethane (0.4 mL) in a 20 mL cylindrical flask was added prop-2-yne-1-amine (0.008 mL, 6.88 mg, 0.125 mmoL) under argon gas flow with stirring at room temperature, and the resulting mixture was stirred at room temperature for 3 hours. The resulting residues were subjected to YAMAZEN medium pressure preparative (Rf value = 0.25 (hexane / ethyl acetate = 20/80 (V/V)) (Silica, M (16 g))), and the fraction comprising the target compound was concentrated under reduced pressure to give U-018-1 (57.6 mg, yield: quantitative) as white solids.
MS (ESI) m/z 900 $(M+H)^+$

18-2. U-018-2

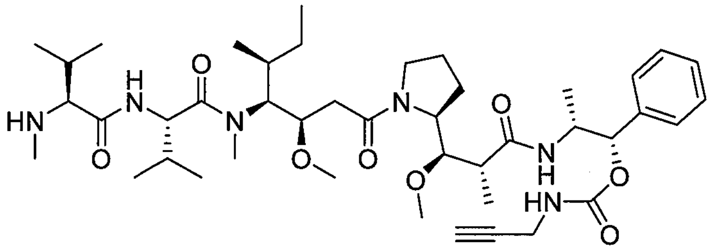

(U-018-2)

To a solution of U-018-1 (67.6 mg, 0.075 mmoL) in dichloromethane (0.2 mL) in a 20 mL cylindrical flask was added trifluoroacetic acid (0.064 mL, 95.36 mg, 0.836 mmoL) under argon gas flow with stirring at room temperature, and the resulting mixture was stirred at room temperature for 3 hours. After the reaction was completed, the reaction mixture was concentrated under reduced pressure, the resulting residues were subjected to preparative HPLC chromatography under the following conditions, and the fraction comprising the target compound was concentrated under reduced pressure to give U-018-2 (17.2 mg, yield: 28.63%) as white solids.
MS (ESI) m/z 799 $(M+H)^+$

Column: Waters SunFire Prep C18 5 μm ODB 19*150 mm
Flow rate: 17 mL/min.
Elution solvent: 0.1% aqueous solution of formic acid (Solution A) -> 0.1% solution of formic acid in acetonitrile (Solution B)
Gradient (Solution B): 20% (0.00 min.) -> 95% (10.00 min.)

18-3. U-018

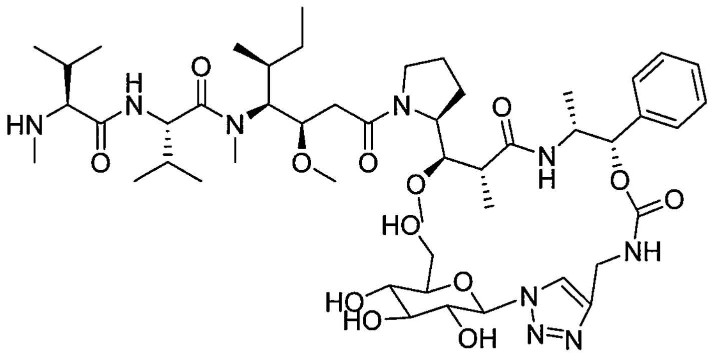

(U-018)

To a solution of U-018-2 (8.8 mg, 0.011 mmol) in water (0.1 mL) in a 20 mL cylindrical flask were added (2R,3R,4S,5S,6R)-2-azide-6-(hydroxymethyl)tetrahydro-2H-pyran-3,4,5-triol (3.8 mg, 3.09 pmol), copper(II) sulfate pentahydrate (3.5 mg, 0.022 mmoL), and sodium ascorbate (7.1 mg, 0.033 mmoL) under argon gas flow with stirring at room temperature, and the resulting mixture was stirred at room temperature for 5 minutes. The resulting residues were subjected to preparative HPLC chromatography under the following conditions, and the fraction comprising the target compound was freeze-dried to give U-018 (3.1 mg, yield: 28.03%) as white solids.
MS (DUIS) m/z 1046 $(M+HCOO)^-$

Column: Waters SunFire Prep C18 5 μm ODB 19*150 mm
Flow rate: 17 mL/min.
Elution solvent: 0.1% aqueous solution of formic acid (Solution A) - 0.1% solution of formic acid in acetonitrile (Solution B)
Gradient (Solution B): 20% (0.00 min.) -> 61% (6.00 min.)

Production example 19. U-019

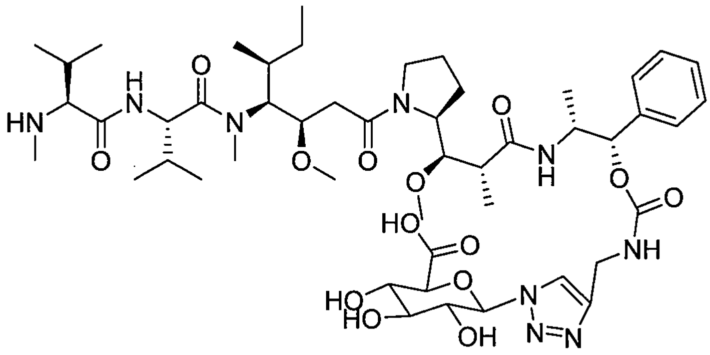

(U-019)

To a solution of the Production example 18-1 (U-018-1) (8.2 mg, 10.26 pmoL) in water (0.2 mL) in a 10 mL cylindrical flask were added (2R,3R,4S,5S,6R)-6-azide-3,4,5-trihydroxytetrahydro-2H-pyran-2-carboxylic acid (2.8 mg, 0.013 mmoL), copper(II) sulfate pentahydrate (2.0 mg, 0.013 mmoL), and sodium ascorbate (3.5 mg, 0.016 mmoL) under argon gas flow with stirring at room temperature, and the resulting mixture was stirred at room temperature for 5 minutes. The resulting residues were subjected to preparative HPLC chromatography under the following conditions, and the fraction comprising the target compound was freeze-dried to give U-019 (5.4 mg, yield: 51.68%) as white solids.
MS (ESI) m/z 1016 $(M-H)^-$

Column: Waters SunFire Prep C18 5 μm ODB 19*150 mm
Flow rate: 17 mL/min.
Elution solvent: 0.1% aqueous solution of formic acid (Solution A) - 0.1% solution of formic acid in acetonitrile (Solution B)
Gradient (Solution B): 20% (0.00 min.) --+ 61% (7.00 min.)

Production example 20. U-020

20-1. U-020-1

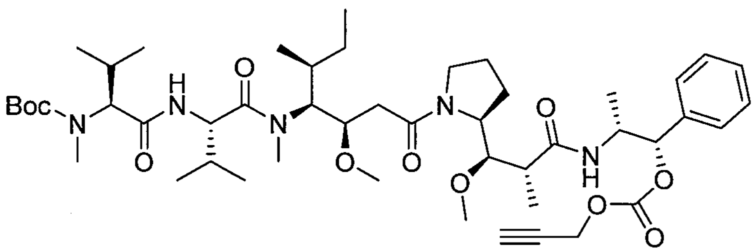

(U-020-1)

To a solution of U-011-2 (50.0 mg, 0.051 mmoL) in dichloromethane (0.2 mL) in a 20 mL cylindrical flask was added prop-2-yn-1-ol (0.00587 mL, 5.71 mg, 0.102 mmoL) under argon gas flow with stirring at room temperature, and the resulting mixture was stirred at room temperature for 45 minutes. Subsequently, 4-dimethylaminopyridine (0.8 mg, 6.55 μmoL) was added thereto under stirring at room temperature, and the resulting mixture was stirred at room temperature for 2.5 hours. Subsequently, prop-2-yn-1-ol (0.00587 mL, 5.71 mg, 0.102 mmoL) was additionally added thereto, and the resulting mixture was stirred for 14 hours. The resulting residues were subjected to YAMAZEN medium pressure preparative (Rf value = 0.3 (hexane / ethyl acetate = 20/80 (V/V) (Silica, M (16 g)))), and the fraction comprising the target compound was concentrated under reduced pressure to give U-020-1 (37.8 mg, yield: 82.57%) as white solids.

MS (ESI) m/z 900 (M+H)+

20-2. U-020-2

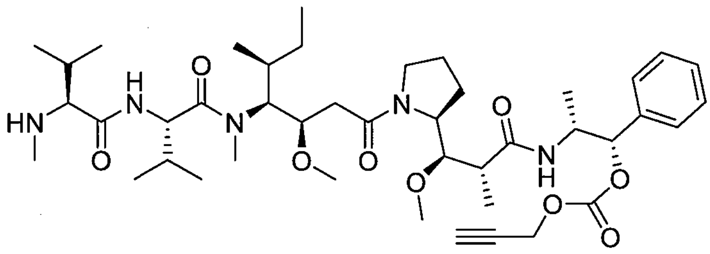

(U-020-2)

To a solution of U-020-1 (36.8 mg, 0.041 mmoL) in dichloromethane (0.2 mL) in a 20 mL cylindrical flask was added trifluoroacetic acid (0.069 mL, 102.81 mg, 0.902 mmoL) under argon gas flow with stirring at room temperature, and the resulting mixture was stirred at room temperature for 2.5 hours. After the reaction was completed, the reaction mixture was concentrated under reduced pressure, the resulting residues were subjected to preparative HPLC chromatography under the following conditions, the fraction comprising the target compound was concentrated under reduced pressure to distill away acetonitrile, and then the resulting residues were freeze-dried to give U-020-2 (18.2 mg, yield: 55.65%) as white solids.
MS (ESI) m/z 800 (M+H)+

Column: Waters SunFire Prep C18 5 μm ODB 19*150 mm
Flow rate: 17 mL/min.
Elution solvent: 0.1% aqueous solution of formic acid (Solution A) - 0.1% solution of formic acid in acetonitrile (Solution B)
Gradient (Solution B): 20% (0.00 min.) → 75% (6.50 min.)

20-3. U-020

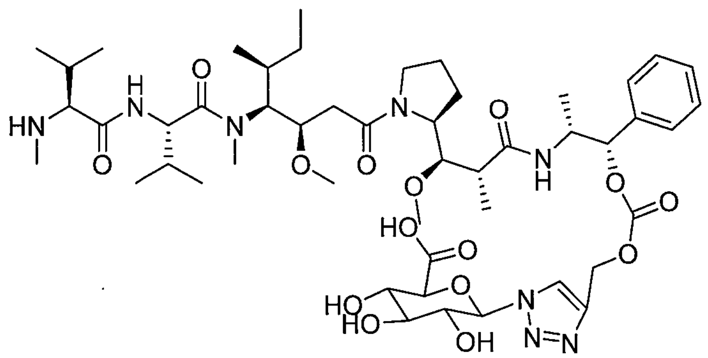

(U-020)

To a solution of U-020-2 (5.8 mg, 7.25 μmol) in water (0.3 mL) in a 20 mL cylindrical flask were added (2R,3R,4S,5S,6R)-6-azide-3,4,5-trihydroxytetrahydro-2H-pyran-2-carboxylic acid (2.3 mg, 10.49 μmol), copper(II) sulfate pentahydrate (0.6 mg, 2.403 pmoL), and sodium ascorbate (0.8 mg, 3.77 μmoL) under argon gas flow with stirring at room temperature, and the resulting mixture was stirred at room temperature overnight. The resulting residues were subjected to preparative HPLC chromatography under the following conditions, and the fraction comprising the target compound was freeze-dried to give U-020 (6.3 mg, yield: 85.26%) as white solids.

MS (ESI) m/z 1020 $(M+H)^+$
Column: Waters SunFire Prep C18 5 μm ODB 19*150 mm
Flow rate: 17 mL/min.
Elution solvent: 0.1% aqueous solution of formic acid (Solution A) - 0.1% solution of formic acid in acetonitrile (Solution B)
Gradient (Solution B): 20% (0.00 min.) → 61% (6.00 min.)

Production example 21. U-021

21-1. U-021-1

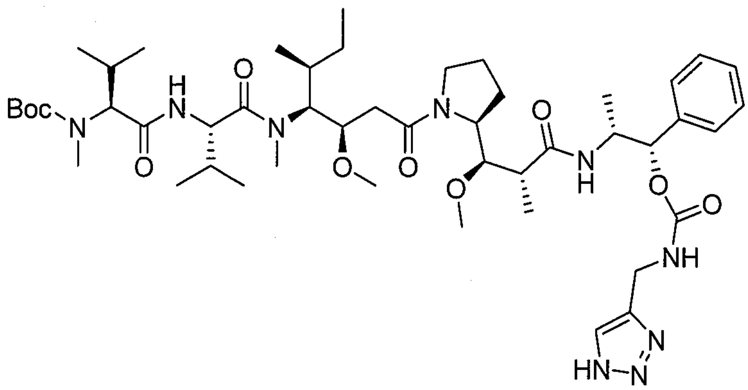

(U-021-1)

To a solution of U-011-2 (25 mg, 0.025 mmol) in dichloromethane (2 mL) in a 20 mL cylindrical flask were added (1H-1,2,3-triazol-4-yl)methaneamine hydrochloride (7.0 mg, 0.052 mmoL) and triethylamine (0.00354 mL, 2.57 mg, 0.025 mmoL) under argon gas flow with stirring at room temperature, and the resulting mixture was stirred at room temperature for 1 hour. Subsequently, N,N-dimethylformamide (1 mL) was added thereto, and the resulting mixture was stirred for 1 hour. After the reaction was completed, to the reaction solution was added water, and the resulting mixed solution was subjected to extraction with ethyl acetate. The resulting organic layer was washed with saturated brine, dried over anhydrous sodium sulfate, filtered, and concentrated under reduced pressure to give crude products of U-021-1. The crude products were used in the next step without further purification.
MS (ESI) m/z 942 $(M+H)^+$

21-2. U-021

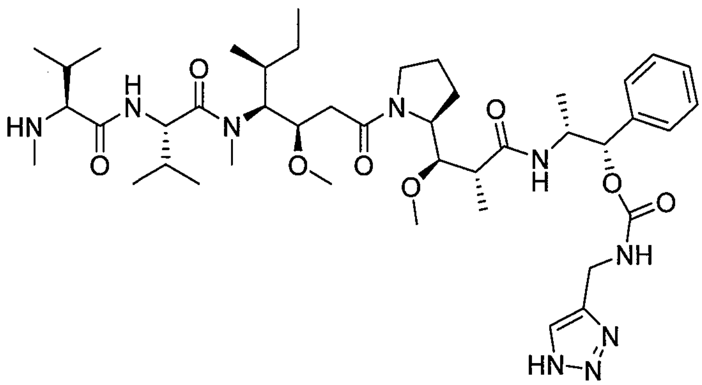

(U-021)

To a solution of the crude products of U-021-1 (284 mg (content: 8.5%)) in dichloromethane (0.5 mL) in a 20 mL cylindrical flask was added trifluoroacetic acid (0.10 mL, 149 mg, 1.307 mmoL) under argon gas flow with stirring at room temperature, and the resulting mixture was stirred at room temperature for 1.5 hours. After the reaction was completed, the reaction mixture was concentrated under reduced pressure. The resulting residues were subjected to preparative HPLC chromatography under the following conditions, the fraction comprising the target compound was concentrated under reduced pressure to distill away acetonitrile, and then the resulting residues were freeze-dried to give trifluoroacetate of U-021 (8.8 mg) as white solids.

Column: Waters SunFire Prep C18 5 μm ODB 19*150 mm
Flow rate: 17 mL/min.
Elution solvent: 0.1% aqueous solution of formic acid (Solution A) - 0.1% solution of formic acid in acetonitrile (Solution B)
Gradient (Solution B): 5% (0.00 min.) -> 95% (10.50 min.)

The resulting solids were dissolved in dichloromethane, washed with an aqueous solution of sodium hydrogen carbonate and saturated brine, and dried over anhydrous sodium sulfate. The resulting residues were concentrated under reduced pressure to give U-021 (7.0 mg, total yield of 21-1 and 21-2: 32.63%) as white solids.
MS (ESI) m/z 840 $(M-H)^-$

Production example 22. U-022

22-1. U-022-1

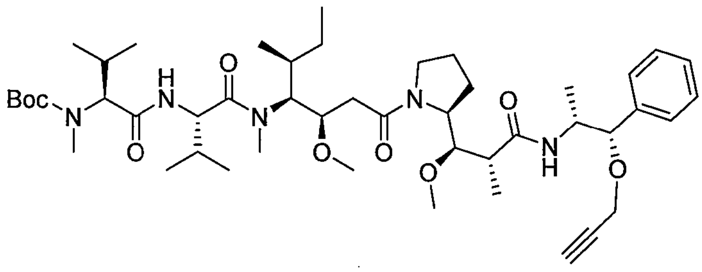

(U-022-1)

To a solution of U-011-1 (400 mg, 0.489 mmoL) in toluene (6 mL) in a 50 mL round-bottom flask were added 3-bromoprop-1-yne (0.11 mL, 173.8 mg, 1.461 mmoL), tetrabutylammonium bromide (33.2 mg, 0.103 mmoL), and 8N sodium hydroxide (0.061 mL) under argon gas flow with stirring at room temperature, and the resulting mixture was stirred at room temperature for 6 hours. After the reaction was completed, to the reaction solution was added a saturated aqueous solution of ammonium chloride, and the resulting mixed solution was subjected to extraction with dichloromethane. The resulting residues were subjected to preparative HPLC chromatography under the following conditions, and the fraction comprising the target compound was concentrated under reduced pressure to give U-022-1 (142.1 mg, yield: 33.95%) as white solids.
MS (ESI) m/z 857 $(M+H)^+$

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 50% (0.00 min.) → 100% (10.00 min.)

22-2. U-022-2

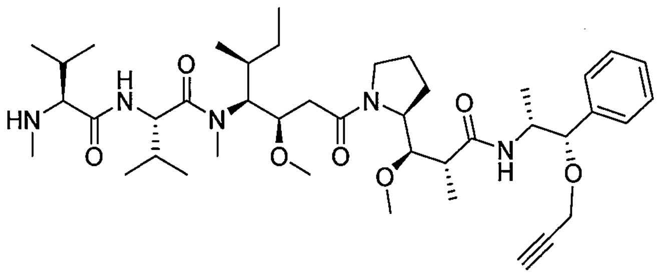

(U-022-2)

To a solution of U-022-1 (142.1 mg, 0.166 mmoL) in dichloromethane (0.4 mL) in a 20 mL cylindrical flask was added trifluoroacetic acid (0.254 mL, 378 mg, 3.32 mmoL) under argon gas flow with stirring at room temperature, and the resulting mixture was stirred at room temperature for 20 hours. After the reaction was completed, the reaction mixture was neutralized by triethylamine, and concentrated under reduced pressure. The resulting residues were subjected to preparative HPLC chromatography under the following conditions, the fraction comprising the target compound was concentrated under reduced pressure to distill away acetonitrile, and then the resulting residues were freeze-dried to give U-022-2 (69.6 mg, yield: 55.47%) as white solids.
MS (DUIS) m/z 757 $(M+H)^+$

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 0% (0.00 min.) → 50% (10.00 min.)

22-3. U-022

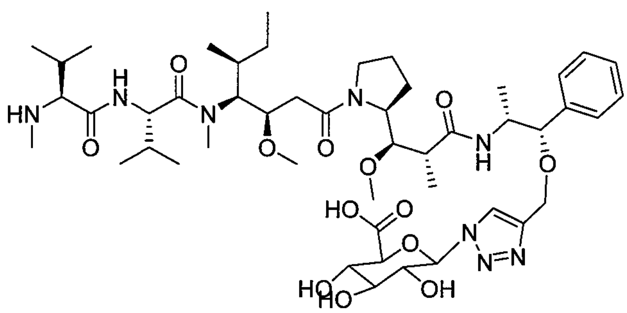

(U-022)

To a solution of U-022-2 (30 mg, 0.040 mmoL) in water (1 mL) in a 30 mL cylindrical flask were added (2S,3S,4S,5R,6R)-6-azide-3,4,5-trihydroxytetrahydro-2H-pyran-2-carboxylic acid (11.2 mg, 0.051 mmol), copper sulfate pentahydrate (3.1 mg, 0.012 mmol), and sodium ascorbate (12.7 mg, 0.060 mmoL) under argon gas flow with stirring at room temperature, and the resulting mixture was stirred at room temperature for 15 hours. The resulting residues were subjected to the following conditions, and the fraction comprising the target compound was concentrated under reduced pressure to give crude products of U-022 as yellow solids.

Column: Waters XSelect HSS C18 SB OBD 5 μm ODB 19*150 mm
Flow rate: 17 mL/min.
Elution solvent:
0.1% aqueous solution of formic acid / 0.1% solution of formic acid in acetonitrile = 90/10 (Solution A) - 0.1% aqueous solution of formic acid / 0.1% solution of formic acid in acetonitrile = 10/90 (Solution B)
Gradient (Solution B): 0% (0.00 min.) -> 0% (5.00 min.) -> 50% (5.10 min.) → 50% (20.00 min.)

The resulting crude products of U-022 were purified under the following conditions, and the resulting residues

were freeze-dried to give U-022 (18.8 mg, yield: 37.72%) as white solids.
MS (ESI) m/z 976 $(M+H)^+$

Column: Asahipak GS-510-20G, GS-310-20G, and GS-310-20G (triconnection) (shodex) 20*500 mm, 13 μm
Flow rate: 7.5 mL/min.
Elution solvent: 0.1% formic acid / 0.1% formic acid in acetonitrile = 90/10
Detection wavelength: 254 nm

Production example 23. U-023

23-1. U-023-1

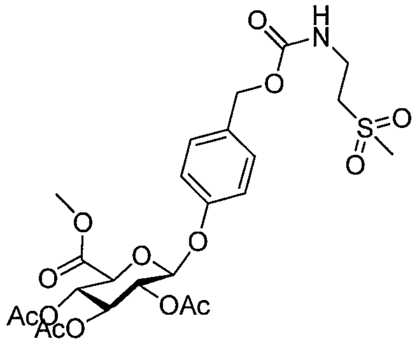

(U-023-1)

To a solution of (2S,3S,4S,5R,6S)-2-(methoxycarbonyl)-6-(4-((((4-nitrophenoxy)carbonyl)oxy)methyl)phenoxy) tetrahydro-2H-pyran-3,4,5-tolyl triacetate (0.7982 g, 1.318 mmoL) in dichloromethane (8 mL) in a 50 mL round-bottom flask were sequentially added N,N-diisopropylethylamine (1.20 mL, 0.86 g, 6.89 mmoL) and 2-(methylsulfonyl)ethan-1-amine hydrochloride (0.2105 g, 1.319 mmoL) under argon atmosphere with stirring at room temperature, and the resulting mixture was stirred at room temperature for 72 hours. After the reaction was completed, the reaction solution was concentrated under reduced pressure, the residues were diluted with ethyl acetate (30 mL), washed with a saturated aqueous solution of sodium hydrogen carbonate (10 mL) five times and then with saturated brine (20 mL), dried over anhydrous magnesium sulfate, filtered, and the resulting filtrate was concentrated under reduced pressure. The resulting residues were subjected to YAMAZEN medium pressure preparative (Silica, L (40 g), hexane / ethyl acetate = 10/90 (V/V), (Rf = 0.51 (hexane / ethyl acetate = 10/90 (V/V)))), and the fraction comprising the target compound was concentrated under reduced pressure to give U-023-1 (677.8 mg, yield: 87.21%) as white foam.
MS (ESI) m/z 635 $(M+HCOOH)^+$

23-2. U-023-2

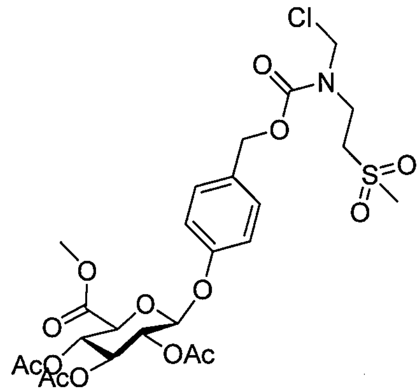

(U-023-2)

To a solution of U-023-1 (100.3 mg, 0.170 mmoL) in dichloromethane (1.5 mL) in a 20 mL cylindrical flask were sequentially added paraformaldehyde (9.30 mg, 0.310 mmoL) and trimethylsilyl chloride (33 μL, 28.25 mg, 0.260 mmoL) under argon atmosphere with stirring at room temperature, and the resulting mixture was stirred at room temperature for 5 hours. The reaction solution was filtered, washed with dichloromethane, and the resulting filtrate was concentrated under reduced pressure at room temperature to give U-023-2 (107.1 mg, yield: 98.67%) as white foam.
MS (ESI) m/z 651 $(M+H_2O)^+$

23-3. U-023-3

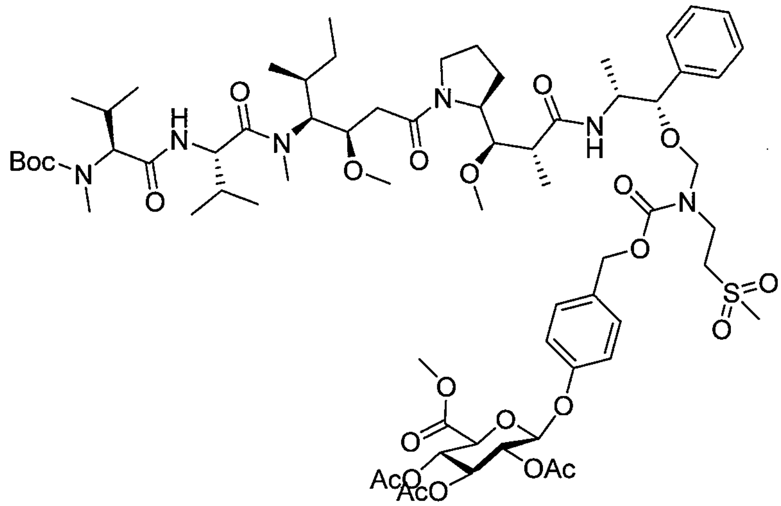

(U-023-3)

To a solution of U-011-1 (100.8 mg, 0.123 mmoL) in dichloromethane (0.5 mL) in a 20 mL cylindrical flask were sequentially added N,N-diisopropylethylamine (150 μL, 111.3 mg, 0.861 mmoL) and a solution of U-023-2 (108.55 mg, 0.170 mmoL) in dichloromethane (1 mL) under argon atmosphere with stirring at room temperature, and the resulting mixture was stirred at room temperature for 22.5 hours. Subsequently, 2,4,6-trimethylpyridine (110 μL, 101.2 mg, 0.835 mmoL) was added thereto at room temperature, then a solution of U-023-2 (171.3 mg, 0.268 mmoL) in dichloromethane (2 mL) was added thereto, the resulting mixture was stirred at room temperature for 21 hours, and then to the reaction solution was sprayed nitrogen to distill away the solvent. Dichloromethane (0.75 mL) was added thereto, then N,N-diisopropylethylamine (250 μL, 185.5 mg, 1.435 mmoL) was added thereto, and the resulting mixture was stirred at room temperature for 2 hours. Subsequently, the mixture was ice-cooled, and a solution of U-023-2 (446.7 mg, 0.700 mmoL) in dichloromethane (3 mL) was added dropwise thereto. After the addition was completed, the resulting mixture was warmed to room temperature, and stirred for 18 hours. The reaction solution was concentrated, diluted with ethyl acetate, sequentially washed with a 5% by weight of aqueous solution of potassium hydrogen sulfate, a saturated aqueous solution

of sodium hydrogen carbonate, and saturated brine, dried over anhydrous magnesium sulfate, filtered, and the resulting filtrate was concentrated under reduced pressure. The resulting residues were subjected to YAMAZEN medium pressure preparative (Fuji Silysia Chromatorex Q-Pack DIOL-60 Size 60, hexane / ethyl acetate = 30/70 (V/V) (Rf = 0.30 (hexane / ethyl acetate = 30/70 (V/V)))), and the fraction comprising the target compound was concentrated under reduced pressure to give crude products of U-023-3 as white foam.

The resulting white foam was purified under the following conditions to give U-023-3 (46.0 mg, yield: 26.3%) as white solids.
MS (ESI) m/z 1420 $(M+H)^+$

Column: YMC-GPC T-30000, T-4000, and T-2000 (triconnection) 21.2*600 mm, 10 μm
Flow rate: 15 mL/min.
Elution solvent: ethyl acetate
Temperature: room temperature
Detection wavelength: 254, 285 nm

23-4. U-023-4

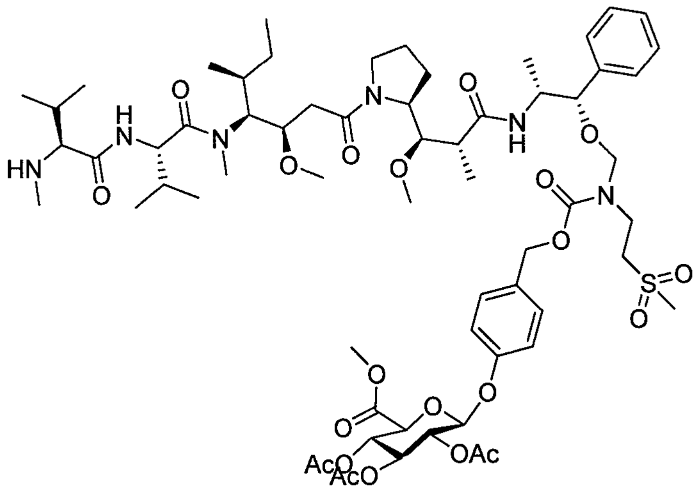

(U-023-4)

To a solution of U-023-3 (43.2 mg, 0.030 mmoL) and 2,6-dimethylpyridine (160 μL, 143 mg, 1.381 mmol) in dichloromethane (20 mL) in a 100 mL round-bottom flask was added trimethylsilyl trifluoromethanesulfonate (240 μL, 295.2 mg, 1.328 mmoL) under argon atmosphere with stirring under ice-cooling, and the resulting mixture was stirred under ice-cooling for 20 hours. Subsequently, to the reaction solution was added a saturated aqueous solution of sodium hydrogen carbonate (20 mL) under ice-cooling, the resulting mixture was stirred at room temperature for a while, and then dichloromethane was added thereto to be subjected to extraction. The resulting aqueous layer was subjected to extraction with dichloromethane, the resulting organic layers were combined, dried over anhydrous sodium sulfate, and concentrated. The resulting residues were purified under the following conditions, and the fraction comprising the target compound was concentrated under reduced pressure to give white foam. Acetonitrile (2 mL) and distilled water (4 mL) were added thereto, and then the resulting mixture was freeze-dried to give U-023-4 (18.3 mg, yield: 45.58%) as a white powder.
MS (ESI) m/z 1320 $(M+H)^+$

Column: Asahipak GS-510-20G, GS-310-20G, and GS-310-20G (triconnection) (shodex) 20*500 mm, 13 μm
Flow rate: 17 mL/min.
Elution solvent: acetonitrile
Detection wavelength: 220 nm

23-5. U-023

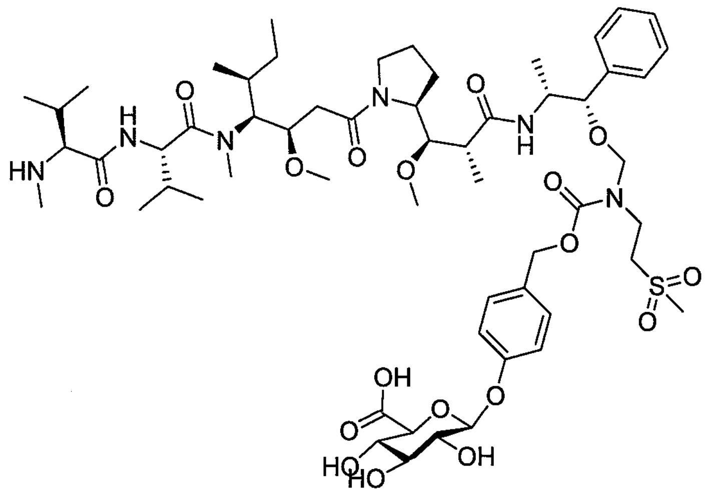

(U-023)

To a solution of U-023-4 (17.1 mg, 0.013 mmoL) in methanol (4 mL) in a 20 mL pear-shaped flask was added 1N lithium hydroxide (52 μL) under air atmosphere under ice-cooling with stirring, then the resulting mixture was warmed to room temperature, and stirred for 9.5 hours. Subsequently, a 50 mM aqueous solution of ammonium formate (4 mL, 12.61 mg, 0.200 mmoL) was added thereto. The resulting mixture was purified under the following conditions, and the fraction comprising the target compound was concentrated under reduced pressure. To the concentrated residues was added acetonitrile (15 mL), and then the resulting mixture was freeze-dried to give U-023 (13.10 mg, yield: 85.71%) as white solids.
MS (ESI) m/z 1180 $(M+H)^+$

Column: XSelect CSH, Prep Fluoro-Phenyl (Waters Corporation) 5 μm 19*150 mm
Flow rate: 17 mL/min.
Elution solvent: 10% aqueous solution of methanol (Solution A) - 90% aqueous solution of methanol (Solution B)
Gradient (Solution B): 50% (0.00 min.) -> 50% (3.00 min.) → 62% (9.50 min.)
Detection wavelength: 220 nm

Production example 24. U-024

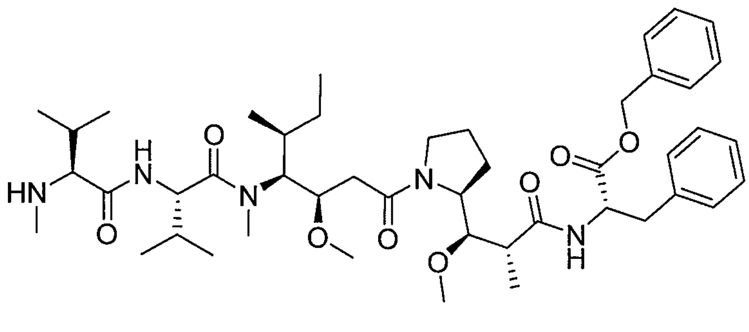

(U-024)

To a solution of MMAF (0.22 g, 0.301 mmoL) in dichloromethane (1 mL) in a 30 mL cylindrical flask was added methanesulfonic acid (58 μL, 0.09 g, 0.893 mmoL) under argon gas flow with stirring at room temperature, and the resulting mixture was stirred at 60°C for 4 hours. After the reaction was completed, to the reaction solution was added a

saturated aqueous solution of sodium hydrogen carbonate, and the resulting mixed solution was subjected to extraction with ethyl acetate. The resulting organic layer was washed with water, dried over anhydrous magnesium sulfate, filtered, and concentrated under reduced pressure. The resulting residues were subjected to YAMAZEN medium pressure preparative (Silica, M (16 g), ethyl acetate / methanol = 100/0 (V/V) → 60/40 (V/V)), and the fraction comprising the target compound was concentrated under reduced pressure to give U-024 (183.9 mg, yield: 74.61%) as colorless foam.
MS (ESI) m/z 821 $(M+H)^+$

Production example 25. U-025

25-1. U-025-1

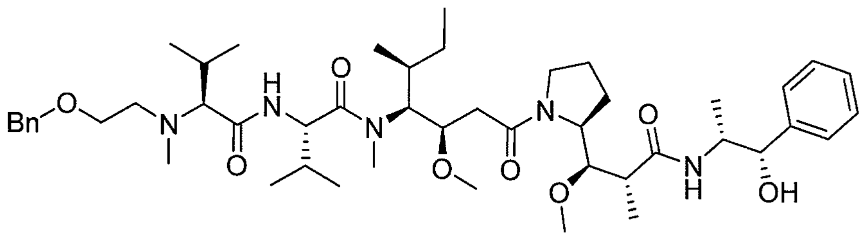

(U-025-1)

To a solution of MMAE (43.5 mg, 0.061 mmoL) in dichloromethane (1 mL) in a 5 mL sample bottle were sequentially added 2-(benzyloxy)acetoaldehyde (10.3 μL, 11.01 mg, 0.067 mmoL), acetic acid (5.2 μL, 5.49 mg, 0.091 mmol), and sodium triacetoxyborohydride (25.1 mg, 0.118 mmoL) with stirring at room temperature, and then the resulting mixture was stirred at room temperature for 1.5 hours. After the reaction was completed, a saturated aqueous solution of sodium hydrogen carbonate (1.5 mL) was added dropwise thereto with stirring at room temperature, and the resulting mixture was stirred at room temperature for 30 minutes. The resulting organic layers were separated, then the resulting aqueous layer was subjected to extraction with dichloromethane, the resulting organic layers were combined, dried over anhydrous sodium sulfate, and filtered. The resulting filtrate was concentrated, the resulting residues were subjected to YAMAZEN medium pressure preparative (Silica, S (7 g), dichloromethane/2-propanol = 95/5 (V/V)) (Rf = 0.47 (dichloromethane/2-propanol = 95/5 (V/V))), and the fraction comprising the target compound was concentrated under reduced pressure to give U-025-1 (43.8 mg, yield: 84.84%) as white foam.
MS (ESI) m/z 853 $(M+H)^+$

25-2. Synthesis of U-025

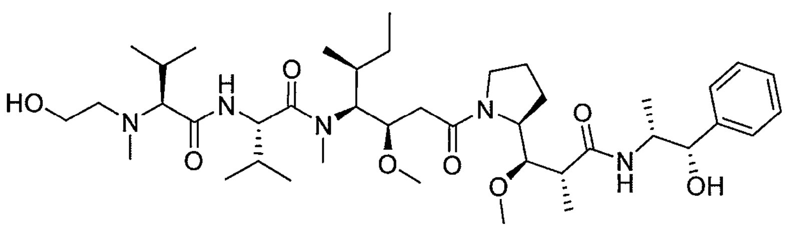

(U-025)

To a solution of U-025-1 (43.8 mg, 0.051 mmoL) in ethanol (2 mL) in a 50 mL pear-shaped flask was added 5% palladium carbon (ASCA-2 type, wetted with 50% water) (10.94 mg, 0.273 mg, 2.57 pmoL) under nitrogen atmosphere, the resulting mixture was degassed under reduced pressure, and then stirred under hydrogen atmosphere at room temperature for 1 hour. Subsequently, acetic acid (0.2 mL) was added thereto, and the resulting mixture was stirred at room temperature for 1 hour. Subsequently, the mixture was stirred with heating at a bath temperature of 60°C for 4.5 hours. After the reaction was completed, the reaction solution was allowed to cool, filtered using Celite 545 (trade name),

washed with ethanol, and the resulting filtrate was concentrated under reduced pressure. The concentrated residues were diluted with dichloromethane, washed with a saturated aqueous solution of sodium hydrogen carbonate, dried over anhydrous sodium sulfate, filtered, and the resulting filtrate was concentrated under reduced pressure. To the concentrated residues were added acetonitrile (2 mL) and distilled water (3 mL), and the resulting mixture was freeze-dried to give U-025 (34.6 mg, yield: 88.34%) as white solids.
MS (ESI) m/z 763 $(M+H)^+$

Production example 26. U-026

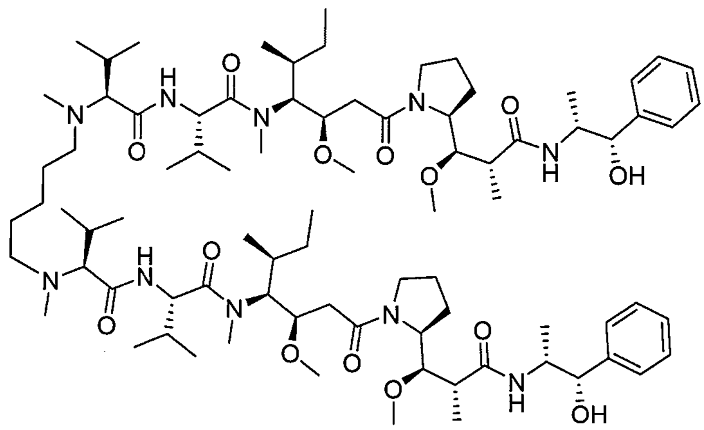

(U-026)

To a solution of MMAE (63.2 mg, 0.088 mmoL) in dichloromethane (1.5 mL) in a 5 mL sample bottle were sequentially added a 5.6 M aqueous solution of glutaraldehyde (8.00 μL, 4.49 mg, 0.045 mmoL), acetic acid (8.00 μL, 8.44 mg, 0.141 mmoL), and sodium triacetoxyborohydride (39.6 mg, 0.187 mmoL) with stirring at room temperature, and then the resulting mixture was stirred at room temperature for 2.5 hours. After the reaction was completed, a saturated aqueous solution of sodium hydrogen carbonate (2 mL) was added dropwise thereto with stirring at room temperature, and after the addition was completed, the resulting mixture was stirred at room temperature for 30 minutes. The resulting organic layers were separated, then the resulting aqueous layer was subjected to extraction with dichloromethane, the resulting dichloromethane layers were combined, dried over anhydrous sodium sulfate, filtered, and the resulting filtrate was concentrated under reduced pressure. The concentrated residues were purified under the following conditions, the fraction comprising the target compound was concentrated under reduced pressure, dissolved in dichloromethane, diethyl ether was added thereto, the resulting mixture was subjected to sonication, the resulting solids were collected by filtration under reduced pressure, washed with diethyl ether, and dried under reduced pressure to give U-026 (36.32 mg, yield: 53.9%) as white solids.
MS (ESI) m/z 753 $(M+2H)^{2+}$

Column: YMC-GPC T-30000, T-4000, and T-2000 (triconnection) 21.2*600 mm, 10 μm
Flow rate: 15 mL/min.
Elution solvent: ethyl acetate
Temperature: room temperature
Detection wavelength: 260 nm

Production example 27. U-027

27-1. U-027-1

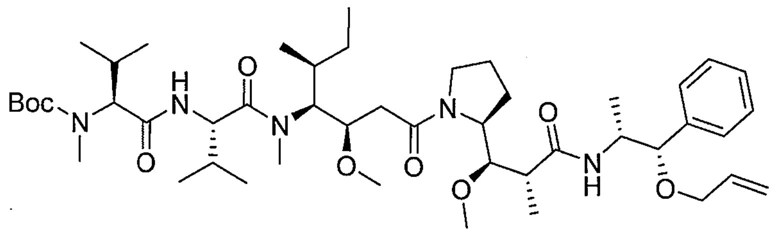

(U-027-1)

To a solution of U-011-1 (102.1 mg, 0.125 mmoL) in toluene (1.3 mL) in a 30 mL pear-shaped flask were added tetrabutylammonium hydrogen sulfate (2.16 mg, 6.36 pmoL) and 3-bromoprop-1-ene (16 μL, 22.37 mg, 0.185 mmoL) under air atmosphere with stirring at room temperature. Subsequently, a 50% by weight of aqueous solution of sodium hydroxide (230 μL, 345 mg, 4.31 mmoL) was added thereto at room temperature, and then the resulting mixture was stirred at room temperature for 5 hours. After the reaction was completed, to the reaction solution were added toluene (2 mL) and water (3 mL), the resulting solution was stirred, and then separated. The resulting organic layer was washed with water (2 mL). The resulting organic layer was diluted with ethyl acetate, sequentially washed with a 5% aqueous solution of potassium hydrogen sulfate, a saturated aqueous solution of sodium hydrogen carbonate, and saturated brine, dried over anhydrous magnesium sulfate, filtered, and the resulting filtrate was concentrated under reduced pressure to give white foam. To the resulting residues was added dichloromethane to dissolve the residues, the resulting solution was subjected to YAMAZEN medium pressure preparative (Chromatorex_COOH_MB100-40/75 (11.0 g), dichloromethane/2-propanol = 100/0 (V/V) - 90/10 (V/V), (Rf = 0.90 (dichloromethane/2-propanol = 80/20 (V/V)))), and the fraction comprising the target compound was concentrated under reduced pressure to give white foam. Acetonitrile (2 mL) and ultrapure water (2 mL) were added thereto, and the resulting mixture was freeze-dried to give U-027-1 (58.3 mg, yield: 54.44%) as white solids. MS (ESI) m/z 859 $(M+H)^+$

27-2. U-027-2

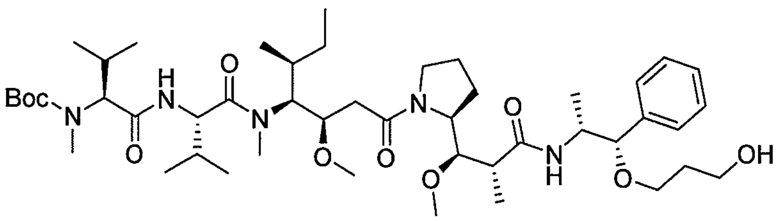

(U-027-2)

To a solution of U-027-1 (54.6 mg, 0.064 mmoL) in tetrahydrofuran (1 mL) in a 20 mL pear-shaped flask was added a 0.5 M solution of 9-BBN in tetrahydrofuran (640 μL, 39.05 mg, 0.320 mmoL) under argon atmosphere with stirring at room temperature, and the resulting mixture was stirred at room temperature for 2.33 hours. Subsequently, a 0.5 M solution of 9-BBN in tetrahydrofuran (360 μL, 21.96 mg, 0.180 mmoL) was additionally added thereto at room temperature, and the resulting mixture was stirred at room temperature for 2.33 hours. Subsequently, the mixture was ice-cooled, methanol (1 mL) was added thereto, then the resulting mixture was warmed to room temperature, stirred at room temperature for 1 hour, and concentrated under reduced pressure. To the concentrated residues were sequentially added tetrahydrofuran (1 mL), tetrahydrofuran (1 mL), and 1N sodium hydroxide (1.0 mL), then the resulting mixture was ice-cooled, a 30% by weight of aqueous solution of hydrogen peroxide (110 μL, 122.1 mg, 1.077 mmoL) was added thereto, and the resulting mixture was stirred under ice-cooling and then stirred at room temperature for 1 hour. Subsequently, water (5 mL) and ethyl acetate (5 mL) were added thereto to separate the mixture. The resulting organic layer was sequentially washed with a 5% by weight of aqueous solution of potassium hydrogen sulfate, a saturated aqueous solution of sodium hydrogen carbonate, and saturated brine, dried over anhydrous magnesium sulfate, filtered, and the resulting filtrate was concentrated under reduced pressure to give a colorless oil. To the resulting residues was added dichloromethane to dissolve the residues, the resulting solution was subjected to YAMAZEN medium pressure preparative (Chromatorex_COOH (LotNo.HU20999)_MB100-40/75 (11.0 g), dichloromethane/2-propanol = 100/0 (V/V) -, 90/10

(V/V)), and the fraction comprising the target compound was concentrated under reduced pressure to give a colorless oil. Acetonitrile (1 mL) and ultrapure water (1 mL) were added thereto, and the resulting mixture was freeze-dried to give U-027-2 (39.2 mg, yield: 70.32%) as white solids.
MS (ESI) m/z 877 $(M+H)^+$

27-2. U-027

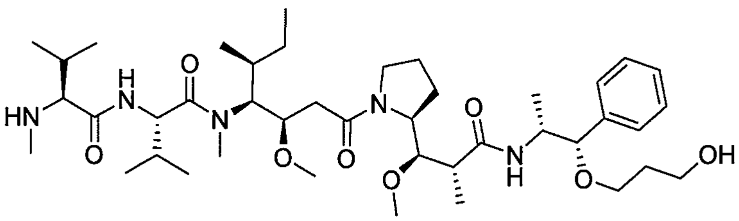

(U-027)

To a solution of U-027-2 (35.6 mg, 0.041 mmoL) in dichloromethane (3 mL) in a 30 mL pear-shaped flask were added 2,6-dimethylpyridine (99 μL, 91.6 mg, 0.856 mmoL) and trimethylsilyl trifluoromethanesulfonate (139 μL, 171 mg, 0.771 mmoL) under argon atmosphere under ice-cooling with stirring, and the resulting mixture was stirred under ice-cooling for 6 hours. To the reaction solution were added dichloromethane (4 mL) and a saturated aqueous solution of sodium hydrogen carbonate (8 mL) under ice-cooling to separate the solution. The resulting aqueous layer was subjected to extraction with dichloromethane, the resulting dichloromethane layers were combined, washed with saturated brine, dried over anhydrous sodium sulfate, filtered, and the resulting filtrate was concentrated under reduced pressure to give a pale yellow oil. The oil was purified under the following conditions, and the fraction comprising the target compound was concentrated under reduced pressure. To the concentrated residues were added acetonitrile and water, and then the resulting mixture was freeze-dried to give U-027 (21.69 mg, yield: 68.79%) as white solids.
MS (ESI) m/z 777 $(M+H)^+$

Column: Waters SunFire Prep C18 5 μm ODB 19*150 mm
Flow rate: 17 mL/min.
Elution solvent: acetonitrile
Detection wavelength: 220 nm

Production example 28. U-028

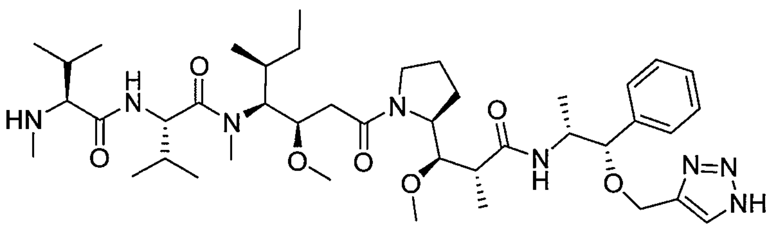

(U-028)

To a solution of U-022-2 (26.4 mg, 0.035 mmol), a solution of 4-methylbenzenesulfonylazide in 11 to 15% toluene (0.095 mL, 85.5 mg, 0.048 mmoL), and 2-aminophenol (4.0 mg, 0.037 mmoL) in acetonitrile (0.3 mL) in a 20 mL cylindrical flask was added copper(II) acetate (3.2 mg, 0.018 mmoL) under argon gas flow with stirring at room temperature, the resulting mixture was stirred at room temperature for 5 hours, and then left to stand for 15 hours. After the reaction was completed, acetonitrile was removed by a nitrogen blow, methanol (1 mL) was added thereto, and the resulting mixture was heated under reflux for 2 hours. The resulting residues were subjected to preparative HPLC chromatography under the following conditions, the fraction comprising the target compound was concentrated under reduced pressure to distill

away acetonitrile, and then the resulting residues were freeze-dried to give crude products of U-028 as white solids.

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 20% (0.00 min.) -> 100% (10.00 min.)

The resulting solids were purified under the following conditions, and then freeze-dried to give U-028 (2.1 mg, yield: 7.53%) as white solids.
MS (DUIS) m/z 799 $(M+H)^+$

Device: LC Forte/R (YMC Co., Ltd.)
Column: CHIRALPAK IB (20 x 250 mm, 5 um) (Daicel Corporation)
Eluent: MeOH
Flow rate: 15 mL/min
Wavelength: 220, 254, 285 nm

Production example 29. U-029

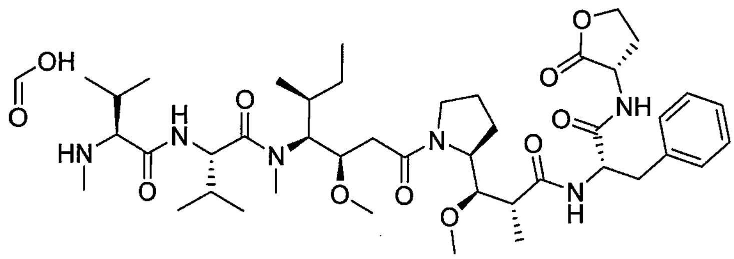

(U-029)

MMAF (49.7 mg, 0.068 mmoL) and (S)-(-)-α-amino-γ-butyrolactone hydrochloride (i.e., L-homoserine lactone hydrochloride) (14.1 mg, 0.102 mmoL) in a 30 mL cylindrical tube were dissolved in N,N-dimethylformamide (1.0 mL), and triethylamine (38 μL, 27.59 mg, 0.273 mmoL) was added thereto. Subsequently, HATU (28.6 mg, 0.075 mmol) was added thereto at room temperature, and the resulting mixture was stirred at room temperature for 12 hours. The reaction solution was concentrated under reduced pressure, the resulting residues were subjected to preparative HPLC chromatography under the following conditions, the fraction comprising the target compound was concentrated under reduced pressure to distill away acetonitrile, and then the resulting residues were freeze-dried to give formate of U-029 (19.6 mg, yield: 33.52%) as colorless foam.
MS (ESI) m/z 816 $(M+H)^+$

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 25% (0.00 min.) → 38% (6.00 min.)

Production example 30. U-030

30-1. U-030-1

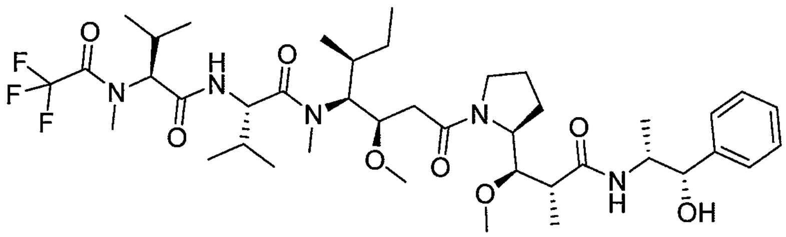

(U-030-1)

To a solution of MMAE (0.7545 g,1.051 mmol) in dichloromethane (15 mL) in a 50 mL pear-shaped flask were sequentially added triethylamine (0.73 mL, 0.53 g, 5.24 mmoL) and 2,2,2-trifluoroacetic anhydride (0.73 mL, 1.1 g, 5.25 mmoL) under argon atmosphere with stirring under ice-cooling, and the resulting mixture was stirred under ice-cooling for 1 hour. Subsequently, methanol (3.8 mL, 3.01 g, 94 mmoL) and triethylamine (0.38 mL, 0.28 g, 2.73 mmoL) were sequentially added thereto under ice-cooling, and the resulting mixture was stirred under ice-cooling for 1 hour. Subsequently, the mixture was concentrated under reduced pressure, ethyl acetate (30 mL) was added thereto, the resulting mixture was sequentially washed with a 5% by weight of aqueous solution of potassium hydrogen sulfate (30 mL), a saturated aqueous solution of sodium hydrogen carbonate (30 mL), and saturated brine (30 mL), dried over anhydrous magnesium sulfate, filtered, and the resulting filtrate was concentrated under reduced pressure. To the resulting residues were added acetonitrile (10 mL) and ultrapure water (8 mL), the resulting mixture was frozen, and freeze-dried to give U-030-1 (0.8320 g, yield: 97.27%) as white solids.
MS (ESI) m/z 815 $(M+H)^+$

30-2. U-030-2

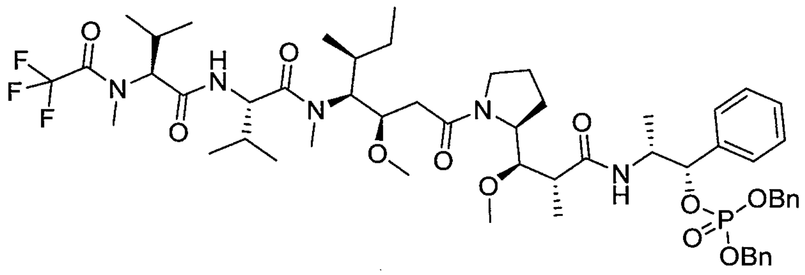

(U-030-2)

To a solution of U-030-1 (0.8011 g,0.984 mmol) and 1H-tetrazole (0.4840 g, 6.91 mmoL) in dichloromethane (30 mL) in a 50 mL pear-shaped flask was added dibenzyl diisopropylphosphoramidite (1.28 mL, 1.33 g, 3.85 mmoL) under argon atmosphere with stirring at room temperature, and the resulting mixture was stirred at room temperature for 2 hours. Subsequently, 30% by weight of hydrogen peroxide water (0.60 mL, 0.67 g, 5.87 mmoL) was added thereto under ice-cooling, and the resulting mixture was stirred under ice-cooling for 1.5 hours.

Subsequently, to sodium thiosulfate (12.75 g) was added a saturated aqueous solution of sodium hydrogen carbonate under ice-cooling to prepare a solution (100 mL), and 20 mL of said solution was added to the above mixture. The resulting organic layer was separated, then washed with saturated brine, dried over anhydrous magnesium sulfate, filtered, and the resulting filtrate was concentrated under reduced pressure.

The resulting residues were subjected to YAMAZEN medium pressure preparative ((Fuji Silysia Chromatorex Q-PACK, DIOL-60), dichloromethane/isopropanol = 99/1 (V/V) → 95/5 (V/V)), and the fraction comprising the target compound was concentrated under reduced pressure to give crude products of U-030-2.

The resulting crude products were purified under the following conditions, and the fraction comprising the target compound was concentrated under reduced pressure. To the resulting residues were added acetonitrile (10 mL) and ultrapure water (8 mL), the resulting mixture was frozen, and freeze-dried to give U-030-2 (0.6915 g, yield: 65.41%) as white solids.
MS (ESI) m/z 1075 $(M+H)^+$

Device: LC Forte/R (YMC Co., Ltd.)

Column: YMC-GPC T-30000, T-4000, and T-2000 (triconnection) 21.2*600 mm, 10 μm
Temperature: room temperature
Eluent: acetonitrile
Flow rate: 25 mL/min
Wavelength: 220 nm

30-3. U-030-3

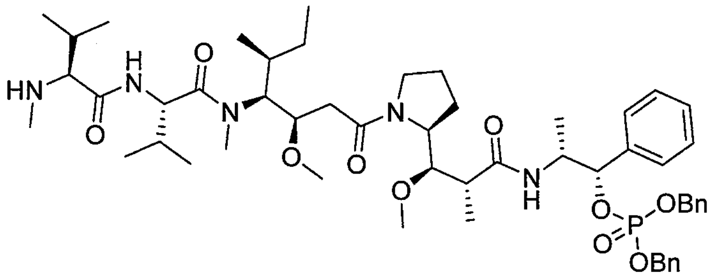

(U-030-3)

To a solution of U-030-2 (331 mg, 0.308 mmol) in methanol (5 mL) in a 50 mL pear-shaped flask was added sodium borohydride (34.6 mg, 0.915 mmol) under argon atmosphere with stirring under ice-cooling, and the resulting mixture was stirred under ice-cooling for 1.25 hours. Subsequently, sodium borohydride (107.4 mg, 2.838 mmol) was added dividedly (three parts) thereto under ice-cooling, and the resulting mixture was stirred under ice-cooling for 3 hours.
After the reaction was completed, dichloromethane (20 mL) and a saturated aqueous solution of sodium hydrogen carbonate (20 mL) were added thereto under ice-cooling, and the resulting organic layer was separated. The resulting aqueous layer was subjected to extraction with dichloromethane (20 mL) three times, the resulting organic layers were combined, dried over anhydrous sodium sulfate, filtered, and the resulting filtrate was concentrated under reduced pressure to give U-030-3 (325 mg, yield: quantitative) as a colorless oil.
MS (ESI) m/z 979 $(M+H)^+$

30-4. U-030

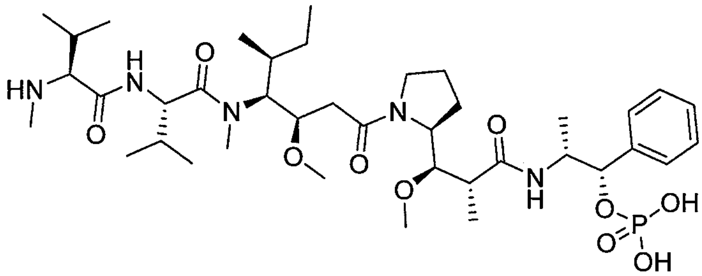

(U-030)

To a solution of U-030-3 (301 mg, 0.308 mmol) in ethanol (30 mL) in a 100 mL pear-shaped flask was added 5% by weight of palladium carbon STD type (wetted with 50% by weight of water, 82.3 mg, 0.019 mmoL) under nitrogen atmosphere with stirring, and the resulting mixture was stirred under hydrogen atmosphere at room temperature for 2 hours.
After the reaction was completed, the mixture was filtered through Celite 545 (trade name), washed with ethanol, and concentrated under reduced pressure. To the resulting residues was added acetonitrile, the resulting solids were collected by filtration, washed with ice-cooled acetonitrile, and dried under reduced pressure to give U-030 (220.3 mg, yield: 89.72%) as white solids.

MS (ESI) m/z 799 $(M+H)^+$

Production example 31. U-031

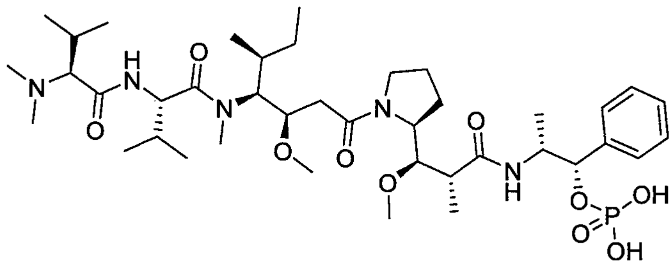

(U-031)

To a solution of the Production example 30 (100.4 mg, 0.126 mmoL) and a 37% aqueous solution of formaldehyde (0.093 mL, 101.37 mg, 1.249 mmoL) in methanol (1 mL) in a 20 mL cylindrical flask was added sodium triacetoxyborohydride (77.8 mg, 0.367 mmoL) under argon atmosphere with stirring at room temperature, and the resulting mixture was stirred at room temperature for 1 hour.

After the reaction was completed, the reaction mixture was concentrated under reduced pressure, and water was added thereto. The resulting aqueous solution was subjected to preparative HPLC chromatography under the following conditions, and the fraction comprising the target compound was concentrated under reduced pressure to give crudely purified products of U-031 (41.0 mg) as white solids.

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 10% (0.00 min.) -> 30% (4.00 min.) -> 30% (9.00 min.)

The resulting crudely purified products of U-031 (41.0 mg) were subjected to preparative HPLC chromatography under the following conditions, and the fraction comprising the target compound was freeze-dried to give U-031 (29.5 mg, yield: 28.87%) as white solids.
MS (ESI) m/z 810 $(M-H)^-$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 10% (0.00 min.) → 30% (5.00 min.) -> 30% (10.00 min.)

Production example 32. U-032

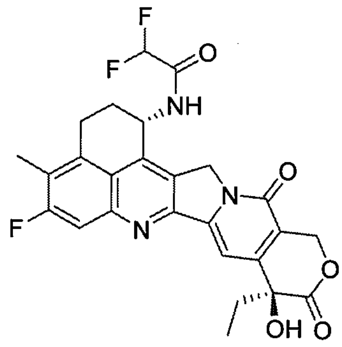

(U-032)

To a solution of exatecan mesylate (13.6 mg, 0.026 mmoL) in dichloromethane (1 mL) in a 10 mL cylindrical flask was added triethylamine (35 μL, 25.41 mg, 0.251 mmoL) under argon gas flow with stirring, and the resulting mixture was ice-cooled. 2,2-Difluoroacetic anhydride (10 μL, 16 mg, 0.092 mmoL) was added thereto under ice-cooling, and the resulting mixture was stirred at 0°C for 2 hours. Subsequently, triethylamine (17 μL, 12.34 mg, 0.122 mmoL) and 2,2-difluoroacetic anhydride (10 μL, 16 mg, 0.092 mmoL) were added thereto under stirring at room temperature, and the resulting mixture was stirred at room temperature for 1 hour. Subsequently, methanol (100 μL, 79.2 mg, 2.472 mmoL) was added thereto under stirring at room temperature, and the resulting mixture was stirred at room temperature for 0.5 hour. Subsequently, triethylamine (60 μL, 43.56 mg, 0.430 mmoL) was added thereto under stirring at room temperature, and the resulting mixture was stirred at room temperature for 0.5 hour.

After the reaction was completed, diethyl ether (2 mL) was added thereto, the resulting mixture was stirred for 5 minutes, filtered through a membrane filter, the resulting solids were washed with ethyl acetate and water, and dried under reduced pressure to give U-032 (7.9 mg, yield: 60.13%) as slightly yellow solids.

MS (ESI) m/z 514 $(M+H)^+$

Production example 33. U-033

33-1. U-033-1

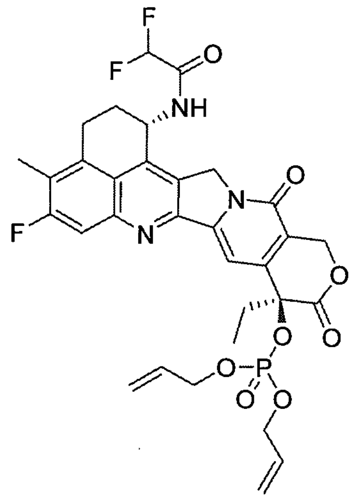

(U-033-1)

To a solution of U-032 (419 mg, 0.816 mmoL) in dichloromethane (12 mL) in a 100 mL cylindrical flask were sequentially added diallyl N,N-diisopropylphosphoramidite (820 μL, 760.96 mg, 3.10 mmoL) and 1H-tetrazole (373 mg, 5.32 mmoL) under argon atmosphere with stirring at room temperature, and the resulting mixture was stirred at room temperature for 5 hours. Subsequently, 30% by weight of hydrogen peroxide water (480 μL, 532.8 mg, 4.70 mmoL) was

added thereto under ice-cooling, and the resulting mixture was stirred under ice-cooling for 1 hour. Subsequently, to sodium thiosulfate pentahydrate (20.0 g, 80.6 mmoL) and sodium hydrogen carbonate (7.0 g, 83.3 mmoL) was added water (100 mL) to prepare a solution, 6 mL of said solution and dichloromethane (6 mL) were added to the above mixture to separate it. The resulting aqueous layer was subjected to extraction with dichloromethane (6 mL), combined with the organic layer, dried over anhydrous magnesium sulfate (0.80 g), filtered, and the resulting filtrate was concentrated under reduced pressure to give concentrated residues (967 mg). To the resulting residues was added a 5% aqueous solution of acetonitrile, the resulting mixture was subjected to sonication, filtered under reduced pressure, and the resulting solids were dried to give U-033-1 (250 mg, yield: 45.48%) as brown solids.
MS (ESI) m/z 1348 $(2M+H)^+$

33-2. U-033

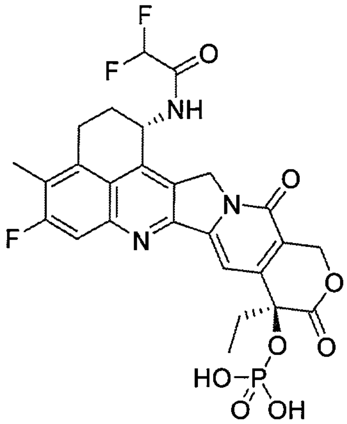

(U-033)

To a solution of U-033-1 (243 mg, 0.361 mmoL) in tetrahydrofuran (5 mL) in a 50 mL round-bottom flask were sequentially added N-methylaniline (84 μL, 83.16 mg, 0.776 mmoL) and tetrakis(triphenylphosphine)palladium (43 mg, 0.037 mmoL) under argon atmosphere with stirring at room temperature, and the resulting mixture was stirred at room temperature for 1 hour.

After the reaction was completed, tert-butyl methyl ether (15 mL) was added thereto, the resulting mixture was stirred for 10 minutes, then the precipitated solids were filtered, washed with tert-butyl methyl ether, and dried under reduced pressure to give crude products of U-033 (256 mg) .

The resulting solids were dissolved in a 30% aqueous solution of acetonitrile (15 mL) containing 2% by weight of ammonium acetate, the resulting solution was subjected to preparative HPLC chromatography under the following conditions, and the fraction comprising the target compound was freeze-dried to give U-033 (81 mg, yield: 37.83%) as pale yellow solids.
MS (ESI) m/z 592 $(M-H)^-$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) -> 60% (6.00 min.) → 90% (6.50 min.) -> 90% (9.00 min.)

Production example 34. U-034

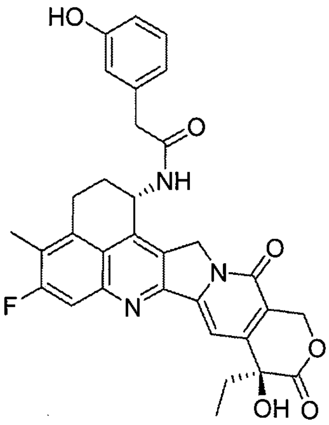

(U-034)

To a solution of exatecan mesylate (53.2 mg, 0.100 mmoL), 2-(3-hydroxyphenyl)acetic acid (18.3 mg, 0.120 mmoL), 1-ethyl-3-(3-dimethylaminopropyl)carbodiimide hydrochloride (38.2 mg, 0.199 mmoL), and 1-hydroxybenzotriazole (15.3 mg, 0.100 mmoL) in N,N-dimethylformamide (1 mL) in a 10 mL cylindrical flask was added triethylamine (28 μL, 20.33 mg, 0.201 mmoL) under argon atmosphere with stirring at room temperature, and the resulting mixture was stirred at room temperature for 2 hours.

After the reaction was completed, the resulting reaction solution was subjected to preparative HPLC chromatography under the following conditions, the fraction comprising the target compound was concentrated under reduced pressure, the precipitated solids were collected by filtration, and washed with water to give U-033 (25.4 mg, yield: 44.56%) as colorless solids.
MS (ESI) m/z 568 $(M-H)^-$

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 50% (0.00 min.) → 95% (5.00 min.)

Production example 35. U-035

35-1. U-035-1

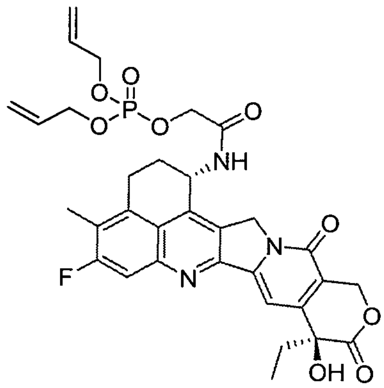

(U-033-1)

To a solution of DXd (162.6 mg, 0.329 mmoL) in N,N-dimethylformamide (4 mL) in a 50 mL pear-shaped flask was added trifluoromethanesulfonic acid (29.0 μL, 49.3 mg, 0.329 mmoL) under argon atmosphere with stirring, and the resulting mixture was stirred at room temperature for 30 minutes. Subsequently, diallyl N,N-diisopropylphosphoramidite

(350 μE, 324.8 mg, 1.324 mmoL) and 1H-tetrazole (163.7 mg, 2.337 mmoL) were added thereto at room temperature, and the resulting mixture was stirred at room temperature for 2 hours.

Subsequently, a 70% aqueous solution of tert-butyl peroxide (270 μL, 253.8 mg, 1.971 mmoL) was added thereto at room temperature, and the resulting mixture was stirred at room temperature for 1.5 hours.

Subsequently, to the reaction solution was added diethyl ether (40 mL), and the resulting mixture was stirred under ice-cooling for a while. The resulting solids were collected by filtration under reduced pressure, washed with diethyl ether, and then dried under reduced pressure to give trifluoromethanesulfonate of U-035-1 (249.6 mg, yield: 94.26%) as light brown solids.
MS (ESI) m/z 654 $(M+H)^+$

35-2. U-035

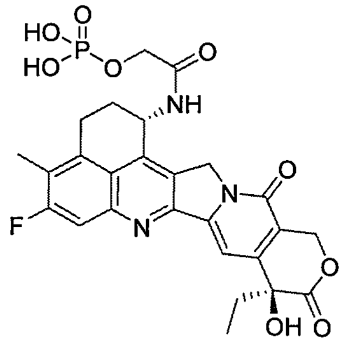

(U-035)

To a solution of trifluoromethanesulfonate of U-035-1 (51.4 mg, 0.064 mmoL) in tetrahydrofuran (5 mL) in a 20 mL pear-shaped flask were sequentially added N-methylaniline (22 μL, 21.78 mg, 0.203 mmoL) and tetrakis(triphenylphosphine)palladium (10.4 mg, 9.00 pmoL) under argon atmosphere with stirring at room temperature, and the resulting mixture was stirred at room temperature for 1.25 hours. Subsequently, tetrakis(triphenylphosphine)palladium (27.9 mg, 0.024 mmoL) was additionally added thereto, then N,N-dimethylformamide (0.5 mL) was added thereto, and the resulting mixture was stirred at room temperature for 0.75 hour.

The reaction solution was concentrated under reduced pressure. To the concentrated residues was added diethyl ether, the resulting mixture was subjected to sonication, the resulting solids were collected by filtration, washed with diethyl ether, and dried under reduced pressure to give gray-brown solids (55.2 mg).

The resulting gray-brown solids were subjected to the following recycle preparative, and the fraction comprising the target compound was freeze-dried to give U-035 (6.4 mg, yield: 17.45%) as pale yellow solids.
MS (ESI) m/z 574 $(M+H)^+$

Device: LC Forte/R (YMC Co., Ltd.)
Column: Xbridege C18, 30x150 mm, 5 μm
Eluent: 0.1% aqueous solution of formic acid (Solution A) - 0.1% solution of formic acid in acetonitrile (Solution B)
Solution composition (Solution B): 30%
Flow rate: 25 mL/min, 21 MPa
Wavelength: 254 nm

Production example 36. U-036

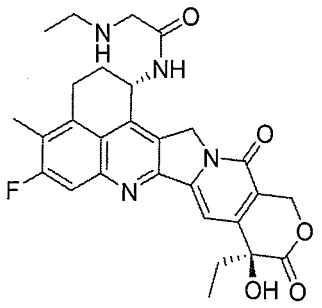

(U-036)

To a solution of N-(((9H-fluoren-9-yl)methoxy)carbonyl)-N-ethylglycine (15.06 mg, 0.046 mmoL) in N,N-dimethylformamide (0.6 mL) in a 20 mL cylindrical flask were added HATU (17.60 mg, 0.046 mmoL) and N,N-diisopropylethylamine (0.022 mL, 16.32 mg, 0.126 mmoL) under argon gas flow with stirring at room temperature, and the resulting mixture was stirred at room temperature for 15 minutes.

Subsequently, exatecan mesylate (21.7 mg, 0.042 mmoL) was added thereto under stirring at room temperature, and the resulting mixture was stirred at room temperature for 1 hour.

Subsequently, piperidine (4.16 μL, 3.58 mg, 0.042 mmoL) was added thereto under stirring at room temperature, and the resulting mixture was stirred at room temperature for 3 hours.

The resulting residues were subjected to preparative HPLC chromatography under the following conditions, and the fraction comprising the target compound (Rt = approximately 1.5 min.) was freeze-dried to give formate of U-036 (6.9 mg, yield: 31.5%) as white solids.

MS (ESI) m/z 521 $(M+H)^+$
Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 15% (0.00 min.) → 55% (10.00 min.)

[Examples] Synthesis of conjugate precursor (I) and synthetic intermediate (II) of conjugate precursor

Synthesis of Example 1

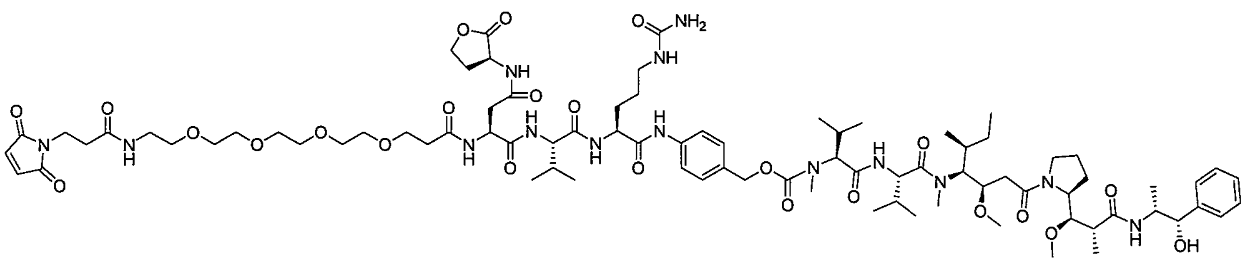

Example 1-1

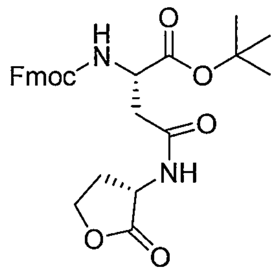

(S)-(-)-α-amino-γ-butyrolactone hydrochloride (i.e., L-homoserine lactone hydrochloride) (1.75 g, 12.72 mmoL) in a 200 mL round-bottom flask was dissolved in N,N-dimethylformamide (60 mL), and then Fmoc-(L)-Asp-tert-butyl (5.23 g, 12.71 mmoL) was added thereto, and triethylamine (3.65 mL, 2.65 g, 26.2 mmoL) was added thereto. Subsequently, HATU (4.83 g, 12.70 mmoL) was added thereto at room temperature, and the resulting mixture was stirred at room temperature for 2 hours. After the reaction was completed, to the reaction solution was added water, and the resulting mixed solution was subjected to extraction with ethyl acetate twice. The resulting organic layer was concentrated under reduced pressure to give the title compound (6.16 g, yield: 97.9%) as a colorless oil.

Example 1-2

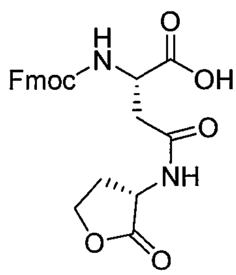

The Example 1-1 (6.16 g, 12.46 mmoL) in a 500 mL round-bottom flask was dissolved in dichloromethane (60 mL), then trifluoroacetic acid (60 mL, 1.421 g, 12.46 mmoL) was added thereto, and the resulting mixture was stirred at room temperature for 2 hours. After the reaction was completed, the solvent was distilled away under reduced pressure. The resulting residues were dissolved in acetonitrile (50 mL), and ethyl acetate (50 mL) was additionally added thereto. The precipitated solids were collected by filtration, and washed with ethyl acetate to give the title compound (3.86 g, yield: 70.68%) as colorless solids.
MS (ESI) m/z 439 $(M+H)^+$

Example 1-3

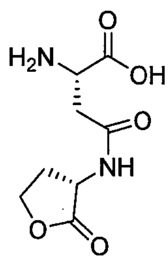

To a solution of the Example 1-2 (298 mg, 0.680 mmoL) in N,N-dimethylformamide (4 mL) in a 30 mL cylindrical flask was added piperidine (0.270 mL, 232.2 mg, 2.73 mmoL) under argon gas flow with stirring at room temperature, and the resulting mixture was stirred at room temperature for 1 hour.

After the reaction was completed, xylene was added thereto, and the solvent was removed under reduced pressure.

To the resulting residues were added diethyl ether (4 mL) and ethyl acetate (8 mL), the resulting mixture was subjected to sonication, and the resulting solids were collected by filtration. To the collected solids was added a 20% aqueous solution of acetonitrile, and the resulting mixture was concentrated under reduced pressure.

To the resulting residues was added ethyl acetate, and the resulting mixture was filtered. To the resulting solids was added diethyl ether, the resulting mixture was filtered, and dried under reduced pressure to give the title compound (143 mg, yield: 97.32%) as white solids. MS (ESI) m/z 215 $(M-H)^-$

Example 1-4

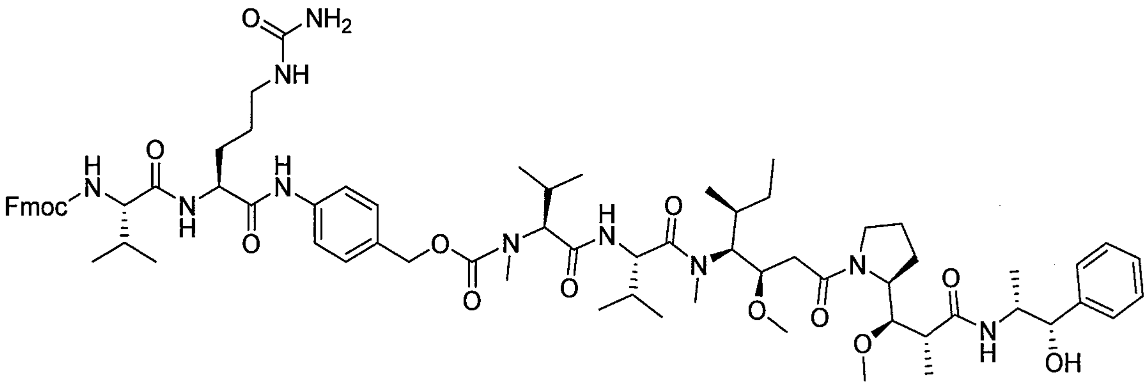

To a solution of Fmoc-(L)-Val-(L)-Cit-PAB-PNP (manufactured by Angene) (292.7 mg, 0.382 mmoL), MMAE (manufactured by MedChemExpress) (259.7 mg, 0.362 mmoL), and 1-hydroxy-7-azabenzotriazole (56.3 mg, 0.414 mmoL) in N,N-dimethylformamide (3 mL) in a 30 mL cylindrical flask was added N,N-diisopropylethylamine (70 μE, 51.94 mg, 0.402 mmoL) under nitrogen atmosphere with stirring, and the resulting mixture was stirred at room temperature for 19.5 hours. After the reaction was completed, the solvent was distilled away under reduced pressure. To the resulting residues was added diethyl ether (20 mL), the precipitated solids were collected by filtration, washed with water, and then washed with diethyl ether to give the title compound (415.8 mg, yield: 85.43%) as beige solids.
MS (DUIS) m/z 673 $(M+2H)^{2+}$

Example 1-5

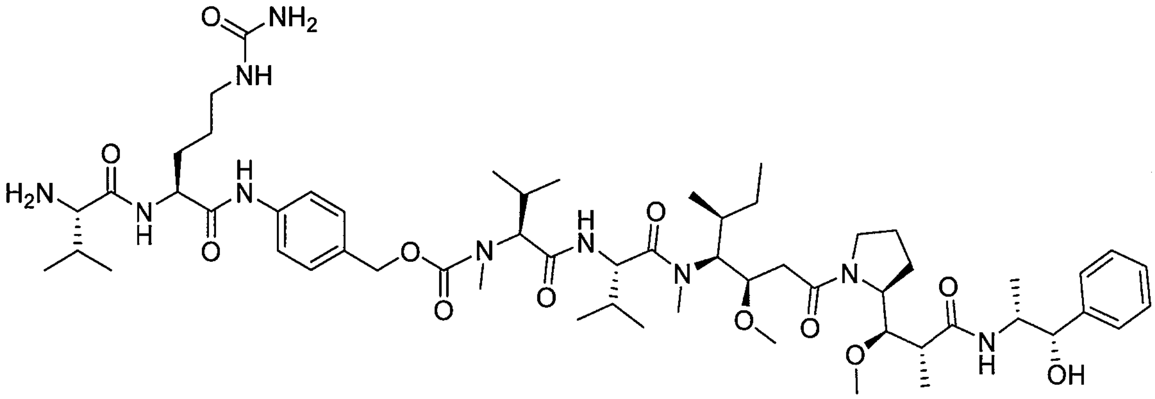

To a solution of the Example 1-4 (414.7 mg, 0.308 mmoL) in N,N-dimethylformamide (5 mL) in a 30 mL pear-shaped flask was added piperidine (0.244 mL, 210 mg, 2.465 mmoL) at room temperature, and then the resulting mixture was stirred at room temperature for 1 hour. The reaction solution was concentrated under reduced pressure. To the resulting residues were added ethyl acetate, acetonitrile, and dichloromethane to dissolve the residues. Then, diethyl ether was added thereto, the resulting mixture was subjected to sonication, the resulting solids were collected by filtration, washed with diethyl ether, and dried under reduced pressure to give the title compound (280.3 mg, yield: 80.96%) as pale yellow solids.

MS (ESI) m/z 1124 $(M+H)^+$

Example 1-6

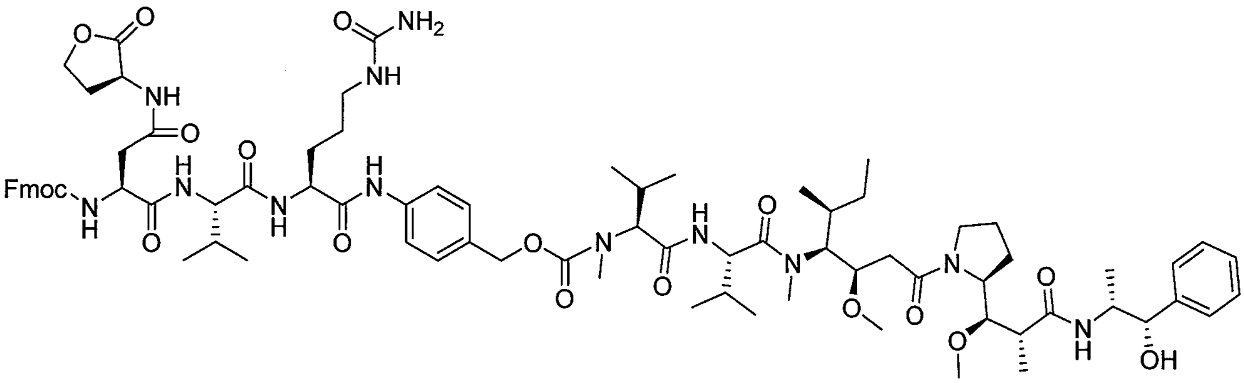

To a solution of the Example 1-2 (130.1 mg, 0.297 mmoL) and the Example 1-5 (309 mg, 0.275 mmoL) in N,N-dimethylformamide (5 mL) in a 30 mL cylindrical flask was added triethylamine (41 μL, 29.77 mg, 0.294 mmoL) at room temperature, then HATU (112.9 mg, 0.297 mmoL) was added thereto at room temperature, and then the resulting mixture was stirred at room temperature for 2 hours. The reaction solution was concentrated under reduced pressure, dichloromethane and 2-propanol were added thereto, and the resulting mixture was concentrated under reduced pressure. Acetonitrile (10 mL) and water (10 mL) were added thereto, the resulting mixture was subjected to sonication, then the resulting solids were collected by filtration, washed with acetonitrile/water (1/1 (V/V)) (10 mL), then washed with diethyl ether (20 mL), and the resulting solids were dried under reduced pressure to give the title compound (342.3 mg, yield: 80.61%) as white solids.
MS (ESI) m/z 773 $(M+2H)^{2+}$

Example 1-7

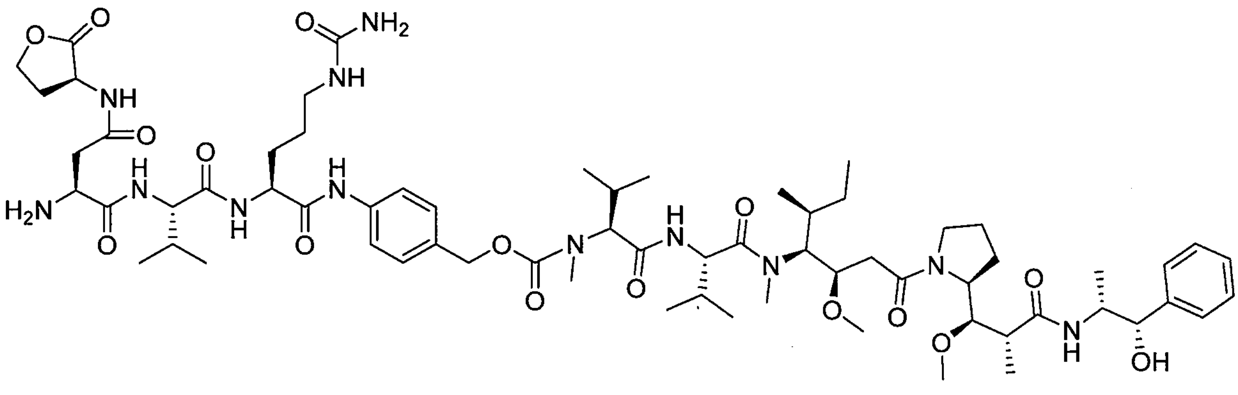

To a solution of the Example 1-6 (49.7 mg, 0.032 mmoL) in N,N-dimethylformamide (450 μL) in a 10 mL pear-shaped flask was added piperidine (10 μL, 8.62 mg, 0.101 mmoL) under argon gas flow with stirring at room temperature, the resulting mixture was stirred at room temperature for 50 minutes, then the solvent was removed under reduced pressure, the resulting residues were washed with diethyl ether (10 mL), and the resulting solids were dried under reduced pressure to give the title compound (36.4 mg, yield: 85.56%) as white solids.
MS (ESI) m/z 1323 $(M+H)^+$

Example 1

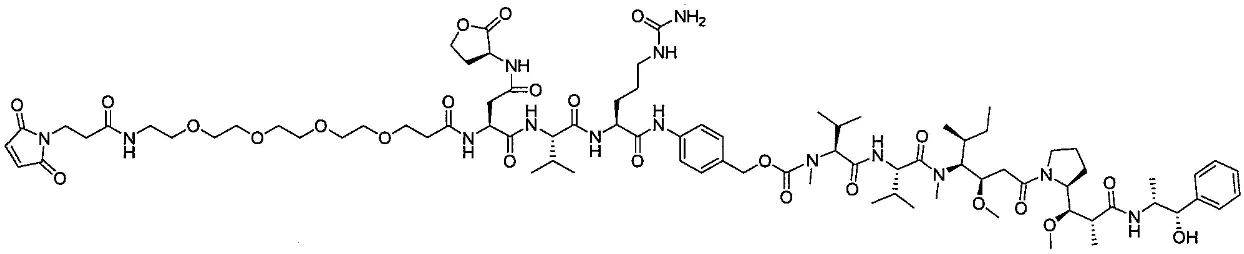

To a solution of the Example 1-7 (10.0 mg, 7.57 μmoL) in N,N-dimethylformamide (0.4 mL) in a 5 mL cylindrical flask was added a solution of N-succinimidyl 1-maleimide-3-oxo-7,10,13,16-tetraoxa-4-azanonadecanoate (8.2 mg, 0.027 mmoL) in acetonitrile (400 μE) under argon atmosphere with stirring, and the resulting mixture was stirred at room temperature for 1 hour.

The resulting reaction solution was subjected to preparative HPLC chromatography under the following conditions, and the fraction comprising the target compound was freeze-dried to give the Example 1 (3.2 mg, yield: 24.59%) as white solids.
MS (ESI) m/z 861 $(M+2H)^{2+}$

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 40% (0.00 min.) → 90% (10.00 min.)

Synthesis of Example 2

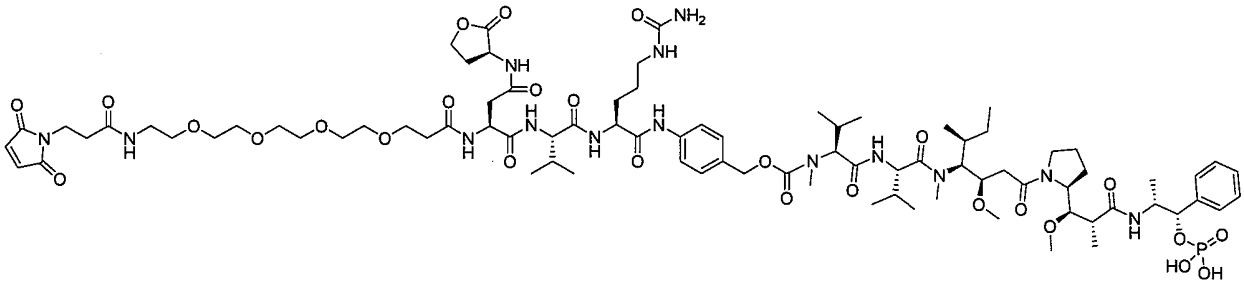

Example 2-1

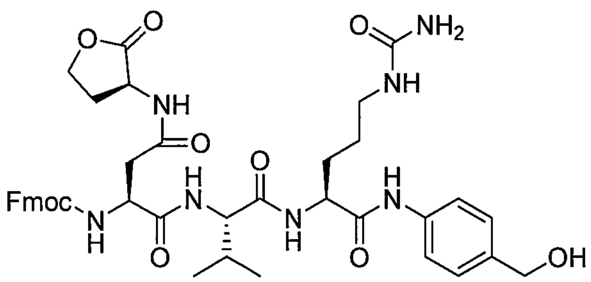

(L)-Val-(L)-Cit-PAB (manufactured by Angene) (1.90 g, 5.01 mmoL) and the Example 1-2 (2.24 g, 5.11 mmoL) in a 200 mL round-bottom flask were suspended in N,N-dimethylformamide (50 mL), triethylamine (0.71 mL, 0.52 g, 5.09 mmoL) and then HATU (1.94 g, 5.10 mmoL) were added thereto, and the resulting mixture was stirred at room temperature for 1 hour. To the resulting reaction solution were added water (30 mL) and acetone (20 mL), the resulting mixture was stirred for 30 minutes, then filtered, washed with water, and then washed with acetone to give the title compound (3.37 g, yield: 84.13%) as slightly yellow solids. MS (ESI) m/z 800 $(M+H)^+$

Example 2-2

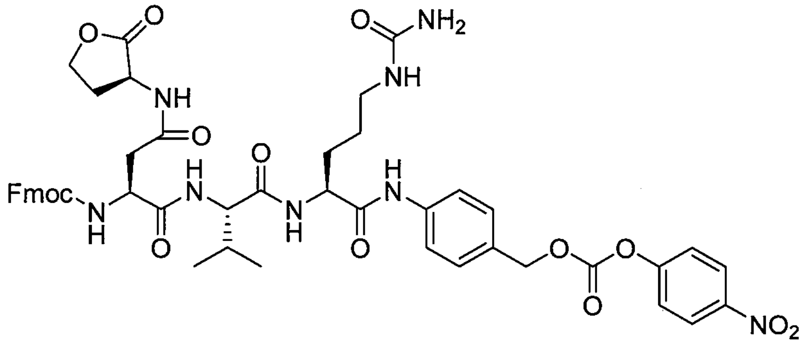

To a solution of the Example 2-1 (1.027 g, 1.284 mmoL) in N,N-dimethylformamide (12 mL) in a 100 mL round-bottom flask were added bis(4-nitrophenyl)carbonate (760 mg, 2.498 mmoL) and N,N-diisopropylethylamine (440 μL, 0.33 g, 2.52 mmoL) under argon gas flow with stirring at room temperature, and the resulting mixture was stirred at room temperature for 3 hours. Subsequently, N,N-dimethylformamide (8 mL) was added thereto under stirring at room temperature, the resulting mixture was stirred at room temperature for 0.5 hour, then the resulting insoluble matters were filtered, and the resulting filtrate was concentrated under reduced pressure. To the resulting residues were added diethyl ether and hexane, the resulting solids were filtered, washed with water four times, then washed with diethyl ether, and dried under reduced pressure to give the title compound (760 mg, yield: 61.34%) as slightly yellow solids.
MS (ESI) m/z 965 $(M+H)^+$

Example 2-3

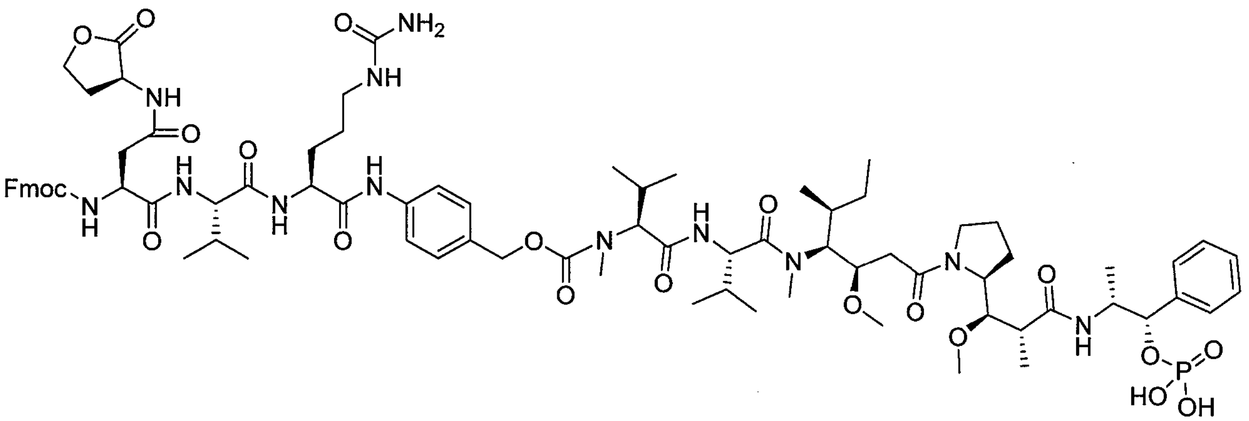

To a solution of the Example 2-2 (76.2 mg, 0.079 mmoL), the U-030 (55.1 mg, 0.069 mmoL), and 1-hydroxy-7-azabenzotriazole (13.0 mg, 0.096 mmoL) in N,N-dimethylformamide (1 mL) in a 10 mL pear-shaped flask was added N,N-diisopropylethylamine (40 μL, 29.68 mg, 0.230 mmoL) at room temperature, and then the resulting mixture was stirred at room temperature for 18 hours. Subsequently, the Example 2-2 (22.3 mg, 0.023 mmoL) was added thereto, and the resulting mixture was stirred at room temperature for 6.5 hours.

After the reaction was completed, the mixture was concentrated under reduced pressure. To the resulting residues was added diethyl ether, and the resulting solids were collected by filtration. To the resulting solids was added acetonitrile/water (1/1 (V/V)) (10 mL), the resulting insoluble matters were removed by filtration, and then freeze-dried to give the title compound (57.4 mg, yield: 51.19%) as white solids.
MS (ESI) m/z 812 $(M+2H)^{2+}$

Example 2-4

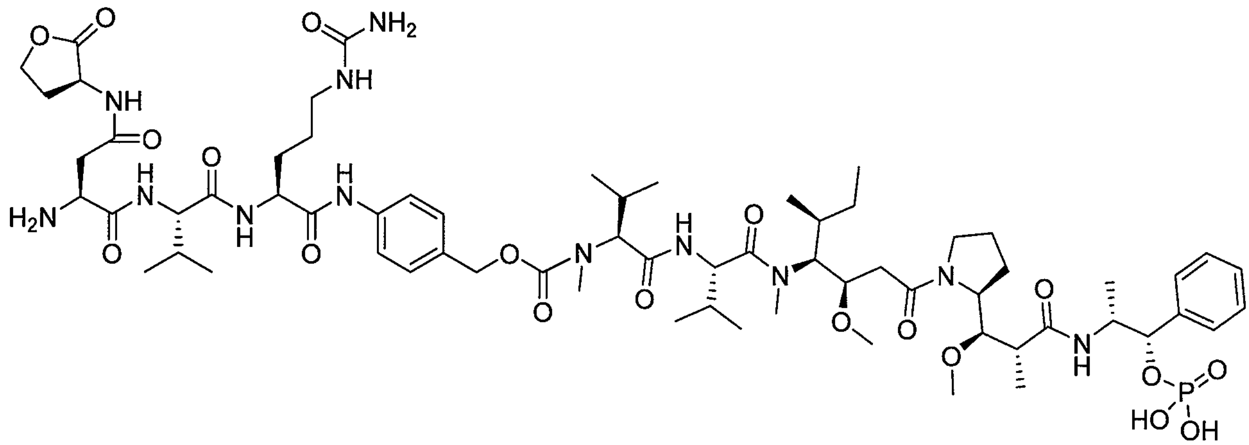

To a solution of the Example 2-3 (57.4 mg, 0.035 mmoL) in N,N-dimethylformamide (2 mL) in a 100 mL pear-shaped flask was added piperidine (11 μL, 9.48 mg, 0.111 mmoL) under argon gas flow with stirring at room temperature, the resulting mixture was stirred at room temperature for 1 hour, then the solvent was removed under reduced pressure, the resulting residues were washed with diethyl ether (10 mL), and the resulting solids were dried under reduced pressure to give the title compound (51.8 mg, yield: 93.23%) as pale yellow solids.
MS (ESI) m/z 1402 $(M+H)^+$

Example 2

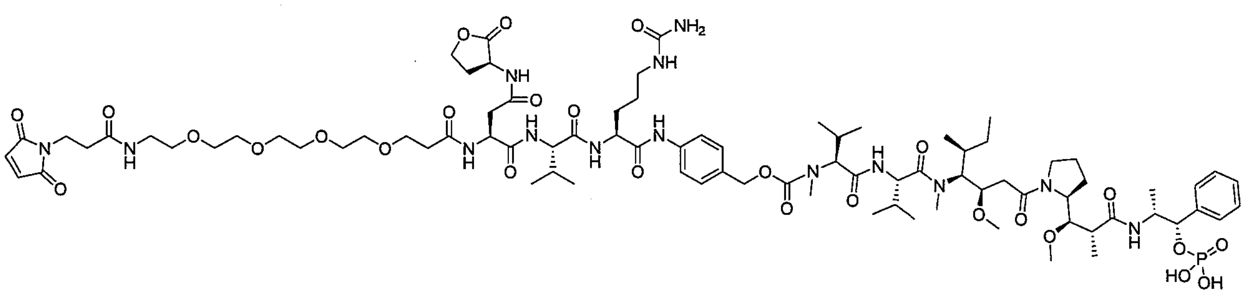

N-succinimidyl 1-maleimide-3-oxo-7,10,13,16-tetraoxa-4-azanonadecanoate (11 mg, 0.021 mmoL) and the Example 2-4 (18 mg, 13 pmoL) instead of the Example 1-7 were used, and reacted in the same manner as the Example 1 to give the title compound (5.2 mg, yield: 22.49%) as white solids. MS (ESI) m/z 901 $(M+2H)^{2+}$

Synthesis of Example 3

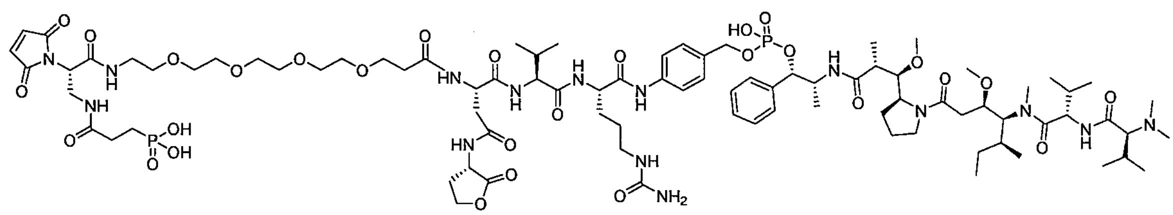

Example 3-1

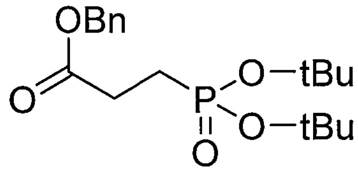

To a solution of di-tert-butyl phosphite (1.94 g, 9.99 mmoL) in acetonitrile (10 mL) in a 100 mL round-bottom flask was added benzyl acrylate (1.68 mL, 1.78 g, 10.98 mmoL) under nitrogen airflow with stirring at room temperature, and the resulting mixture was stirred at 80°C for 6 hours.

After the reaction was completed, the reaction mixture was concentrated under reduced pressure, the resulting residues were subjected to YAMAZEN medium pressure preparative (Silica, L (40 g), hexane / ethyl acetate = 70/30 (V/V) → 50/50 (V/V)), and the fraction comprising the target compound was concentrated under reduced pressure to give the title compound (2.23 g, yield: 62.64%) as a colorless oil.
MS (ESI) m/z 357 $(M+H)^+$

Example 3-2

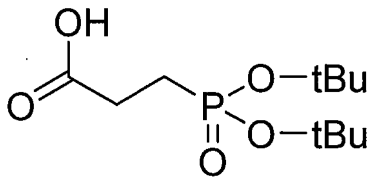

To a solution of the Example 3-1 (2.23 g, 6.26 mmol) in ethanol (22 mL) in a 200 mL round-bottom flask was added 10% by weight of palladium carbon NX-Type (wetted with 50% by weight of water, 1.30 g, 0.611 mmoL) under nitrogen atmosphere with stirring, and the resulting mixture was stirred under hydrogen atmosphere at room temperature for 2 hours.

After the reaction was completed, the reaction mixture was filtered through Celite 545 (trade name), washed with ethanol, and concentrated under reduced pressure to give the title compound (1.68 g, yield: quantitative) as a colorless oil.
MS (ESI) m/z 267 $(M+H)^+$

Example 3-3

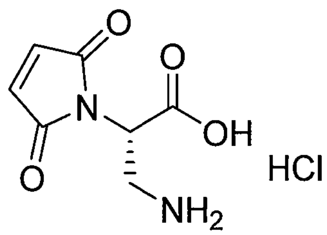

To a solution of (S)-3-((tert-butoxycarbonyl)amino)-2-(2,5-dioxo-2,5-dihydro-1H-pyrrol-1-yl)propanoic acid (i.e., (L)-3-((tert-butoxycarbonyl)amino)-2-(2,5-dioxo-2,5-dihydro-1H-pyrrol-1-yl)propanoic acid) (1.21 g, 4.26 mmoL) (disclosed in WO 2019/195665 pamphlet) in dichloromethane (4 mL) in a 100 mL round-bottom flask was added a 4 M solution of hydrogen chloride in 1,4-dioxane (21.3 mL, 3.11 g, 85 mmol) under argon gas flow with stirring at room temperature, and the resulting mixture was stirred at room temperature for 1 hour.

After the reaction was completed, the solvent was removed under reduced pressure. To the resulting residues was added ethyl acetate, and the resulting solids were filtered. The resulting solids were dissolved in methanol, and the

resulting solution was concentrated under reduced pressure to give the title compound (1.17 g, yield: quantitative) as pale yellow foam.

Example 3-4

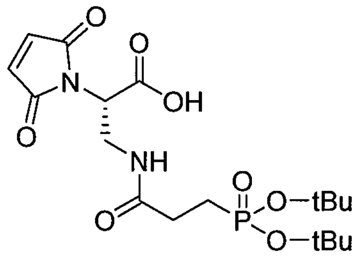

To a solution of the Example 3-2 (0.26 g, 0.976 mmoL) in N,N-dimethylformamide (3 mL) in a 30 mL cylindrical flask were added triethylamine (0.13 mL, 0.09 g, 0.933 mmoL) and HATU (0.37 g, 0.973 mmoL) under argon gas flow with stirring at room temperature, and the resulting mixture was stirred at room temperature for 1 hour to prepare a Solution A.

To a solution of the Example 3-3 (0.21 g, 0.952 mmoL) in N,N-dimethylformamide (3 mL) in a 20 mL cylindrical flask was added triethylamine (0.065 mL, 0.045 g, 0.466 mmoL) under argon gas flow with stirring at room temperature, and the resulting mixture was stirred at room temperature for 10 minutes.

To the above 20 mL cylindrical flask was added the Solution A at room temperature, and the resulting mixture was stirred at room temperature for 6 hours.

After the reaction was completed, to the reaction solution was added water, the resulting solution was filtered through a membrane filter, then the resulting solution was subjected to preparative HPLC chromatography under the following conditions, and the fraction comprising the target compound was freeze-dried to give the title compound (143.9 mg, yield: 34.96%) as colorless solids.
MS (ESI) m/z 433 $(M+H)^+$
Column: Waters XBridge Prep C18 5 μm ODB 19*150 mm

Flow rate: 17 mL/min.
Elution solvent: 0.1% aqueous solution of formic acid (Solution A) - acetonitrile (Solution B)
Gradient (Solution B): 30% (0.00 min.) → 36% (5.00 min.)

Example 3-5

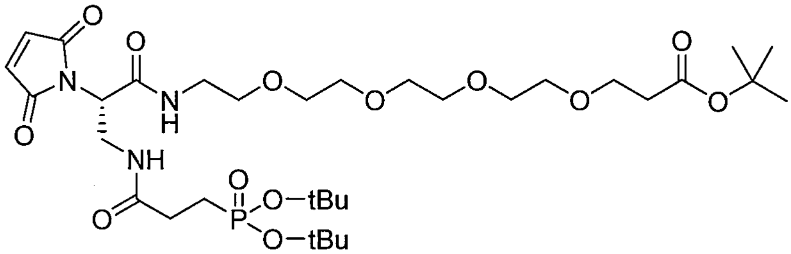

To a solution of the Example 3-4 (143.8 mg, 0.333 mmoL) in N,N-dimethylformamide (3 mL) in a 30 mL pear-shaped flask were added triethylamine (56 μL, 40.66 mg, 0.402 mmoL) and HATU (152.5 mg, 0.401 mmoL) under argon gas flow with stirring at room temperature, and the resulting mixture was stirred at room temperature for 2 minutes. Subsequently, a solution of tert-butyl 1-amino-3,6,9,12-tetraoxapentadecan-15-oate (112.5 mg, 0.350 mmoL) in N,N-dimethylformamide (0.5 mL) was added thereto at room temperature, and the resulting mixture was stirred at room temperature for 1 hour.

After the reaction was completed, the reaction solution was subjected to preparative HPLC chromatography under the following conditions, and the fraction comprising the target compound was freeze-dried to give the title compound (143.5 mg, yield: 58.64%) as colorless solids.
MS (ESI) m/z 734 $(M-H)^-$

Column: Waters SunFire Prep C18 5 um ODB 19*150 mm

Flow rate: 17 mL/min.
Elution solvent: 0.1% aqueous solution of formic acid (Solution A) - acetonitrile (Solution B)
Gradient (Solution B): 20% (0.00 min.) → 60% (8.00 min.)

Example 3-6

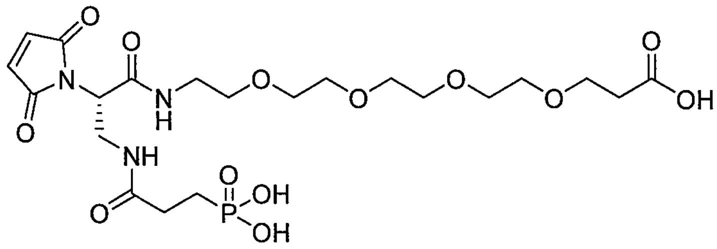

To a solution of the Example 3-5 (63.3 mg, 0.086 mmoL) in dichloromethane (1.5 mL) in a 30 mL pear-shaped flask was added trifluoroacetic acid (150 μL, 223.35 mg, 1.959 mmoL) under nitrogen airflow with stirring at room temperature, and the resulting mixture was stirred at room temperature for 2.5 hours. Subsequently, trifluoroacetic acid (0.5 mL, 744.5 mg, 6.530 mmoL) was added thereto at room temperature, and the resulting mixture was stirred at room temperature for 2 hours.

After the reaction was completed, the reaction solution was concentrated under reduced pressure. To the resulting residues was added acetonitrile/water (1/1 (V/V)) (2 mL), and the resulting mixture was freeze-dried to give the title compound (56.6 mg, yield: quantitative) as white solids.
MS (ESI) m/z 568 $(M+H)^+$

Example 3-7

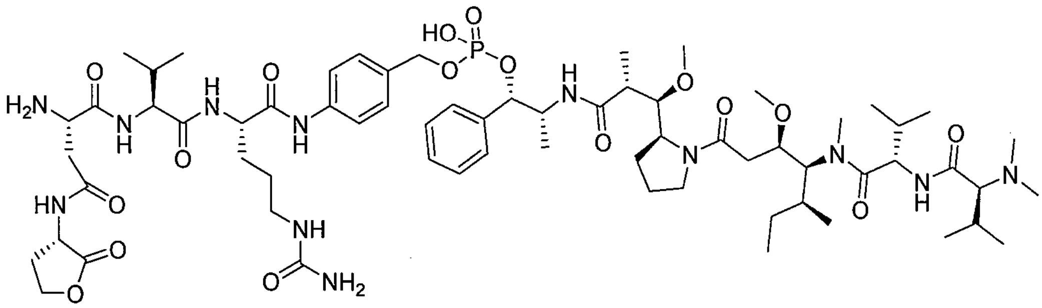

To a solution of the Example 2-1 (340.9 mg, 0.426 mmol) in N,N-dimethylformamide (6 mL) in a 100 mL pear-shaped flask were added 2-cyanoethyl N,N,N',N',-tetraisopropylphosphordiamidite (255 μL, 242.25 mg, 0.804 mmoL) and 1H-tetrazole (56.6 mg, 0.808 mmoL) under argon atmosphere with stirring at room temperature, and the resulting mixture was stirred at room temperature for 15 minutes. Subsequently, triisopropylsilyl trifluoromethanesulfonate (215 μL, 245.1 mg, 0.800 mmoL) was added thereto at room temperature, and the resulting mixture was stirred at room temperature for 30 minutes.

Subsequently, the U-010 (146 mg, 0.199 mmol) and 5-(ethylthio)-1H-tetrazole (109.4 mg, 0.840 mmoL) were added thereto, and the resulting mixture was stirred at room temperature for 1 hour. Subsequently, tert-butyl hydroperoxide (330 μL, 310.2 mg, 2.409 mmol) was added thereto under ice-cooling, and the resulting mixture was stirred at room temperature for 2 hours.

Subsequently, diazabicycloundecene (600 μL, 612 mg, 4.02 mmoL) was added thereto under ice-cooling, and the resulting mixture was stirred at room temperature for 1 hour.

After the reaction was completed, diethyl ether (50 mL) was added thereto, and the resulting supernatant solution was removed. Subsequently, diethyl ether (25 mL) was added thereto, and the resulting supernatant solution was

removed. Subsequently, diethyl ether (25 mL) was added thereto, the resulting supernatant solution was removed, and dried under reduced pressure. The resulting residues were purified under the following recycle preparative conditions, and the fraction comprising the target compound was concentrated under reduced pressure to give the title compound (185.6 mg, yield: 67.8%) as a colorless oil.

MS (ESI) m/z 1372 $(M+H)^+$
Device: LC Forte/R (YMC Co., Ltd.)
Column: XSelect HSS C18 19*150 mm, 5 um

Flow rate: 17 mL/min.
Elution solvent: 0.1% aqueous solution of formic acid (Solution A) - 0.1% solution of formic acid in acetonitrile (Solution B)
Solution composition (Solution B): 50%

Example 3

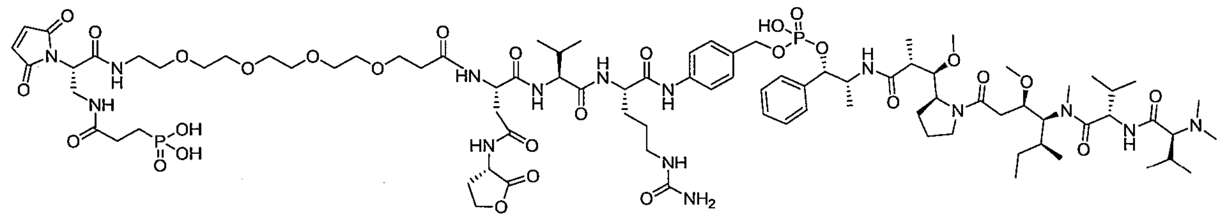

To a solution of the Example 3-6 (11.9 mg, 0.021 mmoL) in N,N-dimethylformamide (600 μL) in a 10 mL cylindrical flask was added triethylamine (9 μL, 6.53 mg, 0.065 mmoL) under argon gas flow with stirring, then HATU (8.7 mg, 0.023 mmoL) was added thereto, and the resulting mixture was stirred at room temperature for 5 minutes. Subsequently, a solution of the Example 3-7 (12.5 mg, 9.11 pmoL) in N,N-dimethylformamide (400 μL) was added thereto at room temperature, and the resulting mixture was stirred at room temperature for 3 hours.

After the reaction was completed, the reaction solution was concentrated under reduced pressure. The resulting residues were subjected to preparative HPLC chromatography under the following conditions, and the fraction comprising the target compound was freeze-dried to give the title compound (1.29 mg, yield: 7.37%) as white solids.
MS (ESI) m/z 961 $(M+2H)^{2+}$

Column: Waters XSELECT HSS T3 Prep 5 μm ODB 19*150 mm
Flow rate: 17 mL/min.
Elution solvent: 0.1% aqueous solution of formic acid (Solution A) - acetonitrile (Solution B)
Gradient (Solution B): 20% (0.00 min.) -> 35% (6.00 min.) → 80% (6.50 min.) → 80% (9.00 min.)

Synthesis of Example 4

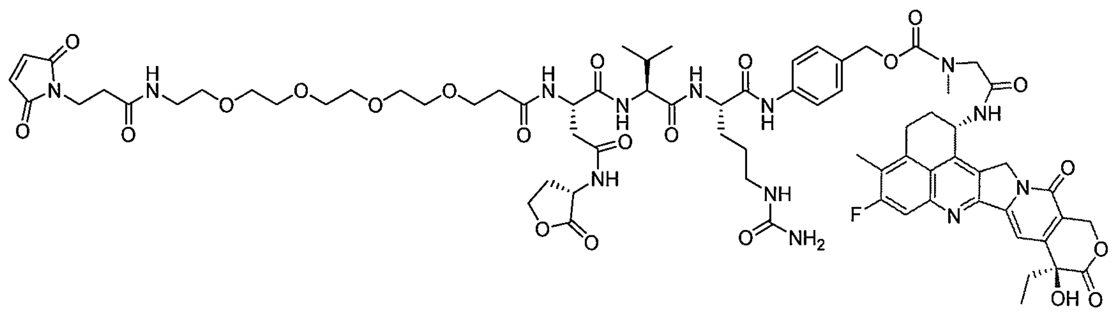

Example 4-1

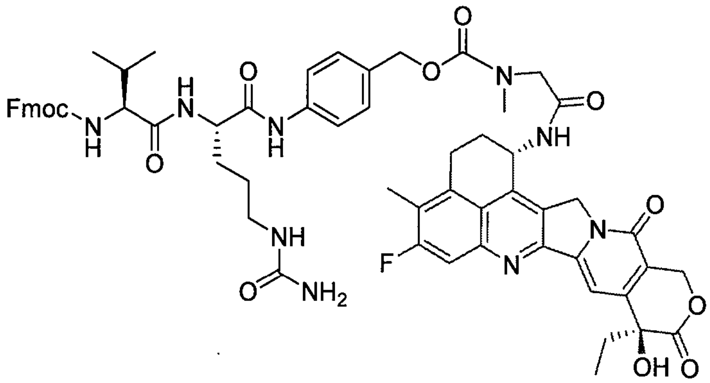

Fmoc-(L)-Val-(L)-Cit-PAB-PNP (manufactured by Angene) (263.5 mg, 0.344 mmoL) and formate of the free base of the U-003 (167.8 mg, 0.304 mmoL) instead of MMAE were reacted in the same manner as the Example 1-4 to give crude products of the title compound (390 mg, yield: quantitative) as light brown solids.
MS (ESI) m/z 1134 $(M+H)^+$

Example 4-2

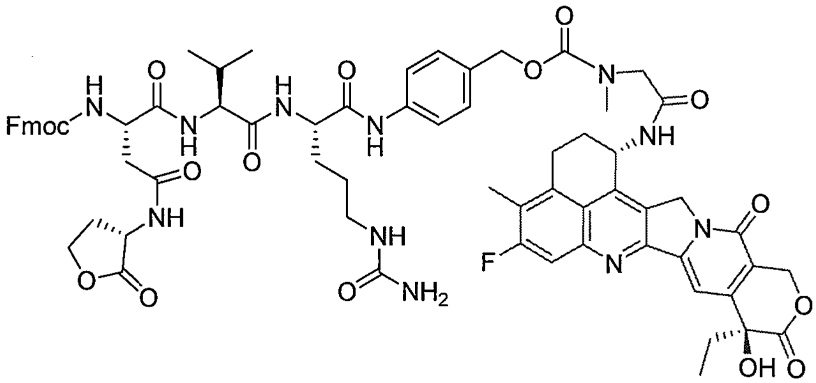

To a solution of the Example 4-1 (103.3 mg, 0.091 mmoL) in N,N-dimethylformamide (2 mL) in a 30 mL cylindrical flask was added piperidine (87 μL, 74.99 mg, 0.881 mmoL) under nitrogen airflow with stirring at room temperature, the resulting mixture was stirred at room temperature for 0.5 hour, and then the solvent was removed under reduced pressure.

To the resulting residues were added the Example 1-2 (115.5 mg, 0.263 mmoL), triethylamine (72 μL, 53.42 mg, 0.528 mmoL), N,N-dimethylformamide (2 mL), and HATU (106.4 mg, 0.280 mmoL) at room temperature, and the resulting mixture was stirred at room temperature for 10 minutes. Subsequently, the Example 1-2 (115.5 mg, 0.263 mmoL), triethylamine (72 μL, 53.42 mg, 0.528 mmoL), and HATU (106.4 mg, 0.280 mmoL) were added thereto at room temperature, and the resulting mixture was stirred at room temperature for 2 hours.

The reaction solution was subjected to preparative HPLC chromatography under the following conditions, and the fraction comprising the target compound was freeze-dried to give the title compound (57.1 mg, yield: 47.05%) as white solids.
MS (ESI) m/z 1333 $(M+H)^+$

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) → 80% (10.00 min.)

Example 4

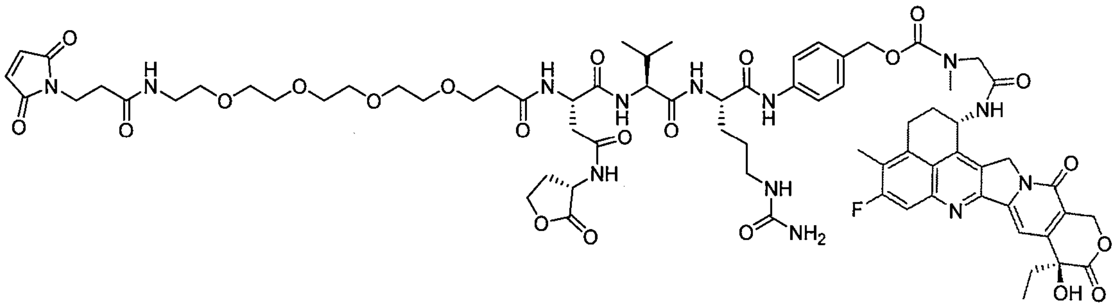

To a solution of the Example 4-2 (10.0 mg, 7.51 pmoL) in N,N-dimethylformamide (0.4 mL) in a 5 mL sample tube was added piperidine (4 μL, 3.44 mg, 0.040 mmoL) under nitrogen airflow with stirring at room temperature, the resulting mixture was stirred at room temperature for 1 hour, and then the solvent was removed under reduced pressure.

To the resulting residues was added N,N-dimethylformamide (0.4 mL), a solution of N-succinimidyl 1-maleimide-3-oxo-7,10,13,16-tetraoxa-4-azanonadecanoate (15.4 mg, 0.030 mmoL) in acetonitrile (0.4 mL) was added thereto with stirring, and the resulting mixture was stirred for 1 hour. Subsequently, triethylamine (4 μL, 2.9 mg, 0.029 mmoL) was added thereto, and the resulting mixture was stirred for 1 hour.

After the reaction was completed, to the reaction solution was added a 50% aqueous solution of acetonitrile (3 mL), the resulting mixture was subjected to preparative HPLC chromatography under the following conditions, and the fraction comprising the target compound was freeze-dried to give the title compound (5.3 mg, yield: 46.81%) as colorless solids.
MS (ESI) m/z 755 $(M+2H)^{2+}$

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) → 60% (10.00 min.)

Synthesis of Example 5

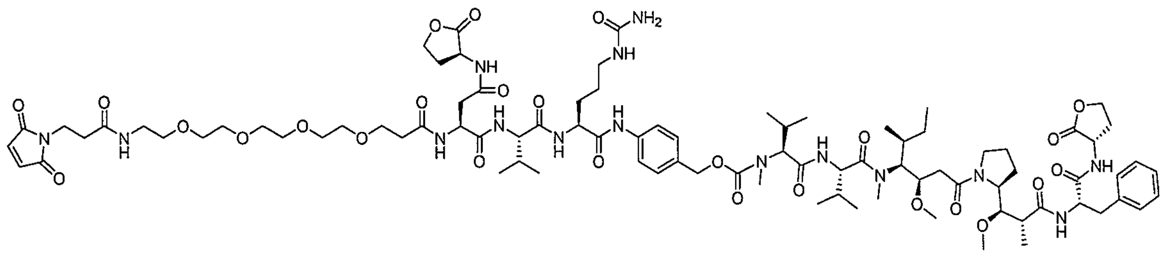

Example 5-1

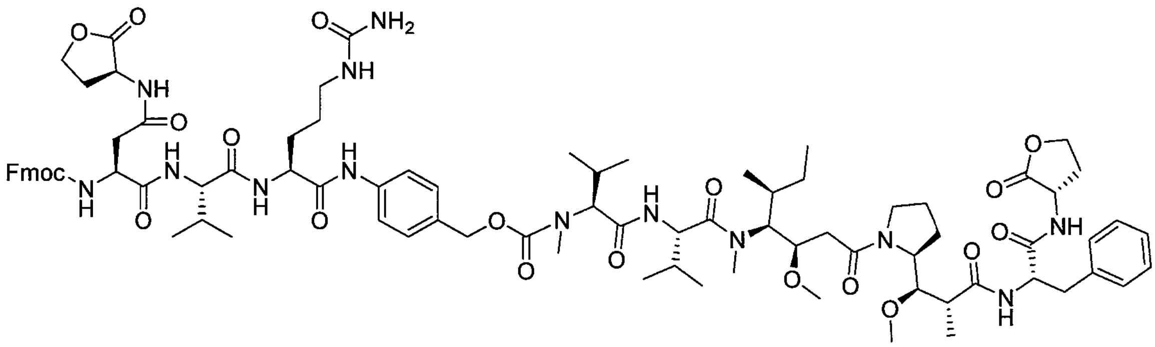

The Example 2-2 (343.0 mg, 0.356 mmoL) and the U-029 (290 mg, 0.356 mmoL) instead of the U-30 were reacted in the same manner as the Example 2-3 to give the title compound (49.7 mg, yield: 8.51%) as a colorless oil. MS (DUIS) m/z 821 $(M+2H)^{2+}$

Example 5

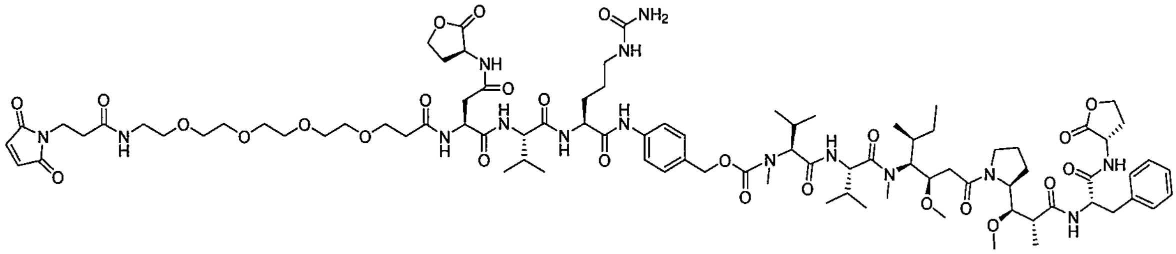

N-succinimidyl 1-maleimide-3-oxo-7,10,13,16-tetraoxa-4-azanonadecanoate (47 mg, 0.092 mmoL) and the Example 5-1 (25 mg, 0.015 mmoL) instead of the Example 4-2 were reacted in the same manner as the Example 4 to give the title compound (21.0 mg, yield: 75.86%) as white solids.
MS (DUIS) m/z 1815 $(M-H)^-$

Synthesis of Example 6

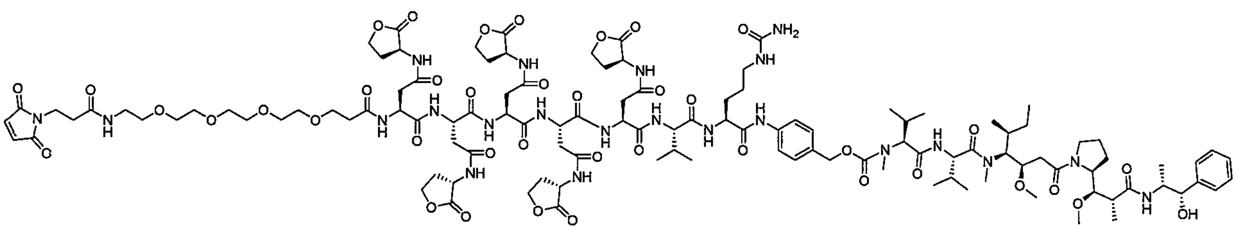

Example 6-1

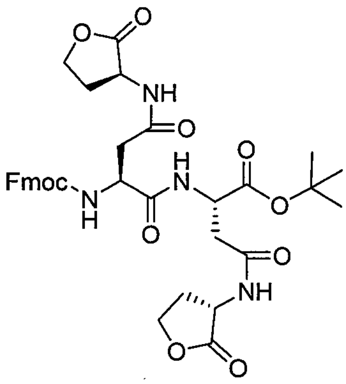

To a solution of the Example 1-1 (0.49 g, 0.991 mmoL) in N,N-dimethylformamide (5 mL) in a 20 mL cylindrical tube was added diazabicycloundecene (75 μL, 0.08 g, 0.498 mmoL) under nitrogen airflow with stirring at room temperature, and the resulting mixture was stirred at room temperature for 1 hour to prepare a Reaction solution A.

Separately, to a solution of the Example 1-2 (0.44 g, 1.004 mmoL) in N,N-dimethylformamide (3 mL) in a 20 mL cylindrical flask were added the above Reaction solution A, then triethylamine (0.28 mL, 0.2 g, 2.009 mmoL), and HATU (0.46 g, 1.210 mmoL) under nitrogen airflow with stirring at room temperature, and the resulting mixture was stirred at room temperature for 1 hour.

After the reaction was completed, to the reaction solution was added tert-butyl methyl ether (8 mL), 1N hydrochloric acid (2.5 mL) and then water (8 mL) were added thereto, and the resulting mixture was stirred at room temperature for 10 minutes. The resulting precipitates were filtered, washed with water, and washed with tert-butyl methyl ether to give the title compound (0.53 g, yield: 77.22%) as colorless solids.
MS (ESI) m/z 693 $(M+H)^+$

Example 6-2

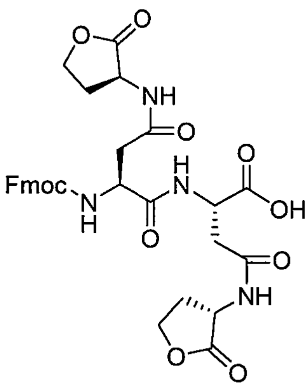

The Example 6-1 (0.21 g, 0.303 mmoL) in a 30 mL cylindrical tube was dissolved in dichloromethane (2 mL), then trifluoroacetic acid (2 mL) was added thereto, and the resulting mixture was stirred at room temperature for 1 hour.

After the reaction was completed, the solvent was distilled away under reduced pressure. To the resulting residues was added acetonitrile (3 mL), the precipitated solids were collected by filtration, and washed with acetonitrile to give the title compound (0.21 g, yield: quantitative) as colorless solids.
MS (ESI) m/z 637 $(M+H)^+$

Example 6-3

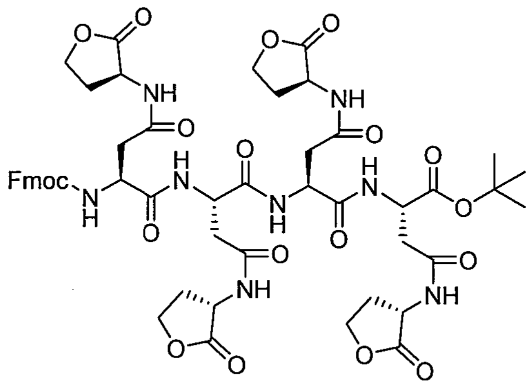

To a solution of the Example 6-1 (0.21 g, 0.303 mmoL) in N,N-dimethylformamide (2 mL) in a 20 mL cylindrical tube was added diazabicycloundecene (23 μL, 0.02 g, 0.153 mmoL) under nitrogen airflow with stirring, and the resulting mixture was stirred at room temperature for 1 hour to prepare a Reaction solution A.

To a solution of the Example 6-2 (0.22 g, 0.306 mmoL) in N,N-dimethylformamide (2 mL) in another 20 mL cylindrical tube were added the Reaction solution A, triethylamine (85 μL, 0.06 g, 0.610 mmoL), and HATU (0.14 g, 0.368 mmoL) under nitrogen airflow with stirring at room temperature, and the resulting mixture was stirred at room temperature for 1 hour.

After the reaction was completed, to the reaction solution were added tert-butyl methyl ether (10 mL), 1N hydrochloric acid (0.76 mL), and water (10 mL), and the resulting mixture was stirred at room temperature for 10 minutes. The precipitated solids were filtered, washed with water and tert-butyl methyl ether, and dried to give the title compound (0.31 g, yield: 93.89%) as colorless solids.
MS (ESI) m/z 1089 $(M+H)^+$

Example 6-4

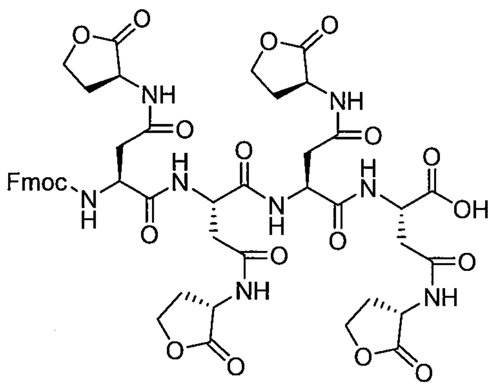

To the Example 6-3 (10.0 mg, 9.18 pmoL) in a 30 mL cylindrical tube was added trifluoroacetic acid (0.1 mL) under nitrogen airflow with stirring at room temperature, and the resulting mixture was stirred at room temperature for 1 hour.

After the reaction was completed, the solvent was concentrated under reduced pressure. To the resulting residues was added acetonitrile (0.3 mL), the precipitated solids were filtered, washed with acetonitrile, and dried to give the title compound (8.4 mg, yield: 88.56%) as colorless solids.
MS (ESI) m/z 1033 $(M+H)^+$

Example 6-5

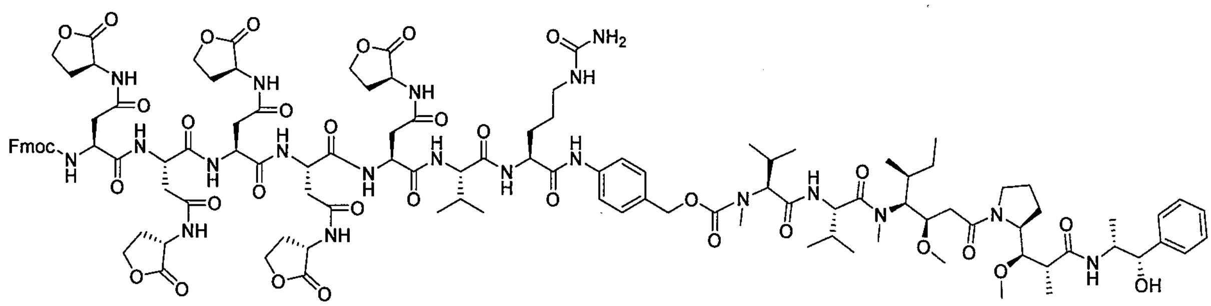

The Example 1-7 (6.9 mg, 5.22 pmoL) instead of the Example 1-5 and the Example 6-4 (6.6 mg, 6.39 pmoL) instead of the Example 1-2 were reacted in the same manner as the Example 1-6 to give the title compound (6.76 mg, yield: 55.41%) as colorless foam.
MS (ESI) m/z 1169 $(M+2H)^{2+}$

Example 6

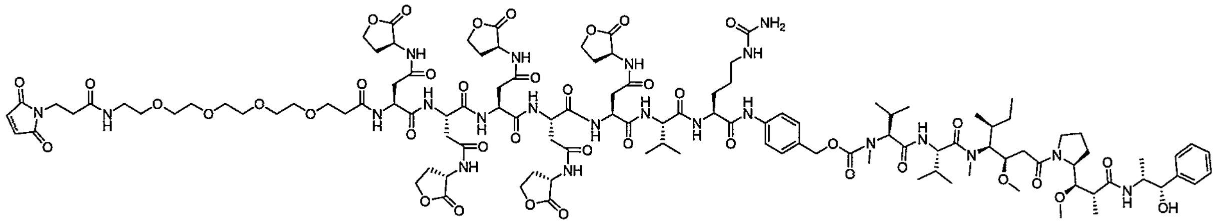

N-succinimidyl 1-maleimide-3-oxo-7,10,13,16-tetraoxa-4-azanonadecanoate (7.2 mg, 0.014 mmoL) and the Example 6-5 (5.3 mg, 2.268 pmoL) instead of the Example 4-2 were reacted in the same manner as the Example 4 to give the title compound (2.62 mg, yield: 45.97%) as colorless solids.
MS (ESI) m/z 1257 $(M+2H)^{2+}$

Synthesis of Example 7

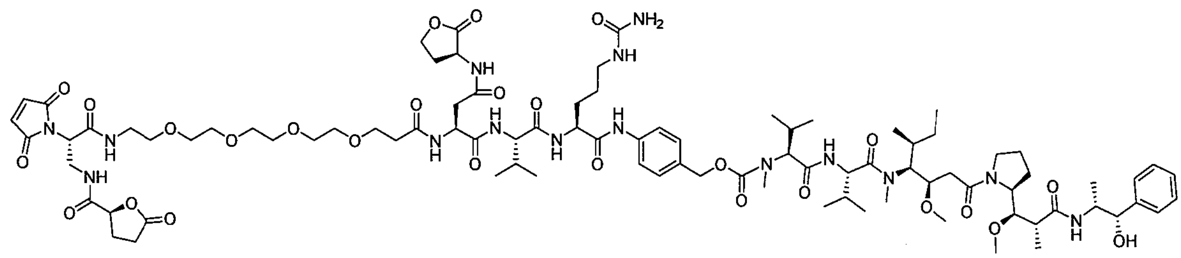

Example 7-1

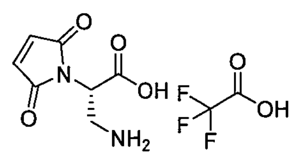

To a solution of (S)-3-((tert-butoxycarbonyl)amino)-2-(2,5-dioxo-2,5-dihydro-1H-pyrrol-1-yl)propanoic acid (i.e., (L)-3-((tert-butoxycarbonyl)amino)-2-(2,5-dioxo-2,5-dihydro-1H-pyrrol-1-yl)propanoic acid) (1.20 g, 4.22 mmoL) (disclosed in WO 2019/195665 pamphlet) in dichloromethane (50 mL) in a 100 mL round-bottom flask was added trifluoroacetic acid (3.24 mL) under nitrogen atmosphere with stirring at room temperature, and the resulting mixture was stirred at room temperature for 4 hours.

After the reaction was completed, the reaction solution was concentrated under reduced pressure, diethyl ether (20 mL) was added thereto, the precipitated solids were filtered, washed with diethyl ether, and dried to give the title compound (0.9734 g, yield: 77.33%) as beige solids.
MS (ESI) m/z 185 $(M+H)^+$

Example 7-2

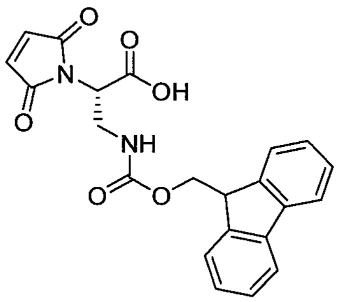

To a solution of the Example 7-1 (591 mg, 1.982 mmoL) and (9H-fluoren-9-yl)methyl carbonochloridate (536 mg, 2.072 mmoL) in tetrahydrofuran (10 mL) in a 100 mL round-bottom flask was added an aqueous solution (5 mL) containing sodium hydrogen carbonate (973 mg, 11.58 mmoL) under air atmosphere with stirring at 0°C, and the resulting mixture was stirred at room temperature for 1 hour.

After the reaction was completed, to the reaction solution was added a 10% by weight aqueous solution of citric acid, and the resulting mixture was subjected to extraction with ethyl acetate. The resulting residues were subjected to YAMAZEN medium pressure preparative (Silica, L (40 g), ethyl acetate / methanol = 100/0 (V/V) → 60/40 (V/V) (Rf = 0.13 (ethyl acetate / methanol = 95/5 (V/V)))), and the fraction comprising the target compound was concentrated under reduced pressure to give the title compound (761 mg, yield: 94.48%) as white foam.
MS (ESI) m/z 407 $(M+H)^+$

Example 7-3

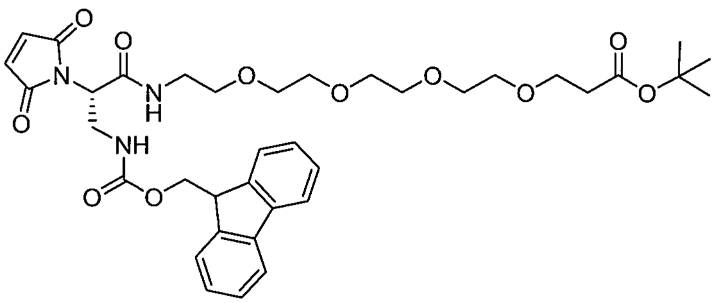

Tert-butyl 1-amino-3,6,9,12-tetraoxapentadecan-15-oate (477 mg, 1.484 mmoL) and the Example 7-2 (692 mg, 1.703 mmoL) instead of the Example 3-4 were reacted in the same manner as the Example 3-5 to give the title compound (918 mg, yield: 87.15%) as an orange oil.
MS (ESI) m/z 710 $(M+H)^+$

Example 7-4

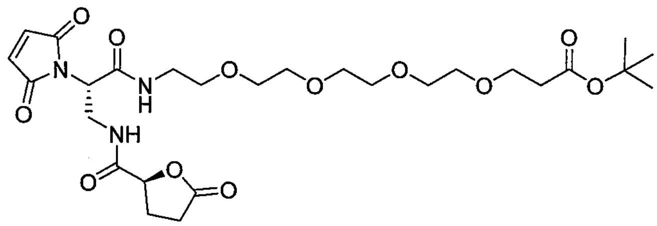

To a solution of the Example 7-3 (780 mg, 1.099 mmoL) in dichloromethane (12 mL) in a 30 mL cylindrical flask was added diazabicycloundecene (75 μL, 75.75 mg, 0.498 mmoL) under argon gas flow with stirring at room temperature, and the resulting mixture was stirred at room temperature for 40 minutes to prepare a Reaction solution A.

To a solution of (S)-5-oxotetrahydrofuran-2-carboxylic acid (211 mg, 1.622 mmoL) and 1-hydroxybenzotriazole (223 mg, 1.650 mmoL) in dichloromethane (12 mL) in another 100 mL round-bottom flask were added the above Reaction solution A and 1-ethyl-3-(3-dimethylaminopropyl)carbodiimide hydrochloride (320 mg, 1.669 mmoL) under argon gas flow with stirring at room temperature, and the resulting mixture was stirred at room temperature for 1 hour.

After the reaction was completed, to the reaction solution was added a 5% by weight aqueous solution of citric acid (10 mL), and the resulting mixture was subjected to extraction with methylene chloride. Subsequently, the reaction mixture was washed with saturated brine (5 mL), dried over anhydrous magnesium sulfate, filtered, and concentrated under reduced pressure.

The resulting residues were subjected to YAMAZEN medium pressure preparative (COOH, M (16 g) (biconnected), dichloroethane/methanol = 100/0 (V/V) → 92/8 (V/V)), and the fraction comprising the target compound was concentrated under reduced pressure to give the title compound (187 mg, yield: 28.38%) as a light brown oil.
MS (ESI) m/z 598 $(M-H)^-$

Example 7-5

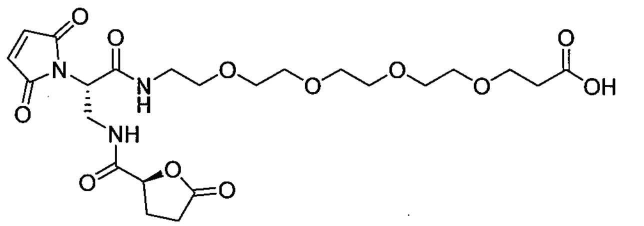

The Example 7-4 (21 mg, 0.035 mmoL) instead of the Example 3-5 was reacted in the same manner as the Example 3-6 to give the title compound (20 mg, yield: quantitative) as a slightly yellow oil.
MS (ESI) m/z 544 $(M+H)^+$

Example 7

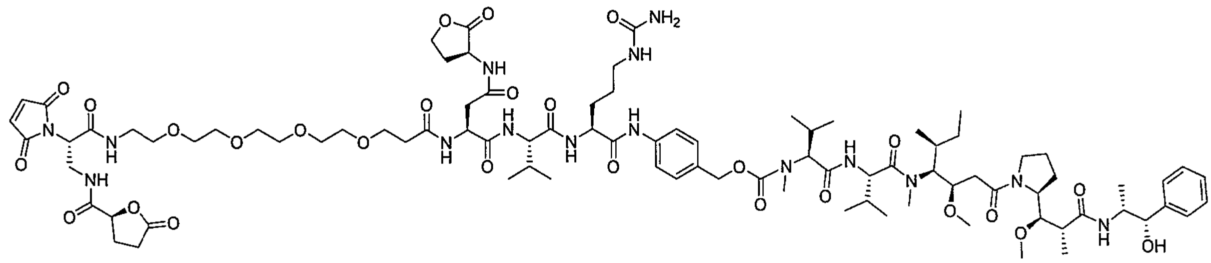

The Example 1-7 (18 mg, 0.014 mmoL) instead of the Example 3-7 and the Example 7-5 (20 mg, 0.037 mmoL) instead of the Example 3-6 were reacted in the same manner as the Example 3 to give the title compound (14.2 mg, yield: 56.44%) as white solids.
MS (ESI) m/z 924 $(M+2H)^{2+}$

Synthesis of Example 8

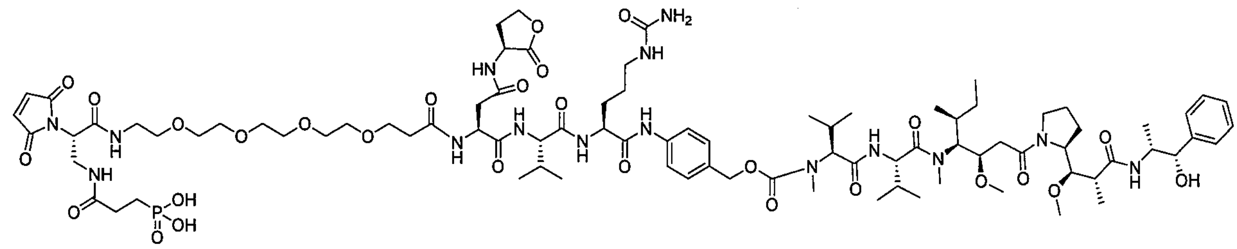

The Example 3-6 (6.10 mg, 10.75 pmoL) and the Example 1-7 (14.33 mg, 10.84 pmoL) instead of the Example 3-7 were reacted in the same manner as the Example 3 to give the title compound (5.91 mg, yield: 29.38%) as white solids. MS (ESI) m/z 936 $(M+2H)^{2+}$

Synthesis of Example 9

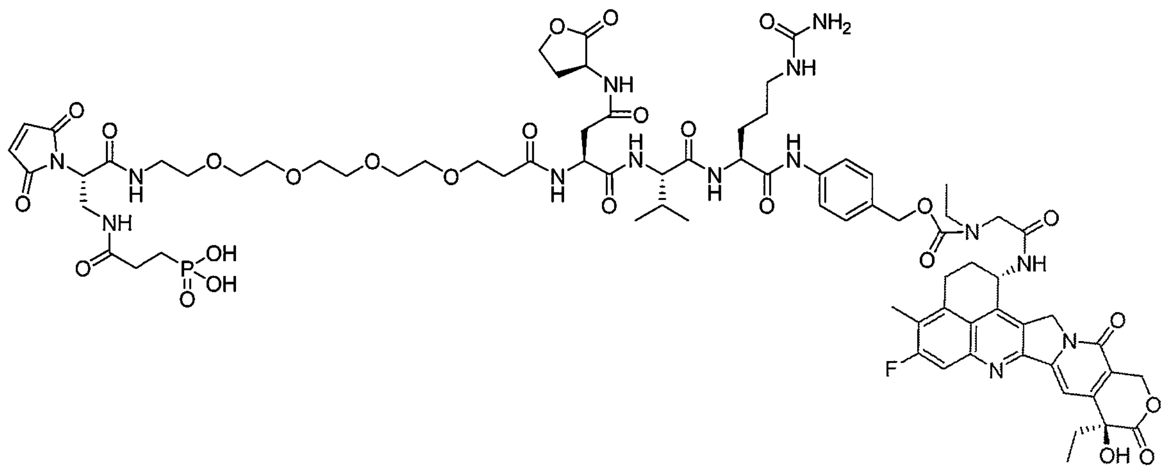

Example 9-1

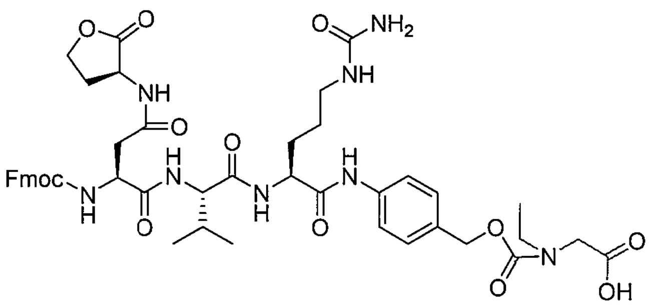

The Example 2-2 (198.0 mg, 0.205 mmoL) and ethylglycine (25.9 mg, 0.251 mmoL) instead of the U-030 were reacted in the same manner as the Example 2-3 to give the title compound (80.5 mg, yield: 42.23%) as slightly yellow solids.
MS (ESI) m/z 929 $(M+H)^+$

Example 9-2

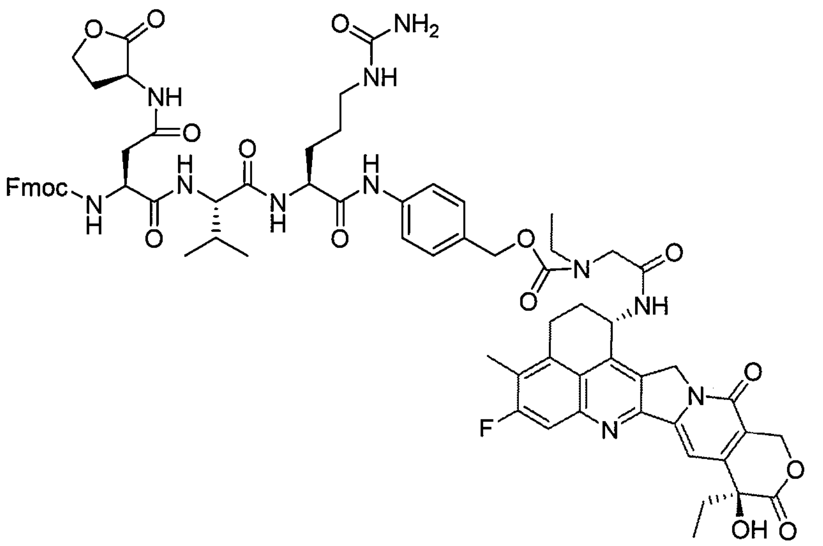

To a solution of exatecan mesylate (65.3 mg, 0.123 mmoL), the Example 9-1 (125 mg, 0.135 mmoL), 1-ethyl-3-(3-dimethylaminopropyl)carbodiimide hydrochloride (46.9 mg, 0.245 mmoL), and 1-hydroxybenzotriazole (18.7 mg, 0.122 mmoL) in N,N-dimethylformamide (0.2 mL) in a 10 mL cylindrical flask was added triethylamine (0.034 mL, 24.68 mg, 0.244 mmoL) under argon atmosphere with stirring at room temperature, and the resulting mixture was stirred at room temperature for 12 hours.

The resulting reaction solution was subjected to preparative HPLC chromatography under the following conditions, the fraction comprising the target compound was concentrated under reduced pressure to distill away acetonitrile, and then the resulting residues were freeze-dried to give the title compound (80.3 mg, yield: 48.55%) as colorless solids.

MS (DUIS) m/z 673 $(M+2H)^{2+}$

Column: Waters SunFire Prep C18 5 um ODB 19*150 mm
Flow rate: 17 mL/min.
Elution solvent: 0.1% aqueous solution of formic acid (Solution A) - acetonitrile (Solution B)
Gradient (Solution B): 50% (0.00 min.) → 62% (6.00 min.)

Example 9

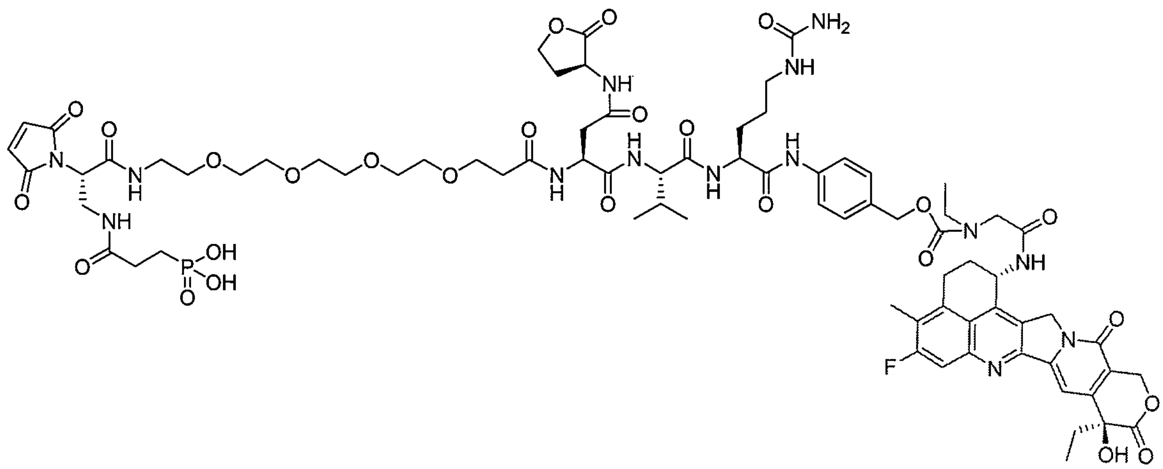

To a solution of the Example 9-2 (12.1 mg, 8.99 pmoL) in N,N-dimethylformamide (0.8 mL) in a 5 mL sample tube was added piperidine (1.74 μL, 1.5 mg, 0.018 mmoL) under airflow with stirring at room temperature, the resulting mixture was stirred at room temperature for 1 hour, and then the solvent was removed under reduced pressure.

To the resulting residues were added N,N-dimethylformamide (0.8 mL), the Example 3-6 (10.1 mg, 0.018 mmoL), HATU (8.2 mg, 0.022 mmoL), and N,N-diisopropylethylamine (11 μE, 8.14 mg, 0.063 mmoL) with stirring, and the resulting mixture was stirred at room temperature for 30 minutes.

After the reaction was completed, to the reaction solution was added a 50% aqueous solution of acetonitrile (5 mL), the resulting mixture was subjected to preparative HPLC chromatography under the following conditions, and the fraction comprising the target compound was freeze-dried to give the title compound (4.7 mg, yield: 31.25%) as white solids.
MS (ESI) m/z 837 $(M+2H)^{2+}$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 20% (0.00 min.) → 20% (2.00 min.) → 70% (8.00 min.)

Synthesis of Example 10

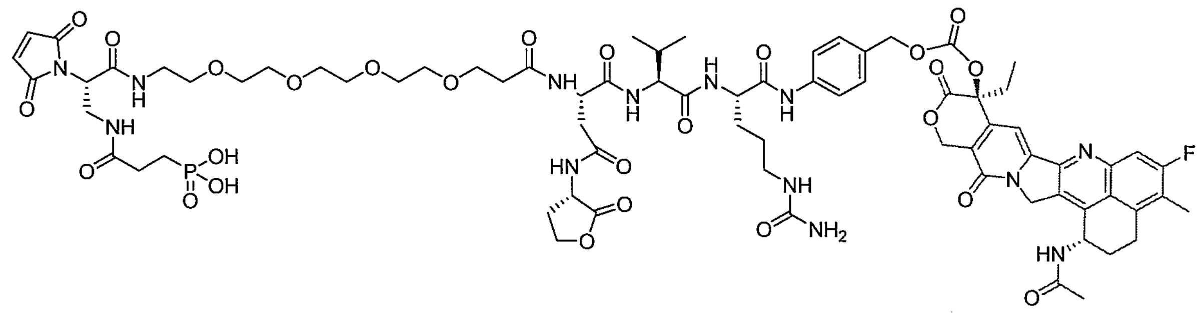

Example 10-1

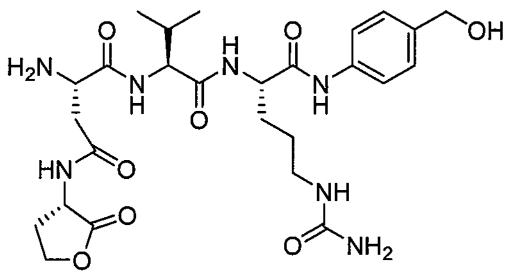

To a solution of the Example 2-1 (4.7 g, 5.88 mmoL) in N,N-dimethylformamide (50 mL) in a 200 mL round-bottom flask was added piperidine (1.45 mL, 1.25 g, 14.64 mmoL) under argon gas flow with stirring at room temperature, and the resulting mixture was stirred at room temperature for 1 hour. Subsequently, piperidine (0.29 mL, 0.25 g, 2.93 mmoL) was added thereto under stirring at room temperature, the resulting mixture was stirred at room temperature for 2 hours, and then the solvent was removed under reduced pressure.

After the reaction was completed, to the resulting residues were added ethyl acetate and diethyl ether, the resulting solids were collected by filtration, and dried under reduced pressure at 50°C for 4 hours to give the title compound (4.98 g, yield: quantitative) as white solids.
MS (ESI) m/z 578 $(M+H)^+$

Example 10-2

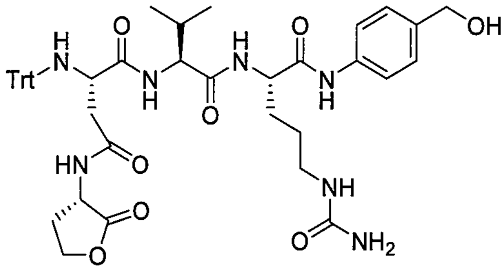

To a solution of the Example 10-1 (4.24 g, 7.34 mmoL) and potassium carbonate anhydrous (1.52 g, 11.00 mmoL) in N,N-dimethylformamide (60 mL) in a 300 mL round-bottom flask was added water (0.3 mL). Trityl chloride (1.03 g, 3.69 mmoL) was added thereto under air atmosphere with stirring at room temperature, and the resulting mixture was stirred at room temperature for 0.5 hour. Subsequently, trityl chloride (1.03 g, 3.69 mmoL) was added thereto under stirring at room temperature, and the resulting mixture was stirred at room temperature for 0.5 hour. Subsequently, trityl chloride (1.01 g, 3.62 mmoL) was added thereto under stirring at room temperature, and the resulting mixture was stirred at room temperature for 0.5 hour. Subsequently, water (0.3 mL) and trityl chloride (0.496 g, 1.779 mmoL) were added thereto under stirring at room temperature, and the resulting mixture was stirred at room temperature for 15 minutes.

To the reaction solution were added ethyl acetate (100 mL), diethyl ether (50 mL), tetrahydrofuran (25 mL), then water (75 mL), and saturated brine (25 mL), the resulting mixture was stirred, and separated. The resulting aqueous layer was concentrated, and then the resulting residues were subjected to extraction with ethyl acetate again. The above resulting organic layers were combined, washed with saturated brine, dried over anhydrous magnesium sulfate, filtered, and concentrated under reduced pressure.

To the resulting residues were added tetrahydrofuran, ethyl acetate, diethyl ether, and hexane, the resulting solids were collected by filtration, and washed with tert-butyl methyl ether to give the title compound (2.69 g, yield: 44.69%) as white solids.
MS (ESI) m/z 820 $(M+H)^+$

Example 10-3

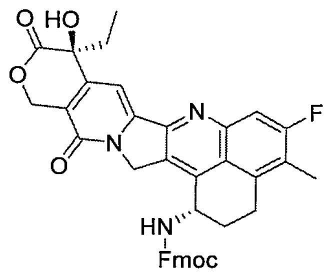

To a solution of exatecan mesylate (1.60 g, 3.01 mmoL) in a mixture of acetonitrile (90 mL) and water (30 mL) in a 300 mL round-bottom flask were added sodium hydrogen carbonate (1.26 g, 15.00 mmoL) and (9H-fluoren-9-yl)methyl carbonochloridate (0.93 g, 3.59 mmoL) under nitrogen atmosphere with stirring at room temperature, and the resulting mixture was stirred at room temperature for 2.5 hours.

After the reaction was completed, to the reaction solution was added water (150 mL), and the resulting mixture was stirred under ice-cooling for 10 minutes. The precipitated solids were separated, and dried under reduced pressure to give the title compound (1.91 g, yield: 96.48%) as slightly yellow solids.
MS (ESI) m/z 658 $(M+H)^+$

Example 10-4

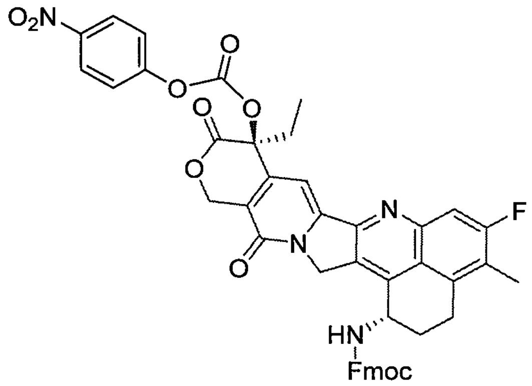

To a solution of the Example 10-3 (6.44 g, 79 mmoL) in dichloromethane (200 mL) in a 500 mL round-bottom flask were added 4-nitrophenyl carbonochloridate (5.92 g, 29.4 mmoL), triethylamine (5.45 mL, 3.96 g, 39.1 mmoL), and 4-dimethylaminopyridine (1.79 g, 14.65 mmoL) under nitrogen atmosphere with stirring at room temperature, and the resulting mixture was stirred at room temperature for 4 hours.

After the reaction was completed, to the reaction solution were added 0.2N hydrochloric acid (120 mL), dichloromethane (150 mL), and water (100 mL), the resulting mixture was filtered through Celite, and the resulting organic layer was separated.

The resulting organic layer was dried over anhydrous magnesium sulfate, filtered, and concentrated under reduced pressure. The resulting residues were subjected to YAMAZEN medium pressure preparative (Silica, L (40 g), hexane / ethyl acetate = 50/50 (V/V) → 0/100 (V/V)), and the fraction comprising the target compound was concentrated under reduced pressure to give the title compound (5.06 g, yield: 62.81%) as brown solids.
MS (ESI) m/z 823 $(M+H)^+$

Example 10-5

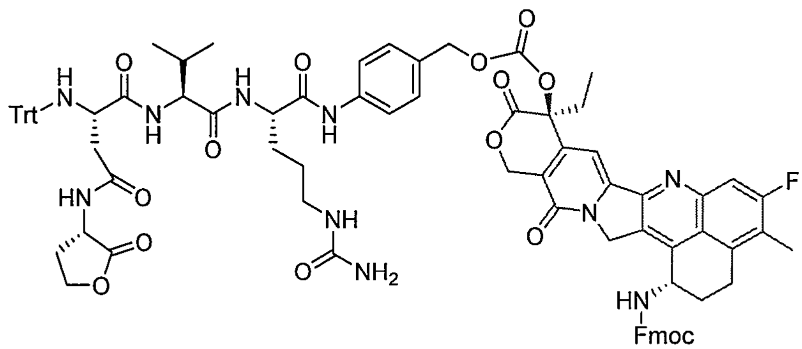

To a solution of the Example 10-4 (3.56 g, 4.33 mmoL) in dichloromethane (108 mL) in a 500 mL round-bottom flask were added the Example 10-2 (3.25 g, 3.96 mmoL), triethylamine (1.22 mL, 0.89 g, 8.75 mmoL), and 4-dimethylaminopyridine (1.02 g, 8.35 mmoL) under argon gas flow with stirring at room temperature, and the resulting mixture was stirred at room temperature for 7.5 hours.

After the reaction was completed, to the reaction solution was added saturated brine, the resulting mixed solution was subjected to extraction with methylene chloride, and the resulting organic layer was concentrated under reduced pressure.

The resulting residues were subjected to YAMAZEN medium pressure preparative (Silica, 3L (135 g), ethyl acetate / methanol = 100/0 (V/V) → 70/30 (V/V) (Rf = 0.22 (ethyl acetate / methanol = 90/10 (V/V)))), and the fraction comprising the target compound was concentrated under reduced pressure to give the title compound (1.86 g, yield: 31.21%) as slightly yellow solids
MS (ESI) m/z 1262 $(M+H-Trt)^+$

Example 10-6

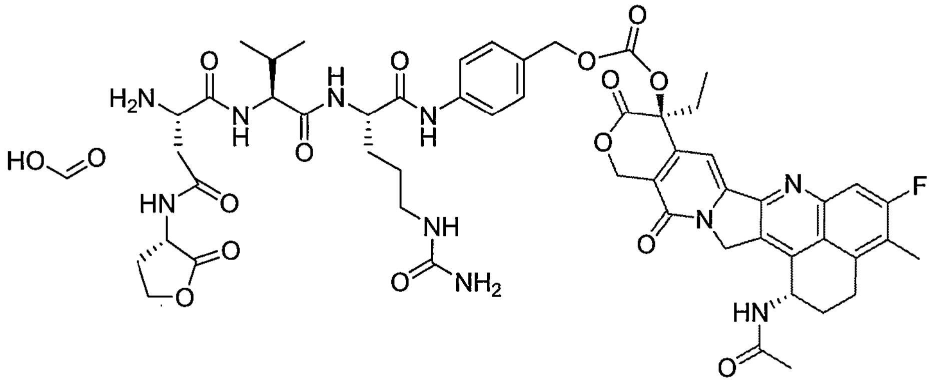

To a solution of the Example 10-5 (15 mg, 9.98 pmoL) in N,N-dimethylformamide (1 mL) in a 10 mL cylindrical flask was added piperidine (10 μL, 8.6 mg, 0.101 mmoL) under air atmosphere with stirring at room temperature, and the resulting mixture was stirred at room temperature for 1 hour.

The reaction solution was concentrated under reduced pressure. To the resulting residues was added dichloromethane (1 mL), triethylamine (4 μL, 2.9 mg, 0.029 mmoL) and then acetic anhydride (3.0 μL, 3.24 mg, 0.032 mmoL) were added thereto under stirring at room temperature, and the resulting mixture was stirred at room temperature for 36 hours.

The solvent was concentrated under reduced pressure, and to the resulting residues was added N,N-dimethylformamide (1 mL). Subsequently, formic acid (0.50 mL, 600 mg, 13.04 mmoL) was added thereto under stirring at room temperature, and the resulting mixture was stirred at room temperature for 1 hour.

The resulting residues were subjected to preparative HPLC chromatography under the following conditions, and the fraction comprising the target compound was freeze-dried to give the title compound (4.9 mg, yield: 43.58%) as colorless solids.
MS (ESI) m/z 1081 $(M+H)^+$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 20% (0.00 min.) → 60% (10.00 min.)

Example 10

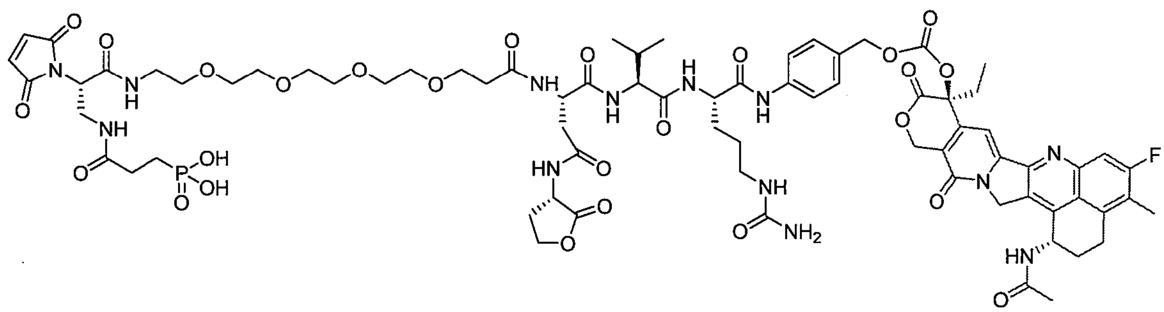

The Example 3-6 (16.1 mg, 0.028 mmoL) and the Example 10-6 (4.9 mg, 4.35 pmoL) instead of the Example 3-7 were reacted in the same manner as the Example 3 to give the title compound (4.1 mg, yield: 57.84%) as white solids. MS (ESI) m/z 816 $(M+2H)^{2+}$

Synthesis of Example 11

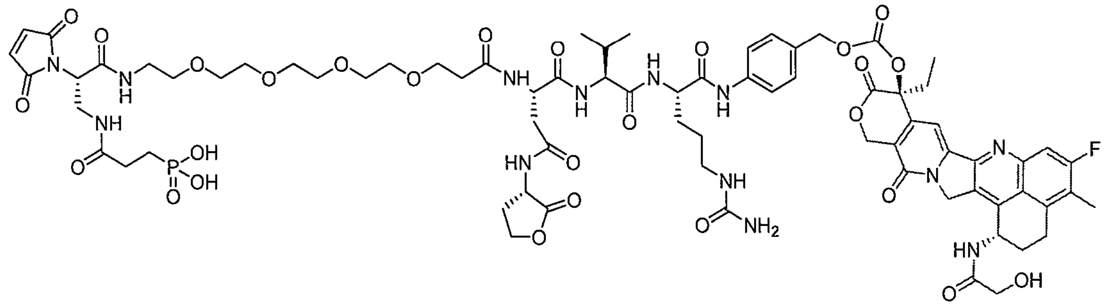

Example 11-1

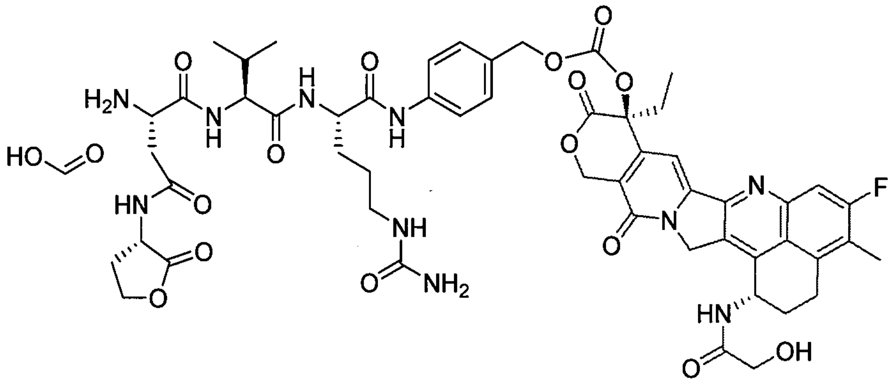

To a solution of the Example 10-5 (66.8 mg, 0.044 mmoL) in N,N-dimethylformamide (2 mL) in a 30 mL pear-shaped flask was added piperidine (22 μL, 18.92 mg, 0.222 mmoL) under air atmosphere with stirring at room temperature, and the resulting mixture was stirred at room temperature for 1 hour.

The reaction solution was concentrated under reduced pressure. To the resulting residues was added N,N-dimethylformamide (2 mL), triethylamine (19 μL, 13.79 mg, 0.136 mmoL), and then 2-hydroxyacetic acid (10.1 mg, 0.133 mmoL) and HATU (50.7 mg, 0.133 mmoL) were added thereto under stirring at room temperature, and the resulting mixture was stirred at room temperature for 3 hours.

Subsequently, formic acid (1 mL, 1200 mg, 26.1 mmoL) was added thereto at room temperature, and the resulting mixture was stirred at room temperature for 1 hour.

The resulting residues were subjected to preparative HPLC chromatography under the following conditions, and the fraction comprising the target compound was freeze-dried to give the title compound (30.6 mg, yield: 60.25%) as light brown solids.
MS (ESI) m/z 1097 $(M+H)^+$

Column: Waters XBridge Prep C18 5 um ODB 19*150 mm
Flow rate: 17 mL/min.
Elution solvent: 0.1% aqueous solution of formic acid (Solution A) - acetonitrile (Solution B)
Gradient (Solution B): 20% (0.00 min.) → 60% (10.00 min.)

Example 11

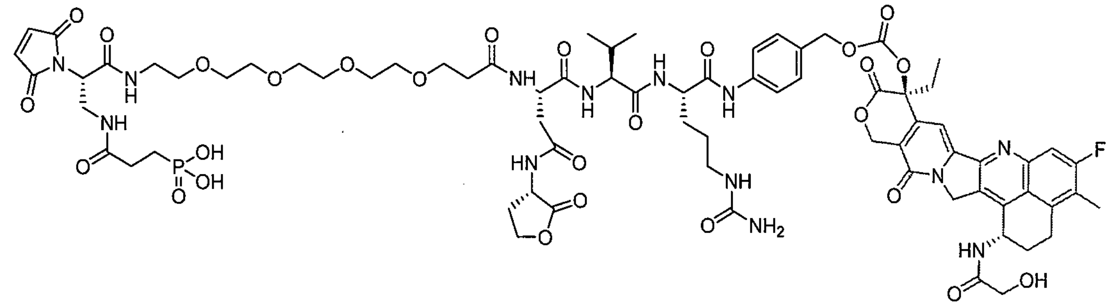

The Example 3-6 (16.5 mg, 0.029 mmoL) and the Example 11-1 (6.6 mg, 5.77 pmoL) instead of the Example 3-7 were reacted in the same manner as the Example 3 to give the title compound (3.6 mg, yield: 37.87%) as white solids. MS (ESI) m/z 824 $(M+2H)^{2+}$

Synthesis of Example 12

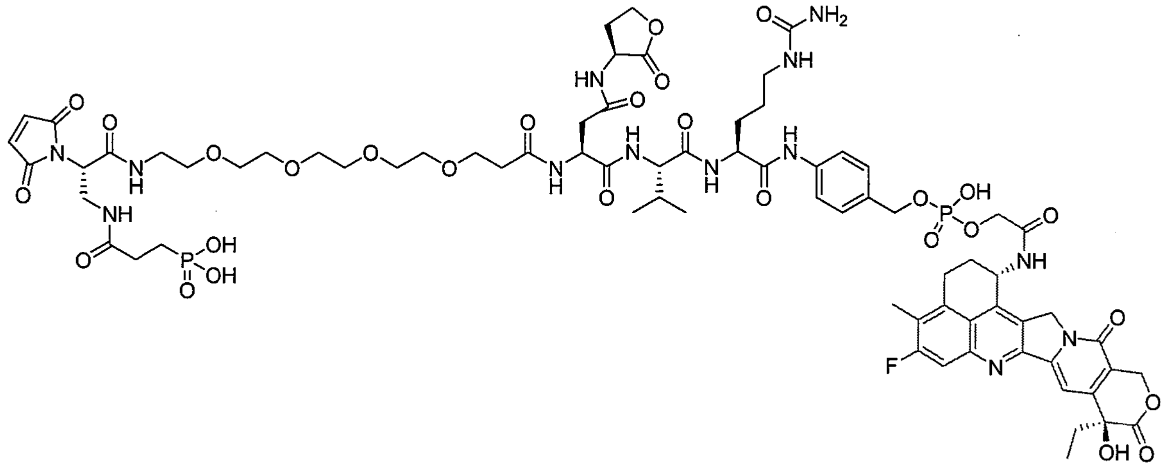

Example 12-1

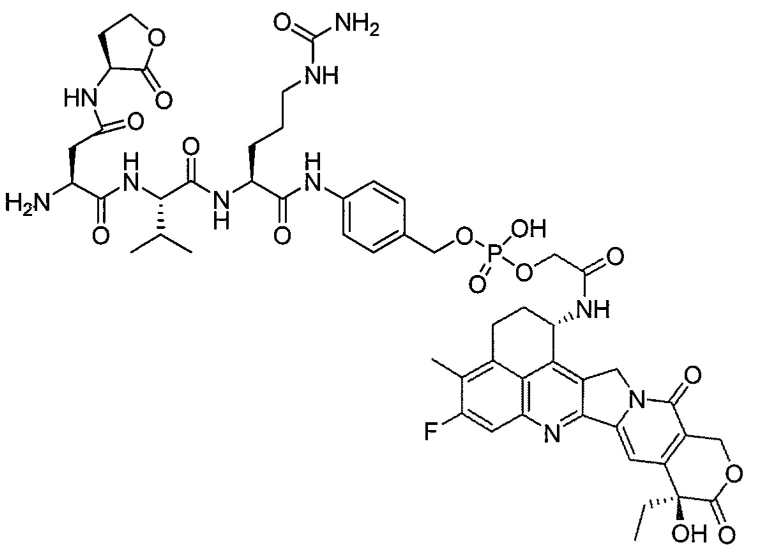

To a solution of the Example 2-1 (162.2 mg, 0.203 mmoL) in N,N-dimethylformamide (3 mL) in a 50 mL pear-shaped flask were added 2-cyanoethyl N,N,N',N'-tetraisopropylphosphordiamidite (130 μL, 123.5 mg, 0.410 mmoL) and 1H-tetrazole (29.8 mg, 0.425 mmoL) under argon atmosphere with stirring at room temperature, and the resulting mixture was stirred at room temperature for 15 minutes. Subsequently, triisopropylsilyl trifluoromethanesulfonate (110 μL, 125.4 mg, 0.409 mmoL) was added thereto at room temperature, and the resulting mixture was stirred at room temperature for 30 minutes.

Subsequently, DXd (100.7 mg, 0.204 mmoL) and 5-(ethylthio)-1H-tetrazole (53.8 mg, 0.413 mmoL) were added thereto, and the resulting mixture was stirred at room temperature for 1 hour. Subsequently, a 70% aqueous solution of tert-butyl hydroperoxide (170 μL, 159.8 mg, 1.241 mmoL) was added thereto at room temperature, and the resulting mixture was stirred at room temperature for 1.6 hours.

Subsequently, diazabicycloundecene (300 μL, 306 mg, 2.01 mmoL) was added thereto under ice-cooling, and the resulting mixture was stirred at room temperature for 1.5 hours.

After the reaction was completed, diethyl ether (30 mL) was added thereto, and the resulting supernatant solution was removed. Subsequently, diethyl ether (15 mL) was added thereto, and the resulting supernatant solution was removed. Subsequently, diethyl ether (15 mL) was added thereto, and the resulting supernatant solution was removed. Subsequently, acetonitrile (20 mL) was added thereto, and the resulting supernatant solution was removed. Subsequently, acetonitrile (10 mL) was added thereto, and the resulting supernatant solution was removed. Subsequently, diethyl ether (20 mL) was added thereto, the resulting supernatant solution was removed, and dried under reduced pressure to give crude products of the title compound. The crude products were purified under the following recycle preparative conditions, and the fraction comprising the target compound was concentrated under reduced pressure to give the title compound (39.8 mg, yield: 17.32%) as beige solids.
MS (ESI) m/z 1133 $(M+H)^+$

Device: LC Forte/R (YMC Co., Ltd.)
Column: XBridge Prep C18 5 μm ODB 19*150 mm
Flow rate: 25 mL/min.
Elution solvent: 0.1% aqueous solution of formic acid (Solution A) - 0.1% solution of formic acid in acetonitrile (Solution B)
Solution composition (Solution B): 40%

Example 12

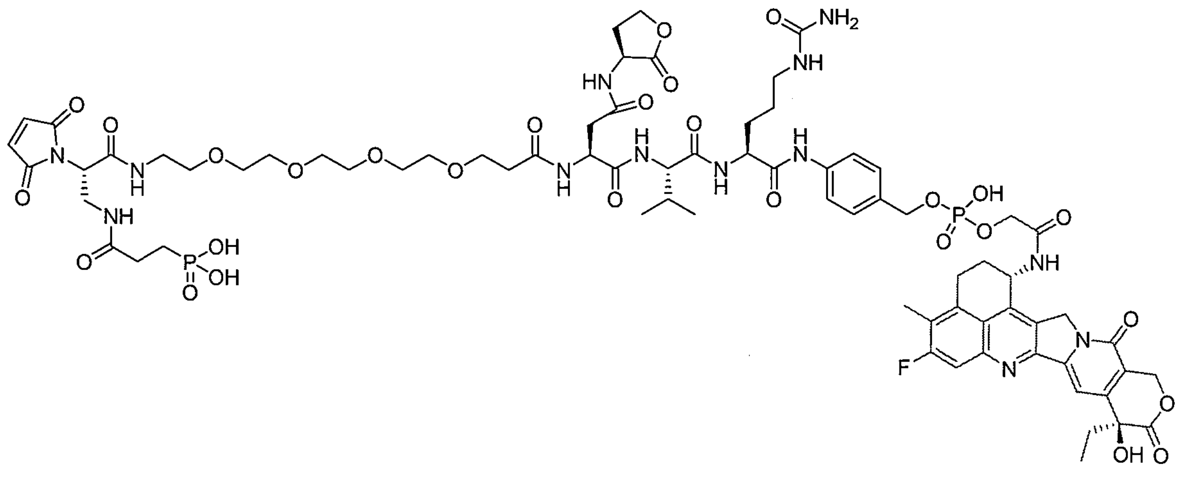

The Example 3-6 (13.2 mg, 0.023 mmoL) and the Example 12-1 (11.5 mg, 10.15 pmoL) instead of the Example 3-7 were reacted in the same manner as the Example 3 to give the title compound (5.78 mg, yield: 33.85%) as white solids.
MS (ESI) m/z 842 $(M+2H)^{2+}$

Synthesis of Example 13

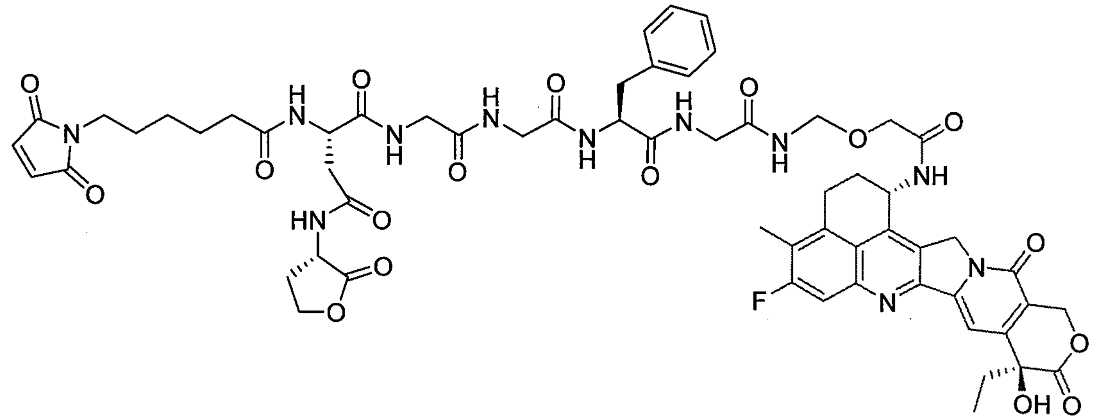

Example 13-1

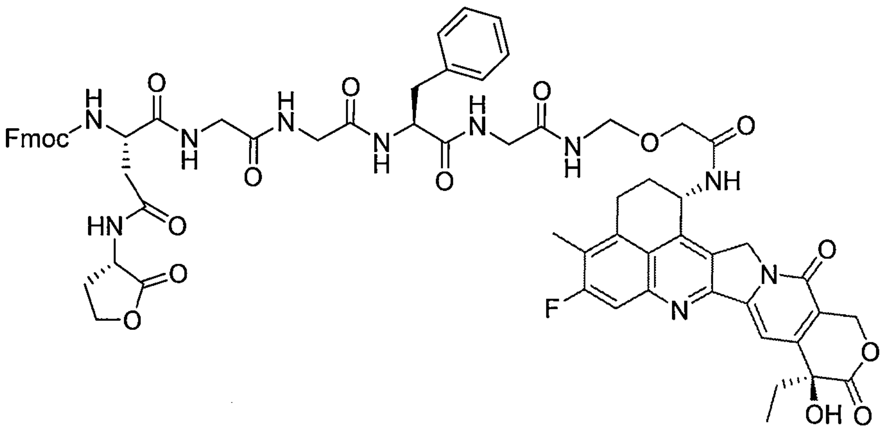

To a solution of N-[(9H-fluoren-9-ylmethoxy)carbonyl]glycylglycyl-L-phenylalanyl-N-[(2-{[(1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl]amino}-2-oxoethoxy)methyl]glycineamide (compound disclosed in JP 6186045 B1) (102.8 mg, 0.040 mmoL, content: 41%) in N,N-dimethylformamide (0.3 mL) was added diazabicycloundecene (9 μL, 9.09 mg, 0.06 mmoL) under argon gas flow with stirring at room temperature, and the resulting mixture was stirred at room temperature for 12 hours. 2N hydrochloric acid (30 μL) was added thereto to prepare a Reaction solution A.

Separately, to a solution of the Example 1-2 (26.1 mg, 0.060 mmoL) in N,N-dimethylformamide (0.2 mL) in a 10 mL cylindrical flask were added triethylamine (8.5 μL, 6.17 mg, 0.061 mmoL) and HATU (22.8 mg, 0.060 mmoL) with stirring at room temperature, the resulting mixture was stirred at room temperature for 30 minutes, added to the above Reaction solution A, and the resulting mixture was stirred at room temperature for 1 hour.

The resulting reaction solution was subjected to preparative HPLC chromatography under the following conditions, the fraction comprising the target compound was concentrated under reduced pressure to distill away acetonitrile, and then the resulting residues were freeze-dried to give the title compound (17.9 mg, yield: 35.8%) as a colorless amorphous.

MS (ESI) m/z 1262 $(M+H)^+$

Column: Waters XBridge Prep C18 5 um ODB 19*150 mm
Flow rate: 17 mL/min.
Elution solvent: 0.1% aqueous solution of formic acid (Solution A) - acetonitrile (Solution B)
Gradient (Solution B): 40% (0.00 min.) → 60% (8.00 min.)

Example 13

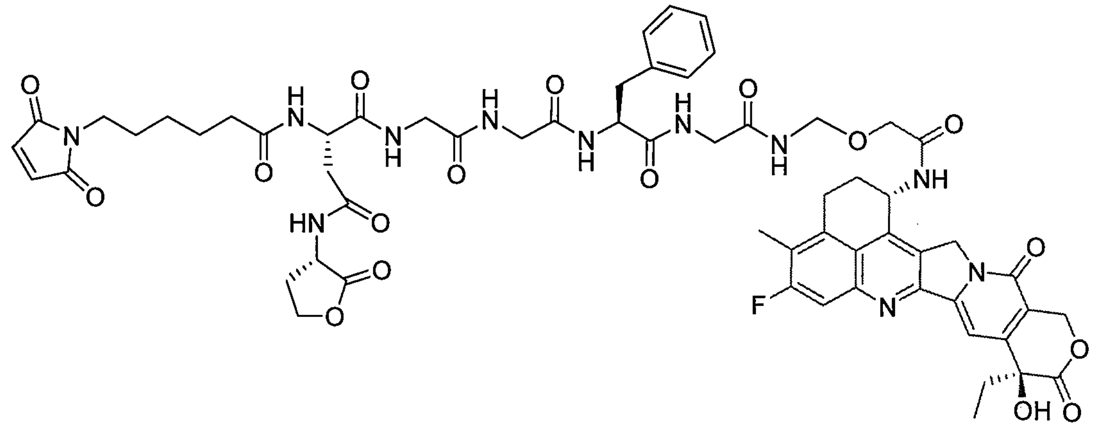

N-succinimidyl 6-maleimidohexanoate (8.75 mg, 0.028 mmoL) instead of N-succinimidyl 1-maleimide-3-oxo-7,10,13,16-tetraoxa-4-azanonadecanoate and the Example 13-1 (17.9 mg, 0.014 mmoL) instead of the Example 4-2 were reacted in the same manner as the Example 4 to give the title compound (3.6 mg, yield: 20.59%) as colorless solids. MS (ESI) m/z 1233 $(M+H)^+$

Synthesis of Example 14

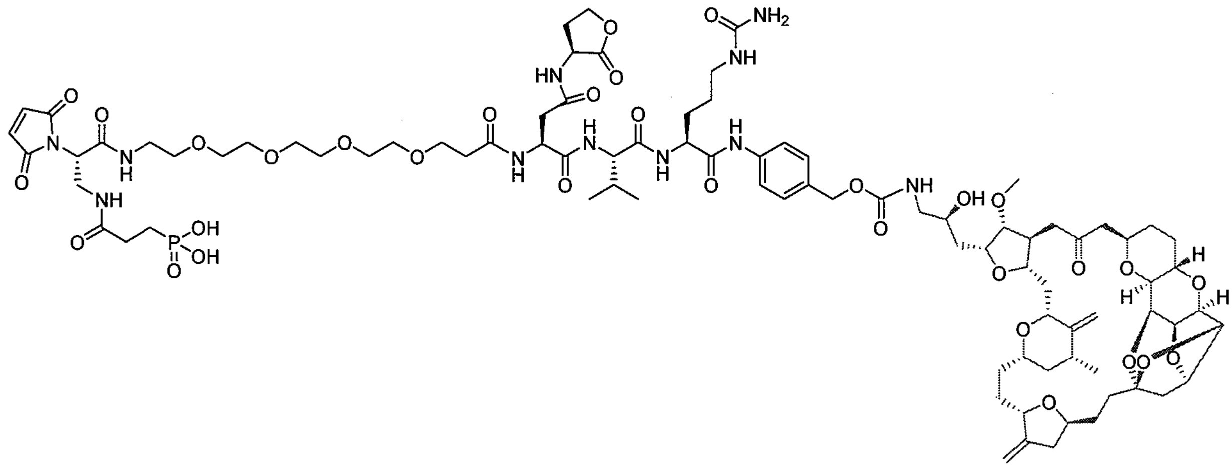

Example 14-1

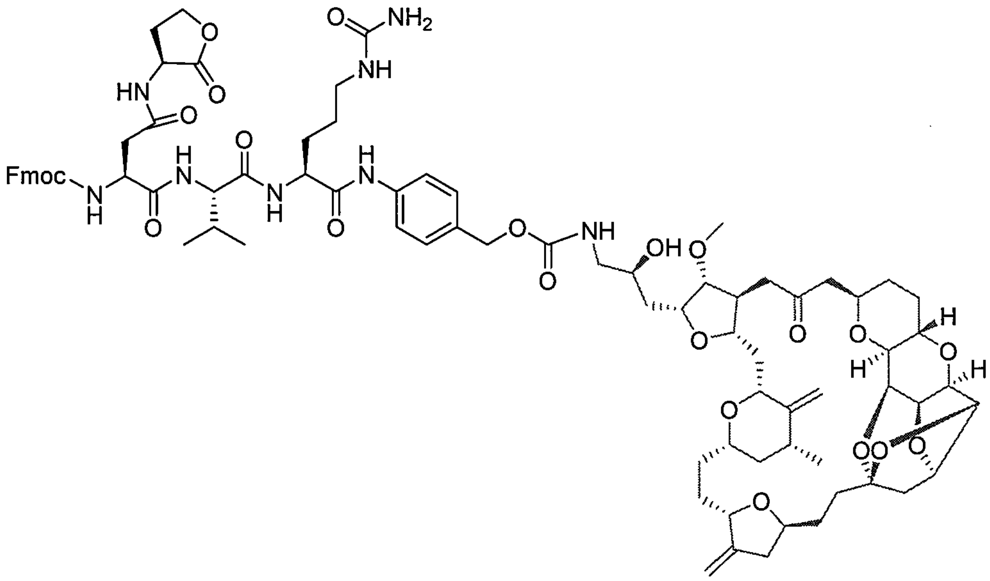

The Example 2-2 (23.1 mg, 0.024 mmoL) and eribulin mesylate (16.4 mg, 0.020 mmoL) instead of the U-030 were reacted in the same manner as the Example 2-3 to give the title compound (21.0 mg, yield: 67.98%) as a colorless amorphous.
MS (DUIS) m/z 1555 $(M+H)^+$

Example 14

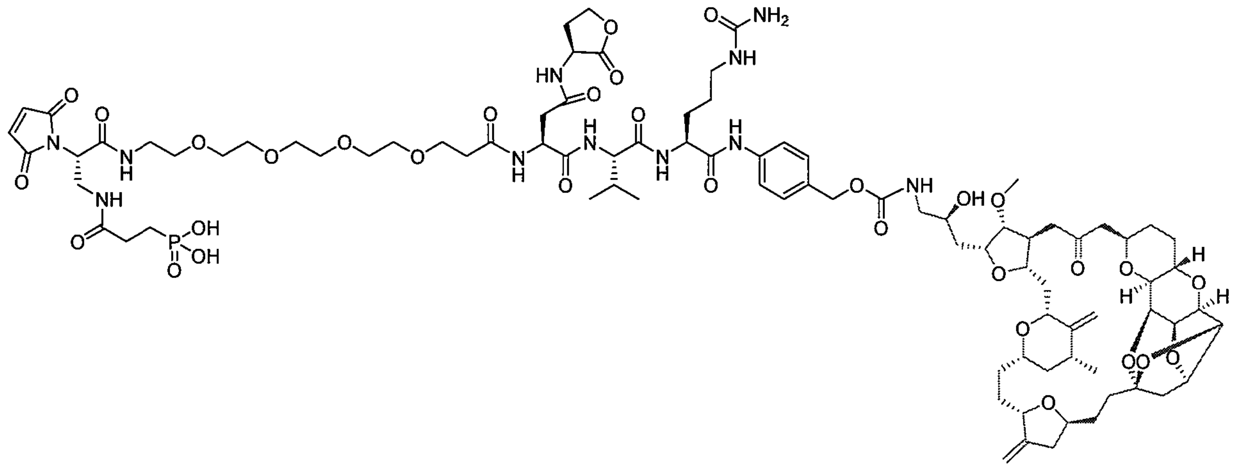

The Example 3-6 (11.7 mg, 0.021 mmoL) and the Example 14-1 (8.3 mg, 5.33 pmoL) instead of the Example 9-2 were reacted in the same manner as the Example 9 to give the title compound (6.9 mg, yield: 68.69%) as pale yellow solids.
MS (ESI) m/z 942 $(M+2H)^{2+}$

Synthesis of Example 15

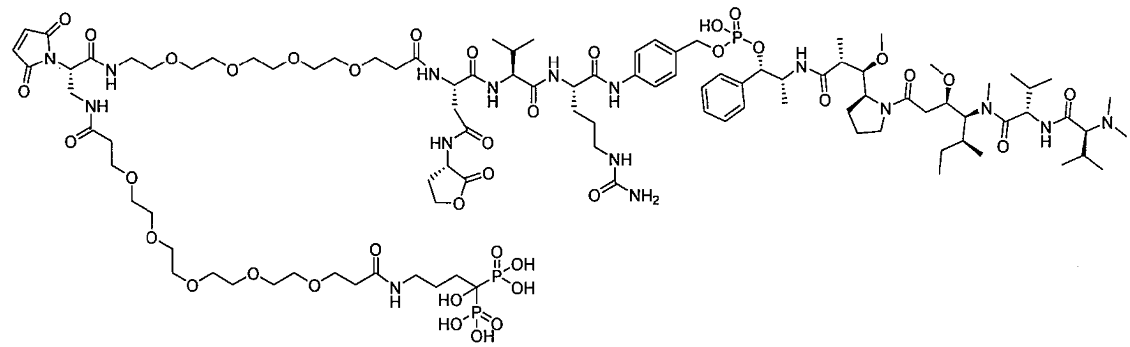

Example 15-1

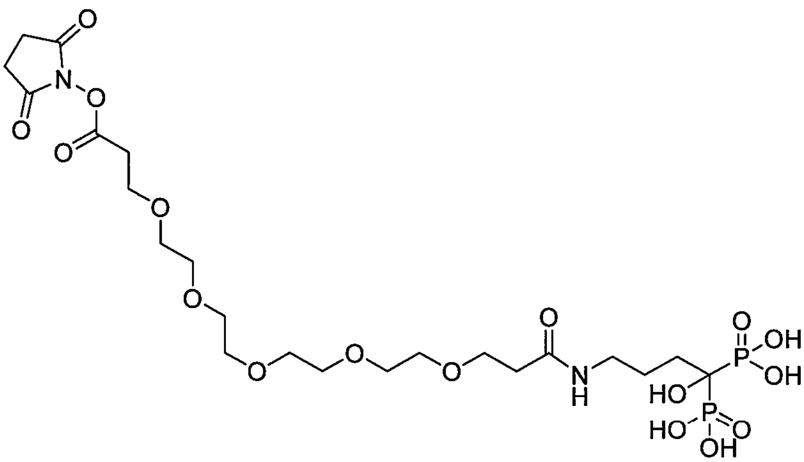

To a solution of bis(2,5-dioxopyrrolidin-1-yl) 4,7,10,13,16-pentaoxanonadecanedioate (1.08 g, 2.028 mmoL) and 1-hydroxypyrrolidine-2,5-dione (46.6 mg, 0.405 mmoL) in N,N-dimethylformamide (16 mL) in a 100 mL cylindrical flask were added dropwise 1-ethyl-3-(3-dimethylaminopropyl)carbodiimide hydrochloride (77.3 mg, 0.403 mmoL) and triethylamine (0.84 mL, 0.61 g, 6.03 mmoL) under argon gas flow with stirring at room temperature, and the resulting mixture was stirred at room temperature for 20 minutes. Subsequently, water (4 mL) was added thereto, then a solution of alendronic acid (0.50 g, 2.007 mmoL) and triethylamine (0.84 mL, 0.61 g, 6.03 mmoL) in a mixture of water (4 mL) / N,N-dimethylformamide (2 mL) was added dropwise thereto under stirring at room temperature, the resulting mixture was stirred at room temperature for 20 minutes, and then 2N hydrochloric acid (3 mL) was added thereto.

The resulting residues were subjected to preparative HPLC chromatography under the following conditions, and the fraction comprising the target compound was freeze-dried to give the title compound (0.31 g, yield: 23.17%) as a colorless oil.

MS (ESI) m/z 667 $(M+H)^+$

Column: Waters XSelect HSS T3 OBD 5 um (19*150 mm)
Flow rate: 17 mL/min.
Elution solvent: 0.1% aqueous solution of formic acid (Solution A) - acetonitrile (Solution B)
Gradient (Solution B): 5% (0.00 min.) $\rightarrow$ 30% (10.00 min.)

Example 15-2

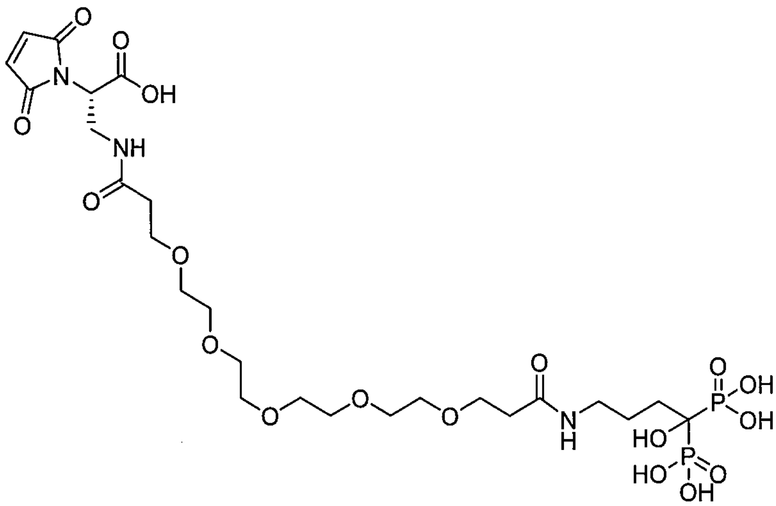

To a solution of the Example 15-1 (0.31 g, 0.465 mmoL) in N,N-dimethylformamide (9 mL) in a 20 mL cylindrical flask were added the Example 3-3 (0.21 g, 0.952 mmoL) and triethylamine (0.65 mL, 0.47 g, 4.66 mmoL) under argon gas flow with stirring at room temperature, the resulting mixture was stirred at room temperature for 1 hour, and then 2N hydrochloric acid (2.3 mL) was added thereto.

The resulting residues were subjected to preparative HPLC chromatography under the following conditions, the fraction comprising the target compound was concentrated under reduced pressure to distill away acetonitrile, and then the resulting residues were freeze-dried to give the title compound (272.7 mg, yield: 79.71%) as a colorless oil.
MS (ESI) m/z 736 $(M+H)^+$

Column: Waters XSelect HSS T3 OBD 5 μm (19*150 mm)
Flow rate: 17 mL/min.
Elution solvent: 0.1% aqueous solution of formic acid (Solution A) - acetonitrile (Solution B)
Gradient (Solution B): 5% (0.00 min.) → 20% (6.00 min.)

Example 15-3

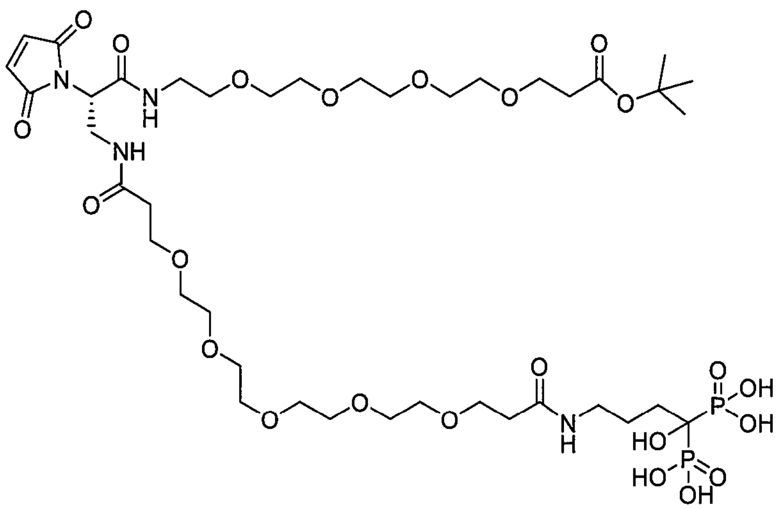

Tert-butyl 1-amino-3,6,9,12-tetraoxapentadecan-15-oate (17.5 mg, 0.054 mmoL) and the Example 15-2 (40.0 mg, 0.054 mmoL) instead of the Example 3-4 were reacted in the same manner as the Example 3-5 to give the title compound (20.4 mg, yield: 36.11%) as a colorless oil.
MS (ESI) m/z 1040 $(M+H)^+$

Example 15-4

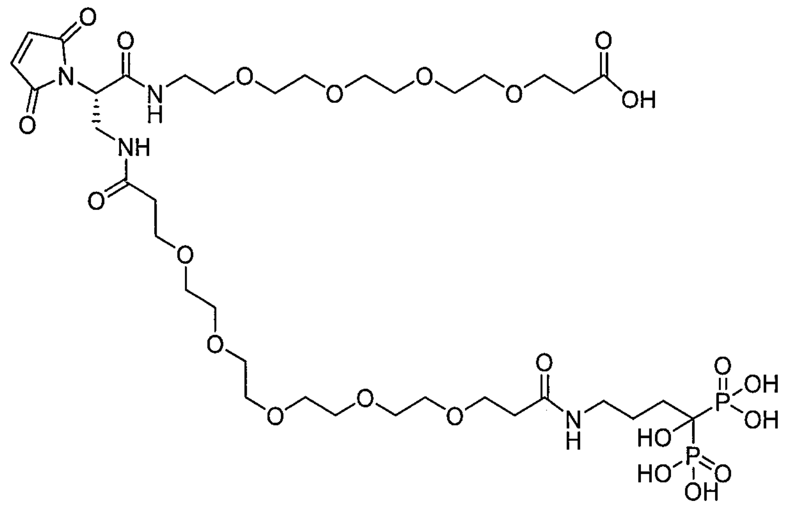

The Example 15-3 (20.3 mg, 0.020 mmoL) instead of the Example 3-5 was reacted in the same manner as the Example 3-6 to give the title compound (9.0 mg, yield: 46.87%) as a colorless oil.
MS (ESI) m/z 983 $(M+H)^+$

Example 15

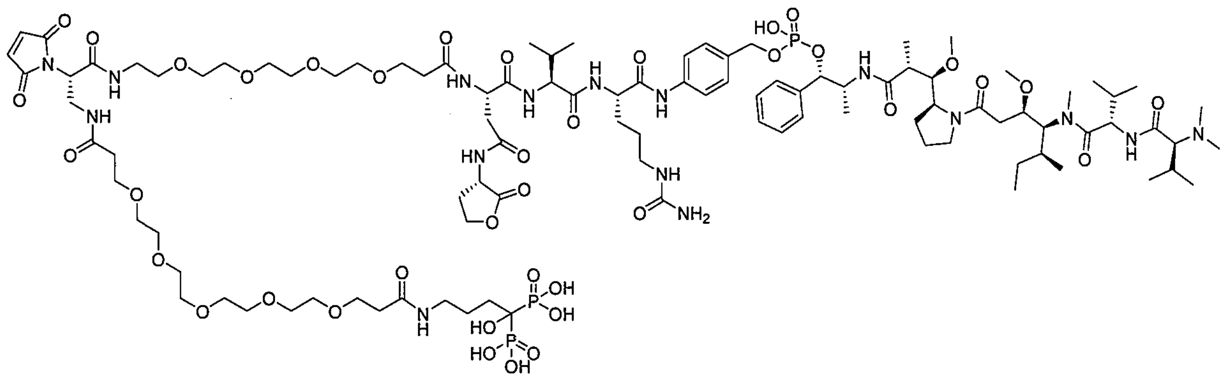

The Example 3-7 (8.4 mg, 6.12 pmoL) and the Example 15-4 (9.0 mg, 9.16 pmoL) instead of the Example 3-6 were reacted in the same manner as the Example 3 to give the title compound (9.97 mg, yield: 69.68%) as colorless solids.
MS (ESI) m/z 1169 $(M+2H)^{2+}$

Synthesis of Example 16

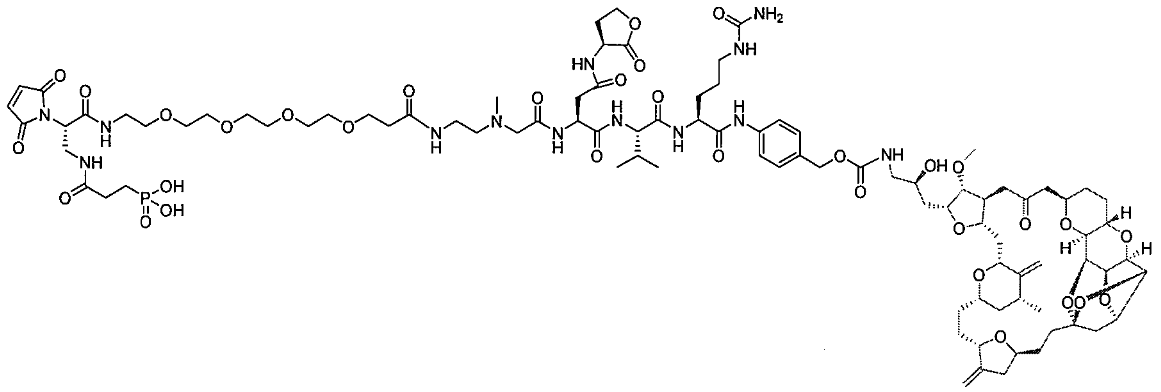

Example 16-1

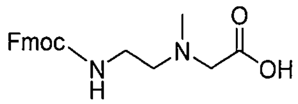

To a solution of (2-((((9H-fluoren-9-yl)methoxy)carbonyl)amino)ethyl)glycine (0.28 g, 0.823 mmoL) in a mixture of methanol (5 mL) / dichloromethane (5 mL) in a 30 mL cylindrical flask were added a 37% aqueous solution of formaldehyde (0.28 mL, 0.31 g, 3.76 mmoL), formic acid (0.26 mL, 0.31 g, 6.78 mmoL), and sodium triacetoxyborohydride (0.26 g, 1.227 mmoL) with stirring at room temperature, and the resulting mixture was stirred at room temperature for 1 hour. Subsequently, sodium triacetoxyborohydride (0.26 g, 1.227 mmoL) was added thereto.

After the reaction was completed, the solvent was concentrated under reduced pressure, then acetonitrile and water were added thereto, and the resulting mixture was filtered through a membrane filter.

The resulting solution was subjected to preparative HPLC chromatography under the following conditions, the fraction comprising the target compound was concentrated under reduced pressure to distill away acetonitrile, and then the resulting residues were freeze-dried to give the title compound (0.15 g, yield: 51.45%) as colorless solids.
MS (ESI) m/z 355 $(M+H)^+$

Column: Waters XBridge Prep C18 5 um ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) → 45% (6.00 min.)

Example 16-2

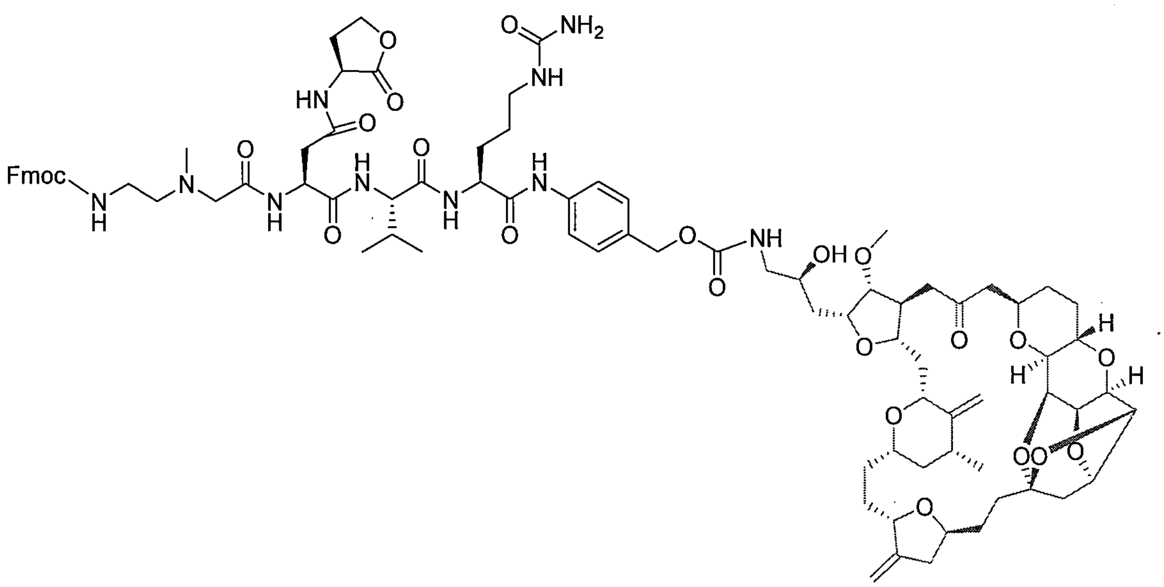

To a solution of the Example 14-1 (7.1 mg, 4.56 pmoL) in N,N-dimethylformamide (0.5 mL) in a 5 mL sample tube was added piperidine (5 μL, 4.3 mg, 0.050 mmoL) under air atmosphere with stirring at room temperature, the resulting mixture was stirred at room temperature for 1 hour, and then concentrated under reduced pressure to prepare Residues A.

To a solution of the Example 16-1 (3.4 mg, 9.59 pmoL) in N,N-dimethylformamide (0.5 mL) in another 5 mL sample tube were added dimethylbenzylamine (1.5 μL, 1.35 mg, 9.98 pmoL) and then HATU (3.4 mg, 8.94 pmoL) under argon atmosphere with stirring at room temperature, and the resulting mixture was stirred at room temperature for 1 hour.

The above reaction solution was added to the Residues A, and the resulting mixture was stirred at room temperature for 1 hour. Subsequently, HATU (1 mg, 2.63 pmoL) was added thereto.

The resulting reaction solution was subjected to preparative HPLC chromatography under the following conditions, and the fraction comprising the target compound was freeze-dried to give the title compound (4.5 mg, yield: 59.05%) as colorless solids.
MS (ESI) m/z 836 $(M+2H)^{2+}$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) → 80% (8.00 min.)

Example 16

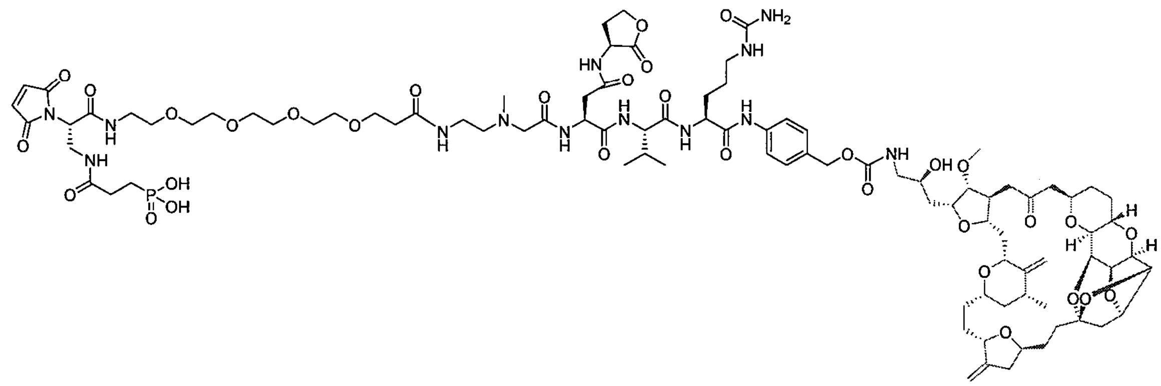

The Example 3-6 (6.0 mg, 10.57 pmoL) and the Example 16-2 (4.5 mg, 2.69 pmoL) instead of the Example 9-2 were reacted in the same manner as the Example 9 to give the title compound (2.40 mg, yield: 44.59%) as colorless solids.
MS (ESI) m/z 1000 $(M+2H)^{2+}$

Synthesis of Example 17

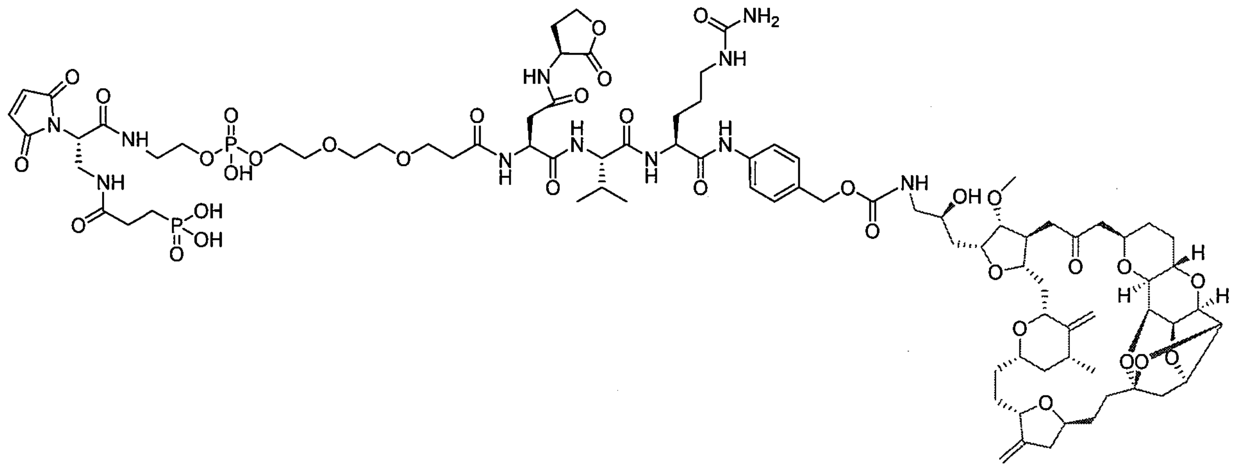

Example 17-1

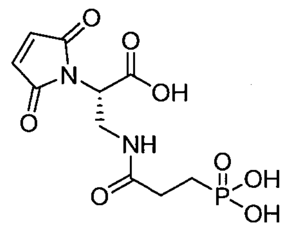

To a solution of the Example 3-2 (0.54 g, 1.470 mmoL) and triethylamine (0.41 mL, 0.3 g, 2.94 mmoL) in N,N-dimethylformamide (5 mL) in a 30 mL cylindrical flask was added HATU (0.59 g, 1.552 mmoL) under argon gas flow with stirring at room temperature, and the resulting mixture was stirred at room temperature for 0.5 hour to prepare a Solution A.

To a solution of the Example 3-3 (0.32 g, 1.451 mmoL) in N,N-dimethylformamide (5 mL) in a 20 mL cylindrical flask was added the Solution A under argon gas flow with stirring at room temperature, and the resulting mixture was stirred at room temperature for 6 hours.

After the reaction was completed, to the reaction solution was added water, the resulting solution was filtered through a membrane filter, then the resulting solution was subjected to preparative HPLC chromatography under the following conditions, and the fraction comprising the target compound was freeze-dried to give the Example 3-4 (62.5 mg) as a colorless oil.
MS (ESI) m/z 433 $(M+H)^+$

Column: Waters XBridge Prep C18 5 $\mu$m ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) $\rightarrow$ 36% (5.00 min.)

The resulting Example 3-4 was left to stand at room temperature for one month to give the title compound as colorless solids.
MS (ESI) m/z 321 $(M+H)^+$

Example 17-2

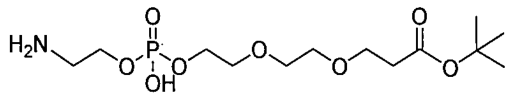

To a solution of tert-butyl 3-(2-(2-hydroxyethoxy)ethoxy)propanoate (0.21 g, 0.896 mmoL) in N,N-dimethylformamide (5 mL) in a 10 mL pear-shaped flask were sequentially added 2-cyanoethyl N,N,N',N'-tetraisopropylphosphordiamidite (0.32 mL, 0.3 g, 1.009 mmoL) and 1H-tetrazole (70.0 mg, 0.999 mmoL), and the resulting mixture was stirred at room temperature for 15 minutes.

Subsequently, (9H-fluoren-9-yl)methyl(2-hydroxyethyl)carbamate (0.31 g, 1.094 mmoL) was added thereto, then 5-(ethylthio)-1H-tetrazole (0.18 g, 1.383 mmoL) was added thereto, and the resulting mixture was stirred at room temperature for 20 minutes.

Subsequently, a 70% aqueous solution of tert-butyl hydroperoxide (0.75 mL, 0.71 g, 5.48 mmoL) was added thereto at room temperature, and then the resulting mixture was stirred at room temperature for 0.5 hour. Subsequently, diazabicycloundecene (1.09 mL, 1.11 g, 7.30 mmoL) was added thereto, and the resulting mixture was stirred for 10 minutes. Subsequently, a 50% aqueous solution of acetonitrile (2 mL) was added thereto, and then 6N hydrochloric acid (1.2 mL) was added thereto.

The resulting residues were subjected to preparative HPLC chromatography under the following conditions, and the fraction comprising the target compound was concentrated under reduced pressure to give the title compound (0.12 g, yield: 37.47%) as colorless solids.
MS (ESI) m/z 358 $(M+H)^+$

Column: Waters XSelect HSS T3 OBD 5 um (19*150 mm)
Flow rate: 17 mL/min.
Elution solvent: 0.1% aqueous solution of formic acid (Solution A) - acetonitrile (Solution B)
Gradient (Solution B): 10% (0.00 min.) → 50% (8.00 min.)

Example 17-3

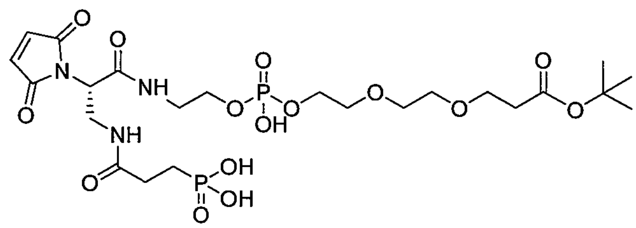

The Example 17-1 (10.4 mg, 0.032 mmoL) instead of the Example 3-4 and the Example 17-2 (15.6 mg, 0.044 mmoL) instead of tert-butyl 1-amino-3,6,9,12-tetraoxapentadecan-15-oate were reacted in the same manner as the Example 3-5 to give the title compound (10.3 mg, yield: 48.08%) as a colorless oil.
MS (ESI) m/z 660 $(M+H)^+$

Example 17-4

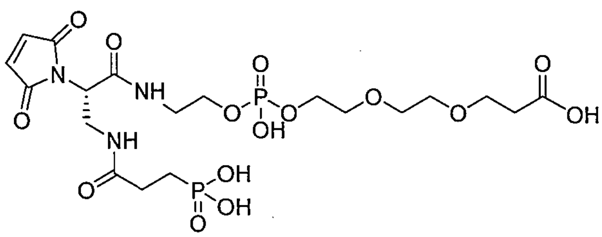

The Example 17-3 (10.3 mg, 0.016 mmoL) instead of the Example 3-5 was reacted in the same manner as the Example 3-6 to give the title compound (8.0 mg, yield: 84.89%) as a colorless oil.

MS (ESI) m/z 604 (M+H)+

Example 17

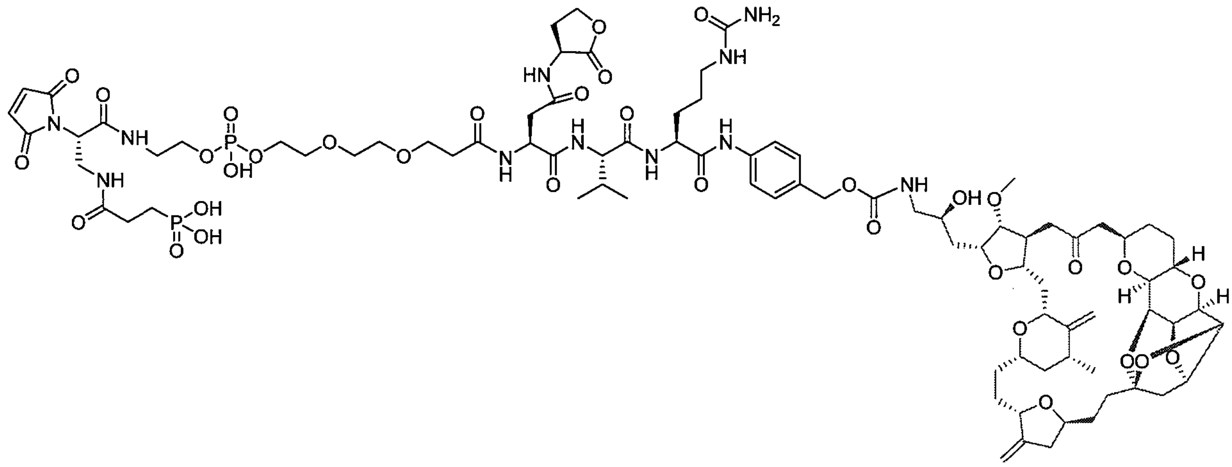

The Example 14-1 (4.4 mg, 2.83 pmoL) instead of the Example 9-2 and the Example 17-4 (8.5 mg, 0.014 mmoL) instead of the Example 3-6 were reacted in the same manner as the Example 9 to give the title compound (4.05 mg, yield: 74.63%) as colorless solids.
MS (ESI) m/z 960 $(M+2H)^{2+}$

Synthesis of Example 18

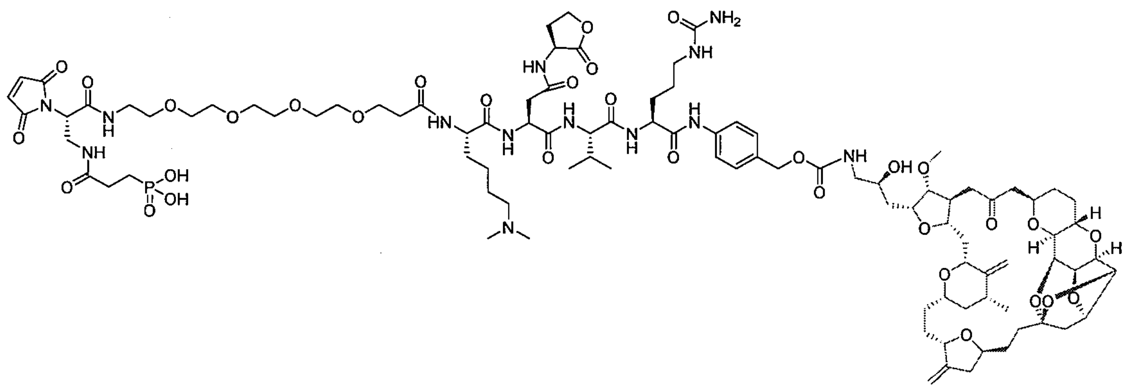

Example 18-1

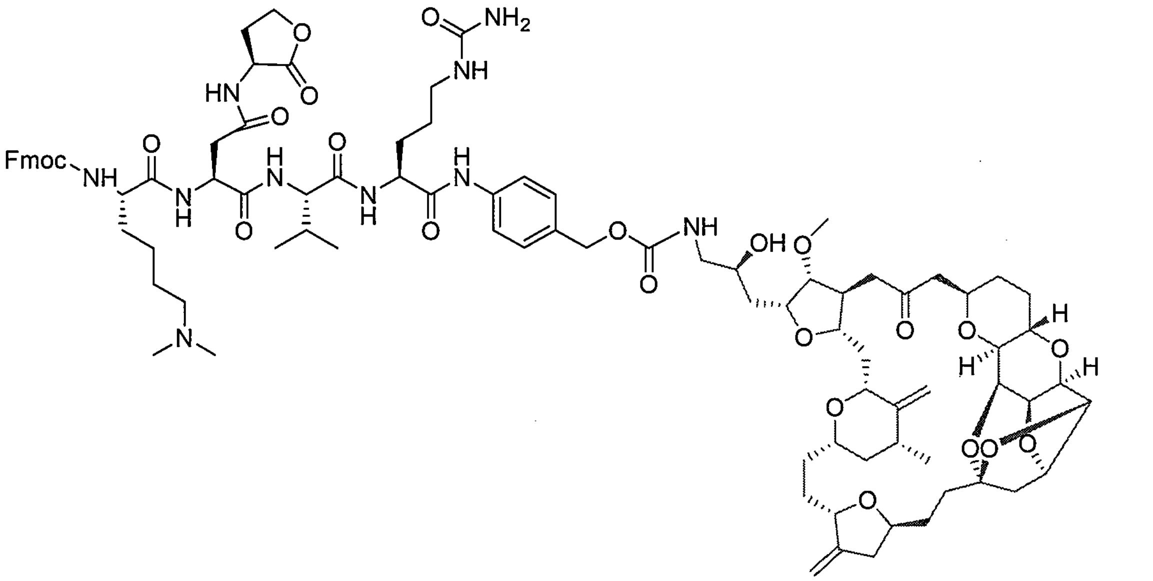

The Example 14-1 (9.5 mg, 6.11 pmoL), and N2-(((9H-fluoren-9-yl)methoxy)carbonyl)-N6,N6-dimethyl-L-lysinehydrochloride (5.3 mg, 0.012 mmoL) instead of the Example 16-1 were reacted in the same manner as the Example 16-2 to give the title compound (7.5 mg, yield: 71.74%) as colorless solids.
MS (ESI) m/z 857 $(M+2H)^{2+}$

Example 18

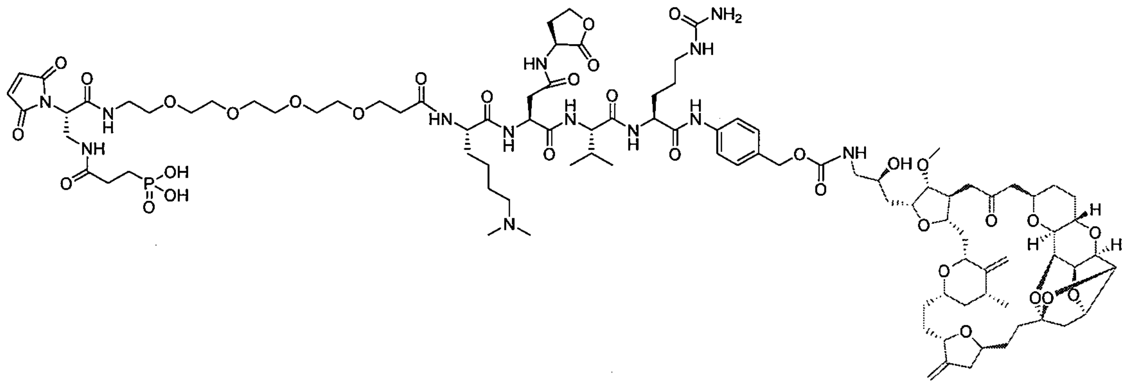

The Example 3-6 (5.0 mg, 8.81 pmoL) and the Example 18-1 (7.5 mg, 4.38 pmoL) instead of the Example 9-2 were reacted in the same manner as the Example 9 to give the title compound (3.8 mg, yield: 42.54%) as colorless solids.
MS (ESI) m/z 1021 $(M+2H)^{2+}$

Synthesis of Example 19

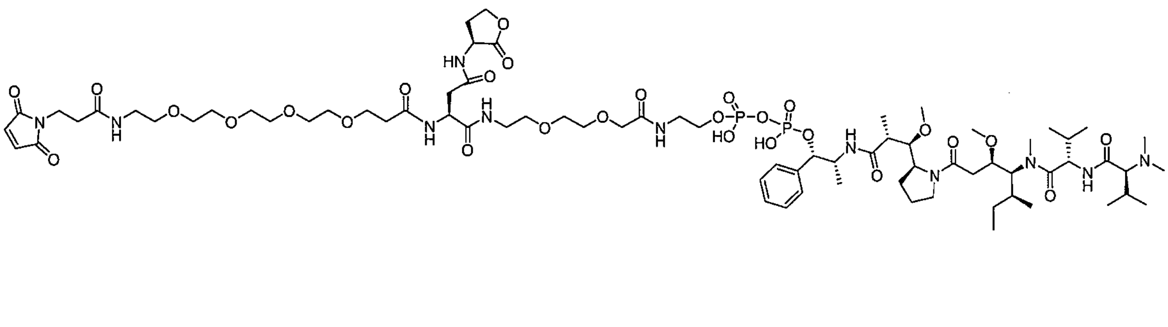

Example 19-1

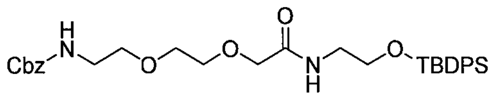

To 2-((tert-butyldiphenylsilyl)oxy)ethan-1-amine (10.78 g, 36.0 mmoL) in a 500 mL round-bottom flask was added a solution of 3-oxo-1-phenyl-2,7,10-trioxa-4-azadodecan-12-oic acid (11.28 g, 37.9 mmoL) in dichloromethane (100 mL) under water-cooling under argon atmosphere with stirring, then 1-ethyl-3-(3-dimethylaminopropyl)carbodiimide hydrochloride (7.28 g, 38.0 mmoL) and 1-hydroxybenzotriazole (1.11 g, 7.25 mmoL) were sequentially added thereto at room temperature, and the resulting mixture was stirred at room temperature for 2 hours.

To the reaction solution were added water (75 mL) and saturated brine (75 mL), the resulting mixture was separated, and the resulting aqueous layer was subjected to extraction with dichloromethane (30 mL) twice. The resulting organic layers were combined, dried over anhydrous magnesium sulfate (10 g), filtered, and the resulting filtrate was concentrated under reduced pressure to give orange syrupy residues (23.19 g).

To the resulting residues was added hexane / ethyl acetate (57/43 (V/V)) (100 mL) to uniformly dissolve the residues, the resulting solution was subjected to YAMAZEN medium pressure preparative (Silica, 5L (3000 g), hexane / ethyl acetate = 40/60 (V/V) (Rf = 0.40 (hexane / ethyl acetate = 40/60 (V/V)))), and the fraction comprising the target compound was concentrated under reduced pressure to give the title compound (20.04 g, yield: 96.19%) as a colorless oil. MS (ESI) m/z 579 $(M+H)^+$

Example 19-2

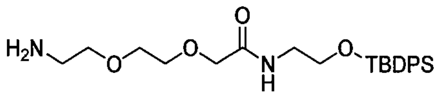

To a solution of the Example 19-1 (21.18 g, 36.6 mmoL) in ethanol (150 mL) in a 500 mL round-bottom flask was added a Pearlman's catalyst (20% Pd, wetted with 50% water, manufactured by Tokyo Chemical Industry Co., Ltd.) (1.28 g, 0.911 mmoL) under nitrogen atmosphere, and then the resulting mixture was stirred under hydrogen atmosphere at room temperature for 5 hours.

The reaction solution was subjected to nitrogen atmosphere, then filtered through Celite 545 (trade name), washed with ethanol, and the resulting filtrate and wash liquid were concentrated under reduced pressure to give the title compound (16.15 g, yield: 99.25%) as a colorless oil. MS (ESI) m/z 445 $(M+H)^+$

Example 19-3

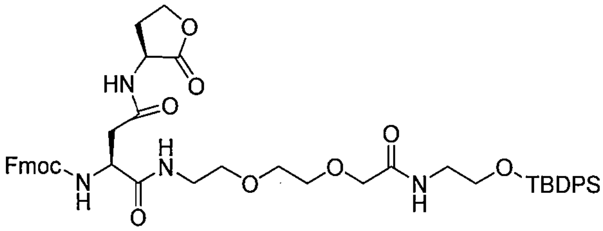

To a solution of the Example 1-2 (2.27 g, 5.11 mmoL) in dichloromethane (20 mL) in a 100 mL round-bottom flask was added the Example 19-2 (2.31 g, 5.27 mmoL) under argon atmosphere, then 1-ethyl-3-(3-dimethylaminopropyl) carbodiimide hydrochloride (1.08 g, 5.63 mmoL) and 1-hydroxybenzotriazole (0.1595 g, 1.042 mmoL) were sequentially added thereto at room temperature, and the resulting mixture was stirred at room temperature for 2 hours.

To the reaction solution were added water (10 mL) and saturated brine (10 mL), the resulting mixture was separated, and the resulting aqueous layer was subjected to extraction with dichloromethane (10 mL) twice. The resulting

organic layers were combined, dried over anhydrous magnesium sulfate, filtered, and the resulting filtrate was concentrated under reduced pressure to give colorless solid residues (6.09 g).

To the resulting residues was added diethyl ether (50 mL), the resulting mixture was subjected to sonication, to the resulting solids was added diethyl ether (50 mL), the resulting mixture was subjected to sonication, and then collected by filtration. The resulting solids were washed with diethyl ether, and dried under reduced pressure to give the title compound (4.26 g, yield: 96.46%) as white solids.
MS (ESI) m/z 865 $(M+H)^+$

Example 19-4

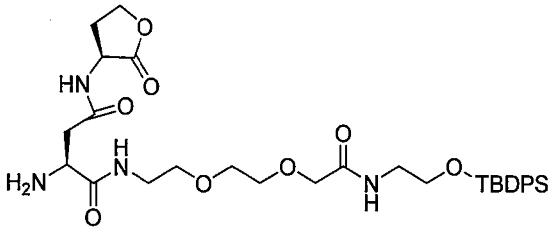

To a solution of the Example 19-3 (4.26 g, 4.92 mmoL) in N,N-dimethylformamide (20 mL) in a 100 mL pear-shaped flask was added piperidine (1.46 mL, 1.26 g, 14.78 mmoL) under argon atmosphere with stirring at room temperature, and the resulting mixture was stirred at room temperature for 0.5 hour.

The reaction solution was concentrated under reduced pressure to give white solid residues (7.72 g). To the resulting residues was added diethyl ether (50 mL), the resulting mixture was subjected to sonication, then left to stand at -20°C for 12 hours, and the resulting supernatant was removed. Subsequently, to the resulting residues was added diethyl ether (25 mL), the resulting mixture was subjected to sonication, then left to stand at -20°C, and the resulting supernatant was removed. Said operation was repeated once again, and then the resulting reaction mixture was dried under reduced pressure to give the title compound (3.05 g, yield: 96.35%) as a slightly yellow oil. MS (ESI) m/z 643 $(M+H)^+$

Example 19-5

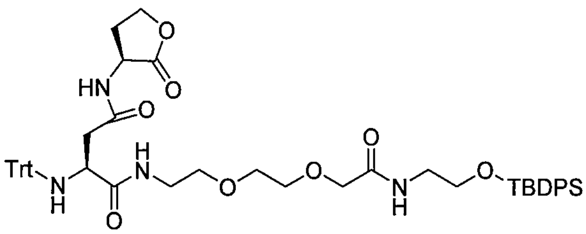

To a solution of the Example 19-4 (3.05 g, 4.74 mmoL) in N,N-dimethylformamide (20 mL) in a 100 mL pear-shaped flask were sequentially added potassium carbonate (1.0075 g, 7.29 mmoL) and trityl chloride (1.70 g, 6.10 mmoL) under argon atmosphere with stirring at room temperature, and then the resulting mixture was stirred at room temperature for 2 hours.

After the reaction was completed, the reaction solution was concentrated. To the concentrated residues was added tetrahydrofuran, the resulting mixture was subjected to sonication, the resulting insoluble matters were filtered, and the resulting filtered residues were washed with tetrahydrofuran. The resulting filtrate and wash liquid were combined, and the resulting mixture was concentrated under reduced pressure to give slightly yellow oily concentrated residues (5.61 g).

To the resulting residues was added dichloromethane to dissolve the residues, Fuji Silysia CHROMATOREX Q-PACK Diol-60 Size 60 (27.0 g) was added thereto, and the resulting mixture was dried under reduced pressure. The resulting residues were subjected to YAMAZEN medium pressure preparative (Fuji Silysia CHROMATOREX Q-PACK Diol-60 (85.0 g), hexane / ethyl acetate = 7/93 (V/V) → 0/100 (V/V), (Rf = 0.50 (ethyl acetate))), and the fraction comprising the target compound was concentrated under reduced pressure to give the title compound (2.82 g, yield: 67.15%) as a colorless oil.
MS (ESI) m/z 885 $(M+H)^+$

Example 19-6

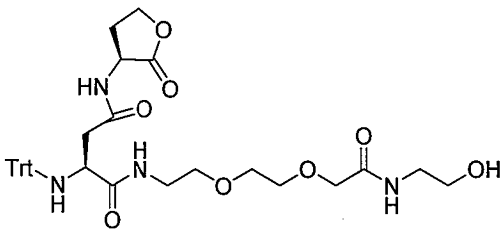

To a solution of the Example 19-5 (2.82 g, 3.19 mmoL) in tetrahydrofuran (50 mL) in a 300 mL round-bottom flask was added a 1 M solution of tetrabutylammonium fluoride in tetrahydrofuran (3.5 mL, 0.96 g, 3.50 mmoL) under argon atmosphere with stirring at room temperature, and the resulting mixture was stirred at room temperature for 2 hours.

The reaction solution was concentrated under reduced pressure, to the resulting residues (4.42 g) was added dichloromethane (50 mL) to dissolve the residues, and then the resulting solution was washed with a saturated aqueous solution of ammonium chloride (50 mL). The resulting aqueous layer was subjected to extraction with dichloromethane (25 mL) twice, the resulting organic layers were combined, dried over anhydrous sodium sulfate (10 g), filtered, and the resulting filtrate was concentrated under reduced pressure to give colorless oily residues (4.28 g).

To the resulting residues was added acetonitrile (45 mL) to dissolve the residues, the resulting solution was subjected to YAMAZEN medium pressure preparative (Silica, L (40 g), A/B = 100/0 (V/V) → 50/50 (V/V), and then ethyl acetate / methanol = 100/0 (V/V) → 40/60 (V/V) (Rf = 0.47 (ethyl acetate)), Solution A; acetonitrile/water/triethylamine = 950/50/1 (V/V/V), Solution B; acetonitrile/water/triethylamine = 850/150/5 (V/V/V)), and the fraction comprising the target compound was concentrated under reduced pressure to give crude products of the title compound (1.76 g) as white foam.

Subsequently, the crude products were subjected to recycle preparative under the following conditions, and the fraction comprising the target compound was concentrated under reduced pressure to give the title compound (1.58 g, yield: 71.62%) as white foam.
MS (ESI) m/z 647 $(M+H)^+$

- Device: LC Forte/R
- Column: GPC system
- Eluent: dichloromethane → acetonitrile
- Flow rate: 15 mL/min.
- Pressure: 12 MPa (dichloromethane), 8.4 MPa (acetonitrile)
- Wavelength: 220 nm

Example 19-7

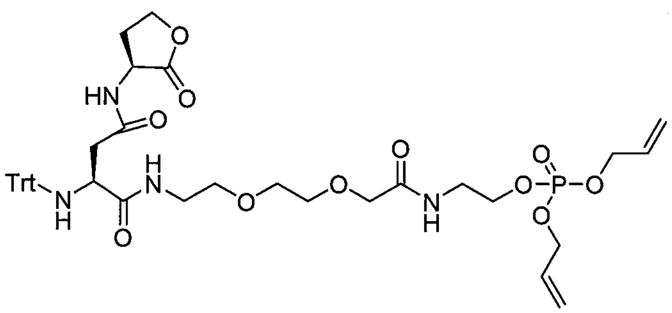

To a solution of the Example 19-6 (1.58 g, 2.282 mmoL) in dichloromethane (30 mL) in a 200 mL round-bottom flask were sequentially added diallyl N,N-diisopropylphosphoramidite (2.58 mL, 2.39 g, 9.76 mmoL) and 1H-tetrazole (1.20 g, 17.13 mmoL) under argon atmosphere with stirring at room temperature, and the resulting mixture was stirred at room temperature for 2 hours.

Subsequently, the mixture was ice-cooled, 30% by weight of hydrogen peroxide water (0.650 mL, 0.72 g, 6.36 mmoL) was added thereto, and the resulting mixture was stirred under ice-cooling for 1 hour.

Subsequently, to sodium thiosulfate pentahydrate (20.0 g, 80.6 mmoL) and sodium hydrogen carbonate (7.0 g, 83.3 mmoL) was added water (100 mL) to prepare a solution, 30 mL of said solution was added to the above mixture, and the resulting solution was separated. The resulting aqueous layer was subjected to extraction with dichloromethane (15 mL) twice, combined with the resulting organic layer, dried over anhydrous sodium sulfate (10.0 g), filtered, and the resulting filtrate was concentrated under reduced pressure to give slightly yellow oily concentrated residues (3.49 g).

To the residues was added dichloromethane (30 mL) to dissolve the residues, Fuji Silysia CHROMATOREX Q-PACK Diol-60 Size 60 (22.0 g) was added thereto, and the resulting mixture was dried under reduced pressure. The resulting residues were subjected to YAMAZEN medium pressure preparative (Fuji Silysia CHROMATOREX Q-PACK Diol-60 (88.0 g), hexane / ethyl acetate = 0/100 (V/V), (Rf = 0.33 (ethyl acetate))), and the fraction comprising the target compound was concentrated under reduced pressure to give the title compound (1.55 g, yield: 84.19%) as white foam.
MS (ESI) m/z 807 $(M+H)^+$

Example 19-8

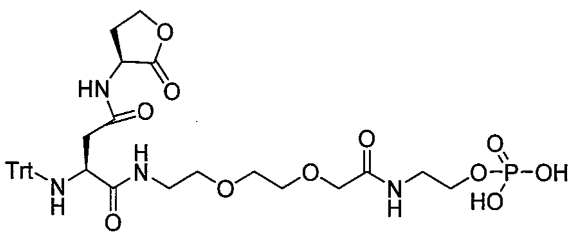

To a solution of the Example 19-7 (1.55 g, 1.921 mmoL) in tetrahydrofuran (33 mL) in a 300 mL round-bottom flask were sequentially added N-methylaniline (0.440 mL, 0.44 g, 4.07 mmoL) and tetrakis(triphenylphosphine)palladium (0.2263 g, 0.196 mmoL) under argon atmosphere with stirring at room temperature, and the resulting mixture was stirred at room temperature for 1 hour.

The reaction solution was concentrated under reduced pressure, then to the resulting pale yellow foam concentrated residues (2.38 g) was added diethyl ether (50 mL), the resulting mixture was subjected to sonication, the resulting solids were collected by filtration, washed with diethyl ether, and dried under reduced pressure to give a N-allyl-N-methylaniline salt of the title compound (1.89 g, yield: 96.34%) as pale yellow solids.
MS (ESI) m/z 727 $(M+H)^+$

Example 19-9

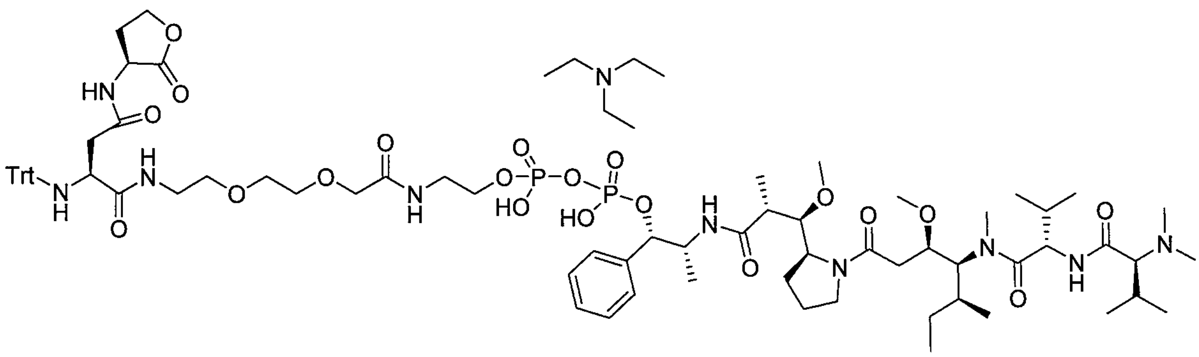

To a solution of the Example 19-8 (0.3958 g, 0.388 mmoL) in N,N-dimethylformamide (2.5 mL) in a 50 mL pear-shaped flask was added carbonyldiimidazole (0.1575 g, 0.971 mmoL) under argon atmosphere with stirring at room temperature, and the resulting mixture was stirred at room temperature for 0.75 hour.

Subsequently, U-031 (0.3096 g, 0.381 mmoL) was added thereto, then the resulting mixture was ice-cooled, zinc chloride (0.4098 g, 3.01 mmoL) was added thereto, then the resulting mixture was warmed to room temperature, and stirred at room temperature for 6 hours.

To the reaction solution was added diethyl ether (25 mL), the resulting mixture was subjected to sonication, and

then the resulting supernatant was removed. To the resulting residues was added diethyl ether (10 mL), and the resulting supernatant was removed. Said operation was repeated once again, and the resulting residues were dried under reduced pressure to give a pale yellow syrup (1.64 g).

To the syrup was added acetonitrile/water/triethylamine (= 850/150/5 (V/V/V)) (20 mL) to dissolve the syrup, the resulting solution was packaged in YAMAZEN injection column (silica gel) size L, subjected to YAMAZEN medium pressure preparative (Silica, L (40 g), acetonitrile/water/triethylamine = 850/150/5 (V/V/V)), and the fraction comprising the target compound was concentrated under reduced pressure. To the resulting residues was added acetonitrile/water (1/1 (V/V)) (30 mL) to dissolve the residues, and then the resulting solution was freeze-dried to give the title compound (517 mg, yield: 83.6%) as white solids.
MS (ESI) m/z 761 $(M+2H)^{2+}$

Example 19-10

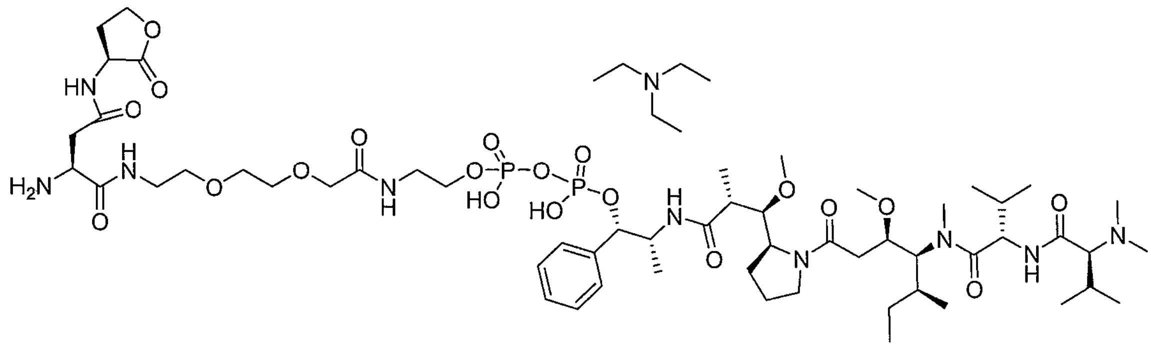

To a solution of the Example 19-9 (512.8 mg, 0.316 mmoL) in N,N-dimethylformamide (3 mL) in a 50 mL round-bottom flask was added formic acid (3 mL, 3660 mg, 80 mmoL) under argon atmosphere with stirring under ice-cooling, and then the resulting mixture was stirred at room temperature for 45 minutes.

Subsequently, diethyl ether (30 mL) was added thereto, the resulting mixture was subjected to sonication, and left to stand at -20°C for 3 hours. The resulting supernatant was removed, then to the resulting residues was added diethyl ether (15 mL), the resulting mixture was stirred for 5 minutes, and then the resulting supernatant was removed. Once again, to the resulting residues was added diethyl ether (15 mL), the resulting mixture was stirred for 5 minutes, and then the resulting supernatant was removed. The resulting residues were dried under reduced pressure to give a colorless syrup (0.7368 g).

To the syrup was added acetonitrile/water/triethylamine (= 850/150/5 (V/V/V)) (15 mL) to dissolve the syrup, then the resulting solution was packaged in YAMAZEN injection column (silica gel) size M, subjected to YAMAZEN medium pressure preparative (Silica, M (16 g), acetonitrile/water/triethylamine = 850/150/5 (V/V/V)), and the fraction comprising the target compound was concentrated under reduced pressure. To the resulting residues was added acetonitrile/water (1/1 (V/V)) (20 mL) to dissolve the residues, and the resulting solution was freeze-dried to give the title compound (328.1 mg, yield: 75.22%) as white solids.
MS (ESI) m/z 640 $(M+2H)^{2+}$

Example 19

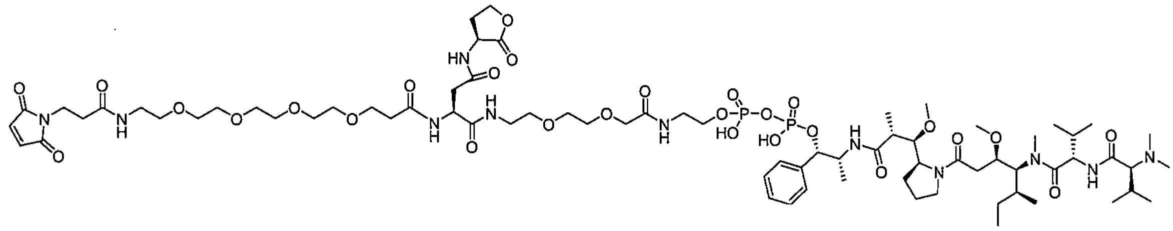

N-succinimidyl 1-maleimide-3-oxo-7,10,13,16-tetraoxa-4-azanonadecanoate (10.0 mg, 0.019 mmoL) and the

Example 19-10 (9.5 mg, 6.89 pmoL) instead of the Example 1-7 were reacted in the same manner as the Example 1 to give the title compound (2.5 mg, yield: 21.65%) as white solids. MS (ESI) m/z 839 $(M+2H)^{2+}$

Synthesis of Example 20

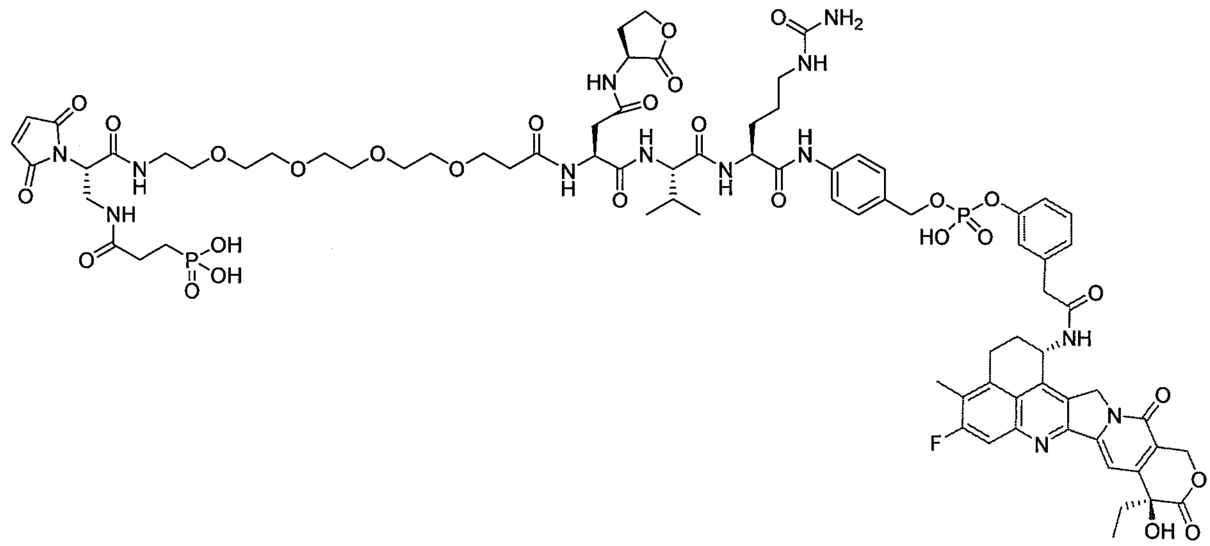

Example 20-1

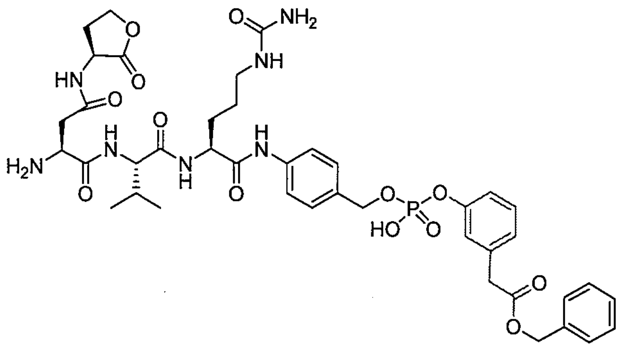

To a solution of benzyl 2-(3-hydroxyphenyl)acetate (72.2 mg, 0.298 mmoL) in N,N-dimethylformamide (2 mL) in a 10 mL pear-shaped flask were sequentially added 2-cyanoethyl N,N,N',N'-tetraisopropylphosphordiamidite (95 μL, 90.25 mg, 0.299 mmoL) and 1H-tetrazole (21.7 mg, 0.310 mmoL), and the resulting mixture was stirred at room temperature for 1 hour. The Example 2-1 (80.0 mg, 0.100 mmoL) was added thereto, then 5-(ethylthio)-1H-tetrazole (26.7 mg, 0.205 mmoL) was added thereto, and the resulting mixture was stirred at room temperature for 0.5 hour.

Subsequently, a 70% aqueous solution of tert-butyl hydroperoxide (41 μL, 38.54 mg, 0.299 mmoL) was added thereto at room temperature, and then the resulting mixture was stirred at room temperature for 0.5 hour. Subsequently, diazabicycloundecene (149 μL, 151.98 mg, 0.998 mmoL) was added thereto, and the resulting mixture was stirred at room temperature for 20 minutes. Formic acid (38 μL, 45.6 mg, 0.991 mmoL) was added thereto, and the resulting mixture was left to stand overnight.

To the resulting reaction solution was added a 50% aqueous solution of acetonitrile, then the resulting mixture was filtered, the resulting filtrate was subjected to preparative HPLC chromatography under the following conditions, the fraction comprising the target compound was concentrated under reduced pressure to distill away acetonitrile, and then the resulting residues were freeze-dried to give the title compound (16.9 mg, yield: 19.16%) as colorless foam.
MS (ESI) m/z 882 $(M+H)^+$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 25% (0.00 min.) → 46% (6.00 min.)

Example 20-2

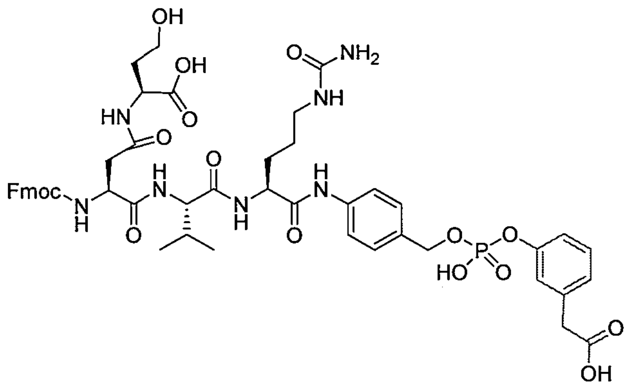

To a solution of the Example 20-1 (11.7 mg, 0.013 mmoL) in N,N-dimethylformamide (200 μL) in a 10 mL pear-shaped flask was added a 2N aqueous solution of sodium hydroxide (33 μL, 2.64 mg, 0.066 mmoL), and the resulting mixture was stirred at room temperature for 2 hours. Subsequently, 2N hydrochloric acid (33 μB) was added thereto.

Subsequently, N,N-dimethylbenzylamine (4 μL, 3.64 mg, 0.027 mmoL) and 9-fluorenylmethyl succinimidyl carbonate (9.0 mg, 0.027 mmoL) were added thereto, and the resulting mixture was stirred at room temperature for 1 hour. Subsequently, N,N-dimethylbenzylamine (4 μL, 3.64 mg, 0.027 mmoL) was added thereto, and the resulting mixture was stirred for 16 hours.

To the resulting reaction solution were added N,N-dimethylformamide and a 50% aqueous solution of acetonitrile, then the resulting mixture was filtered, the resulting filtrate was subjected to preparative HPLC chromatography under the following conditions, the fraction comprising the target compound was concentrated under reduced pressure, and then the resulting residues were freeze-dried to give the title compound (9.4 mg, yield: 68.65%) as colorless foam.
MS (ESI) m/z 1033 $(M+H)^+$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 25% (0.00 min.) → 60% (8.00 min.)

Example 20-3

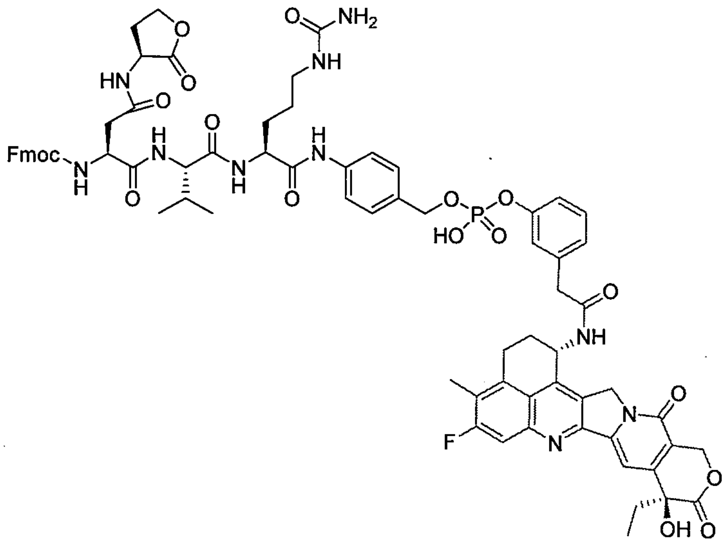

To a solution of exatecan mesylate (5.5 mg, 10.35 pmoL), the Example 20-2 (9.4 mg, 9.11 pmoL), and N,N-dimethylbenzylamine (5.71 μL, 5.2 mg, 0.038 mmoL) in N,N-dimethylformamide (500 μL) in a 10 mL cylindrical flask was added HATU (19.0 mg, 0.050 pmoL) under argon atmosphere with stirring at room temperature, and the resulting mixture was stirred at room temperature for 2 hours.

After the reaction was completed, the resulting reaction solution was subjected to preparative HPLC chromatography under the following conditions, and the fraction comprising the target compound was freeze-dried to give the title compound (6.8 mg, yield: 52.15%) as colorless solids.
MS (ESI) m/z 1432 $(M+H)^+$

Column: Waters SunFire Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 25% (0.00 min.) → 65% (8.00 min.)

Example 20

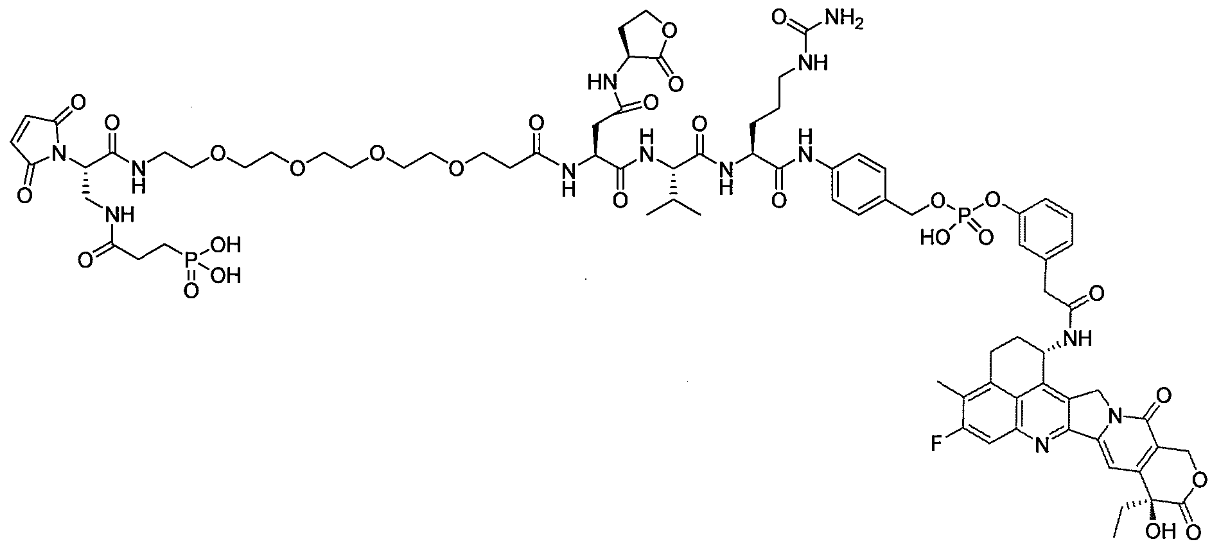

The Example 3-6 (10.8 mg, 0.019 mmoL) and the Example 20-3 (6.8 mg, 4.75 pmoL) instead of the Example 9-2 were reacted in the same manner as the Example 9 to give the title compound (5.16 mg, yield: 61.76%) as colorless solids.
MS (ESI) m/z 880 $(M+2H)^{2+}$

Synthesis of Example 21

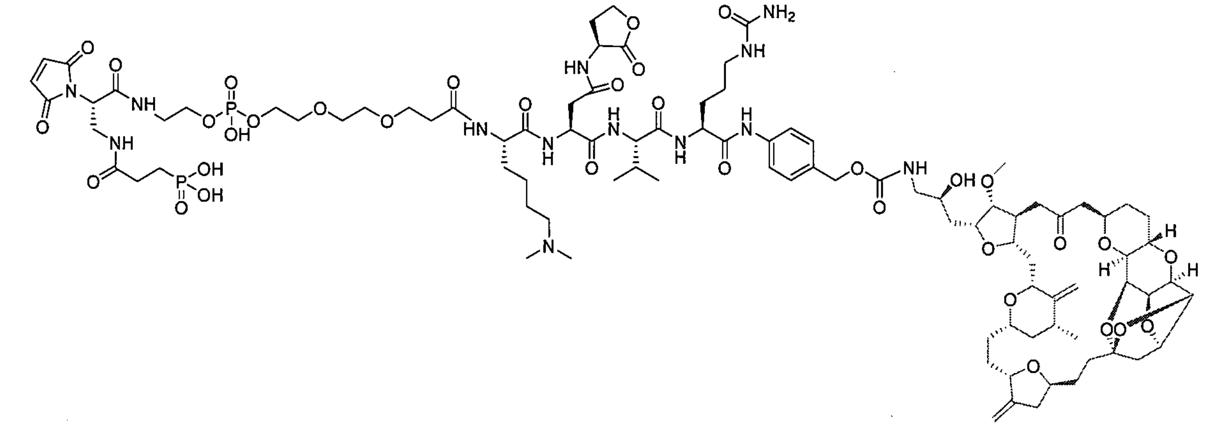

The Example 18-1 (8.0 mg, 4.67 pmoL) instead of the Example 9-2 and the Example 17-4 (15.2 mg, 0.025 mmoL) instead of the Example 3-6 were reacted in the same manner as the Example 9 to give the title compound (4.3 mg, yield: 44.34%) as a colorless amorphous.
MS (ESI) m/z 1036 $(M-2H)^{2-}$

Synthesis of Example 22

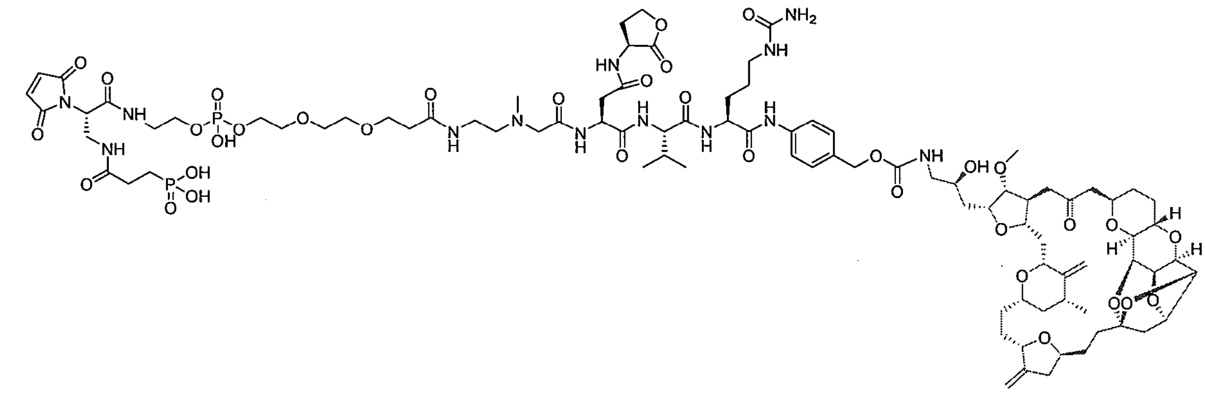

The Example 16-2 (6.8 mg, 4.07 pmoL) instead of the Example 9-2 and the Example 17-4 (6.2 mg, 10.27 pmoL) instead of the Example 3-6 were reacted in the same manner as the Example 9 to give the title compound (2.1 mg, yield: 25.37%) as colorless solids.
MS (ESI) m/z 1016 $(M+2H)^{2+}$

Synthesis of Example 23

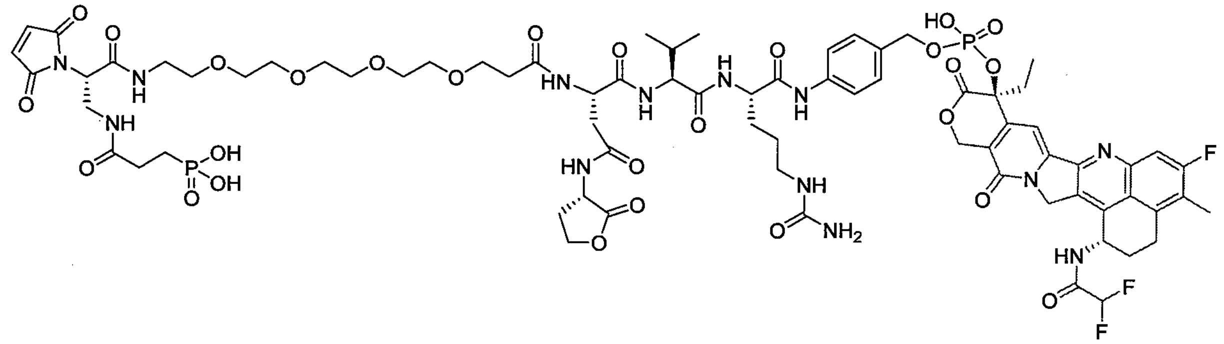

Example 23-1

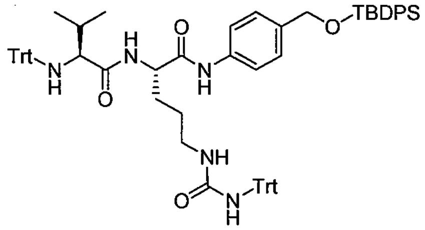

To a solution of (L)-Val-(L)-Cit-PAB (10.0 g, 26.4 mmoL) in tetrahydrofuran (100 mL) in a 500 mL round-bottom flask was added 1H-imidazole (3.59 g, 52.7 mmoL) under argon gas flow with stirring, then the resulting mixture was ice-cooled, tert-butyldiphenylsilyl chloride (7.45 mL, 7.97 g, 29.0 mmoL) was added thereto, and the resulting mixture was stirred at room temperature for 1.5 hours.

Subsequently, tert-butyldiphenylsilyl chloride (0.5 mL, 0.54 g, 1.946 mmoL) was added thereto under stirring under ice-cooling, and the resulting mixture was stirred at room temperature for 1 hour.

After the reaction was completed, to the reaction solution was added ethyl acetate (300 mL), the resulting insoluble matters were filtered, and washed with ethyl acetate (15 mL). To the resulting filtrate were added a saturated aqueous solution of sodium hydrogen carbonate (100 mL) and saturated brine (100 mL), the resulting mixture was stirred at room temperature for 15 minutes, then separated, and the resulting aqueous layer was subjected to extraction with ethyl acetate (20 mL) twice. The resulting organic layers were combined, dried over anhydrous sodium sulfate (5 g), filtered, and the resulting filtrate was concentrated under reduced pressure.

The resulting solids were added to tetrahydrofuran (150 mL), a saturated aqueous solution of ammonium chloride (150 mL) and water (20 mL) were added thereto, and the resulting mixture was separated. The resulting aqueous layer was subjected to extraction with ethyl acetate (20 mL) twice, washed with saturated brine (50 mL), the resulting organic layers were combined, dried over sodium sulfate (5 g), and then concentrated under reduced pressure.

To a solution of the resulting residues in dichloromethane (200 mL) were added triethylamine (36.7 mL, 26.64 g, 263 mmoL) and trityl chloride (36.7 g, 132 mmoL) under ice-cooling, and then the resulting mixture was stirred at room temperature for 1 hour. Subsequently, trityl chloride (36.7 g, 132 mmoL) was added thereto under stirring at room temperature, and the resulting mixture was stirred at room temperature for 1 hour.

Subsequently, triethylamine (36.7 mL, 26.64 g, 263 mmoL) and trityl chloride (36.7 g, 132 mmoL) were added thereto under stirring at room temperature, and the resulting mixture was stirred at room temperature for 1 hour.

Subsequently, trityl chloride (15.0 g, 53.8 mmoL) was added thereto under stirring at room temperature, and the resulting mixture was stirred at room temperature for 30 minutes.

After the reaction was completed, water (40 mL) was added thereto, the resulting mixture was separated, and the resulting aqueous layer was subjected to extraction with dichloromethane (20 mL) twice. The resulting organic layers were combined, washed with saturated brine, dried over anhydrous sodium sulfate, filtered, the resulting filtrate was concentrated under reduced pressure, and the precipitated solids were filtered to give a solution comprising the target compound.

The above solution was concentrated under reduced pressure, the concentration was stopped when white solids were precipitated, and the resulting mixture was stirred under ice-cooling for 30 minutes. The resulting solids were filtered, washed with a mixed solvent (50 mL) of hexane / ethyl acetate (= 70/30 (V/V)), and dried under reduced pressure to give the title compound (30.2459 g, yield: quantitative) as white solids.

MS (ESI) m/z 1103 $(M+H)^+$

Example 23-2

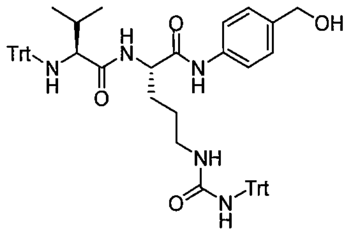

To a solution of the Example 23-1 (30.0 g, 27.2 mmoL) in tetrahydrofuran (270 mL) in a 500 mL round-bottom flask was added 1N tetra-n-butylammonium fluoride (77 mL) at room temperature, and then the resulting mixture was stirred at room temperature for 5 hours.

After the reaction was completed, the reaction solution was concentrated under reduced pressure, the resulting residues were dissolved in dichloromethane (30 mL), the resulting solution was subjected to YAMAZEN medium pressure preparative (Silica, 3L (135 g), dichloromethane /ethyl acetate = 92/8 (V/V) → 44/56 (V/V)), and the fractions comprising the target compound (Rf = 0.45 (dichloromethane /ethyl acetate = 50/50 (V/V))) were collected. The precipitated solids were filtered, washed with dichloromethane, and dried under reduced pressure to give the title compound (16.2604 g, yield: 69.16%) as white solids.
MS (ESI) m/z 864 $(M+H)^+$

Example 23-3

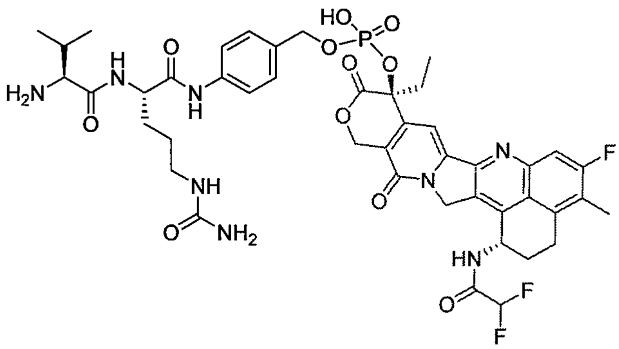

To a solution of the Example 23-2 (434 mg, 0.502 mmoL) in dichloromethane (15 mL) in a 50 mL round-bottom flask were sequentially added 1-allyloxy N,N,N',N'-tetraisopropylphosphinediamine (190 μL, 171.57 mg, 0.595 mmoL) and 1H-tetrazole (44 mg, 0.628 mmoL) under argon atmosphere with stirring at room temperature, and the resulting mixture was stirred at room temperature for 1 hour.

Subsequently, U-032 (99 mg, 0.193 mmoL) and 5-(ethylthio)-1H-tetrazole (80 mg, 0.615 mmoL) were added thereto, and the resulting mixture was stirred under heating at 43°C for 3 hours.

Subsequently, the mixture was ice-cooled, a 70% aqueous solution of tert-butyl hydroperoxide (145 μL, 136.3 mg, 1.059 mmoL) was added thereto under ice-cooling, and the resulting mixture was stirred under ice-cooling for 1 hour.

After the reaction was completed, to the reaction solution was added water (10 mL), and the resulting mixed solution was subjected to extraction with methylene chloride (15 mL). The resulting organic layer was washed with water, then dried over anhydrous magnesium sulfate, filtered, and concentrated under reduced pressure to give a pale yellow oil.

To a solution of the above pale yellow oil in tetrahydrofuran (15 mL) in a 100 mL round-bottom flask were sequentially added N-methylaniline (57 μL, 56.43 mg, 0.527 mmoL) and tetrakis(triphenylphosphine)palladium (47 mg, 0.041 mmoL) under argon atmosphere with stirring at room temperature, the resulting mixture was stirred at room temperature for 2 hours, and then the solvent was removed under reduced pressure.

To the resulting residues was added tert-butyl methyl ether, the resulting mixture was subjected to sonication, and

the resulting supernatant was removed. Subsequently, to the resulting residues was added water, the resulting mixture was subjected to sonication, the resulting supernatant was removed, and dried under reduced pressure to give residues (770 mg).

To a solution of the above residues in dichloromethane (10 mL) in a 100 mL round-bottom flask was added trifluoroacetic acid (166 µL, 245.68 mg, 2.155 mmoL) at room temperature, and the resulting mixture was stirred at room temperature for 3 hours. Subsequently, trifluoroacetic acid (83 µL, 122.84 mg, 1.077 mmoL) was added thereto at room temperature, and the resulting mixture was stirred at room temperature for 1 hour.

After the reaction was completed, tert-butyl methyl ether (20 mL) was added thereto, and the resulting supernatant was removed. The resulting residues were dissolved in a 30% aqueous solution of acetonitrile, the resulting solution was subjected to preparative HPLC chromatography under the following conditions, and the fraction comprising the target compound was freeze-dried to give the title compound (65.1 mg, yield: 35.35%) as slightly yellow solids.
MS (ESI) m/z 955 $(M+H)^+$

Column: Waters XBridge Prep C18 5 µm ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) -> 60% (6.00 min.) - 90% (6.50 min.) -> 90% (9.00 min.)

Example 23-4

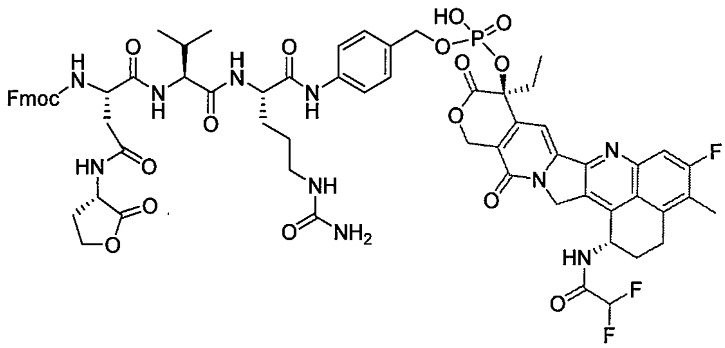

To a solution of the Example 1-2 (39 mg, 0.089 mmoL) in acetonitrile (1.2 mL) in a 20 mL cylindrical flask were added triethylamine (14 µL, 10.16 mg, 0.100 mmoL) and HATU (33 mg, 0.087 mmoL) under argon gas flow with stirring at room temperature, and the resulting mixture was stirred at room temperature for 15 minutes.

Subsequently, a solution of the Example 23-3 (65 mg, 0.068 mmoL) and triethylamine (10 µL, 7.26 mg, 0.072 mmoL) in N,N-dimethylformamide (1.8 mL) was added thereto under stirring at room temperature, and the resulting mixture was stirred at room temperature for 0.5 hour.

Subsequently, the Example 1-2 (12 mg, 0.027 mmoL), HATU (5.0 mg, 0.013 mmoL), and triethylamine (8 µL, 5.81 mg, 0.057 mmoL) were added thereto under stirring at room temperature, and the resulting mixture was stirred at room temperature for 0.5 hour.

After the reaction was completed, the reaction solution was concentrated under reduced pressure. To the resulting residues were added water (4 mL) and acetonitrile (3 mL), the precipitated solids were filtered, washed with ethyl acetate, and dried under reduced pressure to give the title compound (60 mg, yield: 64.09%) as pale yellow solids.
MS (ESI) m/z 1376 $(M+H)^+$

Example 23-5

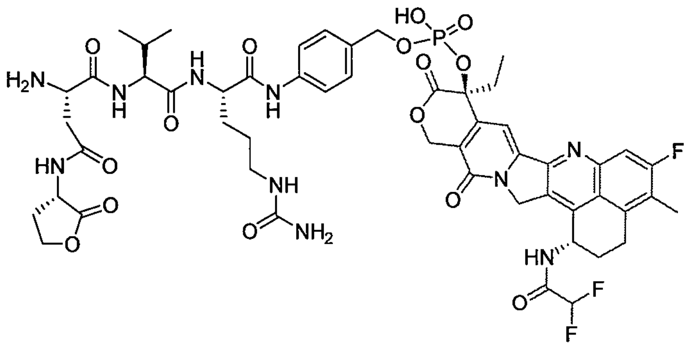

To a solution of the Example 23-4 (60 mg, 0.044 mmoL) in N,N-dimethylformamide (1 mL) in a 10 mL cylindrical flask was added piperidine (0.013 mL, 11.14 mg, 0.131 mmoL) under argon gas flow with stirring at room temperature, the resulting mixture was stirred at room temperature for 1 hour, and then the solvent was removed under reduced pressure.
The resulting residues were washed with ethyl acetate and then with diethyl ether, and dried under reduced pressure to give the title compound (45 mg, yield: 89.46%) as dark brown solids.
MS (ESI) m/z 1154 $(M+H)^+$

Example 23

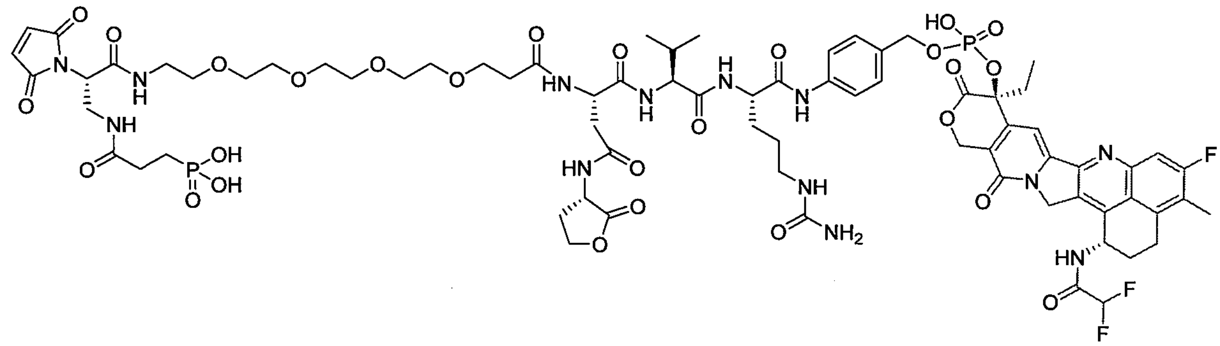

The Example 3-6 (12.5 mg, 0.022 mmoL) and the Example 23-5 (12 mg, 10.41 pmoL) instead of the Example 3-7 were reacted in the same manner as the Example 3 to give the title compound (2.8 mg, yield: 15.8%) as white solids. MS (ESI) m/z 852 $(M+2H)^{2+}$

Synthesis of Example 24

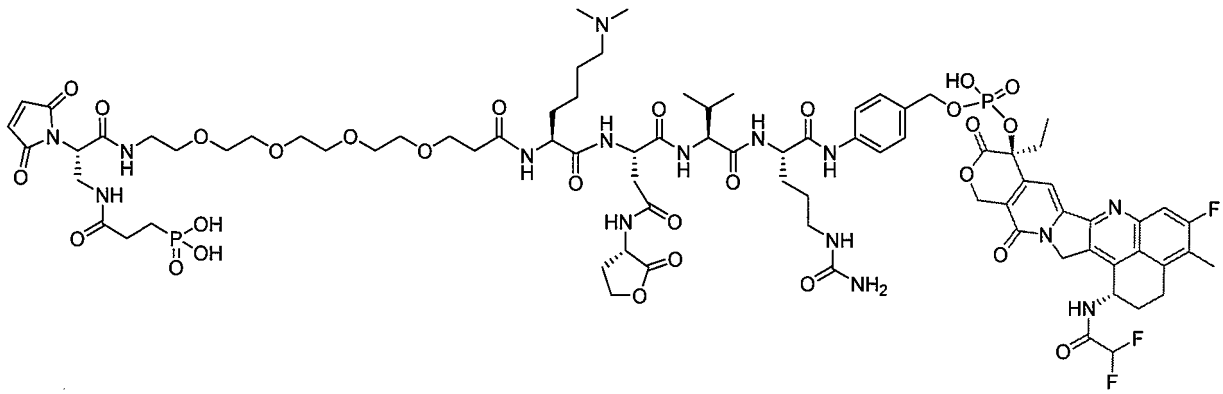

Example 24-1

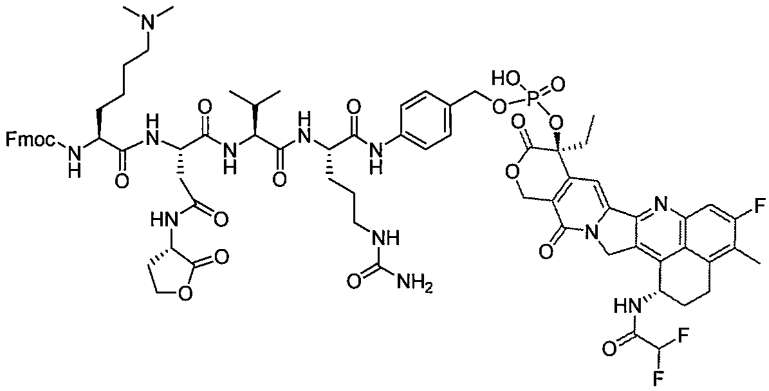

To a solution of the Example 23-4 (49 mg, 0.036 mmoL) in N,N-dimethylformamide (1 mL) in a 10 mL cylindrical flask was added piperidine (10 μL, 8.6 mg, 0.101 mmoL) under argon gas flow with stirring at room temperature, the resulting mixture was stirred at room temperature for 1 hour, then the solvent was removed under reduced pressure, the resulting residues were washed with ethyl acetate and then with diethyl ether, and the resulting solids were dried under reduced pressure.

To a solution of N2-(((9H-fluoren-9-yl)methoxy)carbonyl)-N6,N6-dimethyl-L-lysinehydrochloride (27 mg, 0.068 mmoL) in acetonitrile (1.2 mL) in a 10 mL cylindrical flask were added triethylamine (10 μL, 7.2 mg, 0.072 mmoL) and HATU (18 mg, 0.047 mmoL) under argon gas flow with stirring at room temperature, and the resulting mixture was stirred at room temperature for 15 minutes.

Subsequently, a solution of the above resulting solids and triethylamine (5 μL, 3.63 mg, 0.036 mmoL) in N,N-dimethylformamide (1 mL) was added thereto under stirring at room temperature, the resulting mixture was stirred at room temperature for 1 hour, and then the solvent was removed under reduced pressure. To the resulting residues was added diethyl ether, the resulting mixture was subjected to sonication, then the resulting supernatant was removed, and the resulting solids were dried under reduced pressure to give the title compound (68 mg, yield: quantitative) as dark brown solids.

MS (ESI) m/z 767 $(M+2H)^{2+}$

Example 24-2

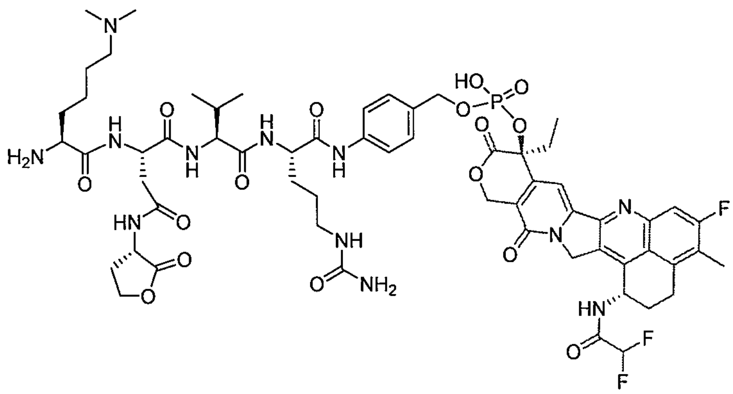

To a solution of the Example 24-1 (68 mg, 0.044 mmoL) in N,N-dimethylformamide (1 mL) in a 20 mL cylindrical flask was added piperidine (10 μL, 8.6 mg, 0.101 mmoL) under argon gas flow with stirring at room temperature, the resulting mixture was stirred at room temperature for 1 hour, and then the solvent was removed under reduced pressure. The resulting residues were subjected to preparative HPLC chromatography under the following conditions, and the fraction comprising the target compound was freeze-dried to give the title compound (12 mg, yield: 20.64%) as white solids.

MS (ESI) m/z 655 $(M+2H)^{2+}$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 20% (0.00 min.) → 50% (6.00 min.) - 90% (6.50 min.) -> 90% (9.00 min.)

Example 24

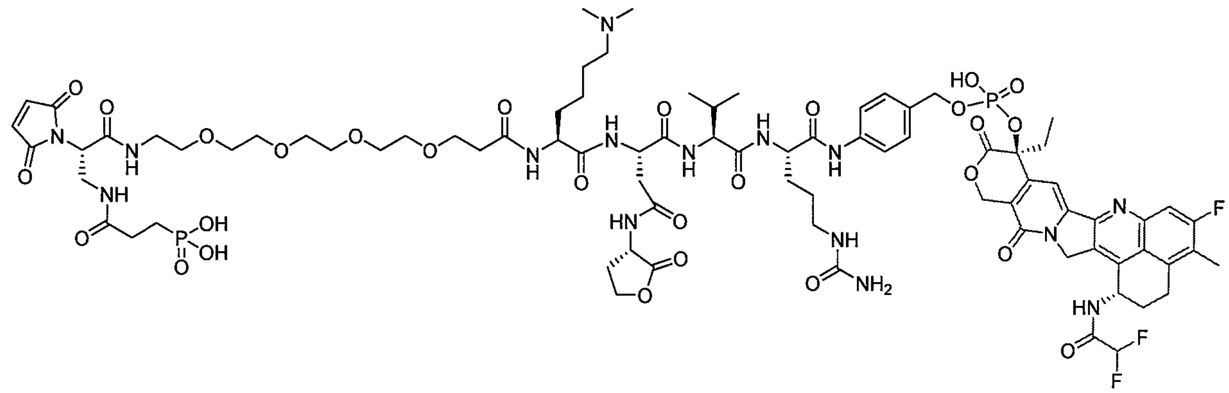

The Example 3-6 (13 mg, 0.023 mmoL) and the Example 24-2 (12 mg, 9.17 pmoL) instead of the Example 3-7 were reacted in the same manner as the Example 3 to give the title compound (8.6 mg, yield: 50.48%) as white solids. MS (ESI) m/z 930 $(M+2H)^{2+}$

Synthesis of Example 25

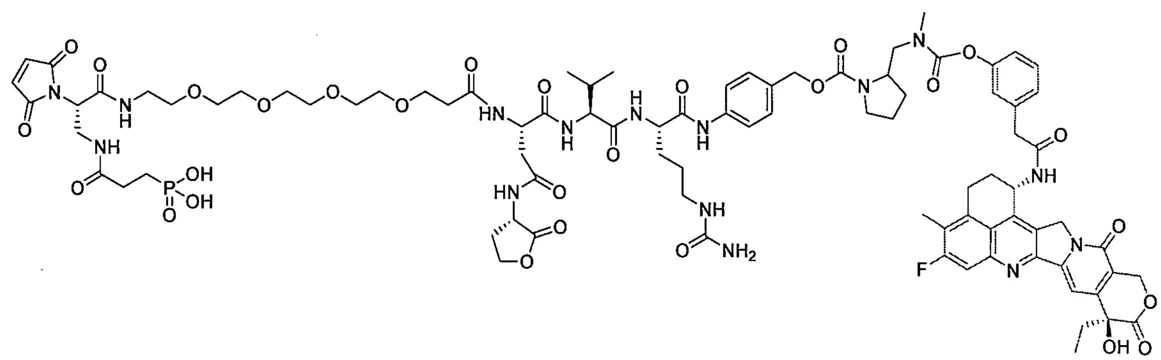

Example 25-1

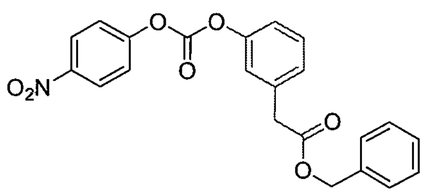

To a solution of benzyl 2-(3-hydroxyphenyl)acetate (3.40 g, 14.03 mmoL) in N,N-dimethylformamide (45 mL) in a 100 mL round-bottom flask were added bis(4-nitrophenyl)carbonate (4.70 g, 15.45 mmoL) and N,N-diisopropylethylamine

(4.90 mL, 3.63 g, 28.1 mmoL) under argon gas flow with stirring at room temperature, and the resulting mixture was stirred at room temperature for 3 hours.

After the reaction was completed, the solvent was concentrated under reduced pressure. To the resulting residues was added water, and the resulting mixed solution was subjected to extraction with ethyl acetate. The resulting organic layer was washed with a saturated aqueous solution of sodium hydrogen carbonate and then with water three times, and concentrated under reduced pressure. To the resulting residues were added ethyl acetate and hexane, the precipitated solids were collected by filtration, and washed with a mixed solvent of hexane / ethyl acetate (= 4/1 (V/V)) to give the title compound (5.88 g, yield: quantitative) as slightly yellow solids.

Example 25-2

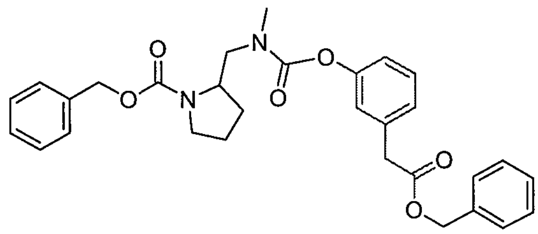

To a solution of the Example 25-1 (149.8 mg, 0.368 mmoL) and benzyl (S)-2-((methylamino)methyl)pyrrolidine-1-carboxylate trifluoroacetate (Angew Chem Int Ed Engl. 2020 Mar 2; 59(10): 4176-41) (112.3 mg, 0.294 mmoL) in dichloromethane (2 mL) in a 30 mL round-bottom flask was added N,N-diisopropylethylamine (0.22 mL, 162.8 mg, 1.260 mmoL) under argon gas flow with stirring at room temperature, and the resulting mixture was stirred at room temperature for 2 hours.

After the reaction was completed, the reaction mixture was concentrated under reduced pressure. The resulting residues were dissolved in N,N-dimethylformamide (5 mL), the resulting solution was subjected to preparative HPLC chromatography under the following conditions, and the fraction comprising the target compound was concentrated under reduced pressure to give the title compound (45.5 mg, yield: 29.91%) as a colorless oil.
MS (ESI) m/z 517 $(M+H)^+$

Column: Waters XBridge Prep C18 5 um ODB 19*150 mm
Flow rate: 17 mL/min.
Elution solvent: 0.1% aqueous solution of formic acid (Solution A) - acetonitrile (Solution B)
Gradient (Solution B): 50% (0.00 min.) → 95% (10.00 min.)

Example 25-3

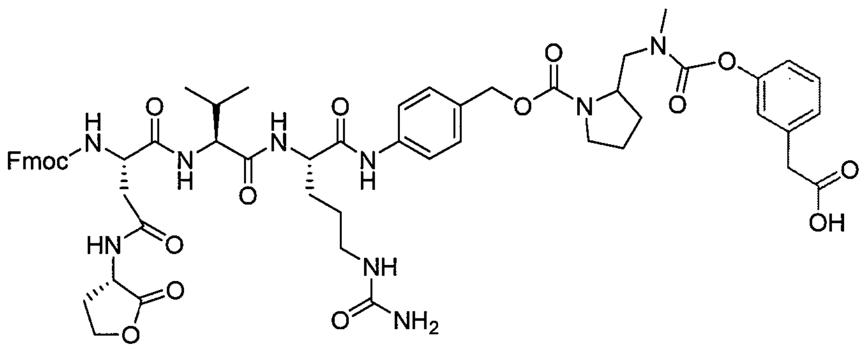

A solution of the Example 25-2 (45.6 mg, 0.088 mmoL), methanesulfonic acid (6.0 μL, 8.88 mg, 0.092 mmol), and 10% palladium carbon NX-Type (wetted with 50% water) (19.9 mg, 9.35 pmoL) in N,N-dimethylformamide (2 mL) in a 30 mL round-bottom flask was stirred under hydrogen atmosphere.

Subsequently, the Example 2-2 (101.1 mg, 0.105 mmoL) and N,N-dimethylformamide (2 mL) were added

thereto, N,N-diisopropylethylamine (61 μL, 45.14 mg, 0.349 mmoL) was added thereto with stirring at room temperature, and the resulting mixture was stirred at room temperature for 2 hours.

The resulting reaction solution was subjected to preparative HPLC chromatography under the following conditions, and the fraction comprising the target compound was concentrated under reduced pressure to give the title compound (24.6 mg, yield: 24.92%) as a colorless oil.
MS (ESI) m/z 1119 $(M+H)^+$

Column: Waters XBridge Prep C18 5 μm ODB 19*150 mm
Flow rate: 17 mL/min.
Elution solvent: 0.1% aqueous solution of formic acid (Solution A) - 0.1% solution of formic acid in acetonitrile (Solution B)
Gradient (Solution B): 30% (0.00 min.) → 80% (10.00 min.)

Example 25-4

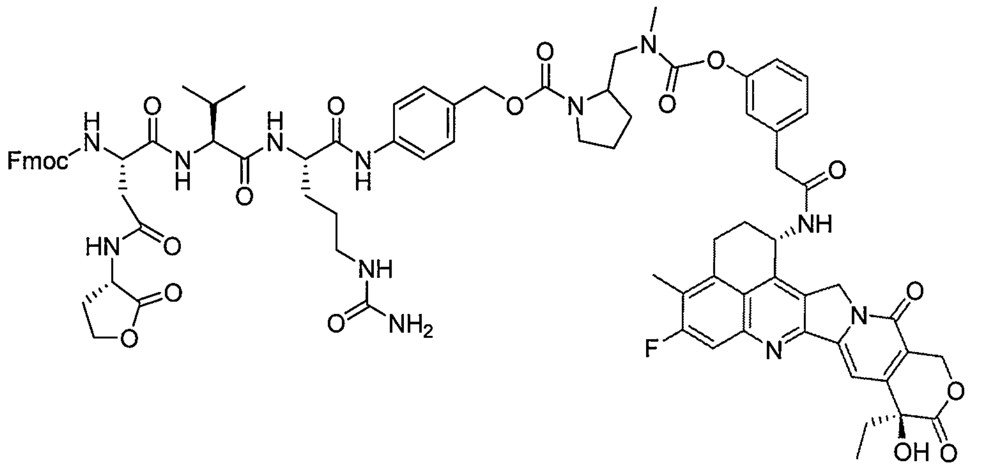

To a solution of the Example 25-3 (23.6 mg, 0.021 mmoL) and exatecan mesylate (10.5 mg, 0.020 mmoL) in N,N-dimethylformamide (2 mL) in a 30 mL round-bottom flask were added triethylamine (8 μL, 5.81 mg, 0.057 mmoL) and HATU (8.6 mg, 0.023 mmoL), and the resulting mixture was stirred at room temperature for 2 hours.

The resulting reaction solution was subjected to preparative HPLC chromatography under the following conditions, and the fraction comprising the target compound was concentrated under reduced pressure to give the title compound (26.2 mg, yield: 86.37%) as a colorless oil.
MS (ESI) m/z 769 $(M+2H)^{2+}$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 40% (0.00 min.) -> 80% (8.00 min.)

Example 25

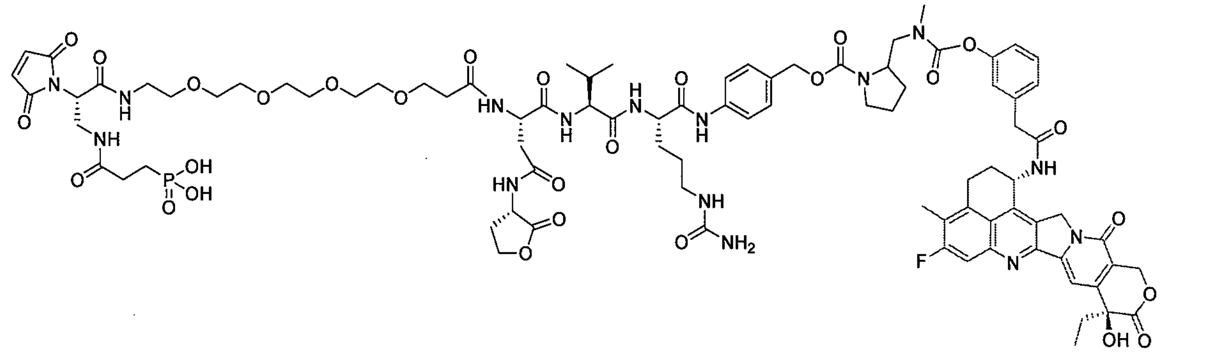

The Example 3-6 (11.9 mg, 0.021 mmoL) and the Example 25-4 (10.0 mg, 6.51 pmoL) instead of the Example 9-2 were reacted in the same manner as the Example 9 to give the title compound (3.8 mg, yield: 30.91%) as colorless solids. MS (ESI) m/z 932 $(M+2H)^{2}$

Synthesis of Example 26

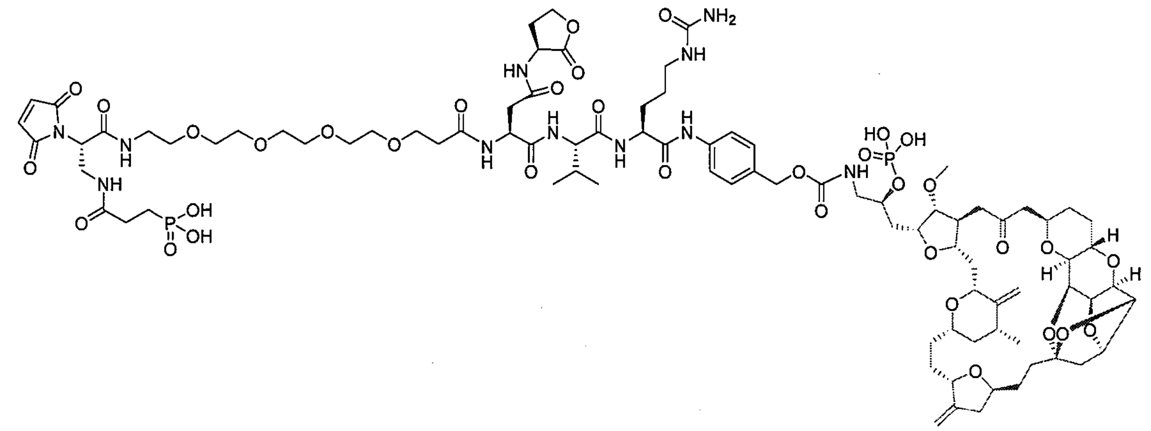

Example 26-1

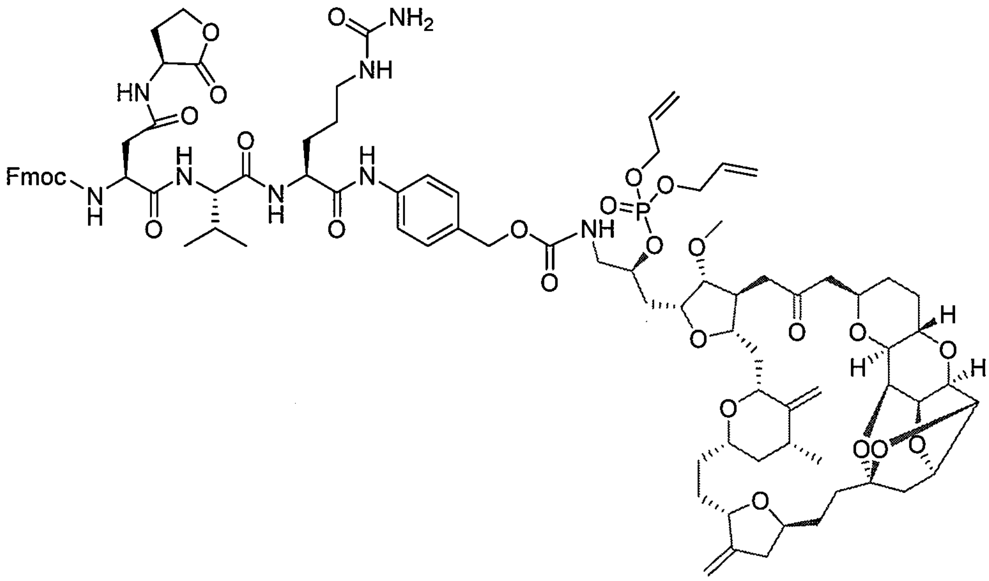

To a solution of the Example 14-1 (19.2 mg, 0.012 mmoL) in N,N-dimethylformamide (0.8 mL) in a 5 mL sample tube was added diallyl N,N-diisopropylphosphoramidite (49 μL, 45.47 mg, 0.185 mmoL) under argon atmosphere with stirring, and then added a solution of 1H-tetrazole (13.0 mg, 0.186 mmoL) in N,N-dimethylformamide (0.2 mL) at room temperature, and the resulting mixture was stirred at room temperature for 1 hour.

Subsequently, 30% by weight of hydrogen peroxide water (19 μL, 21.09 mg, 0.186 mmoL) was added thereto, and the resulting mixture was stirred at room temperature for 1 hour.

The resulting reaction solution was subjected to preparative HPLC chromatography under the following conditions, and the fraction comprising the target compound was freeze-dried to give the title compound (10.2 mg, yield: 48.17%) as colorless foam.

MS (ESI) m/z 859 $(M+2H)^2$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 50% (0.00 min.) → 95% (10.00 min.)

Example 26-2

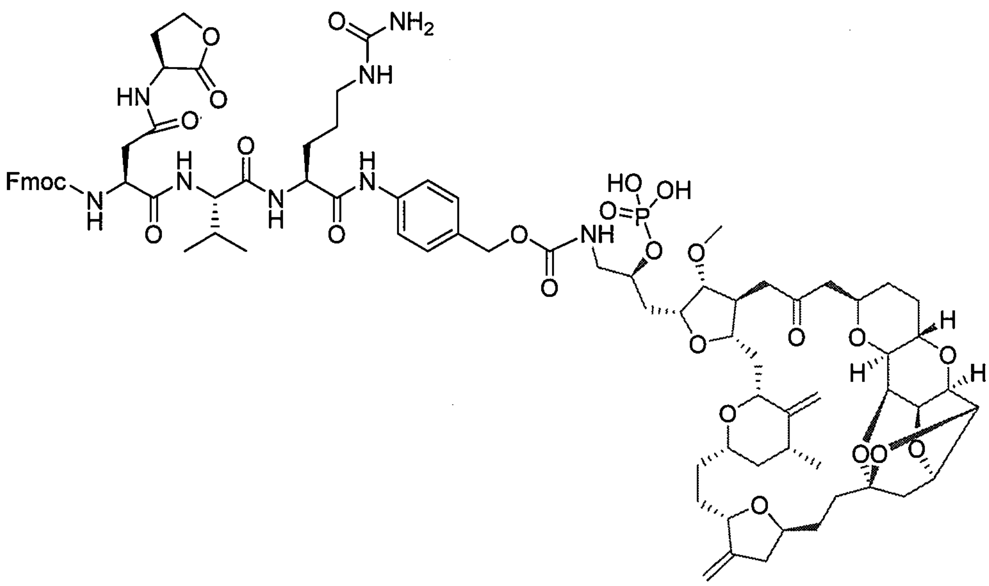

To a solution of the Example 26-1 (15.1 mg, 8.80 pmoL, including those produced by the same method as the Example 26-1) in N,N-dimethylformamide (0.5 mL) in a 5 mL sample tube were sequentially added N-methylaniline (3.5 μL, 3.47 mg, 0.032 mmoL) and tetrakis(triphenylphosphine)palladium (2.0 mg, 1.731 pmoL) under argon atmosphere with stirring at room temperature, and the resulting mixture was stirred at room temperature for 2 hours.

The resulting reaction solution was subjected to preparative HPLC chromatography under the following conditions, and the fraction comprising the target compound was freeze-dried to give the title compound (9.3 mg, yield: 64.61%) as colorless solids.
MS (ESI) m/z 819 $(M+2H)^2$

Column: Waters XBridge Prep C18 5 μm ODB 19*150 mm
Flow rate: 17 mL/min.
Elution solvent: 0.1% aqueous solution of formic acid (Solution A) - acetonitrile (Solution B)
Gradient (Solution B): 30% (0.00 min.) → 70% (8.00 min.)

Example 26

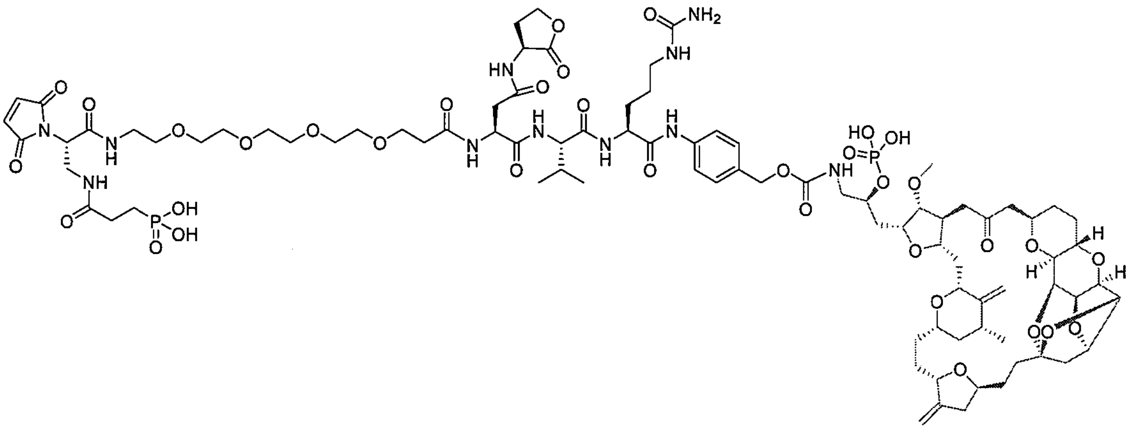

The Example 3-6 (11.9 mg, 0.021 mmoL) and the Example 26-2 (9.3 mg, 5.69 pmoL) instead of the Example 9-2 were reacted in the same manner as the Example 9 to give the title compound (5.4 mg, yield: 48.39%) as colorless solids.
MS (ESI) m/z 982 $(M+2H)^2$

Synthesis of Example 27

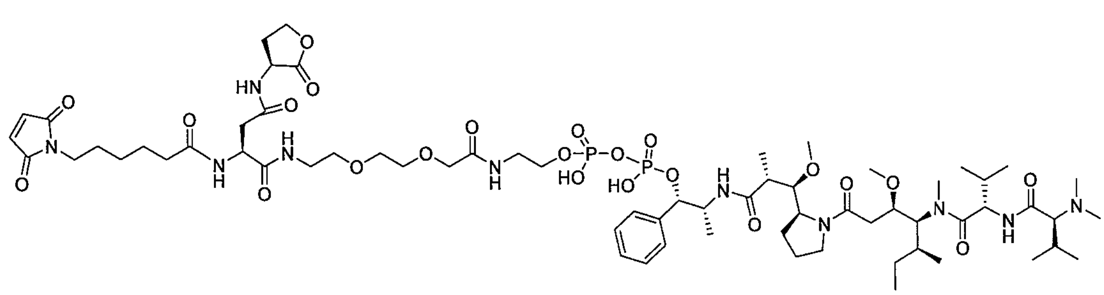

N-succinimidyl 6-maleimidohexanoate (9.7 mg, 0.031 mmoL) instead of N-succinimidyl 1-maleimide-3-oxo-7,10,13,16-tetraoxa-4-azanonadecanoate and the Example 19-10 (20 mg, 0.016 mmoL) instead of the Example 1-7 were reacted in the same manner as the Example 1 to give the title compound (11.1 mg, yield: 48.21%) as colorless solids.
MS (DUIS) m/z 1469 $(M-H)^-$

Synthesis of Example 28

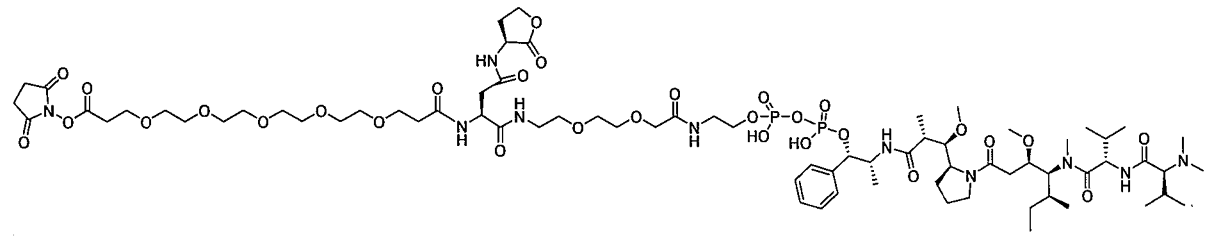

Bis(2,5-dioxopyrrolidin-1-yl) 4,7,10,13,16-pentaoxanonadecanedioate (16.6 mg, 0.031 mmoL) instead of N-succinimidyl 1-maleimide-3-oxo-7,10,13,16-tetraoxa-4-azanonadecanoate and the Example 19-10 (20 mg, 0.016 mmoL) instead of the Example 1-7 were reacted in the same manner as the Example 1 to give the title compound (3.9 mg, yield: 14.85%) as colorless solids.
MS (ESI) m/z 847 $(M-2H)^{2-}$

Synthesis of Example 29

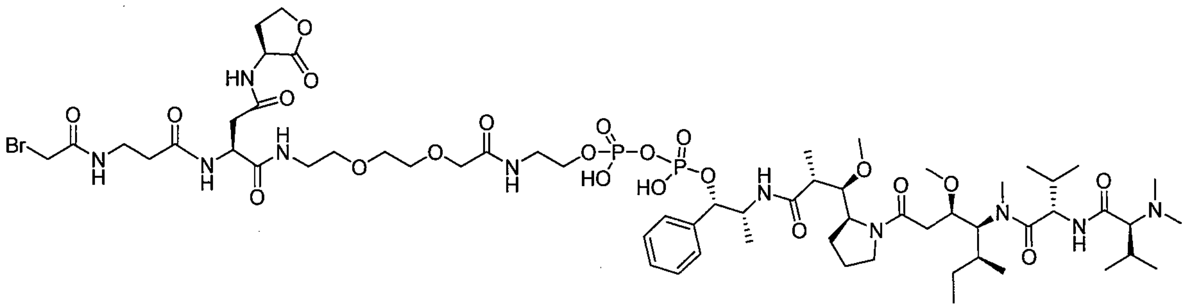

N-succinimidyl 3-(bromoacetoamide)propionate (9.61 mg, 0.031 mmoL) instead of N-succinimidyl 1-maleimide-3-oxo-7,10,13,16-tetraoxa-4-azanonadecanoate and the Example 19-10 (20 mg, 0.016 mmoL) instead of the Example 1-7 were reacted in the same manner as the Example 1 to give the title compound (3.9 mg, yield: 16.95%) as white solids.
MS (DUIS) m/z 736 $(M+2H)^{2+}$

When the "-L-D" moiety and the "-$L^1$-$D^1$" moiety of the conjugate precursor (I) and the synthetic intermediate (II) of conjugate precursor respectively of the present invention comprise a partial structure having an asymmetric center and optical isomers may be present, all optical isomers are encompassed by the present invention.

Further, the present invention also encompasses compounds labeled with various radioactive or nonradioactive isotopes. One or more atom(s) constituting the conjugate precursor (I) and the synthetic intermediate of conjugate precursor of the present invention may also comprise unnatural ratio(s) of atomic isotope(s). Examples of the atomic isotope include deuterium ($^2H$), tritium ($^3H$), iodine-125 ($^{125}I$), and carbon-14 ($^{14}C$). Also, the conjugate precursor (I) and the synthetic intermediate (II) of conjugate precursor of the present invention may be radioactively labeled with radioactive isotopes such as tritium ($^3H$), iodine-125 ($^{125}I$), and carbon-14 ($^{14}C$). All isotope variants of the conjugate precursor (I) and the synthetic intermediate (II) of conjugate precursor of the present invention are encompassed within the scope of the present invention, regardless of whether they are radioactive or not.

[Reference Examples]: Production of antibody-drug conjugate (ADC) by reacting conjugate precursor with antibody (Reference Example 1), and ADC evaluation test (Reference Example 2)

Reference Example 1. Production of antibody-drug conjugate (ADC)

An ADC produced by reacting a conjugate precursor (I) of the present invention with an "antibody or modified antibody" is represented by the following general formula (III) .

A-(B-$Z^2$-L-D)$_a$ general formula (III)

In the above general formula (III), "A-" is a residue of the above defined antibody, "-B-" is a "divalent residue derived from a functional group in antibody" produced by the reaction of "the above defined functional group in antibody" with "the above defined reactive group", $Z^2$ is a "divalent residue derived from a reactive group" produced by the reaction of "the above reactive group" with "the above functional group in antibody", L and D are the same as defined above, and a is an integer of 1 to 10.

Specific examples of the ADC produced by reacting a conjugate precursor (I) of the present invention with an antibody are shown as Exemplification 4, but the present invention is not limited to them.

Exemplification 4. "Specific examples of ADC produced by using conjugate precursor (I)"

Exemplification 4-1

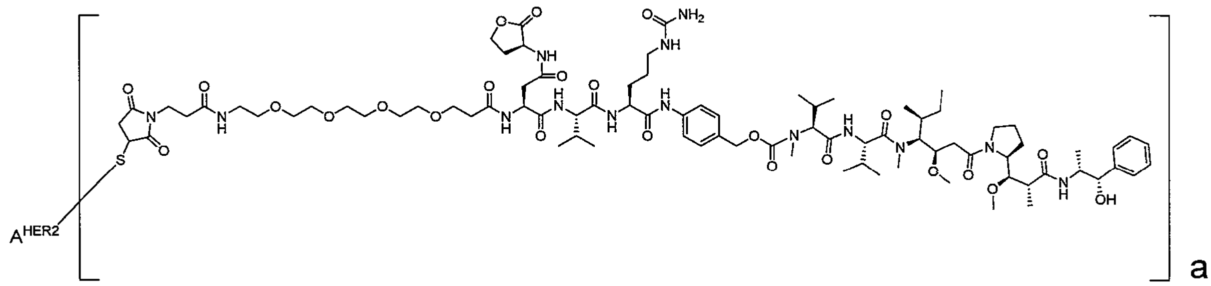

Exemplification 4-2

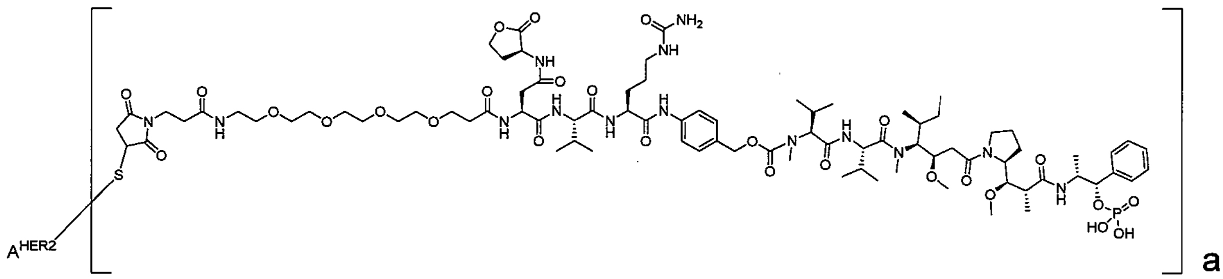

Exemplification 4-3

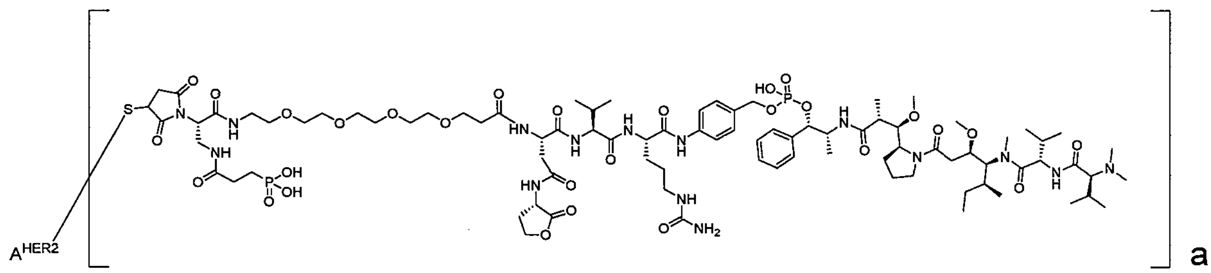

Exemplification 4-3b

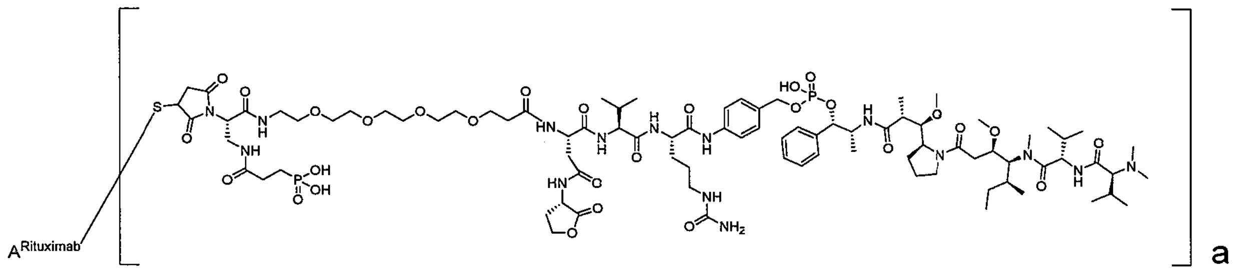

Exemplification 4-3c

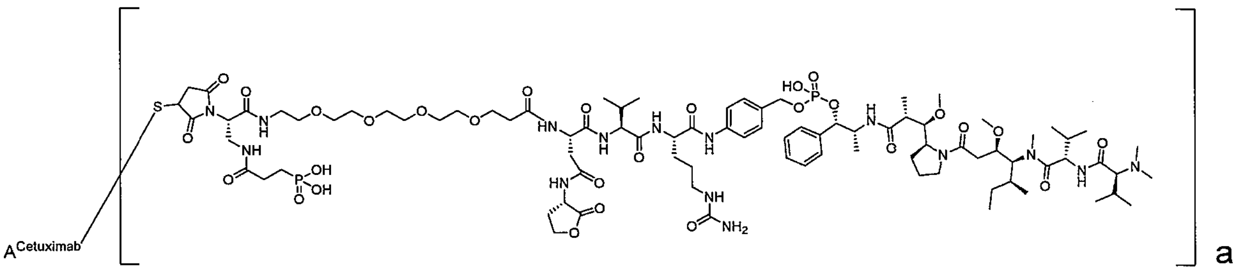

Exemplification 4-4

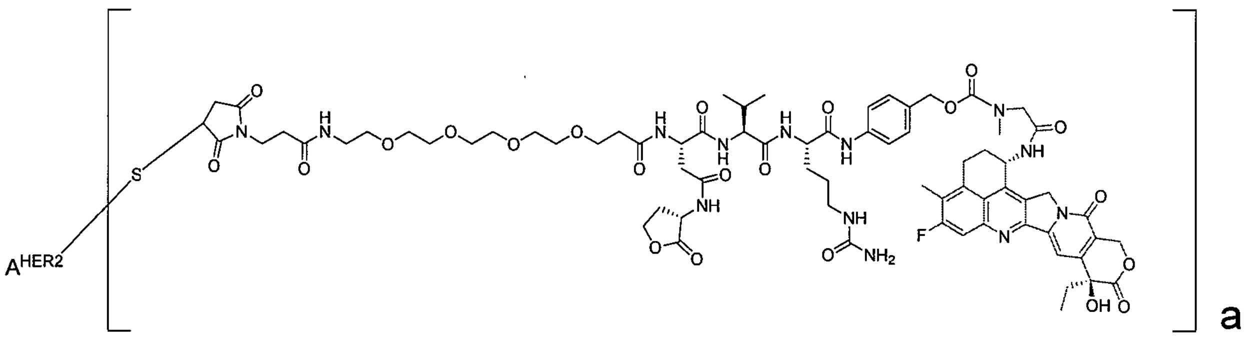

Exemplification 4-5

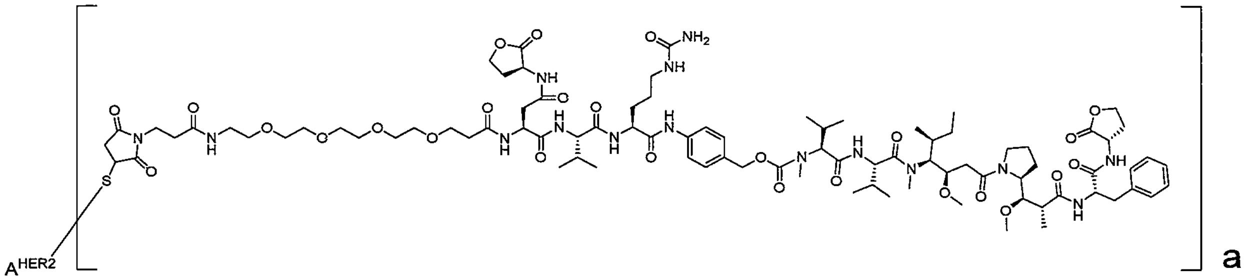

Exemplification 4-6

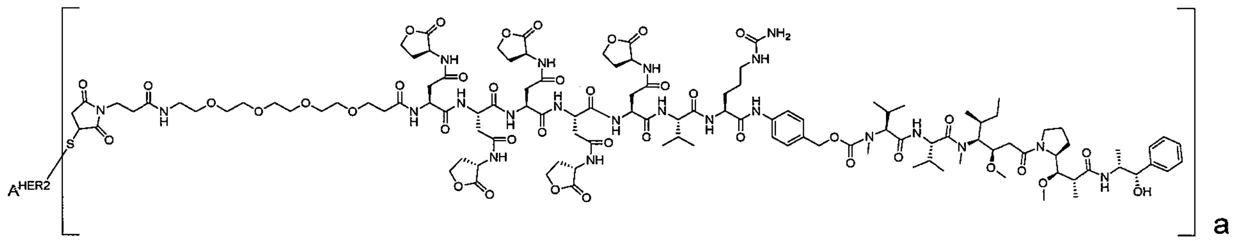

Exemplification 4-7

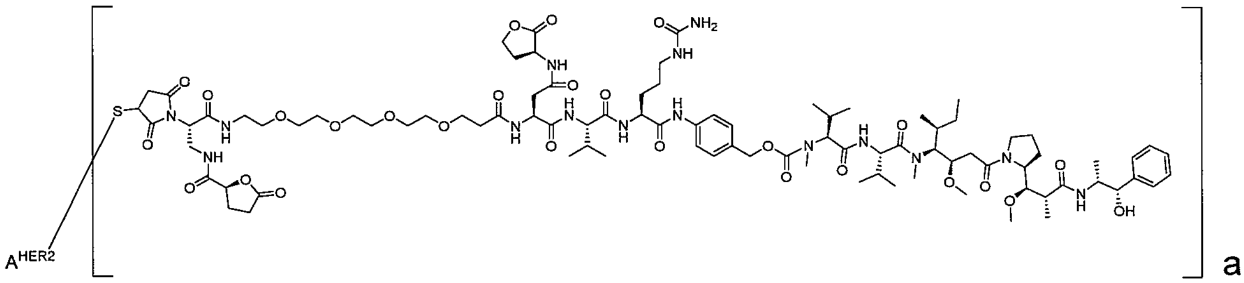

Exemplification 4-8

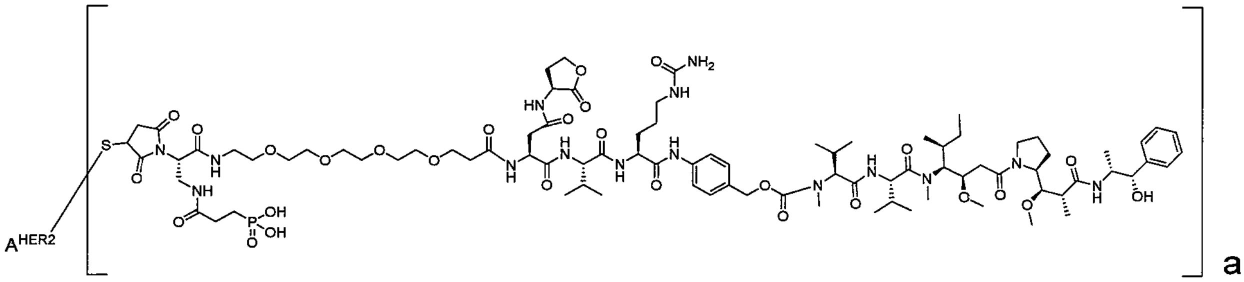

Exemplification 4-9

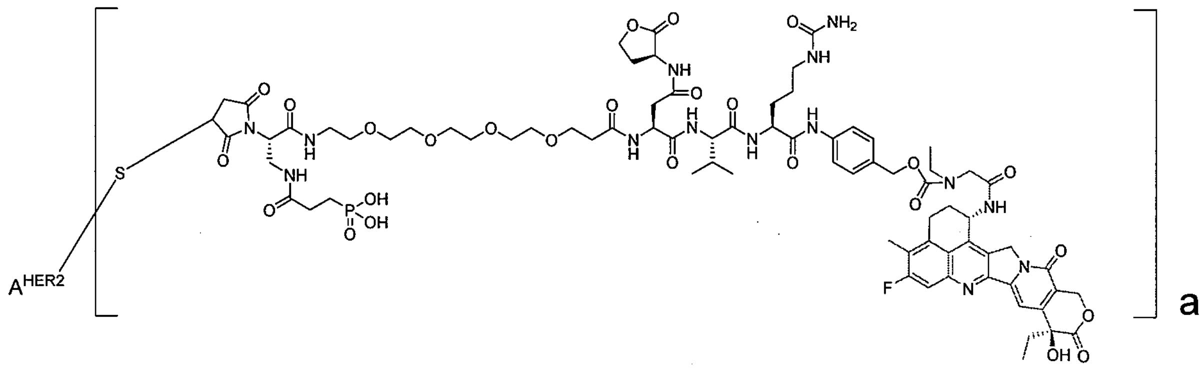

Exemplification 4-10

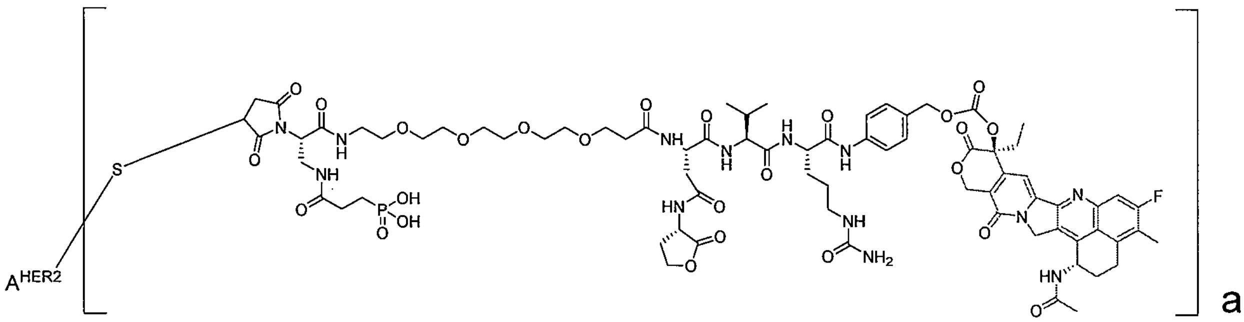

Exemplification 4-11

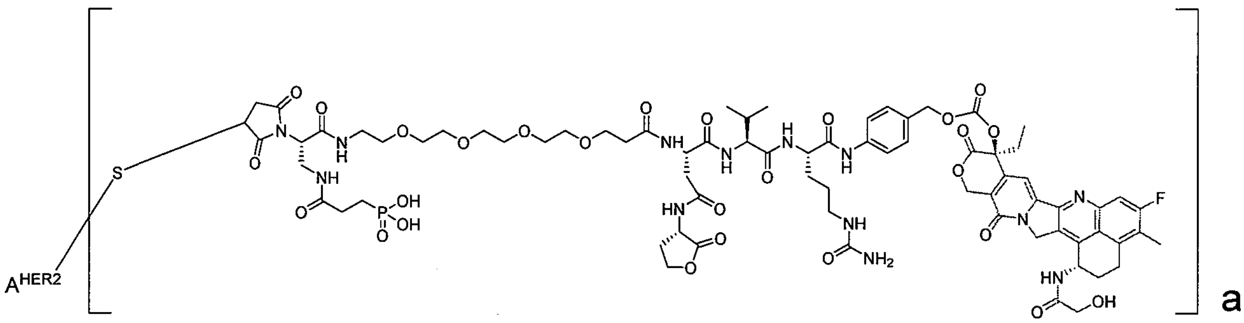

Exemplification 4-12

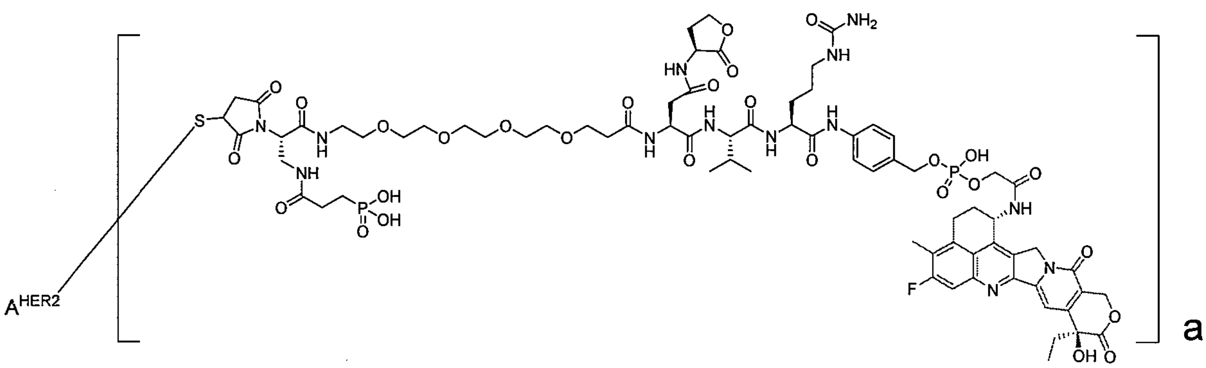

Exemplification 4-13

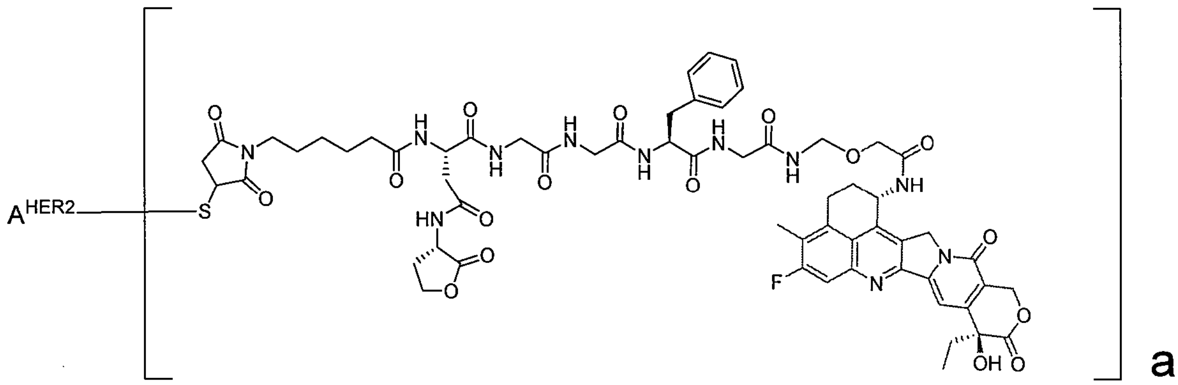

Exemplification 4-14

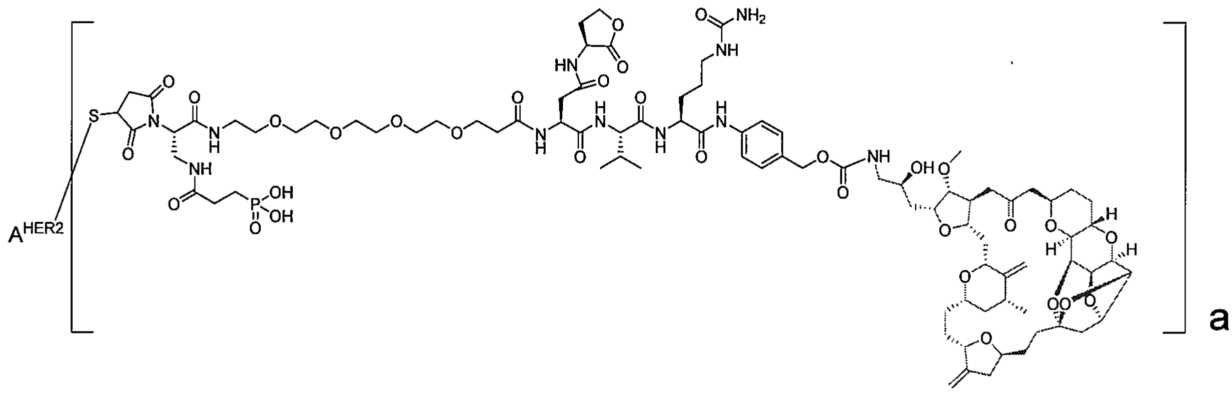

Exemplification 4-15

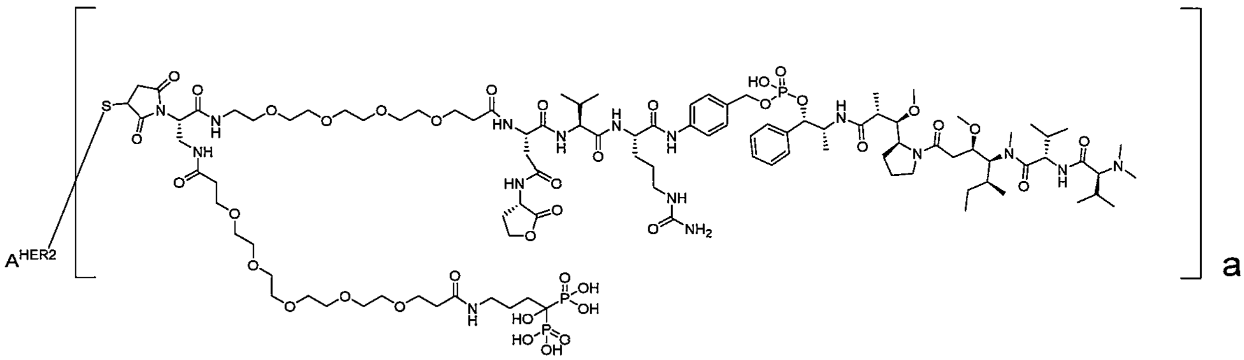

Exemplification 4-16

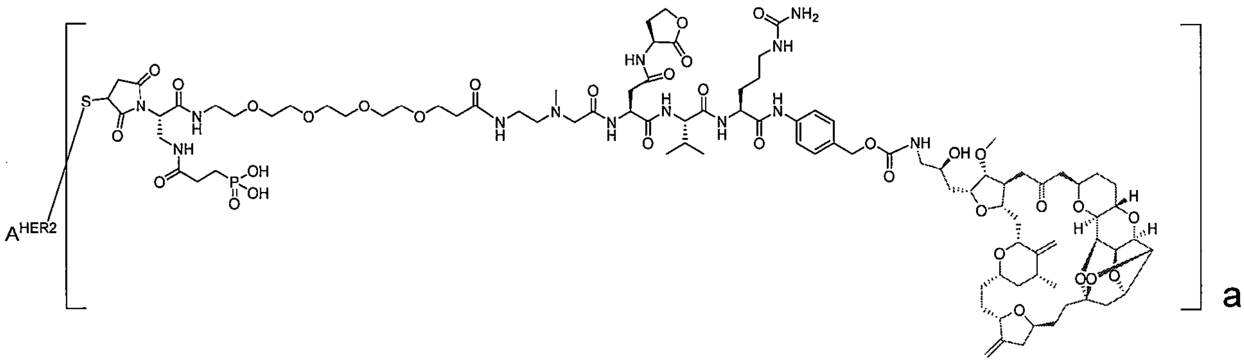

Exemplification 4-17

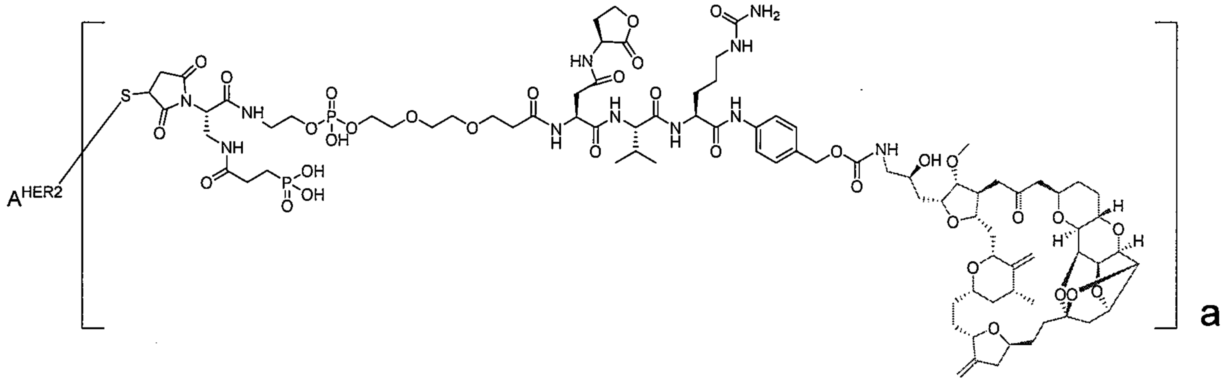

Exemplification 4-18

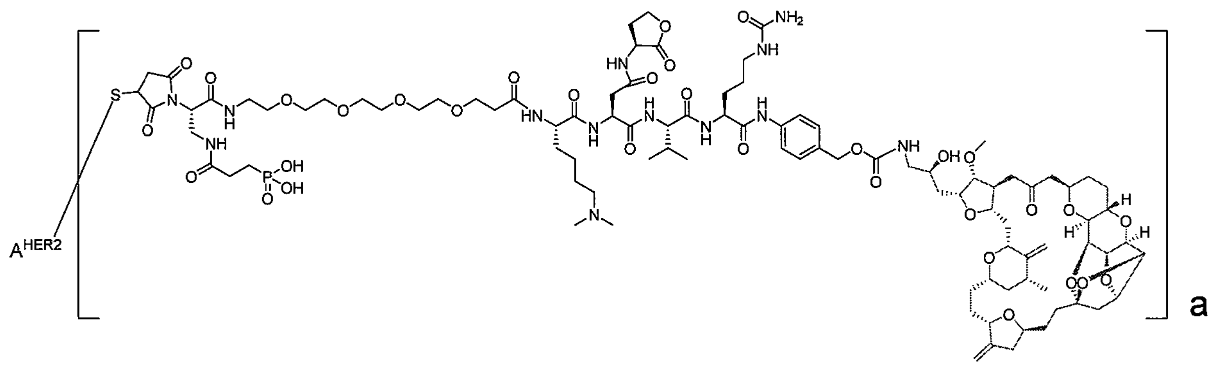

Exemplification 4-19

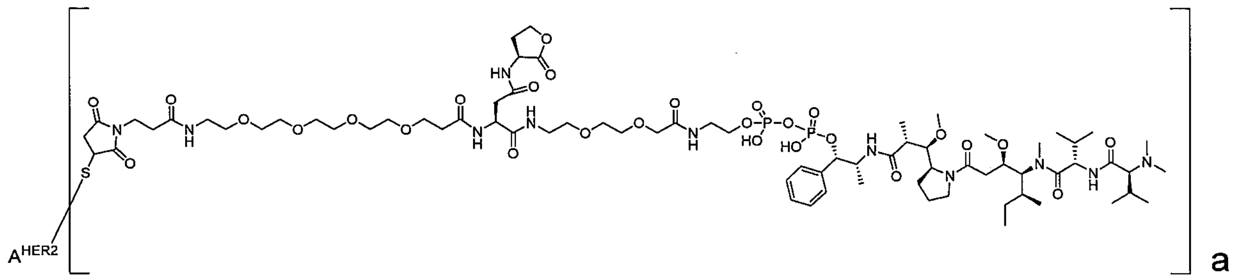

Exemplification 4-20

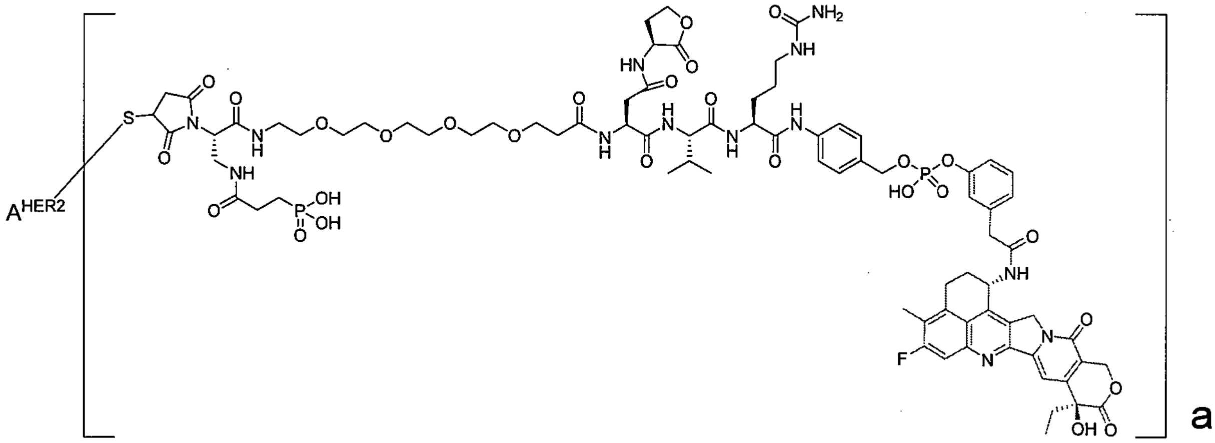

Exemplification 4-21

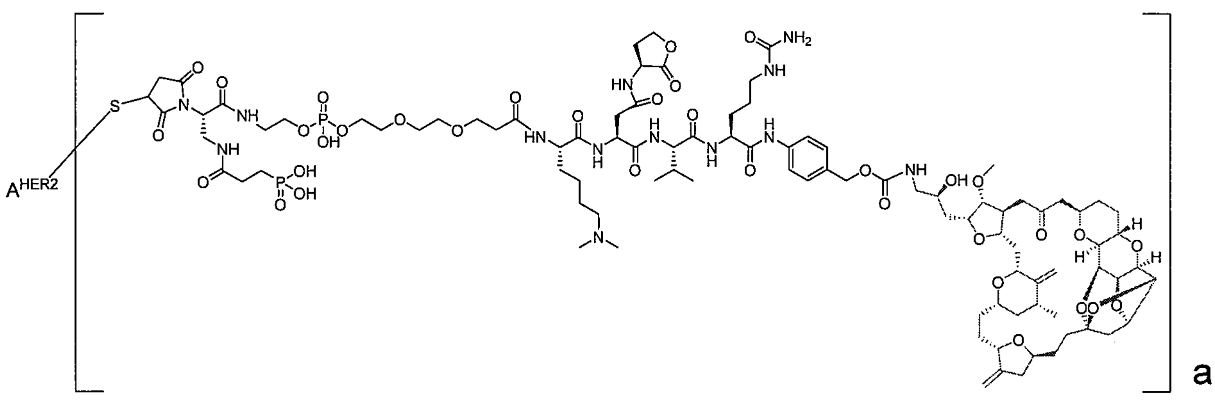

Exemplification 4-22

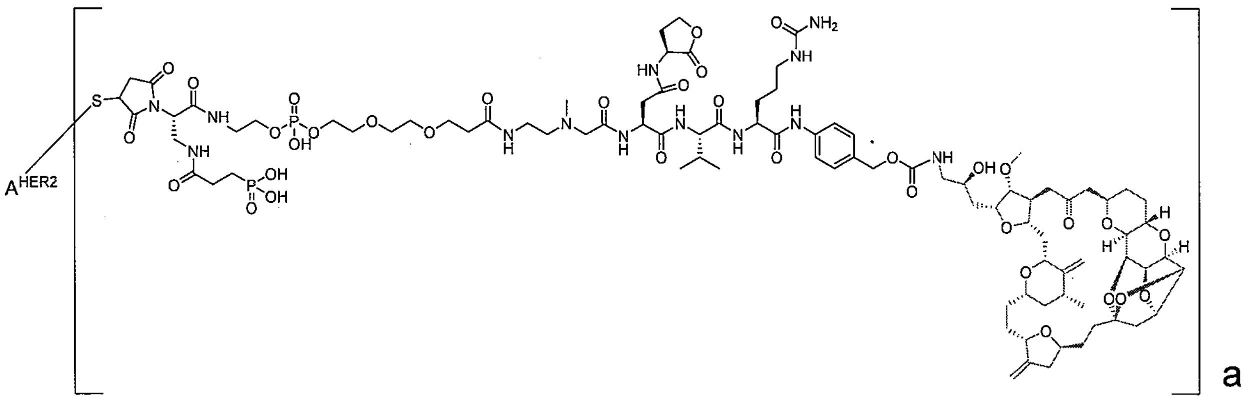

Exemplification 4-23

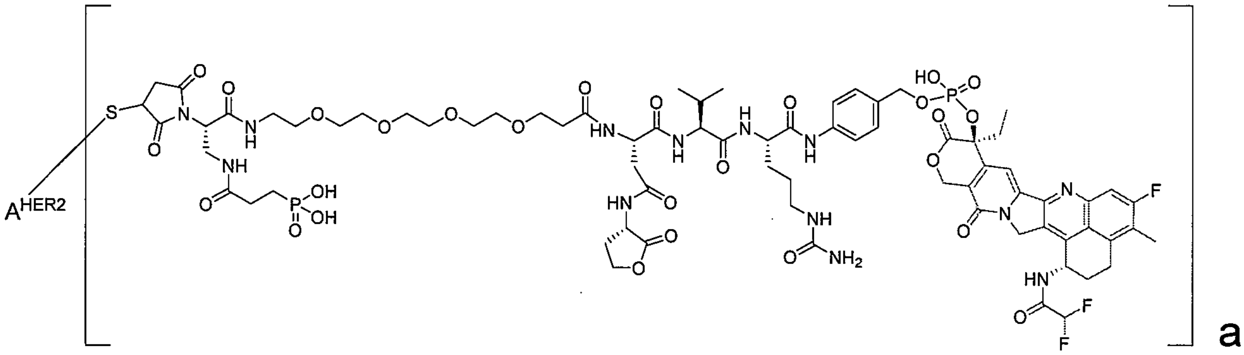

Exemplification 4-24

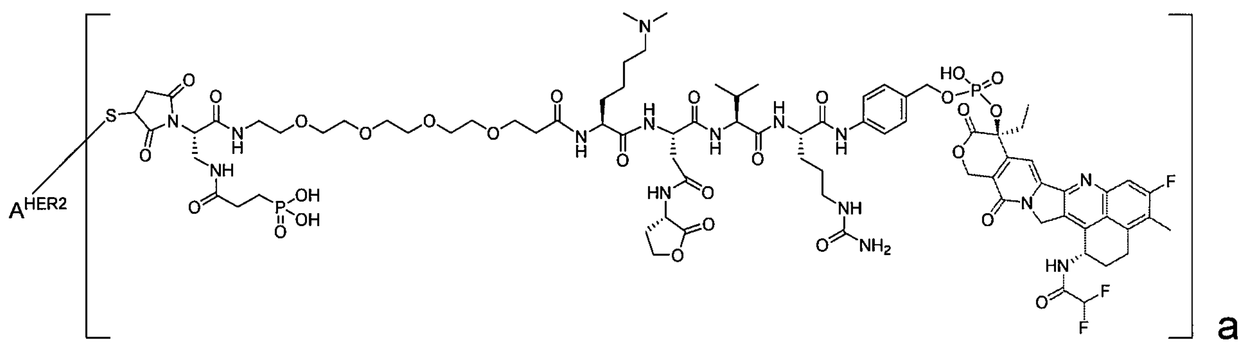

Exemplification 4-25

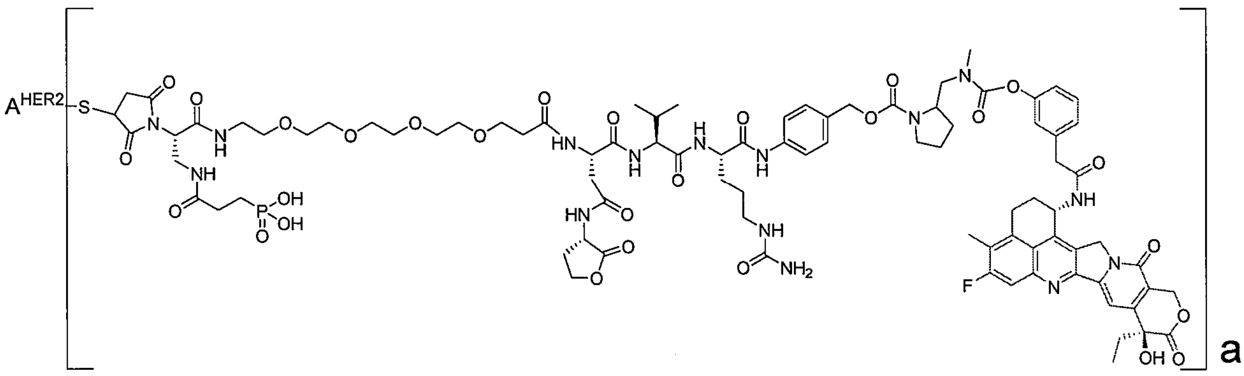

Exemplification 4-26

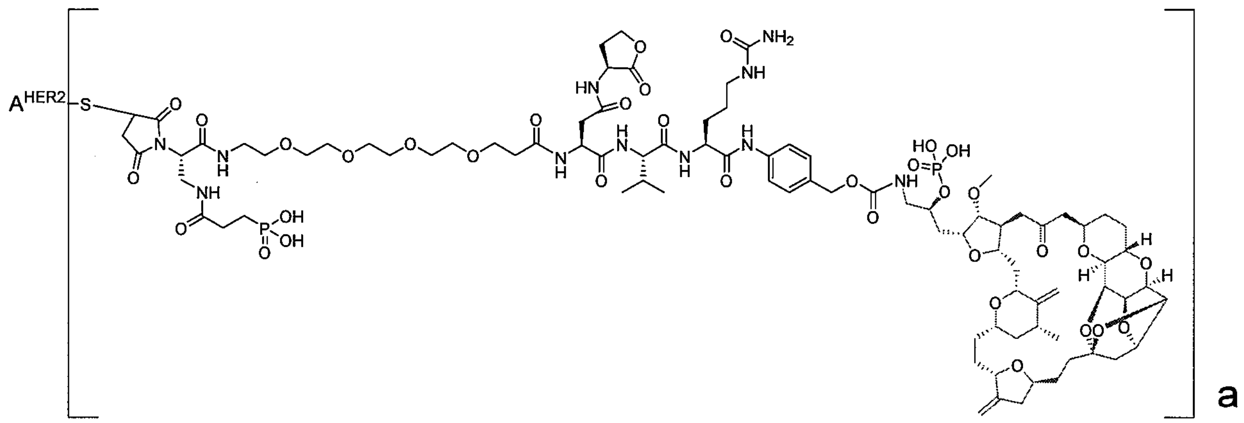

Exemplification 4-27

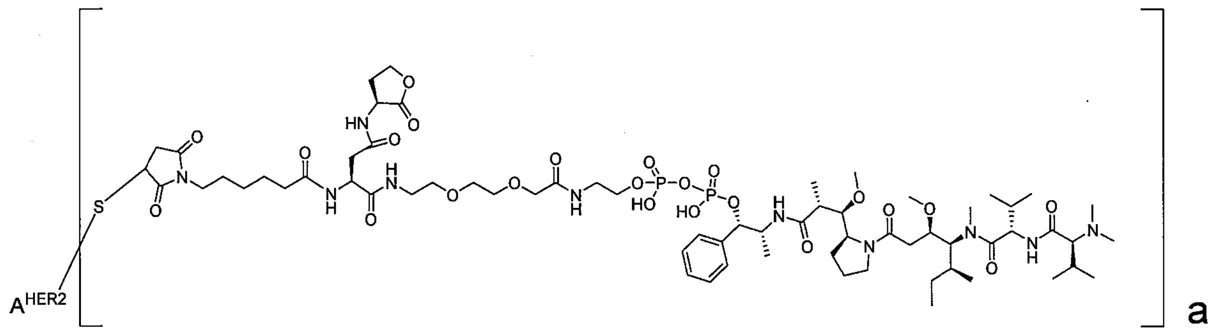

Exemplification 4-28

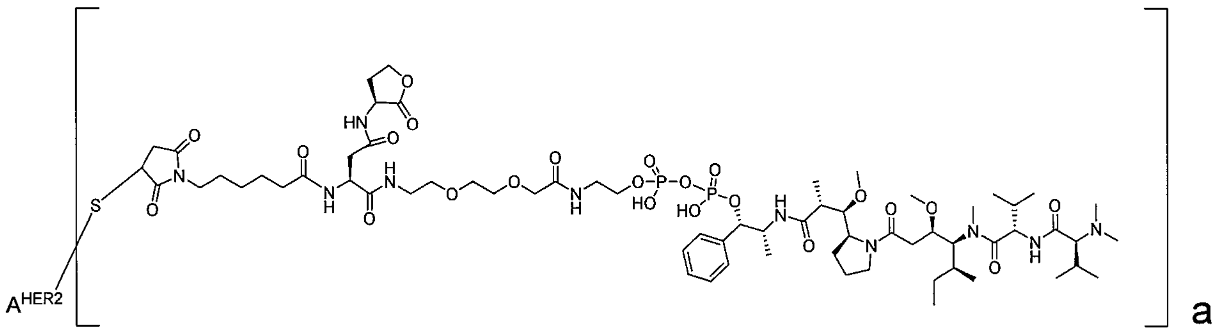

Exemplification 4-29

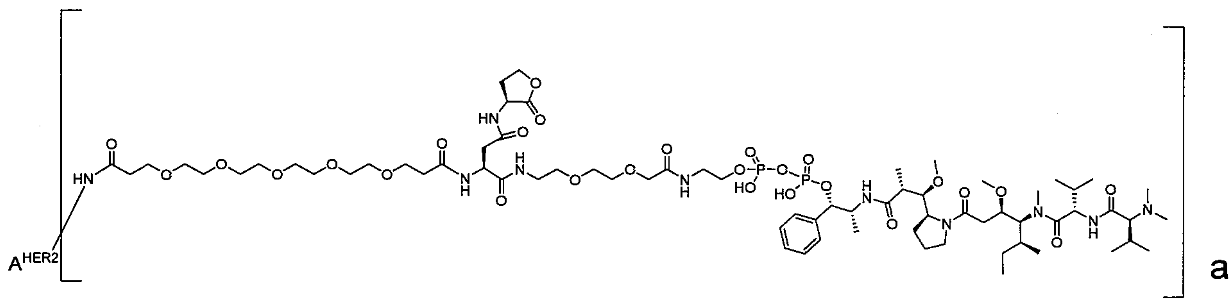

Exemplification 4-30

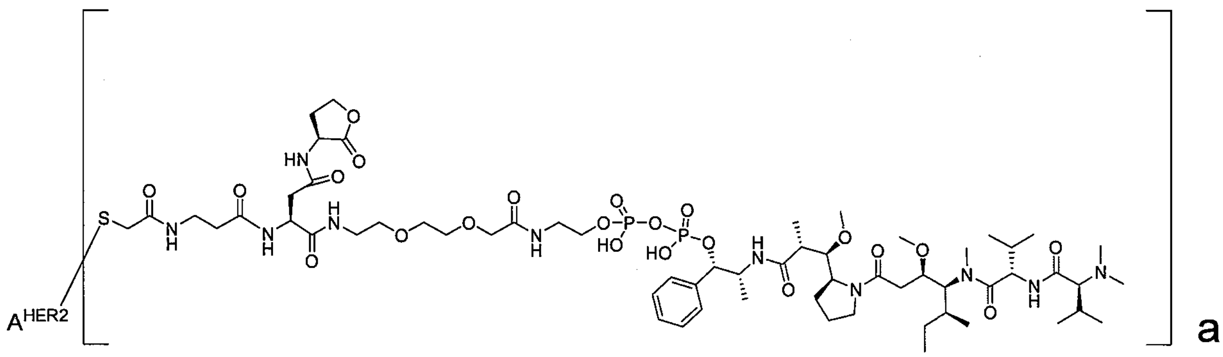

Exemplification 4-31

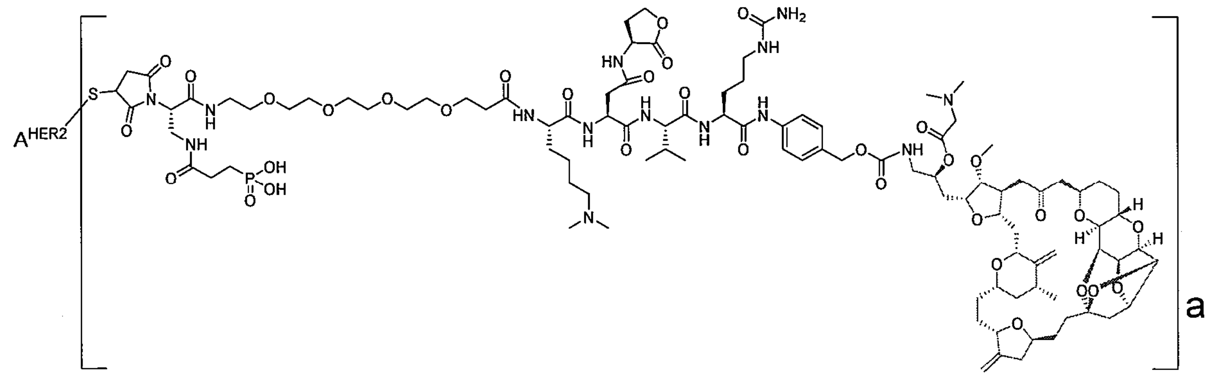

Exemplification 4-32

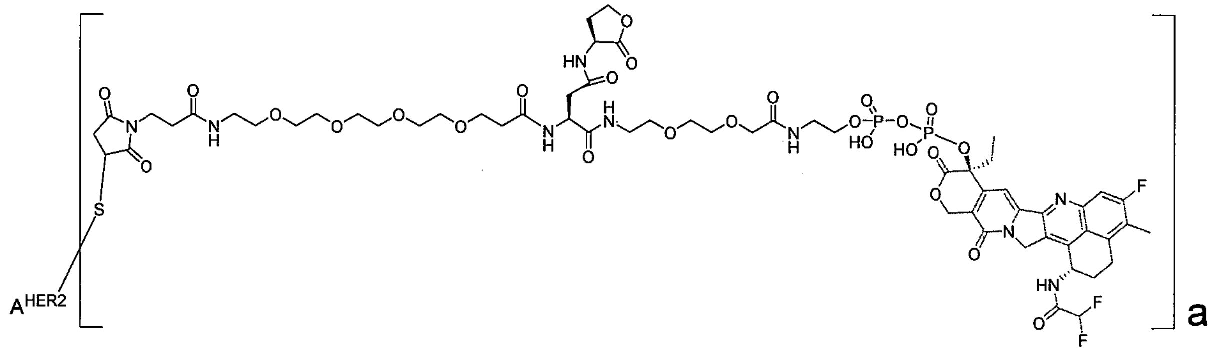

Exemplification 4-33

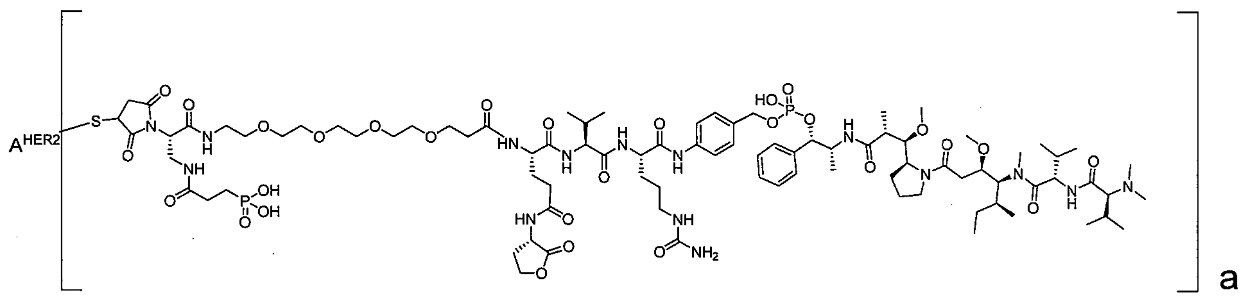

Exemplification 4-34

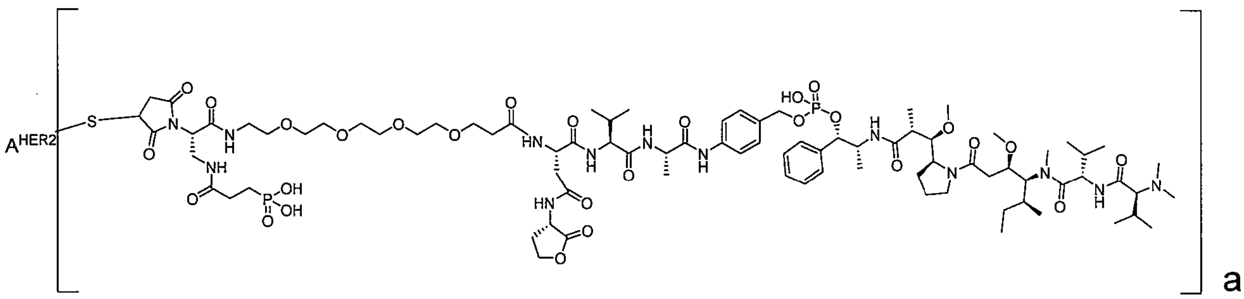

Exemplification 4-35

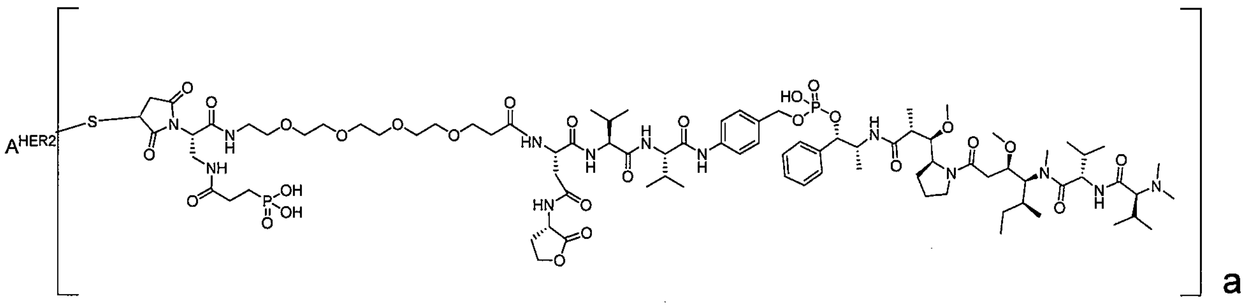

Exemplification 4-36

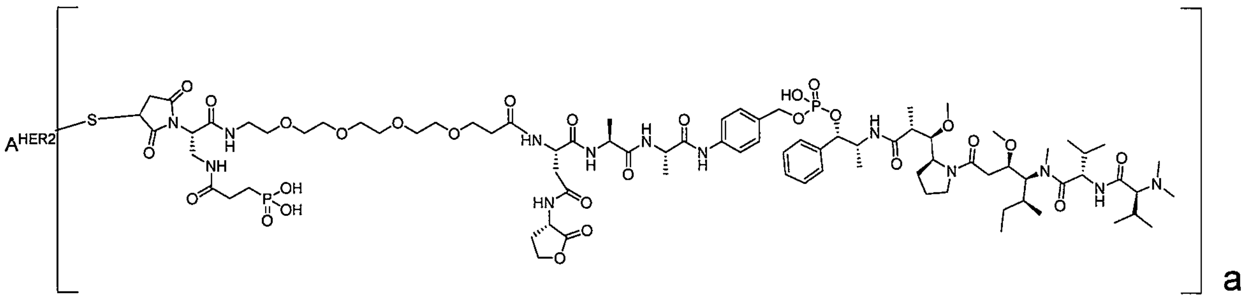

Exemplification 4-37

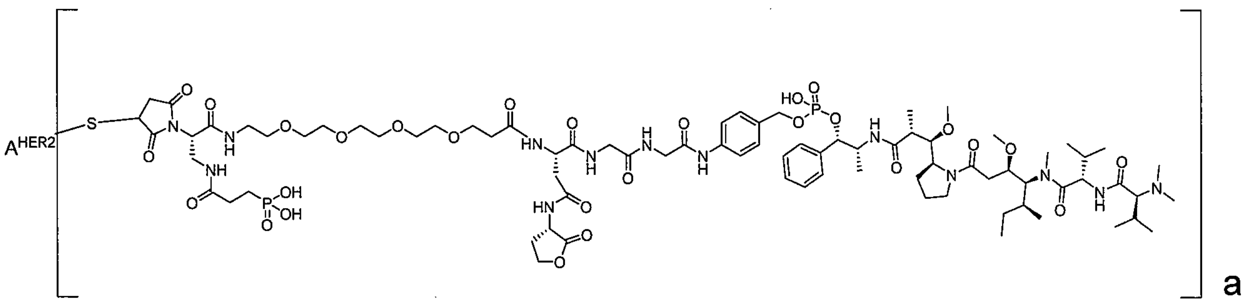

Exemplification 4-38

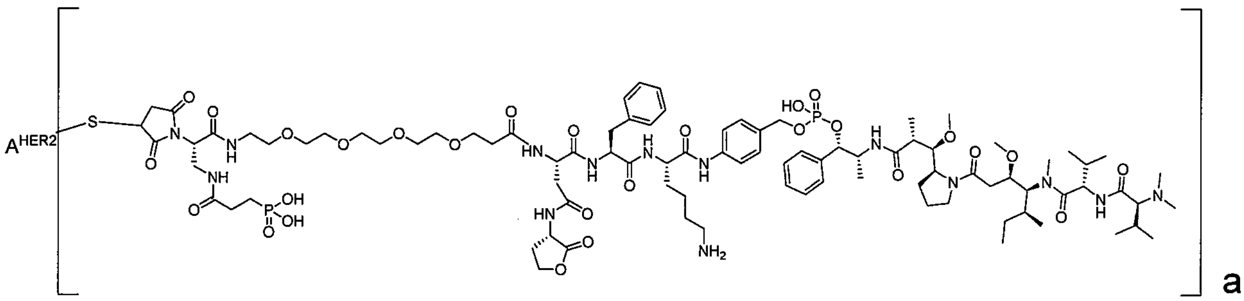

Exemplification 4-39

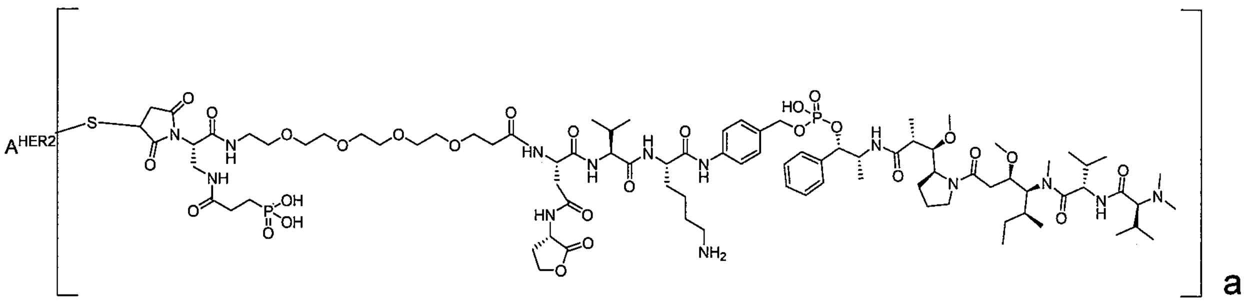

Exemplification 4-40

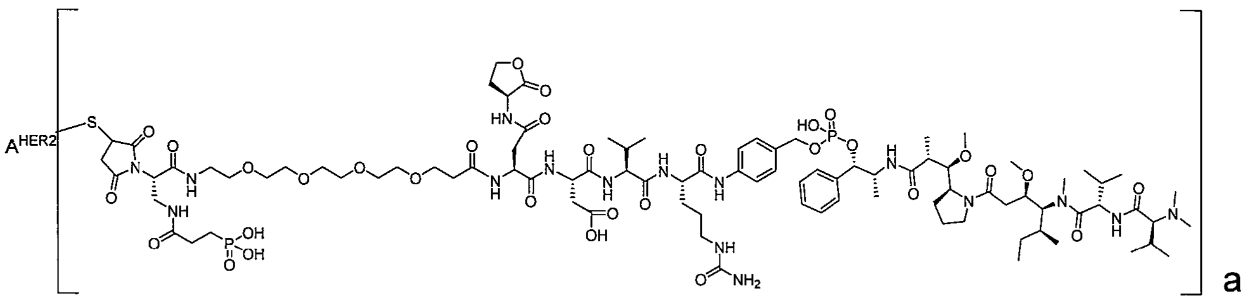

Exemplification 4-41

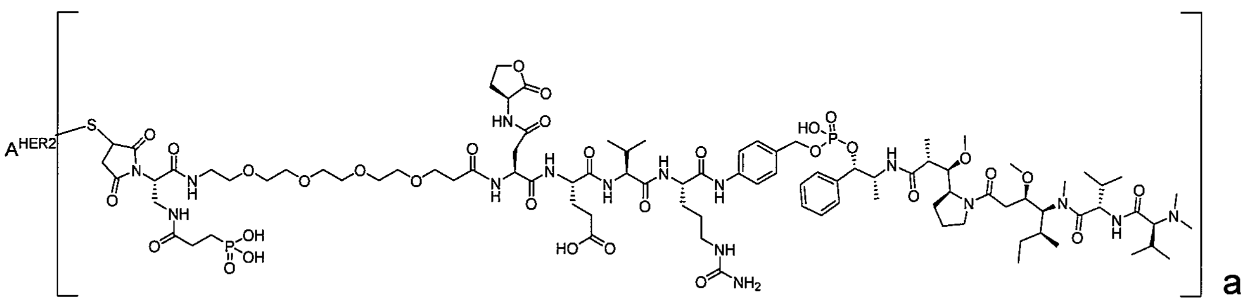

Exemplification 4-42

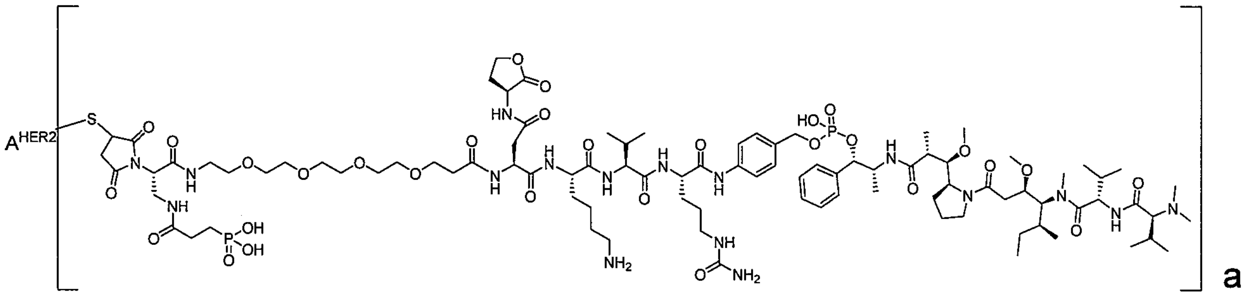

Exemplification 4-43

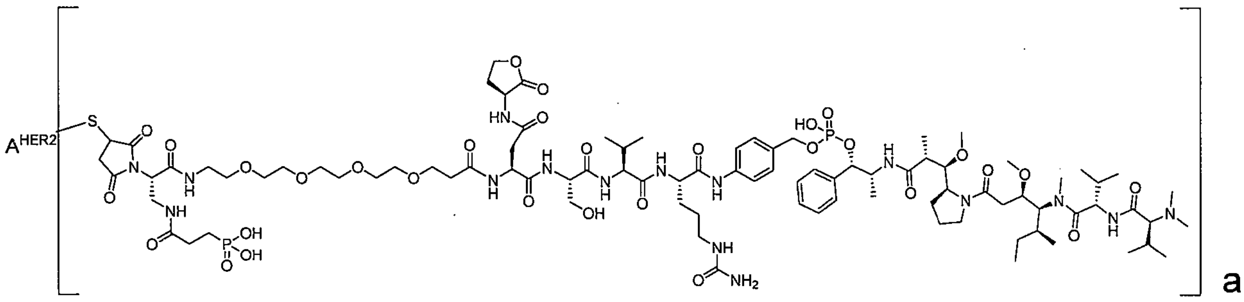

Exemplification 4-44

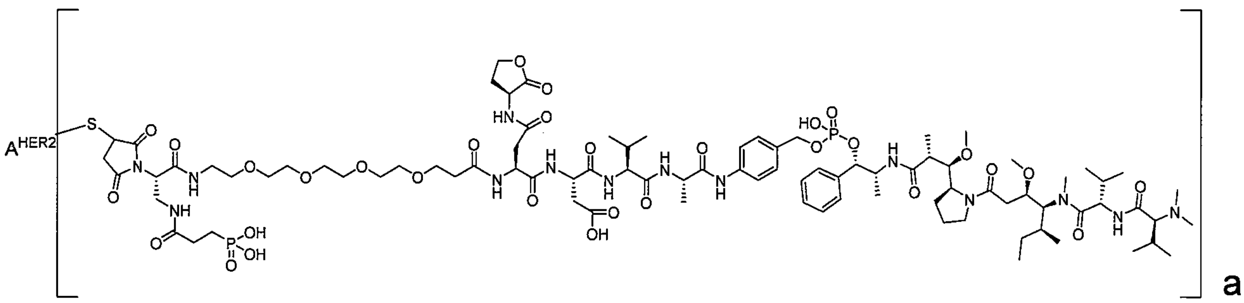

Exemplification 4-45

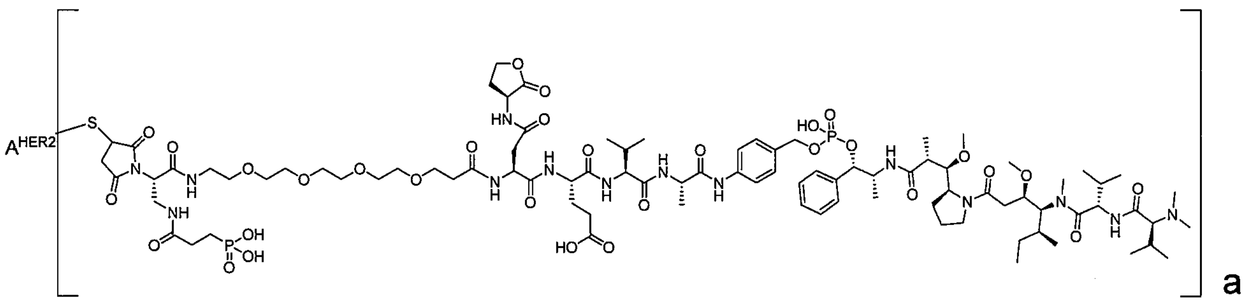

Exemplification 4-46

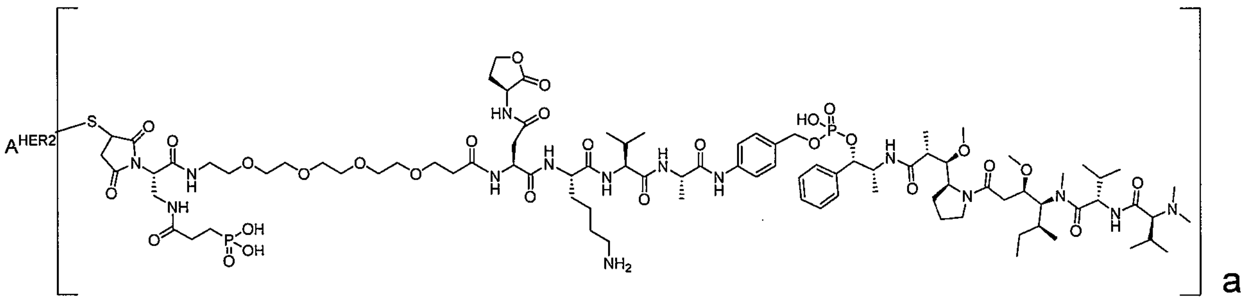

Exemplification 4-47

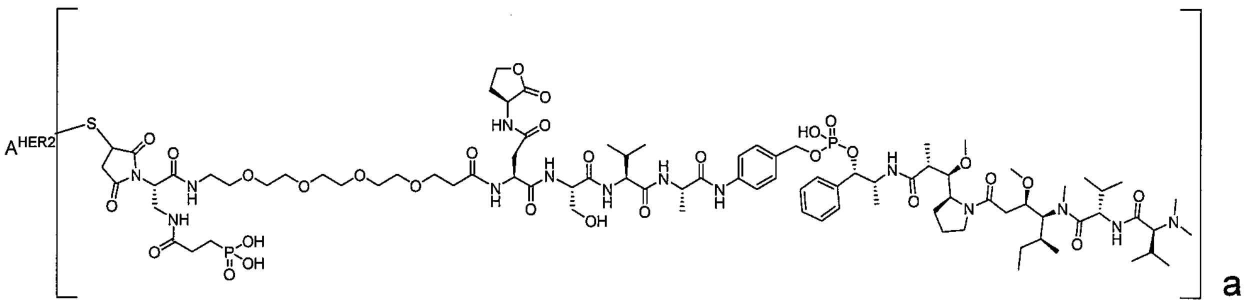

Exemplification 4-48

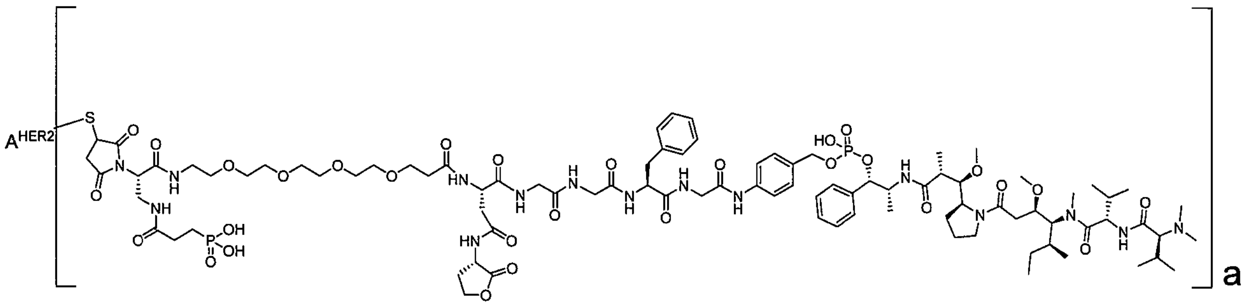

Exemplification 4-49

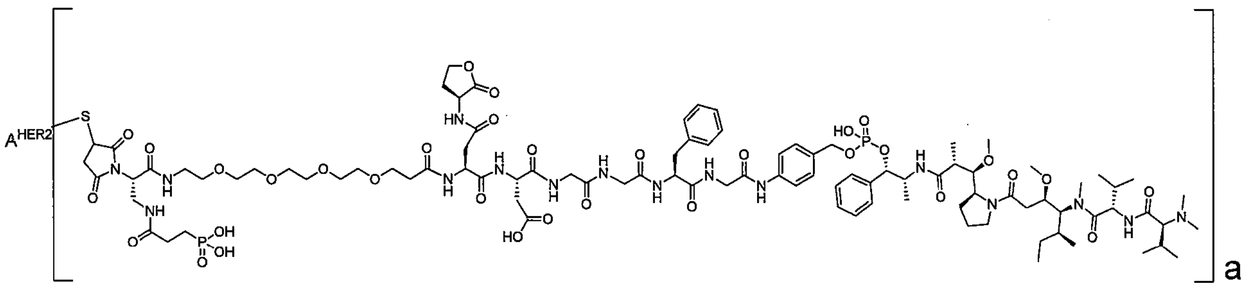

Exemplification 4-50

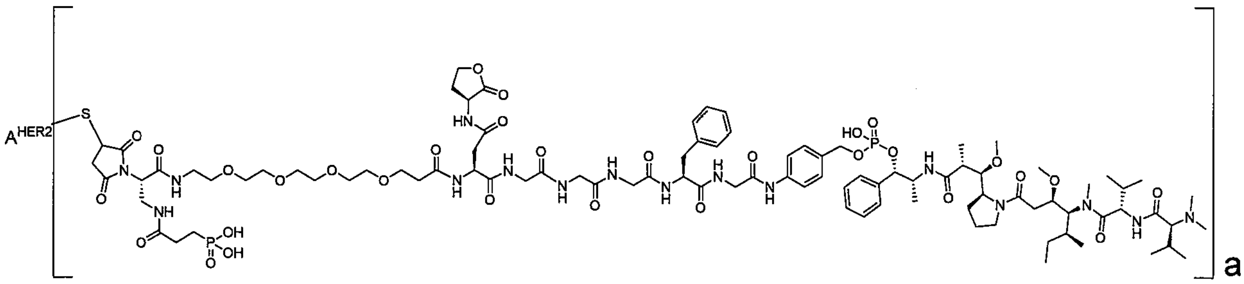

Exemplification 4-51

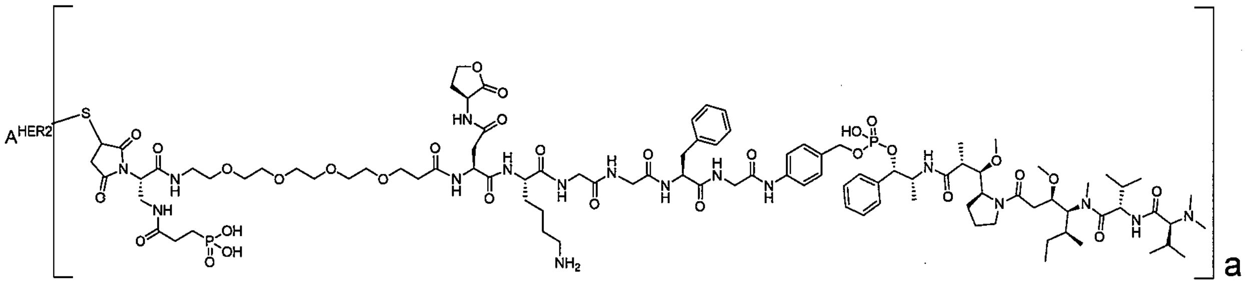

Exemplification 4-52

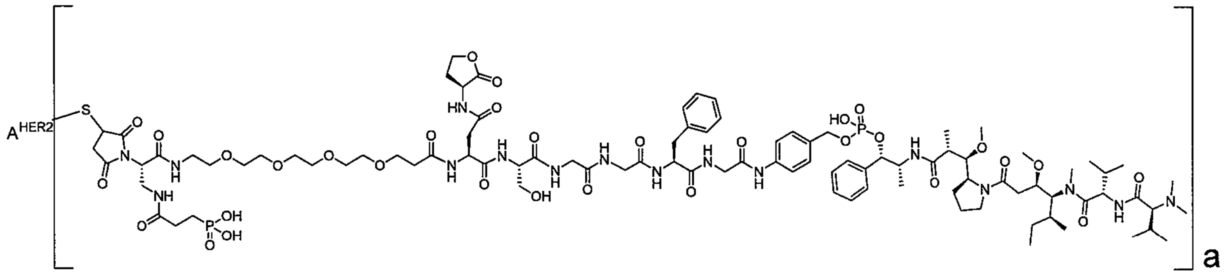

Exemplification 4-53

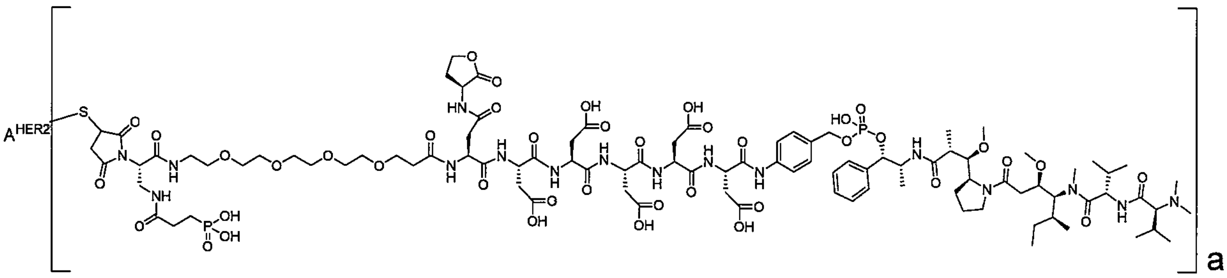

Exemplification 4-54

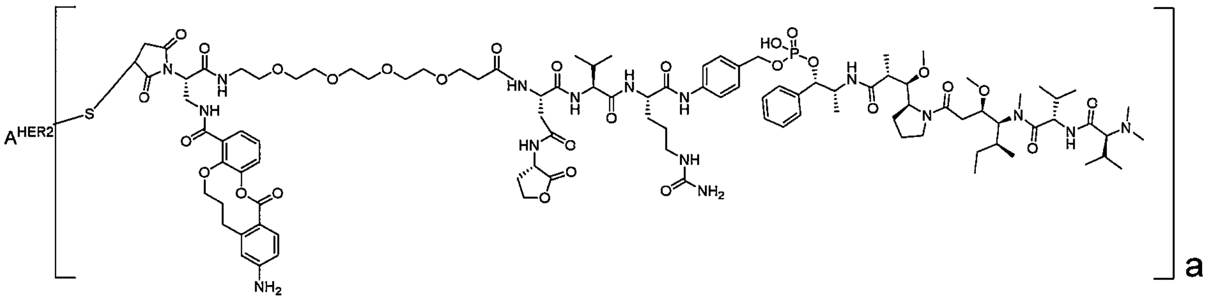

Exemplification 4-55

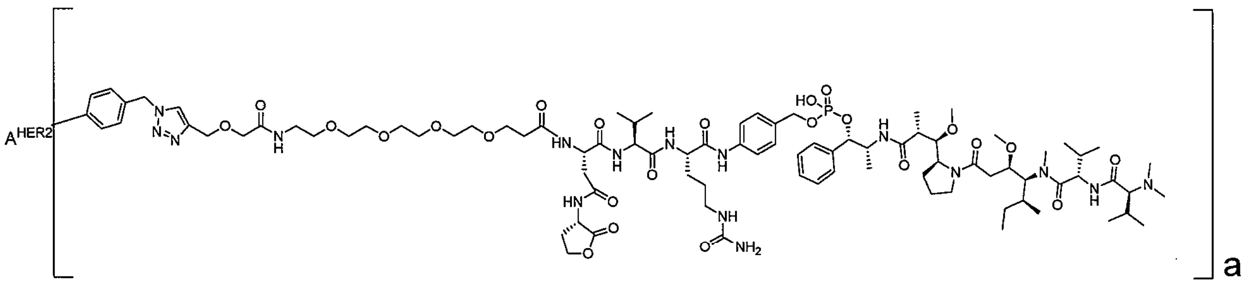

Exemplification 4-55b

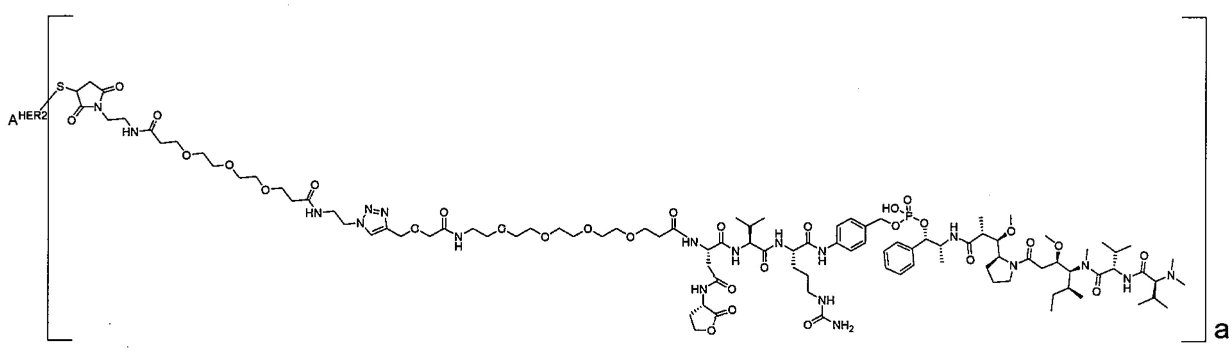

Exemplification 4-56

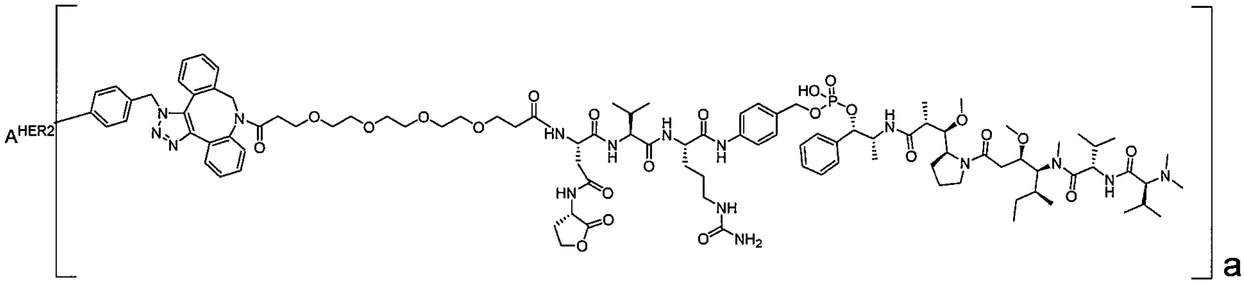

Exemplification 4-56b

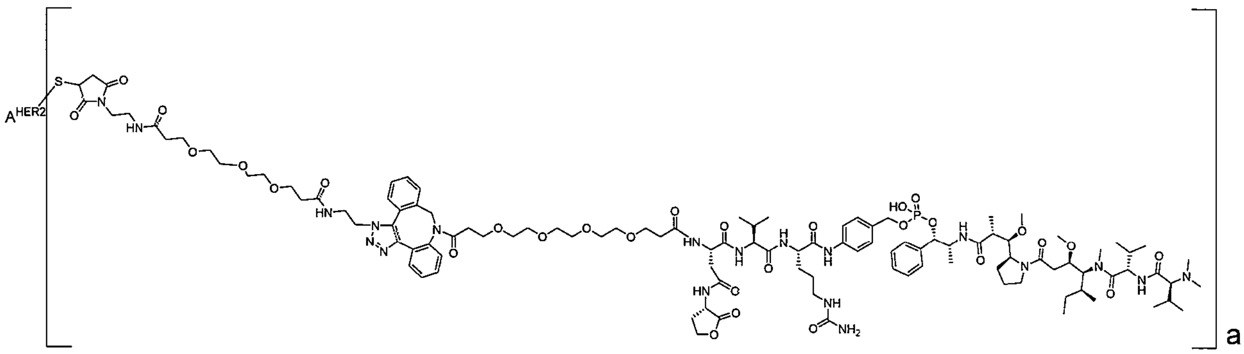

Exemplification 4-57

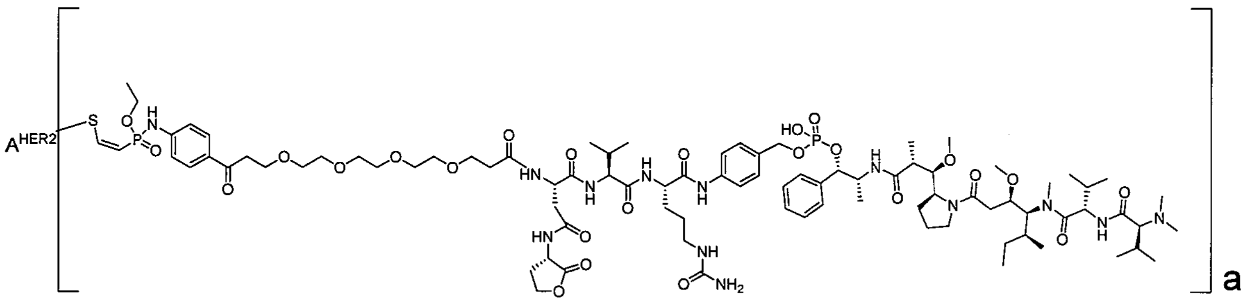

Exemplification 4-58

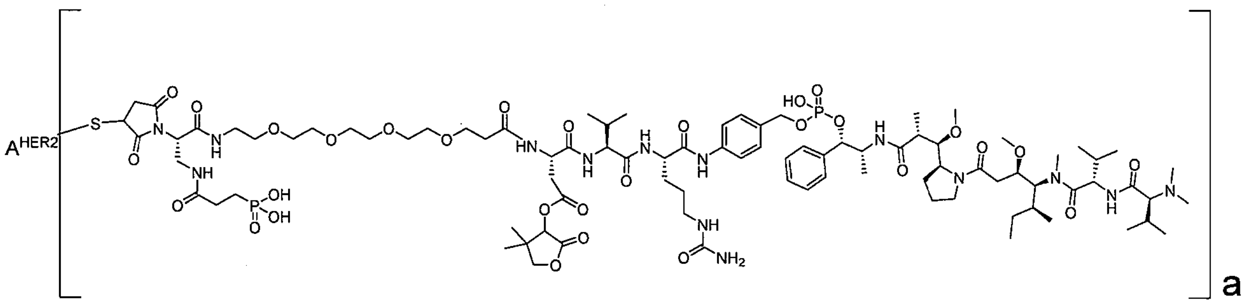

Exemplification 4-59

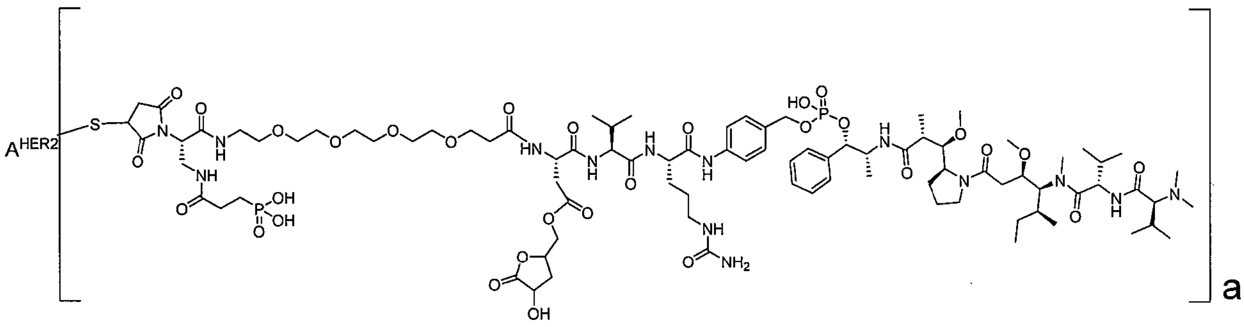

Exemplification 4-60

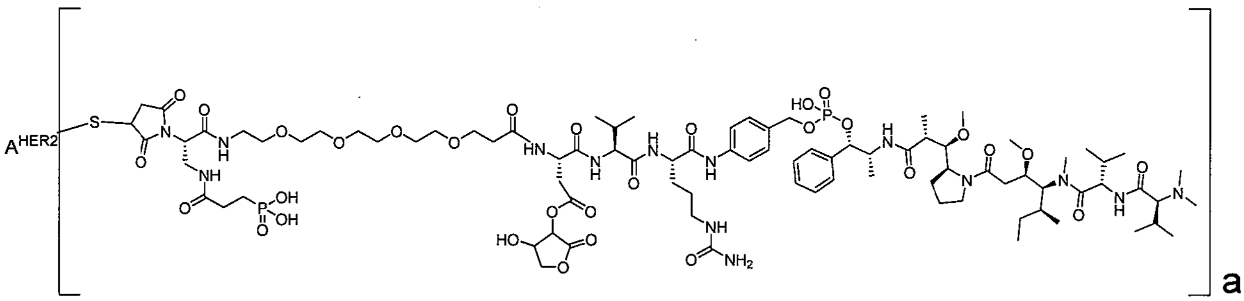

Exemplification 4-61

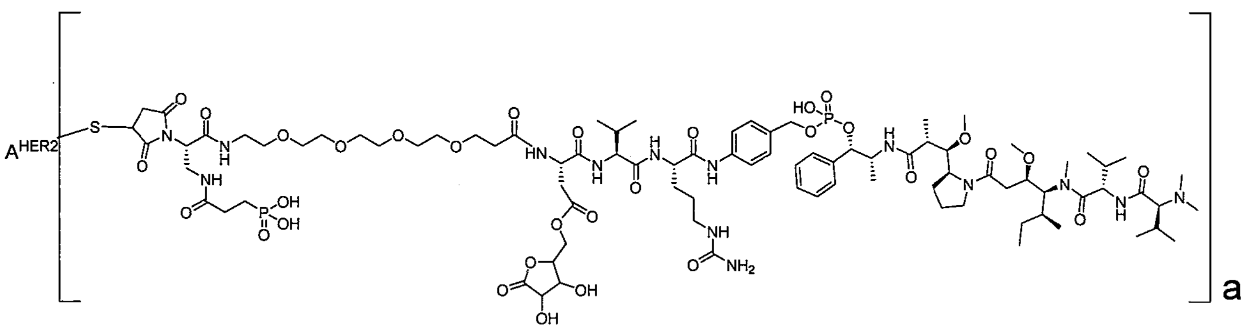

Exemplification 4-62

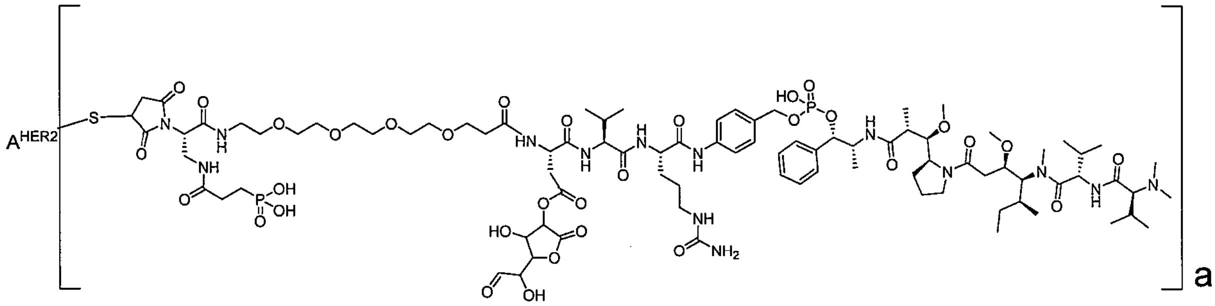

Exemplification 4-63

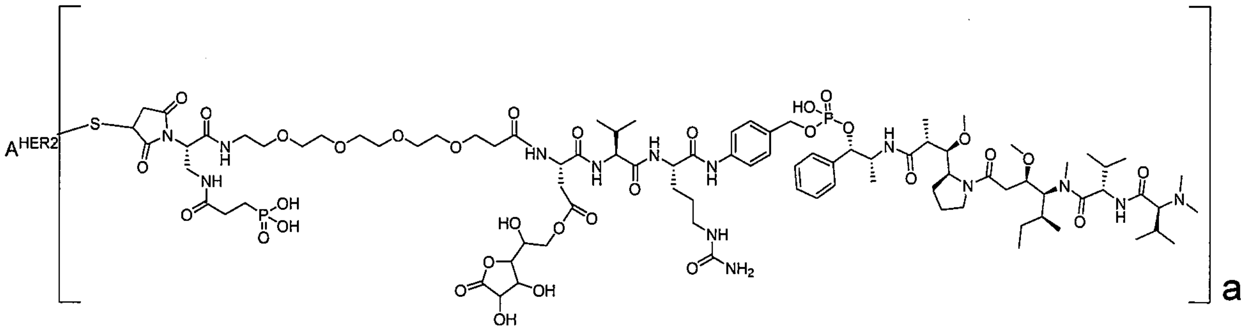

Exemplification 4-64

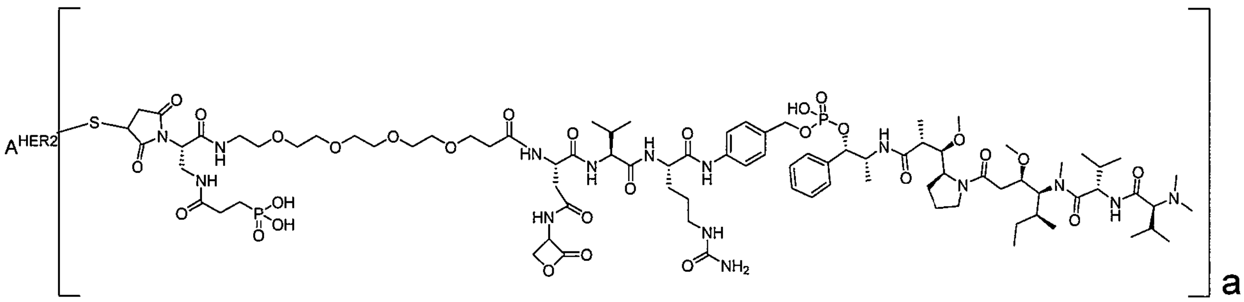

Exemplification 4-65

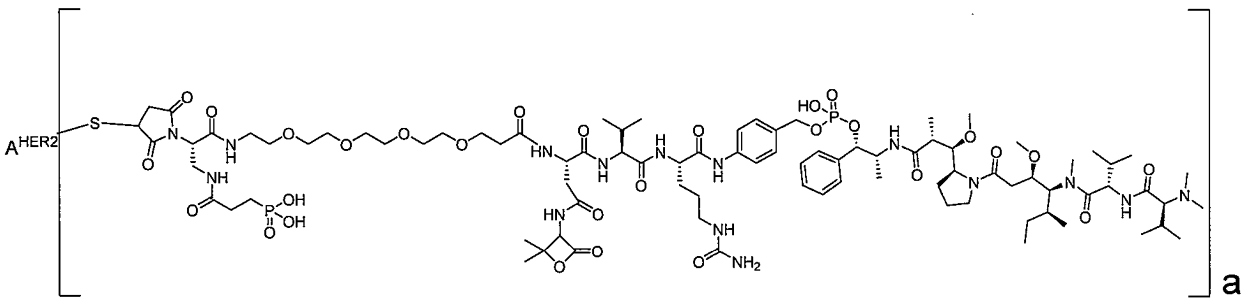

Exemplification 4-66

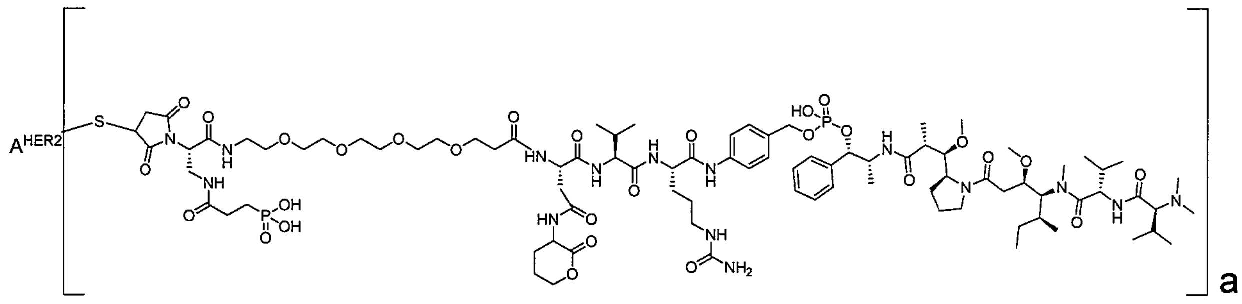

Exemplification 4-67

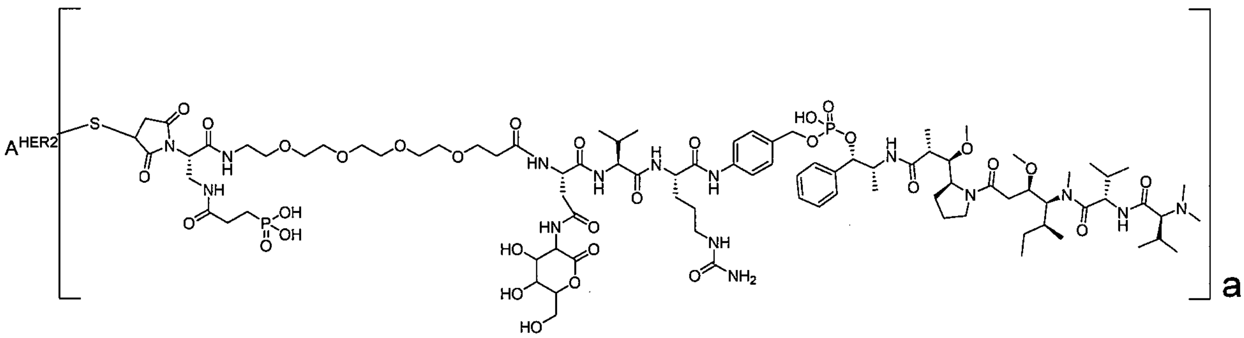

Exemplification 4-68

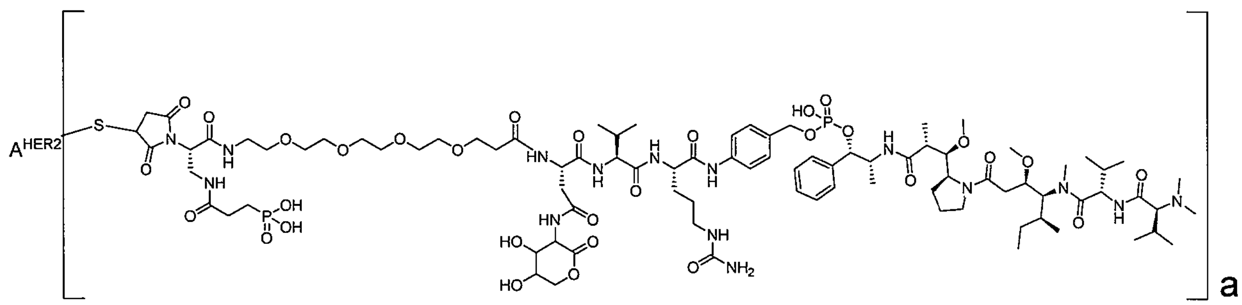

Exemplification 4-69

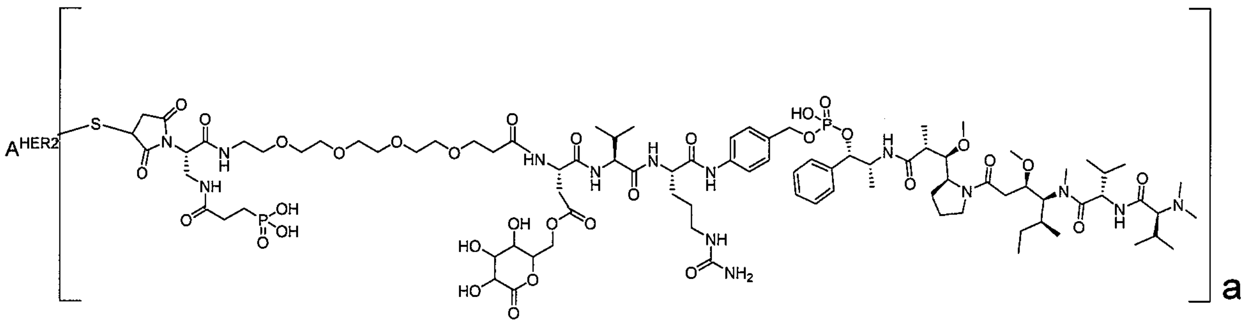

Exemplification 4-70

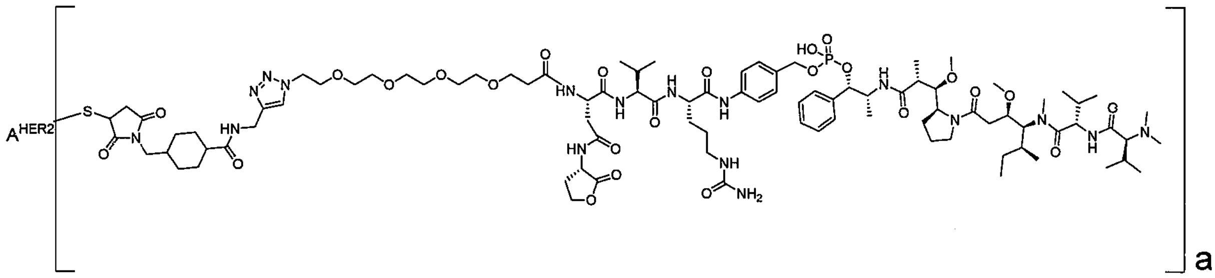

Exemplification 4-70b

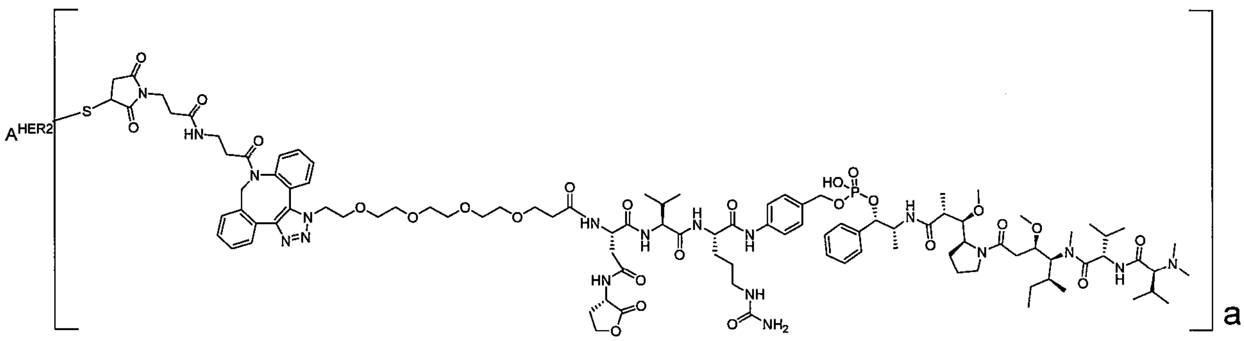

Hereinafter, general methods for producing said ADC are specifically described. Basically, widely used and known methods in the production of ADC or chemical modifications of proteins are appropriately selected and applied with necessary modifications (see the following patent and literatures).

- JP 6186045 B2
- Organic Synthetic Chemistry Vol.42, No.4 (1984)
- Drug Delivery System 34-1, 2019
- Nat. Commun. 2018, 9, 2512
- YAKUGAKU ZASSHI 139, No.2 (2019)
- Angew. Chem. Int. Ed. 2019, 58, 11631-11636•••literature of phosphonamidate
- Chem. Rev. 2015, 115, 2174-2195
- Analytical Sciences January 2018, Vol.35, 5-27

1. Production method-1

The ADC (general formula (III)) produced by using the conjugate precursor (I) of the present invention wherein B

is -S- (thioether bond) (general formula (III-1) may be produced according to, for example, a method represented by the following formula.

Production method of ADC represented by general formula (III-1)

$$A\text{-}(SH)_a + \text{conjugate precursor (I-1)} \rightarrow A\text{-}(S\text{-}Z^2\text{-}L\text{-}D)_a \qquad \text{general formula (III-1)}$$

[wherein

A-$(SH)_a$ is the above defined "antibody" having one or more sulfhydryl group(s) (-SH);
the conjugate precursor (I-1) is a conjugate precursor (I) represented by the above general formula (I) wherein Z is a maleimidyl group (the above formula (i)), an $\alpha$-halogenomethylcarbonyl group (the above formula (ii)), or an ethynylphosphonamidate group (the above formula (iii), Angew. Chem. Int. Ed. 2019, 58, 11631 -11636);
$Z^2$ is a divalent residue (a succinimidylene group, a - $CH_2$-C(=O)- group, or a cis-ethenylphosphonamidate group) produced by reacting the above defined Z with an SH group in the antibody;
the succinimidylene group is represented by the following formula:

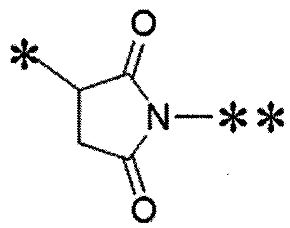

[wherein

* is a point of attachment to the "antibody-S-"; and
** is a point of attachment to "L"]
the cis-ethenylphosphonamidate group is represented by the following formula:

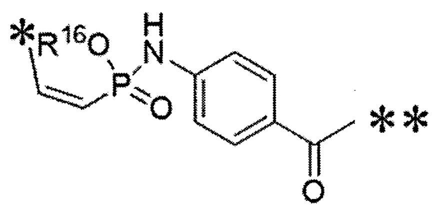

[wherein:

$R^{16}$ is a methyl group, an ethyl group, or a - $CH_2CH_2OCH_2CH_2OH$ group;
* is a point of attachment to the "antibody-S-"; and
** is a point of attachment to "L"] and
L, D, and a are the same as defined above.]

The above production method can be specifically described as follows.

(1)

$$A\text{-}(SH)_a + \text{maleimidyl-L-D} \rightarrow A\text{-}(S\text{-succinimidylene-L-D})_a \qquad \text{general formula (III-1-1)}$$

or
(2)

$$A\text{-}(SH)_a + \text{Hal-}CH_2\text{-C(=O)-L-D} \rightarrow A\text{-}(S\text{-}CH_2\text{-C(=O)-L-D})_a \qquad \text{general formula (III-1-2)}$$

or
(3)

$A\text{-}(SH)_a$ + ethenylphosphonamidate-L-D → $A\text{-}(S\text{-}cis\text{-}ethenylphosphonamidate\text{-}L\text{-}D)_a$
general formula (III-1-3)

[wherein:

A, L, D, a, and Hal are the same as defined above;
the succinimidylene group is represented by the following formula:

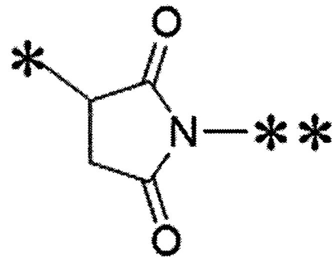

[wherein

* is a point of attachment to "antibody-S-"; and
** is a point of attachment to "L"]; and
the cis-ethenylphosphonamidate group is represented by the following formula:

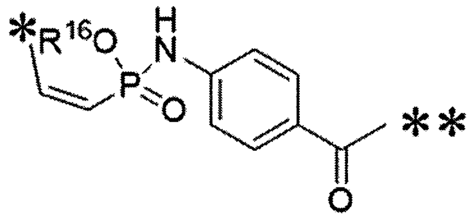

[wherein:

$R^{16}$ is the same as defined above;
* is a point of attachment to "antibody-S-"; and
** is a point of attachment to "L"].]

As described above, the ADC (general formula (III-1)) can be produced by reacting the antibody having sulfhydryl group(s) ($A\text{-}(SH)_a$) with the conjugate precursor (I) synthesized according to the methods described in Examples.

The antibody having sulfhydryl group(s) ($A\text{-}(SH)_a$) can be obtained according to methods well-known to a skilled person (Hermanson, G. T, Bioconjugate Techniques, pp.56-136, pp.456-493, Academic Press(1996)).

Examples of such methods include, but are not limited to, allowing a Traut's reagent to act on amino group(s) of an antibody; allowing a N-succinimidyl-S-acetylthioalkanoate to act on amino group(s) of an antibody, and then allowing hydroxylamine to act thereon; allowing N-succinimidyl-3-(pyridyldithio)propionate to act on an antibody, and then allowing a reducing agent to act thereon; and allowing a reducing agent such as dithiothreitol, 2-mercaptoethanol, and tris(2-carboxyethyl)phosphine hydrochloride (TCEP) to act on an antibody to reduce disulfide bond(s) at the hinge moiety(ies) in the antibody and produce sulfhydryl group(s).

Specifically, TCEP as a reducing agent may be used at 0.3 to 3 molar equivalent(s) per a disulfide at a hinge moiety in an antibody and reacted with an antibody in a buffer solution comprising a chelating agent to produce an antibody in which disulfide(s) at hinge moiety(ies) in the antibody is/are partially or completely reduced. Examples of the chelating agent include ethylenediaminetetraacetic acid (EDTA) and diethylenetriaminepentaacetic acid (DTPA). The chelating agent may be used at a concentration of 1 mM to 20 mM. Examples of the buffer solution which may be used include a sodium phosphate, sodium borate, or sodium acetate solution, and phosphate buffered saline. In a specific example, an antibody may be reacted with TCEP at 4°C to 37°C for 1 to 4 hour(s) to produce an antibody having partially or completely reduced sulfhydryl group(s), $A\text{-}(SH)_a$. Also, 2 to 20 molar equivalents of a conjugate precursor (I-1) may be used per an antibody having sulfhydryl group(s) ($A\text{-}(SH)_a$) to produce an ADC (formula (III-1)) in which 1 to 10 linker-drug unit(s) ("-L-D"

moiety in the above general formula (III)) is/are bound per an antibody.

Specifically, to a buffer solution comprising an antibody having sulfhydryl group(s) (A-$(SH)_a$) is added a solution in which a conjugate precursor (I-1) is dissolved to carry out a reaction. Examples of the buffer solution which may be used include a sodium phosphate, sodium borate, or sodium acetate solution, and phosphate buffered saline. The reaction is carried out at pH 5 to 9, and more preferably at about pH 7. Examples of the solvent to be used for dissolving the conjugate precursor (I-1) include organic solvents such as dimethylsulfoxide (DMSO), dimethylformamide (DMF), dimethylacetamide (DMA), and N-methyl-2-pyridone (NMP). An organic solvent solution in which the conjugate precursor (I-1) is dissolved is added to the buffer solution comprising the antibody having sulfhydryl group(s) (A-$(SH)_a$) at 1 to 20% v/v to carry out a reaction. The reaction temperature is 0 to 37°C and more preferably 10 to 25°C, and the reaction time is 0.5 to 2 hour(s). The reaction may be terminated by inactivating the unreacted reactive group(s) of the conjugate precursor (I-1) by a thiol-containing reagent. Example of the thiol-containing reagent include cysteine and N-acetyl-L-cysteine (NAC). More specifically, NAC may be added to a reaction mixture at 1 to 2 molar equivalent(s) relative to the conjugate precursor (I-1) to be used, and the resulting mixture may be incubated at room temperature for 10 to 30 minutes to terminate the reaction.

2. Production method-2

The ADC (general formula (III)) produced by using the conjugate precursor (I) of the present invention wherein B is -NH- (general formula (III-2) may be produced according to, for example, a method represented by the following formula.

Production method of ADC represented by general formula (III-2)

$$A\text{-}(NH_2)_a + \text{conjugate precursor (I-2)} \rightarrow A\text{-}(NH\text{-}C(=O)\text{-}L\text{-}D)_a \qquad \text{general formula (III-2)}$$

[wherein

A-$(NH_2)_a$ is an antibody having one or more amino group (s) (-$NH_2$);
the conjugate precursor (I-2) is a conjugate precursor represented by the above general formula (I) wherein Z is a carboxy group, or an active ester group thereof such as an N-hydroxysuccinimidyl ester group; and
L, D, and a are the same as defined above.]

The above production method can be specifically described as follows.

(1)

$$A\text{-}(NH_2)_a + HOOC\text{-}L\text{-}D \rightarrow A\text{-}(NH\text{-}C(=O)\text{-}L\text{-}D)_a \qquad \text{general formula (III-2-1)}$$

or
(2)

$$A\text{-}(NH_2)_a + \text{N-hydroxysuccinimidyl ester-L-D} \rightarrow A\text{-}(NH\text{-}C(=O)\text{-}L\text{-}D)_a \qquad \text{general formula (III-2-1)}$$

[wherein A-$(NH_2)_a$ is an antibody having one or more amino group(s) (-$NH_2$); and
L, D, and a are the same as defined above.]

As described above, the ADC (general formula (III-2)) can be produced by reacting an antibody A-$(NH_2)_a$ having amino group(s) (-$NH_2$) with the conjugate precursor (I-2) synthesized according to the methods described in Examples.

Examples of the active ester of the conjugate precursor (I-2) which may be used include the above exemplified N-hydroxysuccinimidyl ester, as well as other active esters such as sulfosuccinimidyl esters, N-hydroxyphthalimidyl esters, N-hydroxysulfophthalimidyl esters, ortho-nitrophenyl esters, para-nitrophenyl esters, 2,4-dinitrophenyl esters, 3-sulfonyl-4-nitrophenyl esters, 3-carboxy-4-nitrophenyl esters, and pentafluorophenyl esters.

Specifically, 2 to 20 molar equivalents of the conjugate precursor (I-2) may be used per an antibody (A-$(NH_2)_a$) having amino group(s) to produce an ADC (general formula (III-2)) in which 1 to 10 linker-drug unit(s) ("-L-D" moiety in the above general formula (III)) is/are bound per an antibody.

Specifically, to a buffer solution comprising an antibody (A-$(NH_2)_a$) may be added a solution in which a conjugate precursor (I-2) is dissolved to carry out a reaction to produce an ADC (general formula (III-2)). Examples of the buffer solution which may be used include a sodium phosphate, sodium borate, or sodium acetate solution, and phosphate buffered saline. The reaction may be carried out at a pH 5 to 9, and more preferably at about pH 7. Examples of the solvent which may be used for dissolving the conjugate precursor (I-2) therein include organic solvents such as dimethylsulfoxide

(DMSO), dimethylformamide (DMF), dimethylacetamide (DMA), and N-methyl-2-pyridone (NMP). An organic solvent solution in which the conjugate precursor (I-2) is dissolved may be added to the buffer solution comprising the antibody ($A-(NH_2)_a$) at 1 to 20% v/v to carry out a reaction. The reaction temperature is 0 to 37°C and more preferably 10 to 25°C. The reaction time is 0.5 to 20 hour(s).

3. Conjugate treatment step in the production of antibody-drug conjugate (ADC)

The antibody-drug conjugate (III) produced according to the above general production methods may be subjected to concentration, buffer exchange, purification, and measurement of antibody concentration and average number of bound linker-drug unit ("-$Z^2$-L-D" moiety) per one antibody molecule according to the following common operations to identify the antibody-drug conjugate.

Conjugate treatment step A: Concentration of aqueous solution of antibody or antibody-drug conjugate

To a container of Amicon Ultra (50,000 MWCO, Millipore Corporation) is added a solution of antibody or antibody-drug conjugate, and the solution of antibody or antibody-drug conjugate is concentrated by centrifugation (centrifuged at 2000 G to 3800 G for 5 to 20 minutes) using a centrifuge (Universal Refrigerated Centrifuge MODEL5922, KUBOTA corporation).

Conjugate treatment step B: Measurement of antibody concentration

The antibody concentration was measured using a plate reader (FlexStation 3, Molecular Devices, LLC) according to the manufacturer's specified method. In the measurement, different 280 nm extinction coefficients (1.3 $mLmg^{-1}cm^{-1}$ to 1.8 $mLmg^{-1}cm^{-1}$) are used depending on antibodies.

Conjugate treatment step C: Buffer exchange of antibody

A NAP-25 column (Cat. No. 17-0854-02, GE Healthcare Japan Corporation) using a Sephadex G-10 carrier is equilibrated with a phosphate buffer solution (pH 7.4) (referred to as "PBS6.0/EDTA" in the present description) according to the method described in the manufacturer's instruction. An aqueous solution of antibody (1.0 mL) is applied thereto per one said NAP-10 column, and then a fraction (1.5 mL) eluted with PBS (1.5 mL) is subjected to preparative separation. Said fraction is concentrated according to the Common operation A, the antibody concentration is measured using the Common operation B, and then the antibody concentration is adjusted using PBS.

Conjugate treatment step D: Purification of antibody-drug conjugate

Any one of buffer solution of a commercially available phosphate buffer solution (PBS7.4, Cat. No. 10010-023, Invitrogen), a sodium phosphate buffer solution comprising sodium chloride (137 mM) (10 mM, pH 6.0; referred to as "PBS6.0" in the present description), or an acetate buffer (10 mM, pH 5.5; referred to as "ABS" in the present description) comprising Sorbitol (5%) is used to equilibrate a NAP-10 column. An aqueous reaction solution of antibody-drug conjugate (about 1.0 mL) is applied to said NAP-10 column, and eluted with a manufacturer's specified amount of the buffer solution to carry out preparative separation of the antibody fraction. A gel filtration purification, in which said preparative fraction is applied to the NAP-10 column again and eluted with the buffer solution, is repeated 2 to 3 times in total to obtain an antibody-drug conjugate in which unbound drug linkers and low molecular weight compounds (tris(2-carboxyethyl) phosphine hydrochloride (TCEP), N-acetyl-L-cysteine (NAC), dimethylsulfoxide, and the like) are removed.

Conjugate treatment step E: Calculation of antibody concentration in antibody-drug conjugate and average number of bound linker-drug unit ("-$Z^2$-L-D" moiety) (DAR) per one antibody molecule using absorbance

The bound drug concentration in antibody-drug conjugate can be calculated by measuring the UV absorbance of an aqueous solution of antibody-drug conjugate at the two wavelengths of 280 nm and 370 nm and then carrying out the following calculation.

The total absorbance at a certain wavelength is the sum of absorbance of all absorptive chemical species present in the system [additive property of absorbance]. Thus, assuming that the molar extinction coefficients of antibody and drug do not change before and after conjugating them, the antibody concentration and drug concentration in antibody-drug conjugate are represented by the following relation equations.

$$A280 = AD,280 + AA,280 = \varepsilon D,280CD + \varepsilon A,280CA \cdots \text{Equation (1)}$$

$$A370 = AD,370 + AA,370 = \varepsilon D,370CD + \varepsilon A,370CA \cdots \text{Equation (2)}$$

In the above equations, A280 represents the absorbance of an aqueous solution of antibody-drug conjugate at 280 nm, A370 represents the absorbance of an aqueous solution of antibody-drug conjugate at 370 nm, "AA,280" represents the absorbance of antibody at 280 nm, "AA,370" represents the absorbance of antibody at 370 nm, "AD,280" represents the absorbance of conjugate precursor (the compound of the above general formula (I); the same applies hereafter) at 280 nm, "AD,370" represents the absorbance of conjugate precursor at 370 nm, "εA,280" represents the molar extinction coefficient of antibody at 280 nm, "εA,370" represents the molar extinction coefficient of antibody at 370 nm, "εD,280" represents the molar extinction coefficient of conjugate precursor at 280 nm, "εD,370" represents the molar extinction coefficient of conjugate precursor at 370 nm, CA represents the antibody concentration in antibody-drug conjugate, and CD represents the drug concentration in antibody-drug conjugate.

In the above equations, values prepared in advance (calculated estimate value or measured value obtained from UV measurement of compound) are used as "εA,280", "εA,370", "εD,280", and "εD,370". For example, "εA,280" can be estimated according to a known calculation method (Protein Science, 1995, vol.4, 2411-2423) using the amino acid sequence of antibody. "εA,370" is usually zero. "εD,280" and "εD,370" can be obtained by measuring the absorbance of a solution in which a conjugate precursor to be used is dissolved at a certain molar concentration and calculated using Lambert-Beer law (absorbance = molar concentration × molar extinction coefficient × cell optical path length). A280 and A370 of an aqueous solution of antibody-drug conjugate are measured, these values are substituted in the formulae (1) and (2), and the simultaneous equation can be solved to calculate the CA and CD. further CD may be divided by CA to calculate the average number of bound linker-drug unit ("-$Z^2$-L-D" moiety) per one antibody.

Conjugate treatment step F: Calculation of DAR in antibody-drug conjugate by cysteine reactivity evaluation

The DAR in the antibody-drug conjugate (general formula (III-1)) wherein "-B-" is "-S-" in general formula (III) can be calculated and estimated by evaluating the reactivity of the conjugate precursor (I-1) with L-cysteine. Namely, if the conjugate precursor (I-1) is reacted with L-cysteine under the same conditions as those of the conjugating reaction and L-cysteine is completely consumed, DAR is estimated to be 8. A specific method is described below.

To a solution of L-cysteine in PBS/EDTA (134 μM, 900 μL) was added a solution of a conjugate precursor (I-1) to be used in conjugating in dimethylsulfoxide (1.51 mM, 100 μL). (Under this condition, the amount of the conjugate precursor is 10 equivalents relative to 8 equivalents of L-cysteine). Then, the resulting mixture is reacted on ice for 1 hour. After the reaction is completed, the resulting solution (480 μL) is added to a microtube, and a solution of 5,5'-dithiobis(2-nitrobenzoic acid) (manufactured by FUJIFILM Wako Pure Chemical Corporation, 047-16401) in ethanol (10 mM, 20 μL) is added thereto. The resulting mixture is reacted at room temperature for 15 minutes. After the reaction, the solution is added to a 96 well plate, and a microplate reader (FlexStation 3, manufactured by Molecular Device, LLC) is used to measure the absorbance (412 nm). The same procedures are carried out using a L-cysteine solution having a known concentration, and a calibration curve is prepared to calculate the L-cysteine concentration in the sample.

L-cysteine residual rate in sample (%) is calculated by the following equation.

$$\text{L-cysteine residual rate in sample (\%)} = (a/121) \times 100$$

a: L-cysteine concentration in sample (μM)

The DAR of an antibody-drug conjugate (general formula (IV-1)) obtained using a conjugate precursor (I-1) in the same lot as that used in the above cysteine reactivity evaluation is calculated and estimated by the following equation.

$$\text{DAR} = 8 \times ((100-b)/100)$$

b: L-cysteine residual rate in sample (%)

Further, in order to ensure an adequate amount of conjugate, conjugates having the same level of average total drugs (for example, ±1 level) prepared under the same conditions may be mixed with each other to prepare a new lot. In such case, the resulting average total drugs fall within the range of average total drugs before mixing.

The antibody-drug conjugate (III) produced by using the conjugate precursor (I) of the present invention or a salt thereof may be left to stand into the atmosphere or recrystallized to absorb moisture, be attached to adsorbed water, or become a hydrate, and such an antibody-drug conjugate or a salt thereof comprising water is also encompassed by the present invention.

4. Use method of antibody-drug conjugate (ADC)

The antibody-drug conjugate (III) produced by using the conjugate precursor (I) of the present invention is useful in the growth inhibition of tumor cells or cancer cells, the induction of cell death in tumor cells or cancer cells, or the

treatment of cancers in patients. Said antibody-drug conjugate may be used in the treatment of cancers under various conditions. Said antibody-drug conjugate may be used for delivering drugs to tumor cells or cancer cells. In one embodiment, the antibody in said antibody-drug conjugate may be bound to or associated with cancer-cell-related antigens or tumor-cell-related antigens, and said antibody-drug conjugate may be incorporated (internalized) into tumor cells or cancer cells through receptor-mediated endocytosis or another internalization mechanism. The antigens may be bound to tumor cells or cancer cells, or may be extracellular matrix proteins related to tumor cells or cancer cells. When said antibody-drug conjugate is incorporated into cells, the drug is released into the cells through a cleavage mechanism. Also, the drug may be cleaved from said antibody-drug conjugate at the outside of tumor cells or cancer cells, and then the drug or drug unit may be penetrated into cells.

In one embodiment, the antibody is bound to tumor cell or cancer cells.

In another embodiment, the antibody is bound to tumor cell or cancer cell antigens present on the surface of tumor cells or cancer cells.

In another embodiment, the antibody is bound to tumor cell or cancer cell antigens which are extracellular matrix proteins related to tumor cells or cancer cells.

Antibody specificity for specific tumor cells or cancer cells may be important for determining tumors or cancers which are most effectively treated. For example, said antibody-drug conjugate which targets cancer cell antigens present on hematopoietic cancers may be useful for treating hematological malignancy (for example, an anti-CD30-, an anti-CD70-, an anti-CD19-, and an anti-CD33-binding antibodies may be useful for treating hematological malignancy). Said antibody-drug conjugate which targets cancer cell antigens present on solid tumors may be useful for treating such solid tumors.

Examples of cancers which may be treated by using the antibody-drug conjugate (III) obtained from the conjugate precursor (I) of the present invention include, but are not limited to, hematopoietic cancers such as lymphoma (Hodgkin's lymphoma and non-Hodgkin's lymphoma) and leukemia, and solid tumors. Examples of hematopoietic cancers include follicular lymphoma, anaplastic large-cell lymphoma, mantle-cell lymphoma, acute myeloblastic leukemia, chronic myeloid leukemia, chronic lymphocytic leukemia, diffuse large B-cell lymphoma, and multiple myeloma. Examples of solid tumors include fibrosarcoma, myxosarcoma, liposarcoma, chondrosarcoma, osteosarcoma, chordoma, angiosarcoma, endotheliosarcoma, lymphangiosarcoma, lymphangioendotheliosarcoma, synovioma, mesothelioma, Ewing's tumor, leiomyosarcoma, rhabdomyosarcoma, colon cancer, colorectal cancer, kidney cancer, pancreatic cancer, bone cancer, breast cancer, ovarian cancer, prostate cancer, esophageal cancer, gastric cancer, oral cancer, nasal cavity cancer, throat cancer, squamous cell cancer, basal cell cancer, adenocarcinoma, sweat gland cancer, sebaceous carcinoma, papillary carcinoma, papillary adenocarcinoma, cystadenocarcinoma, medullary cancer, bronchogenic cancer, renal cell cancer, liver cancer, biliary tract cancer, choriocarcinoma, seminoma, embryonal cancer, Wilms' tumor, cervical cancer, uterine cancer, testicular tumor, small cell lung cancer, bladder cancer, lung cancer, epithelial cancer, glioma, glioblastoma multiforme, astrocytoma, medulloblastoma, craniopharyngioma, ependymoma, pinealoma, angioblastoma, acoustic neuroma, oligodendroglioma, meningioma, skin cancer, melanoma, neuroblastoma, and retinoblastoma.

5. Production example of antibody-drug conjugate (ADC)

The antibody-drug conjugate (ADC) was produced by using the conjugate precursor (I) of the present invention according to the following method. The antibody-drug conjugate obtained in the following each Production example is represented as general formula (III').
general formula (III'):

$$A\text{-}(B\text{-}Z^2\text{-}L\text{-}D)_{a'} \qquad \text{general formula (III')}$$

(wherein

A, B, $Z^2$, L, and D are the same as defined above; and
a' is an average number of bound linker-drug ("-$Z^2$-L-D" moiety) (DAR) per one antibody molecule in the antibody-drug conjugate produced in each Production example, which is a number of 1 to 10, may include a decimal, and is represented herein up to the first decimal place in the present description.)

ADC Production example 1. Production of ADC represented by Exemplification 4-1

1-1. Preparation of anti-HER2 antibody (trastuzumab) by purifying Herceptin

Trastuzumab was prepared by purifying Herceptin (manufactured by Chugai Pharmaceutical Co., Ltd.). EDTA

(manufactured by Thermo Fisher Scientific, AM9260G) was added to phosphate buffered saline (hereinafter referred to as "PBS") (pH 7.2, manufactured by Thermo Fisher Scientific, 20012-043) so that the final concentration would become 10 mM (hereinafter referred to as "PBS/EDTA"). An NAP-10 column (manufactured by GE Healthcare, 17085402) was equilibrated with PBS/EDTA according to the manufacturer's specified method. A solution of Herceptin in PBS/EDTA (1 mL) was applied to the NAP-10 column, and then fraction (1.5 mL) eluted with PBS/EDTA (1.5 mL) was collected. The absorbance at 280 nm of this fraction was measured by using a microplate reader (FlexStation 3, manufactured by Molecular Device) to measure the antibody concentration (1.47 $mLmg^{-1}cm^{-1}$ was used as the extinction coefficient at 280 nm).

1-2. Reduction of trastuzumab:

The concentration of trastuzumab solution obtained in the above 1-1 was adjusted to 1 to 10 mg/mL using PBS/EDTA. 0.99 mL of this solution was placed in a 1.5 mL microtube, and a solution of TCEP hydrochloride (manufactured by FUJIFILM Wako Pure Chemical Corporation, 209-19861) in PBS/EDTA (10 μL, 6.9 equivalents relative to one antibody molecule) was added thereto. The resulting mixture was incubated at 37°C for 2 hours to reduce the disulfide bond at the hinge moiety in the antibody.

1-3. Conjugate formation of reduced trastuzumab and conjugate precursor (I):

To the reduced trastuzumab solution (900 μL) was added a dimethyl sulfoxide solution (manufactured by FUJIFILM Wako Pure Chemical Corporation, 043-07216) containing the compound obtained in the Example 1 (Exemplification No. 2-1) (100 μL, 10 or 20 equivalents relative to one antibody molecule), and the resulting mixture was reacted on ice for 1 hour. Next, a solution of L-Cysteine (manufactured by FUJIFILM Wako Pure Chemical Corporation, 039-20652) in PBS/EDTA (10 μL, 10 equivalents relative to one antibody molecule) was added thereto, and the reaction was further carried out for 15 minutes to stop the reaction of conjugate formation.

1-4. Purification of antibody-drug conjugate:

An NAP-10 column was equilibrated with PBS (pH 7.4, manufactured by Thermo Fisher Scientific, 10010-023) according to the manufacturer's specified method. To this NAP-10 column was applied an aqueous reaction solution of the antibody-drug conjugate obtained in the above 1-3 (1 mL), and the fraction (1.5 mL) eluted with PBS (1.5 mL, pH 7.4) was collected. This collected fraction was applied to the NAP-10 column again, and the similarly eluted fraction was collected.

1-5. Calculation of antibody concentration and average number of bound linker-drug unit ("-$Z^2$-L-D" moiety) per one antibody molecule in antibody-drug conjugate:

The DAR of the antibody-drug conjugate obtained in the above 1-4 was measured according to "Conjugate treatment step E" in the above conjugate treatment steps described in connection with the ADC production method or estimated according to "Conjugate treatment step F". As a result, the DAR of the antibody-drug conjugate of the present ADC Production example 1 was 8.0.

Regarding Examples 3b and 3c, antibody-drug conjugate was produced according to the same method as Example 1, except for an anti-CD20 antibody purified from rituximab BS (Kyowa Kirin Co., Ltd.) or an anti-EGFR antibody purified from Erbitux (Merck BioPharma Co., Ltd.) was used instead of the anti-HER2 antibody used in the Preparation 1-1 of Example 1.

The present ADC Production examples and Comparative ADC Production examples were produced by using each Example, Comparative compound-1 (MC-Val-Cit-PAB-MMAE (MedChemExpress, CAS No. 646502-53-6)), and Comparative compound-2 (Mal-PEG2-Val-Cit-PAB-Eribulin (Chem Scene, CAS No. 2130869-18-8)) and performing the same reactions and treatment operations as in ADC Production example 1 (regarding ADC Production example 11, Example 11 was used in 20 equivalents relative to the antibody). Results of the DAR of the present ADC Production example and Comparative ADC Production example are summarized in the following Table 1 together with ADC Production example.

The Comparative compound-1 refers to MC-Val-Cit-PAB-MMAE.

The Comparative ADC Production example -1 refers to an ADC Production example obtained by using the Comparative compound-1.

The Comparative compound-2 refers to Mal-PEG2-VAl-Cit-PAB-Eribulin.

The Comparative ADC Production example -2 refers to an ADC Production example obtained by using the Comparative compound-2.

Table 1 Produced ADC (III')

| Example No. | ADC Production example No. (structure thereof) | A | B | DAR | |
|---|---|---|---|---|---|
| | | | | Treatment step | a' |
| 1 | ADC Production example No. 1 (Exemplification No. 4-1) | anti-HER2 antibody residue | -S- | F | 8.0 |
| 2 | ADC Production example No. 2 (Exemplification | anti-HER2 antibody residue | -S- | F | 7.9 |
| | No. 4-2) | | | | |
| 3 | ADC Production example No. 3 (Exemplification No. 4-3) | anti-HER2 antibody residue | -S- | F | 8.0 |
| 3b | ADC Production example No. 3 (Exemplification No. 4-3) | anti-CD20 antibody residue | -S- | F | 8.0 |
| 3c | ADC Production example No. 3 (Exemplification No. 4-3) | anti-EGFR antibody residue | -S- | F | 8.0 |
| 4 | ADC Production example No. 4 (Exemplification No. 4-4) | anti-HER2 antibody residue | -S- | E | 7.0 |
| 5 | ADC Production example No. 5 (Exemplification No. 4-5) | anti-HER2 antibody residue | -S- | F | 8.0 |
| 6 | ADC Production example No. 6 (Exemplification No. 4-6) | anti-HER2 antibody residue | -S- | F | 8.0 |
| 7 | ADC Production example No. 7 (Exemplification No. 4-7) | anti-HER2 antibody residue | -S- | F | 8.0 |
| 8 | ADC Production example No. 8 (Exemplification No. 4-8) | anti-HER2 antibody residue | -S- | F | 7.1 |
| 9 | ADC Production example No. 9 (Exemplification No. 4-9) | anti-HER2 antibody residue | -S- | E | 6.7 |
| 10 | ADC Production example No. 10 (Exemplification No. 4-10) | anti-HER2 antibody residue | -S- | E | 7.1 |
| 11 | ADC Production example No. 11 (Exemplification No. 4-11) | anti-HER2 antibody residue | -S- | E | 8.0 |
| 12 | ADC Production example No. 12 (Exemplification No. 4-12) | anti-HER2 antibody residue | -S- | E | 7.0 |
| 13 | ADC Production example No. 13 (Exemplification No. 4-13) | anti-HER2 antibody residue | -S- | E | 6.8 |
| 14 | ADC Production example No. 14 (Exemplification | anti-HER2 antibody residue | -S- | F | 7.9 |
| | No. 4-14) | | | | |
| 15 | ADC Production example No. 15 (Exemplification No. 4-15) | anti-HER2 antibody residue | -S- | F | 7.3 |
| 16 | ADC Production example No. 16 (Exemplification No. 4-16) | anti-HER2 antibody residue | -S- | F | 7.3 |
| 17 | ADC Production example No. 17 (Exemplification No. 4-17) | anti-HER2 antibody residue | -S- | F | 8.0 |
| 18 | ADC Production example No. 18 (Exemplification No. 4-18) | anti-HER2 antibody residue | -S- | F | 6.6 |
| 19 | ADC Production example No. 19 (Exemplification No. 4-19) | anti-HER2 antibody residue | -S- | F | 6.7 |

(continued)

| Example No. | ADC Production example No. (structure thereof) | A | B | DAR | |
|---|---|---|---|---|---|
| | | | | Treatment step | a' |
| 20 | ADC Production example No. 20 (Exemplification No. 4-20) | anti-HER2 antibody residue | -S- | E | 4.8 |
| 21 | ADC Production example No. 21 (Exemplification No. 4-21) | anti-HER2 antibody residue | -S- | F | 7.9 |
| 22 | ADC Production example No. 22 (Exemplification No. 4-22) | anti-HER2 antibody residue | -S- | F | 8.0 |
| 23 | ADC Production example No. 23 (Exemplification No. 4-23) | anti-HER2 antibody residue | -S- | E | 8.8 |
| 24 | ADC Production example No. 24 (Exemplification No. 4-24) | anti-HER2 antibody residue | -S- | E | 9.3 |
| Comparative compound-1 | Comparative ADC Production example-1 | anti-HER2 antibody residue | -S- | F | 8.1 |
| Comparative compound-2 | Comparative ADC Production example-2 | anti-HER2 antibody residue | -S- | F | 8.0 |

Reference Example 2. Evaluation test examples of antibody-drug conjugate (ADC)

Regarding the ADC Production example (III') produced by reacting the Example of the present invention with an antibody, and the antitumor drug used in the present invention were subjected to the following tests.

[Test Example 1. Tubulin polymerization inhibition test]

For measuring the tubulin polymerization inhibitory action, Tubulin Polymerization Assay Kit (manufactured by Cytoskeleton, Inc., BK011P) was used. A PIPES buffer containing porcine tubulin, GTP, $MgCl_2$, EGTA, Glycerol, and a fluorescent reporter was prepared under ice-cooling as a reaction solution. The reaction solution was ice-cooled until it was used. A test compound solution was prepared by an aqueous solution containing 1.5% DMSO, and added to a 96 well plate (manufactured by Corning Inc., 3686) at 5 μL/well. The reaction solution was added thereto at 50 μL/well, the plate was placed in a microplate reader (FlexStation 3, manufactured by Molecular Device, LLC) in which the internal temperature was set to 37°C in advance, and the fluorescence intensity measurement (excitation wavelength: 360 nm, detection wavelength: 420 nm) of each well was started. The fluorescence intensity was measured once every minute, and the measurement was continued for 60 minutes.

The tubulin polymerization inhibition rate (%) was calculated by the following equation.

$$\text{Tubulin polymerization inhibition rate (\%)} = ((a-b)/a)\times 100$$

a: average amount of fluorescence intensity increase in vehicle-treated well (n = 2)
b: average amount of fluorescence intensity increase in test compound-treated well (n = 2)

Amount of fluorescence intensity increase: (average fluorescence intensity after 51 to 60 minutes from measurement start) - (average fluorescence intensity after 1 to 6 minutes from measurement start)

EXSUS (manufactured by CAC Croit Corporation) was used to represent the relationship between the tubulin polymerization inhibition rate and the test compound concentration (sigmoid curve regression), and the test compound concentration in which the tubulin polymerization inhibition rate became 50% ($IC_{50}$ value) was calculated.

In this test, "antitumor drug molecules or analogs thereof, or derivatives thereof (exemplified as U-compounds in the present description)" disclosed in the present description showed excellent tubulin polymerization inhibition activities. For example, each $IC_{50}$ value of U-008 (Production example 8), U-009 (Production example 9), U-010 (Production example 10), U-011 (Production example 11), U-012 (Production example 12), U-013 (Production example 13), U-014 (Production example 14), U-015 (Production example 15), U-016 (Production example 16), U-017 (Production example 17), U-018 (Production example 18), U-019 (Production example 19), U-020 (Production example 20), U-021 (Production

example 21), U-030 (Production example 30), and U-031 (Production example 31) was 3 μM or less, and almost the same level as MMAE tested under the same conditions.

[Test Example 2: Topoisomerase I activity inhibition test]

For measuring topoisomerase I activity inhibitory actions, Human DNA Topoisomerase I Assay Kit (manufactured by ProFoldin, HRA100K) was used. A human topoisomerase I (manufactured by Sigma, T9069) was added to a Tris-HCl buffer containing KCl, DTT, $MgCl_2$, EDTA, and BSA to prepare a reaction solution. The reaction solution (35.2 μL) and a test compound solution (4.4 μL) prepared by an aqueous solution containing 10% DMSO were mixed in a 1.5 mL microtube, and the resulting mixture was reacted at 37°C for 10 minutes. Subsequently, a supercoiled plasmid DNA (4.4 μL) was added thereto, and the resulting mixture was reacted at 37°C for 60 minutes. After the reaction, the solution was added to a 96 well plate (manufactured by Corning Inc., 3904) at 40 μL/well, and a Dye H19 solution (250 μL) diluted 10 times with the buffer included with the kit was added thereto. A microplate reader (Infinite F500, manufactured by TECAN) was used to measure the fluorescence intensity (excitation wavelength: 485 nm, detection wavelength: 535 nm) of each well.

The topoisomerase I activity inhibition rate (%) was calculated by the following equation.

Topoisomerase I activity inhibition rate (%) = ((a - b)/(c - b)) × 100

- a: fluorescence intensity in test compound-treated well
- b: fluorescence intensity in vehicle-treated well
- c: fluorescence intensity in topoisomerase I-free well

EXSUS (manufactured by CAC Croit Corporation) was used to represent the relationship between the topoisomerase I activity inhibition rate and the test compound concentration (sigmoid curve regression), and the test compound concentration in which the topoisomerase I activity inhibition rate became 50% ($IC_{50}$ value) was calculated.

In this test, "antitumor drug molecules or analogs thereof, or derivatives thereof (exemplified as U-compounds in the present description)" disclosed in the present description showed excellent topoisomerase I inhibitory activities. For example, each $IC_{50}$ value of U-001 (Production example 1), U-002 (Production example 2), U-003 (Production example 3), U-004 (Production example 4), U-005 (Production example 5), U-006 (Production example 6), and U-007 (Production example 7) was 15 μM or less, and almost the same level as exatecan tested under the same conditions.

[Test Example 3: Cell growth inhibition test]

3-1. Growth inhibition test of HER2-positive human gastric cancer cell line (NCI-N87)

NCI-N87 is generally used as a HER2-overexpressing cell line (Clin Cancer Res; 22(20) October 15, 2016). NCI-N87 (ATCC, CRL-5822) was cultured in RPMI 1640 Medium (ATCC Modification) (manufactured by Thermo Fisher Scientific, A10491-01) containing 10% fetal bovine serum (manufactured by Corning Inc., 35-011-CV) and 1% penicillin/streptomycin (manufactured by Thermo Fisher Scientific, 15140-122). The cells were seeded to a 96 well plate (manufactured by Corning Inc., 353377) at $1.0 \times 10^3$ cells / 100 μL / well. The cells were cultured under 5% $CO_2$ at 37°C overnight, then the medium was exchanged with new one (95 μL/well), and the cells were treated with a test compound or an anti-HER2 antibody (trastuzumab), which was adjusted to 2 μg/mL with PBS (manufactured by Thermo Fisher Scientific, 10010-023), at 5 μL/well (treatment concentration: 100 ng/mL). The cells were cultured under 5% $CO_2$ at 37°C for 6 days, then CellTiter-Glo (manufactured by Promega, G9241) was added thereto at 100 μL/well, and a microplate reader (FlexStation 3, manufactured by Molecular Device, LLC) was used to measure the luminescence amount of each well.

The growth inhibition rate (%) was calculated by the following equation.

$$\text{Growth inhibition rate (\%)} = ((a-b)/(a-c)) \times 100$$

- a: average luminescence amount in vehicle-treated well (n = 3)
- b: average luminescence amount in test compound-treated well (n = 3)
- c: average luminescence amount before test compound treatment (n = 3)

Each ADC Production example produced by reacting each Example of the present invention with the antibody

showed cell growth inhibitory activity, and among them, the compounds of ADC Production examples 1, 2, 3, 5, 6, 7, 8, 14, 15, 16, 17, 18, 19, 21, and 22 showed highly excellent cell growth inhibitory activities as compared to an anti-HER2 antibody. In this test, the cell growth inhibitory activity of anti-HER2 antibody was 14 to 35%.

3-2. Growth inhibition test of HER2-negative human breast cancer cell line (MDA-MB-231)

MDA-MB-231 is generally used as a triple-negative breast cancer cell line, and known to not express or weakly express HER2 (Breast Cancer (Auckl). 2010 May 20; 4: 35-41). MDA-MB-231 (ECACC, 92020424) was cultured in Leibovitz's L-15 Medium (ATCC Modification) (manufactured by Thermo Fisher Scientific, 11415-064) containing 10% fetal bovine serum (manufactured by Corning Inc., 35-011-CV) and 1% penicillin/streptomycin (manufactured by Thermo Fisher Scientific, 15140-122). The cells were seeded to a 96 well plate (manufactured by Corning Inc., 353377) at $1.0 \times 10^3$ cells / 100 μL / well. The cells were cultured at 37°C overnight, then the medium was exchanged with new one (95 μL/well), and the cells were treated with a test compound, which was adjusted to 2 ug/mL with PBS (manufactured by Thermo Fisher Scientific, 10010-023), at 5 μL/well (treatment concentration: 100 ng/mL). The cells were cultured at 37°C for 6 days, then CellTiter-Glo (manufactured by Promega, G9241) was added thereto at 100 μL/well, and a microplate reader (FlexStation 3, manufactured by Molecular Device, LLC) was used to measure the luminescence amount of each well.

The growth inhibition rate (%) was calculated in the same manner as the Test Example 3-1.

In this test, the cell growth inhibition rate of each ADC Production example was 10% or less, and thus almost no cell growth inhibitory activity was shown.

The above Test Example 3 (3-1 and 3-2) suggests that the antibody-drug conjugate obtained in each Production example was specifically bound to HER2-positive cells, then incorporated into the cells, the antitumor drug bound to said conjugate was released in the cells, and the drug contributed to cell growth inhibitory actions.

[Test Example 4: Tumor growth suppression test in nude mouse with subcutaneously implanted HER2-overexpessing human gastric cancer cell line (NCI-N87)]

A human gastric cancer cell line (NCI-N87) (manufactured by ATCC, Code No. CRL-5822) was cultured in RPMI 1640 medium (manufactured by Thermo Fisher Scientific, A10491-01) containing 10% FBS (manufactured by CORNING Inc., 35-011-CV) and 1% penicillin/streptomycin (manufactured by Thermo Fisher Scientific, 15140-122). A mixed solvent of PBS (manufactured by Thermo Fisher Scientific, 10010-023) and Matrigel (manufactured by CORNING Inc., 356231) (final volume 1:1) was used to prepare a cell suspension of $5.0 \times 10^7$ cells / mL. The prepared cell suspension was subcutaneously injected to the right flank region of BALB/c-nu/nu mice (female, provided by Charles River Laboratories Japan, Inc.) at 0.1 mL per mouse. After the mice were bred for a prescribed time period, the long diameter (mm) and the short diameter (mm) of the tumor was measured by an electronic caliper (manufactured by Mitutoyo Corporation), and the tumor volume was calculated by the following equation.

$$\text{Tumor volume (mm}^3\text{)} = (\text{long diameter}) \times (\text{short diameter}) \times (\text{short diameter}) \times 0.5$$

Individuals having a tumor volume within a range of 50 to 500 $mm^3$ were selected, divided into groups having almost the same level of tumor volume, and then a test compound or the solvent alone was intravenously administered to them. The first day of the administration start was set as Day 0, and the tumor volume was measured at Day 21. The tumor volume increase from Day 0 in the solvent-administered control group was set as 100%, and the tumor volume inhibition rate (%) in each administered dose of a test compound was calculated.

In this test, each compound of ADC Production examples 5, 10, 11, 14, 16, and 17 showed 50% or more as the tumor volume inhibition rate at a dose of 3 mg/kg, each compound of ADC Production examples 1, 2, 3, 4, 6, 7, 8, and 18 showed 100% or more as the tumor volume inhibition rate at a dose of 3 mg/kg, and each compound of ADC Production examples 2, 3, 15, and 19 showed 100% or more as the tumor volume inhibition rate at a dose of 1 mg/kg as shown in Figures 1, 2, 3, and 4. From these results, clear tumor suppression or tumor regression effects of the antibody-drug conjugates of the present invention were confirmed.

[Test Example 5: Metabolism test using human liver lysosomal fraction]

To a reaction composition solution (X10 Catabolic Buffer (manufactured by SEKISUI-Xenotech, LLC., K5200)) (60 μL) in which human liver lysosome (manufactured by SEKISUI-Xenotech, LLC., Cat No. H610.L 2.5 mg/mL) (corresponding to 0.125 mg of protein) was suspended and distilled water (manufactured by FUJIFILM Wako Pure Chemical Corporation, for HPLC) (10 μL) was added 1.2 μM test compound (antibody-drug conjugate produced in ADC Production examples) (25 μL) dissolved in PBS to prepare a reaction solution. After the reaction solution was incubated at

37°C for 24 hours, "an antitumor drug molecule or an analog thereof, or a derivative thereof" released from the antibody-drug conjugate into the reaction solution was quantified by LC-MS/MS (high performance liquid chromatography / mass spectrometer system) under the following conditions.

LC-MS/MS analysis conditions
LC: LC20 or LC30 HPLC system manufactured by Shimadzu Corporation

Column: Phenomenex Kinetex C18 (50x2.1 mm, 2.6 um)
Column temperature: 40°C
Flow rate: 0.4 mL/min
Mobile phase A: 0.1% aqueous solution of formic acid, Mobile phase B: 0.1% solution of formic acid in a mixture of 50% acetonitrile/methanol
Gradient: 0 to 1 min.; A/B = 90/10 → 10/90, 1 to 3 min.; A/B = 10/90, 3 to 3.01 min.; A/B = 10/90 -> 90/10 MS: 3200 QTrap manufactured by AB Sciex Pte. Ltd.
Ionization: ESI
Mode: Positive or Negative

The release rate (%) of drug was calculated by the following equation.

$$\text{Release rate (\%)} = \frac{\text{Quantitative value of antitumor drug molecule (nM)}}{\text{Concentration of test compound in reaction solution (nM) x DAR}} \times 100$$

As shown in the following Table 2, "an antitumor drug molecule or an analog thereof, or a derivative thereof" bound to each antibody-drug conjugate obtained in each ADC Production example 4 was confirmed to be released from each antibody-drug conjugate.

Table 2

| ADC Production example No. | Drug | Release rate (%) |
|---|---|---|
| 1 | MMAE | 68 |
| 2 | U-030 | 96 |
| 3 | U-031 | 81 |
| 4 | U-003 | 76 |
| 5 | U-029 | 7.3 |
| 6 | MMAE | 78 |
| 7 | MMAE | 86 |
| 8 | MMAE | 60 |
| 9 | U-036 | 98 |
| 10 | U-001 | 61 |
| 11 | DXd | 64 |
| 12 | U-035 | 3.5 |
| 13 | DXd | 76 |
| 14 | Eribulin | 61 |
| 15 | U-031 | 84 |
| 16 | Eribulin | 72 |
| 17 | Eribulin | 56 |
| 18 | Eribulin | 75 |
| 19 | U-031 | 1.1 |

(continued)

| ADC Production example No. | Drug | Release rate (%) |
|---|---|---|
| 20 | U-034 | 54 |

[Test Example 6: Metabolism test using human liver Cathepsin B]

To human liver Cathepsin B (manufactured by Sigma-Aldrich Co. LLC, Cat No. C8571 25 μg/vial) (corresponding to 400 Unit/mL) (5 μL), X10 Catabolic Buffer (manufactured by SEKISUI-Xenotech, LLC., K5200) (10 μL), 2.5 mM Bovine Serum Albumin (manufactured by FUJIFILM Wako Pure Chemical Corporation, Cat No. 012-15093) (5 μL), and distilled water (manufactured by FUJIFILM Wako Pure Chemical Corporation, for HPLC) (55 μL) was added 1.2 μM test compound (antibody-drug conjugate prepared in ADC Production examples) (25 μL) dissolved in PBS to prepare a reaction solution. After the reaction solution was incubated at 37°C for 24 hours, "an antitumor drug molecule or an analog thereof, or a derivative thereof" released from the antibody-drug conjugate into the reaction solution was quantified by LC-MS/MS (high performance liquid chromatography / mass spectrometer system) under the following conditions.

LC-MS/MS analysis conditions
LC: LC20 or LC30 HPLC system manufactured by Shimadzu Corporation

Column: Phenomenex Kinetex C18 (50x2.1 mm, 2.6 um)
Column temperature: 40°C
Flow rate: 0.4 mL/min
Mobile phase A: 0.1% aqueous solution of formic acid, Mobile phase B: 0.1% solution of formic acid in a mixture of 50% acetonitrile/methanol
Gradient: 0 to 1 min.; A/B = 90/10 → 10/90, 1 to 3 min.; A/B = 10/90, 3 to 3.01 min.; A/B = 10/90 → 90/10 MS: 3200 QTrap manufactured by AB Sciex Pte. Ltd.
Ionization: ESI
Mode: Positive or Negative

The release rate (%) of drug was calculated by the following equation.

$$\text{Release rate (\%)} = \frac{\text{Quantitative value of antitumor drug molecule (nM)}}{\text{Concentration of test compound in reaction solution (nM)} \times \text{DAR}} \times 100$$

As shown in the following Table 3, "an antitumor drug molecule or an analog thereof, or a derivative thereof" bound to each antibody-drug conjugate obtained in each ADC Production example was confirmed to be released from each antibody-drug conjugate.

Table 3

| ADC Production example No. | Drug | Release rate (%) |
|---|---|---|
| 1 | MMAE | 71 |
| 2 | U-030 | 96 |
| 3 | U-031 | 97 |
| 4 | U-003 | 97 |
| 5 | U-029 | 8.3 |
| 6 | MMAE | 67 |
| 7 | MMAE | 65 |
| 8 | MMAE | 41 |
| 9 | U-036 | 40 |
| 10 | U-001 | 28 |
| 11 | DXd | 30 |

(continued)

| ADC Production example No. | Drug | Release rate (%) |
|---|---|---|
| 12 | U-035 | 106 |
| 13 | DXd | 24 |
| 14 | Eribulin | 50 |
| 15 | U-031 | 102 |
| 16 | Eribulin | 62 |
| 17 | Eribulin | 58 |
| 18 | Eribulin | 64 |
| 19 | U-031 | 0.87 |
| 20 | U-034 | 11 |

[Test Example 7: Pharmacokinetic test]

Trastuzumab or a test compound was intravenously administered to a female mouse (BALB/c, Charles River Laboratories Japan, Inc.). After a prescribed time period, the blood was collected from the jugular vein or abdominal vein, and centrifuged to prepare a serum. The resulting serum was cryopreserved until the measurement.

The serum trastuzumab concentration was measured using SHIKARI Q-TRAS (manufactured by MATRIKS BIOTEK) according to the manufacturer's specified method.

The serum trastuzumab residual rate after 3 days from administration (%) was calculated by the following equation.

Serum trastuzumab residual rate after 3 days (%) = $(a/b) \times 100$

a: serum trastuzumab concentration after 3 days from administration (ng/mL)
b: serum trastuzumab concentration after 5 minutes from administration (ng/mL)

The residual rate of the antibody-drug conjugate obtained in each ADC Production example is shown in Table 4, and all of them are the same level as or greater than trastuzumab. Meanwhile, the residual rate of the Comparative ADC Production example-1 (an antibody-drug conjugate obtained from Comparative compound (MC-Val-Cit-PAB-MMAE (MedChemExpress, CAS No. 646502-53-6))) was 5.4 to 13%.

Table 4

| ADC Production example No. | Serum trastuzumab residual rate after 3 days from administration (%) |
|---|---|
| 1 | 35 |
| 2 | 27 |
| 3 | 32 |
| 4 | 31 |
| 5 | 37 |
| 6 | 57 |
| 7 | 34 |
| 8 | 24 |
| 9 | 20 |
| 10 | 23 |
| 11 | 28 |
| 12 | 18 |

(continued)

| ADC Production example No. | Serum trastuzumab residual rate after 3 days from administration (%) |
|---|---|
| 13 | 25 |
| 14 | 59 |
| 15 | 28 |
| 16 | 64 |
| 17 | 69 |
| 18 | 73 |
| 19 | 28 |
| 20 | 25 |
| 21 | 46 |
| 22 | 47 |
| 23 | 21 |
| 24 | 31 |
| Trastuzumab | 21 to 33 |
| Comparative ADC Production example-1 | 5.4 to 13 |

[Test Example 8: Size Exclusion Chromatography analysis]

As an index of stability of ADC Production examples, Size Exclusion Chromatography analysis (hereinafter referred to as "SEC analysis") was carried out.

SEC analysis conditions:

Device: HITACHI LC-2000
Column: Agilent AdvanceBio SEC 300Å, 7.8x300 mm, 2.7 um
Eluent: phosphate buffered saline (PBS, manufactured by WAKO)
Temperature: room temperature
Flow rate: 0.8 mL/min
Detection wavelength: 220 nm
Injection volume: 5μL
Analysis time: 25 min
Device: HITACHI LC-2000 system
Column: Agilent AdvanceBio SEC 300Å, 7.8x300 mm, 2.7 um
Eluent: phosphate buffered saline (PBS, manufactured by FUJIFILM Wako Pure Chemical Corporation)
Temperature: room temperature
Flow rate: 0.8 mL/min
Detection wavelength: 220 nm
Injection volume: 5 μL
Analysis time: 25 min

The SEC analysis HPLC chart of ADC Production example 2 is shown in Figure 5.

The antibody-drug conjugate of ADC Production example 2 was suggested to be stable, because it showed a sharp main peak, while showed just a small peak that seemed to be caused from an aggregate. For example, ADC Production examples 3, 8, 12, and 15 also showed the same results. Meanwhile, the Comparative ADC Production example-1 showed a broad peak.

[Test Example 9: Measurement of retention time in HPLC of Examples] (reference: Nat. Biotech., 33, 733-735 (2015))

As an index of hydrophilicity of the Examples, the retention time (RT) in HPLC analysis was measured, and compared.

HPLC conditions:

Device: LCMS2020 system_Shimadzu Corporation
Column: Acquity UPLC (registered trademark) BEH C18 (2.1x50 mm, 1.7 μm) _Waters
Eluent: Solution A: 0.1% aqueous solution of formic acid, Solution B: 0.1% solution of formic acid in acetonitrile
Gradient (B%): 25 (0 min) → 95 (1.5 min) → 95 (3.0 min)
Temperature: 40°C
Flow rate: 0.6 mL/min

The results are shown in Tables 5 and 6.

Table 5

| Example No. | Retention time in HPLC measurement (min) |
|---|---|
| 1 | 1.12 |
| 2 | 0.96 |
| 3 | 0.70 |
| 6 | 1.09 |
| 8 | 1.03 |
| Comparative compound-1 (MC-Val-Cit-PAB-MMAE) | 1.24 |

Table 6

| Example No. | Retention time in HPLC measurement (min) |
|---|---|
| 14 | 1.04 |
| 16 | 0.97 |
| 17 | 1.04 |
| 18 | 0.96 |
| 21 | 1.00 |
| 22 | 0.99 |
| 26 | 0.91 |
| Comparative compound-2 (Mal-PEG2-Val-Cit-PAB-Eribulin) | 1.19 |

The Examples for preparing the antibody-drug conjugates of the present invention were confirmed to have shorter RTs than comparative compounds as shown in both Tables 5 and 6. Said results suggest that the hydrophilicity of the "linker-drug" moiety of the antibody-drug conjugate of the present invention is higher than that of the comparative compounds, and thus suggest effects for increasing hydrophilicity by "introducing lactonyl group(s) into linker(s)" and "introducing lactonyl group(s) and other hydrophilic group(s) into linker(s)" which are features of the present invention. Further, the comparison between Examples (Example 1 vs Example 6) also showed that the higher number of lactonyl group(s) in linker(s) resulted in shorter RT, which also suggest effects for increasing hydrophilicity by the lactonyl group(s).

INDUSTRIAL APPLICABILITY

The conjugate precursor (I) or a salt thereof of the present invention is useful as a precursor for producing an excellent antibody-drug conjugate. Also, the synthetic intermediate (II) of conjugate precursor or a salt thereof of the

present invention is useful as a synthetic intermediate of said conjugate precursor (I).

## Claims

1. An antibody-drug conjugate precursor represented by the following general formula (I):

Z-L-D (I)

[wherein:

Z is a reactive group which can react with a functional group present in an antibody or a modified antibody;
D is an antitumor drug residue in which one hydrogen atom or one hydroxy group is removed from any position of an antitumor drug molecule or an analog thereof or a derivative thereof;
L is a linker which links Z to D; and
at least any one of D and L has one or more lactonyl group(s) as substituent(s) or protecting group(s) at any position(s)]

or a salt thereof.

2. The antibody-drug conjugate precursor according to claim 1 or a salt thereof, wherein Z is a maleimidyl group (the following formula (i)), an α-halogenomethylcarbonyl group (the following formula (ii)), an ethynylphosphonamidate group (the following formula (iii)), a carboxy group, an active ester of carboxy group, a sulfhydryl group, a hydroxy group, an amino group, an alkynyl group, a cycloalkynyl group, an azacycloalkynyl group, or an azide group ($-N_3$ group):

formula (i)

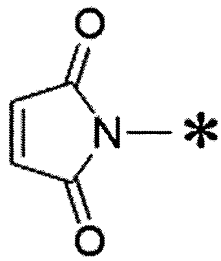

formula (ii)

$$Hal\text{-}CH_2\text{-}C(=O)\text{-}*$$

formula (iii)

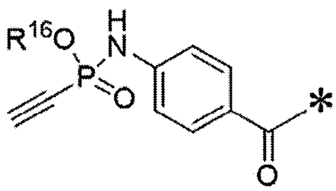

[wherein:

* is a point of attachment to L;
Hal is a fluorine atom, a chlorine atom, a bromine atom, or an iodine atom; and
$R^{16}$ is a methyl group, an ethyl group, or a - $CH_2CH_2OCH_2CH_2OH$ group].

3. The antibody-drug conjugate precursor according to claim 1 or 2 or a salt thereof, wherein Z is a maleimidyl group (the above formula (i)), an α-halogenomethylcarbonyl group (the above formula (ii)), an ethynylphosphonamidate group (the above formula (iii)), a carboxy group, an active ester of carboxy group, an alkynyl group, a cycloalkynyl group, an azacycloalkynyl group, or an azide group (-$N_3$ group).

4. The antibody-drug conjugate precursor according to any one of claims 1 to 3 or a salt thereof, wherein Z is a maleimidyl group (the above formula (i)), an α-halogenomethylcarbonyl group (the above formula (ii)), a carboxy group, or an active ester of carboxy group.

5. The antibody-drug conjugate precursor according to any one of claims 1 to 4 or a salt thereof, wherein D is an antitumor drug residue in which one hydrogen atom or one hydroxy group is removed from any position of camptothecin; auristatin E (AE) or monomethyl auristatin E (MMAE); maytansine; PBD (parabenzodiazepine) dimer; eribulin; 5-FU; PD-318088; AS-703026; TAK-733; LY-3023414; calicheamicin; paclitaxel; docetaxel; mitomycin C; bleomycin; cyclocytidine; vincristine; vinblastine; daunomycin; doxorubicin; dolastatin 10; superdox; ciprofloxacin; cadrofloxacin (CS-940); or an analog of said antitumor drug; or a derivative of said antitumor drug molecule or an analog thereof.

6. The antibody-drug conjugate precursor according to any one of claims 1 to 5 or a salt thereof, wherein D is an antitumor drug residue in which one hydrogen atom or one hydroxy group is removed from any position of camptothecin; auristatin E (AE) or monomethyl auristatin E (MMAE); maytansine; PBD (parabenzodiazepine) dimer; eribulin; 5-FU; PD-318088; AS-703026; TAK-733; LY-3023414; calicheamicin; paclitaxel; docetaxel; mitomycin C; bleomycin; cyclocytidine; vincristine; vinblastine; daunomycin; doxorubicin; dolastatin 10; superdox; or an analog of said antitumor drug; or a derivative of said antitumor drug molecule or an analog thereof.

7. The antibody-drug conjugate precursor according to any one of claims 1 to 6 or a salt thereof, wherein D is an antitumor drug residue in which one hydrogen atom or one hydroxy group is removed from any position of camptothecin; auristatin E (AE) or monomethyl auristatin E (MMAE); maytansine; PBD (parabenzodiazepine) dimer; eribulin; or an analog of said antitumor drug; or a derivative of said antitumor drug molecule or an analog thereof.

8. The antibody-drug conjugate precursor according to any one of claims 1 to 7 or a salt thereof, wherein D is an antitumor drug residue in which one hydrogen atom or one hydroxy group is removed from any position of an antitumor drug or an analog of said antitumor drug in which at least one hydroxy group present in the molecule is phosphorylated; or a derivative of said antitumor drug molecule or an analog thereof.

9. The antibody-drug conjugate precursor according to any one of claims 1 to 8 or a salt thereof, wherein

L is an optionally substituted alkylene group;
one or more methylene group(s) in the chain of said alkylene group is/are optionally replaced with one or more divalent group(s) independently selected from the group consisting of -C($R^1$)($R^2$)-; -O-; -N($R^3$)-; -N($R^3$)-N($R^3$)-; -S-; -Se-; -Si($R^4$)($R^5$)-; -S-S-; -Se-Se-; -SOm-; -SeOn-; - C(=C($R^6$)($R^7$))-; -C(=O)-; -C(=S)-; -C(=N($R^8$))-; -C(=N-O$R^9$)-; -C(=N-N($R^{10}$)($R^{11}$))-; -P(=O)($R^{12}$)-; -P(=O)(O$R^{13}$)-; -OP(=O)($R^{12}$)-O-; -O-P(=O)(O$R^{13}$)-O-; -C($R^{14}$)=; =C($R^{14}$)-; - C($R^{14}$)=C($R^{14}$)-; -N=; =N-; -C≡C-; $-(O-C(R^1)(R^2)-C(R^1)(R^2))_{1-30}-$; $-(C(R^1)(R^2)-C(R^1)(R^2)-0)_{1-30}-$; an optionally substituted alkenylene group; an optionally substituted alkynylene group; an optionally substituted cycloalkylene group; an optionally substituted cycloalkenylene group; an optionally substituted cycloalkynylene group; an optionally substituted azacycloalkynylene group; an optionally substituted arylene group; an optionally substituted heteroarylene group; and an optionally substituted heterocyclylene group;
$R^1$, $R^2$, $R^3$, $R^4$, $R^5$, $R^6$, $R^7$, $R^8$, $R^9$, $R^{10}$, $R^{11}$, $R^{12}$, $R^{13}$, and $R^{14}$ are each independently a group selected from the group consisting of a hydrogen atom, an optionally substituted alkyl group, an optionally substituted alkenyl group, an optionally substituted alkynyl group, an optionally substituted cycloalkyl group, an optionally substituted cycloalkenyl group, an optionally substituted cycloalkynyl group, an optionally substituted azacycloalkynyl group, an optionally substituted aryl group, an optionally substituted heteroaryl group, and an optionally substituted heterocyclyl group,
when $R^3$ is an alkyl group, said alkyl group is optionally combined with an alkyl group in an adjacent methylene group to form a cyclic structure; and
m and n are each independently an integer of 0 to 2.

10. The antibody-drug conjugate precursor according to claim 9 or a salt thereof, wherein

L is an optionally substituted alkylene group;

one or more methylene group(s) in the chain of said alkylene group is/are replaced with one or more divalent group(s) independently selected from the following formula group:
formula group

-O-C(H or $C_1$-$C_4$ alkyl) (H or $C_1$-$C_4$ alkyl)-;
-S-C(H or $C_1$-$C_4$ alkyl) (H or $C_1$-$C_4$ alkyl)-;
-N(H or $C_1$-$C_4$ alkyl)-;
-N(H or $C_1$-$C_4$ alkyl) -O-C(H or $C_1$-$C_4$ alkyl)(C(H or $C_1$-$C_4$ alkyl) -;
-C(H or $C_1$-$C_4$ alkyl)-$SO_{0-2}$-C(H or $C_1$-$C_4$ alkyl)-;
-C(H or $C_1$-$C_4$ alkyl)-S-S-C(H or $C_1$-$C_4$ alkyl)-;
-C(H or $C_1$-$C_4$ alkyl)=C(H or $C_1$-$C_4$ alkyl)-;
-C(H or $C_1$-$C_4$ alkyl) =N-;
-C(H or $C_1$-$C_4$ alkyl)=N-O-;
-C(H or $C_1$-$C_4$ alkyl)=N-N(H or $C_1$-$C_4$ alkyl)-;
-C(=C(H or $C_1$-$C_4$ alkyl) (H or $C_1$-$C_4$ alkyl))-;
-C(=N-O(H or $C_1$-$C_4$ alkyl))-;
-C(=N-N(H or $C_1$-$C_4$ alkyl) (H or $C_1$-$C_4$ alkyl)-;
-O-Si($C_1$-$C_4$ alkyl $CH_3$)($C_1$-$C_4$ alkyl) -O-;
-C(H or $C_1$-$C_4$ alkyl)-N($C_1$-$C_4$ alkyl)-$CH_2$-;
-C(H or $C_1$-$C_4$ alkyl) (H or $C_1$-$C_4$ alkyl)-C(=O)-C(H or $C_1$-$C_4$ alkyl)(H or $C_1$-$C_4$ alkyl)-;
-C(=O)-O-;
-C(-O)-S-;
-C(=O)-N(H or $C_1$-$C_4$ alkyl)-;
-O-C(=O)-O-;
-N(H or $C_1$-$C_4$ alkyl)-C(=O)-N(H or $C_1$-$C_4$ alkyl)-;
-C(=O)-N(H or $C_1$-$C_4$ alkyl) -C(H or $C_1$-$C_4$ alkyl) (H or $C_1$-$C_4$ alkyl)-;
-C(-O)-$(CH_2)_{1-10}$-$(O-CH_2CH_2)_{1-20}$-O-$(CH_2)_{1-10}$-C(=O)-;
-C(=O)-$(CH_2)_{1-10}$-$(O-CH_2CH_2)_{1-20}$-O-$(CH_2)_{1-10}$-$CH_2$-NH-;
-NH-$CH_2$-$(CH_2)_{1-10}$$(O-CH_2CH_2)_{1-20}$-O-$(CH_2)_{1-10}$-$CH_2$-NH-;
-C(=O)-N($C_1$-$C_4$ alkyl)-$CH_2CH_2$-N($C_1$-$C_4$ alkyl)-C(=O)-;

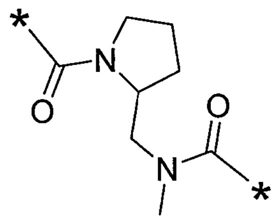

[wherein * is a point of attachment to an adjacent group];
-P(=O)(OH)-O-;
-O-P(=O)(OH)-O-;
-O-P(=S)(OH)-O-;
-O-P(=O)(OH)-S-;
-O-P(=O)(OH)-O-P(=O)(OH)-O-;
-O-P(=O)(OH)-O-P(=O)(OH)-O-P(=O)(OH)-O-;
-CH($CH_2$-$NH_2$)-;
-CH($CH_2$-NH($C_1$-$C_4$ alkyl))-;
-CH($CH_2$-N($C_1$-$C_4$ alkyl)$_2$)-;
-CH($CH_2$-NH-C(=O)-$(CH_2CH_2-O-)_{0-20}$-$C_1$-$C_4$ alkyl)-;
-CH($CH_2$-NH-C(=O)-$(CH_2)_{0-20}$-lactonyl)-;
-CH($CH_2$-NH-C(=O)-$(CH_2CH_2-O-)_{1-20}$-$(CH_2)_{1-10}$-$CH_2$-lactonyl)
-CH($CH_2$-NH-C(=O)-$(CH_2CH_2-O-)_{1-20}$-$(CH_2)_{1-10}$-$CH_2$-NH-lactonyl)-;
-CH($CH_2$-NH-C(=O)-$(CH_2CH_2-O-)_{1-20}$-$(CH_2)_{1-10}$-$CH_2$-NH-C(=O)-lactonyl)-;
-CH($CH_2$-NH-C(=O)-$(CH_2CH_2-O-)_{1-20}$-$(CH_2)_{1-10}$-C(=O)-NH-lactonyl)-;
-CH($CH_2$-NH-$(CH_2)_{1-20}$-$(O)_{0-1}$-(P(=O)$(OH)_2$)-;
-CH($CH_2$-NH-C(=O)-$(CH_2)_{1-20}$-(P(=O)$(OH)_2$)-;
-CH($CH_2$-NH-C(=O)-$(CH_2)_{1-20}$-C(=O)-NH-$(CH_2)_{1-10}$-(P(=O)$(OH)_2$)-;

-CH($CH_2$-NH-C(=O)-($CH_2CH_2$-O-)$_{1-20}$-($CH_2$)$_{1-10}$-$CH_2$-(P(=O)(OH)$_2$)-;
-CH($CH_2$-NH-C(=O)-($CH_2CH_2$-O-)$_{1-20}$-($CH_2$)$_{1-10}$-C(=O)-NH-($CH_2$)$_{1-10}$-(P(=O)(OH)$_2$)-;
-CH($CH_2$-NH-($CH_2$)$_{1-10}$-C(H, OH, Cl, $NH_2$, or $C_1$-$C_4$ alkyl)(P(=O)(OH)$_2$)$_2$)-;
-CH($CH_2$-NH-C(=O)-($CH_2$)$_{1-10}$-C(=O)-NH-($CH_2$)$_{1-10}$-C(H, OH, Cl, $NH_2$, or $C_1$-$C_4$ alkyl)(P(=O)(OH)$_2$)$_2$)-;
-CH($CH_2$-NH-C(=O)-($CH_2CH_2$-O-)$_{1-20}$-($CH_2$)$_{1-10}$-C(=O)-NH-($CH_2$)$_{1-10}$-C(H, OH, Cl, $NH_2$, or $C_1$-$C_4$ alkyl) (P(=O)(OH)$_2$)$_2$)-;
-(cis)-CH=CH-P(=O)(O-$CH_2CH_3$)-NH-(phenylene)-C(=O)-;
-C(=O)-(cyclohexylene)-;
-(succinimidylene)-;
-Ser-;
-Cys-;
-Ama-:

-Asp-;
-Glu-;
-Orn-;
-Lys-;
-ValLys-;
-ValCit-;
-ValAla-;
-GlyGly-C(=O)-NH-(optionally substituted phenylene)-$CH_2$-;
-PheLys-C(=O)-NH-(optionally substituted phenylene)-$CH_2$-;
-ValLys-C(=O)-NH-(optionally substituted phenylene)-$CH_2$-;
-ValCit-C(=O)-NH-(optionally substituted phenylene)-$CH_2$-;
-ValAla-C(=O)-NH-(optionally substituted phenylene)-$CH_2$-;
-AspValCit-C(=O)-NH-(optionally substituted phenylene)-$CH_2$-;
-LysAspValCit-C(=O)-NH-(optionally substituted phenylene)-$CH_2$-;
-GluValCit-C(=O)-NH-(optionally substituted phenylene)-$CH_2$-;
-LysValCit-C(=O)-NH-(optionally substituted phenylene)-$CH_2$-;
-SerValCit-C(=O)-NH-(optionally substituted phenylene)-$CH_2$-;
-AspValAla-C(=O)-NH-(optionally substituted phenylene)-$CH_2$-;
-GluValAla-C(=O)-NH-(optionally substituted phenylene)-$CH_2$-;
-LysValAla-C(=O)-NH-(optionally substituted phenylene)-$CH_2$-;
-SerValAla-C(=O)-NH-(optionally substituted phenylene)-$CH_2$-;
-GlyGlyPheGly-C(=O)-NH-(optionally substituted phenylene)-$CH_2$-;
-AspGlyGlyPheGly-C(=O)-NH-(optionally substituted phenylene)-$CH_2$-;
-GluGlyGlyPheGly-C(=O)-NH-(optionally substituted phenylene)-$CH_2$-;
-LysGlyGlyPheGly-C(=O)-NH-(optionally substituted phenylene)-$CH_2$-;
-SerGlyGlyPheGly-C(=O)-NH-(optionally substituted phenylene)-$CH_2$-;
-AspAspAspAspAsp-C(=O)-NH-(optionally substituted phenylene)-$CH_2$-;
-GlyGly-C(=O)-NH-(optionally substituted phenylene)-$CH_2$-O-C(=O)-;
-PheLys-C(=O)-NH-(optionally substituted phenylene)-$CH_2$-O-C(=O)-;
-ValLys-C(=O)-NH-(optionally substituted phenylene)-$CH_2$-O-C(=O)-;
-ValCit-C(=O)-NH-(optionally substituted phenylene)-$CH_2$-O-C(=O)-;
-ValAla-C(=O)-NH-(optionally substituted phenylene)-$CH_2$-O-C(=O)-;
-AspValCit-C(=O)-NH-(optionally substituted phenylene)-$CH_2$-O-C(=O)-;
-GluValCit-C(=O)-NH-(optionally substituted phenylene)-$CH_2$-O-C(=O)-;
-LysValCit-C(=O)-NH-(optionally substituted phenylene)-$CH_2$-O-C(=O)-;
-SerValCit-C(=O)-NH-(optionally substituted phenylene)-$CH_2$-O-C(=O)-;
-AspValAla-C(=O)-NH-(optionally substituted phenylene)-$CH_2$-O-C(=O)-;
-GluValAla-C(=O)-NH-(optionally substituted phenylene)-$CH_2$-O-C(=O)-;
-LysValAla-C(=O)-NH-(optionally substituted phenylene)-$CH_2$-O-C(=O)-;
-SerValAla-C(=O)-NH-(optionally substituted phenylene)-$CH_2$-O-C(=O)-;
-GlyGlyPheGly-C(=O)-NH-(optionally substituted phenylene)-$CH_2$-O-C(=O)-;
-AspGlyGlyPheGly-C(=O)-NH-(optionally substituted phenylene)-$CH_2$-O-C(=O)-;
-GluGlyGlyPheGly-C(=O)-NH-(optionally substituted phenylene)-$CH_2$-O-C(=O)-;
-LysGlyGlyPheGly-C(=O)-NH-(optionally substituted phenylene)-$CH_2$-O-C(=O)-;
-SerGlyGlyPheGly-C(=O)-NH-(optionally substituted phenylene)-$CH_2$-O-C(=O)-;

-AspAspAspAspAsp-C(=O)-NH-(optionally substituted phenylene)-$CH_2$-O-C(=O)-;
-GlyGlyPheGly-C(=O)-NH-$CH_2$-;
-AspGlyGlyPheGly-C(=O)-NH-$CH_2$-;
(in said amino acid residue, and an amino acid residue in said peptide,

the carboxy group(s) in the side chain(s) of Ama, Asp, and Glu is/are optionally converted into (alkyl) ester(s), (aminoalkyl) ester(s), ((N-alkylamino)alkyl) ester(s), ((N,N-dialkylamino)alkyl) ester(s), ((trialkylammonium)alkyl) ester(s), (lactonyl) ester(s), ((lactonyl)alkyl) ester(s), ((phosphoryl)alkyl) ester(s), or ((bisphosphoryl)alkyl) ester(s), or unsubstituted amide(s), alkylamide(s), dialkylamide(s), ((N-alkylamino)alkyl)amide(s), ((N,N-dialkylamino)alkyl)amide(s), ((trialkylammonium)alkyl)amide(s), (lactonyl)amide(s), ((lactonyl)alkyl)amide(s), (phosphoryl)amide(s), ((phosphoryl)alkyl)amide(s), or ((bisphosphoryl)alkyl)amide(s);
the amino group(s) in the side chain(s) of Lys and Orn optionally has/have one or two alkyl group(s), alkylcarbonyl group(s), (aminoalkyl)carbonyl group(s), ((N-alkylamino)alkyl)carbonyl group(s), ((N,N-dialkylamino)alkyl)carbonyl group(s), ((trialkylammonium)alkyl)carbonyl group(s), lactonyl group(s), (lactonyl)alkyl group(s), (lactonyl) carbonyl group(s), ((lactonyl)alkyl)carbonyl group(s), phosphoryl group(s), (phosphoryl)alkyl group(s), ((phosphoryl)alkyl)carbonyl group(s), (bisphosphoryl)alkyl group(s), or ((bisphosphoryl)alkyl)carbonyl group(s) as substituent(s); and
the hydroxy group in the side chain of Ser and the sulfhydryl group in the side chain of Cys optionally has/have alkyl group(s), alkylcarbonyl group(s), aminoalkyl group(s), (aminoalkyl)carbonyl group(s), (N-alkylamino)alkyl group(s), ((N-alkylamino)alkyl)carbonyl group(s), (N,N-dialkylamino)alkyl group(s), ((N,N-dialkylamino)alkyl) carbonyl group(s), (trialkylammonium)alkyl group(s), ((trialkylammonium)alkyl)carbonyl group(s), lactonyl group(s), (lactonyl)alkyl group(s), (lactonyl)carbonyl group(s), ((lactonyl)alkyl)carbonyl group(s), phosphoryl group(s), (phosphoryl)alkyl group(s), ((phosphoryl)alkyl)carbonyl group(s), (bisphosphoryl)alkyl group(s), or ((bisphosphoryl)alkyl)carbonyl group(s) as substituent(s)); and
1,2,3-triazolylene groups represented by the following formulae:

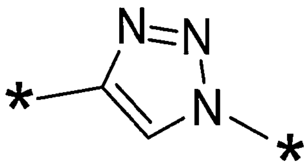

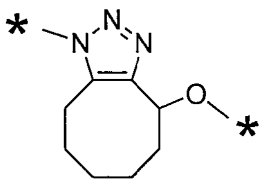

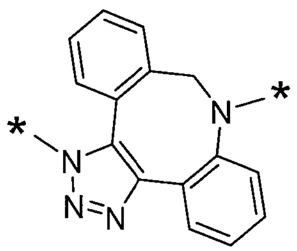

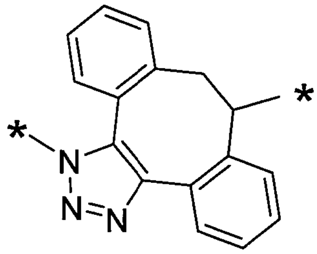

and

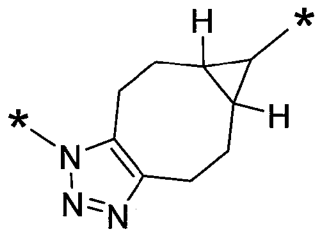

[wherein * is a point of attachment to an adjacent group].

**11.** The antibody-drug conjugate precursor according to claim 9 or a salt thereof, wherein L is any one divalent group selected from the following formula group:

formula group

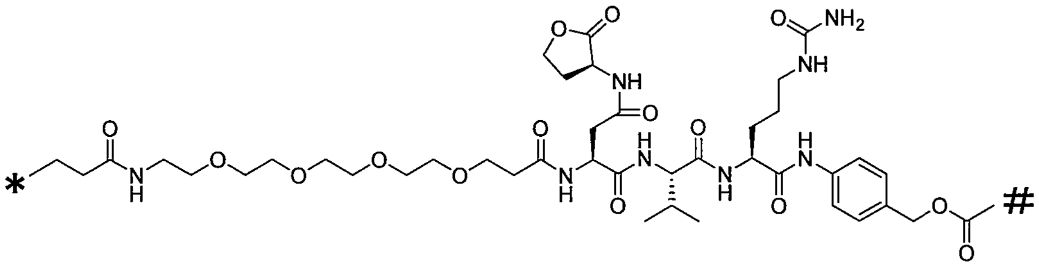

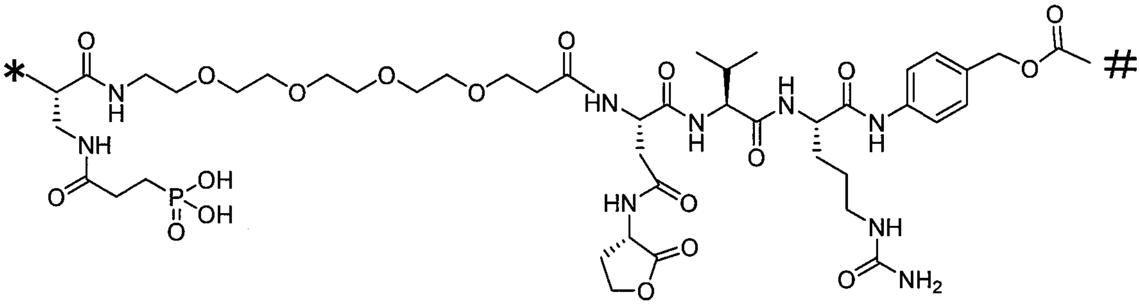

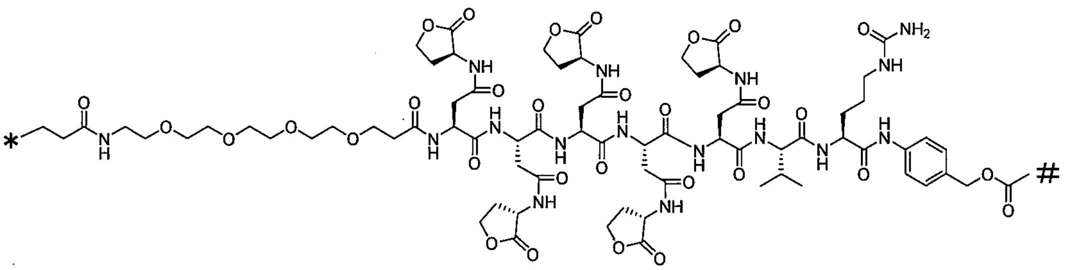

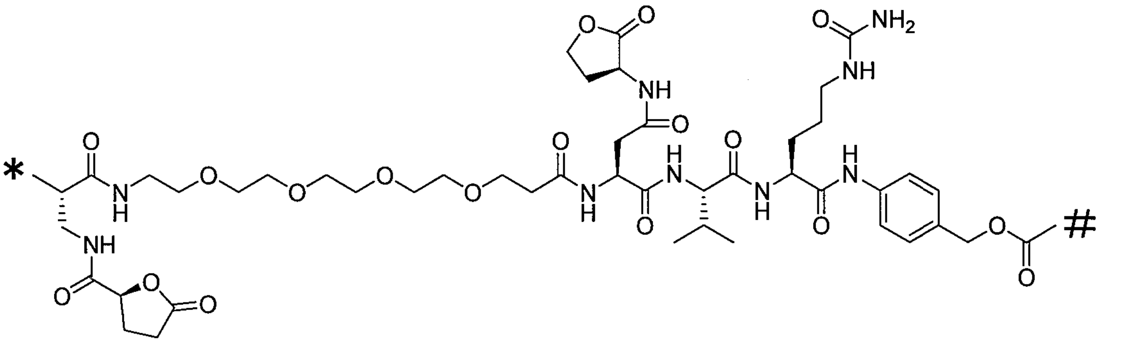

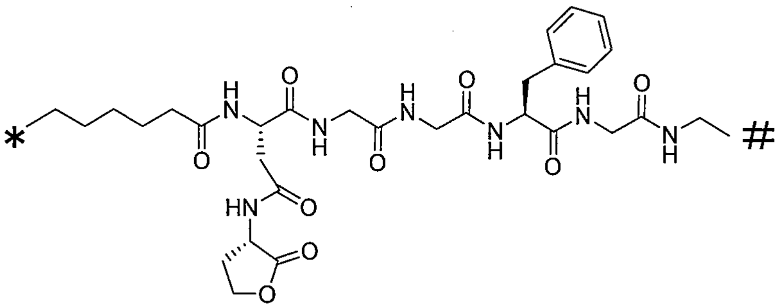

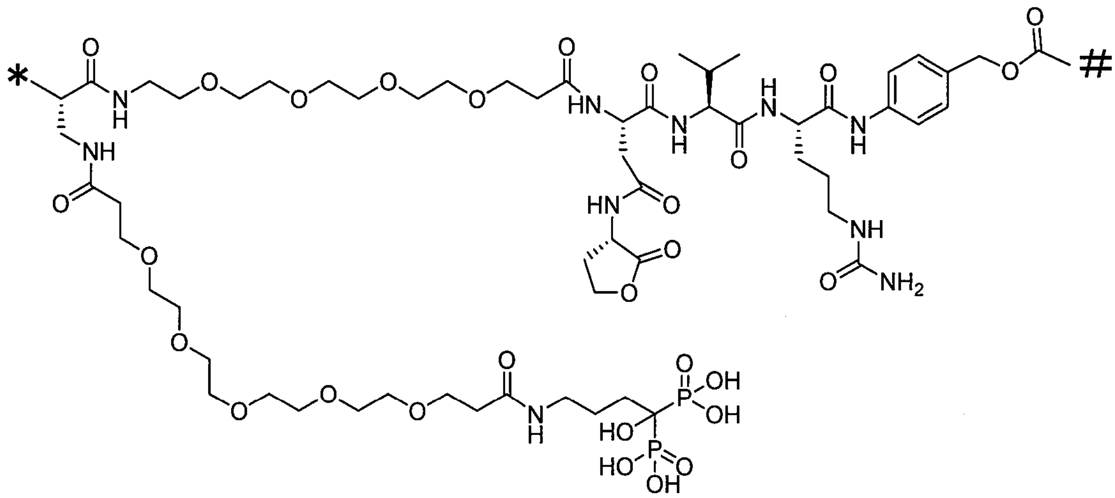

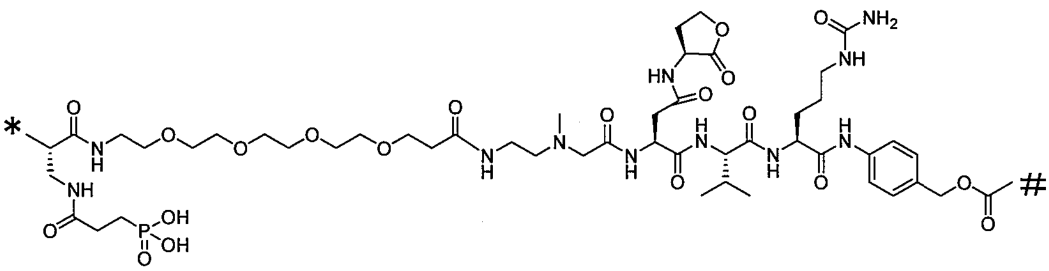

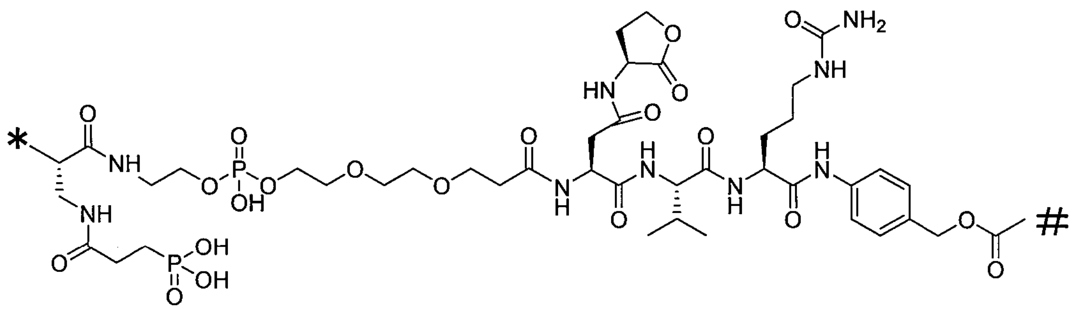

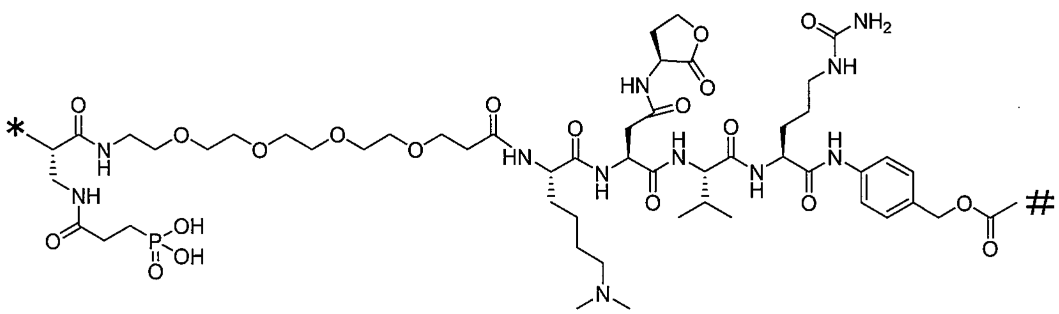

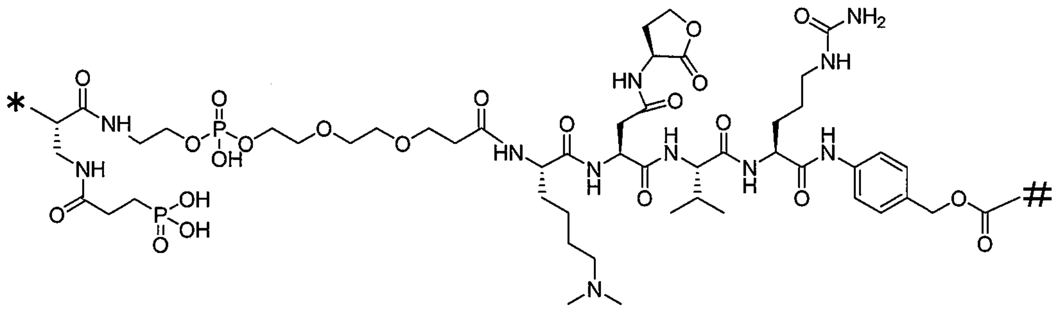

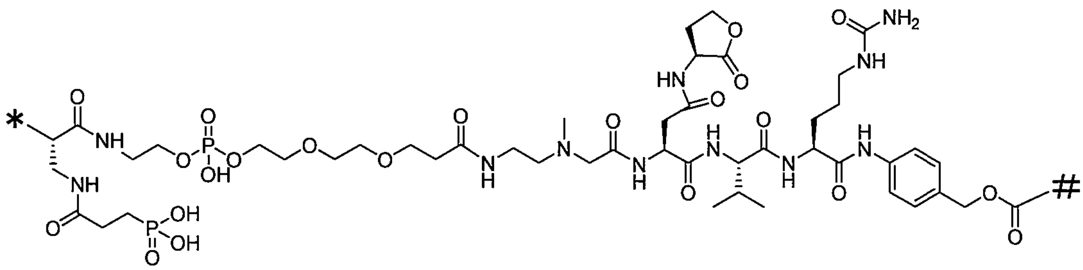

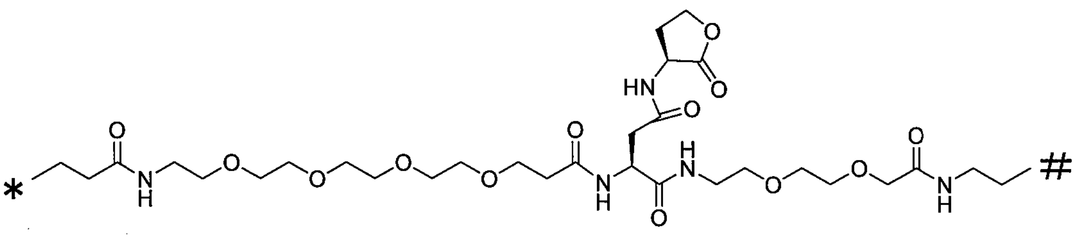

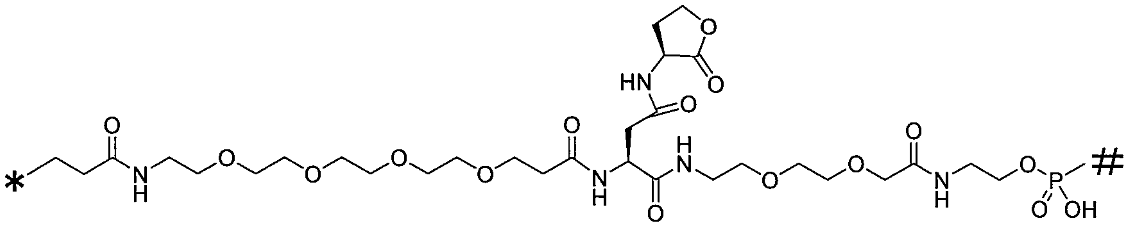

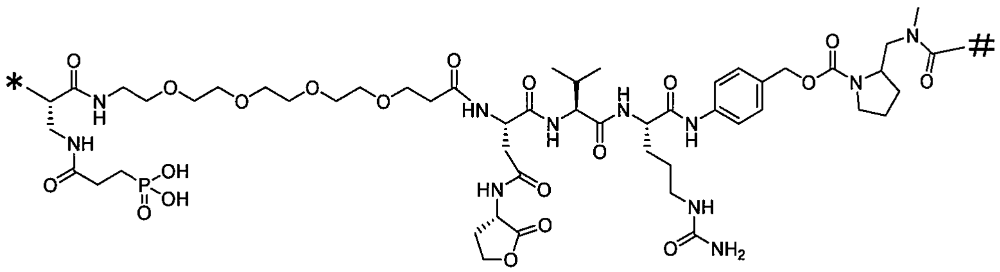

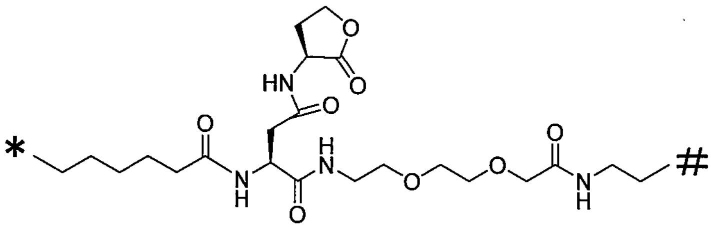

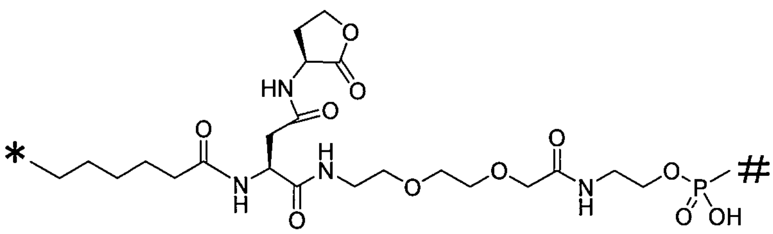

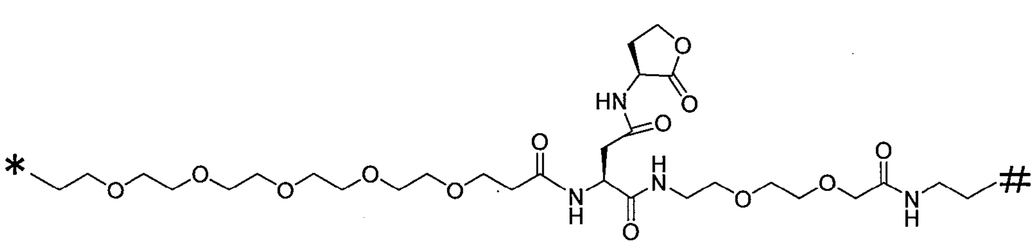

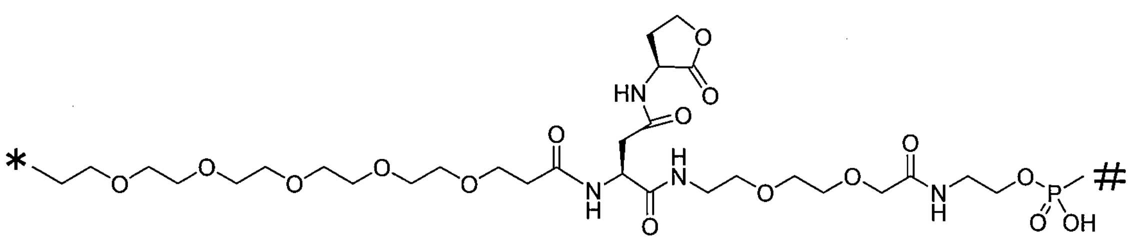

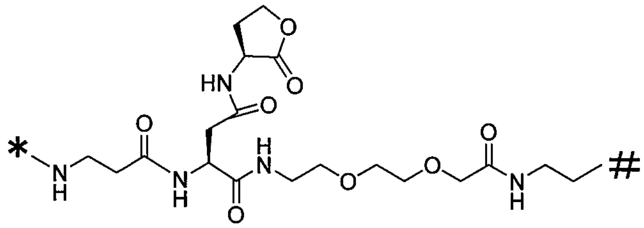

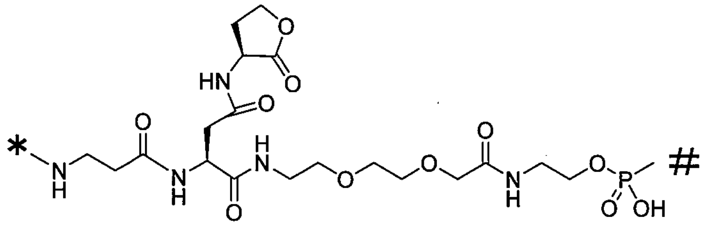

[wherein:

* is a point of attachment to the reactive group Z; and
# is a point of attachment to the antitumor drug residue D].

**12.** The antibody-drug conjugate precursor according to any one of claims 1 to 11 or a salt thereof, wherein

L has one or more group(s) selected from a lactonyl group, a phosphoryl group ($-P(=O)(OH)_2$); a phosphorylalkylcarbonyl group ($-C(=O)$-alkylene-$P(=O)(OH)_2$); a bisphosphorylmethyl group (-C(H, OH, $NH_2$, Cl, or alkyl) $(P(=O)(OH)_2)_2$); an amino group; a monoalkylamino group; a dialkylamino group; a cyclic amino group formed by combining two alkyl groups of said dialkylamino group with the nitrogen atom to which they are attached; and a trialkylammonium group, at any position(s) as substituent(s) or protecting group(s),
wherein when the total number of said group(s) is two or more, they are identical to or different from each other.

**13.** An antibody-drug conjugate precursor selected from the group consisting of:

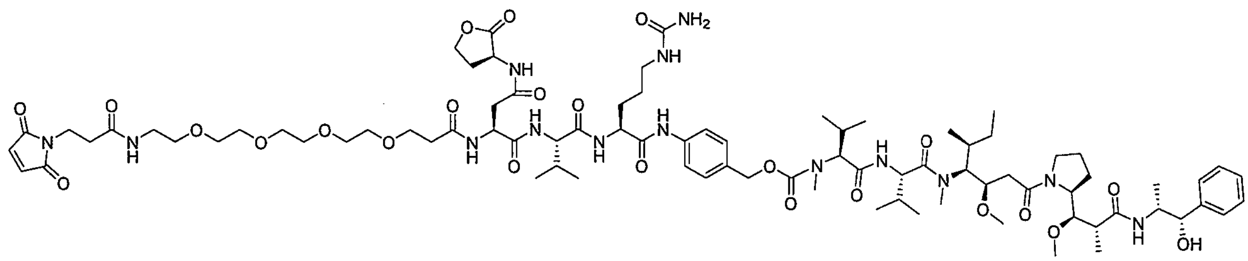

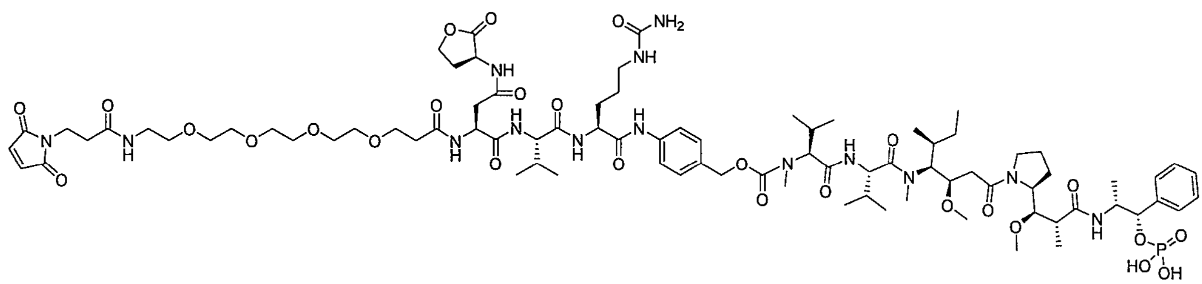

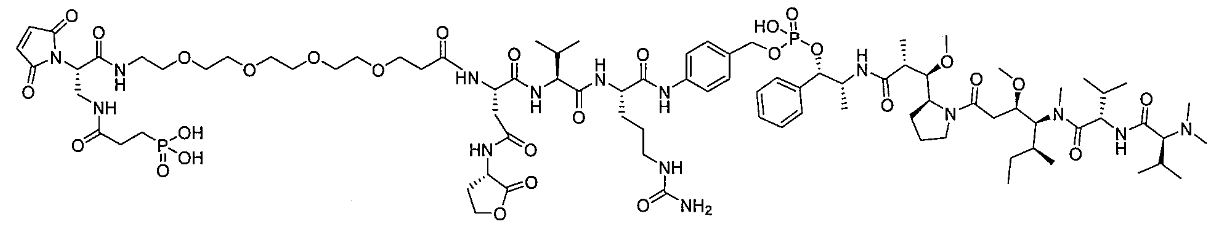

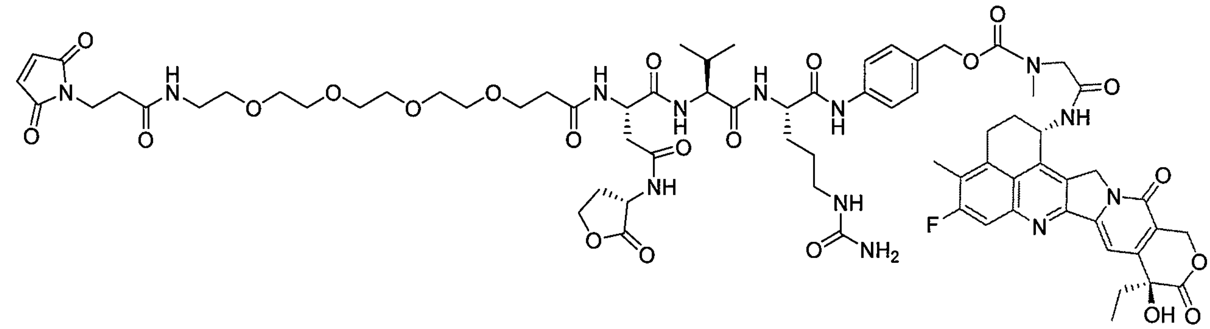

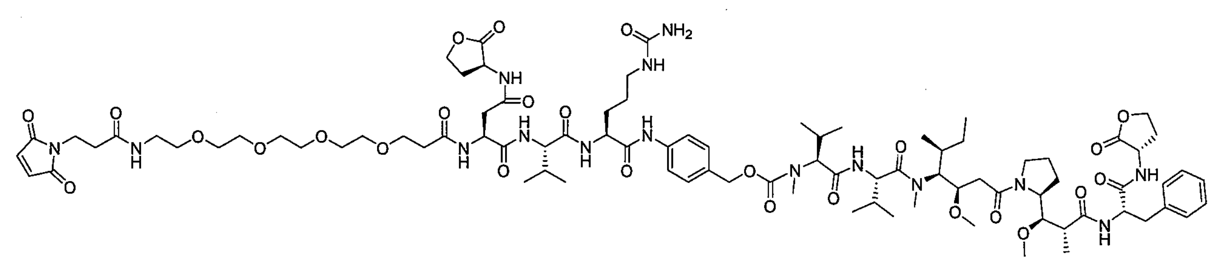

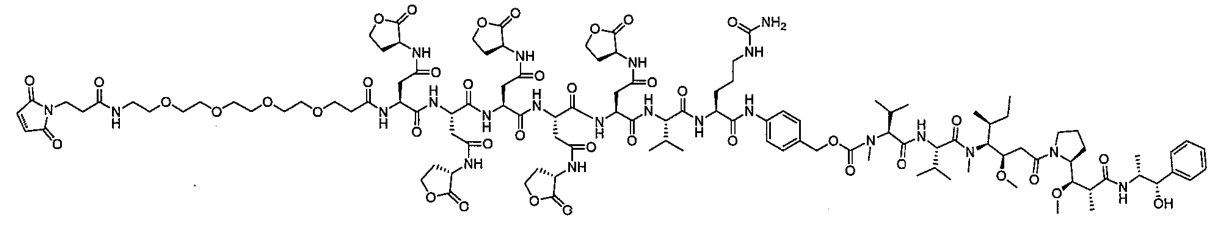

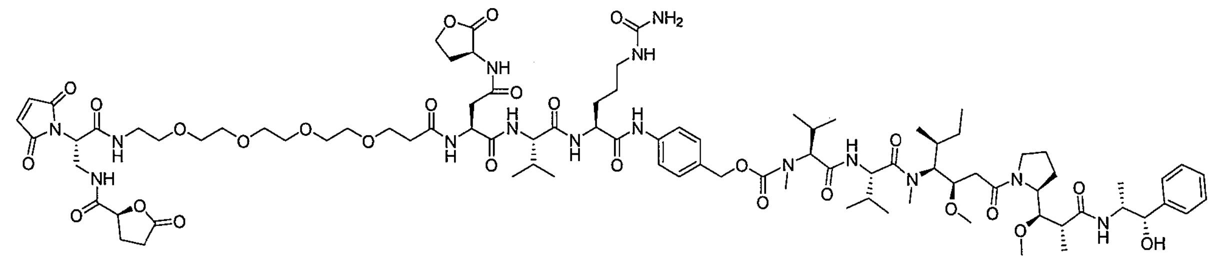

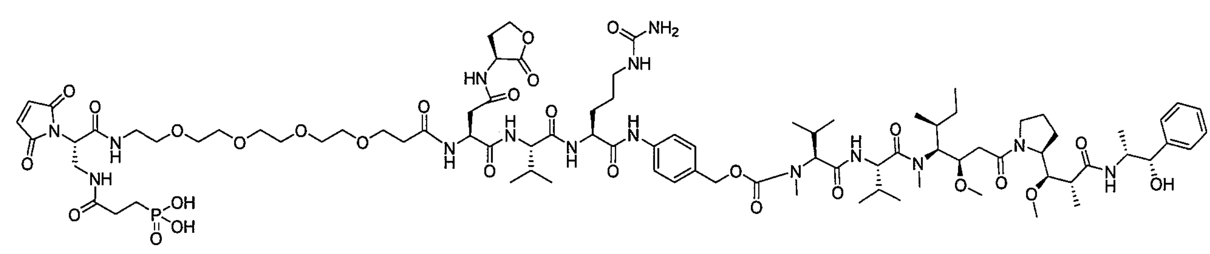

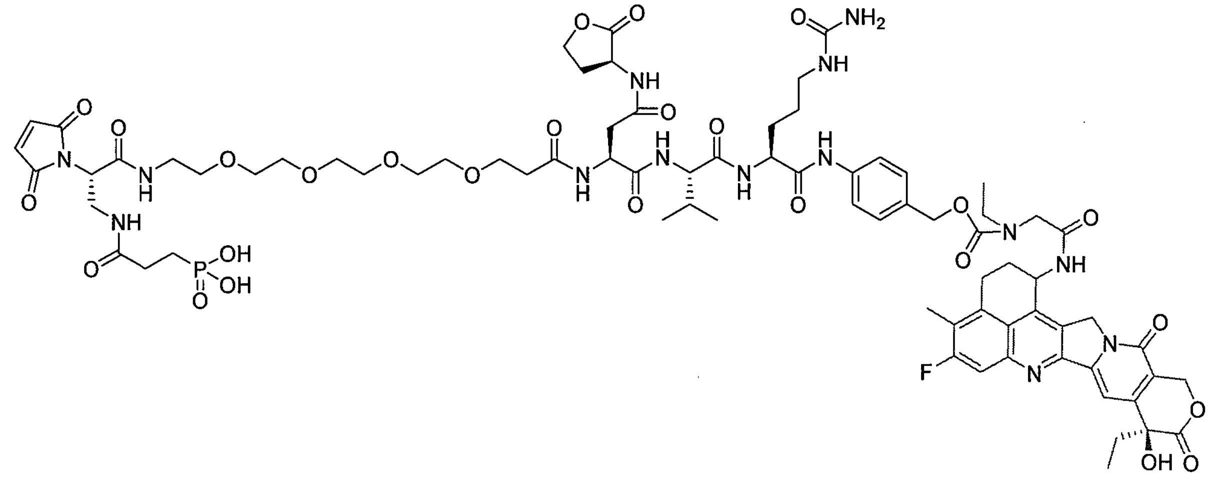

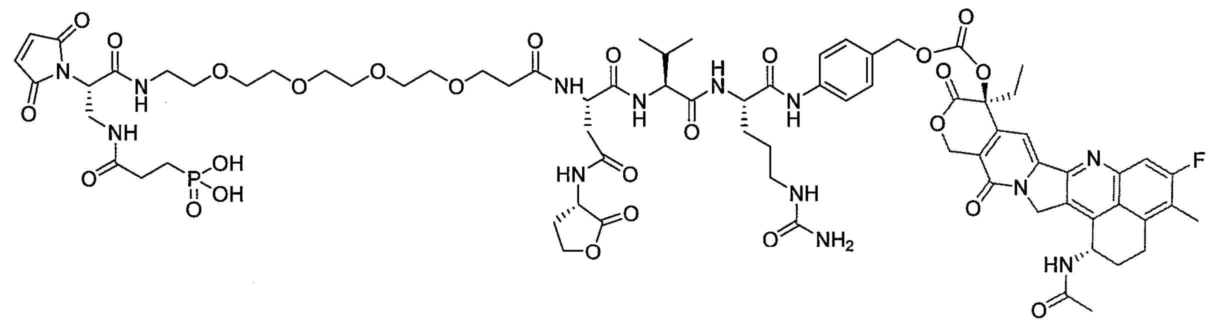

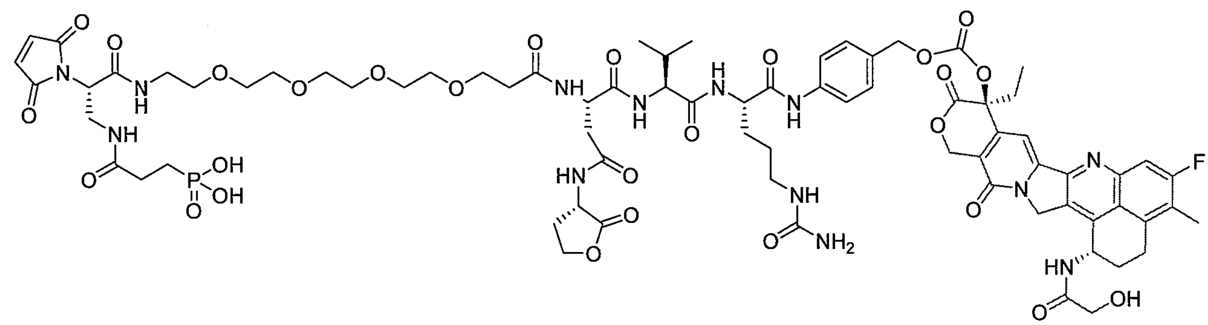

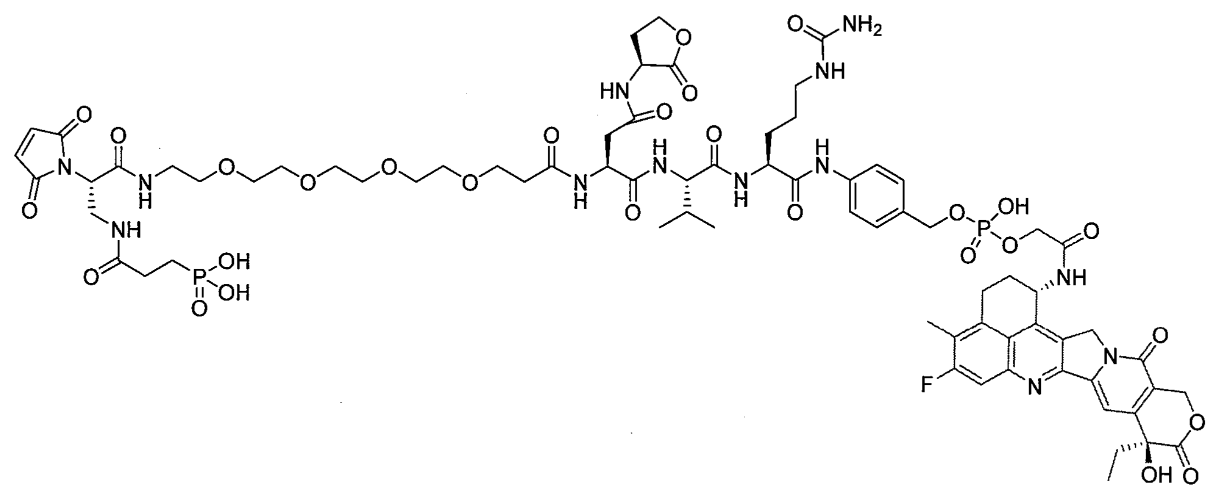

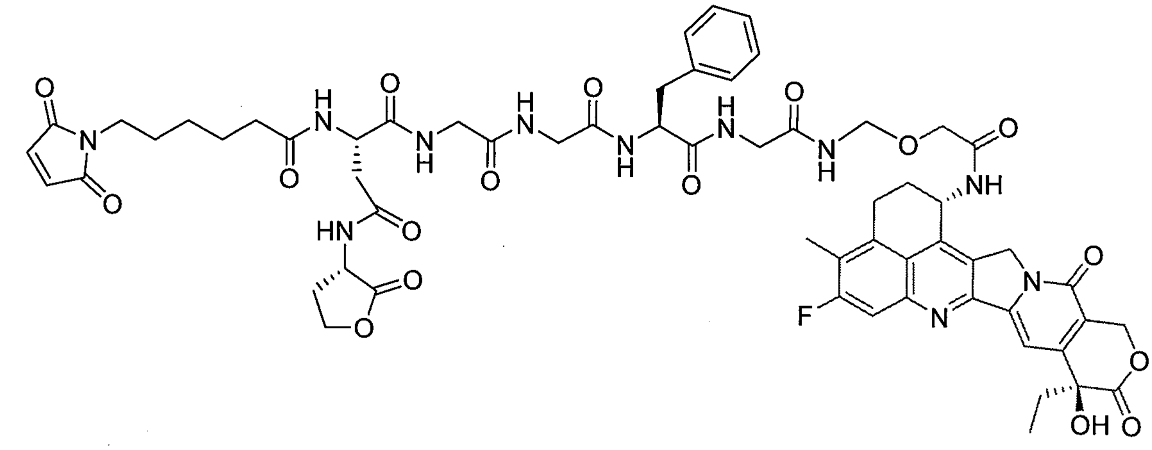

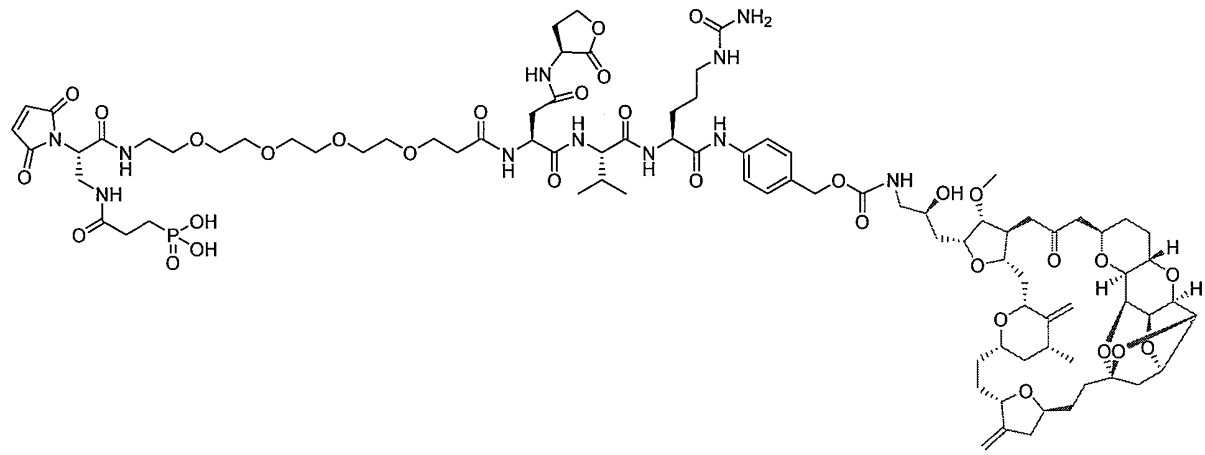

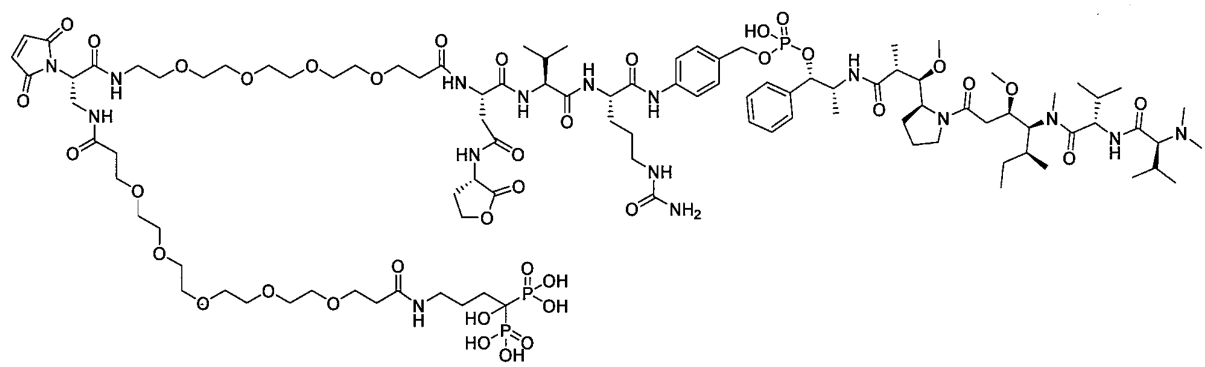

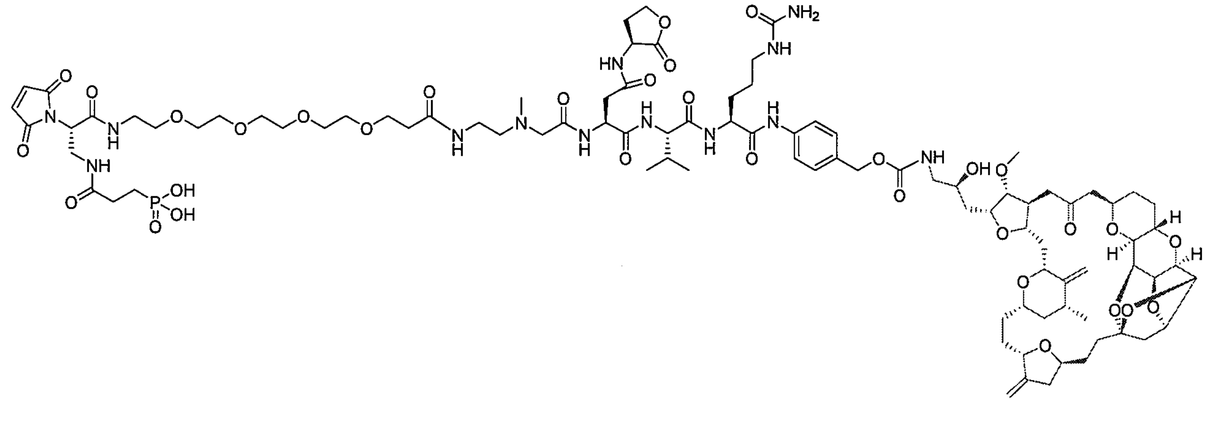

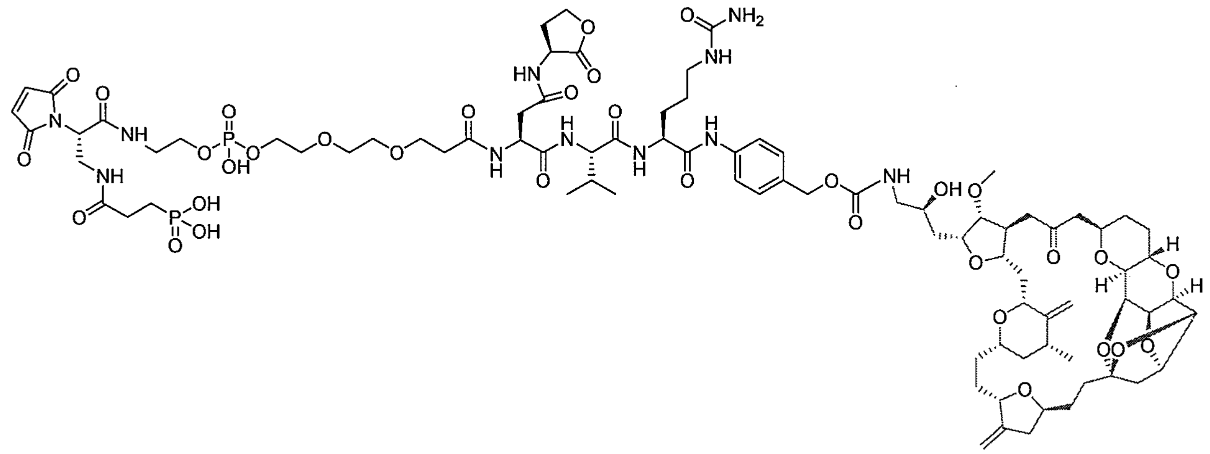

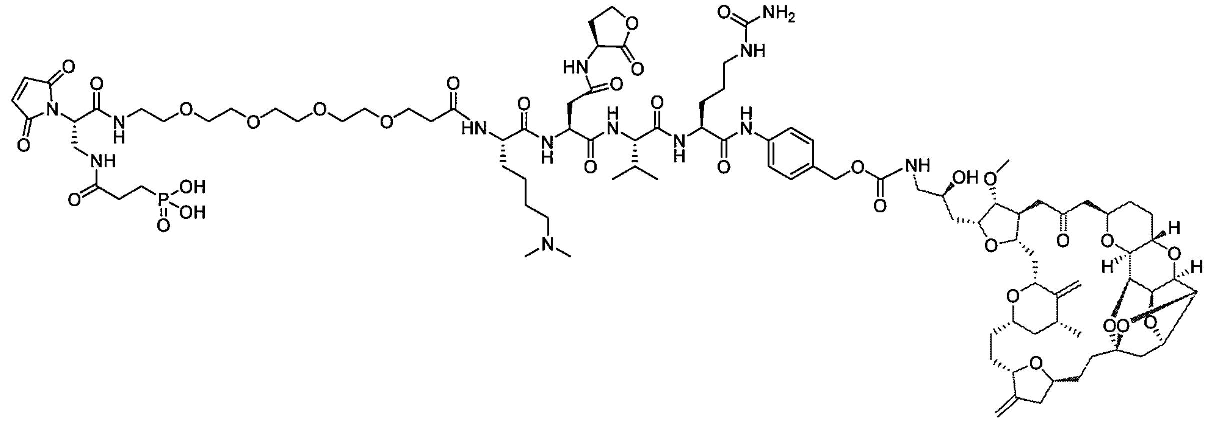

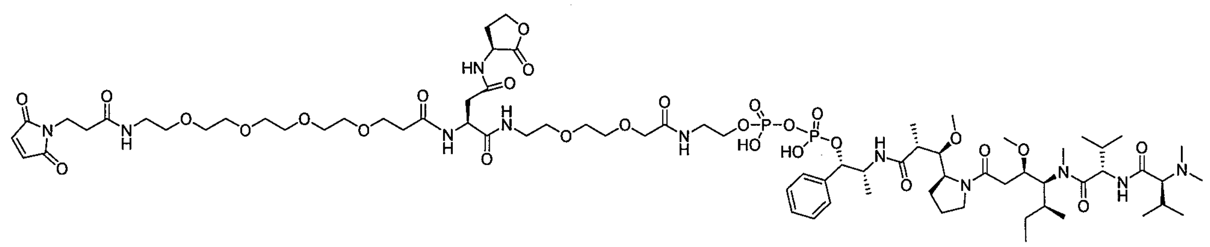

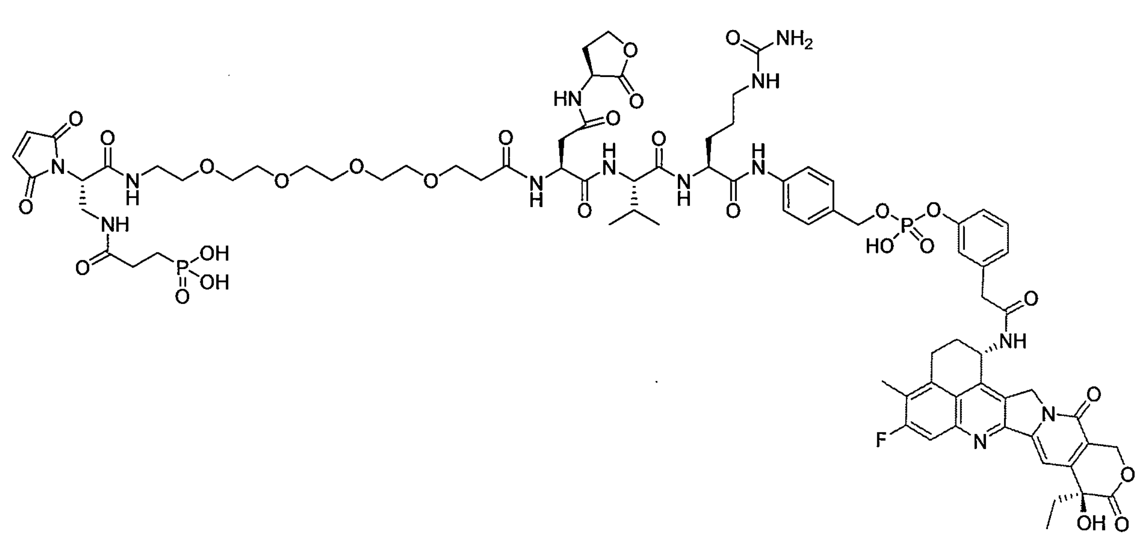

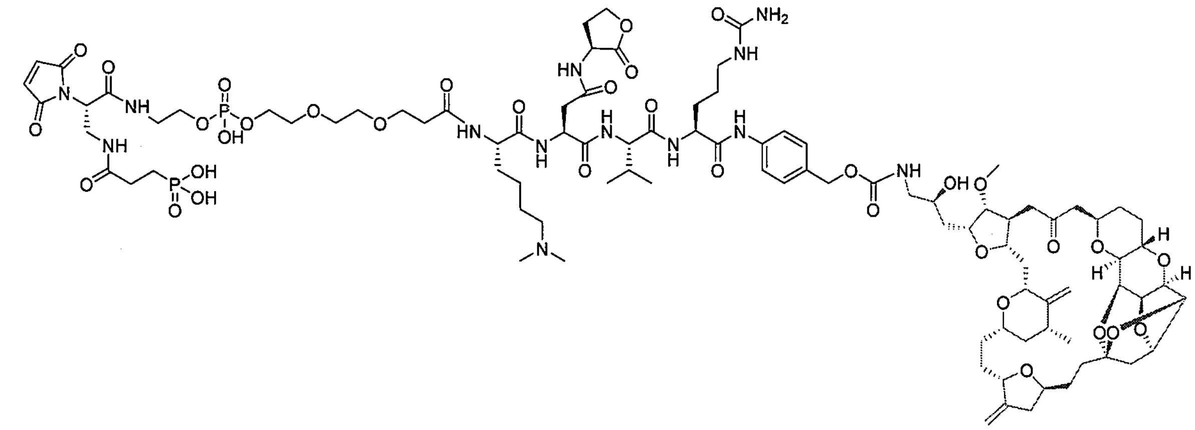

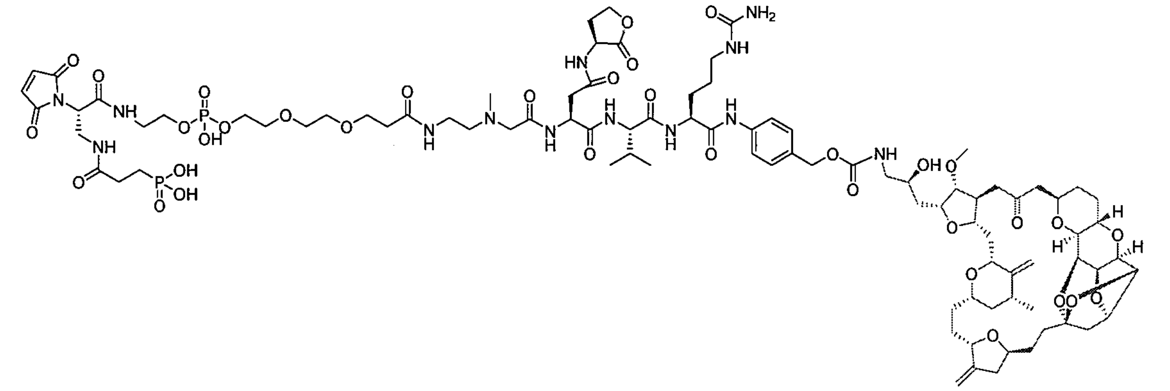

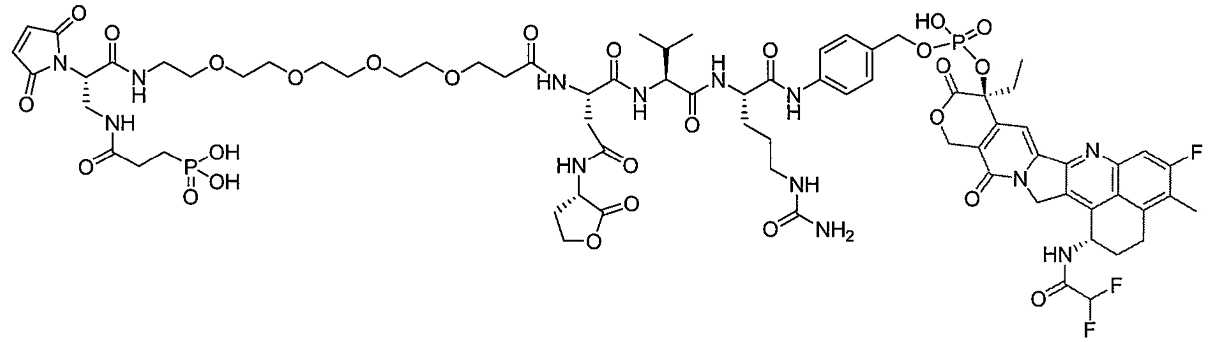

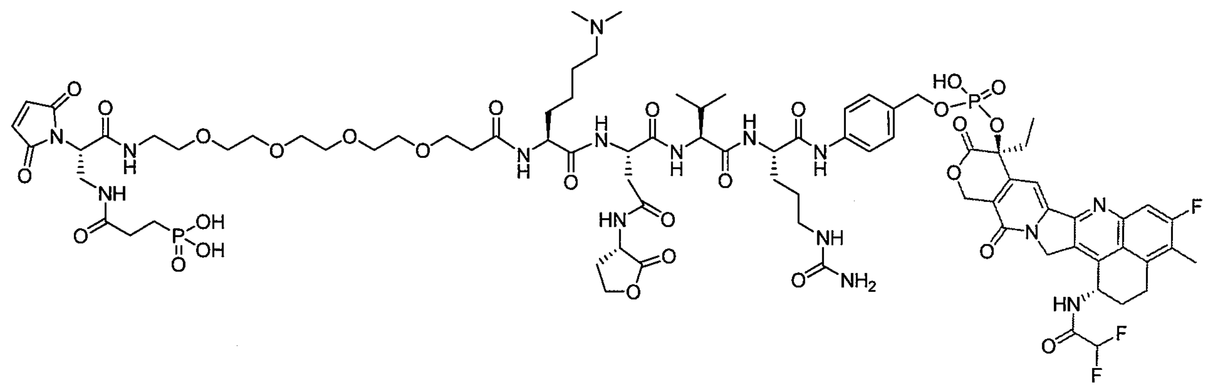

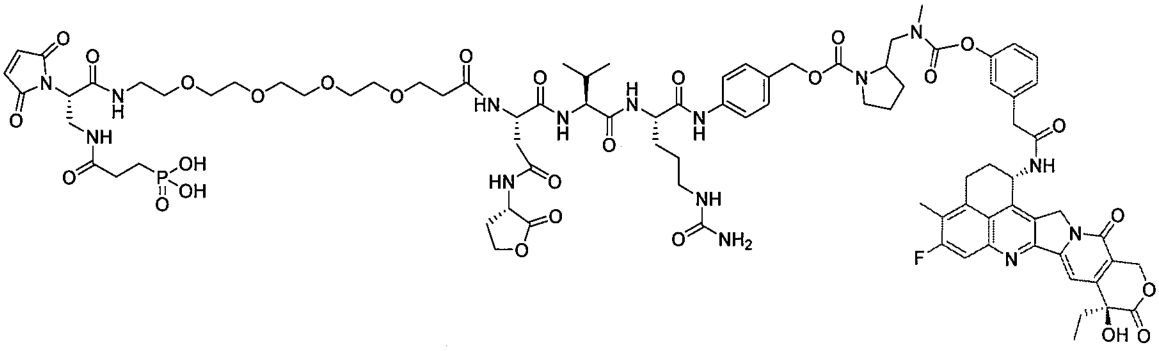

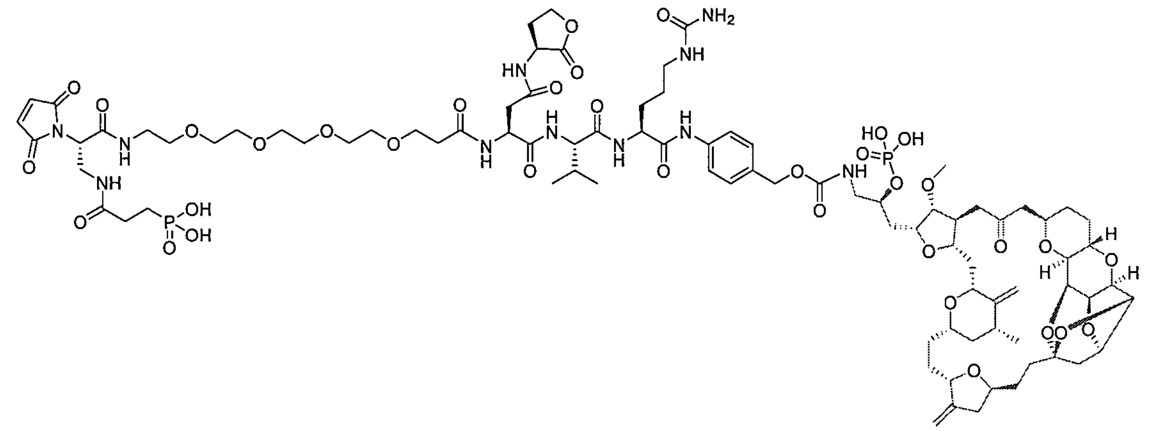

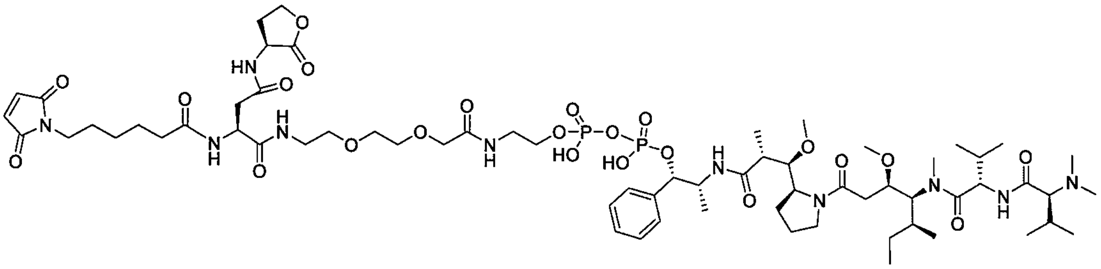

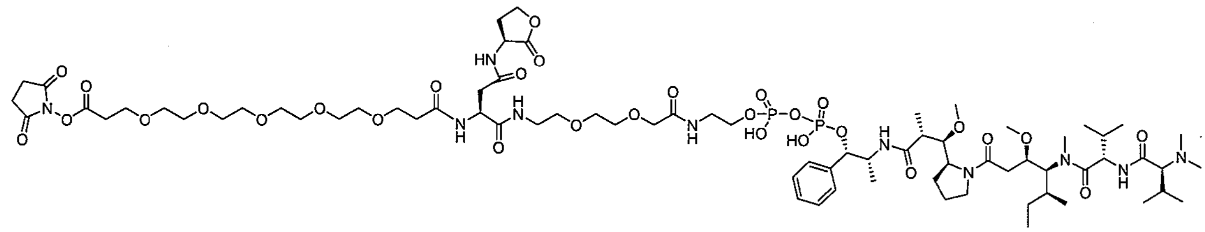

and

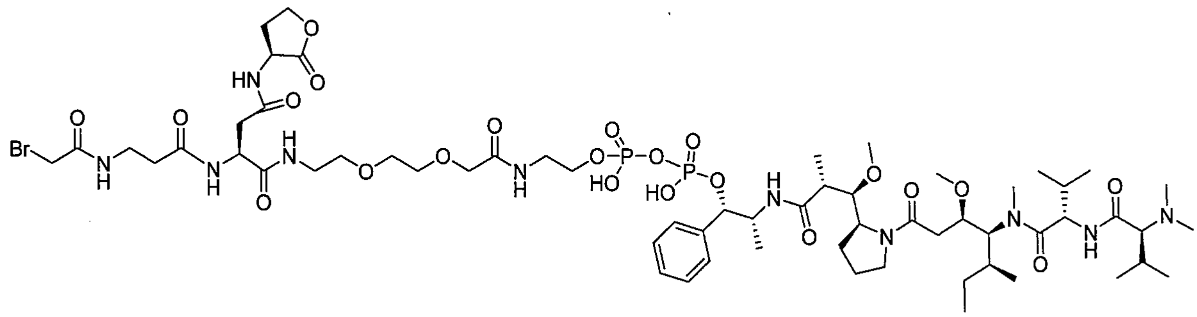

or a salt thereof.

**14.** A synthetic intermediate (II) of the conjugate precursor (I) represented by the following general formula (II) :

$$Z^1\text{-}L^1\text{-}D^1 \qquad (II)$$

[wherein:

$Z^1$ is the reactive group defined in claim 1,
or
a jointing group to joint said reactive group or the antitumor drug residue defined in claim 1 to a linker, wherein said jointing group is optionally in a protected form protected by a protecting group;
$D^1$ is an antitumor drug residue, or a jointing group or a protected form thereof;
when $Z^1$ is the reactive group, then $D^1$ is the jointing group;
$L^1$ is a linker which links $Z^1$ to $D^1$;
when $D^1$ is the antitumor drug residue, then at least any one of $D^1$ and $L^1$ has one or more lactonyl group(s) at any position(s) as substituent(s) or protecting group(s); and
when $D^1$ is the jointing group, then
$L^1$ has one or more group(s) selected from a lactonyl group; a phosphoryl group ($-P(=O)(OH)_2$); a phosphorylalkylcarbonyl group ($-C(=O)$-alkylene-$P(=O)(OH)_2$); a bisphosphorylmethyl group (-C(H, OH, $NH_2$, Cl, or alkyl) $(P(=O)(OH)_2)_2$); an amino group; a monoalkylamino group; a dialkylamino group; a cyclic amino group formed by combining two alkyl groups of said dialkylamino group with the nitrogen atom to which they are attached; and a trialkylammonium group, at any position(s) as substituent(s) or protecting group(s), wherein when the total number of said group(s) is two or more, they are identical to or different from each other]

or a salt thereof.

**15.** The synthetic intermediate (II) according to claim 14 or a salt thereof, wherein the jointing group is a hydroxy group, a nitro group, a cyano group, an amide group, an oxo group, an azide group, an amino group, a monoalkylamino group optionally having substituent(s), an imino group (-N=, also including an imino group in a cyclic amine), a carboxy group, a phosphoryl group, an alkynyl group, a cycloalkynyl group, an azacycloalkynyl group, or a sulfhydryl group, or a protected form thereof.

**16.** The synthetic intermediate (II) according to claim 14 or 15 or a salt thereof, wherein the jointing group is a hydroxy group, a nitro group, a cyano group, an oxo group, an azide group, an amino group, a monoalkylamino group optionally having substituent(s), an imino group (-N=, also including an imino group in a cyclic amine), a carboxy group, a phosphoryl group, an alkynyl group, a cycloalkynyl group, an azacycloalkynyl group, or a sulfhydryl group, or a protected form thereof.

**17.** The synthetic intermediate (II) according to any one of claims 14 to 16 or a salt thereof, wherein the jointing group is a hydroxy group, a nitro group, an oxo group, an azide group, an amino group, a monoalkylamino group optionally having substituent(s), an imino group (-N=, also including an imino group in a cyclic amine), a carboxy group, a phosphoryl group, an alkynyl group, a cycloalkynyl group, an azacycloalkynyl group, or a sulfhydryl group, or a protected form thereof.

**18.** The synthetic intermediate (II) according to any one of claims 14 to 17 or a salt thereof, wherein the jointing group is a hydroxy group, a nitro group, an oxo group, an azide group, an amino group, a monoalkylamino group optionally

having substituent(s), an imino group (-N=, also including an imino group in a cyclic amine), a carboxy group, a phosphoryl group, an alkynyl group, a cycloalkynyl group, or an azacycloalkynyl group, or a protected form thereof.

**19.** The synthetic intermediate (II) according to any one of claims 14 to 18 or a salt thereof, wherein

$L^1$ is an optionally substituted alkylene group; and
one or more methylene group(s) in the chain of said alkylene group is/are replaced with one or more group(s) selected from the divalent groups according to any one of claims 9 to 11.

**20.** The synthetic intermediate (II) according to any one of claims 14 to 19 or a salt thereof, wherein

$Z^1$ is a group selected from the jointing groups according to any one of claims 15 to 18; and
$D^1$ is a group selected from the antitumor drug residues according to any one of claims 5 to 8.

**21.** The synthetic intermediate (II) according to any one of claims 14 to 19 or a salt thereof, wherein
$Z^1$ and $D^1$ are each independently a group selected from the jointing groups according to any one of claims 15 to 18.

**22.** The synthetic intermediate (II) according to any one of claims 14 to 19 or a salt thereof, wherein

$Z^1$ is a group selected from the reactive groups according to any one of claims 2 to 4; and
$D^1$ is a group selected from the jointing groups according to any one of claims 15 to 18.

**23.** The intermediate (II) according to any one of claims 14 to 20 or a salt thereof, wherein

$Z^1$ is a group selected from the jointing groups according to any one of claims 15 to 18;
$D^1$ is a group selected from the antitumor drug residues according to any one of claims 5 to 8; and
at least any one of $D^1$ and $L^1$ has one or more lactonyl group(s) at any position(s) as substituent(s) or protecting group(s), and independently thereof,
at least any one of $D^1$ and $L^1$ has one or more group(s) selected from a phosphoryl group (-P(=O)(OH)$_2$); a phosphorylalkylcarbonyl group (-C(=O)-alkylene-P(=O)(OH)$_2$); a bisphosphorylmethyl group (-C(H, OH, NH$_2$, Cl, or alkyl)(P(=O)(OH)$_2$)$_2$); an amino group; a monoalkylamino group; a dialkylamino group; a cyclic amino group formed by combining two alkyl groups of said dialkylamino group with the nitrogen atom to which they are attached; and a trialkylammonium group, at any position(s) as substituent(s) or protecting group(s),
wherein when the total number of said group(s) is two or more, they are identical to or different from each other.

**24.** The synthetic intermediate (II) according to any one of claims 14 to 19, 21, and 22 or a salt thereof, wherein

$Z^1$ is a group selected from the reactive groups according to any one of claims 2 to 4, or a group selected from the jointing groups according to any one of claims 15 to 18;
$D^1$ is a group selected from the jointing groups according to any one of claims 15 to 18; and
$L^1$ has one or more group(s) selected from a lactonyl group; a phosphoryl group (-P(=O)(OH)$_2$); a phosphorylalkylcarbonyl group (-C(=O)-alkylene-P(=O)(OH)$_2$); a bisphosphorylmethyl group (-C(H, OH, NH$_2$, Cl or alkyl)(P(=O)(OH)$_2$)$_2$); an amino group; a monoalkylamino group; a dialkylamino group; a cyclic amino group formed by combining said dialkyl group; and a trialkylammonium group, at any position(s) as substituent(s) or protecting group(s),
wherein when the total number of said group(s) is two or more, they are identical to or different from each other.

**25.** A synthetic intermediate (II) selected from the group consisting of:

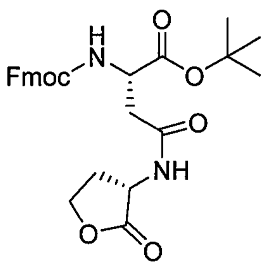

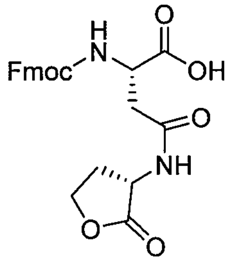

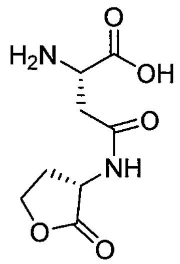

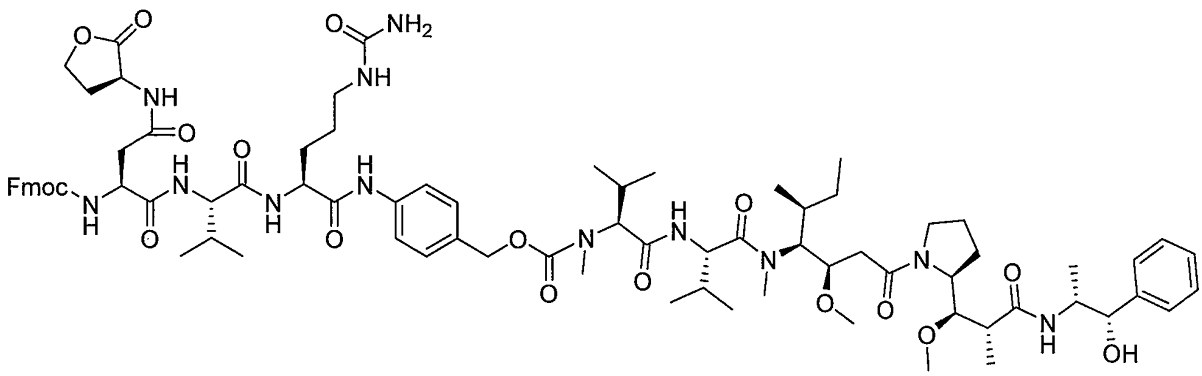

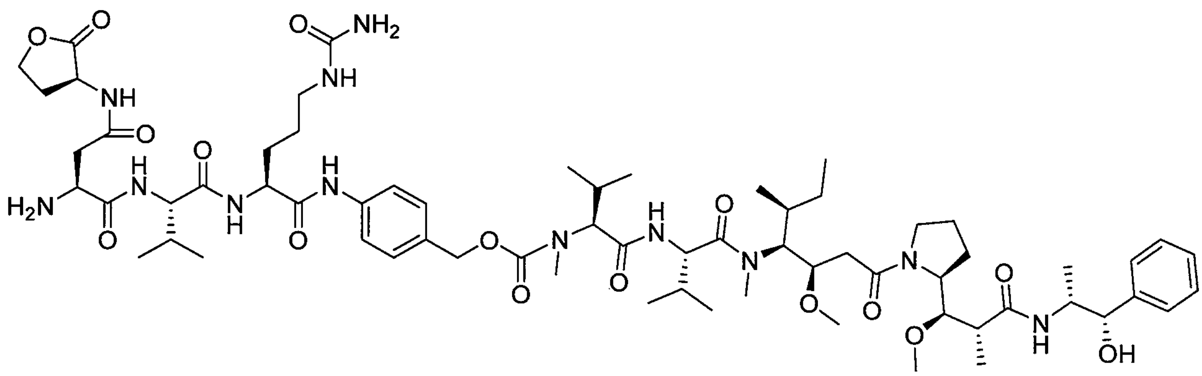

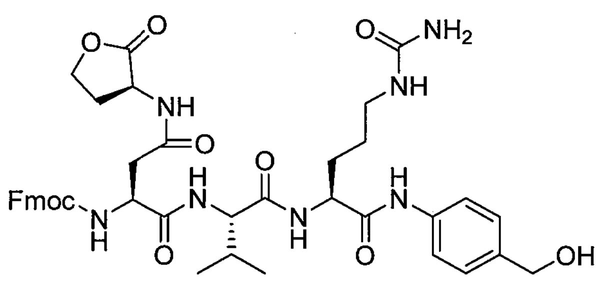

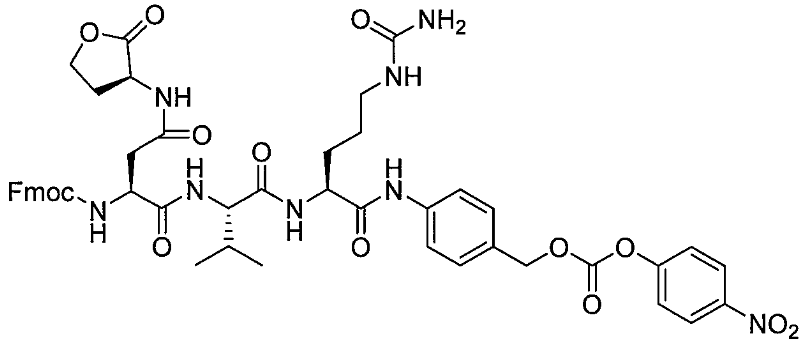

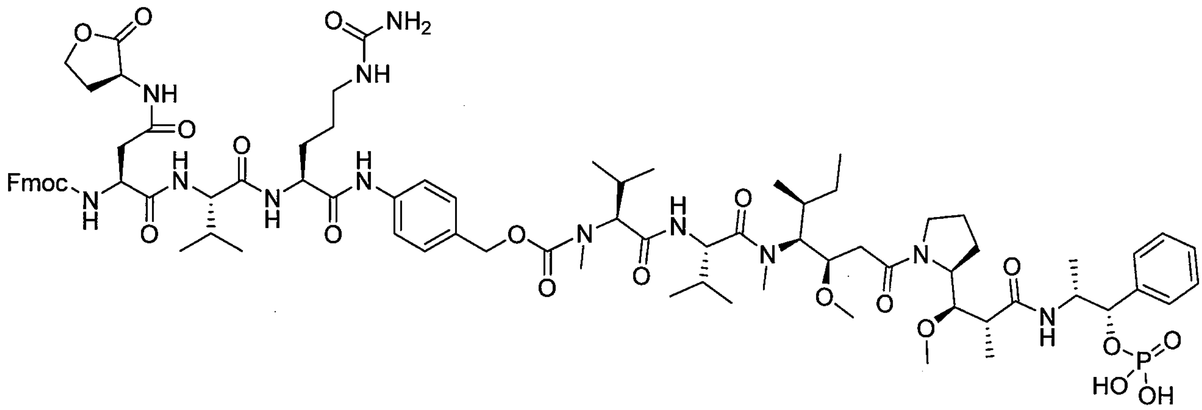

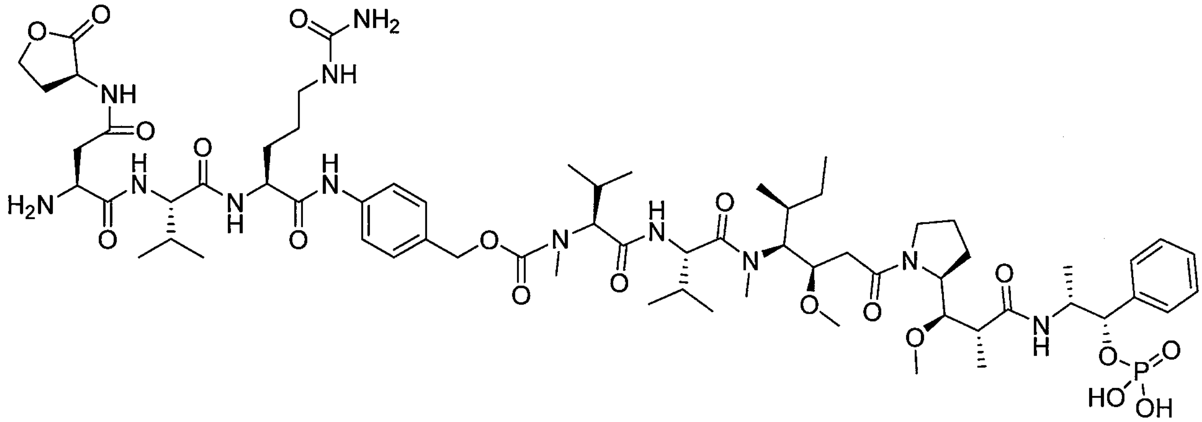

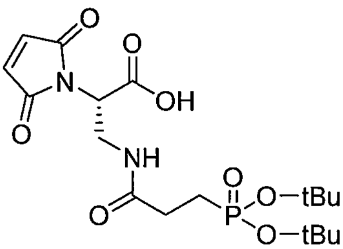

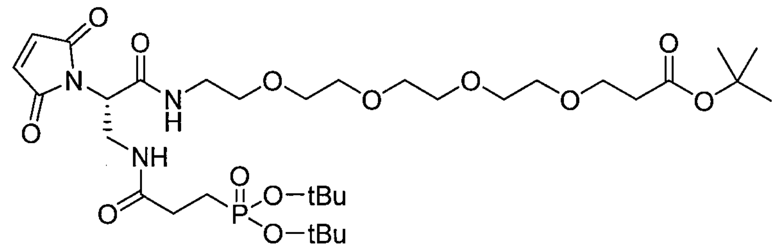

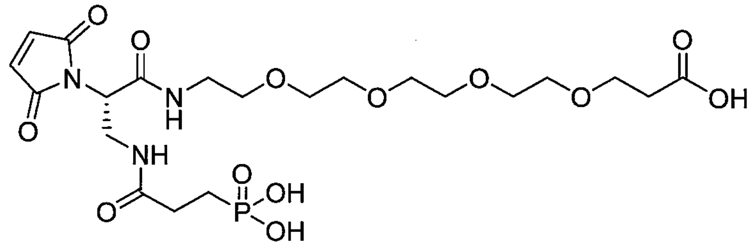

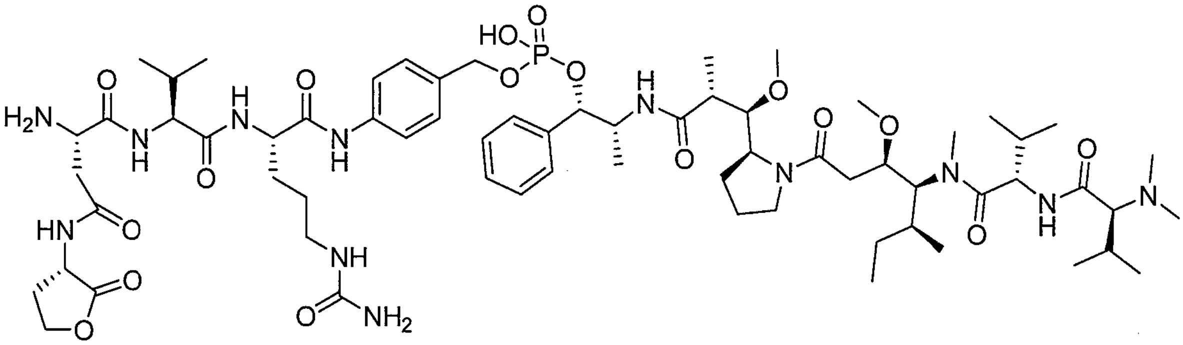

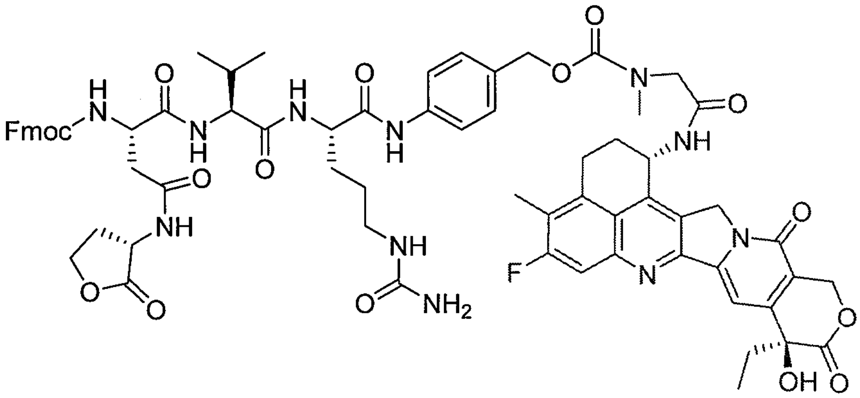

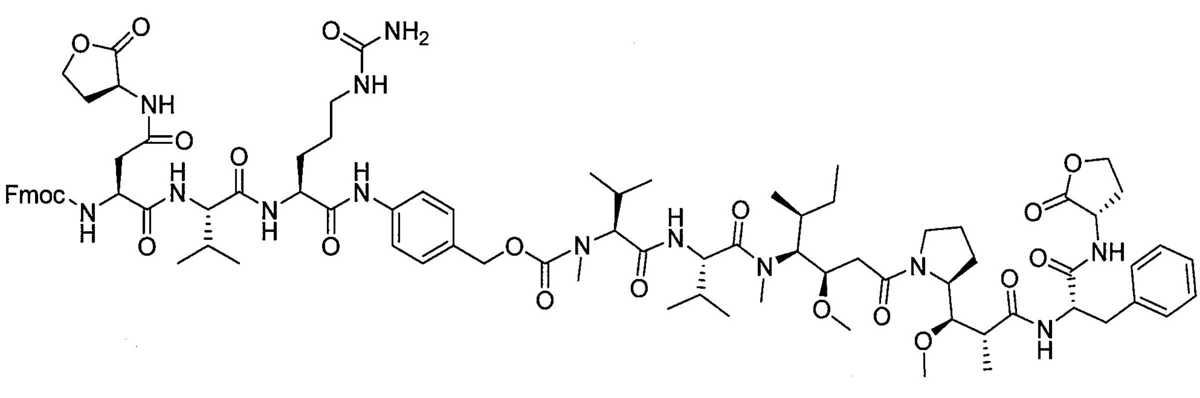

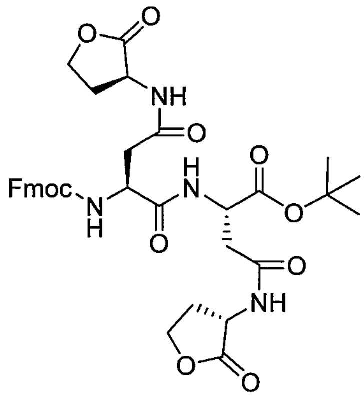

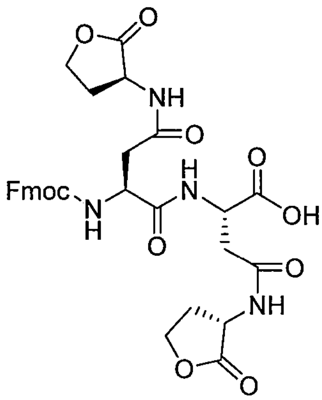

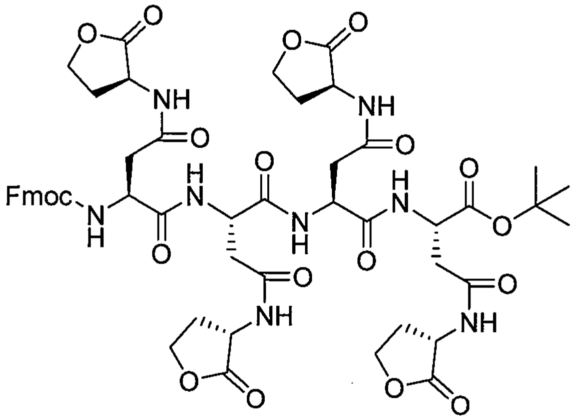

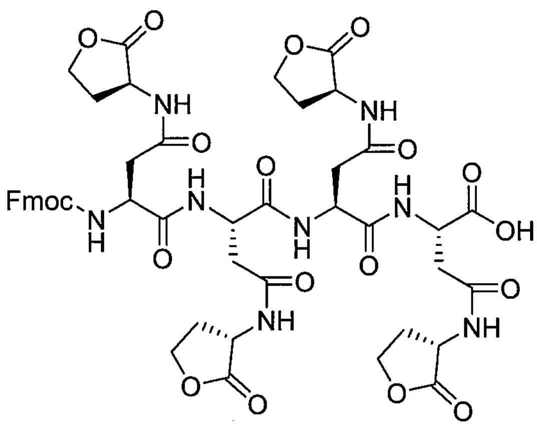

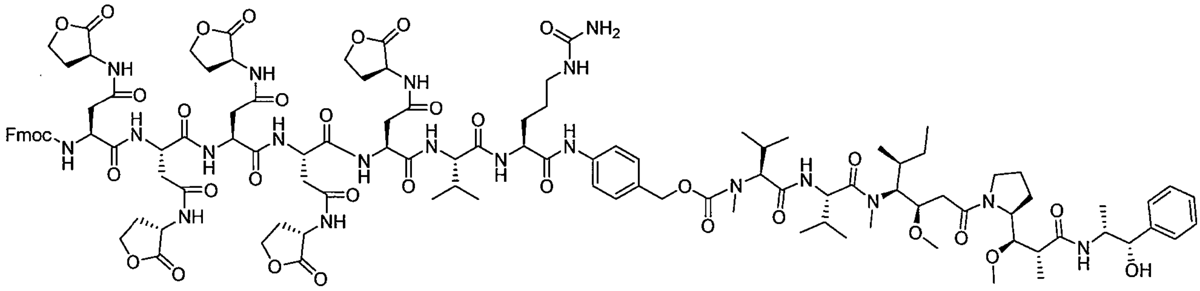

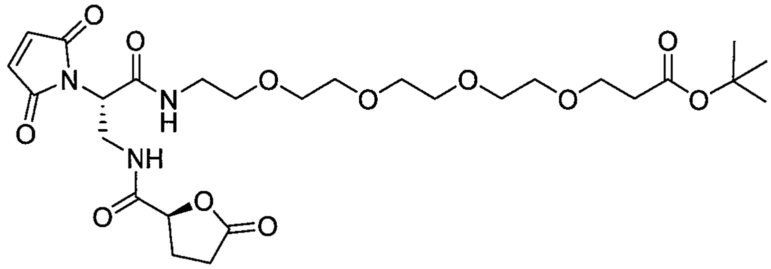

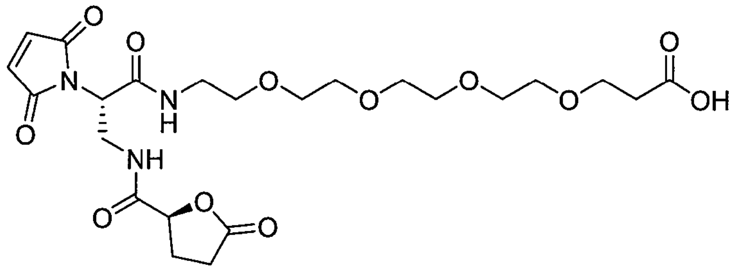

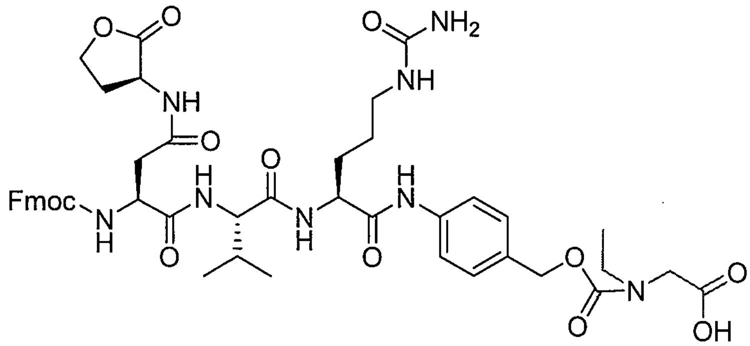

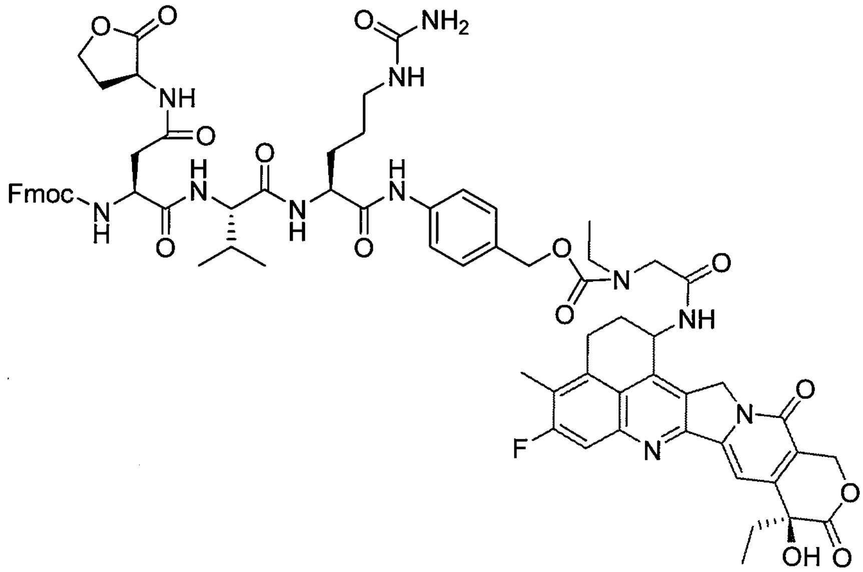

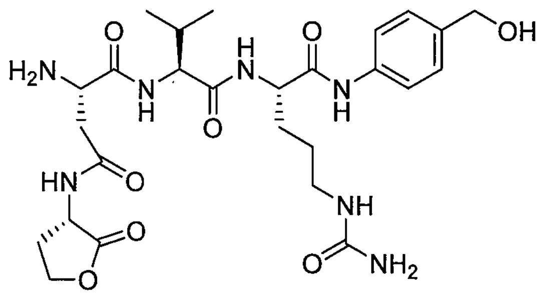

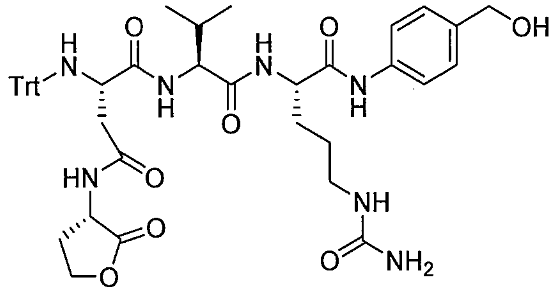

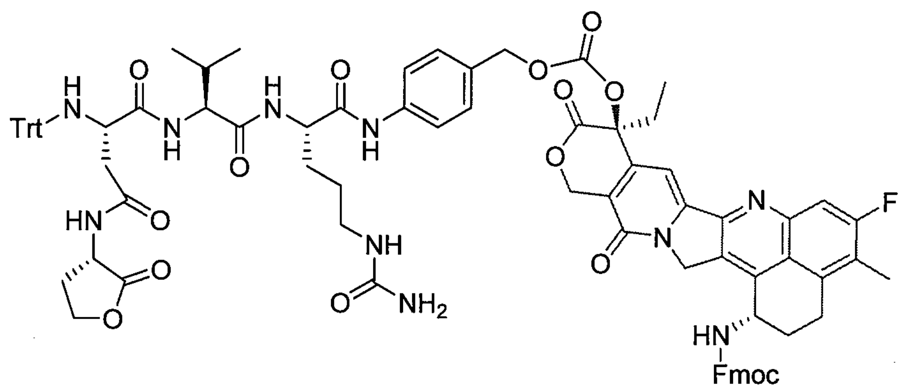

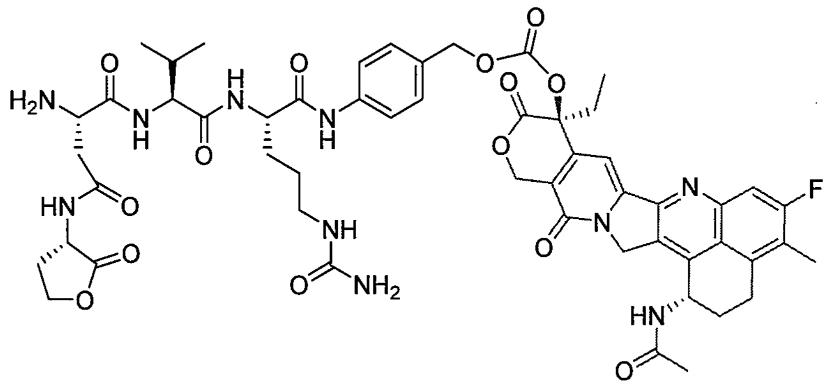

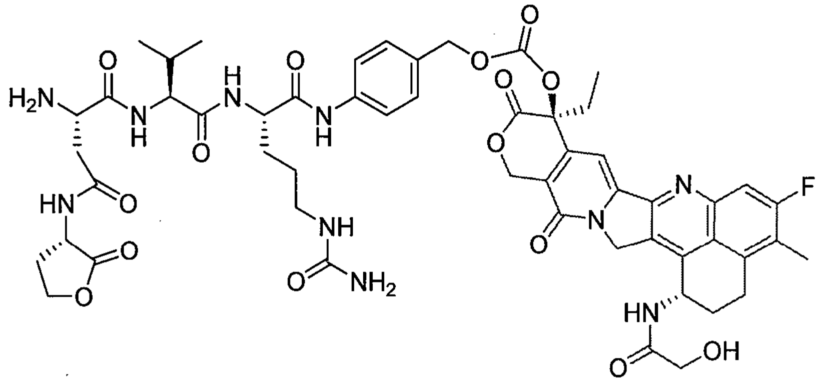

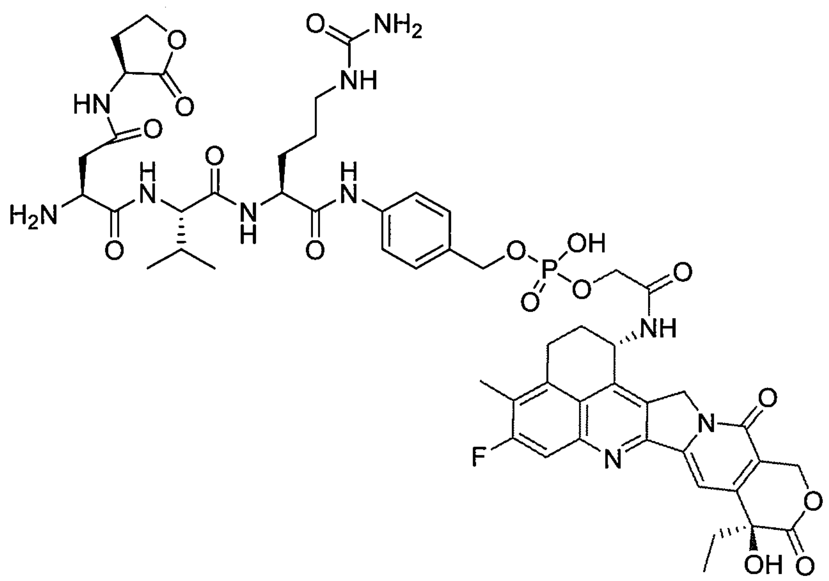

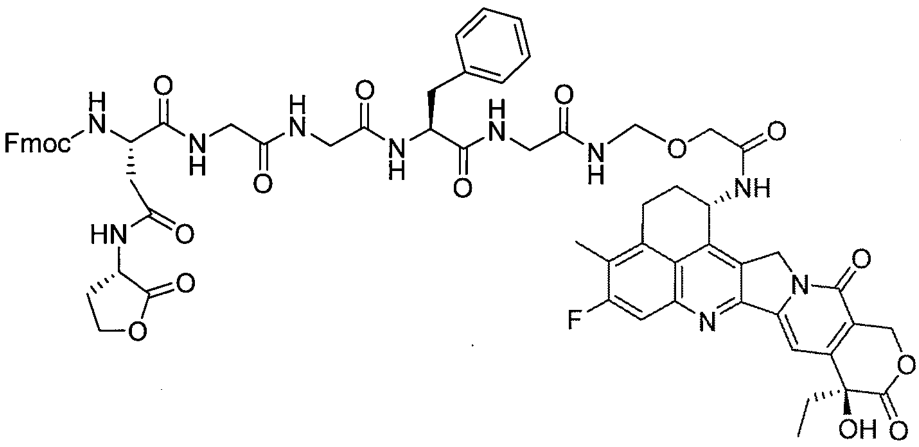

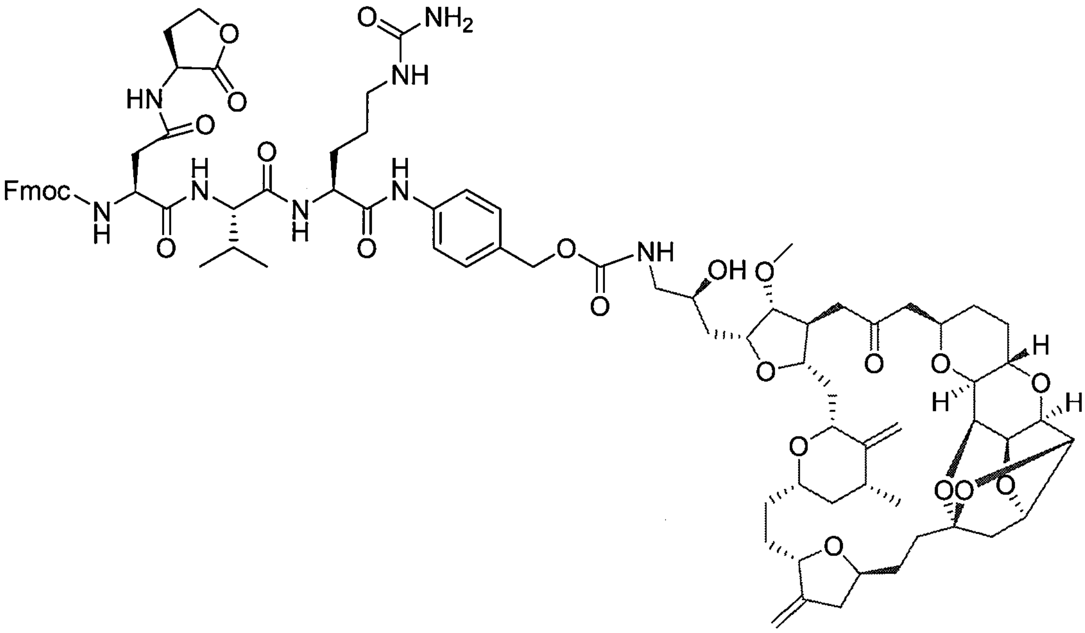

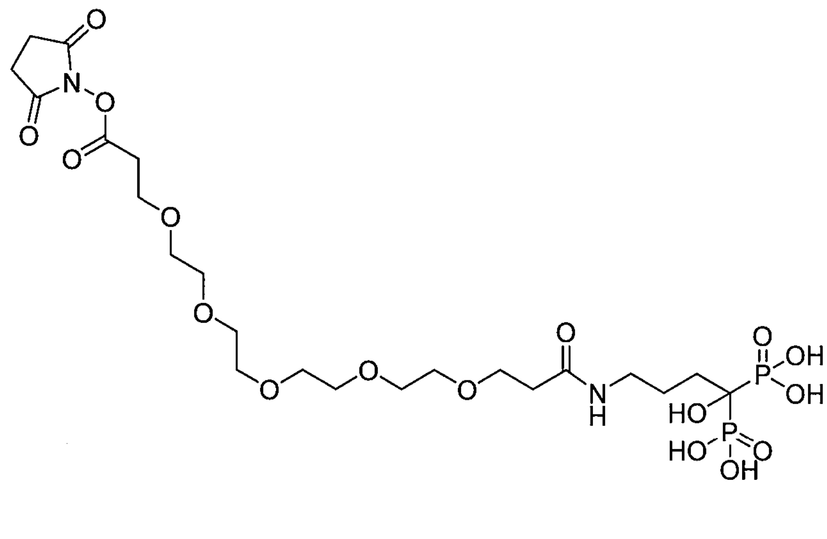

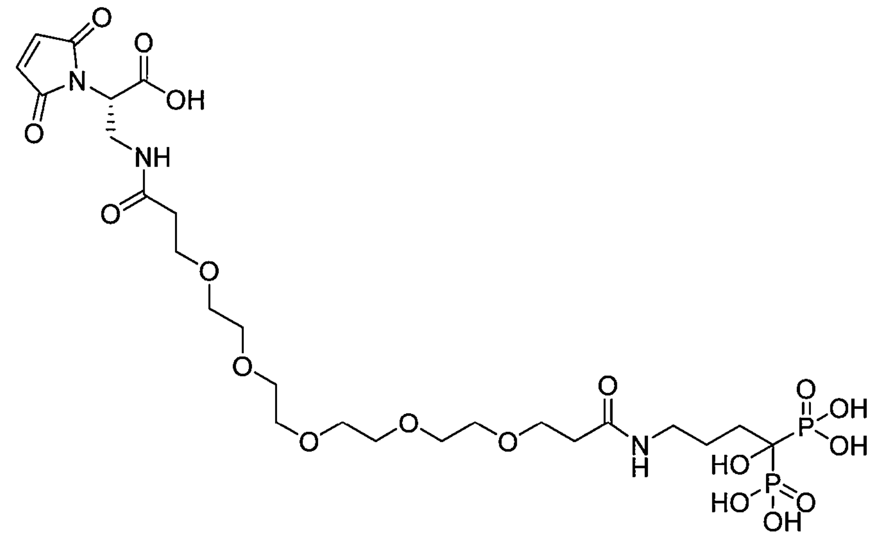

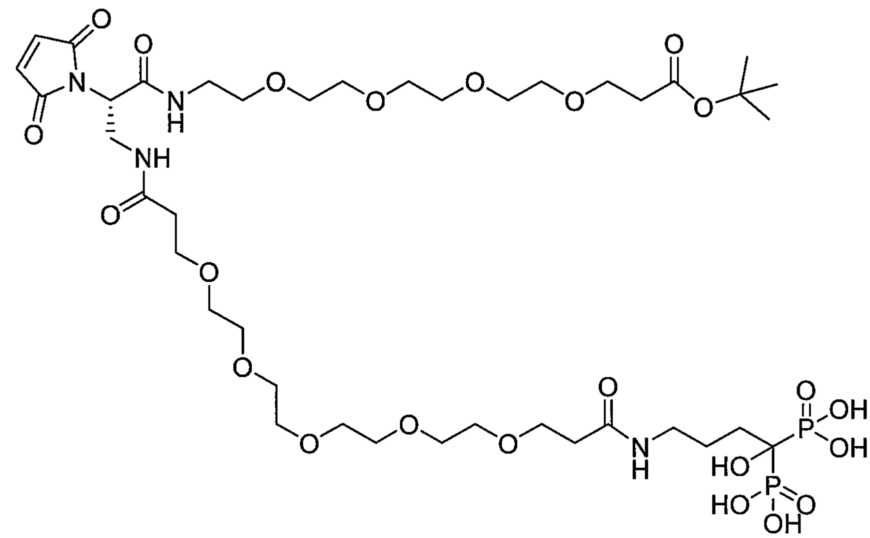

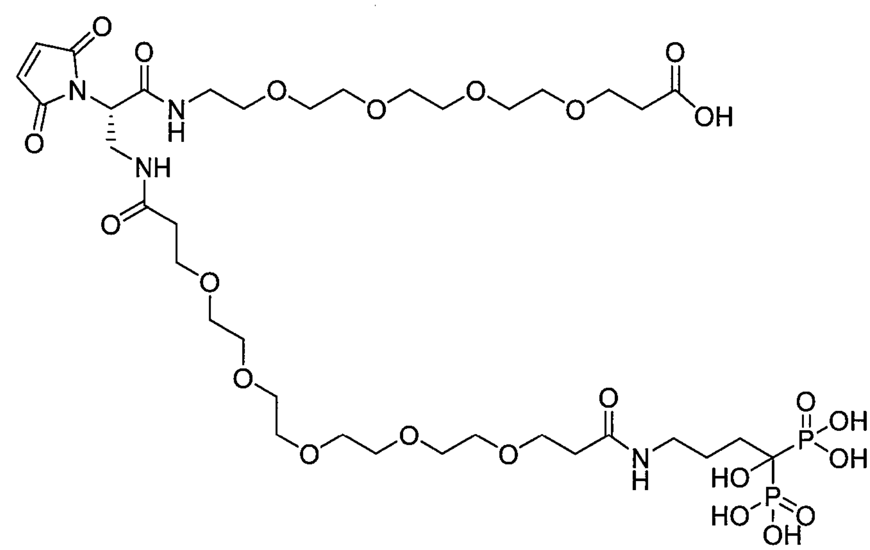

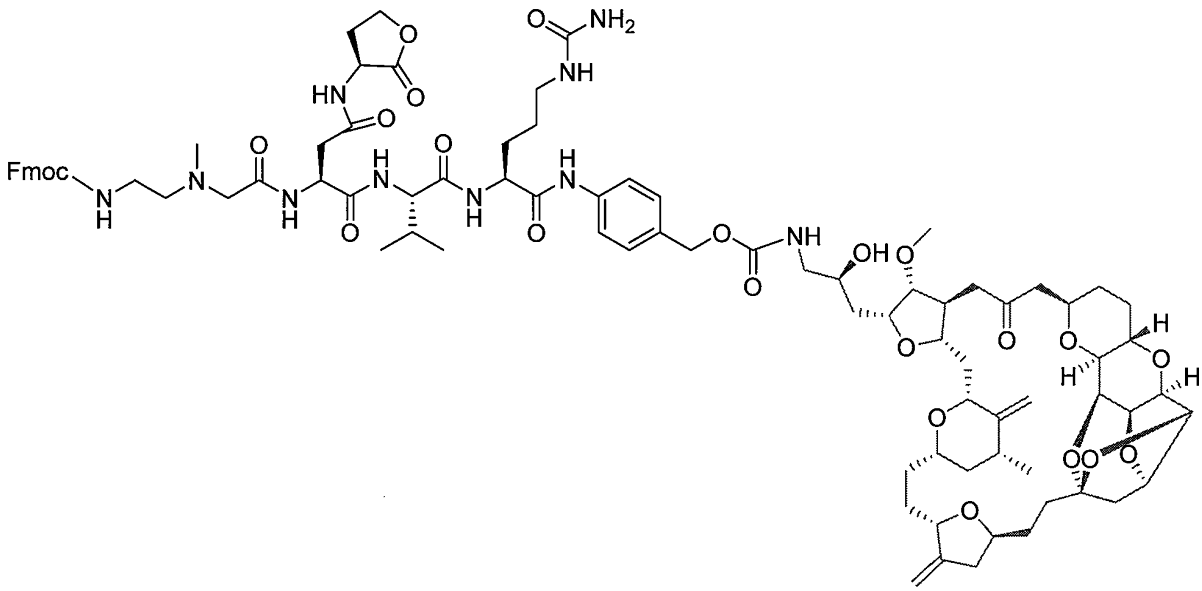

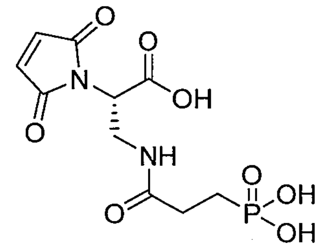

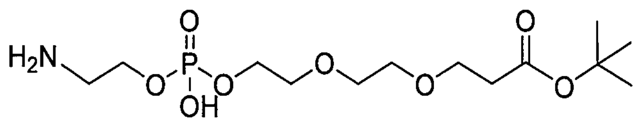

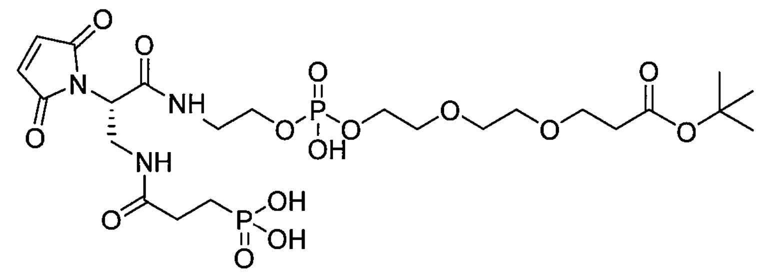

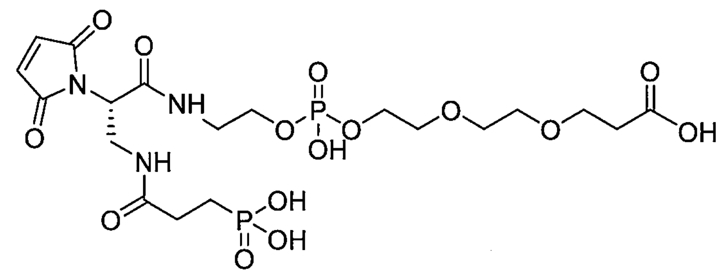

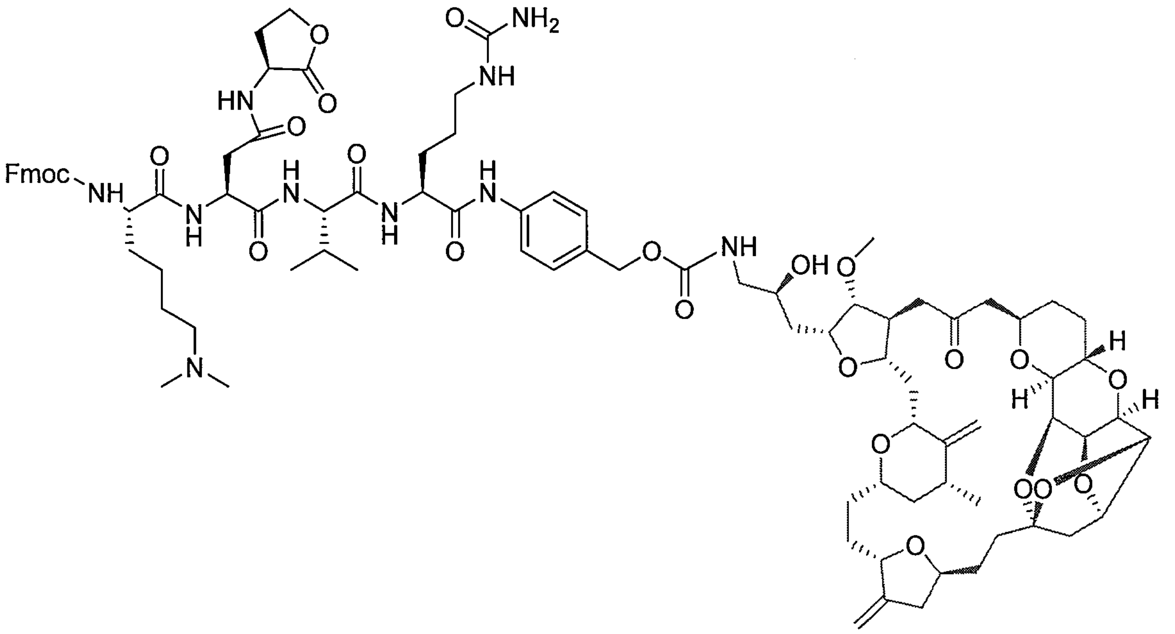

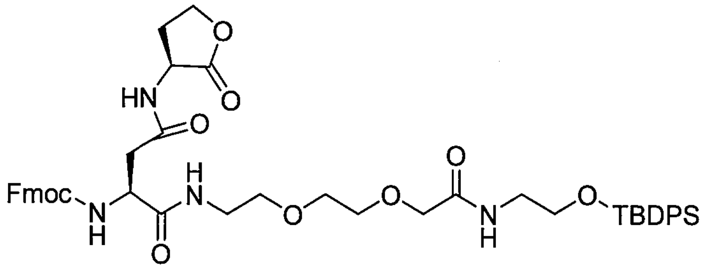

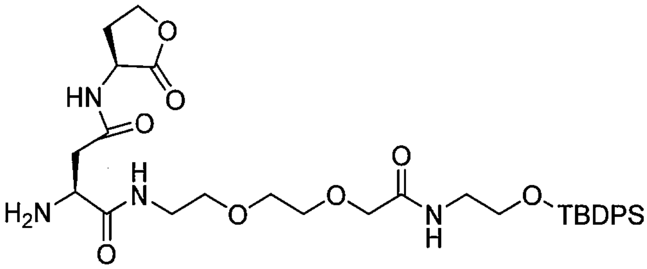

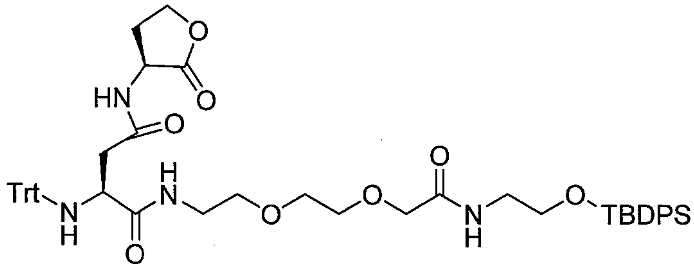

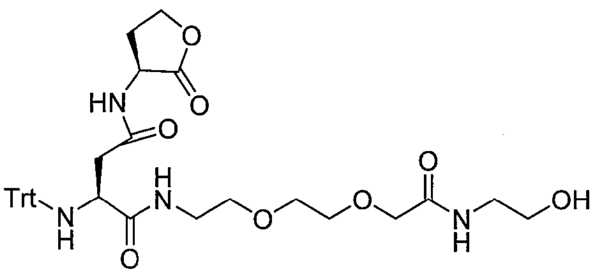

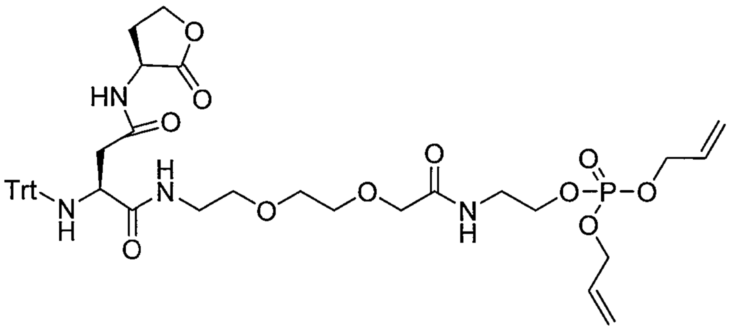

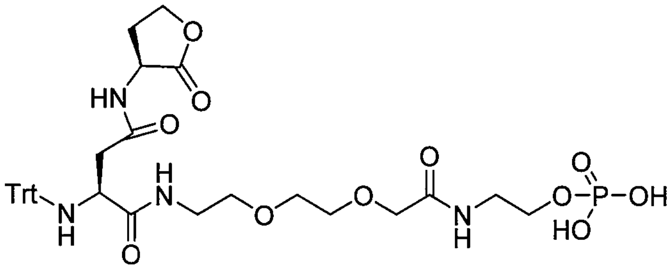

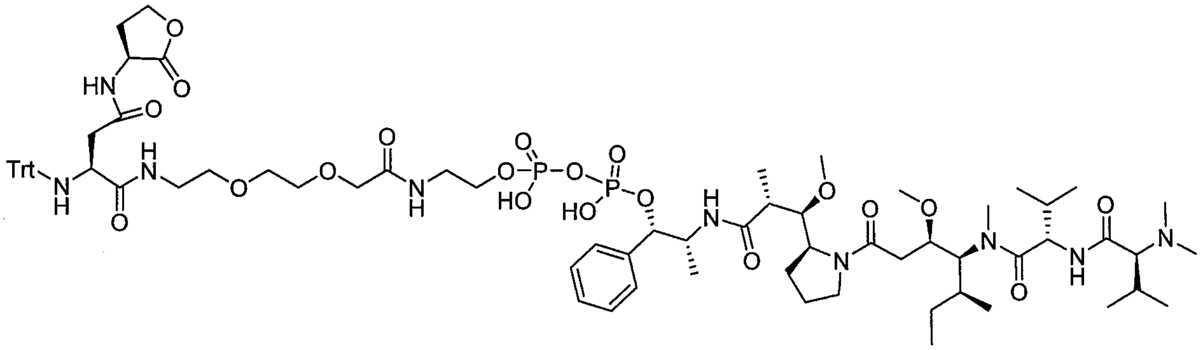

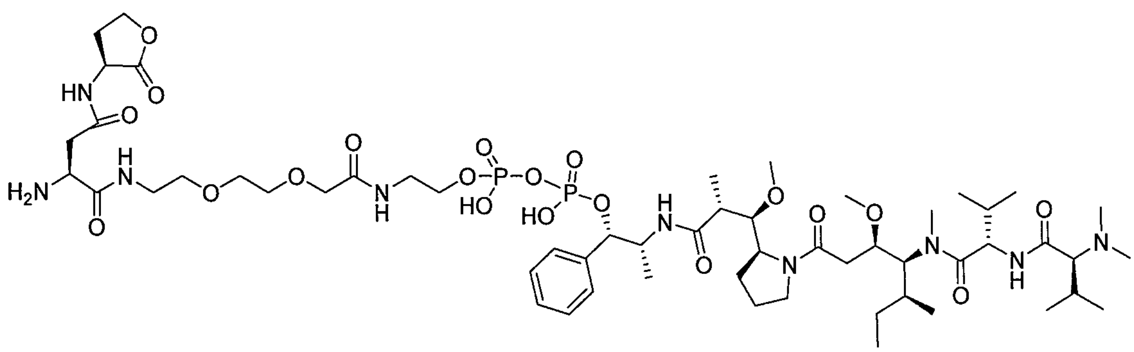

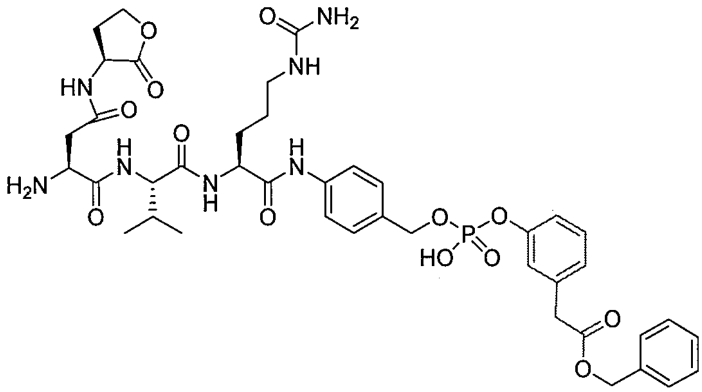

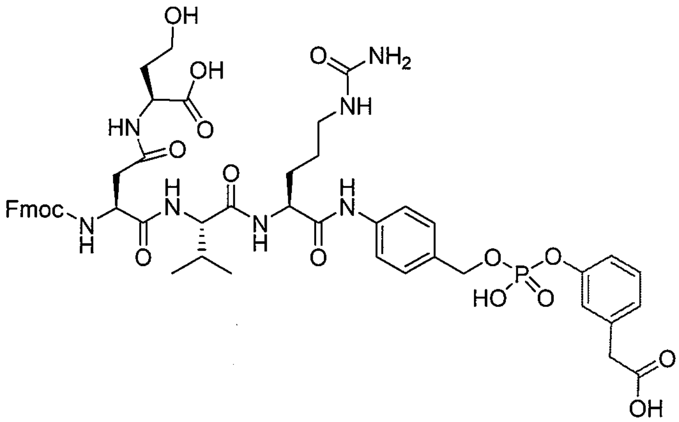

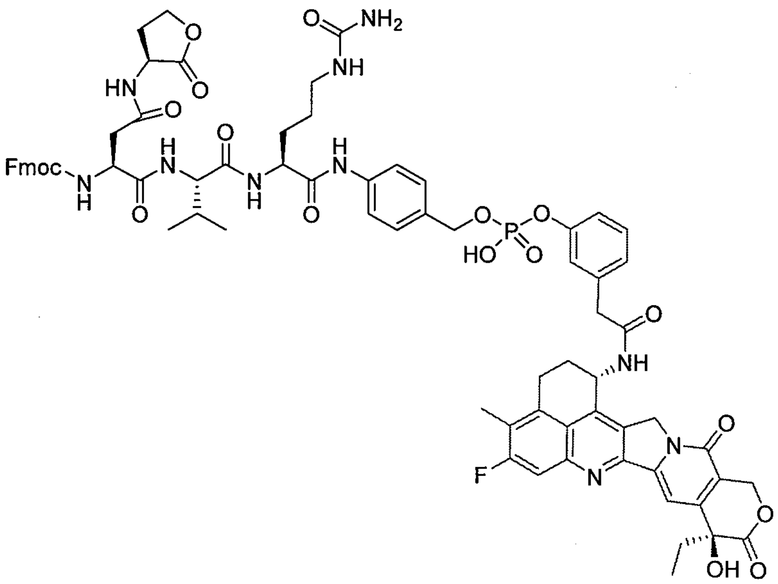

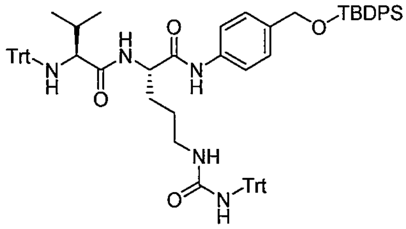

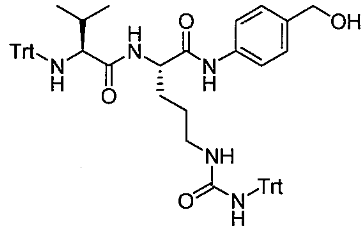

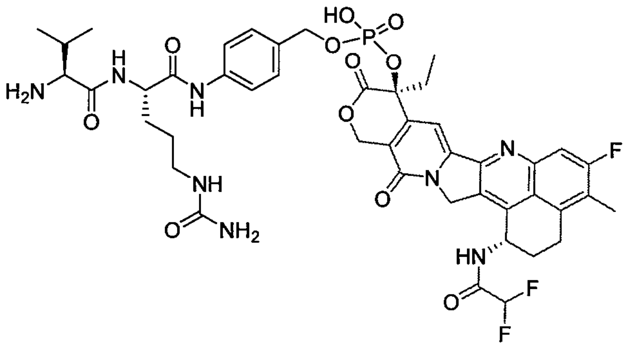

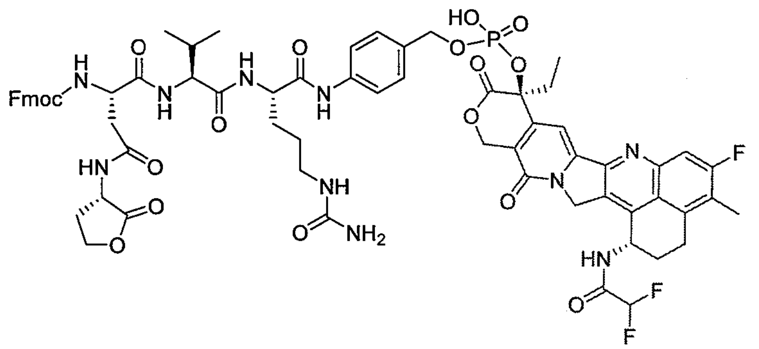

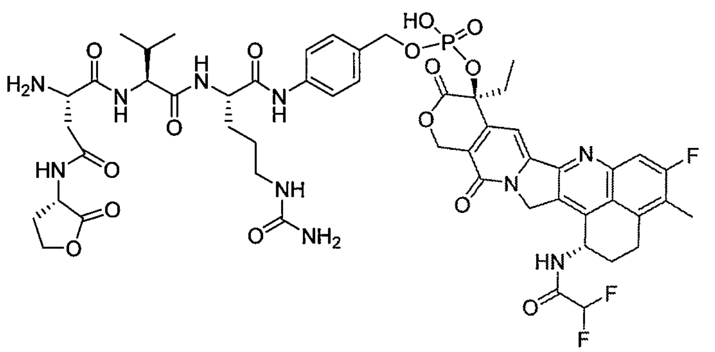

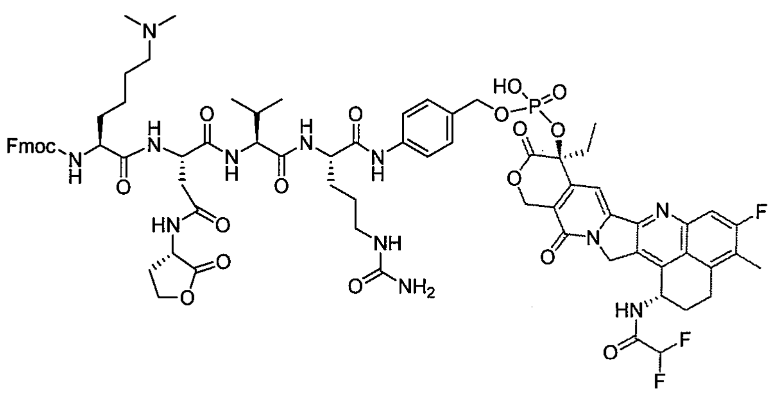

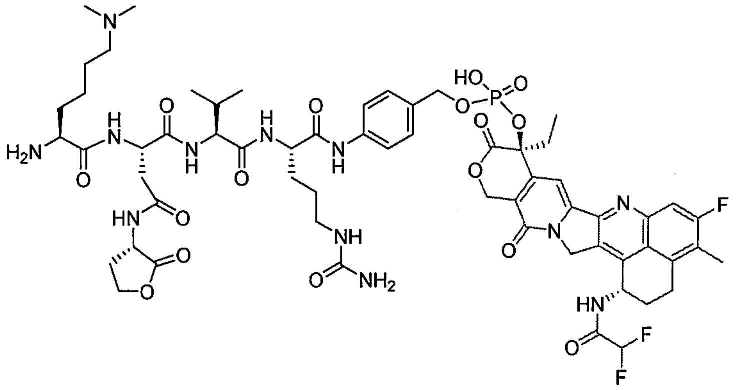

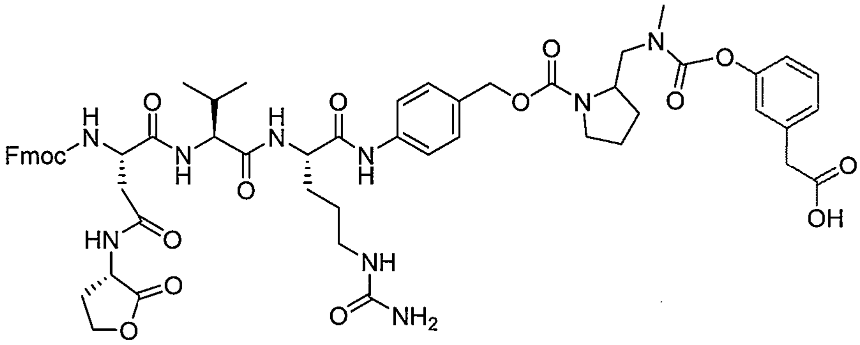

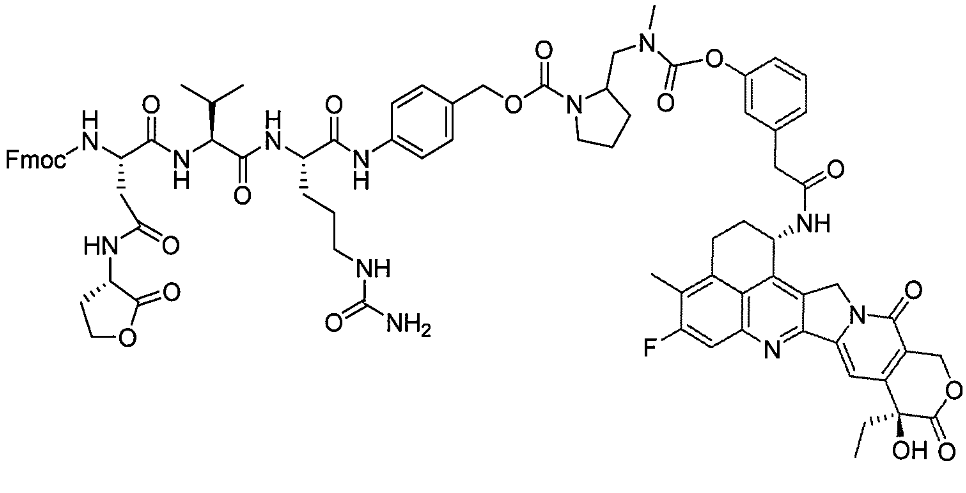

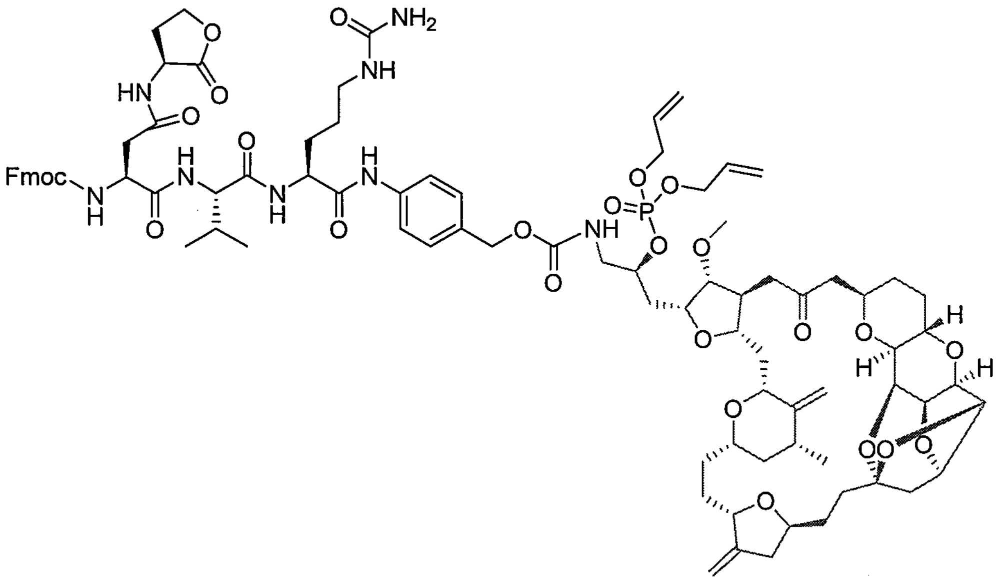

and

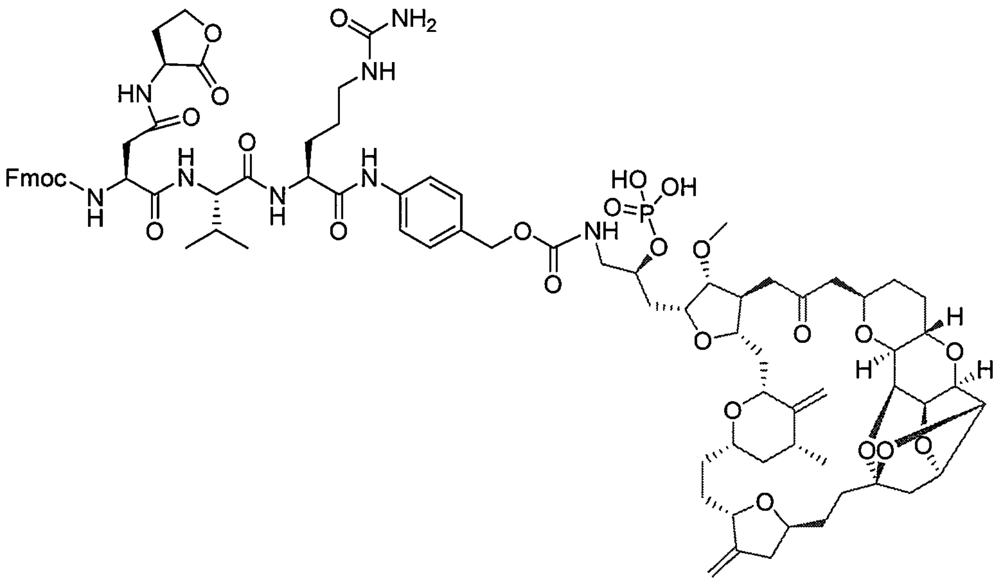

or a salt thereof.

Figure 1

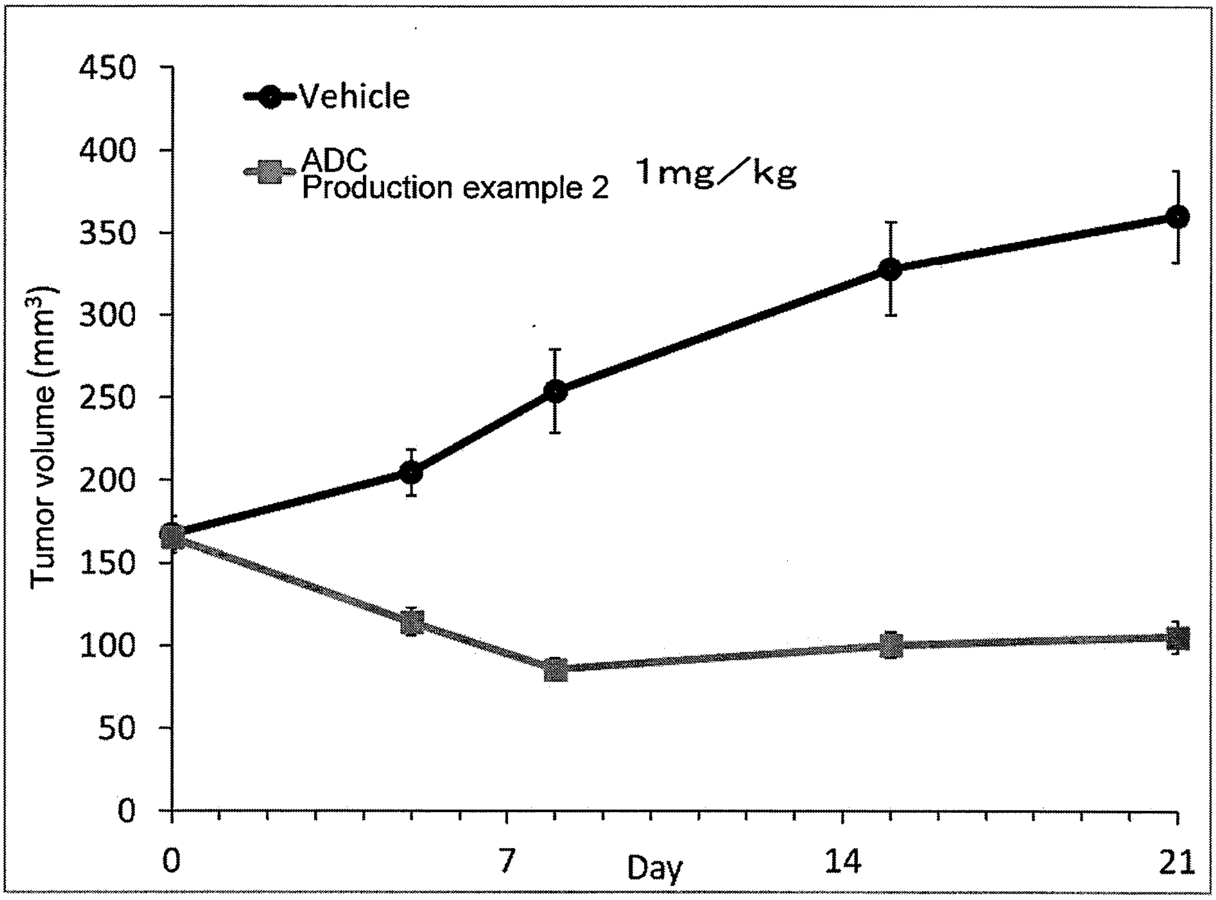
450
400
350
300
250
200
150
100
50
0
Tumor volume (mm3)
Vehicle
ADC
Production example 2
1mg／kg
0
7
Day
14
21

Figure 2

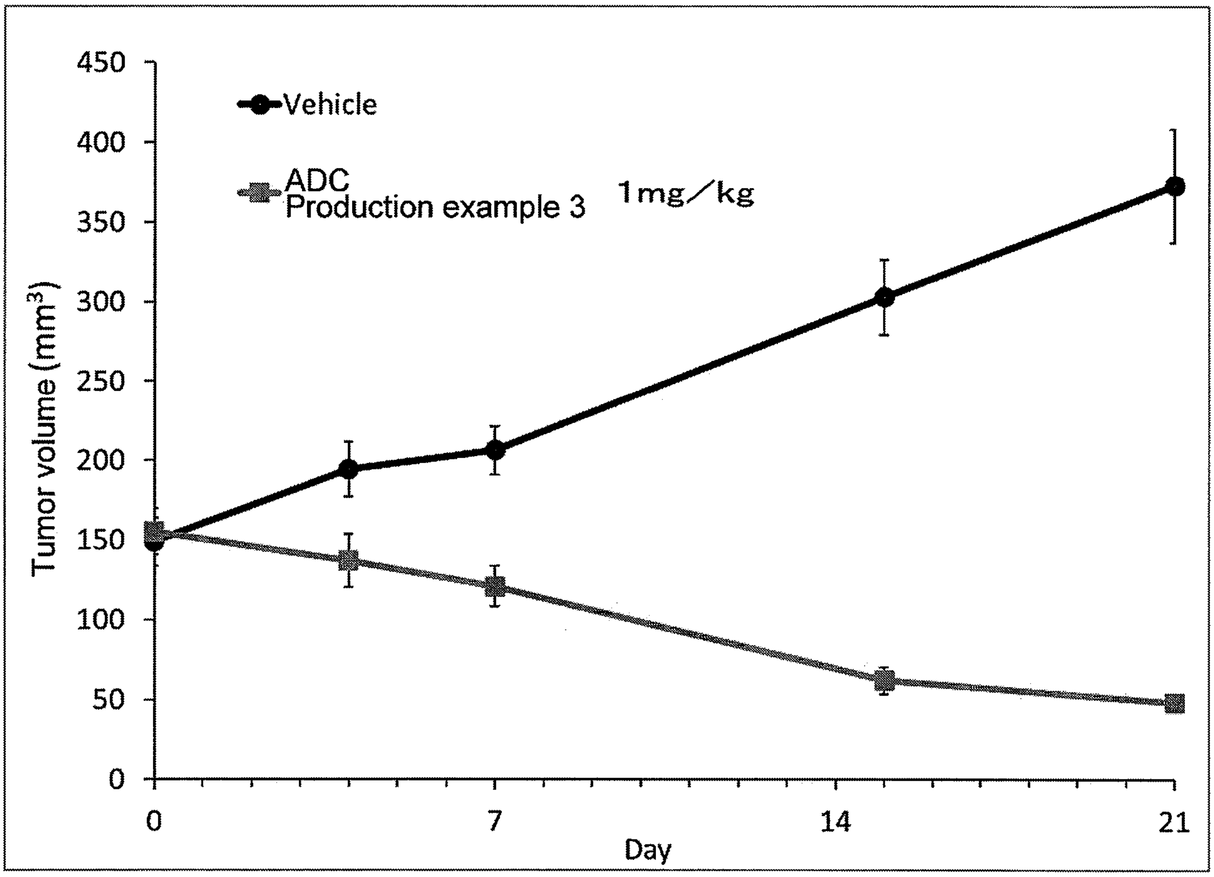
Vehicle
ADC
Production example 3 1mg/kg
Tumor volume (mm3)
450
400
350
300
250
200
150
100
50
0
0
7
14
21
Day

Figure 3

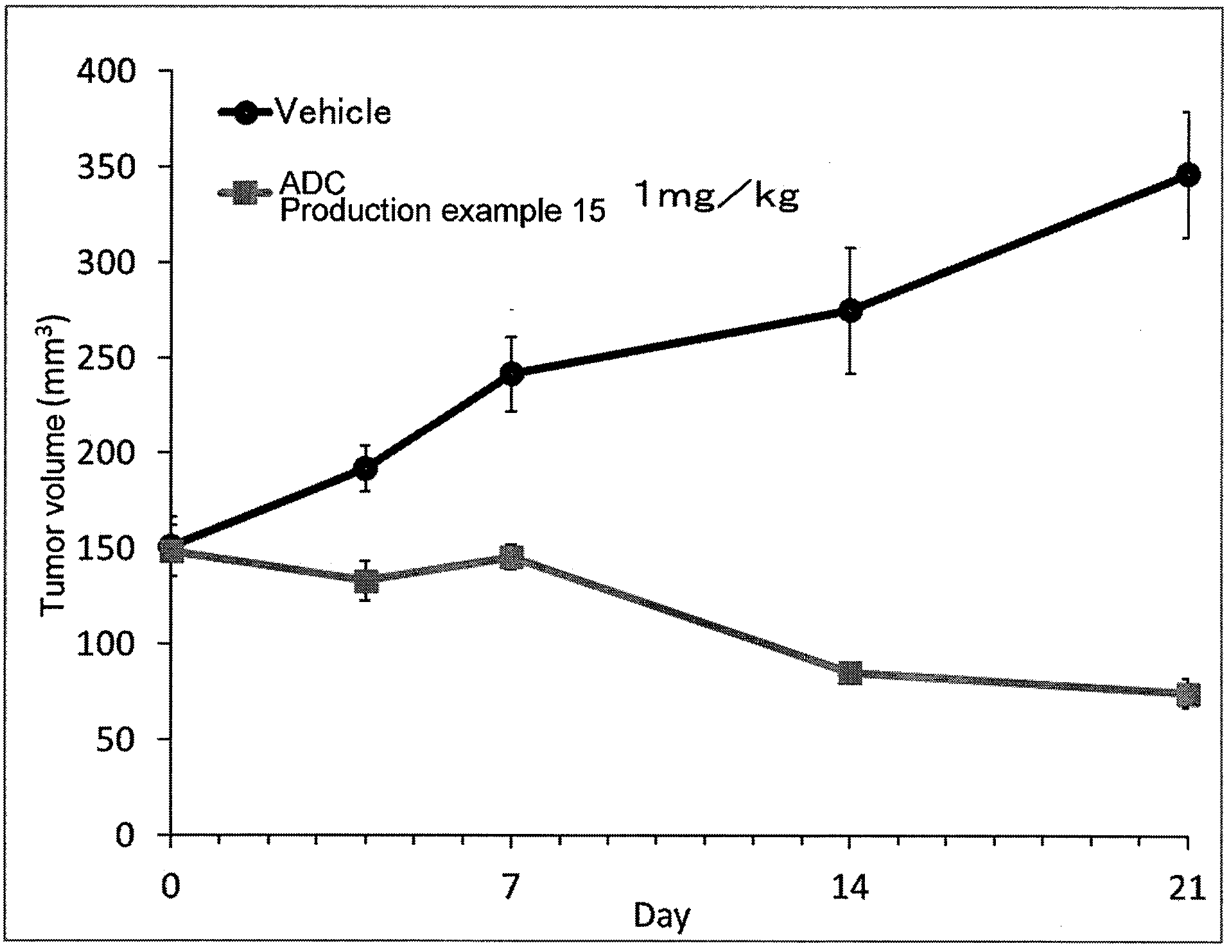
Vehicle
ADC
Production example 15
1mg/kg
Tumor volume (mm3)
400
350
300
250
200
150
100
50
0
0
7
14
21
Day

Figure 4

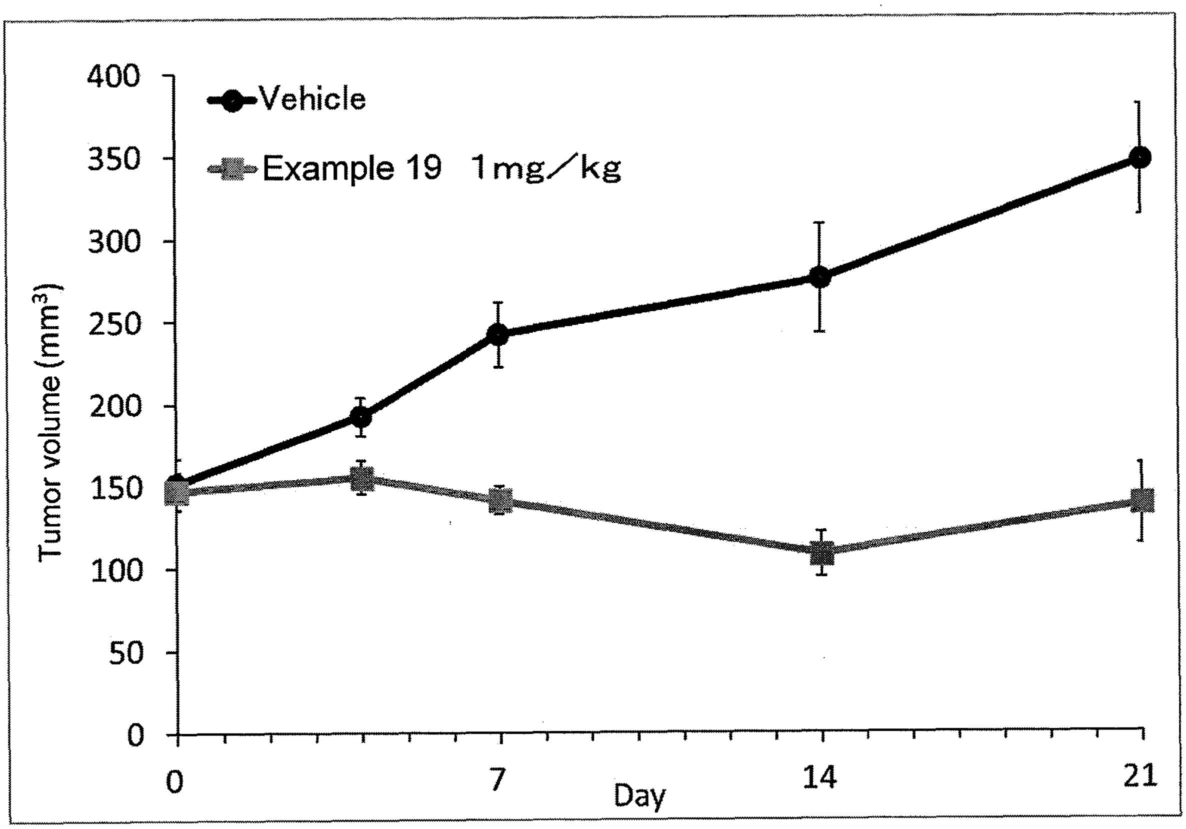
400
350
300
250
200
150
100
50
0
Tumor volume (mm$^3$)
Vehicle
Example 19 1mg/kg
0
7
14
21
Day

Figure 5

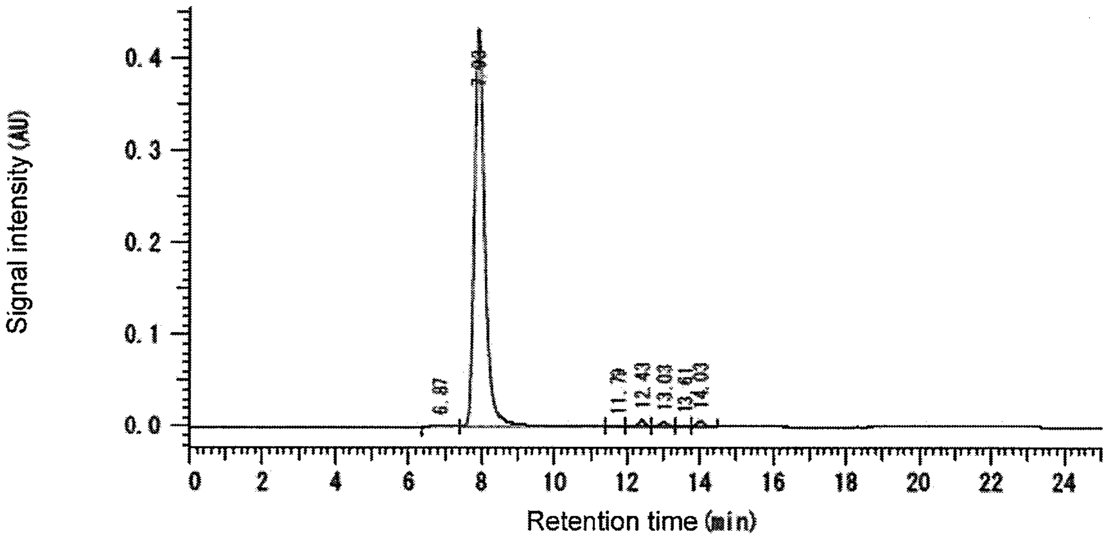


| NO | RT | Area | Concentration1 | BC |
|---|---|---|---|---|
| 1 | 6.87 | 14820 | 0.328 | BV |
| 2 | 7.93 | 4347514 | 96.182 | VV |
| 3 | 11.79 | 6234 | 0.138 | TBV |
| 4 | 12.43 | 48865 | 1.081 | TVV |
| 5 | 13.03 | 41182 | 0.911 | TVV |
| 6 | 13.61 | 14658 | 0.324 | TVV |
| 7 | 14.03 | 46807 | 1.036 | TVB |
| | | 4520080 | 100.000 | |

**INTERNATIONAL SEARCH REPORT**

International application No. **PCT/JP2023/007434**

**A. CLASSIFICATION OF SUBJECT MATTER**

***A61K 47/68***(2017.01)i; ***A61K 31/337***(2006.01)i; ***A61K 31/357***(2006.01)i; ***A61K 31/407***(2006.01)i; ***A61K 31/4375***(2006.01)i; ***A61K 31/4745***(2006.01)i; ***A61K 31/475***(2006.01)i; ***A61K 31/513***(2006.01)i; ***A61K 31/537***(2006.01)i; ***A61K 31/5513***(2006.01)i; ***A61K 31/5517***(2006.01)i; ***A61K 31/675***(2006.01)i; ***A61K 31/7034***(2006.01)i; ***A61K 31/7036***(2006.01)i; ***A61K 31/706***(2006.01)i; ***A61K 38/07***(2006.01)i; ***A61K 38/14***(2006.01)i; ***A61K 39/395***(2006.01)i; ***A61K 45/00***(2006.01)i; ***A61P 35/00***(2006.01)i; ***C07K 2/00***(2006.01)i; ***C07K 14/435***(2006.01)i; ***C07K 16/30***(2006.01)i; ***C07K 19/00***(2006.01)i

FI: A61K47/68; A61P35/00; A61K39/395 L; A61K39/395 C; A61K31/4745; A61K38/07; A61K31/537; A61K31/5517; A61K31/357; A61K39/395 T; A61K31/4375; C07K19/00; C07K14/435; A61K45/00; A61K39/395 N; A61K31/5513; A61K31/513; A61K31/7034; A61K31/7036; A61K31/337; A61K31/407; A61K38/14; A61K31/706; A61K31/475; A61K31/675; C07K2/00; C07K16/30

According to International Patent Classification (IPC) or to both national classification and IPC

**B. FIELDS SEARCHED**

Minimum documentation searched (classification system followed by classification symbols)

A61K47/68; A61K31/337; A61K31/357; A61K31/407; A61K31/4375; A61K31/4745; A61K31/475; A61K31/513; A61K31/537; A61K31/5513; A61K31/5517; A61K31/675; A61K31/7034; A61K31/7036; A61K31/706; A61K38/07; A61K38/14; A61K39/395; A61K45/00; A61P35/00; C07K2/00; C07K14/435; C07K16/30; C07K19/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| X | XIAO, Dian et al. A bifunctional molecule-based strategy for the development of theranostic antibody-drug conjugate. Theranostics. 2021, vol. 11, Issue 6, pp. 2550-2563<br>abstract, fig. 1-2, p. 2552, right column, lines 15-32 | 1-10, 12, 14-24 |
| X | SU, Zheng et al. Antibody-drug conjugates: Recent advances in linker chemistry. Acta Pharmaceutica Sinica B. 2021, vol. 11(12), pp. 3889-3907<br>fig. 7 | 1-10, 12, 14-24 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **17 May 2023** | **30 May 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No. **PCT/JP2023/007434**

**C. DOCUMENTS CONSIDERED TO BE RELEVANT**

| Category* | Citation of document, with indication, where appropriate, of the relevant passages | Relevant to claim No. |
|---|---|---|
| X<br>Y | LIU, Lianqi et al. Synthesis and evaluation of highly releasable and structurally stable antibody-SN-38-conjugates. DRUG DELIVERY. 2021, vol. 28(1), pp. 2603-2617<br>abstract, fig. 1, scheme 1, p. 2605, left column, lines 1-3 | 1-10, 14-22<br>1-10, 12, 14-24 |
| X<br>Y | LAU, Uland Y. et al. Lactone Stabilization is Not a Necessary Feature for Antibody Conjugates of Camptothecins. Mol. Pharmaceutics. 2018, vol. 15, pp. 4063-4072<br>abstract, fig. 1, schemes 1, 2, p. 4065, right column, lines 1-29, p. 4069, lines 11-15 | 1-10, 14-22<br>1-10, 12, 14-24 |
| X<br>Y | WO 2014/061277 A1 (DAIICHI SANKYO COMPANY, LIMITED) 24 April 2014 (2014-04-24)<br>claims, examples | 1-10, 12, 14-24<br>1-10, 12, 14-24 |
| X | MONDAL, Deboprosad et al. Improved Methodology for the Synthesis of a Cathepsin B Cleavable Dipeptide Linker, Widely Used in Antibody-Drug Conjugate Research. Tetrahedron Letters. 2018, vol. 59, pp. 3594-3599<br>abstract, compounds 12a, 12b, 13, scheme 2 | 14-19, 21-22, 24 |
| Y | ANAMI, Yasuaki et al. Glutamic acid-valine-citrulline linkers ensure stability and efficacy of antibody-drug conjugates in mice. NATURE COMMUNICATIONS. 2018, vol. 9:2512, pp. 1-9, DOI: 10.1038/s41467-018-04982-3<br>abstract, fig. 1-4, p. 2, left column, line 27 to p. 3, left column, line 9 | 1-25 |
| Y | US 2020/0115326 A1 (THE BOARD OF REGENTS OF THE UNIVERSITY OF TEXAS SYSTEM) 16 April 2020 (2020-04-16)<br>fig. 13-15, paragraphs [0114]-[0146] | 1-25 |
| Y | DI, Li et al. Development and application of high throughput plasma stability assay for drug discovery. International Journal of Pharmaceutics. 2005, vol. 297, pp. 110-119<br>p. 116, right column, lines 17-21 | 1-25 |

Form PCT/ISA/210 (second sheet) (January 2015)

**INTERNATIONAL SEARCH REPORT**
**Information on patent family members**

International application No.
**PCT/JP2023/007434**

| Patent document cited in search report | Publication date (day/month/year) | Patent family member(s) | Publication date (day/month/year) |
|---|---|---|---|
| WO 2014/061277 A1 | 24 April 2014 | US 2015/0352224 A1<br>claims, examples<br>EP 2910573 A1 | |
| US 2020/0115326 A1 | 16 April 2020 | EP 3630189 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003043583 A
- JP 2016196484 A
- WO 2016001485 A
- WO 2019195665 A
- JP 6186045 B


### Non-patent literature cited in the description


- *Current Opin. Chem. Biol.*, 2010, vol. 14, 529-537
- *Expert Opin. Biol. Ther.*, 14 April 2004, 45-1452
- *Biotechnol Lett.*, 2016, vol. 38, 1655-1664
- *Chem Pharm Bull.*, 2019, vol. 67, 173-185
- *N Engl J Med.*, 2012, vol. 367, 1783-1791
- *N Engl J Med.*, 2018, vol. 378, 331-344
- *Expert Opin. Biol. Ther.*, 08 August 2020, 71-875
- *Drugs Today*, 2021, vol. 57, 653-663
- *Drugs*, 2021, vol. 81, 2141-2147
- *Drugs*, 2021, vol. 81, 1229-1233
- *Clin Cancer Res.*, 2019, vol. 25, 5441-5448
- *Molecules*, 2020, vol. 25, 4764
- *Methods Mol Biol.*, 2020, vol. 2078, 1-22
- *Nat. Rev. Drug Discov.*, 2017, vol. 16, 315
- *Clin Cancer Res.*, 2004, vol. 10, 7063-7070
- *Mol Cancer Ther.*, 2017, vol. 16, 116-123
- *Antibodies (Basel)*, 15 July 2018
- *Nat. Biotech.*, 2015, vol. 33, 733-735
- *Separations*, 2019, vol. 6 (1), 1
- *Journal of Hematology & Oncology*, 2015, vol. 8, 130
- *Frontiers in Immunology*, 2017, vol. 8, 38
- *BioDrugs*, 2010, vol. 24 (2), 89-98
- *MAbs*, 2009, vol. 1 (6), 539-547
- *Journal of Immunology*, 1995, vol. 155 (1), 219-25
- *Immunology Today*, 1983, vol. 4, 72-79
- *Cell Death and Differentiation*, 2008, vol. 15, 751-761
- *Molecular Biology of the Cell*, 2004, vol. 15, 5268-5282
- *BioTechniques*, 2000, vol. 28, 162-165
- **P. G. M. WUTS**. Greene's Protective Groups in Organic Synthesis. JohnWiley & Sons Inc, 2014
- **P. G. M. WUTS**. Greene's Protective Groups in Organic Synthesis. JohnWiley & Sons Inc., 2014
- **HANS KAMERLING**. *Comprehensive Glycoscience From Chemistry to Systems Biology*, 2007
- *Angew Chem Int Ed Engl.*, 02 March 2020, vol. 59 (10), 4176-41
- *Organic Synthetic Chemistry*, 1984, vol. 42 (4)
- *Angew. Chem. Int. Ed.*, 2019, vol. 58, 11631-11636
- *Chem. Rev.*, 2015, vol. 115, 2174-2195
- *Analytical Sciences*, January 2018, vol. 35, 5-27
- *Angew. Chem. Int. Ed.*, 2019, vol. 58, 11631-11636
- **HERMANSON, G. T**. Bioconjugate Techniques. Academic Press, 1996, 456-493
- *Protein Science*, 1995, vol. 4, 2411-2423
- *CHEMICAL ABSTRACTS*, 646502-53-6
- *CHEMICAL ABSTRACTS*, 2130869-18-8
- *Clin Cancer Res*, 15 October 2016, vol. 22 (20)
- *Breast Cancer (Auckl)*, 20 May 2010, vol. 4, 35-41